# EUROPEAN PATENT APPLICATION

(11) **EP 2 390 308 A1**
(43) Date of publication of application: **30.11.2011**
(21) Application number: 11000796.0
(22) Date of filing: 28.05.2002
(51) Int. Cl.: C12N 1/00

(54) **Minicell compositions and methods**

(30) Priority: 25.02.2002 US 359843 P; 24.05.2002 US 154951
(62) Divisional of application: 02747872.6
(71) Applicant: Vaxiion Therapeutics Inc, San Diego, CA 92182-4614 (US)
(72) Inventor: Berkley, Neil, San Diego CA 92117 (US); Giacalone, Matthew, San Diego, CA 92104 (US); Sabbadini, Roger A., Lakeside CA 92040 (US); Surber, Mark, Coronada CA 92118 (US)
(74) Representative: Polypatent

(57) **Abstract**

The invention provides compositions and methods for the production of achromosomal and anucleate cells useful for applications such as diagnositic and therapeutic uses, as well as research tools and agents for drug discovery.

## Description

The invention is drawn to compositions and methods for the production of achromosomal archeabacterial, eubacterial and anucleate eukaryotic cells that are used as, e.g., therapeutics and/or diagnostics, reagents in drug discovery and functional proteomics, research tools, and in other applications as well.

The following description of the background of the invention is provided to aid in understanding the invention, but is not admitted to describe or constitute prior art to the invention. The contents of the articles, patents, and patent applications, and all other documents and electronically available information mentioned or cited in this application, are hereby incorporated by reference in their entirety to the same extent as if each individual publication was specifically and individually indicated to be incorporated by reference. Applicants reserve the right to physically incorporate into this application any and all materials and information from any such articles, patents, patent applications, or other documents.

Minicells are achromosomal cells that are products of aberrant cell division, and contain RNA and protein, but little or no chromosomal DNA. Clark-Curtiss and Curtiss III, Analysis of Recombinant DNA Using Escherichia coli Minicells, 101 Methods in Enzymology 347 (1983); Reeve and Mendelson, Minicells of Bacillus subtilis. A new system for transport studies in absence of macromolecular biosynthesis, 352 Biochim. Biophys. Acta 298-305 (1974). Minicells are capable of plasmid-directed synthesis of discrete polypeptides in the absence of synthesis directed by mRNA from the bacterial chromosome. Meagher et al., Protein Expression in E. coli Minicells by Recombinant Plasmids, 10 Cell 521, 523 (1977); Roozen et al., Synthesis of Ribonucleic Acid and Protein in Plasmid-Containing Minicells of Escherichia coli K-12, 107(1) J. of Bacteriology 21 (1971); and Curtiss III, Research on bacterial conjugation with minicells and minicell-producing E. coli strains, In: Microbial Drug Resistance, Editors Susumu Mitsuhashi & Hajime Hashimoto, p. 169 (Baltimore: University Park Press 1976). Early descriptions of minicells include those of Adler et al., Genetic control of cell division in bacteria, 154 Science 417 (1966), and Adler et al. (Miniature Escherichia coli cells deficient in DNA, 57 Proc. Nat. Acad. Sci (Wash.) 321 (1967)). However, discovery of the production of minicells can arguably be traced to the 1930's (Frazer and Curtiss III, Production, Properties and Utility of Bacterial Minicells, 69 Curr. Top. Microbiol. Immunol. 1-3 (1975)).

Prokaryotic (a.k.a. eubacterial) minicells have been used to produce various eubacterial proteins. See, e.g., Michael Gaâel, et al., The kdpF Subunit Is Part of the K+-translocating Kdp Complex of Escherichia coli and Is Responsible for Stabilization of the Complex in vitro, 274(53) Jn. of Biological Chemistry 37901 (1999); Harlow, et al., Cloning and Characterization of the gsk Gene Encoding Guanosine Kinase of Escherichia coli, 177(8) J. of Bacteriology 2236 (1995); Carol L. Pickett, et al., Cloning, Sequencing, and Expression of the Escherichia coli Cytolethal Distinding Toxin Genes, 62(3) Infection & Immunity 1046 (1994); Raimund Eck & Jörn Belter, Cloning and characterization of a gene coding for the catechol 1,2 dioxygenase of Arthrobacter sp. mA3, 123 Gene 87 (1993); Andreas Schlössser, et al, Subcloning, Nucleotide Sequence, and Expression of trkG, a Gene That Encodes an Integral Membrane Protein Involved in Potassium Uptake via the Trk System of Escherichia coli, 173(10) J. of Bacteriology 3170 (1991); Mehrdad Jannatipour, et al., Translocation of Vibrio harveyi N, N'-Diacetylchitobiase to the Outer Membrane of Escherichia coli 169(8) J. of Bacteriology 3785 (1987); and Jacobs et al., Expression of Mycobacterium leprae genes from a Streptococcus mutans promoter in Escherichia coli K-12, 83(6) Proc. Natl. Acad. Sci. USA 1926 (1986);

Various bacteria have been used, or proposed to be used, as gene delivery vectors to mammalian cells. For reviews, see Grillot-Courvalin et al., Bacteria as gene delivery vectors for mammalian cells, 10 Current Opinion in Biotechnology 477 (1999); Johnsen et al., Transfer of DNA from Genetically Modified Organisms (GMOs), Biotechnological Institute, 1-70 (2000); Sizemore et al., Attenuated Shigella as a DNA delivery vehicle for DNA-mediated immunization, 270(5234) Science 299 (1995); Patrice Courvalin, et al., Gene transfer from bacteria to mammalian cells, 318 C. R. Acad. Sci.1207 (1995); Sizemore, et al. Attenuated bacteria as a DNA delivery vehicle for DNA-mediated immunization, 15(8) Vaccine 804 (1997).

U.S. Patent No. 4,190,495, which issued February 26, 1980, to Curtiss is drawn to minicell producing strains of *E*. *coli* that are stated to be useful for the recombinant expression of proteins.

U.S. Patent No. 4,311,797, which issued January 19, 1982 to Khachatourians is stated to be drawn to a minicell based vaccine. The vaccine is stated to induce the production of antibodies against enteropathogenic *E*. *coli* cells in cattle and is stated to be effective against coliform enteritis.

Eubacterial minicells expressing immunogens from other prokaryotes have been described. Purcell et al., Molecular cloning and characterization of the 15-kilodalton major immunogen of Treponema pallidum, Infect. Immun. 57:3708, 1989.

In "Biotechnology: Promise ... and Peril" (IDRC Reports 9:4-7, 1980) authors Fleury and Shirkie aver that George Khachatourians at the Unveristy of Saskatchewan, Canada, "is working on a vaccine against cholera using 'minicells.'" The minciells are said to contain "genes from the pathogenic agent," and the "pathogen antigens are carried on the surface of the minicells" (p. 5, paragraph brigding the central and right columns).

Lundstrom et al., Secretion of Semliki Forest virus membrane glycoprotein E1 from Bacillus subtilis, Virus Res. 2:69-83, 1985, describe the expression of the E1 protein of the eukaryotic virus, Semliki Forest virus (SFV), in *Bacillus* minicells. The SFV E1 protein used in these studies is not the native E1 protein. Rather, it is a fusion protein in which the N-terminal signal sequence and C-terminal transmembrane domain have been removed and replaced with signal sequences from a gene from *Bacillus amyloliquefaciens.* The authors aver that "E1 is properly translocated through the cell membrane and secreted" (p. 81,1.1. 19-20), and note that "it has been difficult to express viral membrane proteins in prokaryotes" (p. 81, I. 27).

U.S. Patent No. 4,237,224, which issued December 2, 1980, to Cohen and Boyer, describes the expression of *X. Laevis* DNA in *E. coli* minicells.

U.S. patent application Serial No. 60/293,566 (attorney docket Nos. 078853-0401 and 089608-0201), is entitled "Minicell Compositions and Methods," and was filed May 24, 2001, by Sabbadini, Roger A., Berkley, Neil L., and Klepper, Robert E., and is hereby incorporated in its entirety by reference.

Jespersen et al. describes the use of "proteoliposomes" to generate antibodies to the AMPA receptor. Jespersen LK, Kuusinen A, Orellana A, Keinanen K, Engberg J. Use of proteoliposomes to generate phage antibodies against native AMPA receptor. Eur J Biochem. 2000 Mar;267(5):1382-9

The invention is drawn to compositions and methods for the production and use of minicells, including but not limited to eubacterial minicells, in applications such as diagnostics, therapeutics, research, compound screening and drug discovery, as well as agents for the delivery of nucleic acids and other bioactive compounds to cells.

Minicells are derivatives of cells that lack chromosomal DNA and which are sometimes referred to as anucleate cells. Because eubacterial and archeabacterial cells, unlike eukaryotic cells, do not have a nucleus (a distinct organelle that contains chromosomes), these non-eukaryotic minicells are more accurately described as being "without chromosomes" or "achromosomal," as opposed to "anucleate." Nonetheless, those skilled in the art often use the term "anucleate" when referring to bacterial minicells in addition to other minicells. Accordingly, in the present disclosure, the term "minicells" encompasses derivatives of eubacterial cells that lack a chromosome; derivatives of archeabacterial cells that lack their chromosome(s), and anucleate derivatives of eukaryotic cells. It is understood, however, that some of the relevant art may use the terms "anucleate minicells" or anucleate cells" loosely to refer to any of the preceeding types of minicells.

In one aspect, the invention is drawn to a eubacterial minicell comprising a membrane protein that is not naturally found in a prokaryote, i.e., a membrane protein from a eukaryote or an archeabacterium. Such minicells may, but need not, comprise an expression element that encodes and expresses the membrane protein that it comprises. The membrane protein may be one found in any non-eubacterial membrane, including, by way of non-limiting example, a cellular membrane, a nuclear membrane, a nucleolar membrane, a membrane of the endoplasmic reticulum (ER), a membrane of a Golgi body, a membrane of a lysosome a membrane of a peroxisome, a caveolar membrane, an outer membrane of a mitochondrion or a chloroplast, and an inner membrane of a mitochondrion or a chloroplast. By way of non-limiting example, a membrane protein may be a receptor, such as a G-protein coupled receptor; an enzyme, such as ATPase or adenylate cyclase, a cytochrome; a channel; a transporter; or a membrane-bound nucleic acid binding factor, such as a transcription and/or translation factor; signaling components; components of the electon transport chain (ETC); or cellular antigens. A membrane fusion protein, which is generated in vitro using molecular cloning techniques, does not occur in nature and is thus a membrane protein that is not naturally found in a prokaryote, even if the fusion protein is prepared using amino acid sequences derived from eubacterial proteins.

Minicells that have segregated from parent cells lack chromosomal and/or nuclear components, but retain the cytoplasm and its contents, including the cellular machinery required for protein expression. Although chromosomes do not segregate into minicells, extrachromosomal and/or episomal genetic expression elements will segregate, or may be introduced into mincells after segregation from parent cells. Thus, in one aspect, the invention is drawn to minicells comprising an expression element, which may be an inducible expression element, that comprises expression sequences operably linked to an open reading frame (ORF) that encodes the non-eubacterial membrane protein. In a related aspect, the invention is drawn to minicell-producing host cells having an expression element, which may be an inducible expression element, that comprises expression sequences operably linked to an ORF that encodes a non-eubacterial membrane protein. In a related aspect, the invention is drawn to a method of making a eubacterial minicell comprising a membrane protein that is not naturally found in a prokaryote, the method comprising growing minicell-producing host cells, the host cells having an expression element, which may be an inducible expression element, that comprises expression sequences operably linked to an ORF that encodes a non-eubacterial membrane protein; and preparing minicells from the host cells. Optionally, at any point in the method, an inducing agent is provided in order to induce expression of an ORF that encodes a non-eubacterial membrane protein.

In one aspect, the invention is drawn to display produced membrane-associated protein(s) on the surface of the minicell. For purposes of this document, the term "display" is defined as exposure of the structure of interest on the outer surface of the minicell. By way of non-limiting example, this structure may be an internally expressed membrane protein or chimeric construct to be inserted in or associated with the minicell membrane such that the extracellular domain or domain of interest is exposed on the outer surface of the minicell (expressed and displayed on the surface of the minicell or expressed in the parental cell to be displayed on the surface of the segregated minicell). In any scenario, the "displayed" protein or protein domain is available for interaction with extracellular components. A membrane-associated protein may have more than one extracellular domain, and a minicell of the invention may display more than one membrane-associated protein.

The membrane protein displayed by minicells may be a fusion protein, i.e., a protein that comprises a first polypeptide having a first amino acid sequence and a second polypeptide having a second amino acid sequence, wherein the first and second amino acid sequences are not naturally present in the same polypeptide. At least one polypeptide in a membrane fusion protein is a "transmembrane domain" or "membrane-anchoring domain". The transmembrane and membrane-anchoring domains of a membrane fusion protein may be selected from membrane proteins that naturally occur in a eucaryote, such as a fungus, a unicellular eucaryote, a plant and an animal, such as a mammal including a human. Such domains may be from a viral membrane protein naturally found in a virus such as a bacteriophage or a eucaryotic virus, e.g., an adenovirus or a retrovirus. Such domains may be from a membrane protein naturally found in an archaebacterium such as a thermophile.

The displayed domain of a membrane fusion protein may be a binding moiety. By way of non-limiting example, binding moieties used for particular purposes may be a binding moiety directed to a compound or moiety displayed by a specific cell type or cells found predominantly in one type of tissue, which may be used to target minicells and their contents to specific cell types or tissues; or a binding moiety that is directed to a compound or moiety displayed by a pathogen, which may be used in diagnostic or therapeutic methods; a binding moiety that is directed to an undesirable compound, such as a toxin, which may be used to bind and preferably internalize and/or neutralize the undesirable compound; a diseased cell; or the binding moiety may be a domain that allows for the minicells to be covalently or non-covalently attached to a support material, which may be used in compositions and methods for compound screening and drug discovery. By "diseased cell" it is meant pathogen-infected cells, malfunctioning cells, and dysfunctional cells, e.g., cancer cells.

In various aspects, the minicells of the invention comprise one or more biologically active compounds. The term "biologically active" (synonymous with "bioactive") indicates that a composition or compound itself has a biological effect, or that it modifies, causes, promotes, enhances, blocks, reduces, limits the production or activity of, or reacts with or binds to an endogenous molecule that has a biological effect. A "biological effect" may be but is not limited to one that stimulates or causes an immunoreactive response; one that impacts a biological process in an animal; one that impacts a biological process in a pathogen or parasite; one that generates or causes to be generated a detectable signal; and the like. Biologically active compositions, complexes or compounds may be used in therapeutic, prophylactic and diagnostic methods and compositions. Biologically active compositions, complexes or compounds act to cause or stimulate a desired effect upon an animal. Non-limiting examples of desired effects include, for example, preventing, treating or curing a disease or condition in an animal suffering therefrom; limiting the growth of or killing a pathogen in an animal infected thereby; augmenting the phenotype or genotype of an animal; stimulating a prophylactic immunoreactive response in an animal; or diagnosing a disease or disorder in an animal.

In the context of therapeutic applications of the invention, the term "biologically active" indicates that the composition, complex or compound has an activity that impacts an animal suffering from a disease or disorder in a positive sense and/or impacts a pathogen or parasite in a negative sense. Thus, a biologically active composition, complex or compound may cause or promote a biological or biochemical activity within an animal that is detrimental to the growth and/or maintenance of a pathogen or parasite; or of cells, tissues or organs of an animal that have abnormal growth or biochemical characteristics, such as cancer cells.

In the context of diagnostic applications of the invention, the term "biologically active" indicates that the composition, complex or compound can be used for *in vivo* or *ex vivo* diagnostic methods and in diagnostic compositions and kits. For diagnostic purposes, a preferred biologically active composition or compound is one that can be detected, typically (but not necessarily) by virtue of comprising a detectable polypeptide. Antibodies to an epitope found on composition or compound may also be used for its detection.

In the context of prophylactic applications of the invention, the term "biologically active" indicates that the composition or compound induces or stimluates an immunoreactive response. In some preferred embodiments, the immunoreactive response is designed to be prophylactic, *i.e*., prevents infection by a pathogen. In other preferred embodiments, the immunoreactive response is designed to cause the immune system of an animal to react to the detriment of cells of an animal, such as cancer cells, that have abnormal growth or biochemical characteristics. In this application of the invention, compositions, complexes or compounds comprising antigens are formulated as a vaccine.

It will be understood by those skilled in the art that a given composition, complex or compound may be biologically active in therapeutic, diagnostic and prophylactic applications. A composition, complex or compound that is described as being "biologically active in a cell" is one that has biological activity *in vitro* (*i.e*., in a cell culture) or *in vivo* (*i.e*., in the cells of an animal). A "biologically active component" of a composition or compound is a portion thereof that is biologically active once it is liberated from the composition or compound. It should be noted, however, that such a component may also be biologically active in the context of the composition or compound.

In one aspect, the minicells of the invention comprise a therapeutic agent. Such minicells may be used to deliver therapeutic agents. In a preferred embodiment, a minicell comprising a therapeutic agent displays a binding moiety that specifically binds a ligand present on the surface of a cell, so that the minicells may be "targeted" to the cell. The therapeutic agent may be any type of compound or moiety, including without limitation small molecules, polypeptides, antibodies and antibody derivatives and nucleic acids. The therapeutic agent may be a drug; a prodrug, i.e., a compound that becomes biologically active *in vivo* after being introduced into a subject in need of treatment; or an immunogen.

In one aspect, the minicells of the invention comprise a detectable compound or moiety. As is understood by those of skill in the art, a compound or moiety that is "detectable" produces a signal that can detected by spectroscopic, photochemical, biochemical, immunochemical, electromagnetic, radiochemical, or chemical means such as fluorescence, chemifluoresence, or chemiluminescence, electrochemilumenscence, or any other appropriate means. A detectable compound may be a detectable polypeptide, and such polypeptides may, but need not, be incorporated into fusion membrane proteins of the minicell. Detectable polypeptides or amino acid sequences, includes, by way of non-limiting example, a green fluorescent protein (GFP), a luciferase, a beta-galactosidase, a His tag, an epitope, or a biotin-binding protein such as streptavidin or avidin. The detectable compound or moiety may be a radiolabeled compound or a radioisotope. A detectable compound or moiety may be a small molecule such as, by way of non-limiting example, a fluorescent dye; a radioactive iostope; or a compound that may be detected by x-rays or electromagnetic radiation. Image enhancers as those used for CAT and PET scans (e.g., calcium, gallidium) may be used. In another non-limiting example, detectable labels may also include loss of catalytic substrate or gain of catalytic product following catalysis by a minicell displayed, solule cytoplasmic, or secreted enzyme.

In one aspect, the invention is drawn to a minicell comprising one or more bioactive nucleic acids or templates thereof. By way of non-limiting example, a bioactive nucleic acid may be an antisense oligonucleotide, an aptamer, an antisense transcript, a ribosomal RNA (rRNA), a transfer RNA (tRNA), a molecular decoy, or an enzymatically active nucleic acid, such as a ribozyme. Such minicells can, but need not, comprise a displayed polypeptide or protein on the surface of the minicell. The displayed polypeptide or protein may be a binding moiety directed to a compound or moiety displayed by a particular type of cell, or to a compound or moiety displayed by a pathogen. Such minicells can further, but need not, comprise an expression element having eubacterial, archael, eucaryotic, or viral expression sequences operably linked to a nucleotide sequence that serves as a template for a bioactive nucleic acid.

In one aspect, the invention is drawn to immunogenic minicells, i.e., minicells that display an immunogen, vaccines comprising immunogenic minicells, antibodies and antibody derivatives directed to immunogens displayed on immunogenic minicells, and method of making and using immunogenic minicells and antibodies and antibody derivatives produced therefrom in prophylactic, diagnostic, therapeutic and research applications. A preferred immunogen displayed by a minicell is an immunogenic polypeptide, which is preferably expressed from an expression element contained within the minicell in order to maximize the amount of immunogen displayed by the immunogenic minicells. The immunogenic polypeptide can be derived from any organism, obligate intracelluar parasite, organelle or virus with the provisio that, in prophylactic applications, the immunogenic polypeptide is not derived from a prokaryote, including a eubacterial virus. The source organism for the immunogen may be a pathogen. A minicell displaying an immunogen derived from a pathogen is formulated into a vaccine and, in a prophylactic application, used to treat or prevent diseases and disorders caused by or related to the eukaryotic or archeabacterial pathogen.

In a separate aspect, the invention is drawn to minicells that display an immunogen derived from a nonfunctional, dysfunctional and/or diseased cell. By way of non-limiting example, the minicells display an immunogenic polypeptide derived from a hyperproliferative cell, i.e., a cell that is tumorigenic, or part of a tumor or cancer. As another non-limiting example, a cell that is infected with a virus or an obligate intracellular parasite (e.g., *Rickettsiae*) displays an immunogenic polypeptide that is encoded by the genome of the infected cell but is aberrenatly expressed in an infected cell. A vaccine comprising a minicell displaying an immunogen derived from a nonfunctional, dysfunctional and/or diseased cell is used in methods of treating or preventing hyperproliferative diseases or disorders, including without limitation a cell comprising an intracellular pathogen.

In one aspect, the invention is drawn to a minicell comprising a membrane protein that is linked to a conjugatable compound (a.k.a. "attachable compound"). The conjugatable compound may be of any chemical nature and have one or more therapeutic or detectable moities. By way of non-limiting example, a protein having a transmembrane or membrane anchoring domain is displayed and has the capacity to be specifically cross-linked on its extracellular domain. Through this approach, any conjugatable compound of interest may be quickly and easily attached to the outer surface of minicells containing this expressed membrane-spanning domain. In aspects of the invention wherein minicells are used for drug delivery in vivo, a preferred conjugatable compound is polyethylene glycol (PEG), which provides for "stealth" minicells that are not taken as well and/or as quickly by the reticuloendothelial system (RES). Other conjugatable compounds include polysaccharides, polynucleotides, lipopolysaccharides, lipoproteins, glycosylated proteins, synthetic chemical compounds, and/or chimeric combinations of these examples listed.

In various aspects of the invention, the minicell displays a polypeptide or other compound or moiety on its surface. By way of non-limiting example, a non-eubacterial membrane protein displayed by eubacterial minicells may be a receptor. Minicells displaying a receptor may, but need not, bind ligands of the receptor. In therapeutic applications of this aspect of the invention, the ligand is an undesirable compound that is bound to its receptor and, in some aspects, is internalized by the minicells. The non-eubacterial membrane protein displayed by minicells may be a fusion protein, i.e., a protein that comprises a first polypeptide having a first amino acid sequence and a second polypeptide having a second amino acid sequence, wherein the first and second amino acid sequences are not naturally present in the same polypeptide. At least one polypeptide in a membrane fusion protein is a "transmembrane domain" or "membrane-anchoring domain". The transmembrane and membrane-anchoring domains of a membrane fusion protein may be selected from membrane proteins that naturally occur in a eukaryote, such as a fungus, a unicellular eukaryote, a plant and an animal, such as a mammal including a human. Such domains may be from a viral membrane protein naturally found in a virus such as a bacteriophage or a eukaryotic virus, e.g., an adenovirus or a retrovirus. Such domains may be from a membrane protein naturally found in an archaebacterium such as a thermophile.

The displayed domain of a membrane fusion protein may be a binding moiety. By way of non-limiting example, binding moieties used for particular purposes may be a binding moiety directed to a compound or moiety displayed by a specific cell type or cells found predominantly in one type of tissue, which may be used to target minicells and their contents to specific cell types or tissues; or a binding moiety that is directed to a compound or moiety displayed by a pathogen, which may be used in diagnostic or therapeutic methods; a binding moiety that is directed to an undesirable compound, such as a toxin, which may be used to bind and preferably internalize and/or neutralize the undesirable compound; a diseased cell; or the binding moiety may be a domain that allows for the minicells to be covalently or non-covalently attached to a support material, which may be used in compositions and methods for compound screening and drug discovery.

In one aspect, the invention provides minicells that may be used as research tools and/or kits comprising such research tools. The minicells of the invention may be used as is, or incorporated into research tools useful for scientific research regarding all amino acid comprising compounds including, but not limited to membrane-associated proteins, chimeric membrane fusion proteins, and soluble proteins. Such scientific research includes, by way of non-limiting example, basic research, as well as pharmacological, diagnostic, and pharmacogenetic studies. Such studies may be carried out in vivo or in vitro.

In other aspects, the invention is drawn to methods of preparing the minicells, protoplasts, and poroplasts™ of the invention for various applications including but not limited to diagnostic, therapeutic, research and screening applications. In a related aspect, the invention is drawn to pharmaceutical compositions, reagents and kits comprising minicells.

In each aspect and embodiment of the invention, unless stated otherwise, embodiments wherein the minicell is a eubacterial minicell, a poroplast, a spheroplast or a protoplast exist.

In a first aspect, the invention provides a minicell comprising a membrane protein selected from the group consisting of a eukaryotic membrane protein, an archeabacterial membrane protein and an organellar membrane protein. In another embodiment, wherein the minicell comprises a biologically active compound. By way of non-limiting example, the biologically active compound is a radioisotope, a polypeptide, a nucleic acid or a small molecule.

In another embodiment, the minicell comprises a expression construct, wherein the first expression construct comprises expression sequences operably linked to an ORF that encodes a protein. In another embodiment, the ORF encodes the membrane protein. In another embodiment, the expression sequences that are operably linked to an ORF are inducible and/or repressible.

In another aspect, the minicell comprises a second expression construct, wherein the second expression construct comprises expression sequences operably linked to a gene. In another embodiment, the expression sequences that are operably linked to a gene are inducible and/or repressible. In a related embodiment, the gene product of the gene regulates the expression of the ORF that encodes the protein. A factor that "regulates" the expression of a gene or a gene product directly or indirectly initiates, enhances, quickens, slows, terminates, limits or completely blocks expression of a gene. In different embodiments, the gene product of the gene is a nucleic acid or a polypeptide. The polypeptide can be of any type, including but not limited to a membrane protein, a soluble protein or a secreted protein. A membrane protein can be a membrane fusion protein comprising a first polypeptide, which comprises at least one transmembrane domain or at least one membrane anchoring domain; and a second polypeptide.

In one aspect, the invention provides a minicell comprising a membrane fusion protein, the fusion protein comprising a first polypeptide, the first polypeptide comprising at least one transmembrane domain or at least one membrane anchoring domain; and a second polypeptide, wherein the second polypeptide is not derived from a eubacterial protein and is neither a His tag nor a glutathione-S-transferase polypeptide. In various embodiments, the minicell is a eubacterial minicell, a poroplast, a spheroplast or a protoplast. In one embodiment, the minicell comprises a biologically active compound.

In one aspect, the invention provides a minicell comprising a membrane conjugate, wherein the membrane conjugate comprises a membrane protein chemically linked to a conjugated compound. In one embodiment, the conjugated compound is selected from the group consisting of a nucleic acid, a polypeptide, a lipid and a small molecule.

In one aspect, the invention provides a method for making minicells, comprising (a) culturing a minicell-producing parent cell, wherein the parent cell comprises an expression construct, wherein the expression construct comprises a gene operably linked to expression sequences that are inducible and/or repressible, and wherein induction or repression of the gene causes or enhances the production of minicells; and (b) separating the minicells from the parent cell, thereby generating a composition comprising minicells, wherein an inducer or repressor is present within the parent cells during one or more steps and/or between two or more steps of the method. In one embodiment, the method further comprises (c) purifying the minicells from the composition.

Relevant gene products are factors involved in or modulating DNA replication, cellular division, cellular partitioning, septation, transcription, translation, or protein folding. The minicells are separated from parent cells by processes such as centrifugation, ultracentrifugation, density gradation, immunoaffinity, immunoprecipitation and other techniques described herein.

In one embodiment, the minicell is a poroplast, and the method further comprises (d) treating the minicells with an agent, or incubating the minicells under a set of conditions, that degrades the outer membrane of the minicell. The outer membrane is degraded by treatment with an agent selected from the group consisting of EDTA, EGTA, lactic acid, citric acid, gluconic acid, tartaric acid, polyethyleneimine, polycationic peptides, cationic leukocyte peptides, aminoglycosides, aminoglycosides, protamine, insect cecropins, reptilian magainins, polymers of basic amino acids, polymixin B, chloroform, nitrilotriacetic acid and sodium hexametaphosphate; by exposure to conditions selected from the group consisting of osmotic shock and insonation; and by other methods described herein.

In one embodiment, further comprising removing one or more contaminants from the composition. Representative contaminants are LPS and peptidoglycan. In a representative embodiment, LPS is removed by contacting the composition to an agent that binds or degrades LPS. At least about 50%, preferably about 65% to about 75%, more preferably 95%, most preferably 99% or >99% of LPS is removed from an initial preparation of minicells. In a related embodiment, the minicell-producing parent cell comprises a mutation in a gene required for lipopolysaccharide synthesis.

In on embodiment, the minicell is a spheroplast, and the method further comprises (d) treating the minicells with an agent, or incubating the minicells under a set of conditions, that disrupts or degrades the outer membrane; and (e) treating the minicells with an agent, or incubating the minicells under a set of conditions, that disrupts or degrades the cell wall. The agent that disrupts or degrades the cell wall can be. e.g., a lysozyme, and the set of conditions that disrupts or degrades the cell wall can be, e.g., incubation in a hypertonic solution.

In one embodiment, the minicell is a protoplast, and the method further comprises (d treating the minicells with an agent, or incubating the minicells under a set of conditions, that disrupt or degrade the outer membrane; (e) treating the minicells with an agent, or incubating the minicells under a set of conditions, that disrupts or degrades the cell wall, in order to generate a composition that comprises protoplasts; and (f) purifying protoplasts from the composition. In one embodiment, the method further comprises preparing a denuded minicell from the minicell. In one embodiment, the method further comprises covalently or non-covalently linking one or more components of the minicell to a conjugated moiety.

In one aspect, the invention provides a L-form minicell comprising (a) culturing an L-form eubacterium, wherein the eubacterium comprises one or more of the following: (i) an expression element that comprises a gene operably linked to expression sequences that are inducible and/or repressible, wherein induction or repression of the gene regulates the copy number of an episomal expression construct; (ii) a mutation in an endogenous gene, wherein the mutation regulates the copy number of an episomal expression construct; (iii) an expression element that comprises a gene operably linked to expression sequences that are inducible and/or repressible, wherein induction or repression of the gene causes or enhances the production of minicells; and (iv) a mutation in an endogenous gene, wherein the mutation causes or enhances minicell production; (b) culturing the L-form minicell-producing parent cell in media under conditions wherein minicells are produced; and (c) separating the minicells from the parent cell, thereby generating a composition comprising L-form minicells, wherein an inducer or repressor is present within the minicells during one or more steps and/or between two or more steps of the method. In one embodiment, the method further comprises (d) purifying the L-form minicells from the composition.

In one aspect, the invention provides a solid support comprising a minicell, wherein the minicell displays a fusion protein, the fusion protein comprising a first polypeptide that comprises at least one transmembrane domain or at least one membrane anchoring domain, and a second polypeptide. In various embodiments, the second polypeptide comprises a binding moiety or an enzyme moiety.

In one aspect, the invention provides a eubacterial minicell comprising a biologically active compound, wherein the minicell displays a binding moiety, wherein the binding moiety is selected from the group consisting of (a) a eukaryotic membrane protein; (b) an archeabacterial membrane protein; (c) an organellar membrane protein; and (d) a fusion protein, the fusion protein comprising a first polypeptide, the first polypeptide comprising at least one transmembrane domain or at least one membrane anchoring domain; and a second polypeptide, wherein the second polypeptide is not derived from a eubacterial protein and is neither a His tag nor a glutathione-S-transferase polypeptide, and wherein the polypeptide comprises a binding moiety.

In one embodiment, the binding moiety is selected from the group consisting of an antibody, an antibody derivative, a receptor and an active site of a non-catalytic derivative of an enzyme. In a preferred embodiment, the binding moiety is a single-chain antibody. In one embodiment, one of the ORFs encodes a protein that comprises the binding moiety.

In one embodiment, the binding moiety is directed to a ligand selected from the group consisting of an epitope displayed on a pathogen, an epitope displayed on an infected cell and an epitope displayed on a hyperproliferative cell.

In one embodiment, the invention further comprises a first and second nucleic acid, wherein the first nucleic acid comprises eukaryotic expression sequences operably linked to a first ORF, and a second nucleic acid, wherein the second nucleic acid comprises eubacterial expression sequences operably linked to a second ORF.

In one embodiment, the eubacterial expression sequences are induced and/or derepressed when the binding moiety is in contact with a target cell. In a variant embodiment, the eukaryotic expression sequences are induced and/or derepressed when the nucleic acid is in the cytoplasm of a eukaryotic cell. In related embodiments, the protein encoded by the first ORF comprises eukaryotic secretion sequences and/or the protein encoded by the second ORF comprises eubacterial secretion sequences.

In one aspect, the invention provides a method of associating a radioactive compound with a cell, wherein the cell displays a ligand specifically recognized by a binding moiety, comprising contacting the cell with a minicell that comprises the radioactive compound and displays the binding moiety. In a diagnostic embodiment, the amount of radiation emitted by the radioactive isotope is sufficient to be detectable. In a therapeutic embodiment, the amount of radiation emitted by the radioactive isotope is sufficient to be cytotoxic. In one embodiment, the ligand displayed by the cell is selected from the group consisting of an epitope displayed on a pathogen, an epitope displayed on an infected cell and an epitope displayed on a hyperproliferative cell. In one embodiment, the binding moiety is selected from the group consisting of an antibody, an antibody derivative, a channel protein and a receptor, and is preferably a single-chain antibody. In other embodiments, the binding moiety is an aptamer or a small molecule. In one embodiment, the ligand is selected from the group consisting of a cytokine, hormone, and a small molecule.

In one aspect, the invention provides a method of delivering a biologically active compound to a cell, wherein the cell displays a ligand specifically recognized by a binding moiety, comprising contacting the cell with a minicell that displays the binding moiety, wherein the minicell comprises the biologically active compound, and wherein the contents of the minicell are delivered into the cell from a minicell bound to the cell. In one embodiment, the biologically active compound is selected from the group consisting of a nucleic acid, a lipid, a polypeptide, a radioactive compound, an ion and a small molecule.

In one embodiment, the membrane of the minicell comprises a system for transferring a molecule from the interior of a minicell into the cytoplasm of the cell. A representative system for transferring a molecule from the interior of a minicell into the cytoplasm of the cell is a Type III secretion system.

In one embodiment, the minicell further comprises a first and second nucleic acid, wherein the first nucleic acid comprises eukaryotic expression sequences operably linked to a first ORF, and a second nucleic acid, wherein the second nucleic acid comprises eubacterial expression sequences operably linked to a second ORF. In one embodiment, one of the ORFs encodes a protein that comprises the binding moiety. In one embodiment, the eubacterial expression sequences are induced and/or derepressed when the binding moiety is in contact with a target cell. In one embodiment, the eukaryotic expression sequences are induced and/or derepressed when the nucleic acid is in the cytoplasm of a eukaryotic cell. In one embodiment, the protein encoded by the first ORF comprises eukaryotic secretion sequences and/or the protein encoded by the second ORF comprises eubacterial secretion sequences. In one embodiment, the ligand is selected from the group consisting of a cytokine, hormone, and a small molecule.

In one aspect, the invention provides a minicell displaying a synthetic linking moiety, wherein the synthetic linking moiety is covalenty or non-covalently attached to a membrane component of the mincell.

In one aspect, the invention provides a sterically stabilized minicell comprising a displayed moiety that has a longer half-life in vivo than a wild-type minicell, wherein the displayed moiety is a hydrophilic polymer that comprises a PEG moiety, a carboxylic group of a polyalkylene glycol or PEG stearate.

In one aspect, the invention provides a minicell having a membrane comprising an exogenous lipid, wherein a minicell comprising the exogenous lipid has a longer half-life in vivo than a minicell lacking the exogenous lipid, and wherein the minicell is selected from the group consisting of a eubacterial minicell, a poroplast, a spheroplast and a protoplast. In one embodiment, the exogenous lipid is a derivitized lipid which may, by way of non-limiting example, be phosphatidylethanolamine derivatized with PEG, DSPE-PEG, PEG stearate; PEG-derivatized phospholipids, a PEG ceramide or DSPE-PEG.

In one embodiment, the exogenous lipid is not present in a wild-type membrane, or is present in a different proportion than is found in minicells comprising a wild-type membrane. The exogenous lipid can be a ganglioside, sphingomyelin, monosialoganglioside GM1, galactocerebroside sulfate, 1,2-sn-dimyristoylphosphatidylcholine, phosphatidylinositol and cardiolipin.

In one embodiment, the linking moiety is non-covalently attached to the minicell. In one embodiment, one of the linking moiety and the membrane component comprises biotin, and the other comprises avidin or streptavidin. In one embodiment, the synthetic linking moiety is a cross-linker. In one embodiment, the cross-linker is a bifunctional cross-linker.

In one aspect, the invention provides a method of transferring a membrane protein from a minicell membrane to a biological membrane comprising contacting a minicell to the biological membrane, wherein the minicell membrane comprises the membrane protein, and allowing the mincell and the biological membrane to remain in contact for a period of time sufficient for the transfer to occur.

In one embodiment, the biological membrane is a cytoplasmic membrane or an organellar membrane. In one embodiment, the biological membrane is a membrane selected from the group consisting of a membrane of a pathogen, a membrane of an infected cell and a membrane of a hyperproliferative cell. In one embodiment, the biological membrane is the cytoplasmic membrane of a recipient cell, which may be a cultured cell and a cell within an organism. In one embodiment, the biological membrane is present on a cell that has been removed from an animal, the contacting occurs in vitro, after which the cell is returned to the organism.

In one embodiment, the membrane protein is an enzyme. In this embodiment, the membrane protein having enzymatic activity is selected from the group consisting of a cytochrome P450 and a fusion protein, the fusion protein comprising a first polypeptide, the first polypeptide comprising at least one polypeptide, wherein the second polypeptide has enzymatic acitivity.

In one embodiment, the membrane protein is a membrane fusion protein, the membrane fusion protein comprising a first polypeptide, the first polypeptide comprising at least one transmembrane domain or at least one membrane anchoring domain; and a second polypeptide.

In one embodiment, the second polypeptide is a biologically active polypeptide. In one embodiment, the minicell displays ligand or a binding moiety.

In one aspect, the invention provides a minicell that comprises an expression construct comprising an ORF encoding a membrane protein operably linked to expression sequences, wherein the expression sequences are induced and/or derepressed when the minicell is in contact with a target cell.

In one embodiment, the biological membrane is a cytoplasmic membrane or an organellar membrane. In one embodiment, the biological membrane is a membrane selected from the group consisting of a membrane of a pathogen, a membrane of an infected cell and a membrane of a hyperproliferative cell. In one embodiment, the minicell displays a ligand or a binding moiety selected from the group consisting of an antibody, an antibody derivative, an aptamer and a small molecule. In one embodiment, the membrane protein is a membrane fusion protein, the membrane fusion protein comprising a first polypeptide, the first polypeptide comprising at least one transmembrane domain or at least one membrane anchoring domain; and a second polypeptide. In one embodiment, the ligand is selected from the group consisting of a cytokine, hormone, and a small molecule.

In one aspect, the invention provides a pharmaceutical composition comprising a minicell, wherein the minicell displays a membrane protein, wherein the membrane protein is selected from the group consisting of a eukaryotic membrane protein, an archeabacterial membrane protein and an organellar membrane protein. In one embodiment, the membrane protein is selected from the group consisting of a receptor, a channel protein, a cellular adhesion factor and an integrin. In one embodiment, the pharmaceutical formulation further comprises an adjuvant. In one embodiment, the membrane protein comprises a polypeptide epitope displayed by a hyperproliferative cell. In one embodiment, the membrane protein comprises an epitope displayed by a eukaryotic pathogen, an archeabacterial pathogen, a virus or an infected cell.

In one aspect, the invention provides a pharmaceutical composition comprising a minicell, wherein the minicell displays a membrane protein that is a fusion protein, the fusion protein comprising (i) a first polypeptide, the first polypeptide comprising at least one transmembrane domain or at least one membrane anchoring domain; and (ii) a second polypeptide, wherein the second polypeptide is not derived from a eubacterial protein. In one embodiment, the pharmaceutical formulation further comprises an adjuvant. In one embodiment, the second polypeptide comprises a polypeptide epitope displayed by a hyperproliferative cell. In one embodiment, the membrane protein comprises an epitope displayed by a eukaryotic pathogen, an archeabacterial pathogen, a virus or an infected cell.

In one aspect, the invention provides a pharmaceutical composition comprising a minicell, wherein the minicell displays a membrane conjugate, wherein the membrane conjugate comprises a membrane component chemically linked to a conjugated compound. In one embodiment, the membrane protein is selected from the group consisting of a receptor, a channel protein, a cellular adhesion factor and an integrin. In one embodiment, the pharmaceutical further comprises an adjuvant. In one embodiment, the membrane component is a polypeptide comprising at least one transmembrane domain or at least one membrane anchoring domain, or a lipid that is part of a membrane. In one embodiment, the conjugated compound is a polypeptide, and the chemical linkage between the membrane compound and the conjugated compound is not a peptide bond. In one embodiment, the conjugated compound is a nucleic acid. In one embodiment, the conjugated compound is an organic compound. In one embodiment, the organic compound is selected from the group consisting of a narcotic, a toxin, a venom, a sphingolipid and a soluble protein.

In one aspect, the invention provides a method of making a pharmaceutical composition comprising a minicell, wherein the minicell displays a membrane protein, wherein the membrane protein is selected from the group consisting of a eukaryotic membrane protein, an archeabacterial membrane protein and an organellar membrane protein. In one embodiment, the method further comprises adding an adjuvant to the pharmaceutical formulation. In one embodiment, the method further comprises desiccating the formulation. In one embodiment, the method further comprises adding a suspension buffer to the formulation. In one embodiment, the method further comprises making a chemical modification of the membrane protein. In one embodiment, the chemical modification is selected from the group consisting of glycosylation, deglycosylation, phosphorylation, dephosphorylation and proteolysis. In one aspect, the invention provides a method of making a pharmaceutical composition comprising a minicell, wherein the minicell displays a membrane protein that is a fusion protein, the fusion protein comprising (i) a first polypeptide, the first polypeptide comprising at least one transmembrane domain or at least one membrane anchoring domain; and (ii) a second polypeptide, wherein the second polypeptide is not derived from a eubacterial protein.

In one aspect, the invention provides a method of making a pharmaceutical formulation comprising a minicell, wherein the minicell displays a membrane conjugate, wherein the membrane conjugate comprises a membrane component chemically linked to a conjugated compound. In one embodiment, the method further comprises adding an adjuvant to the pharmaceutical formulation. In one embodiment, the membrane component is a polypeptide comprising at least one transmembrane domain or at least one membrane anchoring domain, or a lipid that is part of a membrane. In one embodiment, the conjugated compound is a polypeptide, and the chemical linkage between the membrane compound and the conjugated compound is not a peptide bond. In one embodiment, the conjugated compound is a nucleic acid. In one embodiment, the conjugated compound is an organic compound. In one embodiment, the organic compound is selected from the group consisting of a narcotic, a toxin, a venom, and a sphingolipid.

In one aspect, the invention provides a method of detecting an agent that is specifically bound by a binding moiety, comprising contacting a minicell displaying the binding moiety with a composition known or suspected to contain the agent, and detecting a signal that is modulated by the binding of the agent to the binding moiety. In one embodiment, the agent is associated with a disease. In one embodiment, the minicell comprises a detectable compound. In one embodiment, the binding moiety is antibody or antibody derivative. In one embodiment, the composition is an environmental sample. In one embodiment, the composition is a biological sample. In one embodiment, the biological sample is selected from the group consisting of blood, serum, plasma, urine, saliva, a biopsy sample, feces and a skin patch.

In one aspect, the invention provides a method of in situ imaging of a tissue or organ, comprising administering to an organism a minicell comprising an imaging agent and a binding moiety and detecting the imaging agent in the organism.

In one embodiment, the minicell is a eubacterial minicell, a poroplast, a spheroplast or a protoplast. In one embodiment, the binding moiety is an antibody or antibody derivative. In one embodiment, the binding moiety specifically binds a cell surface antigen. In one embodiment, the cell surface antigen is an antigen displayed by a tumorigenic cell, a cancer cell, and an infected cell. In one embodiment, the cell surface antigen is a tissue-specific antigen. In one embodiment, the method of imaging is selected from the group consisting of magnetic resonance imaging, ultrasound imaging; and computer axaial tomography (CAT). In one aspect, the invention provides a device comprising a microchip operatively associated with a biosensor comprising a minicell, wherein the microchip comprises or contacts the minicell, and wherein the minicell displays a binding moiety.

In one aspect, the invention provides a method of determining the rate of transfer of nucleic acid from a minicell to a cell, comprising (a) contacting the cell to the minicell, wherein the minicell comprises the nucleic acid, for a measured period of time; (b) separating minicells from the cells; (c) measuring the amount of nucleic acid in the cells,wherein the amount of nucleic acid in the cells over the set period of time is the rate of transfer of a nucleic acid from a minicell.

In one aspect, the invention provides a method of determining the amount of a nucleic acid transferred to a cell from a minicell, comprising (a) contacting the cell to the minicell, wherein the minicell comprises an expression element having eukaryotic expression sequences operably linked to an ORF encoding a detectable polypeptide, wherein the minicell displays a binding moiety, and wherein the binding moiety binds an epitope of the cell; and (b) detecting a signal from the detectable polypeptide, wherein a change in the signal corresponds to an increase in the amount of a nucleic acid transferred to a cell.

In one embodiment, the cell is a eukaryotic cell. By way of non-limiting example, a eukaryotic cell can be a plant cell, a fungal cell, a unicellular eukaryote, an animal cell, a mammalian cell, a rat cell, a mouse cell, a primate cell or a human cell.

In one embodiment, the binding moiety is an antibody or antibody derivative. In one embodiment, the binding moiety is a single-chain antibody. In one embodiment, the binding moiety is an aptamer. In one embodiment, the binding moiety is an organic compound. In one embodiment, the detectable polypeptide is a fluorescent polypeptide.

In one aspect, the invention provides a method of detecting the expression of an expression element in a cell, comprising (a) contacting the cell to a minicell, wherein the minicell comprises an expression element having cellular expression sequences operably linked to an ORF encoding a detectable polypeptide, wherein the minicell displays a binding moiety, and wherein the binding moiety binds an epitope of the cell; (b) incubating the cell and the minicell for a period of time effective for transfer of nucleic acid from the minicell to the cell; and (c) detecting a signal from the detectable polypeptide, wherein an increase in the signal corresponds to an increase in the expression of the expression element.

In one embodiment, the cell is a eukaryotic cell and the expression sequences are eukaryotic expression sequences. In one embodiment, the eukaryotic cell is a mammalian cell. In one embodiment, the binding moiety is an antibody or antibody derivative. In one embodiment, the binding moiety is a single-chain antibody. In one embodiment, the binding moiety is an aptamer. In one embodiment, the binding moiety is an organic compound.

In one aspect, the invention provides a minicell comprising at least one nucleic acid, wherein the minicell displays a binding moiety directed to a target compound, wherein the binding moiety is selected from the group consisting of (i) a eukaryotic membrane protein; (ii) an archeabacterial membrane protein; (iii) an organellar membrane protein; and (iv) a fusion protein, the fusion protein comprising a first polypeptide, the first polypeptide comprising at least one transmembrane domain or at least one membrane anchoring domain; and a second polypeptide, wherein the second polypeptide is not derived from a eubacterial protein and is neither a His tag nor a glutathione-S-transferase polypeptide, and wherein the polypeptide comprises a binding moiety.

In one embodiment, the nucleic acid comprises an expression construct comprising expression sequences operably linked to an ORF encoding a protein selected from the group consisting of (i) the eukaryotic membrane protein, (ii) the archeabacterial membrane protein, (iii) the organellar membrane protein; and (iv) the fusion protein.

In one embodiment, the nucleic acid comprises an expression construct comprising expression sequences operably linked to an ORF, wherein the ORF encodes a therapeutic polypeptide. In one embodiment, the therapeutic polypeptide is a membrane polypeptide. In one embodiment, the therapeutic polypeptide is a soluble polypeptide. In one embodiment, the soluble polypeptide comprises a cellular secretion sequence. In one embodiment, the expression sequences are inducible and/or repressible.

In one embodiment, the expression sequences are induced and/or derepressed when the binding moiety displayed by the minicell binds to its target compound. In one embodiment, the nucleic acid comprises an expression construct comprising expression sequences operably linked to an ORF, wherein the ORF encodes a polypeptide having an amino acid sequence that facilitates cellular transfer of a biologically active compound contained within or displayed by the minicell. In one embodiment, the membrane of the minicell comprises a system for transferring a molecule from the interior of a minicell into the cytoplasm of the cell. In one embodiment, the system for transferring a molecule from the interior of a minicell into the cytoplasm of the cell is a Type III secretion system.

In one aspect, the invention provides a method of introducing a nucleic acid into a cell, comprising contacting the cell with a minicell that comprises the nucleic acid, wherein the minicell displays a binding moiety, wherein the binding moiety is selected from the group consisting of (i) a eukaryotic membrane protein; (ii) an archeabacterial membrane protein; (iii) an organellar membrane protein; and (iv) a fusion protein, the fusion protein comprising a first polypeptide, the first polypeptide comprising at least one transmembrane domain or at least one membrane anchoring domain; and a second polypeptide, wherein the second polypeptide is not derived from a eubacterial protein and is neither a His tag nor a glutathione-S-transferase polypeptide, and wherein the polypeptide comprises a binding moiety; and wherein the binding moiety binds an epitope of the cell.

In one embodiment, the nucleic acid comprises an expression construct comprising expression sequences operably linked to an ORF encoding a protein selected from the group consisting of (i) the eukaryotic membrane protein, (ii) the archeabacterial membrane protein, (iii) the organellar membrane protein; and (iv) a fusion protein.

In one embodiment, the nucleic acid comprises an expression construct comprising expression sequences operably linked to an ORF, wherein the ORF encodes a therapeutic polypeptide. In one embodiment, the expression sequences are inducible and/or derepressible. In one embodiment, the expression sequences are induced or derepressed when the binding moiety displayed by the minicell binds its target compound. In one embodiment, the expression sequences are induced or derepressed by a transactivation or transrepression event. In one embodiment, the nucleic acid comprises an expression construct comprising expression sequences operably linked to an ORF, wherein the ORF encodes a polypeptide having an amino acid sequence that facilitates cellular transfer of a biologically active compound contained within or displayed by the minicell.

In one aspect, the invention provides a minicell comprising a nucleic acid, wherein the nucleic acid comprises eukaryotic expression sequences and eubacterial expression sequences, each of which is independently operably linked to an ORF.

In one embodiment, the minicell displays a binding moiety. In one embodiment, the eubacterial expression sequences are induced and/or derepressed when the binding moiety is in contact with a target cell. In one embodiment, the eukaryotic expression sequences are induced and/or derepressed when the nucleic acid is in the cytoplasm of a eukaryotic cell. In one embodiment, the protein encoded by the ORF comprises eubacterial or eukaryotic secretion sequences.

In one aspect, the invention provides a minicell comprising a first and second nucleic acid, wherein the first nucleic acid comprises eukaryotic expression sequences operably linked to a first ORF, and a second nucleic acid, wherein the second nucleic acid comprises eubacterial expression sequences operably linked to a second ORF.

In one embodiment, the minicell displays a binding moiety. In one embodiment, the eubacterial expression sequences are induced and/or derepressed when the binding moiety is in contact with a target cell. In one embodiment, the eukaryotic expression sequences are induced and/or derepressed when the nucleic acid is in the cytoplasm of a eukaryotic cell. In one embodiment, the protein encoded by the first ORF comprises eukaryotic secretion sequences and/or the protein encoded by the second ORF comprises eubacterial secretion sequences.

In one aspect, the invention provides a method of introducing into and expressing a nucleic acid in an organism, comprising contacting a minicell to a cell of the organism, wherein the minicell comprises the nucleic acid.

In one embodiment, the minicell displays a binding moiety. In one embodiment, the nucleic acid comprises a eukaryotic expression construct, wherein the eukaryotic expression construct comprises eukaryotic expression sequences operably linked to an ORF. In one embodiment, the ORF encodes a protein selected from the group consisting of a membrane protein, a soluble protein and a protein comprising eukaryotic secretion signal sequences. In one embodiment, the nucleic acid comprises a eubacterial expression construct, wherein the eubacterial expression construct comprises eubacterial expression sequences operably linked to an ORF. In one embodiment, the minicell displays a binding moiety, wherein the eubacterial expression sequences are induced and/or derepressed when the binding moiety is in contact with a target cell. In one embodiment, the protein encoded by the ORF comprises eubacterial secretion sequences.

In one embodiment, the ligand is a protein that forms a multimer with the target protein, and the ligand interacting atoms are atoms in the defined three-dimensional structure are atoms that are involved in protein-protein interactions. In one embodiment, the ligand is a compound that induces a conformational change in the target protein, and the defined three-dimensional structure is the site of the conformational change. In one embodiment, the method for identifying ligands of a target protein, further comprising identifying the chemical differences in the variant proteins as compared to the target protein. In one embodiment, the invention further comprises mapping the chemical differences onto the defined three-dimensional structure, and correlating the effect of the chemical differences on the defined three-dimensional structure. In one embodiment, the target protein is a wild-type protein. In one aspect, the invention provides a minicell library, comprising two or more minicells, wherein each minicell comprises a different exogenous protein. In one embodiment, the minicell is a eubacterial minicell, a poroplast, a spheroplast or a protoplast. In one embodiment, the exogenous protein is a displayed protein. In one embodiment, the exogenous protein is a membrane protein. In one embodiment, the membrane protein is a receptor. In one embodiment, the protein is a soluble protein that is contained within or secreted from the minicell. In one embodiment, minicells within the library comprise an expression element that comprises expression sequences operably linked to a nucleic acid having an ORF that encodes the exogenous protein. In one embodiment, the nucleic acid has been mutagenized; the mutagenesis can be site-directed or random. In one embodiment, an active site of the exogenous protein has a known or predicted three-dimensional structure, and the a portion of the ORF encoding the active site has been mutagenized. In one embodiment, each of the minicells comprises an exogenous protein that is a variant of a protein having a known or predicted three-dimensional structure.

In one aspect, the invention provides a minicell-producing parent cell, wherein the parent cell comprises one or more of the following (a) an expression element that comprises a gene operably linked to expression sequences that are inducible and/or repressible, wherein induction or repression of the gene regulates the copy number of an episomal expression construct; (b) a mutation in an endogenous gene, wherein the mutation regulates the copy number of an episomal expression construct; (c) an expression element that comprises a gene operably linked to expression sequences that are inducible and/or repressible, wherein induction or repression of the gene causes or enhances the production of minicells; and (d) a mutation in an endogenous gene, wherein the mutation causes or enhances minicell production.

In one embodiment, the invention comprises an episomal expression construct. In one embodiment, the invention further comprises a chromosomal expression construct. In one embodiment, the expression sequences of the expression construct are inducible and/or repressible. In one embodiment, the minicell-producing parent cell comprises a biologically active compound. In one embodiment, the gene that causes or enhances the production of minicells has a gene product that is involved in or regulates DNA replication, cellular division, cellular partitioning, septation, transcription, translation, or protein folding.

In one aspect, the invention provides a minicell-producing parent cell, wherein the parent cell comprises an expression construct, wherein the expression construct comprises expression sequences operably linked to an ORF that encodes a protein, and a regulatory expression element, wherein the regulatory expression element comprises expression sequences operably linked to a regulatory gene that encodes a factor that regulates the expression of the ORF. In one embodiment, the expression sequences of the expression construct are inducible and/or repressible. In one embodiment, the expression sequences of the regulatory expression construct are inducible and/or repressible. In one embodiment, one or more of the expression element or the regulatory expression element is located on a chromosome of the parent cell. In one embodiment, one or more of the expression element or the regulatory expression element is located on an episomal expression construct. In one embodiment, both of the expression element and the regulatory expression element are located on an episomal expression construct, and one or both of the expression element and the regulatory expression element segregates into minicells produced from the parent cell. In one embodiment, the minicell-producing parent cell comprises a biologically active compound. In one embodiment, the biologically active compound segregates into minicells produced from the parent cell. In one embodiment, the ORF encodes a membrane protein or a soluble protein. In one embodiment, the protein comprises secretion sequences. In one embodiment, the gene product of the gene regulates the expression of the ORF. In one embodiment, the gene product is a transcription factor. In one embodiment, the gene product is a RNA polymerase. In one embodiment, the parent cell is MC-T7.

In one aspect, the invention provides a minicell comprising a biologically active compound, wherein the minicell displays a binding moiety, wherein the minicell selectively absorbs and/or internalizes an undesirable compound, and the minicell is a poroplast, spheroplast or protoplast. In one embodiment, the binding moiety is selected from the group consisting of an antibody, an antibody derivative, a receptor and an active site of a non-catalytic derivative of an enzyme. In one embodiment, the binding moiety is a single-chain antibody. In one embodiment, the binding moiety is directed to a ligand selected from the group consisting of an epitope displayed on a pathogen, an epitope displayed on an infected cell and an epitope displayed on a hyperproliferative cell. In one embodiment, the biologically active compound is selected from the group consisting of a radioisotope, a polypeptide, a nucleic acid and a small molecule. In one embodiment, a ligand binds to and is internalized by the minicell or is otherwise inactivated by selective absorption. In one embodiment, the invention provides a pharmaceutical composition comprising the minicell. In one aspect, the invention provides a method of reducing the free concentration of a substance in a composition, wherein the substance displays a ligand specifically recognized by a binding moiety, comprising contacting the composition with a minicell that displays the binding moiety, wherein the binding moiety binds the substance, thereby reducing the free concentration of the substance in the composition. In one embodiment, the substance is selected from the group consisting of a nucleic acid, a lipid, a polypeptide, a radioactive compound, an ion and a small molecule. In one embodiment, the binding moiety is selected from the group consisting of an antibody, an antibody derivative, a channel protein and a receptor.

In one embodiment, the composition is present in an environment including but not limited to water, air or soil. In one embodiment, the composition is a biological sample from an organism, including but not limited to blood, serum, plasma, urine, saliva, a biopsy sample, feces, tissue and a skin patch. In one embodiment, the substance binds to and is internalized by the minicell or is otherwise inactivated by selective absorption. In one embodiment, the biological sample is returned to the organism after being contacting to the minicell.

For a better understanding of the present invention, reference is made to the accompanying detailed description and its scope will be pointed out in the appended claims. All references cited herein are hereby incorporated by reference.

### DRAWINGS

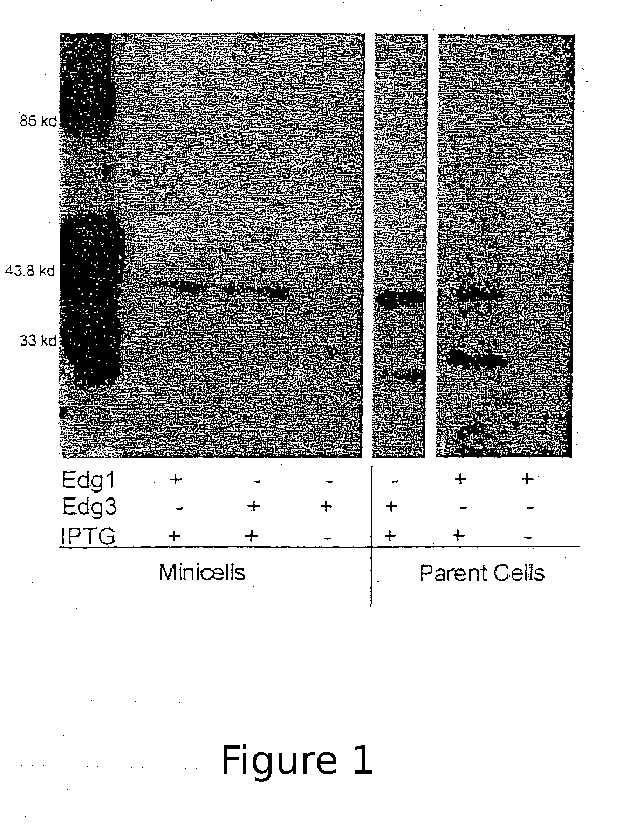

Figure 1 is a Western blot in which Edg-1-6xHis and Edg-3-6xHis proteins expressed in minicells produced from MC-T7 cells.

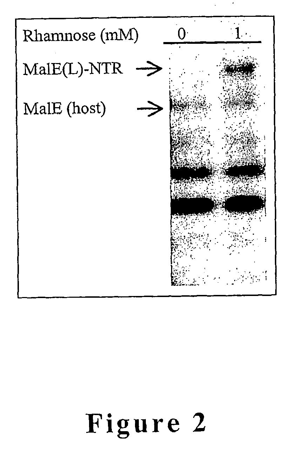

Figure 2 shows induction of MalE(L)-NTR in isolated minicells.

### ABBREVIATIONS AND DEFINITIONS

For brevity's sake, the single-letter amino acid abbreviations are used in some instances herein. Table 1 describes the correspondence between the 1- and 3-letter amino acid abbreviations.

**TABLE 1: THREE- AND ONE- LETTER ABBREVIATIONS FOR AMINO ACIDS**

| Amino acid | Three-letter abbreviation | One-letter symbol |
|---|---|---|
| Alanine | Ala | A |
| Arginine | Arg | R |
| Asparagine | Asn | N |
| Aspartic Acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

A "conjugatable compound" or "attachable compound" is capable of being attached to another compound. The terms "conjugated to" and "cross-linked with" indicate that the conjugatable compound is in the state of being attached to another compound.A "conjugate" is the compound formed by the attachment of a conjugatable compound or conjugatable moiety to another compound.

"Culturing" signifies incubating a cell or organism under conditions wherein the cell or organism can carry out some, if not all, biological processes. For example, a cell that is cultured may be growing or reproducing, or it may be non-viable but still capable of carrying out biological and/or biochemical processes such as replication, transcription, translation, etc.

An agent is said to have been "purified" if its concentration is increased, and/or the concentration of one or more undesirable contaminants is decreased, in a composition relative to the composition from which the agent has been purified. Purification thus encompasses enrichment of an agent in a composition and/or isolation of an agent therefrom.

A "solid support" is any solid or semisolid composition to which an agent can be attached or contained within. Common forms of solid support include, but are not limited to, plates, tubes, and beads, all of which could be made of glass or another suitable material, e.g., polystyrene, nylon, cellulose acetate, nitrocellulose, and other polymers. Semisolids and gels that minicells are suspended within are also considered to be solid supports. A solid support can be in the form of a dipstick, flow-through device, or other suitable configuration.

A "mutation" is a change in the nucleotide sequence of a gene relative to the sequence of the "wild-type" gene. Reference wild-type eubacterial strains are those that have been cultured in vitro by scientists for decades; for example, a wild-type strain of Escherichia coli iss E. coli K-12. Mutations include, but are not limited to, point mutations, deletions, insertions and translocations.

A "trans-acting regulatory domain" is a regulatory part of a protein that is expressed from a gene that is not adjacent to the site of regulatory effect. Trans-acting domains can activate or stimulate (transactivate), or limit or block (transrepress) the gene in question.

A "reporter gene" refers to a gene that is operably linked to expression sequences, and which expresses a gene product, typically a detectable polypeptide, the production and detection of which is used as a measure of the robustness and/or control of expression.

A "detectable compound" or "detectable moiety" produces a signal that can be detected by spectroscopic, photochemical, biochemical, immunochemical, electromagnetic, radiochemical, or chemical means such as fluorescence, chemifluoresence, or chemiluminescence, or any other appropriate means. A "radioactive compound" or "radioactive composition" has more than the natural (environmental) amount of one or more radioisotopes.

By "displayed" it is meant that a portion of the membrane protein is present on the surface of a cell or minicell, and is thus in contact with the external environment of the cell or minicell. The external, displayed portion of a membrane protein is an "extracellular domain" or a "displayed domain." A membrane protein may have more than one displayed domain, and a minicell of the invention may display more than one membrane protein.

A "domain" or "protein domain" is a region of a molecule or structure that shares common physical and/or chemical features. Non-limiting examples of protein domains include hydrophobic transmembrane or peripheral membrane binding regions, globular enzymatic or receptor regions, and/or nucleic acid binding domains.

A "transmembrane domain" spans a membrane, a "membrane anchoring domain" is positioned within, but does not traverse, a membrane. An "extracellular" or "displayed" domain is present on the exterior of a cell, or minicell, and is thus in contact with the external environment of the cell or minicell.

A "eukaryote" is as the term is used in the art. A eukaryote may, by way of non-limiting example, be a fungus, a unicellular eukaryote, a plant or an animal. An animal may be a mammal, such as a rat, a mouse, a rabbit, a dog, a cat, a horse, a cow, a pig, a simian or a human.

A "eukaryotic membrane" is a membrane found in a eukaryote. A eukaryotic membrane may, by way of non-limiting example, a cytoplasmic membrane, a nuclear membrane, a nucleolar membrane, a membrane of the endoplasmic reticulum (ER), a membrane of a Golgi body, a membrane of a lysosome a membrane of a peroxisome, a caveolar membrane, or an inner or outer membrane of a mitochondrion, chloroplast or plastid.

The term "endogenous" refers to something that is normally found in a cell as that cell exists in nature.

The term "exogenous" refers to something that is not normally found in a cell as that cell exists in nature.

A "gene" comprises (a) nucleotide sequences that either (i) act as a template for a nucleic acid gene product, or (ii) that encode one or more open reading frames (ORFs); and (b) expression sequences operably linked to (1) or (2). When a gene comprises an ORF, it is a "structural gene."

By "immunogenic," it is meant that a compound elicits production of antibodies or antibody derivatives and, additionally or alternatively, a T-cell mediated response, directed to the compound or a portion thereof. The compound is an "immunogen."

A "ligand" is a compound, composition or moiety that is capable of specifically bound by a binding moiety, including without limitation, a receptor and an antibody or antibody derivative.

A "membrane protein" is a protein found in whole or in part in a membrane. Typically, a membrane protein has (1) at least one membrane anchoring domain, (2) at least one transmembrane domain, or (3) at least one domain that interacts with a protein having (1) or (2).

An "ORF" or "open reading frame" is a nucleotide sequence that encodes an amino acid sequence of a known, predicted or hypothetical polypeptide. An ORF is bounded on its 5' end by a start codon (usually ATG) and on its 3' end by a stop codon (i.e., TAA or TGA). An ORF encoding a 10 amino acid sequence comprises 33 nucleotides (3 for each of 10 amino acids and 3 for a stop codon). ORFs can encode amino acid sequences that comprise from 10, 25, 50, 125, 150, 175, 200, 250, 300, 350, 400, 450, 500, 600, 700, 800, 900 or more amino acids

The terms "Eubacteria" and"prokaryote" are used herein as these terms are used by those in the art. The terms "eubacterial" and "prokaryotic" encompasses Eubacteria, including both gram-negative and gram-positive bacteria, prokaryotic viruses (e.g., bacteriophage), and obligate intracellular parasites (e.g., Rickettsia, Chlamydia, etc.).

An "active site" is any portion or region of a molecule required for, or that regulates, an activity of the molecule. In the case of a protein, an active site can be a binding site for a ligand or a substrate, an active site of enzyme, a site that directs or undergoes conformational change in response to a signal, or a site of post-translational modification of a protein.

In a poroplast, the eubacterial outer membrane (OM) and LPS have been removed. In a spheroplast, portions of a disrupted eubacterial OM and/or disrupted cell wall either may remain associated with the inner membrane of the minicell, but the membrane is nonetheless porous because the permeability of the disrupted OM has been increased. A membrane is the to be "disrupted" when the membrane's structure has been treated with an agent, or incubated under conditions, that leads to the partial degradation of the membrane, thereby increasing the permeability thereof. In contrast, a membrane that has been "degraded" is essentially, for the applicable intents and purposes, removed. In preferred embodiments, irrespective of the condition of the OM and cell wall, the eubacterial inner membrane is not disrupted, and membrane proteins displayed on the inner membrane are accessible to compounds that are brought into contact with the minicell, poroplast, spheroplast, protoplast or cellular poroplast, as the case may be.

Host cells (and/or minicells) harboring an expression construct are components of expression systems.

An "expression vector" is an artificial nucleic acid molecule into which an exogenous ORF encoding a protein, or a template of a bioactive nucleic acid can be inserted in such a manner so as to be operably linked to appropriate expression sequences that direct the expression of the exogenous gene. Preferred expression vectors are episomal vectors that can replicate independently of chromosomal replication.

By the term "operably linked" it is meant that the gene products encoded by the non-vector nucleic acid sequences are produced from an expression element in vivo.

The term "gene product" refers to either a nucleic acid (the product of transcription, reverse transcription, or replication) or a polypeptide (the product of translation) that is produced using the non-vector nucleic acid sequences as a template.

An "expression construct" is an expression vector into which a nucleotide sequence of interest has been inserted in a manner so as to be positioned to be operably linked to the expression sequences present in the expression vector. Preferred expression constructs are episomal.

An "expression element" is a nucleic acid having nucleotide sequences that are present in an expression construct but not its cognate expression vector. That is, an expression element for a polypeptide is a nucleic acid that comprises an ORF operably linked to appropriate expression sequences. An expression element can be removed from its expression construct and placed in other expression vectors or into chromosomal DNA.

"Expression sequences" are nucleic acid sequences that bind factors necessary for the expression of genes that have been inserted into an expression vector. An example of an expression sequence is a promoter, a sequence that binds RNA polymerase, which is the enzyme that produces RNA molecules using DNA as a template. An example of an expression sequence that is both inducible and repressible is L-arabinose operon (araC). See Schleif R. Regulation of the L-arabinose operon of Escherichia coli. Trends Genet. 2000 Dec;16(12):559-65.

In the present disclosure, "a nucleic acid" or "the nucleic acid" refers to a specific nucleic acid molecule. In contrast, the term "nucleic acid" refers to any collection of diverse nucleic acid molecules, and thus signifies that any number of different types of nucleic acids are present. By way of non-limiting example, a nucleic acid may be a DNA, a dsRNA, a tRNA (including a rare codon usage tRNA), an mRNA, a ribosomal RNA (rRNA), a peptide nucleic acid (PNA), a DNA:RNA hybrid, an antisense oligonucleotide, a ribozyme, or an aptamer.

The invention described herein is drawn to compositions and methods for the production of achromosomal archeabacterial, eubacterial and anucleate eukaryotic cells that are used for diagnostic and therapeutic applications, for drug discovery, and as research tools.

The general advantage of minicells over cell-based expression systems (e.g., eucaryotic cells or bacterial expression systems) is that one may express heterologous membrane bound proteins or over express endogenous membrane bound proteins , cytoplasmic or secreted soluble proteins,or small molecules on the cytoplasmic or extracellular surfaces of the minicells that would otherwise be toxic to live cells. Minicells are also advantageous for proteins that require a particular lipid environment for proper functioning because it is very manipulatable in nature. Other advantages include the stability of the minicells due to the lack of toxicity, the high level of expression that can be achieved in the minicell, and the efficient flexible nature of the minicell expression system. Such minicells could be used for *in vivo* targeting or for selective absorption (i.e., molecular "sponges") and that these molecules can be expressed and "displayed" at high levels. Minicells can also be used to display proteins for low, medium, high, and ultra high throughput screening, crystal formation for structure determination, and for *in vitro* research use only applications such as transfection. Minicells expressing proteins or small molecules, radioisotopes, image-enhancing reagents can be used for *in vivo* diagnostics and for *in vitro* diagnostic and assay platforms. Also, soluble and/or membrane associated signaling cascade elements may be reconstituted in minicells producing encapsulated divices to follow extracellular stimulation events using cytoplasmic reporter events, e.g. transactivation resulting from dimerization of dimerization dependant transcriptional activation or repression of said reporter.

Regarding protein expression, minicells can be engineered to express one or more recombinant proteins in order to produce more protein per surface area of the particle (at least 10X more protein per unit surface area of protein). The proteins or small molecules that are "displayed" on the minicell surfaces can have therapeutic, discovery or diagnostic benefit either when injected into a patient or used in a selective absorption mode during dialysis Proteins that are normally soluble can be tethered to membrane anchoring domains or membrane proteins can be expressed for the purpose of displaying these proteins on the surfaces of the minicell particle in therapeutic, discovery, and diagnostic modes. The types of proteins that can be displayed include but are not limited to receptors (e.g., GPCRs, sphingolipid receptors, neurotransmitter receptors, sensory receptors, growth factor receptors, hormone receptors, chemokine receptors, cytokine receptors, immunological receptors, and complement receptors, FC receptors), channels (e.g., potassium channels, sodium channels, calcium channels.), pores (e.g., nuclear pore proteins, water channels), ion and other pumps (e.g., calcium pumps, proton pumps), exchangers (e.g., sodium/potassium exchangers, sodium/hydrogen exchangers, potassium/hydrogen exchangers), electron transport proteins (e.g., cytochrome oxidase), enzymes and kinases (e.g., protein kinases, ATPases, GTPases, phosphatases, proteases.), structural/linker proteins (e.g., Caveolins, clathrin), adapter proteins (e.g., TRAD, TRAP, FAN), chemotactic/adhesion proteins (e.g., ICAM11, selectins, CD34, VCAM-1, LFA-1,VLA-1), and chimeric/fussion proteins (e.g., proteins in which a normally soluble protein is attached to a transmembrane region of another protein). As a non-limiting example, the small molecules that can be tethered and displayed on the surfaces of the minicells can be carbohydrates (e.g., monosaccharides), bioactive lipids (e.g., lysosphingolipids, PAF, lysophospholipids), drugs (e.g., antibiotics, ion channel activators/inhibitors, ligands for receptors and/or enzymes), nucleic acids (e.g., synthetic oligonucleotides), fluorophores, metals, or inorganic and organic small molecules typically found in combinatorial chemistry libraries. Minicells may either contain (encapsulate) or display on their surfaces radionuclides or image-enhancing reagents both of which could be used for therapeutic and/or diagnostic benefit in vivo or for *in vitro* assays and diagnostic platforms.

For *in vivo* therapeutic uses, minicells can express proteins and/or display small molecules on their surfaces that would either promote an immune response and passage through the RES system (e.g., to eliminate the minicell and its target quickly), or to evade the RES (e.g., to increase the bioavailability of the minicell). Toxicity is reduced or eliminated because the therapeutic agent is not excreted or processed by the liver and thus does not damage the kidneys or liver, because the minicell-based therapeutic is not activated until entry into the target cell (e.g., in the case of cancer therapeutics or gene therapy). Minicells are of the appropriate size (from about 0.005, 0.1, 0.15 or 0.2 micrometers to about 0.25, 0.3, 0.35, 0.4, 0.45 or 0.5 micrometers) to facilitate deep penetration into the lungs in the cases where administration of the minicell-based therapeutic or diagnostic is via an inhalant (Strong, A. A., et al. 1987. An aerosol generator system for inhalation delivery of pharmacological agents. Med. Instrum. 21:189-194). This is due to the fact that minicells can be aerosolized. Without being limited to the following examples, inhalant therapeutic uses of minicells could be applied to the treatment of anaphylactic shock, viral infection, inflammatory reactions, gene therapy for cystic fibrosis, treatment of lung cancers, and fetal distress syndrome.

Minicells can also display expressed proteins that are enzymes that may have therapeutic and/or diagnostic uses. The enzymes that are displayed may be soluble enzymes that are expressed as fusion proteins with a transmembrane domain of another protein. Display of such enzymes could be used for *in vitro* assays or for therapeutic benefit.

Gene therapy applications afforded by minicells generally involve the ability of minicells to deliver DNA to target cells (either for replacement therapy, modifation of cell function or to kill cells). Expression plasmids can be delivered to target cells that would encode proteins that could be cytoplasmic or could have intracellular signal sequences that would target the protein to a particular organelle (e.g., mitochondria, nuclei, endoplasmic reticulum, etc.). In the case where minicells are engulfed by the taget cell, the minicells themselves could have these intracellular targeting sequences expressed on their surfaces so that the minicells could be 'delivered' to intracellular targets.

Minicells used for the following therapeutic, discovery, and diagnostic applications can be prepared as described in this application and then stored and/or packaged by a variety of ways, including but not limited to lyophilization, freezing, mixing with preservatives (e.g., antioxidants, glycerol), or otherwise stored and packaged in a fashion similar to methods used for liposome and proteoliposome formulations.

The small size of minicells (from about 0.005, 0.1, 0.15 or 0.2 micrometers to about 0.25, 0.3, 0.35, 0.4, 0.45 or 0.5 micrometers) makes them suitable for many in vitro diagnostic platforms, including the non-limiting examples of lateral flow, ELISA, HTS, especially those applications requiring microspheres or nanospheres that display many target proteins or other molecules. The use of protoplast or poroplast minicells may be especially useful in this regard. Assay techniques are dependent on cell or particle size, protein (or molecule to be tested) amount displayed on the surface of the cell or particle, and the sensitivity of the assay being measured. In current whole-cell systems, the expression of the protein of interest is limiting, resulting in the higher cell number requirement to satisfy the sensitivity of most assays. However, the relatively large size of cells prevents the incorporation of large numbers of cells in these assays, e.g. 96, 384, and smaller well formats. In contrast, minicells, protoplasts, and poroplasts are smaller in size and can be manipulated to express high levels of the preselected protein, and can be incorporated into small well assay formats.

### I. TYPES OF MINICELLS

Minicells are derivatives of cells that lack chromosomal DNA and which are sometimes referred to as anucleate cells. Because eubacterial and achreabacterial cells, unlike eukaryotic cells, do not have a nucleus (a distinct organelle that contains chromosomes), these non-eukaryotic minicells are more accurately described as being "without chromosomes" or "achromosomal," as opposed to "anucleate." Nonetheless, those skilled in the art often use the term "anucleate" when referring to bacterial minicells in addition to other minicells. Accordingly, in the present disclosure, the term "minicells" encompasses derivatives of eubacterial cells that lack a chromosome; derivatives of archeabacterial cells that lack their chromosome(s) (Laurence et al., Nucleoid Structure and Partition in Methanococcus jannaschii: An Archaeon With Multiple Copies of the Chromosome, Genetics 152:1315-1323, 1999); and anucleate derivatives of eukaryotic cells. It is understood, however, that some of the relevant art may use the terms "anucleate minicells" or anucleate cells" loosely to refer to any of the preceeding types of minicells.

### I.A. Eubacterial Minicells

One type of minicell is a eubacterial minicell. For reviews of eubacterial cell cycle and division processes, see Rothfield et al., Bacterial Cell Division, Annu. Rev. Genet., 33:423-48, 1999; Jacobs et al., Bacterial cell division: A moveable feast, Proc. Natl. Acad. Sci. USA, 96:5891-5893, May, 1999; Koch, The Bacterium's Way for Safe Enlargement and Division, Appl. and Envir. Microb., Vol. 66, No. 9, pp. 3657-3663; Bouche and Pichoff, On the birth and fate of bacterial division sites. Mol Microbiol, 1998. 29: 19-26; Khachatourians et al., Cell growth and division in Escherichia coli: a common genetic control involved in cell division and minicell formation. J Bacteriol, 1973. 116: 226-229; Cooper, The Escherichia coli cell cycle. Res Microbiol, 1990. 141: 17-29; and Danachie and Robinson, "Cell Division: Parameter Values and the Process," in: Escherichia Coli and Salmonella Typhimurium: Cellular and Molecular Biology, Neidhardt, Frederick C., Editor in Chief, American Society for Microbiology, Washington, DC., 1987, Volume 2, pages 1578-1592, and references cited therein; and Lutkenhaus et al., "Cell Division," Chapter 101 in: Escherichia coli and Salmonella typhimurium: Cellular and Molecular Biology, 2nd Ed., Neidhardt, Frederick C., Editor in Chief, American Society for Microbiology, Washington, D.C., 1996, Volume 2, pages 1615-1626, and references cited therein. When DNA replication and/or chromosomal partitioning is altered, membrane-bounded vesicles "pinch off" from parent cells before transfer of chromosomal DNA is completed. As a result of this type of dysfunctional division, minicells are produced which contain an intact outer membrane, inner membrane, cell wall, and all of the cytoplasm components but do not contain chromosomal DNA. See Table 2.

### I.B. Eukaryotic Minicells

The term "eukaryote" is defined as is used in the art, and includes any organism classified as Eucarya that are usually classified into four kingdoms: plants, animals, fungi and protists. The first three of these correspond to phylogenetically coherent groups. However, the eucaryotic protists do not form a group, but rather are comprised of many phylogenetically disparate groups (including slime molds, multiple groups of algae, and many distinct groups of protozoa). See, e.g., Olsen, G., http://www.bact.wisc.edu/microtextbook/. A type of animal of particular interest is a mammal, including, by way of non-limiting example a rat, a mouse, a rabbit, a dog, a cat, a horse, a cow, a pig, a simian and a human.

Chromosomeless eukaryotic minicells (i.e., anucleate cells) are within the scope of the invention. Platelets are a non-limiting example of eukaryotic minicells. Platelets are anucleate cells with little or no capacity for de novo protein synthesis. The tight regulation of protein synthesis in platelets (Smith et al., Platelets and stroke, Vasc Med 4:165-72, 1999) may allow for the over-production of exogenous proteins and, at the same time, under-production of endogenous proteins. Thrombin-activated expression elements such as those that are associated with Bcl-3 (Weyrich et al., Signal-dependent translation of a regulatory protein, Bcl-3, in activated human platelets, Cel Biology 95:5556-5561, 1998) may be used to modulate the expresion of exogneous genes in platelets.

As another non-limiting example, eukaryotic minicells are generated from tumor cell lines (Gyongyossy-Issa and Khachatourians, Tumour minicells: single, large vesicles released from cultured mastocytoma cells (1985) Tissue Cell 17:801-809; Melton, Cell fusion-induced mouse neuroblastomas HPRT revertants with variant enzyme and elevated HPRT protein levels (1981) Somatic Cell Genet 7: 331-344).

Yeast cells are used to generate fungal minicells. See, e.g., Lee et al., !bd1p, a possible spindle pole body associated protein, regulates nuclear division and bud separation in Saccharomyces cerevisiae, Biochim Biophys Acta 3:239-253, 1999; Kopecka et al., A method of isolating anucleated yeast protoplasts unable to synthesize the glucan fibrillar component of the wall J Gen Microbiol 81:111-120, 1974; and Yoo et al., Fission yeast Hrp1, a chromodomain ATPase, is required for proper chromosome segregation and its overexpression interferes with chromatin condensation, Nucl Acids Res 28:2004-2011, 2000. Cell division in yeast is reviewed by Gould and Simanis, The control of septum formation in fission yeast, Genes & Dev 11:2939-51, 1997).

### I.C. Archeabacterial Minicells

The term "archeabacterium" is defined as is used in the art and includes extreme thermophiles and other Archaea. Woese, C.R., L. Magrum. G. Fox. 1978. Archeabacteria. Journal of Molecular Evolution. 11:245-252. Three types of Archeabacteria are halophiles, thermophiles and methanogens. By physiological definition, the Archaea (informally, archaes) are single-cell extreme thermophiles (including thermoacidophiles), sulfate reducers, methanogens, and extreme halophiles. The thermophilic members of the Archaea include the most thermophilic organisms cultivated in the laboratory. The aerobic thermophiles are also acidophilic; they oxidize sulfur in their environment to sulfuric acid. The extreme halophiles are aerobic or microaerophilic and include the most salt tolerant organisms known. The sulfate-reducing Archaea reduce sulfate to sulfide in extreme environment. Methanogens are strict anaerobes, yet they gave rise to at least two separate aerobic groups: the halophiles and a thermoacidophilic lineage (Olsen, G., http://www.bact.wisc.edu/microtextbook/). Non-limiting examples of halophiles include *Halobacterium cutirubrum* and *Halogerax mediterranei.* Non-limiting examples of methanogens include *Methanococcus voltae; Methanococcus vanniela; Methanobacterium thermoautotrophicum; Methanococcus voltae; Methanothermus fervidus*; and *Methanosarcina barkeri.* Non-limiting examples of thermophiles include *Azotobacter vinelandii*; *Thermoplasma acidophilum*; *Pyrococcus horikoshii*; *Pyrococcus furiosus*; and Crenarchaeota (extremely thermophilic archaebacteria) species such as *Sulfolobus solfataricus* and *Sulfolobus acidocaldarius.*

Archeabacterial minicells are within the scope of the invention. Archeabacteria have homologs of eubacterial minicell genes and proteins, such as the MinD polypeptide from *Pyrococcus furiosus* (Hayashi et al., EMBO J 2001 20:1819-28, Structural and functional studies of MinD ATPase: implications for the molecular recognition of the bacterial cell division apparatus). It is thus possible to create Archeabacterial minicells by methods such as, by way of non-limiting example, overexpressing the product of a *min* gene isolated from a prokaryote or an archeabacterium; or by disrupting expression of a *min* gene in an archeabacterium of interest by, e.g., the introduction of mutations thereof or antisense molecules thereto. See, e.g., Laurence et al., Nucleoid Structure and Partition in Methanococcus jannaschii: An Archaeon With Multiple Copies of the Chromosome, Genetics 152:1315-1323, 1999.

In one aspect, the invention is drawn to archael minicells. By physiological definition, the Archaea (informally, archaes) are single-cell extreme thermophiles (including thermoacidophiles), sulfate reducers, methanogens, and extreme halophiles. The thermophilic members of the Archaea include the most thermophilic organisms cultivated in the laboratory. The aerobic thermophiles are also acidophilic; they oxidize sulfur in their environment to sulfuric acid. The extreme halophiles are aerobic or microaerophilic and include the most salt tolerant organisms known. The sulfate-reducing Archaea reduce sulfate to sulfide in extreme environment. Methanogens are strict anaerobes, yet they gave rise to at least two separate aerobic groups: the halophiles and a thermoacidophilic lineage (Olsen, G., http://www.bact.wisc.edu/microtextbook/).

### II. PRODUCTION OF MINICELLS

*Eubacterial* minicells are produced by parent cells having a mutation in, and/or overexpressing, or under expressing a gene involved in cell division and/or chromosomal partitioning, or from parent cells that have been exposed to certain conditions, that result in abberant fission of bacterial cells and/or partitioning in abnormal chromosomal segregation during cellular fission (division). The term "parent cells" or "parental cells" refers to the cells from which minicells are produced. Minicells, most of which lack chromosomal DNA (Mulder et al., The Escherichia coli minB mutation resembles gyrB in Defective nucleoid segregation and decreased negative supercoiling of plasmids. Mol Gen Genet, 1990, 221: 87-93), are generally, but need not be, smaller than their parent cells. Typically, minicells produced from *E. coli* cells are generally spherical in shape and are about 0.1 to about 0.3 um in diameter, whereas whole *E. coli* cells are about from about 1 to about 3 um in diameter and from about about 2 to about 10 um in length. Micrographs of *E. coli* cells and minicells that have been stained with DAPI (4:6-diamidino-z-phenylindole), a compound that binds to DNA, show that the minicells do not stain while the parent *E. coli* are brightly stained. Such micrographs demonstrate the lack of chromosomal DNA in minicells. (Mulder et al., Mol. Gen. Genet. 221:87-93, 1990).

As shown in Table 2, minicells are produced by several different mechanisms such as, by way of non-limiting example, the over expression of genes involved in chromosomal replication and partitioning, mutations in such genes, and exposure to various environmental conditions. "Overexpression" refers to the expression of a polypeptide or protein encoded by a DNA introduced into a host cell, wherein the polypeptide or protein is either not normally present in the host cell, or wherein the polypeptide or protein is present in the host cell at a higher level than that normally expressed from the endogenous gene encoding the polypeptide or protein. For example, in *E*. *coli* cells that overexpress the gene product FtsZ (The FtsZ gene encodes a protein that is involved in regulation of divisions; see Cook and Rothfield, Early stages in development of the Escherichia coli cell-division site. Mol Microbiol, 1994. 14: p. 485-495; and Lutkenhaus, Regulation of cell division in E. coli. Trends Genet, 1990. 6: p. 22-25), there is an increase in the formation of minicells (Begg et al., Roles of FtsA and FtsZ in the activation of division sites. J. Bacteriology, 1997. 180: 881-884). Minicells are also produced by *E. coli* cells having a mutation in one or more genes of the min locus, which is a group of genes that encode proteins that are involved in cell division (de Boer et al., Central role for the Escherichia coli minC gene product in two different cell division-inhibition systems. Proc. Natl. Acad. Sci. USA, 1990. 87: 1129-33; Akerlund et al., Cell division in Escherichia coli minB mutants. Mol Microbiol, 1992. 6: 2073-2083).

Prokaryotes that have been shown to produce minicells include species of *Escherichia, Shigella, Bacillus, Lactobacillus,* and *Campylobacter*. Bacterial minicell-producing species of particular interest are *E. coli* and *Bacillus subtilis. E. coli* is amenable to manipulation by a variety of molecular genetic methods, with a variety of well-characterized expression systems, including many episomal expression systems, factors and elements useful in the present invention. *B. subtilis,* also amenable to genetic manipulation using episomal expression elements, is an important industrial organism involved in the production of many of the world's industrial enzymes (proteases, amylases, etc.), which it efficiently produces and secretes.

In the case of other eubacterial species, homologs of *E. coli* or *B. subilis* genes that cause minicell production therein are known or can be identified and characterized as is known in the art. For example, the *min* regions of the chromosome of *Strepococcus pneumoniae* and *Neisseria* gonorrhoeae have been characterized (Massidda et al., Unconventional organization of the division and cell wall gene cluster of Streptococcus pneumoniae, Microbiology 144:3069-78, 1998; and Ramirez-Arcos et al., Microbiology 147:225-237, 2001 and Szeto et al., Journal of Bacteria 183(21):6253, 2001, respectively). Those skilled in the art are able to isolate minicell producing (*min*) mutants, or prepare compounds inhibitory to genes that induce a minicell production (e.g., antisense to *min* transcripts).

**TABLE 2: Eubacterial Strains, Mutations and Conditions that Promote Minicell Formation**

| **Species** | **Strain** | **Notes** | **References** |
|---|---|---|---|
| *Campylobacter jejuni* | | may occur naturally late in growth cycle | Brock *et al.,* 1987 |
| *Bacillus subtilis* | | Mutations in *div*IVB locus (inc. *minC, minD* | Barak *et al.,* 1999 |
| | | *ripX* mutations | Sciochetti *et al.,* 1999; Lemon *et al*., 2001 |
| | | *smc* mutations | Moriya *et al.,* 1998; Britton *et al.,* 1998 |
| | | *oriC* deletions | Moriya *et al.,* 1997; Hassan *et al.,* 1997 |
| | | *prfA* mutations | Pederson and Setlow, 2001 |
| | | Mutations in *div*IVA locus | Cha *et al.,* 1997 |
| | *B.s*. 168 | ts initiation mutation TsB143 | Sarqent, 1975 |
| *Bacillus cereus* | WSBC 10030 | Induced by exposure to long-chain polyphosphate | Maier *et al.,* 1999 |
| *Shigella flexneri* (2a) | MC-1 | | Gemski *et al*., 1980 |
| *S. dysenteriae* (1) | MC-V | | Gemski *et al*., 1980 |
| *Lactobacillus spp.* | | Variant minicell-producing strains isolated from grains | Pidoux *et al*., 1990 |
| *Neisseria gonorrhoeae* | | deletion or overepression of *min* homologues | Ramirez-Arcos et al., 2001; Szeto et al., 2001 |
| *Escherichia coli* | | *MinA* mutations | Frazer *et al*., 1975; Cohen *et al*. 1976 |
| | | *MinB* mutations and deletions | Adler *et al.,* 1967; Davie *et al.*, 1984; Schaumberg et al.; 1983; Jaffe *et al.*, 1988; Akerlund *et al*., 1992 |
| | CA8000 | *cya, crp* mutations | Kumar *et al*.; 1979 |
| | | *MukA1* mutation | Hiraqa *et al.,* 1996 |
| | | *MukE, mukF* mutations | Yamanaka *et al.,* 1996 |
| | | *hns* mutation | Kaidow *et al.,* 1995 |
| | DS410 | | Heighway *et al.,* 1989 |
| | | χ 1972, χ 1776 and χ 2076 | Curtiss, 1980 |
| | P678-54 | Temperature-sensitive cell division mutations | Adler *et al.* 1967; Allen *et al.,* 1972; Hollenberq *et al.,* 1976 |
| | | Induced by overexpression of minB protein | De Boer *et al.,* 1988 |
| | | Induced by overexpression of minE protein or derivatives | Pichoff *et al.,1995* |
| | | Induced by oveproduction of *fts*Z gene | Ward *et al.,* 1985 |
| | | Induced by overexpression of *sdiA* gene | Wang *et al.,* 1991 |
| | | Induced by overexpression of *min* genes from *Neisseria gonorrhoeae* | Ramirez-Arcos et al., 2001; Szeto et al., 2001 |
| | | Induced by exposure to EGTA | Wachi *et al.,* 1999 |
| Legionella Pneumophila | | Induced by exposure to ampicillin | Elliot et al., 1985 |

Citations for Table 2:
Adler et al., Proc. Natl. Acad. Sci. 57:321-326 (1967)
Akerlund et al., Mol. Microbiol. 6:2073-2083 (1992)
Allen et al., Biochem. Biophys. Res. Communi. 47:1074-1079 (1972)
Barak et al., J. Bacteriol. 180:5237-5333 (1998)
Britton et al., Genes Dev. 12:1254-9 (1998)
Brock et al., Can. J. Microbiol. 33:465-470 (1987)
Cha et al., J. Bacteriol. 179:1671-1683 (1997)
Cohen et al., Genetics 56:550-551 (1967)
Curtiss, Roy III, U.S. Patent No. 4,190,495; Issued February 26, 1980
Davie et al., J. Bacteriol. 170:2106-2112 (1988)
Elliott et al., J. Med. Microbiol, 19:383-390 (1985)
Frazer et al., Curr Top. Immunol. 69:1-84 (1975)
Gemski et al., Infect. Immun. 30:297-302 (1980)
Hassan et al., J. Bacteriol. 179:2494-502 (1997)
Heighway et al., Nucleic Acids Res. 17:6893-6901 (1989)
Hiraga et al., J. Bacteriol. 177:3589-3592 (1995)
Hollenberg et al., Gene 1:33-47 (1976)
Kumar et al., Mol. Gen. Genet. 176:449-450 (1979)
Lemon et al., Proc. Natl. Acad. Sci. USA 98:212-7 (2001)
Maier et al., Appl. Environ. Microbiol. 65:3942-3949 (1999)
Moriya et al., DNA Res 4:115-26 (1997)
Moriya et al., Mol. Microbiol. 29:179-87 (1998)
Markiewicz et al., FEMS Microbiol. Lett. 70:119-123 (1992)
Pederson and Setlow, J. Bacteriol. 182:1650-8 (2001)
Pichoff et al., Mol. Microbiol. 18:321-329 (1995)
Pidoux et al., J. App. Bacteriol. 69:311-320 (1990)
Ramirez-Arcos et al. Microbiol. 147:225-237 (2001)
Sargent M.G., J. Bacteriol. 123:1218-1234 (1975)
Sciochetti et al., J. Bacteriol. 181:6053-62 (1999)
Schaumberg et al., J. Bacteriol. 153:1063-1065 (1983)
Szeto et al., Jour. of Bacter. 183 (21):6253 (2001)
Wachi et al., Biochimie 81:909-913 (1999)
Wang et al., Cell 42:941-949 (1985)
Yamanaka et al., Mol. Gen. Genet. 250:241-251 (1996)

### II.A. Optimized Minicell Construction

Minicells are produced by several different eubacterial strains and mechanisms including the overexpression of endogenous or exogenous genes involved in cell division, chromosomal replication and partitioning, mutations in such genes, and exposure to various chemical and/or physical conditions. For example, in E. coli cells that overexpress the gene product FtsZ (the ftsZ gene encodes a protein that is involved in regulation of cell division; see Cook and Rothfield, Early stages in development of the Escherichia coli cell-division site. Mol Microbiol, 1994. 14: p. 485-495; and Lutkenhaus, Regulation of cell division in E. coli. Trends Genet, 1990. 6: p. 22-25), there is an increase in the formation of minicells (Begg et al., Roles of FtsA and FtsZ in the activation of division sites. J. Bacteriology, 1997. 180: 881-884). Minicells are also produced by E. coli cells having a mutation in one or more genes of the min locus, which is a group of genes that encode proteins that are involved in cell division (de Boer et al., Central role for the Escherichia coli minC gene product in two different cell division-inhibition systems. Proc. Natl. Acad. Sci. USA, 1990. 87: 1129-33; Akerlund et al., Cell division in Escherichia coli minB mutants. Mol Microbiol, 1992. 6: 2073-2083).

Eubacterial cells that have been shown to produce minicells include, but are not limited to species of *Escherichia, Shigella, Bacillus, Lactobacillus, Legionella* and *Campylobacter.* Bacterial minicell-producing species of particular interest are *E. coli* and *Bacillus subtilis.* These organisms are amenable to manipulation by a variety of molecular and genetic methods, with a variety of well-characterized expression systems, including many episomal and chromosomal expression systems, as well as other factors and elements useful in the present invention.

The following sections describe genes that may be manipulated so as to stimulate the production of minicells. The invention may include any of these non-limiting examples for the purpose of preparing minicells. Furthermore, these genes and gene products and conditions, may be used in methodologies to identify other gene(s), gene products, biological events, biochemical events, or physiological events that induce or promote the production of minicells. These methodologies include, but are not limited to genetic selection, protein, nucleic acid, or combinatorial chemical library screen, one- or two-hybrid analysis, display selection technologies, e.g. phage or yeast display, hybridization approaches, e.g. array technology, and other high- or low-throughput approaches.

### II.A.1. Homologs

Homologs of these genes and gene products from other organisms may also be used. As used herein, a "homolog" is defined is a nucleic acid or protein having a nucleotide sequence or amino acid sequence, respectively, that is "identical," "essentially identical," "substantially identical," "homologous" or "similar" (as described below) to a reference sequence which may, by way of non-limiting example, be the sequence of an isolated nucleic acid or protein, or a consensus sequence derived by comparison of two or more related nucleic acids or proteins, or a group of isoforms of a given nucleic acid or protein. Non-limiting examples of types of isoforms include isoforms of differing molecular weight that result from, e.g., alternate RNA splicing or proteolytic cleavage; and isoforms having different post-translational modifications, such as glycosylation; and the like.

### II.A.2. Escherichia coli Genes

Exemplary genes and gene products from *E. coli* the expression and/or sequence of which can be manipulated so as to stimulate minicell production in *E. coli* or any other organism, as can homologs thereof from any species, include without limitation, the bolA gene (Aldea, M., et al. 1988. Identification, cloning, and expression of bolA, an ftsZ-dependent morphogene of Escherichia coli. J. Bacteriol. 170:5196-5176; Aldea, M., et al. 1990. Division genes in Escherichia coli are expressed coordinately to cell septum requirements by gearbox promoters. EMBO J. 9:3787-3794); the chpA gene (Masuda, Y., et al. 1993. chpA and chpB, Escherichia coli chromosomal homologs of the pem locus responsible for stable maintenance of plasmid R100. J. Bacteriol. 175:6850-6856); the chpB gene (Masuda, Y., et al. 1993. chpA and chpB, Escherichia coli chromosomal homologs of the pem locus responsible for stable maintenance of plasmid R100. J. Bacteriol. 175:6850-6856); the chpR (chpAI) gene (Masuda, Y., et al. 1993. chpA and chpB, Escherichia coli chromosomal homologs of the pem locus responsible for stable maintenance of plasmid R100. J. Bacteriol. 175:6850-6856); the chpS (chpBI)gene (Masuda, Y., et al. 1993. chpA and chpB, Escherichia coli chromosomal homologs of the pem locus responsible for stable maintenance of plasmid R100. J. Bacteriol. 175:6850-6856); the crg gene (Redfield, R. J., and A. M. Campbell. 1987. Structurae of cryptic lambda prophages. J. Mol. Biol. 198:393-404); the crp gene (Kumar, S., et al. 1979. Control of minicell producing cell division by cAMP-receptor protein complex in Escherichia coli. Mol. Gen. Genet. 176:449-450); the cya gene (Kumar, S., et al. 1979. Control of minicell producing cell division by cAMP-receptor protein complex in Escherichia coli. Mol. Gen. Genet. 176:449-450); the dicA gene (Labie, C., et al. 1989. Isolation and mapping of Escherichia coli mutations conferring resistance to division inhibition protein DicB. J. Bacteriol. 171:4315-4319); the dicB gene (Labie, C., et al. 1989. Isolation and mapping of Escherichia coli mutations conferring resistance to division inhibition protein DicB. J. Bacteriol. 171:4315-4319; Labie, C., et al. 1990. Minicell-forming mutants of Escherichia coli: suppression of both DicB- and MinD-dependent division inhibition by inactivation of the minC gene product. J. Bacteriol. 1990. 172:5852-5858); the dicC gene (Bejar, S., et al. 1988. Cell division inhibition gene dicB is regulated by a locus similar to lambdoid bacteriophage immunity loci. Mol. Gen. Genet. 212:11-19); the dicF gene (Tetart, F., and J. P. Bouche. 1992. Regulation of the expression of the cell-cycle gene ftsZ by DicF antisense RNA. Division does not require a fixed number of FtsZ molecules. Mol. Microbiol. 6:615-620); the dif gene (Kuempel, P. L., et al. 1991. dif, a recA-independent recombination site in the terminus region of the chromosome of Escherichia coli. New Biol. 3:799-811); the dksA gene (Yamanaka, K., et al. 1994. Cloning, sequencing, and characterization of multicopy suppressors of a mukB mutation in Escherichia coli. Mol. Microbiol. 13:301-312); the dnaK gene (Paek, K. H., and G. C. Walker. 1987. Escherichia coli dnaK null mutants are inviable at high temperature. J. Bacteriol. 169:283-290); the dnaJ gene (Hoffman, H. J., et al. 1992. Activity of the Hsp70 chaperone complex--DnaK, DnaJ, and GrpE--in initiating phage lambda DNA replication by sequestering and releasing lambda P protein. Proc. Natl. Acad. Sci. 89:12108-12111); the fcsA gene (Kudo, T., et al. 1977. Characteristics of a cold-sensitive cell division mutant Escherichia coli K-12. Agric. Biol. Chem. 41:97-107); the fic gene (Utsumi, R., et al. 1982. Involvement of cyclic AMP and its receptor protein in filamentation of an Escherichia coli fic mutant. J. Bacteriol. 151:807-812; Komano, T., et al. 1991. Functional analysis of the fic gene involved in regulation of cell division. Res. Microbiol. 142:269-277); the fis gene (Spaeny-Dekking, L. et al. 1995. Effects of N-terminal deletions of the Escherichia coli protein Fis on the growth rate, tRNA (2Ser) expression and cell morphology. Mol. Gen. Genet. 246:259-265); the ftsA gene (Bi, E., and J. Lutkenhaus. 1990. Analysis of ftsZ, mutations that confer resistance to the cell division inhibitor SulA (SfiA). J. Bacterial. 172:5602-5609; Dai, K, and J. Lutkenhaus. 1992. The proper ration of FtsZ to FtsA is required for cell division to occur in Escherichia coli. J. Bacteriol. 174:6145-6151); the ftsE gene (Taschner, P.E. et al. 1988. Division behavior and shape changes in isogenic ftsZ, ftsQ, ftsA, pbpB, and ftsE cell division mutants of Escherichia coli during temperature shift experiments. J. Bacteriol. 170:1533-1540); the ftsH gene (Ogura, T. et al. 1991. Structure and function of the ftsH gene in Escherichia coli. Res. Microbiol. 142:279-282); the ftsl gene (Begg, K. J., and W. D. Donachie. 1985. Cell shape and division in Escherichia coli: experiments with shape and division mutants. J. Bacteriol. 163:615-622); the ftsJ gene (Ogura, T. et al. 1991. Structure and function of the ftsH gene in Escherichia coli. Res. Microbiol. 142:279-282); the ftsL gene (Guzman, et al. 1992. FtsL, an essential cytoplasmic membrane protein involved in cell division in Escherichia coli. J. Bacteriol. 174:7716-7728); the ftsN gene (Dai, K. et al. 1993. Cloning and characterization of ftsN, an essential cell division gene in Escherichia coli isolated as a multicopy suppressor of ftsA12(Ts). J. Bacteriol. 175:3790-3797); the ftsQ gene (Wang, X. D. et al. 1991. A factor that positively regulates cell division by activating transcription of the major cluster of essential cell division genes of Escherichia coli. EMBO J. 10:3362-3372); the ftsW gene (Khattar, M. M. et al. 1994. Identification of FtsW and characterization of a new ftsW division mutant of Escherichia coli. J. Bacteriol. 176:7140-7147); the ftsX (ftsS) gene (Salmond, G. P. and S. Plakidou. 1984. Genetic analysis of essential genes in the ftsE region of the Escherichia coli genetic map and identification of a new cell division gene, ftsS. Mol. Gen. Genet. 197:304-308); the ftsY gene (Gill, D. R. and G. P. Salmond. 1990. The identification of the Escherichia coli ftsY gene product: an unusual protein. Mol. Microbiol. 4:575-583); the ftsZ gene (Ward, J. E., and J. Lutkenhaus. 1985. Overproduction of FtsZ induces minicell formation. Cell. 42:941-949; Bi, E., and J. Lutkenhaus. 1993. Cell division inhibitors SulA and MinCD prevent formation of the FtsZ ring. J. Bacteriol. 175:1118-1125); the gyrB gene (Mulder, E., et al. 1990. The Escherichia coli minB mutation resembles gyrB in defective nucleoid segregation and decreased negative supercoiling of plasmids. Mol. Gen. Genet. 221:87-93); the hlfB (ftsH)gene (Herman, C., et al. 1993. Cell growth and lambda phage development controlled by the same essential Escherichia coli gene, ftsH/hflB. Proc. Natl. Acad. Sci. 90:10861-10865); the hfq gene (Takada, A., et al. 1999. Negative regulatory role of the Escherichia coli hfq gene in cell division. Biochem. Biophys. Res. Commun. 266:579-583; the hipA gene (Scherrer, R., and H. S. Moyed. 1988. Conditional impairment of cell division and altered lethality in hipA mutants of Escherichia coli K-12. J. Bacteriol. 170:3321-3326); the hipB gene (Hendricks, E. C., et al. 2000. Cell division, guillotining of dimer chromosomes and SOS induction in resolution mutants (dif, xerC and xerD) of Escherichia coli. Mol. Microbiol. 36:973-981 the hns gene (Kaidow, A., et al. 1995. Anucleate cell production by Escherichia coli delta hns mutant lacking a histone-like protein, H-NS. J. Bacteriol. 177:3589-3592); the htrB gene (Karow, M., et al. 1991. Complex phenotypes of null mutations in the htr genes, whole products are essential for Escherichia coli growth at elevated temperatures. Res. Microbiol. 142:289-294); the !pxC(envA)gene (Beall, B., and J. Lutkenhaus. 1987. Sequence analysis, transcriptional organization, and insertional mutagenesis of the envA gene of Escherichia coli. J. Bacteriol. 169:5408-5415; Young, K., et al. 1995. The envA permeability/cell division gene of Escherichia coli encodes the second enzyme of lipid A biosynthesis. UDP-3-O-(R-3-hydroxymyristoyl)-N-acetylglucosamine deacetylase. J. Biol. Chem. 270:30384-30391); the malE gene (Pichoff, S., et al. 1997. MinCD-independent inhibition of cell division by a protein that fuses MalE to the topological specificity factor MinE. J. Bacteriol. 179:4616-4619); the minA gene (Davie, E., et al. 1984. Genetic basis of minicell formation in Escherichia coli K-12. J. Bacteriol. 158:1202-1203); the minB gene (Davie, E., et al. 1984. Genetic basis of minicell formation in Escherichia coli K-12. J. Bacteriol. 158:1202-1203); the minC gene (de Boer, P. A., et al. 1990. Central role for the Escherichia coli minC gene product in two different cell division-inhibition systems. Proc. Natl. Acad. Sci. 87:1129-1133); the minD gene (Labie, C., et al. 1990. Minicell-forming mutants of Escherichia coli: suppression of both DicB- and MinD-dependent division inhibition by inactivation of the minC gene product. J. Bacteriol. 172:5852-5855; Hayashi, I., et al. 2001. Structural and functional studies of MinD ATPase: implications for the molecular recognition of the bacterial cell division apparatus. EMBO J. 20:1819-1828); the minE gene (de Boer, P. A., et al. 1989. A division inhibitor and a topological specificity factor coded for by the minicell locus determine proper placement of the division septum in E. coli. Cell. 56:641-649); the mreB gene (Doi, M., et al. 1988. Determinations of the DNA sequence of the mreB gene and of the gene products of the mre region that function in formation of the rod shape of Escherichia coli cells. J. Bacteriol. 170:4619-4624); the mreC gene (Wachi, M., et al. 1989. New mre genes mreC and mreD, responsible for formation of the rod shape of Escherichia coli cells. J. Bacteriol. 171:6511-6516); the mreD gene (Wachi, M., et al. 1989. New mre genes mreC and mreD, responsible for formation of the rod shape of Escherichia coli cells. J. Bacteriol. 171:6511-6516); the mukA gene (Hiraga, S., et al. 1989. Chromosome partitioning in Escherichia coli: novel mutants producing anucleate cells. J. Bacteriol. 171:1496-1505); the mukB gene (Hiraga, S., et al. 1991. Mutants defective in chromosome partitioning in E. coli. Res. Microbiol. 142:189-194); the mukE gene (Yamanaka, K., et al. 1996. Identification of two new genes, mukE and mukF, involved in chromosome partitioning in Escherichia coli. Mol. Gen. Genet. 250:241-251; Yamazoe, M., et al. 1999. Complex formation of MukB, MukE and MukF proteins involved in chromosome partitioning in Escherichia coli. EMBO J. 18:5873-5884); the mukF gene (Yamanaka, K., et al. 1996. Identification of two new genes, mukE and mukF, involved in chromosome partitioning in Escherichia coli. Mol. Gen. Genet. 250:241-251; Yamazoe, M., et al. 1999. Complex formation of MukB, MukE and MukF proteins involved in chromosome partitioning in Escherichia coli. EMBO J. 18:5873-5884); the parC gene (Kato, J., et al. 1988. Gene organization in the region containing a new gene involved in chromosome partition in Escherichia coli. J. Bacteriol. 170:3967-3977); the parE gene (Roberts, R. C., et al. 1994. The parDE operon of the broad-host-range plasmid RK2 specifies growth inhibition associated with plasmid loss. J. Mol. Biol. 237:35-51 the pbpA gene (Rodriguez, M. C., and M. A. de Pedro. 1990. Initiation of growth in pbpAts and rodAts mutants of Escherichia coli. FEMS Microbiol. Lett. 60:235-239); the pcnB gene (Makise, M., et al. 1999. Identification of a high-copy-number plasmid suppressor of a lethal phenotype caused by mutant DnaA protein which has decreased intrinsic ATPase activity. Biol. Pharm. Bull. 22:904-909); the parF (plsC in E. coli) gene product from Salmonella (Luttinger, A. L., et al. 1991. A cluster of genes that affects nucleoid segregation in Salmonella typhimurium. New Biol. 3:687-697); the rpoS gene (Cam, K., et al. 1995. Sigma S-dependent overexpression of ftsZ in an Escherichia coli K-12 rpoB mutant that is resistant to the division inhibitors DicB and DicF RNA. Mol. Gen. Genet. 248:190-194); the rcsB gene (Gervais, F. G., et al. 1992. The rcsB gene, a positive regulator of colanic acid biosynthesis in Escherichia coli, is also an activator of ftsZ expression. J. Bacteriol. 174:3964-3971 the rcsF gene (Gervais, F. G., and G. R. Drapeau. 1992. Identification, cloning, and characterization of rcsF, a new regulator gene for exopolysaccharide synthesis that suppresses the division mutation ftsZ84 in Escherichia coli K-12. J. Bacteriol. 174:8016-8022); the rodA gene (Rodriguez, M. C., and M. A. de Pedro. 1990. Initiation of growth in pbpAts and rodAts mutants of Escherichia coli. FEMS Microbiol. Lett. 60:235-239); the sdiA (sulB, sfiB) gene (Wang, X. D., et al. 1991. A factor that positively regulates cell division by activating transcription of the major cluster of essential cell division genes of Escherichia coli. EMBO J. 10:3363-3372); the sefA (fabZ) gene (Mohan, S., et al. 1994. An Escherichia coli gene (FabZ) encoding (3R)-hydroxymyristoyl acyl carrier protein dehydrase. Relation to fabA and suppression of mutations in lipid A biosynthesis. J. Biol Chem. 269:32896-32903); the sfic gene (D' Ari, R., and O. Huisman. 1983. Novel mechanism of cell division inhibition associated with the SOS response in Escherichia coli. J. Bacteriol. 156:243-250); the sulA gene (Bi, E., and J. Lutkenhaus. 1990. Interaction between the min locus and ftsZ. J. Bacteriol. 172:5610-5616; Bi, E., and J. Lutkenhaus. 1993. Cell division inhibitors SulA and MinCD prevent formation of the FtsZ ring. J. Bacteriol. 175:1118-1125); the stfZ gene (Dewar, S. J., and W. D. Donachie. 1993. Antisense transcription of the ftsZ-ftsA gene junction inhibits cell division in Escherichia coli. J. Bacteriol. 175:7097-7101 the tolC gene (Hiraga, S., et al. 1989. Chromosome partitioning in Escherichia coli: novel mutants producing anucleate cells. J. Bacteriol. 171:1496-1505; Hiraga, S., et al. 1991. Mutants defective in chromosome partitioning in E. coli. Res. Microbiol. 142:189-194); and the zipA gene (Hale, C. A., and P. A. de Boer. 1997. Direct binding of FtsZ to ZipA, an essential component of the septal ring structure that mediates cell division in E. coli. Cell. 88:175-185).

### II.A.3. Bacillus subtilis Genes

Exemplary genes and gene products from *B. subtilis,* the expression and/or sequence of which can be manipulated so as to stimulate minicell production in *B. subtilis* or any other organism, as can homologs thereof from any species, include without limitation, the divI (divD)gene (Van Alstyne, D., and M. I. Simon. 1971. Division mutants of Bacillus subtilis: isolation of PBS1 transduction of division-specific markers. J. Bacteriol. 108:1366-1379); the divIB (dds, ftsQ) gene (Harry, E. J., et al. 1993. Characterization of mutations in divIB of Bacillus subtilis and cellular localization of the DivIB protein. Mol. Microbiol. 7:611-621; Harry E. J., et al. 1994. Expression of divIB of Bacillus subtilis during vegetative growth. J. Bacteriol. 176:1172-1179); the divIC gene product from B. subtilis or homologues of this gene or gene product found in other members of the Eubacteria, Eucarya or Archaea may be employed to produce minicells (Levin, P. A., and R. Losick. 1994. Characterization of a cell division gene from Bacillus subtilis that is required for vegetative and sporulation septum formation. J. Bacteriol. 176:1451-1459; Katis, V. L. , et al. 1997. The Bacillus subtilis division protein DivIC is a highly abundant membrane-bound protein that localizes to the division site; the divII (divC) gene (Van Alstyne, D., and M. I. Simon. 1971. Division mutations of Bacillus subtilis: isolation and PBS1 transduction of division-specific markers. J. Bacteriol. 108:1366-1379); the divIVA (divD) gene (Cha, J.-H., and G. C. Stewart. 1997. The divIVA minicell locus of Bacillus subtilis. J. Bacteriol. 179:1671-1683); the divIVC (divA) gene (Van Alstyne, D., and M. I. Simon. 1971. Division mutations of Bacillus subtilis: isolation and PBS1 transduction of division-specific markers. J. Bacteriol. 108:1366-1379); the divV (divB) gene (Van Alstyne, D., and M. I. Simon. 1971. Division mutations of Bacillus subtilis: isolation and PBS1 transduction of division-specific markers. J. Bacteriol. 108:1366-1379); the erzA (ytwP) gene (Levin, P. A., et al. 1999. Identification and regulation of a negative regulator of FtsZ ring formation in Bacillus subtilis. Proc. Natl. Acad. Sci. 96:9642-9647); the ftsA (spoIIN) gene (Feucht, A., et al. 2001. Cytological and biochemical characterization of the FtsA cell division protein of Bacillus subtilis. Mol. Microbiol. 40:115-125); the ftsE gene (Yoshida, K., et al. 1994. Cloning and nucleotide sequencing of a 15 kb region of the Bacillus subtilis genome containing the iol operon. Microbiology. 140:2289-2298); the ftsH gene (Deuerling. E., et al. 1995. The ftsH gene of Bacillus subtilis is transiently induced after osmotic and temperature upshift. J. Bacteriol. 177:4105-4112; Wehrl, W., et al. 2000. The FtsH protein accumulates at the septum of Bacillus subtilis during cell division and sporulation. J. Bacteriol. 182:3870-3873); the ftsK gene (Sciochetti, S. A., et al. 2001. Identification and characterization of the dif Site from Bacillus subtilis. J. Bacteriol. 183:1058-1068); the ftsL (yllD)gene (Daniel, R. A., et al. 1998. Characterization of the essential cell division gene ftsL (yllD) of Bacillus subtilis and its role in the assembly of the division apparatus. Mol. Microbiol. 29:593-604); the ftsW gene (Ikeda, M., et al. 1989. Structural similarity among Escherichia coli FtsW and RodA proteins and Bacillus subtilis SpoVE protein, which function in cell division, cell elongation, and spore formation, respectively. J. Bacteriol. 171:6375-6378); the ftsX gene (Reizer, J., et al. 1998. A novel protein kinase that controls carbon catabolite repression in bacteria. Mol. Microbiol. 27:1157-1169); the ftsZ gene (Beall, B., and J. Lutkenhaus). FtsZ in Bacillus subtilis is required for vegetative septation and for asymmetric septation during sporulation. Genes and Dev. 5:447-45); the gcaD gene (Hove-Jensen, B. 1992. Identification of tms-26 as an allele of the gcaD gene, which encodes N-acetylglucosamine 1-phosphate uridyltransferase in Bacillus subtilis. J. Bacteriol. 174:6852-6856); the gid (ylyC) gene (Kunst, F., et al. 1997. The complete genome sequence of the gram-positive bacterium Bacillus subtilis. Nature. 390:237-238); the gidA gene (Ogasawara, N., and H. Yoshikawa. 1992. Genes and their organization in the replication origin region of the bacterial chromosome. Mol. Microbiol. 6:629-634; Nakayashiki, T., and H. Inokuchi. 1998. Novel temperature-sensitive mutants of Escherichia coli that are unable to grow in the absence of wild-type tRNA6Leu. J. Bacteriol. 180:2931-2935); the gidB gene (Ogasawara, N., and H. Yoshikawa. 1992. Genes and their organization in the replication origin region of the bacterial chromosome. Mol. Microbiol. 6:629-634; Nakayashiki, T., and H. Inokuchi. 1998. Novel temperature-sensitive mutants of Escherichia coli that are unable to grow in the absence of wild-type tRNA6Leu. J. Bacteriol. 180:2931-2935); the lytC (cwlB) gene (Blackman, S. A., et al. 1998. The role of autolysins during vegetative growth of Bacillus subtilis 168. Microbiology. 144:73-82); the lytD (cwlG) gene (Blackman, S. A., et al. 1998. The role of autolysins during vegetative growth of Bacillus subtilis 168. Microbiology. 144:73-82); the lytE (cwlF) gene (Ishikawa, S., et al. 1998. Regulation of a new cell wall hydrolase gene, cwlF, which affects cell separation in Bacillus subtilis. J. Bacteriol. 180:23549-2555); the lytF (cwlE, yhdD) gene (Ohnishi, R., et al. 1999. Peptidoglycan hydrolase lytF plays a role in cell separation with CwIF during vegetative growth of Bacillus subtilis. J. Bacteriol. 181:3178-1384); the maf gene (Butler, Y. X., et al. 1993. Amplification of the Bacillus subtilis maf gene results in arrested septum formation. J. Bacteriol. 175:3139-3145); the minC gene (Varley, A. W., and G. C. Stewart. 1992. The divIVB region of the Bacillus subtilis chromosome encodes homologs of Escherichia coli septum placement (minCD) and cell shape (mreBCD) determinants. J. Bacteriol. 174:6729-6742; Barak, I., et al. 1998. MinCD proteins control the septation process during sporulation of Bacillus subtilis. J. Bacteriol. 180:5327-5333); the minD gene (Varley, A. W., and G. C. Stewart. 1992. The divIVB region of the Bacillus subtilis chromosome encodes homologs of Escherichia coli septum placement (minCD) and cell shape (mreBCD) determinants. J. Bacteriol. 174:6729-6742; Barak, I., et al. 1998. MinCD proteins control the septation process during sporulation of Bacillus subtilis. J. Bacteriol. 180:5327-5333); the pbpB gene (Daniel, R. A., and J. Errington. 2000. Intrinsic instability of the essential cell division protein FtsL of Bacillus subtilis and a role for DivIB protinein FtsL turnover. Mol. Microbiol. 35:278-289); the ponA gene (Pederson, L. B., et al. Septal localization of penicillin-binding protein 1 in Bacillus subtilis. J. Bacteriol. 181:3201-3211); the prfA gene (Popham, D. L., and P. Setlow. 1995. Cloning, nucleotide sequence, and mutagenesis of the Bacillus subtilis ponA operon, which codes for penicillin-binding protein (PBP) 1 and a PBP-related factor. J. Bacteriol. 177:326-335); the rodB gene (Burdett, I. D. 1979. Electron microscope study of the rod-to-coccus shape change in a temperature-sensitive rod- mutant of Bacillus subtilis. J. Bacteriol. 137:1395-1405; Burdett, I. D. 1980. Quantitative studies of rod--coccus morphogenesis in a temperature-sensitive rod- mutant of Bacillus subtilis. J. Gen. Microbil. 121:93-103); the secA gene (Sadaie, Y., et al. 1991. Sequencing reveals similarity of the wild-type div+ gene of Bacillus subtilis to the Escherichia coli secA gene. Gene. 98:101-105); the smc gene (Britton, R. A., et al. 1998. Characterization of a prokaryotic SMC protein involved in chromosome partitioning. Genes Dev. 12:1254-1259; Moriya, S., et al. 1998. A Bacillus subtilis gene-encoding protein homologous to eukaryotic SMC motor protein is necessary for chromosome partition. Mol. Microbiol. 29:179-187; Hirano, M., and T. Hirano. 1998. ATP-dependent aggregation of single-stranded DNA by a bacterial SMC homodimer. EMBO J. 17:7139-7148); the spoIIE gene (Feucht, a., et al. 1996. Bifunctional protein required for asymmetric cell division and cell-specific transcription in Bacillus subtilis. Genes Dev. 10:794-803; Khvorova, A., et al. 1998. The spoIIE locus is involved in the Spo0A-dependent switch in the localization of FtsZ rings in Bacillus subtilis. J. Bacteriol. 180:1256-1260; Lucet, I., et al. 2000. Direct interaction between the cell division protein FtsZ and the cell differentiation protein SpoIIE. EMBO J. 19:1467-1475); the spo0A gene (Ireton, K., et al. 1994. spo0J is required for normal chromosome segregation as well as the initiation of sporulation in Bacillus subtilis. J. Bacteriol. 176:5320-5329); the spoIVF gene (Lee, S., and C. W. Price. 1993. The minCD locus of Bacillus subtilis lacks the minE determinant that provides topological specificity to cell division. Mol. Microbiol. 7:601-610); the spo0J gene (Lin, D. C., et el. 1997. Bipolar localization of a chromosome partition protein in Bacillus subtilis. Proc. Natl. Acad. Sci. 94:4721-4726; Yamaichi, Y., and H. Niki. 2000. Active segregation by the Bacillus subtilis partitioning system in Escherichia coli. Proc. NatI. Acad. Sci. 97:14656-14661); the smc gene (Moriya, S., et al. 1998. A Bacillus subtilis gene-encoding protein homologous to eukaryotic SMC motor protein is necessary for chromosome partition. Mol. Microbiol. 29:179-187); the ripX gene (ciochetti, S. A. et al. 1999. The ripX locus of Bacillus subtilis encodes a site-specific recombinase involved in proper chromosome partitioning. J. Bacteriol. 181:6053-6062); and the spoIIIE gene (Wu, L. J., and J. Errington. 1994. Bacillus subtilis spoIIIE protein required for DNA segregation during asymmetric cell division. Science. 264:572-575); the gene corresponding to the B. subtilis mutant alleal ts-31 (Errington, J., and A. D. Richard. Cell division during growth and sporulation. In A. L. Sonenshein, J. A. Hoch., and R. Losick (eds.). Bacillus subtilis and its closest relatives: from genes to cells. American Society for Microbiology, Washington D. C.); the gene corresponding to the B. subtilis mutant alleal ts-526 (Id.); the yacA gene (Kunst, F., et al. 1997. The complete genome sequence of the gram-positive bacterium Bacillus subtilis. Nature. 390:237-238); the yfhF gene (Kunst, F., et al. 1997. The complete genome sequence of the gram-positive bacterium Bacillus subtilis. Nature. 390:237-238); the yfhK gene (Kunst, F., et al. 1997. The complete genome sequence of the gram-positive bacterium Bacillus subtilis. Nature. 390:237-238); the yjoB gene (Kunst, F., et al. 1997. The complete genome sequence of the gram-positive bacterium Bacillus subtilis. Nature. 390:237-238); and the ywbG gene (Smith, T. J., et al. 2000. Autolysins of Bacillus subtilis: multiple enzymes with multiple functions. Microbiology. 146:249-262).

### II.B. Gene Expression in Minicells

### II.B.1. In General

In some aspects of the invention, it may be desirable to alter the expression of a gene and the production of the corresponding gene product. As is known in the art, and is used herein, a "gene product" may be a protein (polypeptide) or nucleic acid. Gene products that are proteins include without limitation enzymes, receptors, transcription factors, termination factors, expression factors, DNA-binding proteins, proteins that effect nucleic acid structure, or subunits of any of the preceding. Gene products that are nucleic acids include, but are not limited to, ribosomal RNAs (rRNAs), transfer RNAs (tRNAs), antisense RNAs, nucleases (including but not limited to catalytic RNAs, ribonucleases, and the like).

Depending on the function of a gene product, and on the type of application of the invention, it may be desirable to increase protein production, decrease protein production, increase protein nucleic acid production and/or increase nucleic acid production. Provided herein are non-limiting examples of genes and gene products that may be manipulated, individually or in combination, in order to modulate the expression of gene products to be included into minicells or parent strains from which minicells are derived. The expression elements so modulated may be chromosomal and/or episomal, and may be expressed constitutively or in a regulated fashion, i.e., repressible and/or inducible. Furthermore, gene products under the regulation may be either monocistronic or polycistronic with other genes or with themselves.

### II.B.2. Protein Production

By way of non-limiting example, increased protein production may occur through increased gene dosage (increased copy number of a given gene under the control of the native or artificial promotor where this gene may be on a plasmid or in more than one copy on the chromosome), modification of the native regulatory elements, including, but not limited to the promotor or operator region(s) of DNA, or ribosomal binding sites on RNA, relevant repressors/silencers, relevant activators/enhancers, or relevant antisense nucleic acid or nucleic acid analog, cloning on a plasmid under the control of the native or artificial promotor, and increased or decreased production of native or artificial promotor regulatory element(s) controlling production of the gene or gene product

By way of non-limiting example, decreased protein production may occur through modification of the native regulatory elements, including, but not limited to the promotor or operator region(s) of DNA, or ribosomal binding sites on RNA, relevant repressors/silencers, relevant activators/enhancers, or relevant antisense nucleic acid or nucleic acid analog, through cloning on a plasmid under the control of the native regulatory region containing mutations or an artificial promotor, either or both of which resulting in decreased protein production, and through increased or decreased production of native or artificial promotor regulatory element(s) controlling production of the gene or gene product.

As used herein with regards to proteins, "intramolecular activity" refers to the enzymatic function or structure-dependent function. By way of non-limiting example, alteration of intramolecular activity may be accomplished by mutation of the gene, in vivo or in vitro chemical modification of the protein, inhibitor molecules against the function of the protein, e.g. competitive, non-competitive, or uncompetitive enzymatic inhibitors, inhibitors that prevent protein-protein, protein-nucleic acid, or protein-lipid interactions, e.g. expression or introduction of dominant-negative or dominant-positive protein or other protein fragment(s), carbohydrate(s), fatty acid(s), lipid(s), and nucleic acid(s) that may act directly or allosterically upon the protein, and/or modification of protein, carbohydrate, fatty acid, lipid, or nucleic acid moieties that modify the gene or gene product to create the functional protein.

As used herein with regards to proteins, "intermolecular function" refers to the effects resulting from an intermolecular interaction between the protein or nucleic acid and another protein, carbohydrate, fatty acid, lipid, nucleic acid, or other molecule(s) in or on the cell or the action of a product or products resulting from such an interaction. By way of non-limiting example, intermolecular or intramolecular function may be the act or result of intermolecular phosphorylation, biotinylation, methylation, acylation, glycosylation, and/or other signaling event; this function may be the result of a protein-protein, protein-nucleic acid, or protein-lipid complex, and/or carrier function, e.g. the capacity to bind, either covalently or non-covalently small organic or inorganic molecules, protein(s), carbohydrate(s), fatty acid(s), lipid(s), and nucleic acid(s); this function may be to interact with the membrane to recruit other molecules to this compartment of the cell; this function may be to regulate the transcription and/or translation of the gene, other protein, or nucleic acid; and this function may be to stimulate the function of another process that is not yet described or understood.

### II.B.3. Nucleic Acid Production

By way of non-limiting example, increased nucleic acid production may occur through increased gene dosage (increased copy number of a given gene under the control of the native or artificial promotor where this gene may be on a plasmid or in more than one copy on the chromosome), modification of the native regulatory elements, including, but not limited to the promotor or operator region(s) of DNA, or ribosomal binding sites on RNA, relevant repressors/siiencers, relevant activators/enhancers, or relevant antisense nucleic acid or nucleic acid analog, cloning on a plasmid under the control of the native or artificial promotor, and increased or decreased production of native or artificial promotor regulatory element(s) controlling production of the gene or gene product.

By way of non-limiting example, decreased nucleic acid production may occur through modification of the native regulatory elements, including, but not limited to the promotor or operator region(s) of DNA, or ribosomal binding sites on RNA, relevant repressors/siiencers, relevant activators/enhancers, or relevant antisense nucleic acid or nucleic acid analog, through cloning on a plasmid under the control of the native regulatory region containing mutations or an artificial promotor, either or both of which resulting in decreased protein production, and through increased or decreased production of native or artificial promotor regulatory element(s) controlling production of the gene or gene product.

As used herein with regards to nucleic acids, "intramolecular activity" refers to a structure-dependent function. By way of non-limiting example, alteration of intramolecular activity may be accomplished by mutation of the gene, in vivo or in vitro chemical modification of the nucleic acid, inhibitor molecules against the function of the nucleic acid, e.g. competitive, non-competitive, or uncompetitive enzymatic inhibitors, inhibitors that prevent protein-nucleic acid interactions, e.g. expression or introduction of dominant-negative or dominant-positive protein or other nucleic acid fragment(s), or other carbohydrate(s), fatty acid(s), and lipid(s) that may act directly or allosterically upon the nucleic acid or nucleic acid-protein complex, and/or modification of nucleic acid moieties that modify the gene or gene product to create the functional nucleic acid.

As used herein with regards to nucleic acids, "intermolecular function" refers to the effects resulting from an intermolecular interaction between the nucleic acid and another nucleic acid, protein, carbohydrate, fatty acid, lipid, or other molecule(s) in or on the cell or the action of a product or products resulting from such an interaction. By way of non-limiting example, intermolecular function may be the act or result of intermolecular or intramolecular phosphorylation, biotinylation, methylation, acylation, glycosylation, and/or other signaling event; this function may be the result of a protein-nucleic acid, and/or carrier function, e.g. the capacity to bind, either covalently or non-covalently small organic or inorganic molecules, protein(s), carbohydrate(s), fatty acid(s), lipid(s), and other nucleic acid(s); this function may be to interact with the membrane to recruit other molecules to this compartment of the cell; this function may be to regulate the transcription and/or translation of the gene, other nucleic acid, or protein; and this function may be to stimulate the function of another process that is not yet described or understood.

### II.C. Genes and Gene Products for Regulation of Expression

As is known in the art, a variety of genes, gene products and expression elements may be manipulated, individually or in combination, in order to modulate the expression of genes and/or production gene products. These include, by way of non-limiting example, RNA polymerases, ribosomes (ribosomal proteins and ribosomal RNAs), transfer RNAs (tRNAs), amino transferases, regulatory elements and promoter regions, transportation of inducible and inhibitory compounds, catabolite repression, general deletions and modifications, cytoplasmic redox state, transcriptional terminators, mechanisms for ribosomal targeting, proteases, chaperones, export apparatus and membrane targeting, and mechanisms for increasing stability and solubility. Each of these is discussed in more detail in the following sections.

### II.C.1. Regulatory Elements and Promoter Regions

Included in the design of the invention are techniques that increase the efficiency of gene expression and protein production in minicells. By way of non-limiting example, these techniques may include utilization and/or modification of regulatory elements and promoter regions. Such manipulations may result in increased or decreased production, and/or changes in the intramolecular and intermolecular functions, of a protein in a segregated minicell or its parent cell prior to minicell formation; in the latter instance, the protein may be one that is desirably retained in segregated minicells.

The production or activity of a desired gene product may be increased by increasing the level and/or activity of a promoter or other regulatory region that acts to stimulate or enhance the production of the desired gene product. The production or activity of a desired gene product may be increased by decreasing the level and/or activity of a promoter or other regulatory region that acts to stimulate or enhance the production of a gene product that acts to reduce or eliminate the level and/or activity of the desired gene product.

### II.C.1.a. Escherichia coli

Regulatory elements, promoters and other expression elements and expression factors from *E. coli* include but are not limited to *acrR* (Ma, D., et al. 1996. The local repressor AcrR plays a modulating role in the regulation of acrAB genes of Escherichia coli by global stress signals. Mol. Microbiol. 19:101-112); *ampD* (Lindquist, S., et al. 1989. Signalling proteins in enterobacterial AmpC beta-lactamase regulation. Mol. Microbiol. 3:1091-1102; Holtje, J. V., et al. 1994. The negative regulator of beta-lactamase induction AmpD is a N-acetyl-anhydromuramyl-L-alanine amidase. FEMS Microbiol. Lett. 122:159-164); *appR* (Diaz-Guerra, L., et al. 1989. appR gene product activates transcription of microcin C7 plasmid genes. J. Bacteriol. 171:2906-2908; Touati, E., et al. 1991. Are appR and katF the same Escherichia coli gene encoding a new sigma transcription initiation factor? Res. Microbiol. 142:29-36); *appY* (Atlung, T., et al. 1989. Isolation, characterization, and nucleotide sequence of appY, a regulatory gene for growth-phase-dependent gene expression in Escherichia coli. J. Bacteriol. 171:1683-1691); araC (Casadaban, M. J., et al. 1976. Regulation of the regulatory gene for the arabinose pathway, araC. J. Mol. Biol. 104:557-566); *arcA* (luchi, S., and E. C. Lin. 1988. arcA (dye), a global regulatory gene in Escherichia coli mediating repression of enzymes in aerobic pathways. Proc. Natl. Acad. Sci. 85:1888-1892; luchi, S., et al. 1989. Differentiation of arcA, arcB, and cpxA mutant phenotypes of Escherichia coli by sex pilus formation and enzyme regulation. J. Bacteriol. 171:2889-2893); *argR* (xerA, Rarg) (Kelln, R. A., and V. L. Zak. 1978. Arginine regulon control in a Salmonella typhimurium--Escherichia coli hybrid merodiploid. Mol. Gen Genet. 161:333-335; Vogel, R. H., et al. 1978. Evidence for translational repression of arginine biosynthetic enzymes in Escherichia coli: altered regulation in a streptomycin-resistant mutant. Mol. Gen. Genet. 162:157-162); *ascG* (Hall, B. G., and L. Xu. Nucleotide sequence, function, activation, and evolution of the cryptic asc operon of Escherichia coli K12. Mol. Biol. Evol. 9:688-706); *aslB* (Bennik, M. H., et al. 2000. Defining a rob regulon in Escherichia coli by using transposon mutagenesis. J. Bacteriol. 182:3794-3801); *asnC* (Kolling, R., and H. Lother. 1985. AsnC: an autogenously regulated activator of asparagine synthetase A transcription in Escherichia coli. J. Bacteriol. 164:310-315); *atoC* (Jenkins, L. S., and W. D. Nunn. 1987. Regulation of the ato operon by the atoC gene in Escherichia coli. J. Bacteriol. 169:2096-2102); *baeR* (Nagasawa, S., et al. 1993. Novel members of the two-component signal transduction genes in Escherichia coli. J. Biochem. (Tokyo). 114:350-357); *baeS* (*Id*.Id.); *barA* (Nagasawa, S., et al. 1992. A novel sensor-regulator protein that belongs to the homologous family of signal-transduction proteins involved in adaptive responses in Escherichia coli. Mol. Microbiol. 6:799-807; Ishige, K., et al. 1994. A novel device of bacterial signal transducers. EMBO J. 13:5195-5202); *basS* (Nagasawa, S., et al. 1993. Novel members of the two-component signal transduction genes in Escherichia coli. J. Biochem. (Tokyo). 114:350-357); *betI* (Lamark, T., et al. 1996. The complex bet promoters of Escherichia coli: regulation by oxygen (ArcA), choline (Betl), and osmotic stress. J. Bacteriol. 178:1655-1662); bglG (bglC, bglS) (Schnetz, K., and B. Rak. 1988. Regulation of the bgl operon of Escherichia coli by transcriptional antitermination. EMBO J. 7:3271-3277; Schnetz, K., and B. Rak. 1990. Beta-glucoside permease represses the bgl operon of Escherichia coli by phosphorylation of the antiterminator protein and also interacts with glucose-specific enzyme III, the key element in catabolite control. Proc. NatI. Acad. Sci. 87:5074-5078); *birA* (*bioR*, *dhbB*) (Barker, D. F., and A. M. Campbell. 1981. Genetic and biochemical characterization of the birA gene and its product: evidence for a direct role of biotin holoenzyme synthetase in repression of the biotin operon in Escherichia coli. J. Mol. Biol. 146:469-492; Barker, D. F., and A. M. Campbell. 1981. The birA gene of Escherichia coli encodes a biotin holoenzyme synthetase. J. Mol. Biol. 146:451-467; Howard, P. K., et al. 1985. Nucleotide sequence of the birA gene encoding the biotin operon repressor and biotin holoenzyme synthetase functions of Escherichia coli. Gene. 35:321-331); *btuR* (Lundrigan, M. D., et al. 1987. Separate regulatory systems for the repression of metE and btuB by vitamin B12 in Escherichia coli. Mol. Gen. Genet. 206:401-407; Lundrigan, M.D., and R. J. Kadner. 1989. Altered cobalamin metabolism in Escherichia coli btuR mutants affects btuB gene regulation. J. Bacteriol. 171:154-161); cadC (Watson, N., et al. 1992. Identification of elements involved in transcriptional regulation of the Escherichia coli cad operon by external pH. J. Bacteriol. 174:530-540); *celD* (Parker, L. L., and B. G. Hall. 1990. Characterization and nucleotide sequence of the cryptic cel operon of Escherichia coli K12. Genetics. 124:455-471); *chaB* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *chaC* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *cpxR* (Danese, P. N., et al. 1995. The Cpx two-component signal transduction pathway of Escherichia coli regulates transcription of the gene specifying the stress-inducible periplasmic protease, DegP. Genes Dev. 9:387-398); *crl* (Arnqvist, A., et al. 1992. The Crl protein activates cryptic genes for curli formation and fibronectin binding in Escherichia coli HB101. Mol. Microbiol. 6:2443-2452); *cspA* (Bae, W., et al. 1999. Characterization of Escherichia coli cspE, whose product negatively regulates transcription of cspA, the gene for the major cold shock protein. Mol. Microbiol. 31:1429-1441); cspE (Id.); *csrA* (Liu, M. Y., et al. 1995. The product of the pleiotropic Escherichia coli gene csrA modulates glycogen biosynthesis via effects on mRNA stability. J. Bacteriol. 177:2663-2672); *cynR* (Anderson, P. M., et al. 1990. The cyanase operon and cyanate metabolism. FEMS Microbiol. Rev. 7:247-252; Sung, Y. C., and J. A. Fuchs. 1992. The Escherichia coli K-12 cyn operon is positively regulated by a member of the lysR family. J. Bacteriol. 174:3645-3650); *cysB* (Jagura-Burdzy, G., and D. Hulanicka. 1981. Use of gene fusions to study expression of cysB, the regulatory gene of the cysteine regulon. J. Bacteriol. 147:744-751); *cytR* (Hammer-Jespersen, K., and A. Munch-Ptersen. 1975. Multiple regulation of nucleoside catabolizing enzymes: regulation of the deo operon by the cytR and deoR gene products. Mol. Gen. Genet. 137:327-335); *dadQ* (*alnR*) (Wild, J., and B. Obrepalska. 1982. Regulation of expression of the dadA gene encoding D-amino acid dehydrogenase in Escherichia coli: analysis of dadA-lac fusions and direction of dadA transcription. Mol. Gen. Genet. 186:405-410); *dadR* (*alnR*) (Wild, J., et al. 1985. Identification of the dadX gene coding for the predominant isozyme of alanine racemase in Escherichia coli K12. Mol. Gen. Genet. 198:315-322); *deoR* (*nucR, tsc, nupG*) (Hammer-Jespersen, K., and A. Munch-Ptersen. 1975. Multiple regulation of nucleoside catabolizing enzymes: regulation of the deo operon by the cytR and deoR gene products. Mol. Gen. Genet. 137:327-335); *dgoR* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *dicA* (Bejar, S., et al. 1988. Cell division inhibition gene dicB is regulated by a locus similar to lambdoid bacteriophage immunity loci. Mol. Gen. Genet. 212:11-19); *dnaK* (*gro*, *groP*, *groPAB*, *groPC*, *groPF*, *grpC*, *grpF*, *seg*) (Bochner, B. R., et al. 1986. Escherichia coli DnaK protein possesses a 5'-nucleotidase activity that is inhibited by AppppA. J. Bacteriol. 168:931-935); dniR (Kajie, S., et al. 1991. Molecular cloning and DNA sequence of dniR, a gene affecting anaerobic expression of the Escherichia coli hexaheme nitrite reductase. FEMS Microbiol. Lett. 67:205-211); *dsdC* (Heincz, M. C., and E. McFall. 1978. Role of the dsdC activator in regulation of D-serine deaminase synthesis. J. Bacteriol. 136:96-103); *ebgR* (Hall, B. G., and N. D. Clarke. 1977. Regulation of newly evolved enzymes. III Evolution of the ebg repressor during selection for enhanced lactase activity. Genetics. 85:193-201); *envY* (Lundrigan, M. D., and C. F. Earhart. 1984. Gene envY of Escherichia coli K-12 affects thermoregulation of major porin expression. J. Bacteriol. 157:262-268); *envZ* (*ompB, perA, tpo*) (Russo, F. D, and T. J. Silhavy. 1991. EnvZ controls the concentration of phosphorylated OmpR to mediate osmoregulation of the porin genes. J. Mol. Biol. 222:567-580); *evgA* (Nishino, K., and A. Yamaguichi. 2001. Overexpression of the response regulator evgA of the two-component signal transduction system modulates multidrug resistance conferred by multidrug resistance transporters. J. Bacteriol. 183:1455-1458); *evgS* (*Id.*); *exuR* (Portalier, R., et al. 1980. Regulation of Escherichia coli K-12 hexuronate system genes: exu regulon. J. Bacteriol. 143:1095-1107); *fadR* (*dec, ole, thdB*) (Simons, R. W., et al. 1980. Regulation of fatty acid degradation in Escherichia coli: isolation and characterization of strains bearing insertion and temperature-sensitive mutations in gene fadR. J. Bacteriol. 142:621-632); *fecI* (Van Hove, B., et al. 1990. Novel two-component transmembrane transcription control: regulation of iron dicitrate transport in Escherichia coli K-12. J. Bacteriol. 172:6749-6758); *fecR* (*Id*.); *fhlA* (Maupin, J. A., and K. T. Shanmugam. 1990. Genetic regulation of formate hydrogenlyase of Escherichia coli: role of the fhlA gene product as a transcriptional activator for a new regulatory gene, fhlB. J. Bacteriol. 172:4798-4806; Rossmann, R., et al. 1991. Mechanism of regulation of the formate-hydrogenlyase pathway by oxygen, nitrate, and pH: definition of the formate regulon. Mol. Microbiol. 5:2807-2814); fhlB (Maupin, J. A., and K. T. Shanmugam. 1990. Genetic regulation of formate hydrogenlyase of Escherichia coli: role of the fhlA gene product as a transcriptional activator for a new regulatory gene, fhlB. J. Bacteriol. 172:4798-4806); *fimB* (*pil*) (Pallesen, L., et al. 1989. Regulation of the phase switch controlling expression of type 1 fimbriae in Escherichia coli. Mol. Microbiol. 3:925-931); *fimE* (*pilH*) (*Id*.); *flhC* (*flaI*) (Liu, X., and P. Matsumura. 1994. The FlhD/FlhC complex, a transcriptional activator of the Escherichia coli flagellar class II operons. J. Bacteriol. 176:7345-7351); *flhD* (*flhB*) (*Id*.); *fliA* (*flaD, rpoF*) (Komeda, Y., et al. 1986. Transcriptional control of flagellar genes in Escherichia coli K-12. J. Bacteriol. 168:1315-1318); *fnr* (*frdB, nirA, nirR*) (Jones, H. M., and R. P. Gunsalus. 1987. Regulation of Escherichia coli fumarate reductase (frdABCD) operon expression by respiratory electron acceptors and the fnr gene product. J. Bacteriol. 169:3340-3349); *fruR* (*fruC, shl*) (Geerse, R. H., at al. The PEP: fructose phosphotransferase system in Salmonella typhimurium: FPr combines enzyme IIIFru and pseudo-HPr activities. Mol. Gen. Genet. 216:517-525); *fucR* (Zhu, Y., and E. C. Lin. 1986. An evolvant of Escherichia coli that employs the L-fucose pathway also for growth on L-galactose and D-arabinose. J. Mol. Evol. 23:259-266); *fur* (Bagg, A., and J. B. Neilands. 1987. Ferric uptake regulation protein acts as a repressor, employing iron (II) as a cofactor to bind the operator of an iron transport operon in Escherichia coli. Biochemistry 26:5471-5477); *gadR* gene product from *Lactococcus lactis* (Sanders, J. W., et al. 1997. A chloride-inducible gene expression cassette and its use in induced lysis of Lactococcus lactis. Appl. Environ. Microbiol. 63:4877-4882); *galR* (von Wilcken-Bergmann, B., and B. Muller-Hill. 1982. Sequence of galR gene indicates a common evolutionary origin of lac and gal repressor in Escherichia coli. Proc. Natl. Acad. Sci. 79:2427-2431); *galS* (*mglD*) (Weickert., M. J., and S. Adhya. 1992. Isorepressor of the gal regulon in Escherichia coli. J. Mol. Biol. 226:69-83); galU (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *gatR* (Nobelmann, B., and J. W. Lengeler. 1996. Molecular analysis of the gat genes from Escherichia coli and of their roles in galactitol transport and metabolism. J. Bacteriol. 178:6790-6795); *gcvA* (Wilson, R. L., et al. 1993. Positive regulation of the Escherichia coli glycine cleavage enzyme system. J. Bacteriol. 175:902-904); *glgS* (Hengge-Aronis, R., et al. 1993. Osmotic regulation of rpoS-dependent genes in Escherichia coli. J. Bacteriol. 175:259-265; Yang, H., et al. 1996. Coordinate genetic regulation of glycogen catabolism and biosynthesis in Escherichia coli via the CsrA gene product. J. Bactgeriol. 178:1012-1017); *glnB* (Bueno, R., et al. 1985. Role of glnB and glnD gene products in regulation of the glnALG operon of Escherichia coli. J. Bacteriol. 164:816-822); *glnG* (*gln, ntrC*) (Pahel, G., and B. Tyler. 1979. A new glnA-linked regulatory gene for glutamine synthetase in Escherichia coli. Proc. NatI. Acad. Sci. 76:4544-4548); *glnL* (*glnR, ntrB*) (MacNeil, T., et al. The products of glnL and glnG are bifunctional regulatory proteins. Mol. Gen. Genet. 188:325-333); *glpR* (Silhavy, T. J., et al. 1976. Periplasmic protein related to the sn-glycerol-3-phosphate transport system of Escherichia coli. J. Bacteriol. 126:951-958); *gltF* (Castano, I., et al. gltF, a member of the gltBDF operon of Escherichia coli, is involved in nitrogen-regulated gene expression. Mol. Microbiol. 6:2733-2741); *gntR* (Peekhaus, N., and T. Conway. 1998. Positive and negative transcriptional regulation of the Escherichia coli gluconate regulon gene gntT by GntR and the cyclic AMP (cAMP)-cAMP receptor protein complex. J. Bacteriol. 180:1777-1785); *hha* (Neito, J. M., et al. The hha gene modulates haemolysin expression in Escherichia coli. Mol. Microbiol. 5:1285-1293); *himD* (*hip*) (Goosen, N., et al. 1984. Regulation of Mu transposition. II. The Escherichia coli HimD protein positively controls two repressor promoters and the early promoter of bacteriophage Mu. Gene. 32:419-426); hrpB gene product from *Pseudomonas solanacearum* (Van Gijsegem, F., et al. 1995. The hrp gene locus of Pseudomonas solanacearum, which controls the production of a type III secretion system, encodes eight proteins related to components of the bacterial flagellar biogenesis complex. Mol. Microbiol. 15:1095-1114); *hybF* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *hycA* (Hopper, S., et al. 1994. Regulated expression in vitro of genes coding for formate hydrogenlyase components of Escherichia coli. J. Biol. Chem. 269:19597-19604); *hydG* (Leonhartsberger, S. et al. 2001. The hydH/G genes from Escherichia coli code for a zinc and lead responsive two-component regulatory system. J. Mol. Biol. 307:93-105); *hydH* (Id.); *iciA* (Thony, B., et al. 1991. iciA, an Escherichia coli gene encoding a specific inhibitor of chromosomal initiation of replication in vitro. Proc. Natl. Acad. Sci. 88:4066-4070); *iclR* (Maloy, S. R., and W. D. Nunn. 1982. Genetic regulation of the glyoxylate shunt in Escherichia coli K-12. J. Bacteriol. 149:173-180); ileR (avr, flrA) (Johnson, D. I., and R. L. Somerville. 1984. New regulatory genes involved in the control of transcription initiation at the thr and ilv promoters of Escherichia coli K-12. Mol. Gen. Genet. 195:70-76); *ilvR* (*Id*.); *ilvU* (Fayerman, J. T., et al. 1979. ilvU, a locus in Escherichia coli affecting the derepression of isoleucyl-tRNA synthetase and the RPC-5 chromatographic profiles of tRNAlle and tRNAVal. J. Bio. Chem. 254:9429-9440); ilvY (Wek, R. C., and G. W. Hatfield. 1988. Transcriptional activation at adjacent operators in the divergent-overlapping ilvY and ilvC promoters of Escherichia coli. J. Mol. Biol. 203:643-663); *inaA* (White, S., et al. 1992. pH dependence and gene structure of inaA in Escherichia coli. J. Bacteriol. 174:1537-1543); *inaR* (Id.); *kdgR* (Nemoz, G., et al. 1976. Physiological and genetic regulation of the aldohexuronate transport system in Escherichia coli. J. Bacteriol. 127:706-718); lacI (Riggs, A. D, and S. Bourgeois. 1968. On the assay, isolation and characterization of the lac repressor. J. Mol. Biol. 34:361-364); *leuO* (Shi, X., and G. N. Bennett. 1995. Effects of multicopy LeuO on the expression of the acid-inducible lysine decarboxylase gene in Escherichia coli. J. Bacteriol. 177:810-814; Klauck, E., et al. 1997. The LysR-like regulator LeuO in Escherichia coli is involved in the translational regulation of rpoS by affecting the expression of the small regulatory DsrA-RNA. Mol. Microbiol. 25:559-569); *leuR* (Theall, G., et al. 1979. Regulation of the biosynthesis of aminoacyl-tRNA synthetases and of tRNA in Escherichia coli. IV. Mutants with increased levels of leucyl- or seryl-tRNA synthetase. Mol. Gen. Genet. 169:205-211); *leuY* (Morgan, S., et al. 1979. Regulation of biosynthesis of aminoacyl-transfer RNA synthetases and of transfer-RNA in Escherichia coli. Arch. Biol. Med. Exp. (Santiago) 12:415-426); *lexA* (Mount, D. W. 1977. A mutant of Escherichia coli showing constitutive expression of the lysogenic induction and error-prone DNA repair pathways. Proc. Natl. Acad. Sci. 74:300-304; Little, J. W., et al. 1980. Cleavage of the Escherichia coli lexA protein by the recA protease. Proc. Natl. Acad. Sci. 77:3225-3229); *lldR* (*lctR*) (Dong, J. M., et al. 1993. Three overlapping lct genes involved in L-lactate utilization by Escherichia coli. J. Bacteriol. 175:6671-6678); /*pp* (Brosius, J. Expression vectors employing lambda-, trp-, lac-, and lpp-derived promoters. 1988. Biotechnology. 10:205-225); *lrhA* (*genR*) (Bongaerts, J., et al. 1995. Transcriptional regulation of the proton translocating NADH dehydrogenase genes (nuoA-N) of Escherichia coli by electron acceptors, electron donors and gene regulators. Mol. Microbiol. 16:521-534); *lrp* (*ihb, livR, lss, lstR, oppl, rblA, mbf*) (lto, K., et al. Multiple control of Escherichia coli lysyl-tRNA synthetase expression involves a transcriptional repressor and a translational enhancer element. Proc. NatI. Acad. Sci. 90:302-306); *lysR* (Gicquel-Sanzey, B. and P. Cossart. 1982. Homologies between different procaryotic DNA-binding regulatory proteins and between their sites of action. EMBO J. 1:591-595; Stragier, P., et al. 1983. Regulation of diaminopimelate decarboxylase synthesis in Escherichia coli. II. Nucleotide sequence of the lysA gene and its regulatory region. J. Mol. Biol. 168:321-331); mall (Reidl, J., et al. 1989. Mall, a novel protein involved in regulation of the maltose system of Escherichia coli, is highly homologous to the repressor proteins GalR, CytR, and LacI. J. Bacteriol. 171:4888-4499); *malT* (*malA*) (Bebarbouille, M., and M. Schwartz. Mutants which make more malT product, the activator of the maltose regulon in Escherichia coli. Mol. Gen. Genet. 178:589-595); *marA* (*cpxB, soxQ*) (Ariza, R. R., et al. Repressor mutations in the marRAB operon that activate oxidative stress genes and multiple antibiotic resistance in Escherichia coli. J. Bacteriol. 176:143-148); *marB* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *marR* (Ariza, R. R., et al. Repressor mutations in the marRAB operon that activate oxidative stress genes and multiple antibiotic resistance in Escherichia coli. J. Bacteriol. 176:143-148); *melR* (Williams, J., et al. 1994. Interactions between the Escherichia coli MelR transcription activator protein and operator sequences at the melAB promoter. Biochem. J. 300:757-763); *metJ* (Smith, A. A., et al. 1985. Isolation and characterization of the product of the methionine-regulatory gene metJ of Escherichia coli K-12. Proc. Natl. Acad. Sci. 82:6104-6108; Shoeman, R., et al. 1985. Regulation of methionine synthesis in Escherichia coli: effect of metJ gene product and S-adenosylmethionine on the in vitro expression of the metB, metL and metJ genes. Biochem. Biophys. Res. Commun. 133:731-739); metR (Maxon, M. E., et al. 1989. Regulation of methionine synthesis in Escherichia coli: effect of the MetR protein on the expression of the metE and metR genes. Proc. Natl. Acad. Sci. 86:85-89); *mglR* (*R-MG*) (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *mhpR* (Ferrandez, A., et al. 1997. Genetic characterization and expression in heterologous hosts of the 3-(3-hydroxyphenyl)propionate catabolic pathway of Escherichia coli K-12. J. Bacteriol. 179:2573-2581); *mhpS* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *micF (stc)* (Aiba, H., et al. 1987. Function of micF as an antisense RNA in osmoregulatory expression of the ompF gene in Escherichia coli. J. Bacteriol. 169:3007-3012); *mprA* (*emrR*) (del Castillo, I., et al. 1990. mprA, an Escherichia coli gene that reduces growth-phase-dependent synthesis of microcins B17 and C7 and blocks osmoinduction of proU when cloned on a high-copy-number plasmid. J. Bacteriol. 172:437-445); mtlR (Figge, R. M., et al. 1994. The mannitol repressor (MtlR) of Escherichia coli. J. Bacteriol. 176:840-847); nagC (nagR) (Plumbridge, J. A. 1991. Repression and induction of the nag regulon of Escherichia coli K-12: the roles of nagC and nagA in maintenance of the uninduced state. Mol. Microbiol. 5:2053-3062); *narL* (*frdR*, *narR*) (Stewart, V. 1982. Requirement of Fnr and NarL functions for nitrate reductase expression in Escherichia coli K-12. J. Bacteriol. 151:1320-1325; Miller, J. B., et al. 1987. Molybdenum-sensitive transcriptional regulation of the chlD locus of Escherichia coli. J. Bacteriol. 169:1853-1860; luchi, S., and E. C. Lin. 1987. Molybdenum effector of fumarate reductase repression and nitrate reductase induction in Escherichia coli. J. Bacteriol. 169:3720-3725); *narP* (Rabin, R. S., and V. Stewart. 1993. Dual response regulators (NarL and NarP) interact with dual sensors (NarX and NarQ) to control nitrate- and nitrite-regulated gene expression in Escherichia coli K-12. J. Bacteriol. 175:3259-3268); nhaR (gene product from E. coli (Rahav-Manor, O., et al. 1992. NhaR, a protein homologous to a family of bacterial regulatory proteins (LysR), regulates nhaA, the sodium proton antiporter gene in Escherichia coli. J. Biol. Chem. 267:10433-10438); *ompR* (cry, envZ, ompB) (Taylor, R. K., et al. Identification of OmpR: a positive regulatory protein controlling expression of the major outer membrane matrix porin proteins of Escherichia coli K-12. J. Bacteriol. 147:255-258); *oxyR* (*mor, momR*) (VanBogelen, R. A, et al. 1987. Differential induction of heat shock, SOS, and oxidation stress regulons and accumulation of nucleotides in Escherichia coli. J. Bacteriol. 169:26-32); *pdhR* (Haydon, D. J., et al. A mutation causing constitutive synthesis of the pyruvate dehydrogenase complex in Escherichia coli is located within the pdhR gene. FEBS Lett. 336:43-47); *phnF* (Wanner, B. L., and W. W. Metcalf. 1992. Molecular genetic studies of a 10.9-kb operon in Escherichia coli for phosphonate uptake and biodegradation. FEMS Microbiol. Lett. 79:133-139); *phoB* (*phoRc, phoT*) (Pratt, C. 1980. Kinetics and regulation of cell-free alkaline phosphatase synthesis. J. Bacteriol. 143:1265-1274); phoP (Kasahara, M., et al. 1992. Molecular analysis of the Escherichia coli phoP-phoQ operon. J. Bacteriol. 174:492-498); phoQ (Id.); *phoR* (*R1pho, nmpB, phoR1)* (Bracha, M., and E. Yagil. 1969. Genetic mapping of the phoR regulator gene of alkaline phosphatase in Escherichia coli. J. Gen. Microbiol. 59:77-81); *phoU* (*phoT*) (Nakata, A., et al. 1984. Regulation of the phosphate regulon in Escherichia coli K-12: regulation of the negative regulatory gene phoU and identification of the gene product. J. Bacteriol. 159:979-985); *poaR* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *poxA* (Chang, Y. Y., and J. E. Cronan Jr. 1982. Mapping nonselectable genes of Escherichia coli by using transposon Tn10: location of a gene affecting pyruvate oxidase. J. Bacteriol. 151:1279-1289); *proQ* (Milner, J. L., and J. M. Wood. 1989. Insertion proQ220::Tn5 alters regulation of proline porter II, a transporter of proline and glycine betaine in Escherichia coli. J. Bacteriol. 171:947-951); *pspA* (Weiner, L., et al. 1991. Stress-induced expression of the Escherichia coli phage shock protein operon is dependent on sigma 54 and modulated by positive and negative feedback mechanisms. Genes Dev. 5:1912-1923); *pspB* (Weiner, L., et al. 1991. Stress-induced expression of the Escherichia coli phage shock protein operon is dependent on sigma 54 and modulated by positive and negative feedback mechanisms. Genes Dev. 5:1912-1923); pspC (Weiner, L., et al. 1991. Stress-induced expression of the Escherichia coli phage shock protein operon is dependent on sigma 54 and modulated by positive and negative feedback mechanisms. Genes Dev. 5:1912-1923); pssR (Sparrow, C. P., and C. R. Raetz. 1983. A trans-acting regulatory mutation that causes overproduction of phosphatidylserine synthase in Escherichia coli. J. Biol. Chem. 258:9963-9967); *purR* (Meng, L. M., et al. 1990. Autoregulation of PurR repressor synthesis and involvement of purR in the regulation of purB, purC, purL, purMN and guaBA expression in Escherichia coli. Eur. J. Biochem. 187:373-379); *putA* (*poaA*) gene product from Salmonella enterica serotype Typhimurium (Menzel, R., and J. Roth. 1981. Regulation of the genes for proline utilization in Salmonella typhimurium: autogenous repression by the putA gene product. J. Mol. Biol. 148:21-44); *pyrl* (Cunin, R., et al. 1985. Structure-function relationship in allosteric aspartate carbamoyltransferase from Escherichia coli. I. Primary structure of a pyrl gene encoding a modified regulatory subunit. J. Mol. Biol. 186:707-713); *rbsR* (Lopilato, J. E., et al. 1984. D-ribose metabolism in Escherichia coli K-12: genetics, regulation, and transport. J. Bacteriol. 158:665-673); rcsA (Gottesman, S., et al. 1985. Regulation of capsular polysaccharide synthesis in Escherichia coli K-12: characterization of three regulatory genes. J. Bacteriol. 162:1111-1119); *rcsB*(*Id*.); *rcsC* (*Id*.); *rcsF* (Grevais, F. G., and G. R. Drapeau. 1992. Identification, cloning, and characterization of rcsF, a new regulator gene for exopolysaccharide synthesis that suppresses the division mutation ftsZ84 in Escherichia coli K-12. J. Bacteriol. 174:8016-8022); *relB* (Christensen, S. K., et al. 2001. RelE, a global inhibitor of translation, is activated during nutritional stress. Proc Natl. Acad. Sci. 98:14328-14333); *rfaH* (*sfrB*) (Pradel, E., and C. A. Schnaitman. 1991. Effect of rfaH (sfrB) and temperature on expression of rfa genes of Escherichia coli K-12. J. Bacteriol. 173:6428-6431); *rhaR* (Tobin, J. F., and R. F. Schleif. 1987. Positive regulation of the Escherichia coli L-rhamnose operon is mediated by the products of tandemly repeated regulatory genes. J. Mol. Biol. 196:789-799); *rhaS* (*Id*.); *mk* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *rob* (Skarstad, K., et al. A novel binding protein of the origin of the Escherichia coli chromosome. J. Biol. Chem. 268:535-5370); *rseA* (*mclA*) (Missiakas, D., et al. 1997. Modulation of the Escherichia coli sigmaE (RpoE) heat-shock transcription-factor activity by the RseA, RseB and RseC proteins. Mol. Microbiol. 24:355-371; De Las Penas, A. 1997. The sigmaE- mediated response to extracytoplasmic stress in Escherichia coli is transduced by RseA and RseB, two negative regulators of sigmaE. Mol. Microbiol. 24:373-385); *rseB* (*Id*.); *rseC* (*Id*.); *rspA* (Huisman, G. W., and T. Kolter. 1994. Sensing starvation: a homoserine lactone--dependent signaling pathway in Escherichia coli. Science. 265:537-539); *rspB* (Shafqat, J., et al. An ethanol-inducible MDR ethanol dehydrogenase/acetaldehyde reductase in Escherichia coli: structural and enzymatic relationships to the eukaryotic protein forms. Eur. J. Biochem. 263:305-311); *rssA* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *rssB* (Muffler, A., et al. 1996. The response regulator RssB controls stability of the sigma(S) subunit of RNA polymerase in Escherichia coli. EMBO J. 15:1333-1339); *sbaA* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *sdaC* (*Id*.); *sdiA* (Sitnikov, D. M., et al. 1996. Control of cell division in Escherichia coli: regulation of transcription of I involves both rpoS and SdiA- mediated autoinduction. Proc. Natl. Acad. Sci. 93:336-341); *serR* (Theall, G., et al. 1979. Regulation of the biosynthesis of aminoacyl-tRNA synthetases and of tRNA in Escherichia coli. IV. Mutants with increased levels of leucyl- or seryl-tRNA synthetase. Mol. Gen. Genet. 169:205-211); *sfsA* (Takeda, K., et al. 2001. Effects of the Escherichia coli sfsA gene on mal genes expression and a DNA binding activity of SfsA. Biosci. Biotechnol. Biochem. 65:213-217); *sfsB* (*nlp, sfs1*) (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *soxR* (Tsaneva, I. R., and B. Weiss. 1990. soxR, a locus governing a superoxide response regulon in Escherichia coli K-12. J. Bacteriol. 172:4197-4205); *soxS* (Wu, J., and B. Weiss. 1991. Two divergently transcribed genes, soxR and soxS, control a superoxide response regulon of Escherichia coli. J. Bacteriol. 173:2864-2871); srlR (gutR) (Csonka, L. N., and A. J. Clark. 1979. Deletions generated by the transposon Tn10 in the srl recA region of the Escherichia coli K-12 chromosome. Genetics. 93:321-343); *tdcA* (Ganduri, Y. L., et al. 1993. TdcA, a transcriptional activator of the tdcABC operon of Escherichia coli, is a member of the LysR family of proteins. Mol. Gen. Genet. 240:395-402); *tdcR* (Hagewood, B. T., et al. 1994. Functional analysis of the tdcABC promoter of Escherichia coli: roles of TdcA and TdcR. J. Bacteriol. 176:6241-6220); thrS (Springer, M., et al. 1985. Autogenous control of Escherichia coli threonyl-tRNA synthetase expression in vivo. J. Mol. Biol. 185:93-104); *torR* (Simon, G., et al. 1994. The torR gene of Escherichia coli encodes a response regulator protein involved in the expression of the trimethylamine N-oxide reductase genes. J. Bacteriol. 176:5601-5606); treR (Horlacher, R., and W. Boos. 1997. Characterization of TreR, the major regulator of the Escherichia coli trehalose system. J. Biol. Chem. 272:13026-13032); *trpR* (Gunsalus, R. P., and C. Yanofsky. 1980. Nucleotide sequence and expression of Escherichia coli trpR, the structural gene for the trp aporepressor. Proc. Natl. Acad. Sci. 77:7117-7121); *tyrR* (Camakaris, H., and J. Pittard. 1973. Regulation of tyrosine and phenylalanine biosynthesis in Escherichia coli K-12: properties of the tyrR gene product. J. Bacteriol. 115:1135-1144); *uhpA* (Kadner, R. J., and D. M. Shattuck-Eidens. 1983. Genetic control of the hexose phosphate transport system of Escherichia coli: mapping of deletion and insertion mutations in the uhp region. J. Bacteriol. 155:1052-1061); *uidR* (*gusR*) (Novel, M., and G. Novel. 1976. Regulation of beta-glucuronidase synthesis in Escherichia coli K-12: pleiotropic constitutive mutations affecting uxu and uidA expression. J. Bacteriol. 127:418-432); *uspA* (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); *uxuR* (Novel, M., and G. Novel. 1976. Regulation of beta-glucuronidase synthesis in Escherichia coli K-12: pleiotropic constitutive mutations affecting uxu and uidA expression. J. Bacteriol. 127:418-432); *wrbA* (Yang, W., et al. 1993. A stationary-phase protein of Escherichia coli that affects the mode of association between the trp repressor protein and operator-bearing DNA. Proc. Natl. Acad. Sci. 90:5796-5800); *xapR* (*pndR*) (Seeger, C., et al. 1995. Identification and characterization of genes (xapA, xapB, and xapR) involved in xanthosine catabolism in Escherichia coli. J. Bacteriol. 177:5506-5516); and *xylR* (Inouye, S., et al. 1987. Expression of the regulatory gene xylS on the TOL plasmid is positively controlled by the xylR gene product. Proc. Natl. Acad. Sci. 84:5182-5186);

### II.C.1.b. Bacillus subtilis

Regulatory elements, promoters and other expression elements and expression elements from *B subtilis* include but are not limited to *abrB* (Perego, M., et al. 1988. Structure of the gene for the transition state regulator, abrB: regulator synthesis is controlled by the spo0A sporulation gene in Bacillus subtilis. Mol. Microbiol. 2:698-699); acoR (Ali, N. O., et al. 2001. Regulation of the acetoin catabolic pathway is controlled by sigma L in Bacillus subtilis. J. Bacteriol. 183:2497-2504); *ahrC* (Klinger, U., et al. 1995. A binding site for activation by the Bacillus subtilis AhrC protein, a repressor/activator of arginine metabolism. Mol. Gen. Genet. 248:329-340); *alaR* (Sohenshein, A. L., J. A. Hoch, and R. Losick (eds.) 2002. Bacillus subtilis and its closest relatives: from genes to cells. American Society for Microbiology, Washington D. C.); *alsR* (Renna, M. C., et al. 1993. Regulation of the Bacillus subtilis alsS, alsD, and alsR genes involved in post-exponential-phase production of acetoin. J. Bacteriol. 175:3863-3875); *ansR* (Sun, D., and P. Setlow. 1993. Cloning and nucleotide sequence of the Bacillus subtilis ansR gene, which encodes a repressor of the ans operon coding for L-asparaginase and L-aspartase. J. Bacteriol. 175:2501-2506); *araR* (Sa-Nogueira, I., and L. J. Mota. 1997. Negative regulation of L-arabinose metabolism in Bacillus subtilis: characterization of the araR (araC) gene. J. Bacteriol. 179:1598-1608); *arfM* (Marino, M., et al. 2001. Modulation of anaerobic energy metabolism of Bacillus subtilis by arfM (ywiD). J. Bacteriol. 183:6815-6821); arsR (Rosenstein, R., et al. 1992. Expression and regulation of the antimonite, arsenite, and arsenate resistance operon of Staphylococcus xylosus plasmid pSX267. J. Bacteriol. 174:3676-3683); *azlB* (Belitsky, B. R., et al. 1997. An Irp-like gene of Bacillus subtilis involved in branched-chain amino acid transport. J. Bacteriol. 179:54485457); *birA* (Bower, S., et al. 1995. Cloning and characterization of the Bacillus subtilis birA gene encoding a repressor of the biotin operon. J. Bacteriol. 177:2572-2575); *bkdR* (Bebarbouille, M., et al. 1999. Role of bkdR, a transcriptional activator of the sigL-dependent isoleucine and valine degradation pathway in Bacillus subtilis. J. Bacteriol. 181:2059-2066); *bltR* (Ahmed, M., et al. 1995. Two highly similar multidrug transporters of Bacillus subtilis whose expression is differentially regulated. J. Bacteriol. 177:3904-3910); *bmrR* (Ahmed, M., et al. 1994. A protein that activates expression of a multidrug efflux transporter upon binding the transporter substrates. J. Biol. Chem. 269:28506-28513); *ccpA* (Henkin, T. M., et al. 1991. Catabolite repression of alpha-amylase gene expression in Bacillus subtilis involves a trans-acting gene product homologous to the Escherichia coli lacl and galR repressors. Mol. Microbiol. 5:575-584); *ccpB* (Chauvaux, S., et al. 1998. CcpB, a novel transcription factor implicated in catabolite repression in Bacillus subtilis. J. Bacteriol. 180:491-497); *ccpC* (Jourlin-Castelli, C., et al. 2000. CcpC, a novel regulator of the LysR family required for glucose repression of the citB gene in Bacillus subtilis. J. Mol. Biol. 295:865-878); *cggR* (Fillinger, S., et al. 2000. Two glyceraldehyde-3-phosphate dehydrogenases with opposite physiological roles in a nonphotosynthetic bacterium. J. Biol. Chem. 275:14031-14037); *cheB* (Bischoff, D. S., and G. W. Ordal. 1991. Sequence and characterization of Bacillus subtilis CheB, a homolog of Escherichia coli CheY, and its role in a different mechanism of chemotaxis. J. Biol. Chem. 266:12301-12305); *cheY* (Bischoff, D. S., et al. 1993. Purification and characterization of Bacillus subtilis CheY. Biochemistry 32:9256-9261); *citR* (Jin, S., and A. L. Sonenshein. 1994. Transcriptional regulation of Bacillus subtilis citrate synthase genes. J. Bacteriol. 176:4680-4690); *citT* (Yamamoto, H., et al. 2000. The CitST two-component system regulates the expression of the Mg-citrate transporter in Bacillus subtilis. Mol. Microbiol. 37:898-912); *codY* (Slack, F. J., et al. 1995. A gene required for nutritional repression of the Bacillus subtilis dipeptide permease operon. Mol. Microbiol. 15:689-702); *comA* (Nakano, M. M., and P. Zuber. 1989. Cloning and characterization of srfB, a regulatory gene involved in surfactin production and competence in Bacillus subtilis. J. Bacteriol. 171:5347-5353); *comK* (Msadek, T., et al. 1994. MecB of Bacillus subtilis, a member of the CIpC ATPase family, is a pleiotropic regulator controlling competence gene expression and growth at high temperature. Proc. Natl. Acad. Sci. 91:5788-5792); *comQ* (Weinrauch, Y., et al. 1991. Sequence and properties of comQ, a new competence regulatory gene of Bacillus subtilis. J. Bacteriol. 173:5685-5693); *cssR* (Hyyrylainen, H. L., et al. 2001. A novel two-component regulatory system in Bacillus subtilis for the survival of severe secretion stress. Mol. Microbiol. 41:1159-1172); *ctsR* (Kruger, E., and M. Hecker. 1998. The first gene of the Bacillus subtilis clpC operon, ctsR, encodes a negative regulator of its own operon and other class III heat shock genes. J. Bacteriol. 180:6681-6688); dctR (Sohenshein, A. L., J. A. Hoch, and R. Losick (eds.) 2002. Bacillus subtilis and its closest relatives: from genes to cells. American Society for Microbiology, Washington D. C.); *degA* (Bussey, L. B., and R. L. Switzer. 1993. The degA gene product accelerates degradation of Bacillus subtilis phosphoribosylpyrophosphate amidotransferase in Escherichia coli. J. Bacteriol. 175:6348-6353); *degU* (Msadek, T., et al. 1990. Signal transduction pathway controlling synthesis of a class of degradative enzymes in Bacillus subtilis: expression of the regulatory genes and analysis of mutations in degS and degU. J. Bacteriol. 172:824-834); *deoR* (Saxild, H. H., et al. 1996. Dra-nupC-pdp operon of Bacillus subtilis: nucleotide sequence, induction by deoxyribonucleosides, and transcriptional regulation by the deoR- encoded DeoR repressor protein. J. Bacteriol. 178:424-434); *exuR* (Sohenshein, A. L., J. A. Hoch, and R. Losick (eds.) 2002. Bacillus subtilis and its closest relatives: from genes to cells. American Society for Microbiology, Washington D. C.); *frn* (Cruz Ramos, H., et al. 1995. Anaerobic transcription activation in Bacillus subtilis: identification of distinct FNR-dependent and -independent regulatory mechanisms. EMBO J. 14:5984-5994); *fruR* (Saier, M. H. Jr. 1996. Cyclic AMP-independent catabolite repression in bacteria. FEMS Microbiol. Lett. 138:97-103); *fur* (Chen, L., et al. 1993. Metalloregulation in Bacillus subtilis: isolation and characterization of two genes differentially repressed by metal ions. J. Bacteriol. 175:5428-5437); gabR (Sohenshein, A. L., J. A. Hoch, and R. Losick (eds.) 2002. Bacillus subtilis and its closest relatives: from genes to cells. American Society for Microbiology, Washington D. C.); *gerE* (Holand, S. K., et al. 1987. The possible DNA-binding nature of the regulatory proteins, encoded by spoIID and gerE, involved in the sporulation of Bacillus subtilis. J. Gen. Microbiol. 133:2381-2391); glcR (Stulke, J., et al. 2001. Characterization of glucose-repression-resistant mutants of Bacillus subtilis: identification of the glcR gene. Arch. Microbiol. 175:441-449); *glcT* (Paulsen, I. T., et al. 1998. Characterization of glucose-specific catabolite repression-resistant mutants of Bacillus subtilis: identification of a novel hexose:H+ symporter. J. Bacteriol. 180:498-504); *glnR* (Schreier, H. J., et al. 1989. Regulation of Bacillus subtilis glutamine synthetase gene expression by the product of the glnR gene. J. Mol. Biol. 210:51-63); *glpP* (Holmberg, C., and B. Rutberg. 1991. Expression of the gene encoding glycerol-3-phosphate dehydrogenase (glpD) in Bacillus subtilis is controlled by antitermination. Mol. Microbiol. 5:2891-2900); *gltC* (Bohannon, D. E. and A. L. Sonenshein. 1989. Positive regulation of glutamate biosynthesis in Bacillus subtilis. J. Bacteriol. 171:4718-4727); *gltR* (Belitsky, B. R., and A. L. Sonenshein. 1997. Altered transcription activation specificity of a mutant form of Bacillus subtilis GltR, a LysR family member. J. Bacteriol. 179:1035-1043); *gntR* (Fujita, Y., and T. Fujita. 1987. The gluconate operon gnt of Bacillus subtilis encodes its own transcriptional negative regulator. Proc. Natl. Acad. Sci. 84:4524-4528); *gutR* (Ye, R., et al. 1994. Glucitol induction in Bacillus subtilis is mediated by a regulatory factor, GutR. J. Bacteriol. 176:3321-3327); *hpr* (Perego, M., and J. A. Hoch. 1988. Sequence analysis and regulation of the hpr locus, a regulatory gene for protease production and sporulation in Bacillus subtilis. J. Bacteriol. 170:2560-2567); *hrcA* (Schulz, A., and W. Schumann. 1996. hrcA, the first gene of the Bacillus subtilis dnaK operon encodes a negative regulator of class I heat shock genes. J. Bacteriol. 178:1088-1093); hutP (Oda, M., et al. 1992. Analysis of the transcriptional activity of the hut promoter in Bacillus subtilis and identification of a cis-acting regulatory region associated with catabolite repression downstream from the site of transcription. Mol. Microbiol. 6:2573-2582); *hxlR* (Sohenshein, A. L., J. A. Hoch, and R. Losick (eds.) 2002. Bacillus subtilis and its closest relatives: from genes to cells. American Society for Microbiology, Washington D. C.); *iolR* (Yoshida, K. I., et al. 1999. Interaction of a repressor and its binding sites for regulation of the Bacillus subtilis iol divergon. J. Mol. Biol. 285:917-929); kdgR (Pujic, P., et al. 1998. The kdgRKAT operon of Bacillus subtilis: detection of the transcript and regulation by the kdgR and ccpA genes. Microbiology. 144:3111-3118); *kipR* (Sohenshein, A. L., J. A. Hoch, and R. Losick (eds.) 2002. Bacillus subtilis and its closest relatives: from genes to cells. American Society for Microbiology, Washington D. C.); *lacR* (Errington, J., and C. H. Vogt. 1990. Isolation and characterization of mutations in the gene encoding an endogenous Bacillus subtilis beta-galactosidase and its regulator. J. Bacteriol. 172:488-490); *levR* (Bebarbouille, M., et al. 1991. The transcriptional regulator LevR of Bacillus subtilis has domains homologous to both sigma 54- and phosphotransferase system-dependent regulators. Proc. natl. Acad. Sci. 88:2212-2216); *lexA* (Lovett, C. M. Jr., and J. W. Roberts. 1985. Purification of a RecA protein analogue from Bacillus subtilis. J. Biol. Chem. 260:3305-3313); *licR* (Tobisch, S., et a. 1997. Identification and characterization of a new beta-glucoside utilization system in Bacillus subtilis. J. Bacteriol. 179:496-506); *licT* (Le Coq, D., et al. 1995. New beta-glucoside (bgl) genes in Bacillus subtilis: the bglP gene product has both transport and regulatory functions similar to those of BglF, its Escherichia coli homolog. J. Bacteriol. 177:1527-1535); *ImrA* (Kumano, M., et al. 1997. A 32 kb nucleotide sequence from the region of the lincomycin-resistance gene (22 degrees-25 degrees) of the Bacillus subtilis chromosome and identification of the site of the lin-2 mutation. Microbiology. 143:2775-2782); *IrpA* gene product from *Pyrococcus furiosus* (Brinkman, A. B., et al. 2000. An Lrp-like transcriptional regulator from the archaeon Pyrococcus furiosus is negatively autoregulated. J. Biol. Chem. 275:38160-38169); *lrpB* (Sohenshein, A. L., J. A. Hoch, and R. Losick (eds.) 2002. Bacillus subtilis and its closest relatives: from genes to cells. American Society for Microbiology, Washington D. C.); *lrpC* (Beloin, C., et al. 1997. Characterization of an lrp-like (lrpC) gene from Bacillus subtilis. Mol. Gen. Genet. 256:63-71); *lytr* (Huang, X., and J. D. Helmann. 1998. Identification of target promoters for the Bacillus subtilis sigma X factor using a consensus-directed search. J. Mol. Biol. 279:165-173); lytT (Sohenshein, A. L., J. A. Hoch, and R. Losick (eds.) 2002. Bacillus subtilis and its closest relatives: from genes to cells. American Society for Microbiology, Washington D. C.); *manR* gene product from *Listeria monocytogenes* (Dalet, K., et al. 2001. A sigma(54)-dependent PTS permease of the mannose family is responsible for sensitivity of Listeria monocytogenes to mesentericin Y105. Microbiology. 147:3263-3269); *mntR* (Que, Q., and J. D. Helmann. 2000. Manganese homeostasis in Bacillus subtilis is regulated by MntR, a bifunctional regulator related to the diphtheria toxin repressor family of proteins. Mol. Microbiol. 35:1454-1468); *msmR* gene product from *Streptococcus mutans* (Russell, R. R., et al. 1992. A binding protein-dependent transport system in Streptococcus mutans responsible for multiple sugar metabolism. J. Biol. Chem. 267:4631-4637); *mta* (Baranova, N. N., et al. 1999. Mta, a global MerR-type regulator of the Bacillus subtilis multidrug-efflux transporters. Mol. Microbiol. 31:1549-1559); *mtlR* (Henstra, S. A., et al. 1999. The Bacillus stearothermophilus mannitol regulator, MtlR, of the phosphotransferase system. A DNA-binding protein, regulated by HPr and iicbmti-dependent phosphorylation. J. Biol. Chem. 274:4754-4763); *mtrB* (Gollnick, P., et al. 1990. The mtr locus is a two-gene operon required for transcription attenuation in the trp operon of Bacillus subtilis. Proc. Natl. Acad. Sci. 87:8726-8730); *nhaX* (Sohenshein, A. L., J. A. Hoch, and R. Losick (eds.) 2002. Bacillus subtilis and its closest relatives: from genes to cells. American Society for Microbiology, Washington D. C.); toxR gene product from *Vibrio cholerae* (Miller, V. L., and J. J. Mekalanos. 1984. Synthesis of cholera toxin is positively regulated at the transcriptional level by toxR. Proc. Natl. Acad. Sci. 81:3471-3475); *padR* gene product from *Pediococcus pentosaceus* (Barthelmebs, L., et al. 2000. Inducible metabolism of phenolic acids in Pediococcus pentosaceus is encoded by an autoregulated operon which involves a new class of negative transcriptional regulator. J. Bacteriol. 182:6724-6731); *paiA* (Sohenshein, A. L., J. A. Hoch, and R. Losick (eds.) 2002. Bacillus subtilis and its closest relatives: from genes to cells. American Society for Microbiology, Washington D. C.); *paiB* (*Id*.); perA (*Id*.); *phoP* (Birkey, S. M., et al. 1994. A pho regulon promoter induced under sporulation conditions. Gene. 147:95-100); pksA (Sohenshein, A. L., J. A. Hoch, and R. Losick (eds.) 2002. Bacillus subtilis and its closest relatives: from genes to cells. American Society for Microbiology, Washington D. C.); *pucR* (Schultz, A. C., et al. 2001. Functional analysis of 14 genes that constitute the purine catabolic pathway in Bacillus subtilis and evidence for a novel regulon controlled by the PucR transcription activator. J. Bacteriol. 183:3293-3302); *purR* (Weng, M., et al. 1995. Identification of the Bacillus subtilis pur operon repressor. Proc. Natl. Acad. Sci. 92:7455-7459); *pyrR* (Martinussen, J., et al. 1995. Two genes encoding uracil phosphoribosyltransferase are present in Bacillus subtilis. J. Bacteriol. 177:271-274); *rbsR* (Rodionov, D. A., et al. 2001. Transcriptional regulation of pentose utilisation systems in the Bacillus/Clostridium group of bacteria. FEMS Microbiol. Lett. 205:305-314); *resD* (Suin, G., et al. 1996. Regulators of aerobic and anaerobic respiration in Bacillus subtilis. J. Bacteriol. 178:1374-1385); *rocR* (Gardan, R., et al. 1997. Role of the transcriptional activator RocR in the arginine-degradation pathway of Bacillus subtilis. Mol. Microbiol. 24:825-837); *rsiX* (Tortosa, P., et al. 2000. Characterization of ylbf, a new gene involved in competence development and sporulation in Bacillus subtilis. Mol. Microbiol. 35:1110-1119); *sacT* (Debarbouille, M., et al. 1990. The sacT gene regulating the sacPA operon in Bacillus subtilis shares strong homology with transcriptional antiterminators. J. Bacteriol. 172:3966-3973); *sacV* (Wong, S. L., et al. 1988. Cloning and nucleotide sequence of senN, a novel 'Bacillus natto' (B. subtilis) gene that regulates expression of extracellular protein genes. J. Gen. Microbiol. 134:3269-3276); *sacY* (Steinmetz, M., et al 1989. Induction of saccharolytic enzymes by sucrose in Bacillus subtilis: evidence for two partially interchangeable regulatory pathways. J. Bacteriol. 171:1519-1523); *senS* (Wang, L. F., and R. H. Dori. 1990. Complex character of senS, a novel gene regulating expression of extracellular-protein genes of Bacillus subtilis. J. Bacteriol. 172:1939-1947); *sinR* (Bai, U., et al. 1993. SinI modulates the activity of SinR, a developmental switch protein of Bacillus subtilis, by protein-protein interaction. Genes Dev. 7:139-148); *slr* (Asayama, M., et al. 1998. Translational attenuation of the Bacillus subtilis spo0B cistron by an RNA structure encompassing the initiation region. Nucleic Acids Res. 26:824-830); *splA* (Fajardo-Cavazos, P., and W. L. Nicholson. 2000. The TRAP-like SplA protein is a trans-acting negative regulator of spore photoproduct lyase synthesis during Bacillus subtilis sporulation. J. Bacteriol. 182:555-560); *spo0A* (Smith, I., et al. 1991. The role of negative control in sporulation. Res. Microbiol. 142:831-839); *spo0F* (Lewandoski, M., et al. 1986. Transcriptional regulation of the spo0F gene of Bacillus subtilis. J. Bacteriol. 168:870-877); *spoIIID* (Kunkel, B., et al. 1989. Temporal and spatial control of the mother-cell regulatory gene spoIIID of Bacillus subtilis. Genes. Dev. 3:1735-1744); *spoVT* (Bagyan, I, et al. 1996. A compartmentalized regulator of developmental gene expression in Bacillus subtilis. J. Bacteriol. 178:4500-4507); *tenA* (Pang, A. S., et al. 1991. Cloning and characterization of a pair of novel genes that regulate production of extracellular enzymes in Bacillus subtilis. J. Bacteriol. 173:46-54); *ten1* (Sohenshein, A. L., J. A. Hoch, and R. Losick (eds.) 2002. Bacillus subtilis and its closest relatives: from genes to cells. American Society for Microbiology, Washington D. C.); *tnrA* (Wray, L. V., Jr., et al. 1996. TnrA, a transcription factor required for global nitrogen regulation in Bacillus subtilis. Proc. Natl. Acad. Sci. 93:8841-8845); *treR* (Schock, F., and M. K. Dahl. 1996. Expression of the tre operon of Bacillus subtilis 168 is regulated by the repressor TreR. J. Bacteriol. 178:4576-4581); *xre* (McDonnell, G. E., et al. 1994. Genetic control of bacterial suicide: regulation of the induction of PBSX in Bacillus subtilis. J. Bacteriol. 176:5820-5830); *xylR* gene product from *Bacillus megaterium* (Rygus, T., et al. 1991. Molecular cloning, structure, promoters and regulatory elements for transcription of the Bacillus megaterium encoded regulon for xylose utilization. Arch. Microbiol. 155:535:542); *yacF* (Sohenshein, A. L., J. A. Hoch, and R. Losick (eds.) 2002. Bacillus subtilis and its closest relatives: from genes to cells. American Society for Microbiology, Washington D. C.); and *zur* (Gaballa, A., and J. D. Helmann. 1998. Identification of a zinc-specific metalloregulatory protein, Zur, controlling zinc transport operons in Bacillus subtilis. J. Bacteriol. 180:5815-5821).

### II.C.1.c. Bacteriophage and Transposable Elements

Regulatory elements, promoters and other expression elements from bacteriophage and transposable elements include without limitation *cl* gene product from bacteriophage lambda mation and/or segregated minicells (Reichardt, L. F. 1975. Control of bacteriophage lambda repressor synthesis: regulation of the maintenance pathway of the cro and cl products. J. Mol. Biol. 93:289-309); (Love, C. A., et al. 1996. Stable high-copy-number bacteriophage lambda promoter vectors for overproduction of proteins in Escherichia coli. Gene. 176:49-53); the c2 gene product from bacteriophage P22 (Gough, M., and S. Tokuno. 1975. Further structural and functional analogies between the repressor regions of phages P22 and lambda. Mol. Gen. Genet. 138:71-79); the cro gene from bacteriophage lambda (Reichardt, L. F. 1975. Control of bacteriophage lambda repressor synthesis: regulation of the maintenance pathway of the cro and cl products. J. Mol. Biol. 93:289-309); the *ant* gene from bacteriophage P22 (Youderian, P. et al. 1982. Sequence determinants of promotor activity. Cell. 30:843-853); the *mnt* gene from bacteriophage P22 (Gough, M. 1970. Requirement for a functional int product in temperature inductions of prophage P22 ts mnt. J. Virol. 6:320-325; Prell, H. H. 1978. Ant-mediated transactivation of early genes in Salmonella prophage P22 by superinfecting virulent P22 mutants. Mol. Gen. Genet. 164:331-334); the *tetR* gene product from the TetR family of bacterial regulators or homologues of this gene or gene product found in Tn10 and other members of the bacteriophage, animal virus, Eubacteria, Eucarya or Archaea may be employed to increase the efficiency of gene expression and protein production in parent cells prior to minicell formation and/or segregated minicells (Moyed, H. S., and K. P. Bertrand. 1983. Mutations in multicopy Tn10 tet plasmids that confer resistance to inhibitory effects of inducers of tet gene expression. J. Bacteriol. 155:557-564); the *mnt* gene product from bacteriophage SP6 mation and/or segregated minicells (Mead, D. A., et al. 1985. Single stranded DNA SP6 promoter plasmids for engineering mutant RNAs and proteins: synthesis of a 'stretched' preproparathyroid hormone. Nucleic Acids Res. 13:1103-1118); and the *mnt* gene product from bacteriophage T7 mation and/or segregated minicells (Steen, R., et al. 1986. T7 RNA polymerase directed expression of the Escherichia coli rrnB operon. EMBO J. 5:1099-1103).

### II.C.1.d. Use of Site-Specific Recombination in Expression Systems

Included in the design of the invention are techniques that increase the efficiency of gene expression and protein production in minicells. By way of non-limiting example, these techniques may include modification of endogenous and/or exogenous regulatory elements responsible for activation and/or repression of proteins to be expressed from chromosomal and/or plasmid expression vectors. By way of non-limiting example, this system may be applied to any of the above regulatory elements/systems. Specifically, each of the above mentioned regulatory systems may be constructed such that the promotor regions are oriented in a direction away from the gene to be expressed, or each of the above mentioned gene(s) to be expressed may be constructed such that the gene(s) to be expressed is oriented in a direction away from the regulatory region promotor. Constructed in this system is a methodology dependent upon site-specific genetic recombination for inversion and induction of the gene of interest (Backman, K., et al. 1984. Use of synchronous site-specific recombination in vivo to regulate gene expression. Bio/Technology 2:1045-1049; Balakrishnan, R., et al. 1994. A gene cassette for adapting Escherichia coli strains as hosts for att-Int-mediated rearrangement and pL expression vectors. Gene 138:101-104; Hasan, N., and W. Szybalaki. 1987. Control of cloned gene expression by promoter inversion in vivo: construction of improved vectors with a multiple cloning site and the Ptac promotor. Gene 56:145-151; Wulfing, C., and A. Pluckthun. 1993. A versatile and highly repressible Escherichia coli expression system based on invertible promoters: expression of a gene encoding a toxic gene product. Gene 136:199-203). These invertible promoters and/or gene regions will allow tight regulation of potentially toxic protein products. By way of non-limiting example, these systems may be derived from bacteriophage lambda, bacteriophage Mu, and/or bacteriophage P22. In any of these potential systems, regulation of the recombinase may be regulated by any of the regulatory systems discussed in section II.C.5 and elsewhere herein.

### II.C.1.e. Use of Copy Number Control Switches

A method that can be used to increase the efficiency of gene expression and protein production in minicells involves the modification of endogenous and/or introduction of exogenous genetic expression systems such that the number of copies of a gene encoding a protein to be expressed can be modulated. Copy number control systems comprise elements designed to modulate copy number in a controlled fashion.

In an exemplary mode, copy number is controlled to decrease the effects of "leaky' (uninduced) expression of toxic gene products. This allows one to maintain the integrity of a potentially toxic gene product during processes such as cloning, culture maintenance, and periods of growth prior to minicell-induction. That is, decreasing the copy number of a gene is expected to decrease the opportunity for mutations affecting protein expression and/or function to arise. Immediately prior to, during and/or after minicell formation, the copy number may be increased to optimize the gene dosage in minicells as desired.

The replication of eubacterial plasmids is regulated by a number of factors, some of which are contained within the plasmid, others of which are located on the chromosome. For reviews, see del Solar, G., et al. 2000. Plasmid copy number control: an ever-growing story. Mol Microbiol. 37:492-500; del Solar, G., et al. 1998. Replication and control of circular bacterial plasmids. Microbiol Mol Biol Rev. 62:434-64; and Filutowicz, M., et al. 1987. DNA and protein interactions in the regulation of plasmid replication. J Cell Sci Suppl. 7:15-31.

By way of non-limiting example, the pcnB gene product, the wildtype form of which promotes increased ColE1 plasmid copy number (Soderbom, F., et al. 1997. Regulation of plasmid R1 replication: PcnB and RNase E expedite the decay of the antisense RNA, CopA. Mol. Microbiol. 26:493-504), is modulated; and/or mutant forms of the pcnB gene are introduced into a cell. In an exemplary cell type that may be used in the methods of the invention, the wildtype pcnB chromosomal gene is replaced with a mutant pcnB80 allele (Lopilato, J., et al. 1986. Mutations in a new chromosomal gene of Escherichia coli K-12, pcnB, reduce plasmid copy number of pBR322 and its derivatives. Mol. Gen. Genet. 205:285-290). In such cells the copy number of a ColE1-derived plasmid is decreased. The cell may further comprise an expression element comprising an inducible promoter operably linked to an ORF encoding the wild-type pcnB. Because the wild-type pcnB gene is dominant to the mutant pcnB80 gene, and because the wild-type pcnB gene product promotes increased ColE1 plasmid copy number, induction of a wild-type pcnB in the pcnB80 background will increase the plasmid copy number of ColE1-derived plasmids. Such copy number control systems may be expressed from the chromosome and/or plasmid to maintain either low or high plasmid copy number in the absence of induction. Other non-limiting examples of gene and/or gene products that may be employed in copy number control systems for ColE1-based replicons include genes or homologs of genes encoding RNA I, RNA II, rop, RNAse H, enzymes involved in the process of polyadenylation, RNAse E, DNA polymerase I, and DNA polymerase III.

In the case of IncFII-derived replicons, non-limiting examples of gene and/or gene products that may be employed in copy number control systems to control plasmid copy include genes or homologs of the copA, copB, repA, and repB genes. Copy number control systems may additionally or alternatively include manipulation of repC, trfA, dnaA, dnaB, dnaC, seqA, genes protein Pi, genes encoding HU protein subunits (hupA, hupB) and genes encoding IHF subunits.

Other elements may also be included to optimize these plasmid copy number control systems. Such additional elements may include the addition or deletion of iteron nucleic acid sequences (Chattoraj, D. K. 2000. Control of plasmid DNA replication by iterons: no longer paradoxical. Mol. Microbiol. 37:467-476), and modification of chaperone proteins involved in plasmid replication (Konieczny, I., et al. 1997. The replication initiation protein of the broad-host-range plasmid RK2 is activated by the ClpX chaperone. Proc Natl Acad Sci USA 94:14378-14382).

### II.C.2. Catabolite Repression

Included in the design of the invention are techniques that increase the efficiency of gene expression and protein production in minicells. By way of non-limiting example, these techniques may include utilization and/or modification of factors and systems involved in the synthesis, degradation or transport of catabolites that modulate the genetic expression of a preselected protein. Such manipulations may result in increased or decreased production, and/or changes in the intramolecular and intermolecular functions, of a protein in a minicell or its parent cell; in the latter instance, the protein may be one that is desirably retained in segregated minicells.

By way of non-limiting example, it is known in the art to use promoters from the *trp*, *cst-1,* and *llp* operons of *E. coli,* which are induced by, respectively, reduced tryptophan levels, glucose starvation, and lactose. Manipulation of the catabolites tryptophan, glucose and lactose, respectively, will influence the degree of expression of genes operably linked to these promoters. >Makrides, Savvas C., Strategies for Achieving High-Level Expression of Genes in Escherichia coli. Microbiological Reviews. 1996. 60:512-538.)

As another non-limiting example, expression elements from the *E. coli* L-arabinose (*ara*) operon are used in expression systems. AraC is a protein that acts as a repressor of *ara* genes in the absence of arabinose, and also as an activator of *ara* genes when arabinose is present. Induction of *ara* genes also involves cAMP, which modulates the activity of CRP (cAMP receptor protein), which in turn is required for full induction of *ara* genes (Schleif, Robert, Regulation of the L-arabinose operon of Escherichia coli. 2000. TIG 16:559-564. Thus, maximum expression from an *ara*-based expression system is achieved by adding cAMP and arabinose to host cells, and optimizing the expression of CRP in hostcells.

As one example, manipulation of the acpS gene or gene product of *E. coli* (Pollacco M.L., and J.E. Cronan Jr. 1981. A mutant of Escherichia coli conditionally defective in the synthesis of holo-[acyl carrier protein]. J. Biol. Chem. 256:5750-5754); or homologs of this gene or its gene product found in other prokaryotes, eukaryotes, archaebacteria or organelles (mitochondria, chloroplasts, plastids and the like) may be employed to increase the efficiency of gene expression and protein production in parent cells prior to minicell formation and/or in segregated minicells.

In addition to *acpS*, other exemplary *E. coli* genes include the *b2383 gene* (Berlyn et al., "Linkage Map of Escherichia coli K-12, Edition 9," Chapter 109 in: Escherichia coli and Salmonella typhimurium: Cellular and Molecular Biology, 2nd Ed., Neidhardt, Frederick C., Editor in Chief, American Society for Microbiology, Washington, DC., 1996, Volume 2, pages 1715-1902, and references cited therein. *b2387* gene; the *celA* gene (Parker L.L., and B.G. Hall. 1990. Characterization and nucleotide sequence of the cryptic cel operon of Escherichia coli K12. Genetics. 124:455-471); the *celB* gene (Cole S.T., and B. Saint-Joanis, and A.P. Pugsley. 1985. Molecular characterisation of the colicin E2 operon and identification of its products. Mol Gen Genet. 198:465-472); the *ce*/*C* gene (Parker L.L., and B.G. Hall. 1990. Characterization and nucleotide sequence of the cryptic cel operon of Escherichia coli K12. Genetics. 124:455-471); the *cmtB* gene (Ezhova N.M., Zaikina, N.A, Shataeva,L.K., Dubinina, N.I., Ovechkina, T.P. and J.V. Kopylova. [Sorption properties of carboxyl cation exchangers with a bacteriostatic effect]. 1980. Prikl Bioikhim Mikrobiol. 16:395-398); the *creB* gene (Berlyn et al., "Linkage Map of Escherichia coli K-12, Edition 9," Chapter 109 in: Escherichia coli and Salmonella typhimurium: Cellular and Molecular Biology, 2nd Ed., Neidhardt, Frederick C., Editor in Chief, American Society for Microbiology, Washington, DC., 1996, Volume 2, pages 1715-1902, and references cited therein; the *creC* gene (Wanner B.L. Gene regulation by phosphate in enteric bacteria. 1993. J Cell Biochem. 51:47-54); the *crp* gene (Sabourn D., and J. Beckwith. Deletion of the Escherichia coli crp gene. 1975. J Bacteriology. 122:338-340); the *crr* (*gsr, iex, tgs, treD*) gene (Jones-Mortimer M.C., and H.L. Kornberg, and r. Maltby, and P.D. Watts. Role of the crr-gene in glucose uptake by Escherichia coli. 1977. FEBS Lett. 74:17-19); the *cya* gene (Bachi B., and H.L. Kornberg. Utilization of gluconate by Escherichia coli. A role of adenosine 3':5'-cyclic monophosphate in the induction of gluconate catabolism. 1975. Biochem J. 150:123-128); the *fruA* gene (Prior T.I., and H.L. Kornberg. Nucleotide sequence of fruA, the gene specifying enzyme lifru of the phosphoenopyruvate-dependent sugar phosphotranssferase system in Escherichia coli K12. 1988. J Gen Microbiol. 134:2757-2768); the *fruB* gene (Bol'shakova T.N. and R.S. Erlagaeva, and Dobrynina Oiu, and V.N. Gershanovich. [Mutation fruB in the fructose regulon affeting beta-galactosidase synthesis and adenylate cyclase activity of E. coli K12]. 1988. Mol Gen Mikrobiol virusol. 3:33-39); the *fruR* gene (Jahreis K., and P.W. Postma, and J.W. Lengeler. Nucleotide sequence of the ilvH-frR gene region of Escherichia coli K12 and Salmonella typhimurium LT2. 1991. Mol Gen Genet. 226:332-336); the *frvA* gene (Berlyn et al., "Linkage Map of Escherichia coli K-12, Edition 9," Chapter 109 in: Escherichia coli and Salmonella typhimurium: Cellular and Molecular Biology, 2nd Ed., Neidhardt, Frederick C., Editor in Chief, American Society for Microbiology, Washington, DC., 1996, Volume 2, pages 1715-1902, and references cited therein); the *frwB* gene *(Id.);* the *frvD* gene (*Id.*); the *gatB* gene (Nobelmann B., and J.W. Lengeler. Molecular analysis of the gat genes from Escherichia coli and of their roles in galactitol transport and metabolism. 1996. J Bacteriol. 178:6790-6795); the *gatC* gene (*Id*.); the *malX* gene (Reidel J., W. Boos. The malX malY operon of Escherichia coli encodes a novel enzyme II of the photophotransferase system recognizing glucose and maltose and an enzyme abolishing the endogenous induction of the maltose system. 1991. J Bacteriol. 173:4862-4876); the *manX* (*gptB, mpt, ptsL, ptsM, ptsX, manIII*) gene (Plumbridge J., and A. Kolb. CAP and Nag repressor binding to the regulatory regions of the nagE-B and manX genes of Escherichia coli. 1991. J Mol Biol. 217:661-679); the *manY* (*pel, ptsM, ptsP, manPII*) gene (Henderson P.J., and R.A. Giddens, and M.C. Jones-Mortimer. Transport of galactose, glucose and their molecular analogues by Escherichia coli K12. 1977. Biochem J. 162:309-320); the *manZ* (*gptB, mpt, ptsM, ptsX*) gene (Williams N., and D.K. Fox, and C. Shea and S. Roseman. Pel, the protein that permits lambda DNA penetration of Escherichia coli, is encoded by a gene in ptsM and is required for mannose utilization by the phosphotransferase system. 1986. Proc Natl Acad Sci USA. 83:8934-8938); the *mtlA* gene (Lengeler J. Mutations affecting transport of the hexitols D-mannitol, D-glucitol, and galactitol in Escherichia coli K-12: isolation and mapping. 1975. J Bacteriol. 124:26-38.); the *nagE* (*pstN*) gene (Rogers M.J., and T. Ohgi, and J. Plumbridge, and D. Soll. Nucleotide sequences of the Escherichia coli nagE and nagB genes: the structural genes for the N-acetylglucosamine transport protein of the bacterial phosphoenolpyruvate: sugar phosphotransferase system and for glucosamine-6-phosphate deaminase. 1988. Gene. 62:197-207); the *pstA* gene (Cox G.B., H. Rosenberg, and J.A. Downie, and S. Silver. Genetic analysis of mutants affected in the Pst inorganic phosphate transport system. 1981. J Bacteriol. 148:1-9); the *pstB* (*gutB*) gene (*Id*.); the *pstG* gene (Cox G.B., H. Rosenberg, and J.A. Downie, and S. Silver. Genetic analysis of mutants affected in the Pst inorganic phosphate transport system. 1981. J Bacteriol. 148:1-9); the *pstH* gene (*Id*.); the *pstl* gene (*Id*.); the *pstN* gene (*Id*.); the *pstO* gene (*Id*.); the *ptxA* (*yifU*) gene (Berlyn et al., "Linkage Map of Escherichia coli K-12, Edition 9," Chapter 109 in: Escherichia coli and Salmonella typhimurium: Cellular and Molecular Biology, 2nd Ed., Neidhardt, Frederick C., Editor in Chief, American Society for Microbiology, Washington, DC., 1996, Volume 2, pages 1715-1902, and references cited therein); the *sgcA* (*yjhL*) gene (*Id*.); the *sgcC* (*yjhN*) gene (*Id*.); the *treB* gene (Boos W., U. Ehmann, H. Forkl, W. Klein, M. Rimmele, and P. Postma. Trehalose transport and metabolism in Escherichia coli. 1990. J. Bacteriol. 172:3450-3461 the *usg* gene (Arps P.J., and M.E. Winkler ME. Structural analysis of the Escherichia coli K-12 hisT operon by using a kanamycin resistance cassette. 1987. J Bacteriol. 169:1061-1070); the *wcaD* gene (Mao Y., and M.P. Doyle, and J. Chen. Insertion mutagenisis of wca reduces acide and heat tolerance of enterohemorrhagic Escherichia coli_O157:H7. 2001. J Bacteriol. 183:3811-3815); the *yadl* gene (Berlyn et al., "Linkage Map of Escherichia coli K-12, Edition 9," Chapter 109 in: Escherichia coli and Salmonella typhimurium: Cellular and Molecular Biology, 2nd Ed., Neidhardt, Frederick C., Editor in Chief, American Society for Microbiology, Washington, DC., 1996, Volume 2, pages 1715-1902, and references cited therein); and the ycgC gene (Gutknecht R., and R. Beutler, and L.F. Garcia-Alles, and U. Baumann, and B. Erni. The dihydroxyacetone kinase of Escherichia coli utilizes a phosphoprotein instead of ATP as phosphoryl donor. 2001. EMBO J. 20:2480-2486).

### II.C.3. General Deletions and Modifications

Included in the design of the invention are techniques that increase the efficiency of gene expression and protein production in minicells. By way of non-limiting example, these techniques may include modification or deletion of endogenous gene(s) from which their respective gene product decreases the induction and expression efficiency of a desired protein in the parent cell prior to minicell formation and/or the segregated minicell. By way of non-limiting example, these protein components may be the enzymes that degrade chemical inducers of expression, proteins that have a dominant negative affect upon a positive regulatory elements, proteins that have proteolytic activity against the protein to be expressed, proteins that have a negative affect against a chaperone that is required for proper activity of the expressed protein, and/or this protein may have a positive effect upon a protein that either degrades or prevents the proper function of the expressed protein. These gene products that require deletion or modification for optimal protein expression and/or function may also be antisense nucleic acids that have a negative affect upon gene expression.

### II.C.4. Transcriptional Terminators

Included in the design of the invention are techniques that increase the efficiency of gene expression and protein production in parental cell cytoplasm prior to minicell formation and/or the segregated minicell cytoplasm. By way of non-limiting example, these techniques may include modification of terminator regions of DNA templates or RNA transcripts so that transcription and/or translation of these nucleic acid regions will terminate at greater efficiency. By way of non-limiting example, these techniques may include stem-loop structures, consecutive translational terminators, polyadenylation sequences, or increasing the efficiency of rho-dependent termination. Stem loop structures may include, but are not limited to, inverted repeats containing any combination of deoxyribonucleic acid or ribonucleic acid molecule, more than one such inverted repeat, or variable inverted repeats such that the rate of transcriptional/translational termination may be moderated dependent on nucleic acid and/or amino acid concentration, e.g. the mechanism of regulatory attenuation (Oxdender et al., Attenuation in the Escherichia coli tryptophan operon: role of RNA secondary structure involving the tryptophan codon region, Proc. Natl. Acad. Sci. 76:5524-5528, 1979). See also, Yager and von Hippel, "Transcript Elongation and Termination in e. Col. And Landick and Yanofsky, "Transcriptional Attenuation," Chapters 76 and 77, respectively in: Escherichia Coli and Salmonella Typhimurium: Cellular and Molecular Biology, Neidhardt, Frederick C., Editor in Chief, American Society for Microbiology, Washington, DC., 1987, Volume 1, pages 1241-1275 and 1276-1301, respectively, and references cited therein.

### II.C.5. Proteases

Included in the design of the invention are techniques that increase the efficiency of gene expression and protein production in minicells. By way of non-limiting example, these techniques may include utilization and/or modification of endogenous and/or exogenous proteases. Such manipulations may result in increased or decreased production, and/or changes in the intramolecular and intermolecular functions, of a protein in a minicell or its parent cell; in the latter instance, the protein may be one that is desirably retained in segregated minicells.

The production or activity of a desired protein gene product may be increased by decreasing the level and/or activity of a protease that acts upon the desired protein. The production or activity of a desired protein gene product may be increased by increasing the level and/or activity of a protease that acts upon a protein that inhibits the production or function of the desired protein.

The production or activity of a desired nucleic acid gene product may be increased be increasing the level and/or activity of a protease that acts upon a protein that that inhibits the production or function of the nucleic acid gene product. The production or activity of a desired nucleic acid gene product may be increased by decreasing the level and/or activity of a protease that acts upon a protein that stimulates or enhances the production or function of the desired nucleic acid gene product.

As one example, manipulation of the alpA gene or gene product from E. coli (Kirby J.E., and J.E. Trempy, and S. Gottesman. Excision of a P4-like cryptic prophage leads to Alp protease expression in Escherichia coli. 1994. J Bacteriol. 176:2068-2081), or homologs of this gene or gene product found in other members of the Prokaryotes, Eukaryotes or Archaebacteria, may be employed to increase the efficiency of gene expression and protein production in parent cells prior to minicell formation and/or segregated minicells postpartum.

In addition to alpA, other exemplary E. coli genes and gene products include the clpA gene and gene product from E. coli (Katayama Y., and S. Gottesman, and J. Pumphrey, and S. Rudikoff, and W.P. Clark, and M.R. Maurizi. The two-component, ATP-dependent Clp protease of Escherichia coli. Purification, cloning, and mutational analysis of the ATP-binding component. 1988, J Biol Chem. 263-15226-15236); the clpB gene product from E. coli (Kitagawa M., and C. Wada, and S. Yoshioka, and T. Yura. Expression of ClpB, an analog of the ATP-dependent proteas regulatory subunit in Escherichia coli, is controlled by a heat shock sigma factor (sigma 32). J Bacteriol. 173:4247-4253); the clpC gene product from E. coli (Msadek T., and F. Kunst, and G. Rapoport. MecB of Bacillus subtilis, a member of the ClpC ATPase family, is a pleiotropic regulator controlling competence gene expression and growth at high temperature. 1994. Proc Natl Acad Sci USA 91:5788-5792); the clpP gene product from E. coli (Maurizi M.R., and W.P. Clark, and Y. Katayama, and S. Rudikoff, and J. Pumphrey, and B. Bowers, and S. Gottesman. Sequence and structure of ClpP, the proteolytic component of the ATP-dependent Clp protease of Escherichia coli. 1990. J biol Chem. 265:12536-12545); the clpX gene product from E. coli (Gottesman S., and W.P. Clark, and V. de Crecy-Lagard, and M.R. Maurizi. ClpX, an alternative subunit for the ATP-dependent Clp protease of Escherichia coli. Sequence and in vivo activities. 1993. J Biol Chem. 268:22618-22626); the clpY gene product from E. coli (Missiakas D., and F. Schwager, J.M. Betton, and C. Georgopoulos, S. Raina. Identification and characterization of HslV HslU (ClpQ ClpY) proteins involved in overall proteolysis of misfolded proteins in Escherichia coli. 1996. EMBO J. 15:6899-6909); the dcp gene product from E. coli (Becker S., and Plapp R. Location of the dcp gene on the physical map of Escherichia coli. 1992. J Bacteriol. 174:1698-1699); the degP (htrA) gene product from E. coli (Lipinska B., and M. Zylicz, and C. Georgopoulos. The HtrA (DegP) protein, essential for Escherichia coli survival at high temperatures, is an endopeptidase. 1990. J Bacteriol. 172:1791-1797); the ggt gene product from E. coli (Finidori J., and Y. Laperche, and R. Haguenauer-Tsapis, and R. Barouki, and G. Guellaen, and J. Hanoune. In vitro biosynthesis and membrane insertion of gamma-glutamyl transpeptidase. 1984. J Biol Chem. 259:4687-4690); the hfl gene product from E. coli (Cheng H. H., and H. Echols. A class of Escherichia coli proteins controlled by the hflA locus. 1987. J Mol Biol. 196:737-740); the hflB gene product from E. coli (Banuett F., and M.A. Hoyt, and L. McFarlane, and H. Echols, and I. Herskowitz. HflB, a new Escherichia coli locus regulating lysogeny and the level of bacteriophage lambda c11 protein. 1986. J Mol Biol. 187:213-224); the hflC gene product from E. coli (Noble J.A., and M.A. Innis, and E.V. Koonin, and K.E. Rudd, and F. Banuett, and I. Herskowitz, The Escherichia coli hflA locus encodes a putative GTP-binding protein and two membrane proteins, one of which contains a protease-like domain. 1993. Proc Natl Acad Sci USA. 90:10866-10870); the hflK gene product from E. coli (Id.); the hftX gene product from E. coli (Noble J.A., and M.A. Innis, and E.V. Koonin, and K.E. Rudd, and F. Banuett, and I. Hertzskowitz. The Escherichia coli hflA locus encodes a putative GTP-binding protein and two membrane proteins, one of which contains a protease-like domain. 1993. Proc Natl Acad Sci USA. 90:10866-10870); the hopD gene product from E. coli (Whitchurch C.B., and J.S. Mattick Escherichia coli contains a set of genes homologous to those involved in protein secretion, DNA uptake and the assembly of type-4 fimbriae in other bacteria. 1994. Gene. 150:9-15); the htrA gene product from E. coli (Lipinska B., and S. Sharma, and C. Georgopoulos. Sequence analysis and regulation of the htrA gene of Escherichia coli: a sigma 32-independent mechanism of heat-inducible transcription. 1988. Nucleicc Acids Res. 16:10053-10067); the hycl gene product from E. coli (Rossmann R., and T. Maier, and F. Lottspeich, and A. Bock. Characterisation of a proteas from Escherichia coli involved in hydrogenase maturation. 1995. Eur J Biochem. 227:545-550); the iap gene product from E. coli (Nakata A., and M. Yamaguchi, and K. Isutani, and M. Amemura. Escherichia coli mutants deficient in the production of alkaline phosphatase isoszymes. 1978. J Bacteriol. 134:287-294); the lep gene product from E. coli (Silver P., and W. Wickner. Genetic mapping of the Escherichia coli leader (signal) peptidase gene (lep): a new approach for determining the map position of a cloned gene. 1983. J Bacteriol. 54:659-572); the Ion gene product from E. coli (Donch J., and J. Greenberg. Genetic analysis of Ion mutants of strain K-12 of Escherichia coli. 1968. Mol Gen Genet. 103:105-115); the Isp gene product from E. coli (Regue M., and J. Remenick, and M. tokunaga, and G.A. Mackie, and H.C. Wu. Mapping of the lipoprotein signal peptidase gene (Isp). 1984. J Bacteriol. 1984 158:632-635); the ompT gene product from E. coli (Akiyama Y., and K. SecY protein, a membrane-embedded secretion factor of E. coli, is cleaved by the ompT proteas in vitro. 1990. Biochem Biophys Res Commun. 167:711-715); the opdA gene product from E. coli (Conllin C.A., and C.G. Miller. Location of the prlC (opdA) gene on the physical map of Escherichia coli. 1993. J Bacteriiol. 175:5731-5732); the orfX gene product from E. coli (Berlyn, M.K.B., et al. 1996. Linkage map of Escherichia coli K-12, Edition 9. In F.C. Neidhardt, R. Curtiss, J.L. Ingraham, E.C.C. Lin, K.B. Low, B. Magasanik, W.S. Reznikoff, M. Riley, M. Schaechter, and H.E. Umbarger (eds.). Escherichia coli and Salmonella typhimurium: Cellular and Molecular Biology, 2nd ed. American Society for Microbiology, Washington D.C.); the pepA gene product from E. coli (Stirling C.J., and S.D. Colloms, and J.F. Collins, and G. Szatmari, and D.J. Sherratt. XerB, an Escherichia coli gene required for plasmid ColE1 site-specific recombination, is identical to pepA, encoding aminopeptidaseA, a protein with substantial similarity to bovine lens leucine aminopeptidase. 1989. EMBO J. 8:1623-1627); the pepD gene product from E. coli (Henrich B., and U. Schroeder, and R.W. Frank, and R. Plapp. Accurate mapping of the Escherichia coli pepD gene by sequence analysis of its 5' flanking region. 1989. Mol Gen Genet. 215:369-373); the pepE gene product from E. coli (Conlin C.A., and T.M. Knox, and C.G. Miller. Cloning and physical map position of an alpha-aspartyl depeptidase gene, pepE, from Escherichia coli. 1994. J Bacteriol. 176:1552-1553); the pepN gene product from E. coli (Miller C.G., and G. Schwartz. Peptidase-deficient mutants of Escherichia coli. 1978. J Bacteriiol. 135:603-611); the pepP gene product from E. coli (Id.); the pepQ gene product from E. coli (Id.); the pepT gene product from E. coli (Miller G.G., and G. Schwartz. Peptidase-deficient mutants of Escherichia coli. 1978. J Bacteriiol. 135:603-611); the pilD gene product from E. coli (Francetic O., and S. Lory, and A.P. Pugsley. A second prepilin peptidase gene in Escherichia coli K-12. 1998, Mol Microbiol. 27:763-775); the pinA gene product from E. coli (Hilliard J.J., and L.D. Simon, and L. Van Melderen, and M.R. Maurizi. PinA inhibits ATP hydrolysis and energy-dependent protein degradation by Lon protease. 1998. J Biol Chem. 273:524-527); the prc(tsp) gene product from E. coli (Nagasawa H., and Y. Sakagami, and A. Suzuki, and H. Suzuki, and H. Hara, and Y. Hirota. Determination of the cleavage site involved in C-terminal processing of penicillin-binding proein 3 of Escherichia coli. 1989. J Bacteriol. 171:5890-5893); the prlC gene product from E. coli (Jiang X., and M. Zhang, and Y. Ding, and J. Yao, and H. Chen, and D. Zhu, and M. Muramatu. Escherichia coli prlC gene encodes a trypsin-like proteinase regulating the cell cycle. 1998. J Biochem (Tokyo) 128:980-985); the protease V gene product from E. coli (Berlyn, M.K.B. et al. 1996. Linkage map of Escherichia coli K-12, Edition 9, In F.C. Neidhardt, R. Curtiss, J.L. Ingraham, E.C.C. Lin, K.B. Low, B. Magasanik, W.S. Reznikoff, M. Riley, M. Schaechter, and H.E. Umbarger (eds.). Escherichia coli and Salmonella typhimurium: Cellular and Molecular Biology, 2nd ed. American Society for Microbiology, Washington, D.C.); the protease VI gene product from E. coli (Id.); the protease In gene product from E. coli (*Id*.); the protease Fa gene product from E. coli or homologues *(Id.);* the protease Mi gene product from E. coli (Id.); the protease So gene product from E. coli *(Id.);* the ptrA gene product from E. coli *(Id.);* the ptrB gene product from E. coli *(Id.);* the sypB gene product from E. coli (Barends S., and A.W. Karzai, and R.T.Sauer, and J. Wower, and B. Kraal. Simultaneous an functional binding of SmpB and EF-Tu-TP to the analyl acceptor arm of tmRNA. 2001. J Mol Biol. 314:9-21); the sohB gene product from E. coli (Baird L., and B. Lipinska, and S. Raina, and C. Georgopoulos. Identification of the Escherichia coli sohB gene, a multicopy suppressor of the HtrA (DegP) null phenotype. 1991. J Bacteriol. 173-5763-5770); the sspA gene product from E. coli (Ichihara S., and T. Suzuki, and M. Suzuki, and C. Mizushima. Molecular cloning and sequencing of the sppA gene and characterization of the encoded proteas IV, a signal peptide peptidase of Escherichia coli. 1986. J Biol Chem. 261;9405-9411); the tesA gene product from E. coli (Cho H., and J.E. Cronan Jr. Escherichia coli thioesterase I, molecular cloning and sequencing of the structural gene and identification as a periplasmic enzyme. 1993 J Biol Chem. 268:9238-9245); the tufA gene product from E. coli (Ang., and J.S. Lee, and J.D. Friesen. Evidence for an internal promoter preceding tufA in the str operon of Escherichia coli. J Bacteriol. 149:548-553); the tufB gene product from E. coli (Mihajima A., and M.Shibuya, and Y. Kaziro. Construction and characterization of the two hybrid Col1E1 plasmids carrying Escherichia coli tufB gene. 1979. FEBS Lett. 102:207-210); the ybaU gene product from E. coli (Berlyn, M.K.B., et al. 1996. Linkage map of Escherichia coli K-12, Edition 9. In F.C. Neidhardt, R. Curtiss, J.L. Ingraham, E.C.C. Lin, K.B. Low, B. Magasanik, W.S. Resnikoff, M. Riley, M. Schaechter, and H.E. Umbarger (eds.). Escherichia coli and Salmonella typhimurium: Cellular and Molecular Biology, 2nd ed. American Society for Microbiology, Washington, D.C.); the ssrA gene (tmRNA, 10sA RNA) product from E. coli (Oh B.K., and A.K. Chauhan, and K. Isono, and D. Apirion. Location of a gene (ssrA) for a small, stable RNA 910Sa RNA) in the Escherichia coli chromosome. 1990. J Bacteriol. 172:4708-4709); and the ssrB gene from E. coli (Berlyn, M.K.B., et al. 1996. Linkage map of Escherichia coli K-12, Edition 9. In F.C. Neidhardt, R. Curtiss, J.L. Ingraham, E.C.C. Lin, K.B. Low, B. Magasanik, W.S. Reznikoff, M. Riley, M. Schaechter, and H.E. Ummbarger 9eds.). Escherichia coli and Salmonella typhimurium: Cellular and Molecular Biology, 2nd ed. American Society for Microbiology, Washington, D.C.).

### II.C.6. Chaperones

Included in the design of the invention are techniques that increase the efficiency of gene expression and functional protein production in minicells. By way of non-limiting example, these techniques may include modification of chaperones and chaperonins, i.e., endogenous and/or exogenous protein components that monitor the unfolded state of translated proteins allowing proper folding and/or secretion, membrane insertion, or soluble multimeric assembly of expressed proteins in the parental cell prior to minicell formation and/or the segregated minicell cytoplasm, membrane, periplasm, and/or extracellular environment. See Gottesman et al., Protein folding and unfolding by Escherichia coli chaperones and chaperonins, Current Op. Microbiol. 3:197-202, 2000; and Mayhew et al., "Molecular Chaperone Proteins," Chapter 61 in: Escherichia coli and Salmonella typhimurium: Cellular and Molecular Biology, 2nd Ed., Neidhardt, Frederick C., Editor in Chief, American Society for Microbiology, Washington, DC., 1996, Volume 1, pages 922-937, and references cited therein.

These applications may, but are not limited to increased or decreased chaperone production, increased or decreased intramolecular activity of a chaperone, increased or decreased physiological function of a chaperone, or deletion, substitution, inversion, translocation or insertion into, or mutation of, a gene encoding a chaperone. By way of non-limiting example, increased production of a chaperone may occur through increased chaperone gene dosage (increased copy number of a given gene under the control of the native or artificial promotor where this gene may be on a plasmid or in more than one copy on the chromosome), modification of the native regulatory elements, including, but not limited to the promotor or operator region(s) of DNA, or ribosomal binding sites on RNA, relevant repressors/silencers, relevant activators/enhancers, or relevant antisense nucleic acid or nucleic acid analog, cloning on a plasmid under the control of the native or artificial promotor, and increased or decreased production of native or artificial promotor regulatory element(s) controlling production of the chaperone gene or gene product. By way of non-limiting example, decreased production of a chaperone may occur through modification of the native regulatory elements, including, but not limited to the promotor or operator region(s) of DNA, or ribosomal binding sites on RNA, relevant repressors/silencers, relevant activators/enhancers, or relevant antisense nucleic acid or nucleic acid analog, through cloning on a plasmid under the control of the native regulatory region containing mutations or an artificial promotor, either or both of which resulting in decreased chaperone production, and through increased or decreased production of native or artificial promotor regulatory element(s) controlling production of the chaperone gene or gene product. By definition, intramolecular activity refers to the enzymatic function, structure-dependent function, e.g. the capacity of chaperone to interact in a protein-protein, protein-nucleic acid, or protein-lipid complex, and/or carrier function, e.g. the capacity to bind, either covalently or non-covalently small organic or inorganic molecules, protein(s), carbohydrate(s), fatty acid(s), lipid(s), and nucleic acid(s). By way of non-limiting example, alteration of intramolecular activity may be accomplished by mutation of the chaperone gene, in vivo or in vitro chemical modification of Chaperone, inhibitor molecules against the function of chaperone, e.g. competitive, non-competitive, or uncompetitive enzymatic inhibitors, inhibitors that prevent protein-protein, protein-nucleic acid, or protein-lipid interactions, e.g. expression or introduction of dominant-negative or dominant-positive chaperone or other protein fragment(s), or other carbohydrate(s), fatty acid(s), lipid(s), and nucleic acid(s) that may act directly or allosterically upon Chaperone, and/or modification of protein, carbohydrate, fatty acid, lipid, or nucleic acid moieties that modify the chaperone gene or gene product to create the functional protein. By definition, physiological function refers to the effects resulting from an intramolecular interaction between Chaperone and other protein, carbohydrate, fatty acid, lipid, nucleic acid, or other molecule(s) in or on the cell or the action of a product or products resulting from such an interaction. By way of non-limiting example, physiological function may be the act or result of intermolecular phosphorylation, biotinylation, methylation, acylation, glycosylation, and/or other signaling event; this function may be the result of a protein-protein, protein-nucleic acid, or protein-lipid interaction resulting in a functional moiety; this function may be to interact with the membrane to recruit other molecules to this compartment of the cell; this function may be to regulate the transcription and/or translation of chaperone, other protein, or nucleic acid; and this function may be to stimulate the function of another process that is not yet described or understood.

By way of non-limiting example, chaperone genes may be any of the E. coli genes listed below, as well as any homologs thereof from prokaryotes, exukariutes, arcahebacteria, or organelles (mitochondria, chloroplasts, plastids, etc.). Exemplary E. coli genes encoding chaperones include, by way of non-limiting example, the cbpA gene (Shiozawa T., and C. Ueguchi, and T. Mizuno. The rpoD gene functions as a multicopy suppressor for mutations in the chaperones, CbpA, DnaJ and DnaK, in Escherichia coli. 1996 FEMS Microbiol Lett. 138:245-250): the clpB gene (Squires C. L., and S. Pedersen, and B. M. Ross, and C. Squires. ClpB is the Escherichia coli heat shock protein F84.1. 1991. J Bacteriol. 173:4254-4262); the dnaK gene (Kroczynska B., and S. Y. Blond. Cloning and characterization of a new soluble murine J-domain protein that stimulates BiP, Hsc70 and DnaK ATPase activity with different efficiencies. 2001. Gene. 273:267-274); the dnaJ gene (Kedzierska S., and E. Matuszewska. The effect of co-overproduction of DnaK/DnaJ/GrpE and ClpB proteins on the removal of heat-aggregated proteins from Escherichia coli Delta clpB mutant cells--new insight into the role of Hsp70 in a functional cooperation with Hsp100. 2001. FEMS Microbiol Lett. 204:355-360); the ecpD gene (Raina S., and D. Missiakas, and L. Baird, and S. Kumar, and C. Georgopoulos. Identification and transcriptional analysis of the Escherichia coli htrE operon which is homologous to pap and related pilin operons. 1993. J Bacteriol. 175:5009-5021); the ffh gene (Muller, M., et al. 1002. Protein traffic in bacteria: multiple routes from the ribosome to and across the membrane. Prog. Nucleic Acid Res. Mol. Biol. 66:107-157); 4.5S RNA (Muller, M., et al. 1002. Protein traffic in bacteria: multiple routes from the ribosome to and across the membrane. Prog. Nucleic Acid Res. Mol. Biol. 66:107-157); the FtsY gene (Muller, M., et al. 1002. Protein traffic in bacteria: multiple routes from the ribosome to and across the membrane. Prog. Nucleic Acid Res. Mol. Biol. 66:107-157);the fimC gene (Klemm P., and B. J. Jorgensen, and I. van Die, and H. de Ree, and H. Bergmans. The fim genes responsible for synthesis of type 1 fimbriae in Escherichia coli, cloning and genetic organization. 1985. Mol Gen Genet. 199:410-414); the groE gene (Burton Z. F., and D. Eisenberg. A procedure for rapid isolation of both groE protein and glutamine synthetase from E coli. 1980. Arch Biochem Biophys. 205:478-488); the groL gene (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); the groS gene (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); the hptG gene (Berlyn, M. K. B., et al. 1996. Linkage map of Escherichia coli K-12, Edition 9. In F. C. Neidhardt, R. Curtiss, J. L. Ingraham, E. C. C. Lin, K. B. Low, B. Magasanik, W. S. Reznikoff, M. Riley, M. Schaechter, and H. E. Umbarger (eds.). Escherichia coli and Salmonella typhimurium: cellular and molecular biology, 2nd ed. American Society for Microbiology, Washington D. C.); the hscA gene (Takahashi Y., and M. Nakamura. Functional assignment of the ORF2-iscS-iscU-iscA-hscB-hscA-fdx-ORF3 gene cluster involved in the assembly of Fe-S clusters in Escherichia coli.1999. J Biochem (Tokyo). 126:917-926); the ibpA gene (Lund P. A. Microbial molecular chaperones. 2001. Adv Microb Physiol. 44:93-140); the papJ gene (Tennent, J. M., et al. 1990. Integrity of Escherichia coli P pili during biogenesis: properties and role of PapJ. Mol. Microbiol. 4:747-758); the secB gene (Lecker, S., et al. 1989. Three pure chaperone proteins of Escherichia coli--SecB, trigger factor and GroEL--form soluble complexes with precursor proteins in vitro. EMBO J. 8:2703-2709); and the tig gene (Lecker, S., et al. 1989. Three pure chaperone proteins of Escherichia coli--SecB, trigger factor and GroEL--form soluble complexes with precursor proteins in vitro. EMBO J. 8:2703-2709); the secE gene (Muller, M., et al. 1002. Protein traffic in bacteria: multiple routes from the ribosome to and across the membrane. Prog. Nucleic Acid Res. Mol. Biol. 66:107-157); and the secY gene (Muller, M., et al. 1002. Protein traffic in bacteria: multiple routes from the ribosome to and across the membrane. Prog. Nucleic Acid Res. Mol. Biol. 66:107-157).

### II.C.7. Export Apparatus and Membrane Targeting

Included in the design of the invention are techniques that increase the efficiency of gene expression and protein production in parental cells prior to minicell formation and/or in the segregated minicells. By way of non-limiting example, these techniques may include construction of chimeric proteins including, but not limited to, coupling the expressed protein of interest with native Eubacterial, Eukaryotic, Archeabacterial or organellar leader sequences to drive membrane insertion or secretion of the protein of interest to the periplasm or extracellular environment. In addition to using native leader sequences, these minicell expression constructs may also include proteolytic cleavage sites to remove the leader sequence following insertion into the membrane or secretion. These proteolytic cleavage sites may be native to the organism from which the minicell is derived or non-native. In the latter example, also included in the system are the non-native protease that recognizes the non-native proteolytic cleavage site.

Non-limiting examples of these leader sequences may be the leader from the STII protein (Voss, T., et al. 1994. Periplasmic expression of human interferon-alpha 2c in Escherichia coli results in a correctly folded molecule. Biochem. J. 298:719-725), maltose binding protein (malE) (Ito, K. 1982. Purification of the precursor form of maltose-binding protein, a periplasmic protein of Escherichia coli. J. Biol. Chem. 257:9895-9897), phoA (Jobling, M. G., et al. 1997. Construction and characterization of versatile cloning vectors for efficient delivery of native foreign proteins to the periplasm of Escherichia coli. Plasmid. 38:158-173), lamB (Wong, E. Y., et al. 1988. Expression of secreted insulin-like growth factor-1 in Escherichia coli. Gene. 68:193-203), ompA (Loo, T., et al. 2002. Using secretion to solve a solubility problem: high-yield expression in Escherichia coli and purification of the bacterial glycoamidase PNGase F. Protein Expr. Purif. 24:90-98), or pelB (Molloy, P. E., et al. 1998. Production of soluble single-chain T-cell receptor fragments in Escherichia coli trxB mutants. Mol. Immunol. 35:73-81).

In addition to these leader sequences, mutations in the cellular export machinery may be employed to increase the promiscuity of export to display or export sequences with non-optimized leader sequences. Non-limiting examples of genes that may be altered to increase export promiscuity are mutations in secY (prlA4) (Derman, A. I., et al. 1993. A signal sequence is not required for protein export in prlA mutants of Escherichia coli. EMBO J. 12:879-888), and secE (Harris, C. R., and T. J. Silhavy. 1999. Mapping an interface of SecY (PrlA) and SecE (PrlG) by using synthetic phenotypes and in vivo cross-linking. J. Bacteriol. 181:3438-3444).

### II.C.8. Increasing Stability and Solubility

Included in the design of the invention are techniques that increase the efficiency of gene expression and protein production in parental cells prior to minicell formation and/or in the segregated minicells. By way of non-limiting example, these techniques may include construction of chimeric/fusion proteins including, but not limited to, coupling the expressed protein of interest with native Eubacterial, Eukaryotic, Archeabacterial or organellar solublizing sequences. As used herein, "solublizing sequences" are complete or truncated amino acid sequences that increase the solubility of the expressed membrane protein of interest. This increased solubility may be used to increase the lifetime of the soluble state until proper membrane insertion may take place. By way of non-limiting example, these soluble chimeric fusion proteins may be ubiquitin (Power, R. F., et al. 1990. High level expression of a truncated chicken progesterone receptor in Escherichia coli. J. Biol. Chem. 265:1419-1424), thioredoxin (LaVallie, E. R., et al. 1993. A thioredoxin gene fusion expression system that circumvents inclusion body formation in the E. coli cytoplasm. Biotechnology (N. Y.) 11:187-193; Kapust, R. B., and D. S. Waugh. 1999. Escherichia coli maltose-binding protein is uncommonly effective at promoting the solubility of polypeptides to which it is fused. Protein Sci. 8:1668-1674), the dsbA gene product (Winter, J., et al. 2001. Increased production of human proinsulin in the periplasmic space of Escherichia coli by fusion to DsbA. J. Biotechnol. 84:175-185), the SPG protein (Murphy, J. P., et al. 1992. Amplified expression and large-scale purification of protein G'. Bioseparation 3:63-71), the malE gene product (maltose-binding protein) (Hampe, W., et al. 2000. Engineering of a proteolytically stable human beta 2-adrenergic receptor/maltose-binding protein fusion and production of the chimeric protein in Escherichia coli and baculovirus-infected insect cells. J. Biotechnol. 77:219-234; Kapust et al., Escherichia coli maltose-binding protein is uncommonly effective at promoting the solubility of polypeptides to which it is fused, Protein Sci. 8:1668-1674, 1999), glutathione-s-transferase (GST); and/or nuclease A (Meeker et al., A fusion protein between serum amyloid A and staphylococcal nuclease--synthesis, purification, and structural studies, Proteins 30:381-387, 1998). In addition to these proteins, Staphylococcal protein A, beta-galactosidase, S-peptide, myosin heavy chain, dihydrofolate reductase, T4 p55, growth hormone N terminus, E. coli Hemolysin A, bacteriophage lambda cII protein, TrpE, and TrpLE proteins may also be used as fusion proteins to increase protein expression and/or solubility (Makrides, Stratagies for Achieving High-Level Expression of Genes in Escherichia coli, Microbiol. Rev. 60:512-538).

### III. PREPARATION OF MINICELLS

### III.A. Parent Cell Mutations

Although it has been reported that relatively few molecules of endogenous RNA polymerase segregate into minicells (Shepherd et al., Cytoplasmic RNA Polymerase in Escherichia coli, J Bacteriol 183:2527-34, 2001), other reports and results indicate that many RNA Polymerase molecules follow plasmids into minicells (Funnell and Gagnier, Partition of P1 plasmids in Escherichia cole mukB chromosomal partition mutants, J Bacteriol 177:2381-6, 1995). In any event, applicants have discovered that the introduction of an exogenous RNA polymerase to minicell-producing cells enhances expression of episomal elements in minicells. Such enhanced expression may allow for the successful expression of proteins in minicells, wherein such proteins are expressed poorly or not at all in unmodified minicells. In order to maximize the amount of RNA transcription from episomal elements in minicells, minicell-producing cell lines that express an RNA polymerase specific for certain episomal expression elements may be used. An example of an *E*. *coli* strain of this type, designated MC-T7, was created and used as is described in the Examples. Those skilled in the art will be able to make and use equivalent strains based on the present disclosure and their knowledge of the art.

Minicell-producing cells may comprise mutations that augment preparative steps. For example, lipopolysaccharide (LPS) synthesis in *E*. *coli* includes the lipid A biosynthetic pathway. Four of the genes in this pathway have now been identified and sequenced, and three of them are located in a complex operon that also contains genes involved in DNA and phospholipid synthesis. The *rfa* gene cluster, which contains many of the genes for LPS core synthesis, includes at least 17 genes. The *rfb* gene cluster encodes protein involved in O-antigen synthesis, and *rfb* genes have been sequenced from a number of serotypes and exhibit the genetic polymorphism anticipated on the basis of the chemical complexity of the O antigens. See Schnaitman and Klena, Genetics of lipopolysaccharide biosynthesis in enteric bacteria, Microbiol. Rev. 57:655-82, 1993. When present, alone, or in combination, the *rfb* and *oms* mutations cause alterations in the eubacterial membrane that make it more sensitive to lysozyme and other agents used to process minicells. Similarly, the *rfa* (Chen, L., and W. G. Coleman Jr. 1993. Cloning and characterization of the Escherichia coli K-12 rfa-2 (rfaC) gene, a gene required for lipopolysaccharide inner core synthesis. J. Bacteriol. 175:2534-2540), *IpcA* (Brooke, J. S., and M. A. Valvano. 1996. Biosynthesis of inner core lipopolysaccharide in enteric bacteria identification and characterization of a conserved phosphoheptose isomerase. J. Biol. Chem. 271:3608-3614), and *IpcB* (Kadrman, J. L., et al. 1998. Cloning and overexpression of glycosyltransferases that generate the lipopolysaccharide core of Rhizobium leguminosarum. J. Biol. Chem. 273:26432-26440) mutations, when present alone or in combination, cause alterations in lipopolysaccharides in the outer membrane causing cells to be more sensitive to lysozyme and agents used to process minicells. In addition, such mutations can be used to reduce the potential antigenicity and/or toxicity of minicells.

### III.B. Culturing Conditions

Included in the design of the invention are the conditions to grow parental cells from which minicells will be produced. Non-limiting examples herein are drawn to conditions for growing *E. coli* parental cells to produce minicells derived from *E. coli* parental cells. Non-limiting examples for growth media may include rich media, e.g. Luria broth (LB), defined minimal media, e.g. M63 salts with defined carbon, nitrogen, phosphate, magnesium, and sulfate levels, and complex minimal media, e.g. defined minimal media with casamino acid supplement. This growth may be performed in culture tubes, shake flasks (using a standard air incubator, or modified bioreactor shake flask attachment), or bioreactor. Growth of parental cells may include supplemented additions to assist regulation of expression constructs listed in the sections above. These supplements may include, but are not limited to dextrose, phosphate, inorganic salts, ribonucleic acids, deoxyribonucleic acids, buffering agents, thiamine, or other chemical that stimulates growth, stabilizes growth, serves as an osmo-protectant, regulates gene expression, and/or applies selective pressure to mutation, and/or marker selection. These mutations may include an amino acid or nucleotide auxotrophy, while the selectable marker may include transposable elements, plasmids, bacteriophage, and/or auxotrophic or antibiotic resistance marker. Growth conditions may also require temperature adjustments, carbon alternations, and/or oxygen-level modifications to stimulate temperature sensitive mutations found in designed gene products for a given desired phenotype and optimize culture conditions.

By way of non-limiting example, production of minicells and protein production may occur by using either of two general approaches or any combination of each. First, minicells may be formed, purified, and then contained expression elements may be stimulated to produce their encoded gene products. Second, parental cells, from which the minicells are derived, may be stimulated to express the protein of interest and segregate minicells simultaneously. Finally, any timing variable of minicell formation and protein production may be used to optimize protein and minicell production to best serve the desired application. The two general approaches are shown in the sections below.

### III.C. Manipulation of Genetic Expression in Minicell Production

Included in the design of the invention are methods that increase the efficiency, rate and/or level of gene expression and protein production in parent cells and/or minicells. Such methods include, but are not limited to, the following.

By way of non-limiting example, parental cells are grown overnight in the appropriate media. From this culture, the cells are subcultured into the same media and monitored for growth. At the appropriate cell density, the cultures are induced for minicell production using any of the switching mechanisms discussed in section II.B. regulating any construct discussed in section II.A Non-limiting examples of this minicell-inducing switching mechanism may be the *ileR* gene product regulating the production of the hns minicell-inducing gene product or the *melR* gene product regulating the production of the minB minicell-inducing gene product. Following minicell induction, the culture is allowed to continue growth until the desired concentration of minicells is obtained. At this point, the mincells are separated from the parental cells as described in section II.E. Purified minicells are induced for protein production by triggering the genetic switching mechanism that segregated into the minicell upon separation from the parental cell. By way of non-limiting example, this genetic switching mechanism may be any of those discussed in section I.B. regulating the production of any protein of interest. Furthermore, at this point or during the production of minicells the peripheral gene expression, production, and assembly machinery discussed in section II.C. may be triggered to assist in this process. By way of non-limiting example, the groEL complex may be triggered using the temperature sensitive lambda cl inducible system from a co-segregant plasmid to assist in the proper assembly of the expressed protein of interest.

### III.D. Separation of Minicells From Parent Cells

A variety of methods are used to separate minicells from parent cells (i.e., the cells from which the minicells are produced) in a mixture of parent cells and minicells. In general, such methods are physical, biochemical and genetic, and can be used in combination.

### III.D.1. Physical Separation of Minicells from Parent Cells

By way of non-limiting example, minicells are separated from parent cells glass-fiber filtration (Christen et al., Gene 23:195-198, 1983), and differential and zonal centrifugation (Barker et al., J. Gen. Microbiol. 111:387-396, 1979), size-exclusion chromatography, e.g. gel-filtration, differential sonication (Reeve, J. N., and N. H. Mendelson. 1973. Pronase digestion of amino acid binding components on the surface of Bacillus subtilis cells and minicells. Biochem. Biophys. Res. Commun. 53:1325-1330), and UV-irradiation (Tankersley, W. G., and J. M. Woodward. 1973. Induction and isoloation of non-replicative minicells of Salmonella typhimuium and their use as immunogens in mice. Bacteriol. Proc. 97).

Some techniques involve different centrifugation techniques, e.g., differential and zonal centrifugation. By way of non-limiting example, minicells may be purified by the double sucrose gradient purification technique described by Frazer and Curtiss, Curr. Topics Microbiol. Immunol. 69:1-84, 1975. The first centrifugation involves differential centrifugation, which separates parent cells from minicells based on differences in size and/or density. The percent of sucrose in the gradient (graduated from about 5 to about 20%), Ficol or glycerol is designed to allow only parent cells to pass through the gradient.

The supernatant, which is enriched for minicells, is then separated from the pellet and is spun at a much higher rate (e.g., ≥11,000 g). This pellets the minicells and any parent cells that did not pellet out in the first spin. The pellet is then resuspended and layered on a sucrose gradient.

The band containing minicells is collected, pelleted by centrifugation, and loaded on another gradient. This procedure is repeated until the minicell preparation is essentially depleted of parent cells, or has a concentration of parent cells that is low enough so as to not interfere with a chosen minicell application or activity. By way of non-limiting example, buffers and media used in these gradient and resuspension steps may be LB, defined minimal media, e.g. M63 salts with defined carbon, nitrogen, phosphate, magnesium, and sulfate levels, complex minimal media, e.g. defined minimal media with casamino acid supplement, and/or other buffer or media that serves as an osmo-protectant, stabilizing agent, and/or energy source, or may contain agents that limit the growth of contaminating parental cells, e.g azide, antibiotic, or lack an auxotrophic supplemental requirement, e.g. thiamine.

Other physical methods may also be used to remove parent cells from minicell preparations. By way of non-limiting example, mixtures of parent cells and minicells are frozen to -20°C and then thawed slowly (Frazer and Curtiss, Curr. Topics Microbiol. Immunol. 69:1-84, 1975).

### III.D.2. Biochemical Separation of Minicells From Parent Cells

Contaminating parental cells may be eliminated from minicell preparations by incubation in the presence of an agent, or under a set of conditions, that selectively kills dividing cells. Because minicells can neither grow nor divide, they are resistant to such treatments.

Examples of biochemical conditions that prevent or kill dividing parental cells is treatment with a antibacterial agent, such as penicillin or derivatives of penicillin. Penicillin has two potential affects. First, penicillin prevent cell wall formation and leads to lysis of dividing cells. Second, prior to lysis dividing cells form filaments that may assist in the physical separation steps described in section III.E.1. In addition to penicillin and its derivatives, other agents may be used to prevent division of parental cells. Such agents may include azide. Azide is a reversible inhibitor of electron transport, and thus prevents cell division. As another example, D-cycloserine or phage MS2 lysis protein may also serve as a biochemical approach to eliminate or inhibit dividing parental cells. (Markiewicz et al., FEMS Microbiol. Lett. 70:119-123, 1992). Khachatourians (U.S. Patent No. 4,311,797) states that it may be desirable to incubate minicell/parent cell mixtures in brain heart infusion broth at 36°C to 38°C prior to the addition of penicillin G and further incubations.

### III.D.3. Genetic Separation of Minicells From Parent Cells

Alternatively or additionally, various techniques may be used to selectively kill, preferably lyse, parent cells. For example, although minicells can internally retain M13 phage in the plasmid stage of the M13 life cycle, they are refractory to infection and lysis by M13 phage (Staudenbauer et al., Mol. Gen. Genet. 138:203-212, 1975). In contrast, parent cells are infected and lysed by M13 and are thus are selectively removed from a mixture comprising parent cells and minicells. A mixture comprising parent cells and minicells is treated with M13 phage at an M.O.I. = 5 (phage:cells). The infection is allowed to continue to a point where ≥50% of the parent cells are lysed, preferably ≥75%, more preferably ≥95% most preferably ≥99%; and ≤25% of the minicells are lysed or killed, preferably ≤15%, most preferably ≤1 %.

As another non-limiting example of a method by which parent cells can be selectively killed, and preferably lysed, a chromosome of a parent cell may include a conditionally lethal gene. The induction of the chromosomal lethal gene will result in the destruction of parent cells, but will not affect minicells as they lack the chromosome harboring the conditionally lethal gene. As one example, a parent cell may contain a chromosomal integrated bacteriophage comprising a conditionally lethal gene. One example of such a bacteriophage is an integrated lambda phage that has a temperature sensitive repressor gene (e.g., lambda cl857). Induction of this phage, which results in the destruction of the parent cells but not of the achromosomal minicells, is achieved by simply raising the temperature of the growth media. A preferred bacteriophage to be used in this method is one that kills and/or lyses the parent cells but does not produce infective particles. One non-limiting example of this type of phage is one that lyses a cell but which has been engineered to as to not produce capsid proteins that are surround and protect phage DNA in infective particles. That is, capsid proteins are required for the production of infective particles.

As another non-limiting example of a method by which parent cells can be selectively killed or lysed, toxic proteins may be expressed that lead to parental cell lysis. By way of non-limiting example, these inducible constructs may employ a system described in section II.B. to control the expression of a phage holing gene. Holin genes fall with in at least 35 different families with no detectable orthologous relationships (Grundling, A., et al. 2001. Holins kill without warning. Proc. Natl. Acad. Sci. 98:9348-9352) of which each and any may be used to lyse parental cells to improve the purity of minicell preparations.

Gram negative eubacterial cells and minicells are bounded by an inner membrane, which is surrounded by a cell wall, wherein the cell wall is itself enclosed within an outer membrane. That is, proceeding from the external environment to the cytoplasm of a minicell, a molecule first encounters the outer membrane (OM), then the cell wall and finally, the inner membrane (IM). In different aspects of the invention, it is preferred to disrupt or degrade the OM, cell wall or IM of a eubacterial minicell. Such treatments are used, by way of non-limiting example, in order to increase or decrease the immunogenicity, and/or to alter the permeability characteristics, of a minicell.

Eubacterial cells and minicells with altered membranes and/or cell walls are called "poroplasts™" "spheroplasts," and "protoplasts." Herein, the terms "spheroplast" and "protoplast" refer to spheroplasts and protoplasts prepared from minicells. In contrast, "cellular spheroplasts" and "cellular protoplasts" refer to spheroplasts and protoplasts prepared from cells. Also, as used herein, the term "minicell" encompasses not only minicells per se but also encompasses poroplasts™, spheroplasts and protoplasts.

In a poroplast, the eubacterial outer membrane (OM) and LPS have been removed. In a spheroplast, portions of a disrupted eubacterial OM and/or disrupted cell wall either may remain associated with the inner membrane of the minicell, but the membrane and cell wall is nonetheless porous because the permeability of the disrupted OM and cell wall has been increased. A membrane is said to be "disrupted" when the membrane's structure has been treated with an agent, or incubated under conditions, that leads to the partial degradation of the membrane, thereby increasing the permeability thereof. In contrast, a membrane that has been "degraded" is essentially, for the applicable intents and purposes, removed. In preferred embodiments, irrespective of the condition of the OM and cell wall, the eubacterial inner membrane is not disrupted, and membrane proteins displayed on the inner membrane are accessible to compounds that are brought into contact with the minicell, poroplast, spheroplast, protoplast or cellular poroplast, as the case may be.

### IV. ASSAYING MINICELLS

### IV.A. Efficiency of Minicell Production

The level of minicell production will vary and may be evaluated using methods described herein. Relatively high levels of minicell production are generally preferred. Conditions in which about 40% of cells are achromosomal have been reported (see, e.g., Hassan et al., Suppression of initiation defects of chromosome replication in Bacillus subtilis dnaA and oric-deleted mutants by integration of a plasmid replicon into the chromosomes, J Bacteriol 179:2494-502, 1997). Procedures for identifying strains that give high yields of minicells are known in the art; see, e.g., Clark-Curtiss and Curtiss III, Analysis of Recombinant DNA Using Escherichira coli Minicells, Meth. Enzol. 101:347-362, 1983.

Minicell production can be assessed by microscopic examination of late log-phase cultures. The ratio of minicells to normal cells and the frequency of cells actively producing minicells are parameters that increase with increasing minicell production.

### IV.B. Detecting Protein Synthesis in Minicells

Methods for detecting and assaying protein production are known in the art. See, e.g., Clark-Curtiss and Curtiss III, Meth Enzol 101:347-362, 1983. As an exemplary procedure, transformed *E. coli* minicell-producing cells are grown in LB broth with the appropriate antibiotic overnight. The following day the overnight cultures are diluted 1:50 in fresh media, and grown at 37°C to mid-log phase. If it is desired to eliminate whole cells, an antibiotic that kills growing (whole) cells but not quiescent cells (minicells) may be used. For example, in the case of cells that are not ampicillin resistant, ampicillin (100 mg per ml is added), and incubation is allowed to continue for about 2 more hrs. Cultures are then centrifuged twice at low speed to pellet most of the large cells. Minicells are pelleted by spinning 10 min at 10,000 rpm, and are then resuspended in M63 minimal media supplemented with 0.5% casamino acids, and 0.5 mM cAMP, or M9 minimal medium supplemented with 1 mM MgSO₄, 0.1 mM CaCl₂, 0.05% NaCl, 0.2% glucose, and 1 ng per ml thiamine. Labeled (³⁵S) methonine is added to the minicells for about 15 to about 90 minutes, and minicells are immediately collected afterwards by centrifugation for 10 min at 4°C and 14,000 rpm. Cells are resuspended in 50 to 100 µg Laemmeli-buffer, and disrupted by boiling and vortexing (2 min for each step). Incorporation of ³⁵S-methionine was determined by measuring the amount of radioactivity contained in 1 µl of the lysate after precipitation of proteins with trichloroacetic acid (TCA). Minicell lysates (50,000 to 100,000 cpm per lane) are subjected to PAGE on, e.g., 10% polyacrylamide gels in which proteins of known size are also run as molecular weight standards. Gels are fixed and images there of are generated by, e.g., autoradiography or any other suitable detection systems.

### V. GENETIC EXPRESSION IN MINICELLS

Various minicells of the invention use recombinant DNA expression systems to produce a non-eubacterial protein, which may be a membrane protein that is preferably "displayed" on the surface of minicells, a membrane protein that projects portions not associtiated with a membrane towards the interior of a minicell, or a soluble protein present in the exterior of the minicells. By "displayed" it is meant that a protein is present on the surface of a cell (or minicell) and is thus in contact with the external environment of the cell. Non-limiting examples of displayed exogenous proteins of the invention include mammalian receptors and fusion proteins comprising one or more transmembrane domains. In other aspects of the invention, minicells use expression elements to produce bioactive nucleic acids from templates therefor.

### V.A. Expression Systems

In vivo and in vitro protein expression systems provide a variety of techniques that allow scientists to transcribe and translate amino acid polypeptides proteins from recombinant DNA templates (Kaufman, Overview of vector design for mammalian gene expression. Mol Biotechnol, 2001. 16: 151-160; and Kozak, Initiation of translation in prokaryotes and eukaryotes. Gene, 1999. 234: 187-208).

Although minicells are virtually depleted of chromosomal DNA (Tudor et al., Presence of nuclear bodies in some minicells of Escherichia coli. J Bacteriol, 1969. 98: 298-299), it has been reported that minicells have all the elements required to express nucleotide sequences that are present in episomal expression elements therein (Levy, Very stable prokaryote messenger RNA in chromosomeless Escherichia coli minicells. Proc Natl Acad Sci USA, 1975. 72: 2900-2904; Hollenberg et al., Synthesis of high molecular weight polypeptides in Escherichia coli minicells directed by cloned Saccharomyces cerevisiae 2-micron DNA. Gene, 1976. 1: 33-47; Crooks et al., Transcription of plasmid DNA in Escherichia coli minicells. Plasmid, 1983. 10: 66-72; Clark-Curtiss, Analysis of recombinant DNA using Escherichia coli minicells. Methods Enzymol, 1983. 101: 347-362).

Preferred expression vectors and constructs according to the invention are episomal genetic elements. By "episomal" it is meant that the expression construct is not always linked to a cell's chromosome but may instead be retained or maintained in host cells as a distinct molecule entity. Minicells can retain, maintain and express episomal expression constructs such as, e.g., plasmids, bacteriophage, viruses and the like (Crooks et al., Plasmin 10:66-72, 1983; Clark-Curtiss, Methods Enzymology 101:347-62, 1983; Witkiewicz et al., Acta. Microbiol. Pol. A 7:21-24, 1975; Ponta et al., Nature 269:440-2, 1977). By "retained" it is meant that the episomal expression construct is at least temporarily present and expressed in a host parent cell and/or minicell; by "maintained" it is meant that the episomal expression construct is capable of autonomous replication within a host parent cell and/or minicell. In the context of episomal elements, the term "contained" encompasses both "retained" and "maintained." A preferred type of an episomal element according to the invention is one that is always an extrachromocomal element, or which is part of a chromosome but becomes an extrachromosomal element before or during minicell production.

The fact that minicells do not contain chromosomal DNA but do contain episomal expression elements, such as plasmids, that can be used as templates for RNA synthesis means that the only proteins that are actively produced in minicells are those that are encoded by the expression elements that they contain. Minicell-producing *E. coli* cells can be made competent and transformed with expression elements that direct the expression of proteins encoded by the expression elements. An expression element segregates into minicells as they are produced. In isolated minicells that contain expression elements, there is a single DNA template RNA for transcription. Therefore, the only nucleic acids and proteins that are actively produced (expressed) by minicells are those that are encoded by sequences on the expression vector. In the context of the invention, sequences that encode amino acid sequences are designated "open reading frames" or "ORFs." One feature of minicell expression systems of interest as regards the present invention is that endogenous (i.e., chromosomally located) genes are not present and are thus not expressed, whereas genes present on the episomal element are expressed (preferably over-expressed) in the minicells. As a result, the amount of endogenous proteins, including membrane proteins, decreases as the minicells continue to express genes located on episomal expression constructs.

The minicell system can reduce or eliminate undesirable features associated with the transcription and translation of endogenous proteins from the *E. coli* chromosome. For example, expression of proteins in minicell systems results in low background signal ("noise") when radiolabeled proteins produced using recombinant DNA technology (Jannatipour et al., Translocation of Vibrio Harveyi N,N'-Dlacetylchitobiase to the outer membrane of Escherichia coli. J. Bacteriol, 1987. 169: 3785-3791). A high background signal can make it difficult to detect a protein of interest. In whole cell *E. coli* systems, endogenous proteins (encoded by the bacterial chromosome) are labeled as well as the protein(s) encoded by the expression element; whereas, in minicell systems, only the proteins encoded by the expression element in the minicells are labeled.

### V.B. Modulating Genetic Expression in Minicells

Gene expression in minicells, and/or in minicell-producing (parent) cells, involves the coordinated activity of a variety of expression factors, regulatory elements and expression sequences. Any of these may be modified to alter the extent, timing or regulation of expression of a gene of interest in minicells and/or their parent cells. Often, the goal of the manipulations is to increase the efficiency of protein production in minicells. However, increased expression may, in some instances, desirably include increased or "tight" negative regulation. This may reduce or eliminate selective pressure created by toxic gene products, and allow for functional expression in a controlled fashion by removing the negative regulation and/or inducing expression of the gene product at a preselected time. By way of non-limiting example, these techniques may include modification or deletion of endogenous gene(s) from which their respective gene product decreases the induction and expression efficiency of a desired protein in the parent cell prior to minicell formation and/or the segregated minicell. By way of non-limiting example, these protein components may be the enzymes that degrade chemical inducers of expression, proteins that have a dominant negative affect upon a positive regulatory elements, proteins that have proteolytic activity against the protein to be expressed, proteins that have a negative affect against a chaperone that is required for proper activity of the expressed protein, and/or this protein may have a positive effect upon a protein that either degrades or prevents the proper function of the expressed protein. These gene products that require deletion or modification for optimal protein expression and/or function may also be antisense nucleic acids that have a negative affect upon gene expression.

### VI. FUSION (CHIMERIC) PROTEINS

In certain aspects of the invention, a fusion protein is expressed and displayed by minicells. One class of fusion proteins of particular interest are those that are displayed on the surface of minicells, e.g., fusion proteins comprising one or more transmembrane domains. Types of displayed fusion proteins of particular interest are, by way of non-limiting example, those that have an extracellular domain that is a binding moiety or an enzymatic moiety. By way of non-limiting example, the fusion protein ToxR-PhoA has been expressed in and displayed on the surface of minicells. The ToxR-PhoA fusion protein comprises a polypeptide corresponding to the normally soluble enzyme, alkaline phosphatase, anchored to the minicell membrane by the single transmembrane domain of ToxR (see the Examples). The fusion protein retains the activity of the enzyme in the context of the minicell membrane in which it is bound. Nearly all of the fusion protein is oriented so that the enzyme's catalytic domain is displayed on the outer surface of the minicell.

### VI.A. Generation of Fusion Proteins

Polypeptides, which are polymers of amino acids, are encoded by another class of molecules, known as nucleic acids, which are polymers of structural units known as nucleotides. In particular, proteins are encoded by nucleic acids known as DNA and RNA (deoxyribonucleic acid and ribonucleic acid, respectively).

The nucleotide sequence of a nucleic acid contains the "blueprints" for a protein. Nucleic acids are polymers of nucleotides, four types of which are present in a given nucleic acid. The nucleotides in DNA are adenine, cytosine and guanine and thymine, (represented by A, C, G, and T respectively); in RNA, thymine (T) is replaced by uracil (U). The structures of nucleic acids are represented by the sequence of its nucleotides arranged in a 5' ("5 prime") to 3' ("3 prime") direction, e.g.,
5'-A-T-G-C-C-T-A-A-A-G-C-C-G-C-T-C-C-C-T-C-A-3'

In biological systems, proteins are typically produced in the following manner. A DNA molecule that has a nucleotide sequence that encodes the amino acid sequence of a protein is used as a template to guide the production of a messenger RNA (mRNA) that also encodes the protein; this process is known as transcription. In a subsequent process called translation, the mRNA is "read" and directs the synthesis of a protein having a particular amino acid sequence.

Each amino acid in a protein is encoded by a series of three contiguous nucleotides, each of which is known as a codon. In the "genetic code," some amino acids are encoded by several codons, each codon having a different sequence; whereas other amino acids are encoded by only one codon sequence. An entire protein (i.e., a complete amino acid sequence) is encoded by a nucleic acid sequence called a reading frame. A reading frame is a continuous nucleotide sequence that encodes the amino acid sequence of a protein; the boundaries of a reading frame are defined by its initiation (start) and termination (stop) codons.

The process by which a protein is produced from a nucleic acid can be diagrammed as follows:

DNA (A-T-G)-(A-A-G)-(C-C-G)-(C-T-C)-(C-C-T)- ... (etc.)
↓ Transcription
RNA (A-U-G)-(A-A-G)-(C-C-G)-(C-U-C)-(C-C-U)- ... (etc.)
↓ Translation
Protein Met - Pro - Lys - Ala - Ala - ... (etc.)

A chimeric reading frame encoding a fusion protein is prepared as follows. A "chimeric reading frame" is a genetically engineered reading frame that results from the fusion of two or more normally distinct reading frames, or fragments thereof, each of which normally encodes a separate polypeptide. Using recombinant DNA techniques, a first reading frame that encodes a first amino acid sequence is linked to a second reading frame that encodes a second amino acid sequence in order to generate a chimeric reading frame. Chimeric reading frames may also include nucleotide sequences that encode optional fusion protein elements (see below).

A hypothetical example of a chimeric reading frame created from two normally separate reading frames is depicted in the following flowchart.

### First Open Reading Frame and "Protein-1":

DNA-1 (A-T-G)-(A-A-G)-(C-C-G)-(C-T-C)-(C-C-T)- ... (etc.)
↓ Transcription
RNA-1 (A-U-G)-(A-A-G)-(C-C-G)-(C-U-C)-(C-C-U)- ... (etc.)
↓ Translation
Protein-1 Met - Pro - Lys - Ala - Ala - ... (etc.)

### Second Open Reading Frame and "Protein-2":

DNA-2 (T-G-G)-(G-T-T)-(A-C-T)-(C-A-C)-(T-C-A)- ... (etc.)
↓ Transcription
RNA-2 (U-G-G)-(G-U-U)-(A-C-U)-(C-A-C)-(U-C-A)- ... (etc.)
↓ Translation
Protein-2 Trp - Val - Thr - His - Ser - ... (etc.)

### Chimeric Reading Frame that encodes a Fusion Protein having sequences from Protein-1 and Protein-2:

DNA-Chimera (A-T-G)-(A-A-G)-(C-C-G)-(C-A-C)-(T-C-A)-(etc.)
↓ Transcription
RNA-Chimera (A-U-G)-(A-A-G)-(C-C-G)-(C-A-C)-(U-C-A)-(etc.)
↓ Translation
Fusion Protein Met - Pro - Lys - His - Ser - (etc.)

In order for a chimeric reading frame to be functional, each normally distinct reading frame therein must be fused to all of the other normally distinct reading frames in a manner such that all of the reading frames are in frame with each other. By "in frame with each other" it is meant that, in a chimeric reading frame, a first nucleic acid having a first reading frame is covalently linked to a second nucleic acid having a second reading frame in such a manner that the two reading frames are "read" (translated) in register with each other. As a result, the chimeric reading frame encodes one extended amino acid sequence that includes the amino acid sequences encoded by each of the normally separate reading frames. A fusion protein is thus encoded by a chimeric reading frame.

The fusion proteins of the invention are used to display polypeptides on minicells. The fusion proteins comprise (1) at least one polypeptide that is desired to be displayed by minicells (a "displayed polypeptide") and (2) at least one membrane polypeptide, e.g., a transmembrane or a membrane anchoring domain. For various aspects of the invention, optional fusion protein elements, as defined herein, may also be included if required or desired.

### VI.B. Optional Fusion Protein Elements

The fusion proteins of the invention may optionally comprise one or more non-biologically active amino acid sequences, i.e., optional fusion protein elements. Such elements include, but are not limited to, the following optional fusion protein elements. It is understood that a chimeric reading frame will include nucleotide sequences that encode such optional fusion protein elements, and that these nucleotide sequences will be positioned so as to be in frame with the reading frame encoding the fusion protein. Optional fusion protein elements may be inserted between the displayed polypeptide and the membrane polypeptide, upstream or downstream (amino proximal and carboxy proximal, respectively) of these and other elements, or within the displayed polypeptide and the membrane polypeptide. A person skilled in the art will be able to determine which optional element(s) should be included in a fusion protein of the invention, and in what order, based on the desired method of production or intended use of the fusion protein.

Detectable polypeptides are optional fusion protein elements that either generate a detectable signal or are specifically recognized by a detectably labeled agent. An example of the former class of detectable polypeptide is green fluorescent protein (GFP). Examples of the latter class include epitopes such as a "His tag" (6 contiguous His residues, a.k.a. 6xHis), the "FLAG tag" and the c-myc epitope. These and other epitopes can be detected using labeled antibodies that are specific for the epitope. Several such antibodies are commercially available.

Attachment (support-binding) elements are optionally included in fusion proteins and can be used to attach minicells displaying a fusion protein to a preselected surface or support. Examples of such elements include a "His tag," which binds to surfaces that have been coated with nickel; streptavidin or avidin, which bind to surfaces that have been coated with biotin or "biotinylated" (see U.S. Patent 4,839,293 and Airenne et al., Protein Expr. Purif. 17:139-145, 1999); and glutathione-s-transferase (GST), which binds to surfaces coated with glutathione (Kaplan et al., Protein Sci. 6:399-406, 1997; U.S. Patent 5,654,176). Polypeptides that bind to lead ions have also been described (U.S. Patent 6,111,079).

Spacers (a.k.a. linkers) are amino acid sequences that are optionally included in a fusion protein in between other portions of a fusion protein (e.g., between the membrane polypeptide and the displayed polypeptide, or between an optional fusion protein element and the remainder of the fusion protein). Spacers can be included for a variety of reasons. For example, a spacer can provide some physical separation between two parts of a protein that might otherwise interfere with each other via, e.g., steric hindrance. The ability to manipulate the distance between the membrane polypeptide and the displayed polypeptide allows one to extend the displayed polypeptide to various distances from the surface of minicells.

### VI.C. Interactions with Receipient Cells

Many Gram-negative pathogens use a type III secretion machine to translocate protein toxins across the bacterial cell envelope (for a review, see Cheng LW, Schneewind O. Type III machines of Gram-negative bacteria: delivering the goods. Trends Microbiol 2000 May;8(5):214-20). For example, pathogenic Yersinia spp. export over a dozen Yop proteins via a type III mechanism, which recognizes secretion substrates by signals encoded in yop mRNA or chaperones bound to unfolded Yop proteins. A 70-kb virulence plasmid found in pathogenic Yersinia spp. to survive and multiply in the lymphoid tissues of the host. The virulence plasmid encodes the Yop virulon, an integrated system allowing extracellular bacteria to inject bacterial proteins into cells. The Yop virulon comprises a variety of Yop proteins and a dedicated type III secretion apparatus, called Ysc (for a review, see Cornelis GR, Boland A, Boyd AP, Geuijen C, Iriarte M, Neyt C, Sory MP, Stainier I. The virulence plasmid of Yersinia, an antihost genome. Microbiol Mol Biol Rev 1998 62(4):1315-52).

### VII. MINICELL DISPLAY

In a related, non-limiting embodiment of the invention, minicells expressing a particular antigen (e.g., protein, carbohydrate, small molecule, lipid) on their surfaces (described elsewhere in this application) are used to generate an immunogenic response. The advantages of presenting an antigen on the surfaces of minicells are that the minicells themselves may be an adjuvant that stimulates the immune response, particularly if administered subcutaneously (SC) or intramuscularly (IM). Moreover, the minicells are not readily eliminated by the renal system and are present in the circulatory system of an immunized animal for a longer time. In addition, small molecules could be tethered to the minicell in a way that presents the desired moiety of the molecule. Animals are presented with minicell-based immunogens, and the antibodies produced in the animals are prepared and used in therapeutic, diagnostic, research and screening applications. Although this aspect of the invention may be used to make antibodies to any molecule displayed on their surface, the extracellular domains of membrane proteins are of particular interest.

### VIII. APTAMERS

Traditionally, techniques for detecting and purifying target molecules have used polypeptides, such as antibodies, that specifically bind such targets. While nucleic acids have long been known to specifically bind other nucleic acids (e.g., ones having complementary sequences), aptamers (i.e., nucleic acids that bind non-nucleic target molecules) have been disclosed. See, e.g., Blackwell et al., Science (1990) 250:1104-1110; Blackwell et al., Science (1990) 250:1149-1152; Tuerk et al., Science (1990) 249:505-510; Joyce, Gene (1989) 82:83-87; and U.S. Patent 5,840,867 entitled "Aptamer analogs specific for biomolecules".

As applied to aptamers, the term "binding" specifically excludes the "Watson-Crick"-type binding interactions (i.e., A:T and G:C base-pairing) traditionally associated with the DNA double helix. The term "aptamer" thus refers to a nucleic acid or a nucleic acid derivative that specifically binds to a target molecule, wherein the target molecule is either (i) not a nucleic acid, or (ii) a nucleic acid or structural element thereof that is bound through mechanisms other than duplex- or triplex-type base pairing. Such a molecule is called a "non-nucleic molecule" herein.

### VIII.A. Structures of Nucleic Acids

"Nucleic acids," as used herein, refers to nucleic acids that are isolated a natural source; prepared in vitro, using techniques such as PCR amplification or chemical synthesis; prepared in vivo, e.g., via recombinant DNA technology; or by any appropriate method. Nucleic acids may be of any shape (linear, circular, etc.) or topology (single-stranded, double-stranded, supercoiled, etc.). The term "nucleic acids" also includes without limitation nucleic acid derivatives such as peptide nucleic acids (PNA's) and polypeptide-nucleic acid conjugates; nucleic acids having at least one chemically modified sugar residue, backbone, internucleotide linkage, base, nucleoside, or nucleotide analog; as well as nucleic acids having chemically modified 5' or 3' ends; and nucleic acids having two or more of such modifications. Not all linkages in a nucleic acid need to be identical.

Nucleic acids that are aptamers are often, but need not be, prepared as oligonucleotides.
Oligonucleotides include without limitation RNA, DNA and mixed RNA-DNA molecules having sequences of lengths that have minimum lengths of 2, 4, 6, 8, 10, 11, 12, 13, 14, 15, 17, 18, 19, 20, 21, 22, 23, 24 or 25 nucleotides, and maximum lengths of about 100, 75, 50, 40, 25, 20 or 15 or more nucleotides, irrespectively. In general, a minimum of 6 nucleotides, preferably 10 nucelotides, more preferably 14 to 20 nucleotides, is necessary to effect specific binding.

In general, the oligonucleotides may be single-stranded (ss) or double-stranded (ds) DNA or RNA, or conjugates (e.g., RNA molecules having 5' and 3' DNA "clamps") or hybrids (e.g., RNA:DNA paired molecules), or derivatives (chemically modified forms thereof). However, single-stranded DNA is preferred, as DNA is often less labile than RNA. Similarly, chemical modifications that enhance an aptamer's specificity or stability are preferred.VIII.B. Chemical Modifications of Nucleic Acids

Chemical modifications that may be incorporated into aptamers and other nucleic acids include, with neither limitation nor exclusivity, base modifications, sugar modifications, and backbone modifications.

Base modifications: The base residues in aptamers may be other than naturally occurring bases (e.g., A, G, C, T, U, 5MC, and the like). Derivatives of purines and pyrimidines are known in the art; an exemplary but not exhaustive list includes aziridinylcytosine, 4-acetylcytosine, 5-fluorouracil, 5-bromouracil, 5-carboxymethylaminomethyl-2-thiouracil, 5-carboxymethylaminomethyluracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine (5MC), N6-methyladenine, 7-methylguanine, 5-methylaminomethyluracil, 5-methoxyaminomethyl-2-thiouracil, beta-D-mannosylqueosine, 5-methoxyuracil, 2-methylthio-N-6-isopentenyladenine, uracil-5-oxyacetic acid methylester, pseudouracil, queosine, 2-thiocytosine, 5-methyl-2-thiouracil, 2-thiouracil, 4-thiouracil, 5-methyluracil, uracil-5-oxyacetic acid, and 2,6-diaminopurine. In addition to nucleic acids that incorporate one or more of such base derivatives, nucleic acids having nucleotide residues that are devoid of a purine or a pyrimidine base may also be included in aptamers.

Sugar modifications: The sugar residues in aptamers may be other than conventional ribose and deoxyribose residues. By way of non-limiting example, substitution at the 2'-position of the furanose residue enhances nuclease stability. An exemplary, but not exhaustive list, of modified sugar residues includes 2' substituted sugars such as 2'-O-methyl-, 2'-O-alkyl, 2'-O-allyl, 2'-S-alkyl, 2'-S-allyl, 2'-fluoro-, 2'-halo, or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs and abasic nucleoside analogs such as methyl riboside, ethyl riboside or propylriboside.

Backbone modifications: Chemically modified backbones include, by way of non-limiting example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Chemically modified backbones that do not contain a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages, including without limitation morpholino linkages; siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; and amide backbones.

### IX. POLYPEPTIDIC BINDING MOIETIES

A variety of binding moities can be attached to a minicell of the invention for a variety of purposes. In a preferred embodiment, the binding moiety is directed to a ligand that is displayed by a cell into which it is desired to deliver the therapeutic content of a minicell.

### IX.A. Antibodies and Antibody Derivatives

The term "antibody" is meant to encompass an immunoglobulin molecule obtained by in vitro or in vivo generation of an immunogenic response, and includes polyclonal, monospecific and monoclonal antibodies, as well as antibody derivatives, e.g single-chain antibody fragments (scFv). An "immunogenic response" is one that results in the production of antibodies directed to one or more proteins after the appropriate cells have been contacted with such proteins, or polypeptide derivatives thereof, in a manner such that one or more portions of the protein function as epitopes. An epitope is a single antigenic determinant in a molecule. In proteins, particularly denatured proteins, an epitope is typically defined and represented by a contiguous amino acid sequence. However, in the case of nondenatured proteins, epitopes also include structures, such as active sites, that are formed by the three-dimensional folding of a protein in a manner such that amino acids from separate portions of the amino acid sequence of the protein are brought into close physical contact with each other.

Wildtype antibodies have four polypeptide chains, two identical heavy chains and two identical light chains. Both types of polypeptide chains have constant regions, which do not vary or vary minimally among antibodies of the same class (*i.e*., IgA, IgM, etc.), and variable regions. Variable regions are unique to a particular antibody and comprise an "antigen binding domain" that recognizes a specific epitope. Thus, an antibody's specificity is determined by the variable regions located in the amino terminal regions of the light and heavy chains.

As used herein, the term "antibody" encompasses derivatives of antibodies such as antibody fragments that retain the ability to specifically bind to antigens. Such antibody fragments include Fab fragments (*i.e*., an antibody fragment that contains the antigen-binding domain and comprises a light chain and part of a heavy chain bridged by a disulfide bond); Fab' (an antibody fragment containing a single anti-binding domain comprising an Fab and an additional portion of the heavy chain through the hinge region); F(ab')2 (two Fab' molecules joined by interchain disulfide bonds in the hinge regions of the heavy chains; the Fab' molecules may be directed toward the same or different epitopes); a bispecific Fab (an Fab molecule having two antigen binding domains, each of which may be directed to a different epitope); a single chain Fab chain comprising a variable region, a.k.a., a sFv (the variable, antigen-binding determinative region of a single light and heavy chain of an antibody linked together by a chain of about 10 to about 25 amino acids).

The term "antibody" includes antibodies and antibody derivatives that are produced by recombinant DNA techniques and "humanized" antibodies. Humanized antibodies have been modified, by genetic manipulation and/or in vitro treatment to be more human, in terms of amino acid sequence, glycosylation pattern, etc., in order to reduce the antigenicity of the antibody or antibody fragment in an animal to which the antibody is intended to be administered (Gussow et al., Methods Enz. 203:99-121, 1991).

A single-chain antibody (scFv) is a non-limiting example of a binding moiety that may be displayed on minicells. Single-chain antibodies are produced by recombinant DNA technology and may be incorporated into fusion proteins. The term "single chain" denotes the fact that scFv's are found in a single polypeptide. In contrast, wildtype antibodies have four polypeptide chains, two identical heavy chains and two identical light chains. Both types of polypeptide chains have constant regions, which do not vary or vary minimally among antibodies of the same class (*i.e.*, IgA, IgM, etc.), and variable regions. An antibody's specificity is determined by the variable regions located in the amino terminal regions of the light and heavy chains. The variable regions of a light chain and associated heavy chain form an "antigen binding domain" that recognizes a specific epitope. In a single chain antibody, the amino acid sequences of the variable light and variable heavy regions of an antibody are present in one comtiguous polypeptide. Methods of producing single chain antibodies are known in the art. See, for example, U.S. Patents 4,946,778; 5,260,203; 5,455,030; 5,518,889; 5,534,621; 5,869,620; 6,025,165; 6,027,725 and 6,121,424.

Antibody derivatives and other polypeptides that are binding moieties can be isolated from protein display libraries, in which a library of candidate binding agents is displayed on a phage or other agent that comprises a nucleic acid encoding the protein it displays. Thus, an agent that binds to the target compound can be isolated, and nucleic acid prepared therefrom, providing for the rapid isolation of binding moieties and nucleic acids that can be used to produce them. For reviews, see Benhar I. Biotechnological applications of phage and cell display. Biotechnology Adv. 2001 (19):1-33; FitzGerald K. In vitro display technologies - new tools for drug discovery. Drug Discov Today. 2000 5(6):253-258; and Hoogenboom HR, Chames P. Natural and designer binding sites made by phage display technology. Immunol Today. 2000 Aug;21 (8):371-8.

A variety of protein display systems are known in the art and include various phage display systems such as those described in Jung S, Arndt K, Müller K, Plückthyn A. Selectively infective phage (SIP) technology: scope and limitations. J Immunol Methods. 1999 (231):93-104; Katz B. Structural and mechanistic determinants of affinity and specificity of ligands discovered or engineered by phage display. Annu Rev Biophys Biomol Struct. 1997 (26):27-45; Forrer P. Jung S, Pluckthun A. Beyond binding: using phage display to select for structure, folding and enzymatic activity in proteins. Curr Opin Struct Biol. 1999 Aug;9(4):514-20; Rondot S, Koch J, Breitling F, Dubel S. A helper phage to improve single-chain antibody presentation in phage display. Nat Biotechnol. 2001 Jan;19(1):75-8. Giebel LB, Cass RT, Milligan DL, Young DC, Arze R, Johnson CR. Screening of cyclic peptide phage libraries identifies ligands that bind streptavidin with high affinities. Biochemistry. 1995 Nov 28;34(47):15430-5; de Kruif J, Logtenberg T. Leucine zipper dimerized bivalent and bispecific scFv antibodies from a semi-synthetic antibody phage display library. J Biol Chem. 1996 Mar 29;271(13):7630-4; Hoogenboom HR, Henderikx P, de Haard H. Creating and engineering human antibodies for immunotherapy. Adv Drug Deliv Rev. 1998 Apr 6;31(1-2):5-31; Helfrich W, Haisma HJ, Magdolen V, Luther T, Bom VJ, Westra J, van der Hoeven R, Kroesen BJ, Molema G, de Leij L. A rapid and versatile method for harnessing scFv antibody fragments with various biological effector functions. J Immunol Methods. 2000 Apr 3;237(1-2):131-45; Hoess RH. Bacteriophage lambda as a vehicle for peptide and protein display. Curr Pharm Biotechnol 2002 Mar;3(1):23-8; Baek H, Suk KH, Kim YH, Cha S. An improved helper phage system for efficient isolation of specific antibody molecules in phage display. Nucleic Acids Res. 2002 Mar 1;30(5):e18; and Rondot S, Koch J, Breitling F, Dubel S. A helper phage to improve single-chain antibody presentation in phage display. Nat Biotechnol. 2001 Jan;19(1):75-8.

Other display systems include without limitation "Yeast Display" (Curr Opin Biotechnol 1999 Oct;10(5):422-7. Applications of yeast in biotechnology: protein production and genetic analysis. Cereghino GP, Cregg JM.); "Baculovirus Display" (Kost TA, Condreay JP. Recombinant baculoviruses as expression vectors for insect and mammalian cells. Curr Opin Biotechnol. 1999 Oct;10(5):428-33; and Liang M, Dubel S, Li D, Queitsch I, Li W, Bautz EK. Baculovirus expression cassette vectors for rapid production of complete human IgG from phage display selected antibody fragments. J Immunol Methods. 2001 Jan 1;247(1-2):119-30); "Ribosome Display" (Hanes J, Schaffitzel C, Knappik A, Pluckthun A. Picomolar affinity antibodies from a fully synthetic naive library selected and evolved by ribosome display. Nat Biotechnol. 2000 Dec;18(12):1287-92; Hanes J, Jermutus L, Pluckthun A. Selecting and evolving functional proteins in vitro by ribosome display. Methods Enzymol. 2000;328:404-30; Schaffitzel C, Hanes J, Jermutus L, Pluckthun A. Ribosome display: an in vitro method for selection and evolution of antibodies from libraries. J Immunol Methods. 1999 Dec 10;231(1-2):119-35; Hanes J, Jermutus L, Weber-Bornhauser S, Bosshard HR, Pluckthun A. Ribosome display efficiently selects and evolves high-affinity antibodies in vitro from immune libraries. Proc Natl Acad Sci U S A. 1998 Nov 24;95(24):14130-5; Hanes J, Pluckthun A. In vitro selection and evolution of functional proteins by using ribosome display. Proc Natl Acad Sci U S A. 1997 May 13;94(10):4937-42; Coia G, Pontes-Braz L, Nuttall SD, Hudson PJ, Irving RA. Panning and selection of proteins using ribosome display. J Immunol Methods. 2001 Aug 1;254(1-2):191-7.; Irving RA, Coia G, Roberts A, Nuttall SD, Hudson PJ. Ribosome display and affinity maturation: from antibodies to single V-domains and steps towards cancer therapeutics. J Immunol Methods. 2001 Feb 1;248(1-2):31-45); and "Bacterial Display" (Hoischen C, Fritsche C, Gumpert J, Westermann M, Gura K, Fahnert B. Novel bacterial membrane surface display system using cell wallless L-forms of Proteus mirabilis and Escherichia coli. Appl Environ Microbiol. 2002 Feb;68(2):525-31; Etz H, Minh DB, Schellack C, Nagy E, Meinke A. Bacterial phage receptors, versatile tools for display of polypeptides on the cell surface. J Bacteriol. 2001 Dec;183(23):6924-35; Patel D, Vitovski S, Senior HJ, Edge MD, Hockney RC, Dempsey MJ, Sayers JR. Continuous affinity-based selection: rapid screening and simultaneous amplification of bacterial surface-display libraries. Biochem J. 2001 Aug 1;357(Pt 3):779-85; Lang H. Outer membrane proteins as surface display systems. Int J Med Microbiol. 2000 Dec;290(7):579-85; Earhart CF. Use of an Lpp-OmpA fusion vehicle for bacterial surface display. Methods Enzymol. 2000;326:506-16; Benhar I, Azriel R, Nahary L, Shaky S, Berdichevsky Y, Tamarkin A, Wels W. Highly efficient selection of phage antibodies mediated by display of antigen as Lpp-OmpA' fusions on live bacteria. J Mol Biol. 2000 Aug 25;301(4):893-904; Xu Z, Lee SY. Display of polyhistidine peptides on the Escherichia coli cell surface by using outer membrane protein C as an anchoring motif. Appl Environ Microbiol. 1999 Nov;65(11):5142-7; Daugherty PS, Olsen MJ, Iverson BL, Georgiou G. Development of an optimized expression system for the screening of antibody libraries displayed on the Escherichia coli surface. Protein Eng. 1999 Jul;12(7):613-21; Chang HJ, Sheu SY, Lo SJ. Expression of foreign antigens on the surface of Escherichia coli by fusion to the outer membrane protein traT. J Biomed Sci. 1999 Jan;6(1):64-70; Maurer J, Jose J, Meyer TF. Autodisplay: one-component system for efficient surface display and release of soluble recombinant proteins from Escherichia coli. J Bacteriol. 1997 Feb;179(3):794-804.

Antibodies, particularly single-chain antibodies, directed to surface antigens specific for a particular cell type may also be used as cell- or tissue-specific targeting elements. Single-chain antibody amino acid sequences have been incorporated into a variety of fusion proteins, including those with transmembrane domains and/or membrane-anchoring domains. See, for example, Kuroki et al., "Specific Targeting Strategies of Cancer Gene Therapy Using a Single-Chain Variable Fragment (scFv) with a High Affinity for CEA," Anticancer Res., pp. 4067-71, 2000; U.S. Patent 6,146,885, to Dornburg, entitled "Cell-Type Specific Gene Transfer Using Retroviral Vectors Containing Antibody-Envelope Fusion Proteins"; Jiang et al., "In Vivo Cell Type-Specific Gene Delivery With Retroviral Vectors That Display Single Chain Antibodies," Gene Ther. 1999, 6:1982-7; Engelstadter et al., "Targeting Human T Cells By Retroviral Vectors Displaying Antibody Domains Selected From A Phage Display Library," Hum. Gene Ther. 2000, 11:293-303; Jiang et al., "Cell-Type-Specific Gene Transfer Into Human Cells With Retroviral Vectors That Display Single-Chain Antibodies," J. Virol 1998,72:10148-56; Chu et al., "Toward Highly Efficient Cell-Type-Specific Gene Transfer With Retroviral Vectors Displaying Single-Chain Antibodies," J. Virol 1997, 71:720-5; Chu et al., "Retroviral Vector Particles Displaying The Antigen-Binding Site Of An Antibody Enable Cell-Type-Specific Gene Transfer," J. Virol 1995, 69:2659-63; Chu et al., "Cell Targeting With Retroviral Vector Particles Containing Antibody-Envelope Fusion Proteins," Gene Ther. 1994, 1:292-9; Esshar et al., "Specific activation and targeting of cytotoxic lymphocytes through chimeric single chains consisting of antibody-binding domains and the □ or 0 subunits of the immunoglobulin and T-cell receptors," Proc. Natl. Acad. Sci. USA, 1993, Vol. 90:720-724; Einfeld et al., "Construction of a Pseudoreceptor That Mediates Transduction by Adenoviruses Expressing a Ligand in Fiber or Penton Base," J. Virol. 1999, 73:9130-9136; Marin et al., "Targeted Infection of Human Cells via Major Histocompatibility Complex Class I Molecules by Moloney Murine Leukemia Virus-Derived Viruses Displaying Single-Chain Antibody Fragment-Envelope Fusion Proteins," J. Virol., 1996, 70:2957-2962; Somia et al., "Generation of targeted retroviral vectors by using single-chain variable fragment: An approach to in vivo gene delivery," Proc. Natl. Acad. Sci. USA, 1995, 92:7570-7574; Liu et al., "Treatment of B-Cell Lymphoma With Chimeric IgG and Single-Chain Fv Antibody-Interleukin-2 Fusion Proteins," Blood, 1998, 92:2103-2112; Martin et al., "Retrovirus Targeting by Tropism Restriction to Melanoma Cells," J. Virol., 1999, 73:6923-6929; Ramjiawan et al., "Noninvasive Localization of Tumors by Immunofluorescence Imaging Using a Single Chain Fv Fragment of a Human Monoclonal Antibody with Broad Cancer Specificity," Amer. Cancer Society, 2000, 89:1134-1144; Snitkovsky et al., "A TVA-Single-Chain Antibody Fusion Protein Mediates Specific Targeting of a Subgroup A Avian Leukosis Virus Vector to Cells Expressing a Tumor-Specific Form of Epidermal Growth Factor Receptor," J. Virol., 2000, 74:9540-9545; Chu et al., "Toward Highly Efficient Cell-Type-Specific Gene Transfer with Retroviral Vectors Displaying Single-Chain Antibodies," J. Virol., 1997, 71:720-725; Kulkarni et al.,Programmed cell death signaling via cell-surface expression of a single-chain antibody transgene,Transplantation 2000 Mar 27;69(6):1209-17.

### IX.B. Nucleic Acid Binding Domains

Nucleic acid binding polypeptide domains may bind nucleic acids in a sequence-dependent or sequence- independent fashion and/or in a manner that is specific for various nucleic acids having different chemical structures (e.g., single- or double-stranded DNA or RNA, RNA:DNA hybrid molecules, etc.). Non-limiting examples of membrane-based transcription factors and DNA-binding protein include Smad proteins (Miyazono et al., TGF-beta signaling by Smad proteins (Review), Adv Immunol 75:115-57, 2000); SREBPs (sterol regulatory element binding proteins) (Ye et al., Asparagine-proline sequence within membrane-spanning segment of SREBP triggers intramembrane cleavage by site-2 protease, Proc Natl Acad Sci USA 97:5123-8, 2000; Shimomura et al., Cholesterol feeding reduces nuclear forms of sterol regulatory element binding proteins in hamster liver, Proc Natl Acad Sci USA 94:12354-9, 1997; Brown and Goldstein, The SREBP pathway: regulation of cholesterol metabolism by proteolysis of a membrane-bound transcription factor (Review), Cell 89:331-40, 1997; Scheek et al., Sphingomyelin depletion in cultured cells blocks proteolysis of sterol regulatory element binding proteins at site 1, Proc Natl Acad Sci USA 94:11179-83, 1997); mitochondrial DNA-binding membrane proteins, e.g., Abf2p and YhmZp (Cho et al., A novel DNA-binding protein bound to the mitochondrial inner membrane restores the null mutation of mitochondrial histone Abf2p in Saccharomyces cerevisiae, Mol Cell Biol 18:5712-23, 1998); and bacterial DNA-binding membrane proteins (Smith et al., Transformation in Bacillus subtilis: purification and partial characterization of a membrane-bound DNA-binding protein., J Bacteriol 156:101-8, 1983).

### IX.C. Attaching Binding Moities, or Other Compounds, to Minicells

Binding compounds or moieties can be chemically attached (conjugated) to minicells via membrane proteins that are displayed on the minicells. The compound to be conjugated to minicells (the "attachable compound") may of any chemical composition, i.e., a small molecule, a nucleic acid, a radioisotope, a lipid or a polypeptide. One type of attachable compound that can be covalently attached to minicells is a binding moitiety, e.g., an antibody or antibody derivative. Another non-limiting example of attachable compounds is polyethylene glycol ("PEG"), which lowers the uptake in vivo of minicells by the reticuloendothelical system (RES). Another non-limiting example of creating stealth minicells to avoid the RES is to express proteins or other molecules on the surfaces of minicells whose lipid compositions have been modified, such as anionic lipid-rich minicells.

By way of non-limiting example, it is possible to prepare minicells that express transmembrane proteins with cysteine moieties on extracellular domains. Linkage of the membrane protein may be achieved through surface cysteinyl groups by, e.g., reduction with cysteinyl residues on other compounds to form disulfide bridges (S=S). If appropriate cysteinyl residues are not present on the membrane protein they may be introduced by genetic manipulation. The substitution of cysteine for another amino acid may be achieved by methods well-known to those skilled in the art, for example, by using methods described in Maniatis, Sambrook, and Fritsch (Molecular Cloning: A Laboratory Manual, Cold Spring Harbor Laboratory Press, 1989). As a non-limiting example, bioactive lysosphingolipids (e.g., sphingosine, sphingosine-1-phosphate, sphingosylphosphoryl choline) are covalently linked to proteins expressed on the surfaces of minicells such that these bioactive lipids are on the surface of the minicells and accessible for therapeutic or diagnostic uses in vivo or in vitro.

When the attachable moiety and the membrane protein both have a reduced sulfhydryl group, a homobifunctional cross-linker that contains maleimide, pyridyl disulfide, or beta-alpha-haloacetyl groups may be used for cross-linking. Examples of such cross-linking reagents include, but are not limited to, bismaleimidohexane (BMH) or 1,4-Di-[3'-(2'-pyridyldithio)propionamido]butane (DPDPB). Alternatively, a heterobifunctional cross-linker that contains a combination of maleimide, pyridyl disulfide, or beta-alpha-haloacetyl groups can be used for cross-linking.

As another non-limiting example, attachable moieties may be chemically conjugated using primary amines. In these instances, a homobifunctional cross-linker that contains succiminide ester, imidoester, acylazide, or isocyanate groups may be used for cross-linking. Examples of such cross-linking reagents include, but are not limited to: Bis[2-(succinimidooxycarbonyloxy)ethyl]sulfone (BSOCOES); Bis[2-(sulfosuccinimidooxycarbonyloxy)ethyl]sulfone (sulfo-BSCOCOES); Disuccinimidyl suberate (DSS); Bis-(Sulfosuccinimidyl) Suberate (BS3); Disuccinimidyl glutarate (DSG); Dithiobis(succinimidylpropionate) (DSP); Dithiobois(sulfosuccinimidylpropionate) (DTSSP); Disulfosuccinimidyl tartrate (sulfo-DST); Dithio-bis-maleimidoethane (DTME); Disuccinimidyl tartrate (DST); Ethylene glycolbis(sulfosuccinimidylsuccinate) (sulfo-EGS); Dimethyl malonimidate·2 HCl (DMM); Ethylene glycolbis(succinimidylsuccinate) (EGS); Dimethyl succinimidate·2 HCl (DMSC); Dimethyl adipimidate·2 HCl (DMA); Dimethyl pimelimidate·2 HCl (DMP); and Dimethyl suberimidate·2·HCl (DMS), and Dimethyl 3,3'-dithiobispropionimidate·2 HCl (DTBP). Heterobifunctional crosslinkers that contains a combination of imidoester or succinimide ester groups may also be used for cross-linking.

As another non-limiting example, attachable moieties may be chemically conjugated using sulfhydryl and primary amine groups. In these instances, heterobifunctional cross-linking reagents are preferable used. Examples of such cross-linking reagents include, but are not limited to: N-succinimidyl 3-(2- pyridyldithio)propionate (DPDP); N-succinimidyl 6-[3'-(2-pyridyldithio)-propionamido] hexanoate (sulfo-LC-SPDP); m-maleimidobenzoyl-N-hydoxysuccinimide ester (MBS); m-maleimidobenzoyl-N-hydoxysulfosuccinimide ester (sulfo-MBS); succinimidyl 4-[P-maleimidophenyl] butyrate (SMPB); sulfosuccinimidyl 4-[p-maleimidophenyl] butyrate (sulfo-SMPB); N-[y-Maleimidobutyryloxy] succinimide ester (GMBS), N-[ε-maleimidobutyryloxy] sulfosuccinimide ester (sulfo-GMBS); N-[γ-maleimidocaproyloxy] succinimide ester (EMCS); N-[ε-maleimidocaproyloxy] sulfosuccinimide ester (sulfo-EMCS); N-succinimidyl(4-iodoacetyl)aminobenzoate (SIAB); sulfosuccinimidyl(4-iodacetyl)aminobenzoate (sulfo-SIAB); succinimidyl 4-(N-maleimidomethyl)cyclohexane-1-carboxylate (SMCC); sulfosuccinimidyl 4-(N-maleimidomethyl)cyclohexane-1-carboxylate (sulfo-SMCC); succiminidyl-4-(N-maleimidomethyl)cyclohexane-1-carboxy-(6-amido-caproate) (LC-SMCC); 4-succinimidyloxycarbonyl-methyl-(2-pyridyldithio) toluene (SMPT); and sulfo-LC-SMPT.

As an exemplary protocol, a minicell suspension is made 5 mM EDTA/PBS, and a reducing solution of 2-mercaptoethylamine in 5 mM EDTA/PBS is added to the minicells. The mixture is incubated for 90 minutes at 37°C. The minicells are washed with EDTA/PBS to remove excess 2-mercaptoethylamine. The attachable moiety is dissolved in PBS, pH 7.2. A maleimide crosslinker is added to the solution, which is then incubated for 1 hour at room temperature. Excess maleimide is removed by column chromatography.

The minicells with reduced sulfhydryl groups are mixed with the derivatized compounds having an attachable moiety. The mixture is allowed to incubate at 4°C for 2 hours or overnight to allow maximum coupling. The conjugated minicells are washed to remove unreacted (unattached) compounds having the attachable moiety. Similar protocols are used for expressed membrane proteins with other reactive groups (e.g., carboxyl, amine) that can be conjugated to an attachable moiety.

### IX.D. Non-Genetic Methods for Directing Compounds to Membranes

Included within the scope of the invention are compounds that can be inserted into the membrane of segregated minicells. Such compounds include attachable moieties that are chemically conjugated to the surface of a minicell, and compounds that associate with and/or insert into a membrane "spontaneously," i.e., by virtue of their chemical nature. By way of non-limiting example, proteins that "spontaneously" insert into membranes include but are not limited to Thykaloid membrane proteins (Woolhead et al., J. Biol. Chem. 276:14607-14613, 2001), the mitochondrial adenine nucleotide translocator (Jacotot et al., J. Exp. Med. 193:509-519, 2001), and polypeptides obtained using the methods of Hunt et al. (Spontaneous, pH-dependent membrane insertion of a transbilayer alphahelix, Biochem 36:15177-15192, 1997). Lipids, gangliosides, sphingomyelins, plasmalogens glycosyl diacylglycerols, and sterols can also be incorporated into the membranes of segregated minicells.

### X. MEMBRANE PROTEINS

In certain aspects of the invention, membrane proteins from non-eubacterial organisms are expressed and displayed by minicells. The cellular membrane (a.k.a. the "plasma membrane") is a lipid bilayer that forms the boundary between the interior of a cell and its external environment. The term "membrane proteins" refers to proteins that are found in membranes including without limitation cellular and organellar membranes.

### X.A. Types of Membrane Proteins

### X.A.1. In General

Membrane proteins consist, in general, of two types, peripheral membrane proteins and integral membrane proteins.

Integral membrane proteins can span both layers (or "leaflets") of a lipid bilayer. Thus, such proteins may have extracellular, transmembrane, and intracellular domains. Extracellular domains are exposed to the external environment of the cell, whereas intracellular domains face the cytosol of the cell. The portion of an integral membrane protein that traverses the membrane is the "transmembrane domain." Transmembrane domains traverse the cell membrane often by one or more regions comprising 15 to 25 hydrophobic amino acids which are predicted to adopt an alpha-helical conformation.

Intergral membrane proteins are classified as bitopic or polytopic (Singer, (1990) Annu. Rev. Cell Biol. 6:247-96). Bitopic proteins span the membrane once while polytopic proteins contain multiple membrane-spanning segments.

A peripheral membrane protein is a membrane protein that is bound to the surface of the membrane and is not integrated into the hydrophobic layer of a membrane region. Peripheral membrane proteins do not span the membrane but instead are bound to the surface of a membrane, one layer of the lipid bilayer that forms a membrane, or the extracellular domain of an integral membrane protein.

The invention can be applied to any membrane protein, including but not limited to the following exemplary receptors and membrane proteins. The proteins include but are not limited to are receptors (e.g., GPCRs, sphingolipid receptors, neurotransmitter receptors, sensory receptors, growth factor receptors, hormone receptors, chemokine receptors, cytokine receptors, immunological receptors, and compliment receptors, FC receptors), channels (e.g., potassium channels, sodium channels, calcium channels.), pores (e.g., nuclear pore proteins, water channels), ion and other pumps (e.g., calcium pumps, proton pumps), exchangers (e.g., sodium/potassium exchangers, sodium/hydrogen exchangers, potassium/hydrogen exchangers), electron transport proteins (e.g., cytochrome oxidase), enzymes and kinases (e.g., protein kinases, ATPases, GTPases, phosphatases, proteases.), structural/linker proteins (e.g., Caveolins, clathrin), adapter proteins (e.g., TRAD, TRAP, FAN), chemotactic/adhesion proteins (e.g., ICAM11, selectins, CD34, VCAM-1, LFA-1,VLA-1), and phospholipases such as PI-specific PLC and other phospholipiases.

### X.A.2. Receptors

Within the scope of the invention are any receptor, including without limitation:
The nuclear receptors, e.g the nuclear export receptor;
The peripheral (mitochondrial) benzodiazephine receptor (Gavish et al., "Enigma of the Peripheral Benzodiazephine Receptor," Pharmacological Reviews, Vol. 51, No. 4);
Adrenergic and muscarinic receptors (Brodde et al., "Adrenergic and Muscarinic Receptors in the Human Heart", Pharmacological Review, Vol. 51, No. 4);
Gamma-aminobutyric acid_{A} receptors (Barnard et al., "International Union of Pharmacology. IV. Subtypes of γ-Aminobutyric AcidA Receptors: Classification on the Basis of Submit Structure and Receptor Function," Pharmacological Reviews, Vol. 50, No. 2);
Kinin Bi receptors (Marceau et al., "The B1 Receptors for Kinins," Pharmacological Reviews, Vol. 50, No. 3);
Chemokine receptors (Murphy et al., "International Union of Pharmacology. XXII. Nomenclature for Chemokine Receptors" Pharmacological Reviewa, Vol. 52, No. 1);
Glycine and NMDA Receptors (Danysz et al., "Glycine and N-Methyl-D-Aspartate Receptors: Physiological Significance and Possible Therapeutic Applications," Pharmacological Reviews, Vol. 50, No. 4);
Glutamate receptor ion channels (Dingledine et al., "The Glutamate Receptor Ion Channels", Pharmacological Reviews, Vol. 51, No. 1);
Purine and pyrimidine receptors including purinergic (e.g., P2) receptors (Ralevic et al., "Receptors for Purines and Pyrimidines", Pharmacological Reviews, Vol. 50, No. 3); CNS receptors and membrane transporters (E. Sylvester Vizi, "Role of High-Affinity Receptors and Membrane Transporters in Nonsynaptic Communication and Drug Action in the Central Nervous System," Pharmacological Reviews, Vol. 52, No. 1);
Opoid receptors, including but not limited to the ♀ -opioid receptor (Quock et al., "The ♀ -Opioid Receptor: Molecular Pharmacology, Signal Transduction and the Determination of Drug Efficacy", Pharmacological Review, Col. 51, No. 3);
Angiotensin II receptors (Gasparo et al., "International Union of Pharmacology. XXIII. The Angiotensin II Receptors" Pharmalogical Review, Vol. 52, No. 3);
Cholecystokinin receptors (Noble et al., "International Union of Pharmacology. XXI. Structure, Distribution, and Functions of Cholecystokinin Receptors", Pharmacological Reviews, Vol. 51, No. 4)
Hormone receptors, including but not limited to, the estrogen receptor; the glucocorticoid receptor; and the insulin receptor;
Receptors found predominantly in the central nervous system, including but not limited to, neuronal nicotinic acetylcholine receptors; the dopamine D2/D3 receptor; GABA receptors; central cannabinoid receptor CB1; opoid receptors, e.g., the kappa opioid receptor, and the methadone-specific opioid receptor; nicotinic acetylcholine receptors; serotonin receptors, e.g., the serotonin 5-HT3 receptor, the serotonin 5-HT4 receptor, and the serotonin-2 receptor; and dopamine receptors, e.g., the dopamine D2/D3 receptor; and the neurotensin receptor;
Receptors for growth factors, including but not limited to, the erythropoietin receptor; the FGF receptor; the EGF receptor; the VEGF receptor; VEGF receptor-2 protein; VEGF-receptor protein (KDR); fibroblast growth factor receptor; the p75 nerve growth factor receptor; epidermal growth factor receptor; IGF-1 receptor; platelet factor-4 receptor; alpha platelet-derived growth factor receptor; hepatocyte growth factor receptor; and human fibroblast growth factor receptor;
Receptors for sphingolipids and lysophospholipids such as the Edg family of GPCRs;
Receptors for interleukins, e.g., receptors for interleukin-1 (IL-1), IL-2, IL-3, IL-4, IL-5, IL-6, IL-7, IL-8, IL-9, IL-10, IL-11, IL-12, IL-13, *et seq*.; and
Various receptors, including by way of non-limiting example, receptors described in U.S. patents 6,210,967 (DNA encoding a mammalian LPA receptor and uses thereof); 6,210,921 (CAR: a novel coxsackievirus and adenovirus receptor; 6,211,343 (Lactoferrin receptor protein; 6,218,509 (LH/CG receptor, DNA and use thereof; 6,214,972 (DNA encoding prostaglandin receptor DP); 6,221,613 (DNA encoding a human melanin concentrating hormone receptor (MCH1) and uses thereof); 6,221,660 (DNA encoding SNORF25 receptor); 6,225,080 (Mu-subtype opioid receptor); 6,222,015 (Estrogen receptor); 6,228,610 (Human metabotropic glutamate receptor subtypes (hmR4, hmR6, hmR7) and related DNA compounds); 6,235,496 (Nucleic acid encoding mammalian mu opioid receptor); 6,258,556 (cDNA and genomic clones encoding human .mu. opiate receptor and the purified gene product); 6,245,531 (Polynucleotide encoding insect ecdysone receptor); 6,225,531 Glucan elicitor receptor, DNA molecule coding therefor, fungus-resistant plants transformed with the DNA molecule and method for creating the plants); 6,245,893 (Receptor that binds anti-convulsant compounds); 6,248,712 (Urokinase-type plasminogen activator receptor; 6,248,554 (DNA sequence coding for a BMP receptor); 6,248,520 (Nucleic acid molecules encoding nuclear hormone receptor coactivators and uses thereof); 6,242,251 (Rhesus neuropeptide Y5 receptor); 6,252,056 (Human lysophosphatidic acid receptor and use thereof); 6,255,472 (Isolated nucleic acid molecule encoding a human skeletal muscle-specific receptor); 6,291,207 (Herpes virus entry receptor protein); 6,291,206 (BMP receptor proteins); 6,291,195 (DNA encoding a human melanin concentrating hormone receptor (MCH1) and uses thereof); 6,344,200 (Lactoferrin receptor protein); 6,335,180 (Nucleic acid sequences encoding capsaicin receptor and uses thereof); 6,265,184 (Polynucleotides encoding chemokine receptor 88C); 6,207,799 (Neuropeptide Y receptor Y5 and nucleic acid sequences); 6,290,970 (Transferrin receptor protein of Moraxella); 6,326,350 (Transferrin receptor subunit proteins of Neisseria meningitidis); 6,313,279 (Human glutamate receptor and related DNA compounds); 6,313,276 (Human endothelin receptor); 6,307,030 (Androgen receptor proteins, recombinant DNA molecules coding for such, and use of such compositions); 6,306,622 (cDNA encoding a BMP type II receptor); 6,300,087 (DNA encoding a human serotonin receptor (5-HT4B) and uses thereof); 6,297,026 (Nucleic acids encoding the C140 receptor); 6,277,976 (Or-1, an orphan receptor belonging to the nuclear receptor family); 6,274,708 (Mouse interleukin-11 receptor); 6,271,347 (Eosinophil eotaxin receptor); 6,262,016 (Transferrin receptor genes); 6,261,838 (Rat melanocortin receptor MC3-R); 6,258,943 (Human neurokinin-3 receptor); 6,284,870 (Gamma retinoic acid receptor); 6,258,944 (OB receptor isoforms and nucleic acids encoding them); 6,261,801 (Nucleic acids encoding tumor necrosis factor receptor 5); 6,261,800 (Luteinizing hormone/choriogonadotropin (LH/CG) receptor); 6,265,563 (Opioid receptor genes); 6,268,477 (Chemokine receptor 88-C); 6,316,611 (Human N-methyl-D-aspartate receptor subunits, nucleic acids encoding same and uses therefor); 6,316,604 (Human C3b/C4b receptor (CR1)); 6,287,855 (Nucleic acid encoding rat galanin receptor (GALR2)); 6,268,221 (Melanocyte stimulating hormone receptor and uses); and 6,268,214 (Vectors encoding a modified low affinity nerve growth factor receptor).

### X.B. Membrane Anchoring Domains

A membrane-anchoring domain can be incorporated into a fusion protein of the invention. Non-limiting examples of membrane anchoring domains include those derived from Prostaglandin H2 synthases (PGHS-1 and - 2) (Nina et al., Anchoring of a monotopic membrane protein: the binding of prostaglandin H2 synthase-1 to the surface of a phospholipid bilayer, Eur. Biophys. J. 29:439-54, 2000; Otto and Smith, Photolabeling of prostaglandin endoperoxide H synthase-1 with 3-trifluoro-3-(m-[125l]iodophenyl)diazirine as a probe of membrane association and the cyclooxygenase active site, J Biol Chem 271:9906-10, 1996; and Otto and Smith, The orientation of prostaglandin endoperoxide synthases-1 and -2 in the endoplasmic reticulum, J Biol Chem 269:19868-75, 1994; those derived from carboxypeptidase E (EC 3.4.17.10) (Fricker et al., Identification of the pH-dependent membrane anchor of carboxypeptidase E (EC 3.4.17.10), J. Biol. Chem., 265, 2476-2482, 1990); and peptide convertase 3 (PC3) (Smeekens et al., Identification of a cDNA encoding a second putative prohormone convertase related to PC2 in AtT20 cells and islets of Langerhans, Proc Natl Acad Sci USA 88, 340-344, 1990).

### X.C. Transmembrane Domains

A variety of types and examples of transmembrane domain are known. Proteins with up to 12 transmembrane domains are known (Fujiwara et al., Identification of thyroid hormone transporters in humans: different molecules are involved in a tissue-specific manner, Endocrinology 2001 142:2005-12; Sharina et al., Mutational analysis of the functional role of conserved arginine and lysine residues in transmembrane domains of the murine reduced folate carrier, Mol Pharmacol 2001 59:1022-8). However, the invention is not limited to any particular number of transmembrane domains.

Monotropic ("single pass") domains, which traverse a membrane once, include by way of non-limiting example, those found in receptors for epidermal growth factor (EGF), receptors for tumor necrosis factor (TNF) and the like. Polytropic ("multipass") proteins traverse a membrane two or more times. Non-limiting examples of polytropic proteins are as follows.

Biotropic ("2 passes") membrane proteins include, but are not limited to: EnvZ of E. coli; the peroxisomal membrane protein Pex11-1p p (Anton et al., ARF- and coatomer-mediated peroxisomal vesiculation, Cell Biochem Biophys 2000;32 Spring:27-36); pleitropic drug ABC transporters of S. cervisiae (Rogers et al., The pleitropic drug ABC transporters from Saccharomyces cerevisiae, J Mol Microbiol Biotechnol 2001 3:207-14); and human and rate urate transporters hUAT and rUAT (Lipkowitz et al., Functional reconstitution, membrane targeting, genomic structure, and chromosomal localization of a human urate transporter, J Clin Invest 2001 107:1103-15).

Tritropic ("3 pass") membrane proteins include, but are not limited to: the ethylene receptor ETR1 of Arabidopsis; the Cauliflower Card Expression protein CC1 (Palmer et al., A Brassica oleracea Gene Expressed in a Variety-Specific Manner May Encode a Novel Plant Transmembrane Receptor, Plant Cell Physiol 2001 42:404-413); and a splice variant of the mitochondrial membrane protein hMRS3/4 (Li et al., Characterization of a novel human putative mitochondrial transporter homologous to the yeast mitochondrial RNA splicing proteins 3 and 4, FEBS Lett 2001 494:79-84).

Tetraspanins or tetraspans are non-limiting examples of membrane proteins with four transmembrane domains. (Levy et al., J. Biol. Chem, 226:14597-14602, 1991; Tomlinson et al., J. Immol. 23:136-40, 1993; and Barclay et al., (In) The Leucocyte antigen factbooks, Academic press, London, 1993). These proteins are collectively known as the 'transmembrane 4 superfamily' (TM4) because they span the plasma membrane four times. The proteins known to belong to this family include, but are not limited to: mammalian antigen CD9 (MIC3), a protein involved in platelet activation and aggregation; mammalian leukocyte antigen CD37, expressed on B lymphocytes; mammalian leukocyte antigen CD53 (OX-44), which may be involved in growth regulation in hematopoietic cells; mammalian lysosomal membrane protein CD63 (Melanoma-associated antigen ME491; antigen AD1); mammalian antigen CD81 (cell surface protein TAPA-1), which may play an important role in the regulation of lymphoma cell growth; mammalian antigen CD82 (Protein R2; Antigen C33; Kangai 1 (KAI1)), which associates with CD4 or CD8 and delivers costimulatory signals for the TCR/CD3 pathway; mammalian antigen CD151 (SFA-1); Platelet-endothelial tetraspan antigen 3 (PETA-3); mammalian TM4SF2 (Cell surface glycoprotein A15; TALLA-1; MXS1); mammalian TM4SF3 (Tumor-associated antigen CO-029); mammalian TM4SF6 (Tspan-6; TM4-D); mammalian TM4SF7 (Novel antigen 2 (NAG-2); Tspan-4); mammalian Tspan-2; Mammalian Tspan-3 (TM4-A); mammalian Tetraspan NET-5; and Schistosoma mansoni and japonicum 23 Kd surface antigen (SM23 / SJ23).

Non-limiting examples of membrane proteins with six transmembrane domains include the EBV integral membrane protein LMP-1, and a splice variant of the mitochondrial protein hMRS3/4 (Li et al., Characterization of a novel human putative mitochondrial transporter homologous to the yeast mitochondrial RNA splicing proteins 3 and 4, FEBS Lett 2001 Apr 6;494(1-2):79-84). Proteins with six transmembrane domains also include STEAP (six transmembrane epithelial antigens of the prostate) proteins (Afar et al., U.S. Patent 6,329,503). The prototype member of the STEAP family, STEAP-1, appears to be a type IIIa membrane protein expressed predominantly in prostate cells in normal human tissues. Structurally, STEAP-1 is a 339 amino acid protein characterized by a molecular topology of six transmembrane domains and intracellular N- and C-termini, suggesting that it folds in a "serpentine" manner into three extracellular and two intracellular loops.

Literally hundreds of 7-pass membrane proteins are known. G-protein coupled receptors (GPCRs), including without limitation beta-adreno receptors, adrenergic receptors, EDG receptors, adenosine receptors, B receptors for kinins, angiotensin receptors, and opiod receptors are of particular interest. GPCRs are described in more detail elsewhere herein.

A non-limiting example of a protein with 9 transmembrane domains is Lipocalin-1 interacting membrane receptor (Wojnar et al., Molecular cloning of a novel Lipocalin-1 interacting human cell membrane receptor (LIMR) using phage-display, J Biol Chem 2001 3; [epub ahead of print]).

Proteins with both transmembrane and anchoring domains are known. For example, AMPA receptor subunits have transmembrane domains and one membrane-anchoring domain.

A variety of databases that describe known, and software programs that predict, membrane anchoring and transmembrane domains are available to those skilled in the art. See, for example Gcrdb.dba GCRDb [G Protein Coupled Receptor database], Tmbase.dba Tmbase [database of transmembrane domains], Prodom.srv ProDom [Protein domains], Tmap.srv TMAP [Protein transmembrane segments prediction], Tm7.srv TM7 [Retrieval of data on G protein-coupled receptors], and Memsat.sof MEMSAT [transmembrane structure prediction program].

Quentin and Fichant (J Mol Microbiol Biotechnol 2000 2:501-4, ABCdb: an ABC transporter database) have described a database devoted to the ATP-binding cassette (ABC) protein domains (ABCdb), the majority of which energize the transport of compounds across membranes. In bacteria, ABC transporters are involved in the uptake of a wide range of molecules and in mechanisms of virulence and antibiotic resistance. In eukaryotes, most ABC transporters are involved in drug resistance, and many are associated with diseases. ABCdb can be accessed via the World Wide Web (http://ir2lcb.mrs-mrs.fr/ABCdb/). See also Sanchez-Fernandez et al., The Arabidopsis thaliana ABC protein superfamily: a complete inventory, J Biol Chem 2001 May 9; [epub ahead of print], and Rogers et al., The pleitropic drug ABC transporters from Saccharomyces cerevisiae, J Mol Microbiol Biotechnol 2001 Apr; 3(2):207-14.

### XI. RECOMBINANT DNA EXPRESSION

In order to achieve recombinant expression of a fusion protein, an expression cassette or construct capable of expressing a chimeric reading frame is introduced into an appropriate host cell to generate an expression system. The expression cassettes and constructs of the invention may be introduced into a recipient eubacterial or eukaryotic cell either as a nonreplicating DNA or RNA molecule, which may be a linear molecule or, more preferably, a closed covalent circular molecule. Since such molecules are incapable of autonomous replication, the expression of the gene may occur through the transient expression of the introduced sequence. Alternatively, permanent expression may occur through the integration of the introduced DNA sequence into the host chromosome.

### XI.A. Recombinant DNA Expression Systems

A variety of eubacterial recombinant DNA expression systems may be used to produce the fusion proteins of the invention. Host cells that may be used in the expression systems of the present invention are not strictly limited, provided that they are suitable for use in the expression of the fusion protein of interest and can produce minicells. Non-limiting examples of recognized eubacterial hosts that may be used in the present invention include bacteria such as *E. coli, Bacillus, Streptomyces, Pseudomonas, Salmonella, Serratia,* and the like.

Eubacterial expression systems utilize plasmid and viral (bacteriophage) expression vectors that contain replication sites and control sequences derived from a species compatible with the host may be used. Suitable phage or bacteriophage vectors include λgt10, λgt11 and the like. Suitable virus vectors may include pMAM-neo, pKRC and the like. Appropriate eubacterial plasmid vectors include those capable of replication in *E. coli* (such as, by way of non-limiting example, pBR322, pUC118, pUC119, ColEl, pSC101, pACYC 184, πVX. See "Molecular Cloning: A Laboratory Manual" 1989). *Bacillus* plasmids include pC194, pC221, pT127, and the like (Gryczan, In: The Molecular Biology of the Bacilli, Academic Press, NY, pp. 307-329, 1982). Suitable *Streptomyces* plasmids include p1J101 (Kendall et al., J. Bacteriol. 169:4177-4183, 1987), and *Streptomyces* bacteriophages such as C31 (Chater et al., In: Sixth International Symposium on Actinomycetales Biology, Akademiai Kaido, Budapest, Hungary, pp. 45-54, 1986). *Pseudomonas* plasmids are reviewed by John et al. (Rev. Infect. Dis. 8:693-704, 1986), and Izaki (Jpn. J. Bacteriol. 33:729-742, 1978). See also Brent et al., "Vectors Derived From Plasmids," Section II, and Lech et al. "Vectors derived from Lambda and Related Bacteriophages" Section III, in Chapter 1 of Short Protocols in Molecular Biology, 2nd Ed., Ausubel et al., eds., John Wiley and Sons, New York, 1992, pages 1-13 to 1-27; Lech et al. "Vectors derived from Lambda and Related Bacteriophages" Section III and Id. pages 1-28 to page 1-52.

To express a protein, including but not limited to a fusion protein, in a eubacterial cell, it is necessary to operably link the ORF encoding the protein to a functional eubacterial or viral promoter. Such promoters may be either constitutive or, more preferably, regulatable (*i.e*., inducible or derepressible). Examples of constitutive promoters include the *int* promoter of bacteriophage lambda, the *bla* promoter of the beta-lactamase gene sequence of pBR322, and the cat promoter of the chloramphenicol acetyl transferase gene sequence of pPR325, and the like. Examples of inducible eubacterial promoters include the major right and left promoters of bacteriophage lambda (P_{L} and P_{R}), the *trp, recA, lacZ, lacl,* and *gal* promoters of *E. coli*, the alpha-amylase (Ulmanen et al., J. Bacteriol. 162:176-182, 1985) and the sigma-28-specific promoters of *B. subtilis* (Gilman et al., Gene Sequence 32:11-20, 1984), the promoters of the bacteriophages of *Bacillus* (Gryczan, in: The Molecular Biology of the Bacilli, Academic Press, Inc., NY, 1982), and *Streptomyces* promoters (Ward et al., Mol. Gen. Genet. 203:468-478, 1986). Eubacterial promoters are reviewed by Glick (Ind. Microbiot. 1:277-282, 1987), Cenatiempo (Biochimie 68:505-516, 1986), and Gottesman (Ann. Rev. Genet. 18:415-442, 1984).

Proper expression also requires the presence of a ribosome-binding site upstream of the gene sequence-encoding sequence. Such ribosome-binding sites are disclosed, for example, by Gold et al. (Ann. Rev. Microbiol. 35:365-404, 1981). The selection of control sequences, expression vectors, transformation methods, and the like, are dependent on the type of host cell used to express the gene. As used herein, "cell", "cell line", and "cell culture" may be used interchangeably and all such designations include progeny. Thus, the words "transformants" or "transformed cells" include the primary subject cell and cultures derived therefrom, without regard to the number of transfers. It is also understood that all progeny may not be precisely identical in DNA content, due to deliberate or inadvertent mutations. However, as defined, mutant progeny have the same functionality as that of the originally transformed cell.

Mammalian expression systems utilize host cells such as HeLa cells, cells of fibroblast origin such as VERO or CHO-K1, or cells of lymphoid origin and their derivatives. Preferred mammalian host cells include SP2/0 and J558L, as well as neuroblastoma cell lines such as IMR 332, which may provide better capacities for correct post-translational processing. Non-limiting examples of mammalian extrachromosomal expression vectors include pCR3.1 and pcDNA3.1, and derivatives thereof including but not limited to those that are described by and are commercially available from Invitrogen (Carlsbad, CA).

Several expression vectors are available for the expression of polypeptides in mammalian host cells. A wide variety of transcriptional and translational regulatory sequences may be employed, depending upon the nature of the host. The transcriptional and translational regulatory signals may be derived from viral sources, such as adenovirus, bovine papilloma virus, cytomegalovirus (CMV), simian virus, or the like, where the regulatory signals are associated with a particular gene sequence which has a high level of expression. Alternatively, promoters from mammalian expression products, such as actin, collagen, myosin, and the like, may be employed. Transcriptional initiation regulatory signals may be selected which allow for repression or activation, so that expression of the gene sequences can be modulated. Of interest are regulatory signals that are temperature-sensitive since, by varying the temperature, expression can be repressed or initiated, or are subject to chemical (such as metabolite) regulation.

Preferred eukaryotic plasmids include, for example, BPV, vaccinia, SV40, 2-micron circle, and the like, or their derivatives. Such plasmids are well known in the art (Botstein et al., Miami Wntr. Symp. 19:265-274, 1982; Broach, in: The Molecular Biology of the Yeast Saccharomyces: Life Cycle and Inheritance, Cold Spring Harbor Laboratory, Cold Spring Harbor, NY, p. 445-470, 1981; Broach, Cell 28:203-204, 1982; Bollon et al., J. Clin. Hematol. Oncol. 10:39-48, 1980; Maniatis, In: Cell Biology: A Comprehensive Treatise, Vol. 3, Gene Sequence Expression, Academic Press, NY, pp. 563-608, 1980).

Expression of polypeptides in eukaryotic hosts generally involves the use of eukaryotic regulatory regions. Such regions will, in general, include a promoter region sufficient to direct the initiation of RNA synthesis. Preferred eukaryotic promoters include, for example, the promoter of the mouse metallothionein I gene sequence (Hamer et al., J. Mol. Appl. Gen. 1:273-288, 1982); the TK promoter of Herpes virus (McKnight, Cell 31:355-365, 1982); the SV40 early promoter (Benoist et al., Nature (London) 290:304-31, 1981); and the yeast gal4 gene sequence promoter (Johnston et al., Proc. Natl. Acad. Sci. (USA) 79:6971-6975, 1982; Silver et al., Proc. Natl. Acad. Sci. (USA) 81:5951-5955, 1984).

Expression sequences and elements are also required for efficient expression. Non-limiting examples include Kozak and IRES elements in eukaryotes, and Shine-Delgarno sequences in prokaryotes, which direct the initiation of translation (Kozak, Initiation of translation in prokaryotes and eukaryotes. Gene, 1999. 234: 187-208; Martinez-Salas et al., Functional interactions in internal translation initiation directed by viral and cellular IRES elements, Jour. of Gen. Virol. 82:973-984, 2001); enhancer sequences; optioanl sites for repressor and inducers to bind; and recognition sites for enaymes that cleave DNA or RNA in a site-specific manner. Translation of mRNA is generally initiated at the codon which encodes the first methionine; if so, it is preferable to ensure that the linkage between a eukaryotic promoter and a preselected ORF does not contain any intervening codons that encode a methionine (*i.e*., AUG). The presence of such codons results either in the formation of a fusion protein with an uncharacterized N-terminal extension (if the AUG codon is in the same reading frame as the ORF) or a frame-shift mutation (if the AUG codon is not in the same reading frame as the ORF).

### XI.B. Expression of Membrane Proteins

Presently, the most commonly used expression systems for the expression of integral membrane proteins are eukaryotic and eubacterial whole cell expression systems. Although minicells have been used to express several eubacterial membrane proteins, the production of non-eubacterial membrane proteins has not been reported. One aspect of the invention is the discovery that the minicell expression system can be made to express and preferably display integral membrane proteins from non-eubacterial organisms.

A "recombinant expression system" (or simply "expression system") is one that directs the production of exogenous gene products in a host cell or minicell of choice. By "expressed" it is meant that a gene product of interest (which can be a protein or nucleic acid) is produced in the expression system of choice.

Host cells (and/or minicells) harboring an expression construct are components of expression systems. An "expression vector" is an artificial nucleic acid molecule into which an exogenous ORF encoding a protein, or a template of a bioactive nucleic acid can be inserted in such a manner so as to be operably linked to appropriate expression sequences that direct the expression of the exogenous gene. By the term "operably linked" it is meant that the part of a gene that is transcribed is correctly aligned and positioned with respect to expression sequences that promote, are needed for and/or regulate this transcription. The term "gene product" refers to either a nucleic acid (the product of transcription, reverse transcription, or replication) or a polypeptide (the product of translation) that is produced using the non-vector nucleic acid sequences as a template.

In some applications, it is preferable to use an expression construct that is an episomal element. If the episomal expression construct expresses (or, preferably in some applications, over-expresses) a an ORF that has been incorporated into the episomal expression construct, the minicells will direct the production of the polypeptide encoded by the ORF. At the same time, any mRNA molecules transcribed from a chromosomal gene prior to minicell formation that have been transferred to the minicell are degraded by endogenous RNases without being replaced by new transcription from the (absent) bacterial chromosome.

Chromosomally-encoded mRNAs will not be produced in minicells and will be "diluted" as increasing amounts of mRNAs transcribed from the episomal element are generated. A similar dilution effect is expected to increase the relative amount of episomally-generated proteins relative to any chromosomally-encoded proteins present in the minicells. It is thus possible to generate minicells that are enriched for proteins encoded by and expressed from episomal expression constructs.

Although by no means exhaustive, a list of episomal expression vectors that have been expressed in eubacterial minicells is presented in Table 3.

It is also possible to transform minicells with exogenous DNA after they have been prepared or separated from their parent cells. For example, phage RNA is produced in minicells after infection by lambda phage (Witkiewicz and Taylor, Ribonucleic acid synthesis after adsorption of the bacteriophage lambda on Escherichia coli minicells, Acta Microbiol Pol A 7:21-4, 1975), even though replication of lambda phage may not occur in minicells (Witkiewicz and Taylor, The fate of phage lambda DNA in lambda-infected minicells, Biochim Biophys Acta 564:31-6, 1979).

Because it is the most characterized minicell-producing species, many of these episomal elements have been examined in minicells derived from *E*. *coli.* It is understood by practitioners of the art, however, that many episomal elements that are expressed in *E. coli* also function in other eubacterial species, and that episomal expression elements for minicell systems in other species are available for use in the invention disclosed herein.

Alternatively, in one aspect of the invention, bacterial minicells are prepared that contain expression elements that are prepared from shuttle vectors. A "shuttle vector" has sequences required for its replication and maintenance in cells from two different species of organisms, as well as expression elements, at least one of which is functional in bacterial cells, and at least one of which is functional in yeast cells. For example, *E*. *coli*-yeast shuttle vectors are known in the art and include, by way of non-limiting example, those derived from Yip, Yrp, Ycp and Yep. Preferred *E*. *coli*-yeast shuttle vectors are episomal elements that can segregrate into yeast minicells (i.e., Yrp, Ycp and Yep. Particularly preferred are expression vectors of the Yep (yeast episomal plasmid) class, and other derivatives of the naturally occurring yeast plasmid known as the 2µm circle. The latter vectors have relatively high transformation frequencies and are stably maintained through mitosis and meiosis in high copy number.

**Table 3: Episomal Elements That Segregate Into Escherichia coli Minicells**

| **EPISOMAL ELEMENT** | **REFERENCES** | |
|---|---|---|
| **Plasmids** | | |
| R6K, R1DRD19 | Nesvera *et al.,* Folia Microbiol. (Praha) 23:278-285 (1978) | |
| PSC101 | Fox *et al.,* Blood 69:1394-1400 (1987) | |
| PBR322 | Fox *et al.,* Blood 69:1394-1400 (1987) | |
| F element | Cohen *et al.,* Proc. Natl. Acad. Sci. 61:61-68 (1968); Khachatourians G.G., Biochim. Biophys. Acta. 561:294-300 (1979) | |
| NR1 | Hochmannova et al., Folia Microbiol. (Praha) 26:270-276 | |
| R6δ1 | Hochmannova et al., Folia Microbiol. (Praha) 26:270-276 | |
| PTTQ18 | Rigg *et al.,* Arch. Oral. Biol. 45:41-52 (2000) | |
| PGPR2.1 | Rigg of *P. et al.,* Arch. Oral. Biol. 45:41-52 (2000); expresses cell surface antigen *gingivalis* | |
| "mini-plasmid" derivative of RK2 | Firshein *et al*., J. Bacteriol. 150:1234-1243 (1982) | |
| ColE1 | Rashtchian et al., J. Bacteriol. 165:82-87 (1986); Witkiewicz et al., Acta. Microbiol. Pol. A 7:21-24 (1975) | |
| PSC101 | Rashtchian et al., J. Bacteriol. 165:82-87 (1986); Curtiss, Roy, III, U.S. Patent No. 4,190,495; Issued February 26, 1980 | |
| pACYC184 | Chang *et al.,* J. Bacteriol. 134:1141-1156 (1978); Rose, Nucleic Acids Res 16:355 (1988) | |
| Co1lb, Co1lb7 *DRD*& | Skorupska et al., Acta. Microbiol. Pol.A 8:17-26 (1976) | |
| pUC19 | Heighway et al., Nucleic Acids Res. 17:6893-6901 (1989) | |
| R-plasmid | Hochmannova et al., Folia Microbiol. (Praha) 25:11-15 (1980) | |
| PCR1 | Hollenberg et al., Gene 1:33-47 (1976); yeast shuttle vector | |

| **Bacteriophage** | | |
|---|---|---|
| Lambda | | Witkiewicz *et al.,* Acta. Microbiol. Pol. A 7:21-24 (1975) |
| M13 | | Staudenbauer et al., Mol. Gen. Genet. 138:203-212 (1975) |
| T7 | | Libby, Mech Ageing Dev. 27:197-206 (1984) |
| P1 | | Curtiss, Roy, III, U.S. Patent No. 4,190,495; Issued 2/26/80; |
| | | J Bacteriol 1995;177:2381-6, Partition of P1 plasmids in Escherichia coli mukB chromosomal partition mutants, Funnell and Gagnier. |

For expression of membrane proteins, and/or other proteins of interest in the recipient cell, ORFs encoding such proteins are operably linked to eukaryotic expression sequences that are appropriate for the recipient cell. For example, in the case of *E. coli*-yeast shuttle vectors, the ORFs are operably linked to expression sequences that function in yeast cells and/or minicells. In order to assess the effectiveness of a gene delivery vehicle, or a gene therapy expression element, an ORF encoding a detectable polypeptide (e.g., GFP, beta-galactosidase) is used. Because the detectable polypeptide is operably linked to eukaryotic expression elements, it is not expressed unless it has been transferred to its recipient (eukaryotic) cell. The signal from the detectable polypeptide thus correlates with the efficiency of gene transfer by a gene delivery agent, or the degree of expression of a eukaryotic expression element.

Gyuris and Duda (High-efficiency transformation of Saccharoymces cells by bacterial minicell protoplast fusion, Mol Cel Biol 6:329507, 1986) allegedly demonstrated the transfer of plasmid molecular by fusing minicell protoplasts with yeast protoplasts. Gyuris and Duda state that 10% of Saccharomyces cerevisiae cells were found to contain transforming DNA sequences. However, the plasmids did not contain eukaryotic expression elements, were not shuttle vectors, and genetic expression of the plasmids in yeast cells was not examined.

### XII. USES OF MINICELLS IN RESEARCH

Research products are designed for any specific type of application. These products may be packaged and distributed as, by way of non-limiting example, kits, chemicals, solutions, buffers, powders, solids, filters, columns, gels, matrixes, emulsions, pellets, capsules, and aerosols. Kits and reagents for certain research applications may be required by regulatory agency to be labeled "research use only" in order to indicate that the reagents are not intended for use in humans.

### XII.B. Transfection

Transfection is the process of introducing genetic material into eukaryotic and archaebacterial cells using biological, biochemical or physical methods. This process allows researchers to express and study target proteins in cultured cells (research use) as well as to deliver genetic material to cells in vivo or ex vivo systems (gene therapy). There are a variety of techniques which allow for the introduction and expression of proteins into target cells. These include mechanical transfection (Biolistic particles and Electroporation), calcium phosphate, DEAE-dextran/polybrene, viral based techniques and lipid based techniques.

The genetic material and/or nucleic acid to be delivered can be, by way of non-limiting example, nucleic acids that repair damaged or missing genes, nucleic acids for research applications, nucleic acids that kill a dysfunctional cell such as a cancer cell, antisense oligonucleotides to reduce or inhibit expression of a gene product, genetic material that increases expression of another gene, nucleotides and nucleotide analogs, peptide nucleic acids (PNAs), tRNAs, rRNAs, catalytic RNAs, RNA:DNA hybrid molecules, and combinations thereof.

The genetic material may comprise a gene expressing a protein. exemplary proteins include, but are not limited to, receptors (e.g., GPCRs, sphingolipid receptors, neurotransmitter receptors, sensory receptors, growth factor receptors, hormone receptors, chemokine receptors, cytokine receptors, immunological receptors, and compliment receptors, FC receptors), channels (e.g., potassium channels, sodium channels, calcium channels.), pores (e.g., nuclear pore proteins, water channels), ion and other pumps (e.g., calcium pumps, proton pumps), exchangers (e.g., sodium/potassium exchangers, sodium/hydrogen exchangers, potassium/hydrogen exchangers), electron transport proteins (e.g., cytochrome oxidase), enzymes and kinases (e.g., protein kinases, ATPases, GTPases, phosphatases, proteases), structural/linker proteins (e.g., Caveolins, clathrin), adapter proteins (e.g., TRAD, TRAP, FAN), chemotactic/adhesion proteins (e.g., ICAM11, selectins, CD34, VCAM-1, LFA-1,VLA-1), and chimeric/fusion proteins (e.g., proteins in which a normally soluble protein is attached to a transmembrane region of another protein).

A minicell that is used to deliver therapeutic agents may comprise and display a binding moiety. By way of non-limiting example, binding moieties used for particular purposes may be a binding moiety directed to a compound or moiety displayed by a specific cell type or cells found predominantly in one type of tissue, which may be used, among other things, to target minicells and their contents to specific cell types or tissues. A preferred binding moiety is an antibody or antibody derivative. Other binding moieties include, but are not limited to, receptors, enzymes, ligands, binding peptides, fusion proteins, small molecules conjugated to transmembrane proteins, ligands conjugated to transmembrane proteins, viral fusion proteins, and fusion/chimeric proteins.

A minicell containing genetic material may be to a target cell by methods including, but not limited to, receptor mediated endocytosis, cell fusion, or phagocytosis (Aderem et al., Mechanism of Phagocytosis in Macrophages, Annu. Rev. Immunol. 17:593-623, 1999). The minicell gene delivery system is used to deliver genetic material in culture for research applications as well as to cells in vivo as part of gene therapy or other therapeutic applications.

By way of non-limiting example, a minicell may express a protein such as invasin to induce receptor mediated endocytosis (Pepe et al., "Yersinia enterocolitica invasin: A primary role in the initiation of infection," Proc. Natl. Acad. Sci. U.S.A. 90:6473-6477, 1993; Alrutz et al., "Involvement of focal adhesion kinase in invasin-mediated uptake," Proc. Natl. Acad. Sci. U.S.A. 95:13658-13663, 1998). Invasin interacts with the Beta2 Integrin protein and causes it to dimerize. Upon dimerization the Beta2 Integrin signals for an endocytotic event. Thus a minicell expressing the invasin protein will be taken up by cells expressing Beta2 Integrin via endocytosis.

Another non-limiting example of the minicell gene delivery and transfection system using invasin involves the expression of invasin following a targeting event. In this example, a minicell expresses a targeting protein that is capable of bringing the minicell in contact with a specific target cell. Upon contact with the target cell, the minicell will be induced to transcribe and translate invasin. The induction is accomplished via signaling events or with a transcription factor dimerization event. The minicells can be engineered to contain targeting proteins that induce protein expression only upon contact with a specific target cell. By way of non-limiting example, the invasin is expressed only at the target cell where it induces endocytosis, thus preventing the minicell from entering any cell but the target cell.

Proteins can be induced and expressed post contact with target cells include but are not limited to antibodies and antibody derivatives, receptors, enzymes, ligands, binding peptides, fusion proteins, small molecules conjugated to transmembrane proteins, ligands conjugated to transmembrane proteins, viral fusion proteins, antibiotics, apoptotic proteins, hormones, toxins, poisons, and fusion/chimeric proteins.

Another non-limiting example of gene delivery or transfection using the minicell involves the use of the type III secretion apparatus of bacteria. The type III secretion apparatus is expressed in the minicell and used to transfer genetic material to a target cell.

Another non-limiting example of gene delivery and transfection using minicells involves minicells that have been engineered to contain anionic lipids or cationic lipids (Axel et al., "Toxicity, Uptake Kinetics and Efficacy of New Transfection Reagents: Increase of Oligonucleotide Uptake," Jour. of Vasc. Res. 040:1-14, 2000). Many types of lipids have been shown to induce or enhance transfection and gene delivery in a variety of cell types. Minicells containing such lipids could be used to transfer genetic material to specific cell types. Minicells can also be engineered to express targeting proteins that would allow the minicell to associate tightly with a target cell, which will facilitate the lipid interactions and gene transfer.

Another non-limiting example of gene delivery or transfection using minicells involves the use of ligands to induce receptor mediated endocytosis. By way of non-limiting example, the ligand is expressed on the surface of the minicell, or is attached to the surface of the minicell. A minicell containing genetic material is then able to associate with a target cell expressing the target receptor for the ligand. The receptor/ligand interaction will result in the endocytosis of the minicell into the target cell where the minicell would release and deliver the genetic material.

Another non-limiting example of gene delivery or transfection using minicells involves the use of fusion proteins, such as but not limited to viral capsid proteins. In this example the fusion protein would be expressed or attached to the outside of the minicell. The fusion protein would then induce fusion of a target cell with the minicell upon contact. The contact could be initiated via random non-targeting events or via the use of specific targeting proteins. In both cases the end result would be the fusion of the minicell with a target cell and the delivery of the genetic material.

### XIII. MINICELL-BASED DELIVERY OF BIOLOGICALLY ACTIVE AGENTS

### XIII.A. General Considerations

The minicells of the invention are capable of encapsulating and/or loading into a membrane a variety of substances, including but not limited to biologically active agents, including but not limited to diagnostic and therapeutic agents. Biologically active agents include, but are not limited to, nucleic acids, e.g., DNA, RNA, gene therapy constructs, ribozymes, antisense and other synthetic oligonucleotides including those with chemical modifications; peptide nucleic acids (PNAs); proteins; synthetic oligopeptides; peptomimetics; small molecules; radioisotopes; antibiotics; antibodies and antibody derivatives; and combinations and/or prodrugs of any of the preceding.

The surface of a minicell may be chemically altered in order to have certain properties that are desirable for their use as drug delivery agents. By way of non-limiting example, minicells may be chemically conjugated to polyethylene glycol (PEG), which provides for "stealth" minicells that are not taken as well and/or as quickly by the reticuloendothelial system (RES). Other ccompounds that may be attached to minicells include without limitation polysaccharides, polynucleotides, lipopolysaccharides, lipoproteins, glycosylated proteins, synthetic chemical compounds, and/or combinations of any of the preceding.

A minicell that is used to deliver therapeutic agents may comprise and display a binding moiety. By way of non-limiting example, binding moieties used for particular purposes may be a binding moiety directed to a compound or moiety displayed by a specific cell type or cells found predominantly in one type of tissue, which may be used, among other things, to target minicells and their contents to specific cell types or tissues. A preferred binding moiety is an antibody or antibody derivative, which are described in deatil elsewhere herein. Other binding moieties include, but are not limited to, receptors, enzymes, ligands, binding peptides, fusion proteins, small molecules conjugated to transmembrane proteins, ligands conjugated to transmembrane proteins, viral fusion proteins, and fusion/chimeric proteins.

### XIII.B. Cellular Uptake

In addition to binding moieties, proteins and other compounds that induce or enhance the uptake or fusion of the minicell with the target gene can be displayed on the surface of a minicell for applications involving the delivery of therapeutic agents, gene therapy, and/or transfection or other research applications. See, generally, Adhesion Protein Protocols, Vol. 96, Dejana, E. and Corada, M., eds., Humana Press, 1999.

### XIII.B.1. Cellular Uptake Sequences from Eukaryotic Cells

Eukaryotic adhesion receptors, which mediate intercellular adhesion , can be used as agents or targets for cellular uptake. There are at least three distinct classes of adhesive molecules that leukocytes employ during their adhesive interactions (a) integrins, including but not limited to LEC-CAMS/Selectins (ELAM-1, LAM-11Leu8ITQ1, and GMP140/PADGEM); (b) those belonging to the immunoglobulin superfamily including but not limited to CD2(LFA-2), CD3/TCR, CD4, CD8, CD28, CD44,CD54 (ICAM-1), ICAM-2, CD58 (LFA-3), VCAM-1,B7; and (c) Class I and II Major Histocompatability Antigens (MHC).

The adhesion receptors that belong to the integrin family and control intercellular interactions are of partciular interest. At least ten different structurally related cell surface heterodimeric (alpha and beta complexes) molecules have been defined as integrins and further classified into subfamilies (Springer T. A., 1990, Nature 346:425-434; Hynes, R. O., 1987, Cell 48:549-554; Moller, G. Editor, 1990, Immunol. Rev. 114.:1-217). Each subfamily has a unique beta subunit, designated integrin beta1 (CD29), integrin beta2 (CD18), and integrin beta3 (CD61), each of which can associate with multiple alpha subunits, each with at least one di-valent cation binding site. The integrin family includes receptors for extracellular matrix components such as fibronectin, laminin, vitronectin, and collagen which recognize Arg-Gly-Asp in their ligands and utilize the beta1 or beta3 subunits (Springer T. A., 1990, Nature 346:425-434; Hynes, R. O., 1987, Cell 48:549-554; Hemler, M. E., 1988, Immunol. Today 9:109-113; Patarroyo, M., and Makgoba, M. W., 1989, Scand. J. Immunol. 30:129-164; Moller, G. Editor, 1990, lmmunol. Rev. 11.4:1-217). There are at least six distinct alpha subunits alpha1 (CD49a), alpha2 (CD49b), alpha3 (CD49c), alpha4 (CD49d), alpha5 (CD49e), and alpha6 (CD49f) capable of associating with beta1 (CD29). The beta1 integrins are expressed on many nonhematopoietic and leukocyte cell types and are thought to play an active role in tissue organization by binding to extracellular matrix components found in many tissues and in the basement membranes underlying muscles, nervous system, epithelium and endothelium. While the expression of many beta1 integrins on leukocytes requires consistent activation, their expression on nonhematopoietic cells does not (Hemler, M. E., 1988, Immunol. Today 9:109-113; Patarroyo, M., and Makgoba, M. W., 1989, Scand. J. Immunol. 30:129-164). The complexity of the integrin family has been increased by the discovery of novel beta subunits beta3 (CD61), beta4 and beta5 that can associate with alpha 4, alpha 6, and alpha V subunits (Springer T. A., 1990, Nature 346:425-434; Hemler, M. E., 1988, Immunol. Today 9:109-113). This combinatorial use of alpha and beta subunits confers considerable diversity in ligand recognition and also helps regulate communications between the inside and outside of the cell.

By way of non-limiting example, a minicell display an adhesion receptor, or a fusion protein that has a transmembrane domain linked to a functional portion of an adhesion receptor. Such minicells will bind to cells displaying the ligand for the adhesion receptor.

### XIII.B.2. Cellular Uptake Sequences from Prokaryotes

Bacterial adhesion proteins are another source of polypetides that are used to stimulate uptake of minicells. See, generally, Handbook of Bacterial Adhesion: Priniciples, Methods, and Applications, Yuehuei H. An; Richard J. Friedman, eds., Humana Press, 2000; and Hultgren et al., "Bacterial Adhesions and Their Assembly," Chapter 150 in: Eschericia coli and Salmonella typhimurium: Cellular and Molecular Biology, 2nd Ed., Neidhardt, Frederick C., Editor in Chief, American Society for Microbiology, Washington, D.C., 1996, Volume 2, pages 1903-1999, and references cited therein.

By way of non-limiting example, a minicell may express a protein such as invasin to induce receptor mediated endocytosis (Pepe et al., Yersinia enterocolitica invasin: A primary role in the initiation of infection, Proc. Natl. Acad. Sci. U.S.A. 90:6473-6477, 1993; Alrutz et al., Involvement of focal adhesion kinase in invasin-mediated uptake, Proc. Natl. Acad. Sci. U.S.A. 95:13658-13663, 1998). Invasin interacts with the Beta2 Integrin protein and causes it to dimerize. Upon dimerization the Beta2 Integrin signals for an endocytotic event. Thus a minicell expressing the invasin protein will be taken up by cells expressing Beta2 Integrin via endocytosis.

As another non-limiting example, the pneumococcal adhesin protein CpbA interacts with the human polyimmunoglobulin receptor (hplgR) as either a part of the outer surface of a bacterial cell or as a free molecule Zhang et al. (Cell 102:827-837, 2000). The regions of CpbA:hplgR interaction were mapped using a series of large peptide fragments derived from CpbA. CpbA (Swiss-Prot Accession No. 030874) contains a choline binding domain containing residues 454-663 and two N-terminal repetitive regions called R1 and R2 that are contained in residues 97-203 and 259-365, respectively. Polypeptides containing R1 and R2 interact with hplgR, whereas polypeptides containing other sequences from CpbA do not bind to hplgR. The R1 and/or R2 sequences of the CpbA polypeptide, and/or essentially identical, substantially identical, or homologous amino acid sequences, are used to facilitate the uptake of minicells by cells.

Another non-limiting example of gene delivery or transfection using the minicell involves the use of the type III secretion apparatus of bacteria. The type III secretion apparatus is expressed in the minicell and used to transfer genetic material to a target cell.

Other non-limiting examples of a minicell gene delivery and transfection targeting moiety are ETA (detoxified exotoxin a) protein delivery element described in U.S. Patent No. 6,086,900 to Draper; Interalin and related proteins from Listeria species (Galan, Alternative Strategies for Becoming an Insider: Lessons from the Bacterial World, Cell 103:363-366,2000); Intimin from pathogenic E. coli strains (Frankel et al., Intimin and the host cell - is it bound to end in Tir(s)? Trends in Microbiology 9:214-218); and SpeB, streptococcal pyrogenic exotoxin B (Stockbauer et al., A natural variant of the cysteine protease virulence factor of group A Streptococcus with an arginine-glycine-aspartic acid (RGD) motif preferentially binds human integrins αvβ3 and αIIbβ3 Proc. Natl. Acad. Sci. U.S.A. 96:242-247, 1999).

### XIII.B.3. Cellular Uptake Sequences from Viruses

Cellular uptake sequences derived from viruses include, but are not limited to, the VP22 protein delivery element derived from herpes simplex virus-1 and vectors containing sequences encoding the VP22 protein delivery element are commercially available from Invitrogen (Carlsbad, CA; see also U.S. Patent No. 6,017,735 to Ohare et al.); and the Tat protein delivery element derived from the amino acid sequence of the Tat protein of human immunodeficiency virus (HIV). See U.S. Patents 5,804,604; 5,747,641; and 5,674,980.

### XIII.B.4. Lipids

Another non-limiting example of gene delivery and transfection using minicells involves minicells that have been engineered to contain anionic lipids or cationic lipids (Axel et al., Toxicity, Uptake Kinetics and Efficacy of New Transfection Reagents: Increase of Oligonucleotide Uptake, Jour. of Vasc. Res. 040:1-14, 2000). Many types of lipids have been shown to induce or enhance transfection and gene delivery in a variety of cell types. Minicells containing such lipids could be used to transfer genetic material to specific cell types. Minicells can also be engineered to express targeting proteins that would allow the minicell to associate tightly with a target cell, which will facilitate the lipid interactions and gene transfer.

Another non-limiting example of gene delivery or transfection using minicells involves the use of ligands to induce receptor mediated endocytosis. By way of non-limiting example, the ligand is expressed on the surface of the minicell, or is attached to the surface of the minicell. A minicell containing genetic material is then able to associate with a target cell expressing the target receptor for the ligand. The receptor/ligand interaction will result in the endocytosis of the minicell into the target cell where the minicell would release and deliver the genetic material.

Another non-limiting example of gene delivery or transfection using minicells involves the use of fusion proteins, such as but not limited to viral capsid proteins. In this example the fusion protein would be expressed or attached to the outside of the minicell. The fusion protein would then induce fusion of a target cell with the minicell upon contact. The contact could be initiated via random non-targeting events or via the use of specific targeting proteins. In both cases the end result would be the fusion of the minicell with a target cell and the delivery of the genetic material.

### XIII.C. Post-Targeting Expression of Cellular Uptake Sequences

Another non-limiting example of the minicell gene delivery and transfection system using invasin involves the expression of invasin following a targeting event. In this example, a minicell expresses a targeting protein that is capable of bringing the minicell in contact with a specific target cell. Upon contact with the target cell, the minicell will be induced to transcribe and translate invasin. The induction is accomplished via signaling events or with a transcription factor dimerization event. The minicells can be engineered to contain targeting proteins that induce protein expression only upon contact with a specific target cell. By way of non-limiting example, the invasin is expressed only at the target cell where it induces endocytosis, thus preventing the minicell from entering any cell but the target cell.

Proteins can be induced and expressed post contact with target cells include but are not limited to antibodies and antibody derivatives, receptors, enzymes, ligands, binding peptides, fusion proteins, small molecules conjugated to transmembrane proteins, ligands conjugated to transmembrane proteins, viral fusion proteins, antibiotics, apoptotic proteins, hormones, toxins, poisons, and fusion/chimeric proteins.

### XIII.D. Intracellular Targeting and Organellar Delivery

After delivery to and entry into a targeted cell, a minicell may be designed so as to be degraded, thereby releasing the therapeutic agent it encapsulates into the cytoplasm of the cell. The minicell and/or therapeutic agent may include one or more organellar delivery elements, which targets a protein into or out of a specific organelle or organelles. For example, the ricin A chain can be included in a fusion protein to mediate its delivery from the endosome into the cytosol. Additionally or alternatively, delivery elements for other organelles or subcellular spaces such as the nucleus, nucleolus, mitochondria, the Golgi apparatus, the endoplasmic reticulum (ER), the cytoplasm, etc. are included Mammalian expression constructs that incorporate organellar delivery elements are commercially available from Invitrogen (Carlsbad, CA: pShooter™ vectors). An H/KDEL (*i.e*., His /Lys-Asp-Glu-Leu sequence) may be incorporated into a fusion protein of the invention, preferably at the carboxy-terminus, in order to direct a fusion protein to the ER (see Andres et al., J. Biol. Chem. 266:14277-142782, 1991; and Pelham, Trends Bio. Sci. 15:483-486, 1990).

Another type of organellar delivery element is one which directs the fusion protein to the cell membrane and which may include a membrane-anchoring element. Depending on the nature of the anchoring element, it can be cleaved on the internal or external leaflet of the membrane, thereby delivering the fusion protein to the intracellular or extracellular compartment, respectively. For example, it has been demonstrated that mammalian proteins can be linked to i) myristic acid by an amide-linkage to an N-terminal glycine residue, to ii) a fatty acid or diacylglycerol through an amide- or thioether-linkage of an N-terminal cysteine, respectively, or covalently to iii) a phophotidylinositol (PI) molecule through a C-terminal amino acid of a protein (for review, see Low, Biochem. J. 244:1-13, 1987). In the latter case, the PI molecule is linked to the C-terminus of the protein through an intervening glycan structure, and the PI then embeds itself into the phopholipid bilayer; hence the term "GPI" anchor. Specific examples of proteins know to have GPI anchors and their C-terminal amino acid sequences have been reported (see Table 1 and Figure 4 in Low, Biochemica et Biophysica Acta, 988:427-454, 1989; and Table 3 in Ferguson, Ann. Rev. Biochem., 57:285-320, 1988). Incorporation of GPI anchors and other membrane-targeting elements into the amino- or carboxy-terminus of a fusion protein can direct the chimeric molecule to the cell surface.

### XIII.E. Minicell-Based Gene Therapy

The delivery of nucleic acids to treat diseases or disorders is known as gene therapy (Kay et al., Gene Therapy, Proc. Natl. Acad. Sci. USA 94:12744-12746, 1997). It has been proposed to use gene therapy to treat genetic disorders as well as pathogenic diseases. For reviews, see Desnick et al., Gene Therapy for Genetic Diseases, Acta Paediatr. Jpn. 40:191-203, 1998; and Bunnell et al., Gene Therapy for Infectious Diseases, Clinical Microbiology Reviews 11:42-56, 1998).

Gene delivery systems use vectors that contain or are attached to therapeutic nucleic acids. These vectors facilitate the uptake of the nucleic acid into the cell and may additionally help direct the nucleic acid to a preferred site of action, e.g., the nucleus or cytoplasm (Wu et al., "Delivery Systems for Gene Therapy," Biotherapy 3:87-95, 1991). Different gene delivery vectors vary with regards to various properties, and different properties are desirable depending on the intended use of such vectors. However, certain properties (for example, safety, ease of preparation, etc.) are generally desirable in most circumstances.

The minicells of the invention may be used as delivery agents for any therapeutic or diagnostic agent, including without limitation gene therapy constructs. Minicells that are used as delivery agents for gene therepay constructs may, but need not be, targeted to specific cells, tissues, organs or systems of an organism, of a pathogen thereof, using binding moieties as described in detail elsewhere herein.

In order to enhance the effectiveness of gene delivery vectors in, by way of non-limiting example, gene therapy and transfection, it is desirable in some applications of the invention to target specific cells or tissues of interest (targeted cells or tissues, respectively). This increases the effective dose (the amount of therapeutic nucleic acid present in the targeted cells or tissues) and minimizes side effects due to distribution of the therapeutic nucleic acid to other cells. For reviews, see Peng et al., "Viral Vector Targeting," Curr. Opin. Biotechnol. 10:454-457, 1999; Gunzburg et al., "Retroviral Vector Targeting for Gene Therapy," Cytokines Mol. Ther. 2:177-184, 1996.; Wickham, "Targeting Adenovirus," Gene Ther. 7:110-114, 2000; Dachs et al., "Targeting Gene Therapy to Cancer: A Review," Oncol. Res. 9:313-325, 1997; Curiel, "Strategies to Adapt Adenoviral Vectors for Targeted Delivery," Ann NY Acad. Sci. 886:158-171, 1999; Findeis et al., "Targeted Delivery of DNA for Gene Therapy via Receptors," Trends Biotechnol. 11:202-205, 1993.

Some targeting strategies make use of cellular receptors and their natural ligands in whole or in part. See, for example, Cristiano et al., "Strategies to Accomplish Gene Delivery Via the Receptor-Mediated Endocytosis Pathway," Cancer Gene Ther., Vol. 3, No. 1, pp. 49-57, Jan. - Feb. 1996.; S.C. Philips, "Receptor-Mediated DNA Delivery Approaches to Human Gene Therapy," Biologicals, Vol. 23, No. 1, pp. 13-6, March 1995; Michael et al., "Strategies to Achieve Targeted Gene Delivery Via the Receptor-Mediated Endocytosis Pathway," Gene Ther., Vol. 1, No. 4, pp. 223-32, July 1994; Lin et al., "Antiangiogenic Gene Therapy Targeting The Endothelium-Specific Receptor Tyrosine Kinase Tie2," Proc. Natl. Acad. Sci., USA, Vol. 95, pp. 8829-8834,1998. Sudimack et al., "Targeted Drug Delivery Via the Folate Receptor," Adv. Drug Deliv., pp. 147-62, March 2000; Fan et al., "Therapeutic Application of Anti-Growth Factor Receptor Antibodies," Curr. Opin. Oncol., Vol. 10, No. 1, pp. 67-73, January 1998; Wadhwa et al., "Receptor Mediated Glycotargeting," J. Drug Target, Vol. 3, No. 2, pp. 111-27, 1995; Perales et al., "An Evaluation of Receptor-Mediated Gene Transfer Using Synthetic DNA-Ligand Complexes," Eur. J. Biochem, Vol. 1, No 2, pp. 226, 255-66, December 1994; Smith et al., "Hepatocyte-Directed Gene Delivery by Receptor-Mediated Endocytosis," Semin Liver Dis., Vol. 19, No. 1, pp. 83-92, 1999.

Antibodies, particularly single-chain antibodies, to surface antigens specific for a particular cell type may also be used as targeting elements. See, for example, Kuroki et al., "Specific Targeting Strategies of Cancer Gene Therapy Using a Single-Chain Variable Fragment (scFv) with a High Affinity for CEA," Anticancer Res., pp. 4067-71, 2000; U.S. Patent 6,146,885, to Dornburg, entitled "Cell-Type Specific Gene Transfer Using Retroviral Vectors Containing Antibody-Envelope Fusion Proteins"; Jiang et al., "In Vivo Cell Type-Specific Gene Delivery With Retroviral Vectors That Display Single Chain Antibodies," Gene Ther. 1999, 6:1982-7; Engelstadter et al., "Targeting Human T Cells By Retroviral Vectors Displaying Antibody Domains Selected From A Phage Display Library," Hum. Gene Ther. 2000, 11:293-303; Jiang et al., "Cell-Type-Specific Gene Transfer Into Human Cells With Retroviral Vectors That Display Single-Chain Antibodies," J. Virol 1998,72:10148-56; Chu et al., "Toward Highly Efficient Cell-Type-Specific Gene Transfer With Retroviral Vectors Displaying Single-Chain Antibodies," J. Virol 1997, 71:720-5; Chu et al., "Retroviral Vector Particles Displaying The Antigen-Binding Site Of An Antibody Enable Cell-Type-Specific Gene Transfer," J. Virol 1995, 69:2659-63; and Chu et al., "Cell Targeting With Retroviral Vector Particles Containing Antibody-Envelope Fusion Proteins," Gene Ther. 1994, 1:292-9.

Minicells are used to deliver DNA-based gene therapy to targeted cells and tissues. Double minicell transformants are used not only to target a particular cell/tissue type (*e.g*. HIV-infected T-cells) but are also engineered to fuse with and enter targeted cells and deliver a protein-based toxin (*e.g*., antibiotic, or pro-apoptotic gene like Bax), an antisense expression construct (*e.g*., antisense to a transcription factor), or antisense oligonucleotides (*e.g*., antisense to an anti-apoptotic gene such as Bcl-2. The doubly-transformed minicells express not only these cell death promoters, but also only target particular cells/tissues, thus minimizing toxicity and lack of specificity of gene therapy vectors. By "doubly-transformed" it is meant that the minicell comprises 2 expression elements, one eubacterial, the other eukaryotic. Alternaively, shuttle vectors, which comprise eubacterial and eukaryotic expression elementsin one vector, may be used.

Minicell-based gene therapy is used to deliver expression plasmids that could correct protein expression deficiencies or abnormalities. As a non-limiting example, minicell inhalants are targeted to pulmonary alveolar cells and are used to deliver chloride transporters that are deficient or otherwise material in cystic fibrosis. Protein hormone deficiencies (*e.g*., dwarfism) are corrected by minicell expression systems (*e.g*., growth hormone expression in pituitary cells). Duchene's muscular dystrophy is characterized by a mutation in the dystrophin gene; this condition is corrected by minicell-based gene therapy. Angiogenesis treatment for heart patients is made effective by FGF or VGEF-producing minicells targeted to the heart. In this case, plasmid-driven over-expression of these grown factors is preferred.

### XIV. THERAPEUTIC USES OF MINICELLS

In addition to minicell-based gene therapy, minicells can be used in a variety of therapeutic modalities. Non-limiting examples of these modalities include the following applications.

### XIV.A. Diseases and Disorders

Diseases and disorders to which the invention can be applied include, by way of non-limiting example, the following.
Diseases and disorders that involve the respiratory system, such as cystic fibrosis, lung cancer and tumors, asthma, pathogenic infections, allergy-related diseases and disorders, , such as asthma; allergic bronchopulmonary aspergillosis; hypersensitivity pneumonia, eosinophilic pneumonia; emphysema; bronchitis; allergic bronchitis bronchiectasis; cystic fibrosis; hypersensitivity pneumotitis; occupational asthma; sarcoid, reactive airway disease syndrome, interstitial lung disease, hyper-eosinophilic syndrome, parasitic lung disease and lung cancer, asthma, adult respiratory distress syndrome, and the like;
Diseases and disorders of the digestive system, such as those of the gastrointestinal tract, including cancers, tumors, pathogenic infections, colitis; ulcerative colitis, diverticulitis, Crohn's disease, gastroenteritis, inflammatory bowel disease, bowel surgery ulceration of the duodenum, a mucosal villous disease including but not limited to coeliac disease, past infective villous atrophy and short gut syndromes, pancreatitis, disorders relating to gastroinstestinal hormones, Crohn's disease, and the like;
Diseases and disorders of the skeletal system, such as spinal muscular atrophy, rheumatoid arthritis, osteoarthritis, osteoporosis, multiple myeloma-related bone disorder, cortical-striatal-spinal degeneration, and the like;
Autoimmune diseases, such as Rheumatoid arthritis (RA), multiple sclerosis (MS), Sjogren's syndrome, sarcoidosis, insulin dependent diabetes mellitus (IDDM), autoimmune thyroiditis, reactive arthritis, ankylosing spondylitis, scleroderma, polymyositis, dermatomyositis, psoriasis, vasculitis, Wegener's granulomatosis, Crohn's disease and ulcerative colitis amyotrophic lateral sclerosis, multiple sclerosis, autoimmune gastritis, systemic lupus erythematosus, autoimmune hemolytic anemia, autoimmune neutropenia, systemic lupus erythematosus, graft vs. host disease, bone marrow engraftment, some cases of Type I diabetes, and the like;
Neurological diseases and disorders, such as depression, bipolar disorder, schizophrenia, Alzheimer's disease, Parkinson's disease, familial tremors, Gilles de la Tourette syndrome, eating disorders, Lewy-body dementia, chronic pain and the like;
Pathological diseases and resultant disorders such as bacterial infections such as infection by Escherichia, Shigella, Salmonella; sepsis, septic shock, and bacteremia; infections by a virus such as HIV, adenovirus, smallpox virus, hepatovirus, and the like; and AIDS-related encephalitis, HIV-related encephalitis, chronic active hepatitis, and the like;
Proliferative disease and disorders, such as acute lymphoblastic leukemia, acute myelogenous leukemia, chronic myelogenous leukemia, metastatic melanoma, Kaposi's sarcoma, multiple myeloma, breast cancer, anal cancer, vulvar cancer, and the like; and
Various diseases, disorders and traumas including, but not limited to, apoptosis mediated diseases, inflammation, cerebral ischemia, myocardial ischemia, aging, sarcoidosis, granulomatous colitis, scleroderma, degenerative diseases, necrotic diseases, alopecia, neurological damage due to stroke, diffuse cerebral cortical atrophy, Pick disease, mesolimbocortical dementia, thalamic degeneration, Huntington chorea, cortical-basal ganglionic degeneration, cerebrocerebellar degeneration, familial dementia with spastic paraparesis, polyglucosan body disease, Shy-Drager syndrome, olivopontocerebellar atrophy, progressive supranuclear palsy, dystonia musculorum deformans, Hallervorden-Spatz disease, Meige syndrome, acanthocytic chorea, Friedreich ataxia, Holmes familial cortical cerebellar atrophy, Gerstmann-Straussler-Scheinker disease, progressive spinal muscular atrophy, progressive balbar palsy, primary lateral sclerosis, hereditary muscular atrophy, spastic paraplegia, glomeralonephritis, chronic thyroiditis, Grave's disease, thrombocytopenia, myasthenia gravis, psoriasis, peroneal muscular atrophy, hypertrophic interstitial polyneuropathy, heredopathia atactica polyneuritiformis, optic neuropathy, and ophthalmoplegia.

A variety of diseases and disorders caused or exacerbated by pathogens may be treated using the invention. For a comprehensive description of pathogens and associated diseases and disorders, see Zinsser Microbiology, 20th Ed., Joklik, ed., Appelton-Century-Crofts, Norwalk, CT, 1992, and references cited therein.

Minicells could also be used for replacement therapy (via gene therapy) in a variety of conditions known to be caused by protein or proteins that are either absent (e.g. Duchene's Muscular Dystrophy), reduced in level (Dwarfism) or abberant (Sickle-cell anemia).

For a comprehensive description of diseases and disorders that may be treated using the invention, see The Merck Manual of Diagnosis and Therapy, 17th Ed., Beers et al., eds.; published edition, Merck and Co., Rahway, N.J., 1999; on-line edition, Medical Services, Usmedsa, USHH, http://www.merck.com/pubs/mmanual/, and references cited therein.

### XIV.B. Antibacterial and Antiparasitic Applications

Minicells may be used to kill pathogenic bacteria, protozoans, yeast and other fungi, parasitic worms, viruses and other pathogens by mechanisms that either do or do not rely on selective absorption. Antibiotics can be delivered to pathogenic organisms after first being targeted by the proteins or small molecules on the surfaces of the minicells that promote binding of the minicells to the surfaces of the pathogen. Fusion or injection of minicell contents into the pathogenic cell can result in the death or disablement of the pathogen and thus lower the effective dose of an antibiotic or gene therapeutic agent. Delivery of antibiotics tethered to or encapsulated by the minicells will reduce the effective dose of an antibiotic and will reduce its elimination by the renal system. In the case of delivering encapsulated molecules (e.g., antibiotics), purified/isolated minicells expressing membrane-bound proteins for targeting can be incubated with the molecules *in vitro* prior to administration. This would be particularly applicable to the use of protoplast minicells or poroplast minicells that have their outer membrane and cell wall or outer membrane only removed, respectively, thus facilitating the diffusion of the small molecule into the intact minicell.

Without being limited by the following example, minicells can be use as antibacterial agents by expressing on the surfaces of the minicells antigens, receptors, antibodies, or other targeting elements that will target the minicell to the pathogenic organism and facilitate the entry of plasmids, proteins, small molecules in order to gain access to or entry into the organism. Antibiotics may be encapsulated by minicells post isolation from the parent strain so that the antibiotic will not be effective against the minicell-producing bacteria itself. Since minicells are not able to reproduce, the antibiotic will not be lethal to the minicell delivery vehicle, but only to the targeted pathogen. In another non-limiting example, lyosgenic factors e.g., complement may be expressed on the surfaces of the minicells or encapsulated by same as to promote lysis of the pathogen.

Minicells can also be engineered to express toxic proteins or other elements upon binding to the pathogen. Induction of minicell protein expression can be an event that is coincident with targeting or triggered by minicell binding to the target pathogen, thus making minicells toxic only when contact is made with the pathogenic organism. Minicells can be engineered to express fusion/chimeric proteins that are tethered to the membrane by transmembrane domains that have signaling moieties on the cytoplasmic surfaces of the minicells, such as kinases or transcription factors. In one non-limiting example, a minicell fusion membrane-bound protein could be expressed containing an extracellular domain with a receptor, scFv, or other targeting protein that binds to the pathogen. The second segment of the chimera could be a transmembrane domain of a protein such as the EGF receptor or ToxR (that would tether the fusion protein to the membrane). Importantly, the cytoplasmic domain of the fusion protein could be a kinase that phosphorylates a bacterial transcription factor present in the minicell or could be fused to a transcription factor that would be expressed on the cytoplasmic surface of the minicell. The expression plasmid that was previously introduced into the minicells would then be activated by promoters utilizing the activated bacterial transcription factor pre-existing in the minicells or that which may be introduced by the minicell. Without being limited to the following example, the binding event could be signaled by a fusion protein containing elements of a receptor (e.g., EGF) or by an adhesion protein (e.g., an integrin) that require oligomerization. In the example of the use of integrins, bacterial or other transcription factors that also require dimerization could be cloned as fusion proteins such that the binding event would be signaled by a dimerization of two or more identical recombinant chimeric proteins that have association-dependent transcription factors tagged to the C-terminus of the fusion protein. The minicells would only be toxic when contact is made with the pathogen.

The proposed mechanism of induction coincident with targeting is not limited to the antiparasitic uses of minicells but can be used in other therapeutic situations where minicells are used to express proteins of therapeutic benefit when directed against eucaryotic cells of the organism (e.g., kill cancer cells with protein toxins expressed only after binding of the minicell to the cancer cell).

Transfer of DNA-containing plasmids or other expression element, antisense DNA, etc. may be used to express toxic proteins in the target cells or otherwise inhibit transcription and/or translation in the pathogenic organism or would otherwise be toxic to the cell. Without being limited by the following example, minicells can be used to transfer plasmids expressing growth repressors, DNAses, or other proteins or peptides (e.g., pro-apoptotic) that would be toxic to the pathogen.

### XIV.C. Cancer Therapy

Fusion proteins expressed in minicells are used for cancer therapy. In a non-limiting example, phage display antibody libraries are used to clone single chain antibodies against tumor-associated (tumor-specific) antigens, such as MUCH-1 or EGFvIII. Fusion proteins expressing these antibodies, and further comprising a single-pass transmembrane domain of an integral membrane protein, are used to "present" the antibody to the surface of the minicells. Injected minicells coated with anti-tumor antibodies target the tumor and deliver pro-apoptotic genes or other toxic substances to the tumor. The minicells are engulfed by the tumor cells by processes such receptor-mediated endocytosis (by, e.g., macrophages). By way of non-limiting example, toxR-invasin could be expressed on the surfaces of the minicells to promote endocytosis through the interaction between invasin and beta2-integrins on the surfaces of the target cells.

Fusion proteins possessing viral fusion-promoting proteins facilitate entry of the minicell to the tumor cell for gene therapy or for delivery of chemotherapy bioactive proteins and nucleic acids. In these and similar applications, the minicell may contain separate eukaryotic and eubacterial expression elements, or the expression elements may be combined into a single "shuttle vector."

### XV. DIAGNOSTIC USES OF MINICELLS

Minicells are transformed with plasmids expressing membrane-bound proteins, such as receptors, that bind to specific molecules in a particular biological sample such as blood, urine, feces, sweat, saliva or a tissue such as liver or heart. Minicells can also be used for delivery of therapeutic agents across the blood-brain barrier to the brain. This modality is used, by way of non-limiting example, for imaging purposes, and for the delivery of therapeutic agents, e.g., anti-depressants, and agents for the treatment of cancer, obesity, insomnia, schizophrenia, compulsive disorders and the like. Recombinant expression systems are incorporated into minicells where the plasmid-driven protein expression construct could be the produce a single gene product or a fusion protein, such as a soluble protein for the particular ligand fused with a transmembrane domain of a different gene. The fusion protein then acts as a membrane bound receptor for a particular ligand or molecule in the sample. Conventional cloning techniques (e.g., PCR) are used to identify genes for binding proteins, or phage display is used to identify a gene for a single-stranded variable antibody gene expressing binding protein for a particular ligand. The protein product is preferably a soluble protein. By constructing a plasmid containing this gene plus the transmembrane domain of a known single-pass membrane protein such as that of the EGF receptor, a fusion protein may be expressed on the surfaces of the minicells as an integral membrane protein with an extracellular domain that is preferably capable of binding ligand.

In another type of fusion protein, the transmembrane domain of the EGF receptor is fused to a known soluble receptor for a particular ligand, such as the LBP (lipopolysaccharide binding protein) or the extracellular domain of CD14 receptor protein, both of which bind the bacterial endotoxin, LPS (lipopolysaccharide). The LBP/EGF or CD14/EGF fusion protein is used to measure LPS in the serum of patients suspected of sepsis.

The minicell system is used to express receptors such as those of the EDG (endothelial cell differentiation gene) family (e.g., EDG 1-9) that recognize sphingolipids such as sphingosine-1-phosphate (S1P), sphingosylphosphoryl choline (SPC) and the lysophospholipid, lysophosphatidic acid (LPA). Since these proteins are 7-pass integral membrane proteins, no additional transmembrane domains of another protein are needed, and the receptor protein is thus not a fusion protein.

Truncated or mutant forms of a protein of interest are useful in a diagnostic assay. For example, a protein that is an ligand-binding enzyme can be altered so as to bind its substrate of interest but can no longer convert substrate into product. One example of this application of minicell technology is the expression of a truncated or mutant lactic dehydrogenase which is able to bind lactic acid, but is not able to convert lactic acid to pyruvate. Similarly, hexokinase deriviatives are used in minicells for glucose monitoring.

Minicells as diagnostic tools can be used either *in vitro* or *in vivo.* In the *in vitro* context, the minicells are used in an ELISA format or in a lateral flow diagnostic platform to detect the presence and level of a desired analyte. A sample (tissue, cell or body fluid sample) is taken and then tested *in vitro.* One advantage of the minicell system in detecting substances in tissue, cells or in body fluids is that the minicells can be used *in vitro* assays where the minicell is labeled with either a radioactive or fluorescent compound to aid in its detection in a an ELISA format or lateral flow platform. Thus, the use of secondary antibody detection systems is obviated.

As an *in vivo* diagnostic, minicells can be radiolabeled. One method of labeling is to incubate minicells for a short time (about 8 hr) with a T_{1/2} tracer (e.g., Tn99M) that is useful for detecting tumor metastases. The Tn99M accumulates in cells and loads into minicells after isolation or into the parent bacteria during growth phase. As Tn99M is oxidized by either the parent *E. coli* strain or by the minicells after isolation, the Tn99M is retained by the cell. lodine-labled proteins may also be used (Krown et al., TNF-alpha receptor expression in rat cardiac myocytes: TNF-alpha inhibition of L-type Ca2+ transiets, FEBS Letters 376:24-30, 1995).

One non-limiting example of *in vivo* detection of cancer making use of radiolabeled minicells is the use of the minicells to express chimeric membrane-bound single-chain antibodies against tumor-specific antigens (TSA) expressed on malignant melanoma or other transformed cells. Such TSAs include, but are not limited to, the breast cancer associated MUC1 antigen and variant forms of the EGFR (EGFvIII). By way of non-limiting example, minicells expressing antibodies to melanoma cells can be injected (IV) into a patient and then subjected to CAT scan of the lymphatic drainage in order to determine if a metastasis has occurred. This diagnostic technique obviates the need for lymph node dissection.

Another example of an *in vivo* diagnostic is to use the minicell system to express antibodies against oxidized low-density lipoproteins (LDL). Oxidized LDLs are associated with atherogenic plaques. Radiolabeled minicells (prepared as above) are injected IV into a person prior to nuclear imaging for image enhancement. MRI image contrast enhancement is performed by preparing minicells complexed (loaded) with contrast enhancers such as paramagnetic relaxivity agents and magnetic susceptibility agents.

In diagnostic as well as other applications, minicells preferentially detect a diagnostic marker, i.e., a marker associated with a disease or disorder. A diagnostic marker is statistically more like to occur in individuals sufferening from a disease than in those who are not diseased. Preferably, a diagnostic marker directly causes or is produced during a disease; however, the association may be no more than a correlation.

### XVI. PHARMACEUTICAL COMPOSITIONS

Another aspect of the invention is drawn to compositions, including but not limited to pharmaceutical compositions. According to the invention, a "composition" refers to a mixture comprising at least one carrier, preferably a physiologically acceptable carrier, and one or more minicell compositions. The term "carrier" defines a chemical compound that does not inhibit or prevent the incorporation of the biologically active peptide(s) into cells or tissues. A carrier typically is an inert substance that allows an active ingredient to be formulated or compounded into a suitable dosage form (e.g., a pill, a capsule, a gel, a film, a tablet, a microparticle (e.g., a microsphere), a solution; an ointment; a paste, an aerosol, a droplet, a colloid or an emulsion etc.). A "physiologically acceptable carrier" is a carrier suitable for use under physiological conditions that does not abrogate (reduce, inhibit, or prevent) the biological activity and properties of the compound. For example, dimethyl sulfoxide (DMSO) is a carrier as it facilitates the uptake of many organic compounds into the cells or tissues of an organism. Preferably, the carrier is a physiologically acceptable carrier, preferably a pharmaceutically or veterinarily acceptable carrier, in which the minicell composition is disposed.

A "pharmaceutical composition" refers to a composition wherein the carrier is a pharmaceutically acceptable carrier, while a "veterinary composition" is one wherein the carrier is a veterinarily acceptable carrier. The term "pharmaceutically acceptable carrier" or "veterinarily acceptable carrier" includes any medium or material that is not biologically or otherwise undesirable, *i.e*., the carrier may be administered to an organism along with a minicell composition without causing any undesirable biological effects or interacting in a deleterious manner with the complex or any of its components or the organism. Examples of pharmaceutically acceptable reagents are provided in The United States Pharmacopeia, The National Formulary, United States Pharmacopeial Convention, Inc., Rockville, Md. 1990, hereby incorporated by reference herein into the present application. The terms "therapeutically effective amount" or "pharmaceutically effective amount" mean an amount sufficient to induce or effectuate a measurable response in the target cell, tissue, or body of an organism. What constitutes a therapeutically effective amount will depend on a variety of factors, which the knowledgeable practitioner will take into account in arriving at the desired dosage regimen.

The compositions of the invention can further comprise other chemical components, such as diluents and excipients. A "diluent" is a chemical compound diluted in a solvent, preferably an aqueous solvent, that facilitates dissolution of the composition in the solvent, and it may also serve to stabilize the biologically active form of the composition or one or more of its components. Salts dissolved in buffered solutions are utilized as diluents in the art. For example, preferred diluents are buffered solutions containing one or more different salts. A preferred buffered solution is phosphate buffered saline (particularly in conjunction with compositions intended for pharmaceutical administration), as it mimics the salt conditions of human blood. Since buffer salts can control the pH of a solution at low concentrations, a buffered diluent rarely modifies the biological activity of a biologically active peptide.

An "excipient" is any more or less inert substance that can be added to a composition in order to confer a suitable property, for example, a suitable consistency or to form a drug. Suitable excipients and carriers include, in particular, fillers such as sugars, including lactose, sucrose, mannitol, or sorbitol cellulose preparations such as, for example, maize starch, wheat starch, rice starch, agar, pectin, xanthan gum, guar gum, locust bean gum, hyaluronic acid, casein potato starch, gelatin, gum tragacanth, methyl cellulose, hydroxypropylmethyl-cellulose, polyacrylate, sodium carboxymethylcellulose, and/or polyvinylpyrrolidone (PVP). If desired, disintegrating agents can also be included, such as cross-linked polyvinyl pyrrolidone, agar, or alginic acid or a salt thereof such as sodium alginate. Other suitable excipients and carriers include hydrogels, gellable hydrocolloids, and chitosan. Chitosan microspheres and microcapsules can be used as carriers. See WO 98/52547 (which describes microsphere formulations for targeting compounds to the stomach, the formulations comprising an inner core (optionally including a gelled hydrocolloid) containing one or more active ingredients, a membrane comprised of a water insoluble polymer (e.g., ethylcellulose) to control the release rate of the active ingredient(s), and an outer layer comprised of a bioadhesive cationic polymer, for example, a cationic polysaccharide, a cationic protein, and/or a synthetic cationic polymer; U.S. patent no. 4,895,724. Typically, chitosan is cross-linked using a suitable agent, for example, glutaraldehyde, glyoxal, epichlorohydrin, and succinaldehyde. Compositions employing chitosan as a carrier can be formulated into a variety of dosage forms, including pills, tablets, microparticles, and microspheres, including those providing for controlled release of the active ingredient(s). Other suitable bioadhesive cationic polymers include acidic gelatin, polygalactosamine, polyamino acids such as polylysine, polyhistidine, polyornithine, polyquaternary compounds, prolamine, polyimine, diethylaminoethyldextran (DEAE), DEAE-imine, DEAE-methacrylate, DEAE-acrylamide, DEAE-dextran, DEAE-cellulose, poly-p-aminostyrene, polyoxethane, copolymethacrylates, polyamidoamines, cationic starches, polyvinylpyridine, and polythiodiethylaminomethylethylene.

The compositions of the invention can be formulated in any suitable manner. Minicell compositions may be uniformly (homogeneously) or non-uniformly (heterogenously) dispersed in the carrier. Suitable formulations include dry and liquid formulations. Dry formulations include freeze dried and lyophilized powders, which are particularly well suited for aerosol delivery to the sinuses or lung, or for long term storage followed by reconstitution in a suitable diluent prior to administration. Other preferred dry formulations include those wherein a composition according to the invention is compressed into tablet or pill form suitable for oral administration or compounded into a sustained release formulation. When the composition is intended for oral administration but is to be delivered to epithelium in the intestines, it is preferred that the formulation be encapsulated with an enteric coating to protect the formulation and prevent premature release of the minicell compositions included therein. As those in the art will appreciate, the compositions of the invention can be placed into any suitable dosage form. Pills and tablets represent some of such dosage forms. The compositions can also be encapsulated into any suitable capsule or other coating material, for example, by compression, dipping, pan coating, spray drying, etc. Suitable capsules include those made from gelatin and starch. In turn, such capsules can be coated with one or more additional materials, for example, and enteric coating, if desired. Liquid formulations include aqueous formulations, gels, and emulsions.

Some preferred embodiments concern compositions that comprise a bioadhesive, preferably a mucoadhesive, coating. A "bioadhesive coating" is a coating that allows a substance (e.g., a minicellcomposition) to adhere to a biological surface or substance better than occurs absent the coating. A "mucoadhesive coating" is a preferred bioadhesive coating that allows a substance, for example, a composition according to the invention, to adhere better to mucosa occurs absent the coating. For example, micronized particles (e.g., particles having a mean diameter of about 5, 10, 25, 50, or 100 µm) can be coated with a mucoadhesive. The coated particles can then be assembled into a dosage form suitable for delivery to an organism. Preferably, and depending upon the location where the cell surface transport moiety to be targeted is expressed, the dosage form is then coated with another coating to protect the formulation until it reaches the desired location, where the mucoadhesive enables the formulation to be retained while the composition interacts with the target cell surface transport moiety.

The compositions of the invention may be administered to any organism, preferably an animal, preferably a mammal, bird, fish, insect, or arachnid. Preferred mammals include bovine, canine, equine, feline, ovine, and porcine animals, and non-human primates. Humans are particularly preferred. Multiple techniques of administering or delivering a compound exist in the art including, but not limited to, oral, rectal (e.g. an enema or suppository) aerosol (e.g., for nasal or pulmonary delivery), parenteral, and topical administration. Preferably, sufficient quantities of the biologically active peptide are delivered to achieve the intended effect. The particular amount of composition to be delivered will depend on many factors, including the effect to be achieved, the type of organism to which the composition is delivered, delivery route, dosage regimen, and the age, health, and sex of the organism. As such, the particular dosage of a composition incorporated into a given formulation is left to the ordinarily skilled artisan's discretion.

Those skilled in the art will appreciate that when the compositions of the present invention are administered as agents to achieve a particular desired biological result, which may include a therapeutic or protective effect(s) (including vaccination), it may be necessary to combine the fusion proteins of the invention with a suitable pharmaceutical carrier. The choice of pharmaceutical carrier and the preparation of the fusion protein as a therapeutic or protective agent will depend on the intended use and mode of administration. Suitable formulations and methods of administration of therapeutic agents include those for oral, pulmonary, nasal, buccal, ocular, dermal, rectal, or vaginal delivery.

Depending on the mode of delivery employed, the context-dependent functional entity can be delivered in a variety of pharmaceutically acceptable forms. For example, the context-dependent functional entity can be delivered in the form of a solid, solution, emulsion, dispersion, micelle, liposome, and the like, incorporated into a pill, capsule, tablet, suppository, areosol, droplet, or spray. Pills, tablets, suppositories, areosols, powders, droplets, and sprays may have complex, multilayer structures and have a large range of sizes. Aerosols, powders, droplets, and sprays may range from small (1 micron) to large (200 micron) in size.

Pharmaceutical compositions of the present invention can be used in the form of a solid, a lyophilized powder, a solution, an emulsion, a dispersion, a micelle, a liposome, and the like, wherein the resulting composition contains one or more of the compounds of the present invention, as an active ingredient, in admixture with an organic or inorganic carrier or excipient suitable for enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used include glucose, lactose, mannose, gum acacia, gelatin, mannitol, starch paste, magnesium trisilicate, talc, corn starch, keratin, colloidal silica, potato starch, urea, medium chain length triglycerides, dextrans,and other carriers suitable for use in manufacturing preparations, in solid, semisolid, or liquid form. In addition auxiliary, stabilizing, thickening and coloring agents and perfumes may be used. Examples of a stabilizing dry agent includes triulose, preferably at concentrations of 0.1 % or greater (See, e.g., U.S. Patent No. 5,314,695). The active compound is included in the pharmaceutical composition in an amount sufficient to produce the desired effect upon the process or condition of diseases.

### XVII. SMALL MOLECULES

The term "small molecule" includes any chemical or other moiety that can act to affect biological processes. Small molecules can include any number of therapeutic agents presently known and used, or can be small molecules synthesized in a library of such molecules for the purpose of screening for biological function(s). Small molecules are distinguished from macromolecules by size. The small molecules of this invention usually have molecular weight less than about 5,000 daltons (Da), preferably less than about 2,500 Da, more preferably less than 1,000 Da, most preferably less than about 500 Da.

Small molecules include without limitation organic compounds, peptidomimetics and conjugates thereof. As used herein, the term "organic compound" refers to any carbon-based compound other than macromolecules such nucleic acids and polypeptides. In addition to carbon, organic compounds may contain calcium, chlorine, fluorine, copper, hydrogen, iron, potassium, nitrogen, oxygen, sulfur and other elements. An organic compound may be in an aromatic or aliphatic form. Non-limiting examples of organic compounds include acetones, alcohols, anilines, carbohydrates, monosaccharides, oligosaccharides, polysaccharides, amino acids, nucleosides, nucleotides, lipids, retinoids, steroids, proteoglycans, ketones, aldehydes, saturated, unsaturated and polyunsaturated fats, oils and waxes, alkenes, esters, ethers, thiols, sulfides, cyclic compounds, heterocylcic compounds, imidizoles and phenols. An organic compound as used herein also includes nitrated organic compounds and halogenated (e.g., chlorinated) organic compounds. Methods for preparing peptidomimetics are described below. Collections of small molecules, and small molecules identified according to the invention are characterized by techniques such as accelerator mass spectrometry (AMS; see Turteltaub et al., Curr Pharm Des 2000 6(10):991-1007, Bioanalytical applications of accelerator mass spectrometry for pharmaceutical research; and Enjalbal et al., Mass Spectrom Rev 200019(3):139-61, Mass spectrometry in combinatorial chemistry.)

Preferred small molecules are relatively easier and less expensively manufactured, formulated or otherwise prepared. Preferred small molecules are stable under a variety of storage conditions. Preferred small molecules may be placed in tight association with macromolecules to form molecules that are biologically active and that have improved pharmaceutical properties. Improved pharmaceutical properties include changes in circulation time, distribution, metabolism, modification, excretion, secretion, elimination, and stability that are favorable to the desired biological activity. Improved pharmaceutical properties include changes in the toxicological and efficacy characteristics of the chemical entity.

### XVIII. POLYPEPTIDES AND DERIVATIVES

### XVIII.A. Polypeptides

As used herein, the term "polypeptide" includes proteins, fusion proteins, oligopeptides and polypeptide derivatives, with the exception that peptidomimetics are considered to be small molecules herein. Although they are polypeptides, antibodies and their derivatives are described in a separate section. Antibodies and antibody derivatives are described in a separate section, but antibodies and antibody derivatives are, for purposes of the invention, treated as a subclass of the polypeptides and derivatives.

A "protein" is a molecule having a sequence of amino acids that are linked to each other in a linear molecule by peptide bonds. The term protein refers to a polypeptide that is isolated from a natural source, or produced from an isolated cDNA using recombinant DNA technology; and has a sequence of amino acids having a length of at least about 200 amino acids.

A "fusion protein" is a type of recombinant protein that has an amino acid sequence that results from the linkage of the amino acid sequences of two or more normally separate polypeptides.

A "protein fragment" is a proteolytic fragment of a larger polypeptide, which may be a protein or a fusion protein. A proteolytic fragment may be prepared by in vivo or in vitro proteolytic cleavage of a larger polypeptide, and is generally too large to be prepared by chemical synthesis. Proteolytic fragments have amino acid sequences having a length from about 200 to about 1,000 amino acids.

An "oligopeptide" is a polypeptide having a short amino acid sequence (i.e., 2 to about 200 amino acids). An oligopeptide is generally prepared by chemical synthesis.

Although oligopeptides and protein fragments may be otherwise prepared, it is possible to use recombinant DNA technology and/or in vitro biochemical manipulations. For example, a nucleic acid encoding an amino acid sequence may be prepared and used as a template for in vitro transcription/translation reactions. In such reactions, an exogenous nucleic acid encoding a preselected polypeptide is introduced into a mixture that is essentially depleted of exogenous nucleic acids that contains all of the cellular components required for transcription and translation. One or more radiolabeled amino acids are added before or with the exogenous DNA, and transcription and translation are allowed to proceed. Because the only nucleic acid present in the reaction mix is the exogenous nucleic acid added to the reaction, only polypeptides encoded thereby are produced, and incorporate the radiolabelled amino acid(s). In this manner, polypeptides encoded by a preselected exogenous nucleic acid are radiolabeled. Although other proteins are present in the reaction mix, the preselected polypeptide is the only one that is produced in the presence of the radiolabeled amino acids and is thus uniquely labeled.

As is explained in detail below, "polypeptide derivatives" include without limitation mutant polypeptides, chemically modified polypeptides, and peptidomimetics.

The polypeptides of this invention, including the analogs and other modified variants, may generally be prepared following known techniques. Preferably, synthetic production of the polypeptide of the invention may be according to the solid phase synthetic method. For example, the solid phase synthesis is well understood and is a common method for preparation of polypeptides, as are a variety of modifications of that technique [Merrifield (1964), J. Am. Chem. Soc., 85: 2149; Stewart and Young (1984), Solid Phase polypeptide Synthesis, Pierce Chemical Company, Rockford, III.; Bodansky and Bodanszky (1984), The Practice of polypeptide Synthesis, Springer-Verlag, New York; Atherton and Sheppard (1989), Solid Phase polypeptide Synthesis: A Practical Approach, IRL Press, New York]. See, also, the specific method described in Example 1 below.

Alternatively, polypeptides of this invention may be prepared in recombinant systems using polynucleotide sequences encoding the polypeptides. For example, fusion proteins are typically prepared using recombinant DNA technology.

### XVIII.B. Polypeptide Derivatives

A "derivative" of a polypeptide is a compound that is not, by definition, a polypeptide, i.e., it contains at least one chemical linkage that is not a peptide bond. Thus, polypeptide derivatives include without limitation proteins that naturally undergo post-translational modifications such as, e.g., glycosylation. It is understood that a polypeptide of the invention may contain more than one of the following modifications within the same polypeptide. Preferred polypeptide derivatives retain a desirable attribute, which may be biological activity; more preferably, a polypeptide derivative is enhanced with regard to one or more desirable attributes, or has one or more desirable attributes not found in the parent polypeptide. Although they are described in this section, peptidomimetics are taken as small molecules in the present disclosure.

### XVIII.C. Mutant Polypeptide Derivatives

A polypeptide having an amino acid sequence identical to that found in a protein prepared from a natural source is a "wildtype" polypeptide. Mutant oligopeptides can be prepared by chemical synthesis, including without limitation combinatorial synthesis.

Mutant polypeptides larger than oligopeptides can be prepared using recombinant DNA technology by altering the nucleotide sequence of a nucleic acid encoding a polypeptide. Although some alterations in the nucleotide sequence will not alter the amino acid sequence of the polypeptide encoded thereby ("silent" mutations), many will result in a polypeptide having an altered amino acid sequence that is altered relative to the parent sequence. Such altered amino acid sequences may comprise substitutions, deletions and additions of amino acids, with the proviso that such amino acids are naturally occurring amino acids.

Thus, subjecting a nucleic acid that encodes a polypeptide to mutagenesis is one technique that can be used to prepare mutant polypeptides, particularly ones having substitutions of amino acids but no deletions or insertions thereof. A variety of mutagenic techniques are known that can be used in vitro or in vivo including without limitation chemical mutagenesis and PCR-mediated mutagenesis. Such mutagenesis may be randomly targeted (i.e., mutations may occur anywhere within the nucleic acid) or directed to a section of the nucleic acid that encodes a stretch of amino acids of particular interest. Using such techniques, it is possible to prepare randomized, combinatorial or focused compound libraries, pools and mixtures.

Polypeptides having deletions or insertions of naturally occurring amino acids may be synthetic oligopeptides that result from the chemical synthesis of amino acid sequences that are based on the amino acid sequence of a parent polypeptide but which have one or more amino acids inserted or deleted relative to the sequence of the parent polypeptide. Insertions and deletions of amino acid residues in polypeptides having longer amino acid sequences may be prepared by directed mutagenesis.

### XVIII.D. Chemically Modified Polypeptides

As contemplated by this invention, the term "polypeptide" includes those having one or more chemical modification relative to another polypeptide, i.e., chemically modified polypeptides. The polypeptide from which a chemically modified polypeptide is derived may be a wildtype protein, a mutant protein or a mutant polypeptide, or polypeptide fragments thereof; an antibody or other polypeptide ligand according to the invention including without limitation single-chain antibodies, bacterial proteins and polypeptide derivatives thereof; or polypeptide ligands prepared according to the disclosure. Preferably, the chemical modification(s) confer(s) or improve(s) desirable attributes of the polypeptide but does not substantially alter or compromise the biological activity thereof. Desirable attributes include but are limited to increased shelf-life; enhanced serum or other in vivo stability; resistance to proteases; and the like. Such modifications include by way of non-limiting example N-terminal acetylation, glycosylation, and biotinylation.

### XVIII.D.1. Polypeptides with N-Terminal or C-Terminal Chemical Groups

An effective approach to confer resistance to peptidases acting on the N-terminal or C-terminal residues of a polypeptide is to add chemical groups at the polypeptide termini, such that the modified polypeptide is no longer a substrate for the peptidase. One such chemical modification is glycosylation of the polypeptides at either or both termini. Certain chemical modifications, in particular N-terminal glycosylation, have been shown to increase the stability of polypeptides in human serum (Powell et al. (1993), Pharma. Res. 10: 1268-1273). Other chemical modifications which enhance serum stability include, but are not limited to, the addition of an N-terminal alkyl group, consisting of a lower alkyl of from 1 to 20 carbons, such as an acetyl group, and/or the addition of a C-terminal amide or substituted amide group.

### XVIII.D.2. Polypeptides with a Terminal D-Amino Acid

The presence of an N-terminal D-amino acid increases the serum stability of a polypeptide that otherwise contains L-amino acids, because exopeptidases acting on the N-terminal residue cannot utilize a D-amino acid as a substrate. Similarly, the presence of a C-terminal D-amino acid also stabilizes a polypeptide, because serum exopeptidases acting on the C-terminal residue cannot utilize a D-amino acid as a substrate. With the exception of these terminal modifications, the amino acid sequences of polypeptides with N-terminal and/or C-terminal D-amino acids are usually identical to the sequences of the parent L-amino acid polypeptide.

### XVIII.D.3. Polypeptides With Substitution of Natural Amino Acids By Unnatural Amino Acids

Substitution of unnatural amino acids for natural amino acids in a subsequence of a polypeptide can confer or enhance desirable attributes including biological activity. Such a substitution can, for example, confer resistance to proteolysis by exopeptidases acting on the N-terminus. The synthesis of polypeptides with unnatural amino acids is routine and known in the art (see, for example, Coller, et al. (1993), cited above).

### XIX.E. Peptidomimetics

In general, a polypeptide mimetic ("peptidomimetic") is a molecule that mimics the biological activity of a polypeptide but is no longer peptidic in chemical nature. By strict definition, a peptidomimetic is a molecule that contains no peptide bonds (that is, amide bonds between amino acids). However, the term peptidomimetic is sometimes used to describe molecules that are no longer completely peptidic in nature, such as pseudo-peptides, semi-peptides and peptoids. Examples of some peptidomimetics by the broader definition (where part of a polypeptide is replaced by a structure lacking peptide bonds) are described below. Whether completely or partially non-peptide, peptidomimetics according to this invention provide a spatial arrangement of reactive chemical moieties that closely resembles the three-dimensional arrangement of active groups in the polypeptide on which the peptidomimetic is based. As a result of this similar active-site geometry, the peptidomimetic has effects on biological systems that are similar to the biological activity of the polypeptide.

There are several potential advantages for using a mimetic of a given polypeptide rather than the polypeptide itself. For example, polypeptides may exhibit two undesirable attributes, i.e., poor bioavailability and short duration of action. Peptidomimetics are often small enough to be both orally active and to have a long duration of action. There are also problems associated with stability, storage and immunoreactivity for polypeptides that are not experienced with peptidomimetics.

Candidate, lead and other polypeptides having a desired biological activity can be used in the development of peptidomimetics with similar biological activities. Techniques of developing peptidomimetics from polypeptides are known. Peptide bonds can be replaced by non-peptide bonds that allow the peptidomimetic to adopt a similar structure, and therefore biological activity, to the original polypeptide. Further modifications can also be made by replacing chemical groups of the amino acids with other chemical groups of similar structure. The development of peptidomimetics can be aided by determining the tertiary structure of the original polypeptide, either free or bound to a ligand, by NMR spectroscopy, crystallography and/or computer-aided molecular modeling. These techniques aid in the development of novel compositions of higher potency and/or greater bioavailability and/or greater stability than the original polypeptide (Dean (1994), BioEssays, 16: 683-687; Cohen and Shatzmiller (1993), J. Mol. Graph., 11: 166-173; Wiley and Rich (1993), Med. Res. Rev., 13: 327-384; Moore (1994), Trends Pharmacol. Sci., 15: 124-129; Hruby (1993), Biopolymers, 33: 1073-1082; Bugg et al. (1993), Sci. Am., 269: 92-98, all incorporated herein by reference].

Thus, through use of the methods described above, the present invention provides compounds exhibiting enhanced therapeutic activity in comparison to the polypeptides described above. The peptidomimetic compounds obtained by the above methods, having the biological activity of the above named polypeptides and similar three-dimensional structure, are encompassed by this invention. It will be readily apparent to one skilled in the art that a peptidomimetic can be generated from any of the modified polypeptides described in the previous section or from a polypeptide bearing more than one of the modifications described from the previous section. It will furthermore be apparent that the peptidomimetics of this invention can be further used for the development of even more potent non-peptidic compounds, in addition to their utility as therapeutic compounds.

Specific examples of peptidomimetics derived from the polypeptides described in the previous section are presented below. These examples are illustrative and not limiting in terms of the other or additional modifications.

### XIX.E.1. Peptides With A Reduced Isostere Pseudopeptide Bond

Proteases act on peptide bonds. It therefore follows that substitution of peptide bonds by pseudopeptide bonds confers resistance to proteolysis. A number of pseudopeptide bonds have been described that in general do not affect polypeptide structure and biological activity. The reduced isostere pseudopeptide bond is a suitable pseudopeptide bond that is known to enhance stability to enzymatic cleavage with no or little loss of biological activity (Couder, et al. (1993), Int. J. Polypeptide Protein Res. 41:181-184, incorporated herein by reference). Thus, the amino acid sequences of these compounds may be identical to the sequences of their parent L-amino acid polypeptides, except that one or more of the peptide bonds are replaced by an isostere pseudopeptide bond. Preferably the most N-terminal peptide bond is substituted, since such a substitution would confer resistance to proteolysis by exopeptidases acting on the N-terminus.

### XIX.E.2. Peptides With A Retro-Inverso Pseudopeptide Bond

To confer resistance to proteolysis, peptide bonds may also be substituted by retro-inverso pseudopeptide bonds (Dalpozzo, et al. (1993), Int. J. Polypeptide Protein Res. 41:561-566, incorporated herein by reference). According to this modification, the amino acid sequences of the compounds may be identical to the sequences of their L-amino acid parent polypeptides, except that one or more of the peptide bonds are replaced by a retro-inverso pseudopeptide bond. Preferably the most N-terminal peptide bond is substituted, since such a substitution will confer resistance to proteolysis by exopeptidases acting on the N-terminus.

### XIX.E.3. Peptoid Derivatives

Peptoid derivatives of polypeptides represent another form of modified polypeptides that retain the important structural determinants for biological activity, yet eliminate the peptide bonds, thereby conferring resistance to proteolysis (Simon, et al., 1992, Proc. Natl. Acad. Sci. USA, 89:9367-9371 and incorporated herein by reference). Peptoids are oligomers of N-substituted glycines. A number of N-alkyl groups have been described, each corresponding to the side chain of a natural amino acid.

### XX. KITS

The invention provides for diagnostic and therapeutic kits related useful for therapeutic, diagnostic, and research applications. Exemplary kits are disclosed in U.S. Patents 5,773,024; 6,017,721; and 6,232,127 B1. The kits of the invention incorporate minicells, and/or include methods of using minicells described herein.

### XX.A. Diagnostic and Research Use Kit Components

In one embodiment, the invention relates to kits for determining the diagnosis or prognosis of a patient. These kits preferably comprise devices and reagents for measuring one or more marker levels in a test sample from a patient, and instructions for performing the assay. Optionally, the kits may contain one or more means for converting marker level(s) to a prognosis. Such kits preferably contain sufficient reagents to perform one or more such determinations.

More specifically, a diagnostic kit of the invention comprises any of the following reagents and/or components in any combination.
(1) A detectable or detectably labeled first reagent that binds a ligand of interest. The binding reagent can, but need not, be an antibody or an antibody derivative comprising a detectable moiety. The sphingolipid-binding reagent is stored in an openable container in the kit, or is bound to a surface of a substrate such that it is accessible to other reagents. Examples of the latter include test strips.
(2) If the first reagent in neither detectable nor detectably labeled, the kit may comprise a detectable or detectably labeled second reagent that binds to the first reagent (e.g., a secondary antibody) or which produces a detectable signal when in close proximity to the first reagent (e.g., as results from fluorescent resonance energy transfer FRET). In either case, the signal produced from the second reagent correlates with the amount of ligand in the sample.
(3) One or more positive control reagents. Typically, these reagents comprise a compound that is known to produce a signal in the assay. In one embodiment, the positive control reagents are standards, i.e., comprise a known amount of a detectable or detectably labeled compound, the signal from which may be compared to the signal from a test sample. In addition to serving as positive control reagents, they may be used to develop calibration curves that relate the amount of signal to the known concentration of a detectable or detectably labeled compound. The signal from a test sample is compared to the calibration curve in order to determine what concentration of the detectable or detectably labeled compound corresponds to the signal from the test sample. In this embodiment, the kit provides quantitative measurements of the amount of a ligand in a test sample.
(4) One or more negative control reagents. Typically, these control reagents may comprise buffer or another solution that does not contain any of the detectable or detectably labeled first or second reagents and should thus not produce any detectable signal. Any signal that is detected reflects the background level of "noise" in the assay. Another type of negative control reagent contains most of the components necessary for the signal of the assay to be produced, but lacks at least one such component and therefor should not produce a signal. Yet another type of negative control reagent contains all of the components necessary for the signal of the assay to be produced, but also contains an inhibitor of the process that produced the signal.
(5) One or more auxiliary reagents for use in the diagnostic assays of the kit, e.g., buffers, alcohols, acid solutions, etc. These reagents are generally available in medical facilities and thus are optional components of the kit. However, these reagents preferably are included in the kit to ensure that reagents of sufficient purity and sterility are used, since the resulting protein conjugates are to be administered to mammals, including humans, for medical purposes, and to provide kits that can be used in situations where medical facilities are not readily available, e.g., when hiking in places located far from medical facilities, or in situations where the presence of these auxiliary reagents allows for the immediate treatment of a patient outside of a medical facility as opposed to treatment that arrives at some later time).
(6) Instructions to a person using a kit for its use. The instructions can be present on one or more of the kit components, the kit packaging and/or a kit package insert.XX.B. Therapeutic Kit Components

A therapeutic kit of the invention comprises any of the following reagents and/or components in any combination.
(1) One or more therapeutic agents.
(2) If the therapeutic agent(s) are not formulated for delivery via the alimentary canal, which includes but is not limited to sublingual delivery, a device capable of delivering the therapeutic agent through some other routes. One type of device for parenteral delivery is a syringe that is used to inject the therapeutic agent into the body of an animal in need of the therapeutic agent. Inhalation devices may also be used.
(3) Separate containers, each of which comprises one or more reagents of the kit. In a preferred embodiment, the containers are vials contain sterile, lyophilized formulations of a therapeutic composition that are suitable for reconstitution. Other containers include, but are not limited to, a pouch, tray, box, tube, or the like. Kit components may be packaged and maintained sterilely within the containers.
(4) Instructions to a person using a kit for its use. The instructions can be present on one or more of the kit components, the kit packaging and/or a kit package insert. Such instructions include, by way of non-limiting example, instructions for use of the kit and its reagents, for reconstituting lyophilized reagents or otherwise preparing reagents.

A preferred kit of the present invention comprises the elements useful for performing an immunoassay. A kit of the present invention can comprise one or more experimental samples (i.e., formulations of the present invention) and one or more control samples bound to at least one pre-packed dipstick or ELISA plate, and the necessary means for detecting immunocomplex formation (e.g., labelled secondary antibodies or other binding compounds and any necessary solutions needed to resolve such labels, as described in detail above) between antibodies contained in the bodily fluid of the animal being tested and the proteins bound to the dipstick or ELISA plate. It is within the scope of the invention that the kit can comprise simply a formulation of the present invention and that the detecting means can be provided in another way.

An alternative preferred kit of the present invention comprises elements useful for performing a skin test. A kit of the present invention can comprise at least one pre-packed syringe and needle apparatus containing one or more experimental samples and/or one or more control samples. A kit according to the invention may be designed for both diagnostic and therapeutiuc applications. Any combination of the above elements XX.A.(1)-(6) and XX.B.(1)-(4) may be used in a kit, optionally with additional reagents, standards, sample containers, an the like.

### XXI. IMMUNOGENIC MINICELLS

### XXI.A. In General

Minicells are used to immunize subjects. An organism is said to be "immunized" when, after contact with an immunogen, the organism produces antibodies directed to the immunogen, or has increased proliferation or activity of cytotoxic and/or helper T cells, or both. Increased proliferation or activity of T cells may be particularly desirable in the case of parasites that cause a decrease in T cell proliferation.

The use of minicells to present antigens has several potential advantages. An intact membrane protein can be presented in its native form on the surface of an immunogenic minicell, rather than as a denatured protein or as oligopeptides derived from the amino acid sequence of a membrane protein, which allows for antibodies to be developed that are directed to epitopes which, due to protein folding, occur only in the native protein. The minicell surface may naturally be, or may be modified to be, an adjuvant. Moreover, pharmacokinetic properties of minicells, as discussed elsewhere herein, may be improved relative to other forms of administration.

The applications of immunogenic minicells include, but are not limited to, research, prophylactic, diagnostic and therapeutic applications.

In research applications, immunogenic minicells are used to generate antibodies to an antigen displayed on a minicell. Such antibodies are used to detect an antigen, which may be a chemical moiety, molecule, virus, organelle, cell, tissue, organ, or organism that one wishes to study. Classically, such antibodies have been prepared by immunizing an animal, often a rat or a rabbit, and collecting antisera therefrom. Molecular biology techniques can be used to prepare antibodies and antibody fragments, as is described elsewhere herein. Single-chain antibody fragments (scFv) may also be identified, purified, and characterized using minicells displaying a membrane protein or membrane bound chimeric soluble protein.

In prophylactic applications, immunogenic minicells are used to stimulate a subject to produce antibodies and/or activate T cells, so that the subject is "pre-immunized" before contact with a pathogen or hyperproliferative cell. Thus, in the case of a pathogens, the subject is protected by antibodies and/or T cells that are specifically directed to the pathogen before infection.

In therapeutic applications, immunogenic minicells are used in immunotherapy.

Certain aspects of the invention involve active immunotherapy, in which treatment relies on the in vivo stimulation of the endogenous host immune system to react against pathogens or tumors due to the administration of agents that cause, enhance or modulate an immune response. Such agents include, but are not limited to, immunogens, adjuvants, cytokines and chemokines.

Other therapeutic applications involve passive immunotherapy, in which treatment involves the delivery of agents (such as antibodies or effector cells) that are specifically directed to an immunogen of a pathogen or a hyperproliferative cell, and does not necessarily depend on an intact host immune system. Examples of effector cells include T cells; T lymphocytes, such as CD8+ cytotoxic T lymphocytes and CD4+ T-helper tumor-infiltrating lymphocytes; killer cells, such as Natural Killer (NK) cells and lymphokine-activated killer cells.

### XXI.B. Hyperproliferative Disorders

The immunogenic minicells of the invention can be used to treat hyperproliferative disorders by inducing an immune response to an antigen associated therewith. The term "hyperproliferative disorder" refers to disorders characterized by an abnormal or pathological proliferation of cells, for example, cancer, psoriasis, hyperplasia and the like.

For reviews of immunotherapy as applied to hyperproliferative disorders, see Armstrong et al., Cellular immunotherapy for cancer, BMJ 323:1289-1293, 2001; Evans, Vaccine therapy for cancer - fact or fiction?, Proc R Cell Physicians Edinb 31:9-16, 2001; Ravindranath and Morton, "Active Specific Immunotherapy with Vaccines," Chapter 61 in: Holland-Frei Cancer Medicine, Fifth Edition, Bast, Robert C., et al., editors, B.C. Decker, Inc., Hamilton, 2000, pages 800-814.

Types of cancers include without limitation fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, lymphoma, multiple myeloma, Waldenstrom's macroglobulinemia, and heavy chain disease.

Tumor specific antigens (TSAs), tumor-associated differentiation antigens (TADAs) and other antigens associated with cancers and other hyperproliferative disorders include, but are not limited to, C1 IAC, a human cancer associated protein (Osther, U.S. Patent 4,132,769); the CA125 antigen, an antigen associated with cystadenocarcinoma of the ovary, (Hanisch et al., Carbohydr. Res. 178:29-47, 1988; O'Brien, U.S. Patent No. 4,921,790); CEA, an antigen present on many adenocarcinomas (Horig et al., Strategies for cancer therapy using carcinembryonic antigen vaccines, Expert Reviews in Molecular Medicine, http://www-ermm.cbcu.cam.ac.uk: 1, 2000); CORA (carcinoma or orosomucoid-related antigen) described by Toth et al. (U.S. Patent No. 4,914,021); DF3 antigen from human breast carcinoma (Kufe, in U.S. Patent Nos. 4,963,484 and 5,053,489); DU-PAN-2, a pancreatic carcinoma antigen (Lan et al., Cancer Res. 45:305-310, 1985); HCA, a human carcinoma antigen (Codington et al., U.S. Patent 5,693,763); Her2, a breast cancer antigen (Fendly et al., The Extracellular Domain of HER2/neu Is a Potential Immunogen for Active Specific Immunotherapy of Breast Cancer, Journal of Biological Response Modifiers 9:449-455, 1990); MSA, a breast carcinoma glycoprotein (Tjandra et al., Br. J. Surg. 75:811-817, 1988); MFGM, a breast carcinoma antigen (Ishida et al., Tumor Biol. 10:12-22, 1989); PSA, prostrate specific antigen (Nadji et al., Prostatic-specific-antigen, Cancer 48:1229-1232, 1981); STEAP (six transmembrane epithelial antigens of the prostate) proteins (Afar et al., U.S. Patent 6,329,503); TAG-72, a breast carcinoma glycoprotein (Kjeldsen et al., Cancer Res. 48:2214-2220, 1988); YH206, a lung carcinoma antigen (Hinoda et al., Cancer J. 42:653-658, 1988); the p97 antigen of human melanoma (Estin et al., Recombinant Vaccinia Virus Vaccine Against the Human Melanoma Antigen p97 for Use in Immunotherapy, Proc. Natl Acad. Sci. USA, 85:1052-1056, 1988); and the melanoma specific antigen described by Pfreundschuh in U.S. Patent 6,025,191);

### XXI. B. Intracellular Pathogens

In certain aspects of the invention, vaccines comprising immunogenic minicells are used to prevent or treat diseases caused by intracellular pathogens. Vaccines may be prepared that stimulate cytotoxic T cell responses against cells infected with viruses including, but not limited to, hepatitis type A, hepatitis type B, hepatitis type C, influenza, varicella, adenovirus, herpes simplex type I (HSV-I), herpes simplex type II (HSV-II), rinderpest, rhinovirous, echovirus, rotavirus, respiratory syncytial virus, papilloma virus, papova virus, cytomegalovirus, echinovirus, arbovirus, huntavirus, coxsackie virus, mumps virus, measles virus, rubella virus, polio virus, human immunodeficiency virus type I (HIV-I), and human immunodeficiency virus type II (HIV-II). Vaccines also may be prepared that stimulate cytotoxic T cell responses against cells infected with intracellular obligates, including but not limited to Chlamydia, Mycobacteria and Rickettsia. Vaccines also may be prepared that stimulate cytotoxic T cell responses against cells infected with intracellular protozoa, including, but not limited to, leishmania, kokzidioa, and trypanosoma.

The causative agent of Lyme disease, the spirochete Borrelia burgdorfei, is also of interest. The outer surface proteins (Osps) A, B and C of B. burgdorfei are known antigens that are lipoproteins that associate with membranes. Amino-terminal cysteine residues in Osp proteins are the sites of triacyl lipid modifications that serve as membrane-anchoring moities. The N-terminal portions of the Osp proteins are highly conserved and are preferred portions for display on immunogenic minicells.

### XXI.C. Eukaryotic Pathogens

In addition to intracellular pathogens, other eukaryotic pathogens exist and may also be treated using immunogenic minicells displayed antigens therefrom. A number of antigens have been used to develop antiparasitic vaccines, e.g. the recombinant 45w protein of Taenia ovis; EG95 oncosphere proteins of Echinococcus granulosis; cathepsin L antigen of the liver fluke, Fasciola hepatica; and the H11 antigen of Haemonchus contortus (Dalton et al., Parasite vaccines--a reality?, Vet Parasitol 98:149-167, 2001). Other eukaryotic pathogens include, but are not limited to:
Protozoans, including but not limited to, Entamoeba histolytica, a pathogenic amoeba that causes amoebic dysentery and occasionally digests its way through the intestinal wall to invades other organs, which may cause morbidity; Balantinium coli, a ciliate that causes diarrhea in humans; Giardia lamblia, a flagellate that causes diarrhea and abdominal pain, along with a chronic fatigue syndrome that is otherwise asymptomatic and difficult to diagnose; Trypanosoma brucei, a hemoflagellate causing sleeping sickness; and Trypanosoma cruzi, the cause of Chagas disease);
Plasmodia, sporozoan obligate intracellular parasites of liver and red blood cells, including but not limited to P. falciparum, the causative agent of malaria. Dozens of P. falciparum antigens have been identified, e.g., CSP-1, STARP, SALSA, SSP-2, LSA-1, EXP-1, LSA-3, RAP-1, RAP-2, SERA-1, MSP-1, MSP-2, MSP-3, MSP-4, MSP-5, AMA-1, EBA-175, RESA, GLURP, EMP-1, Pfs25, Pfg27, Pf35, Pf55, Pfs230, Pfg27, Pfs16, Pfs28 and Pfs45/48.
Helminthes including but not limited to Ascaris lumbricoides (roundworm); Enterobius vermicularis (pinworm); Trichuris trichiuria (whipworm); and Fasciola hepatica (liver fluke);
Taenia sp. (tapeworms and cestodes);
Schistosoma (trematodes), such as Schistoma mansoni, which comprises the Sm32 antigen (asparaginyl endopeptidase), which can induce antibody formation in mice (Chlichlia et al., DNA vaccination with asparaginyl endopeptidase (Sm32) from the parasite Schistosoma mansoni: anti-fecundity effect induced in mice, Vaccine 20:439-447, 2001); and acetylcholinesterase (Arnon et al., Acetylcholinesterase of Schistoma mansoni-Functional correlates, Protein Science 8:2553-2561, 1999); and
Ticks and other invertebrates, including but not limited to insects, arachnids, etc. For example, a description of a vaccine against the cattle tick Boophilus microplus has been described (Valle et al., The evaluation of yeast derivatives as adjuvants for the immune response to the Bm86 antigen in cattle, BMC Biotechnol. 1:2, 2001)

### XXI.D. Formulation and Adminstration of Immunogenic Minicells

Vaccine formulations of immunogenic minicells include a suitable carrier. Because minicells may be destroyed by digestion, or prevented from acting due to antibody secretion in the gvut, they are preferably administered parenterally, including, for example, administration that is subcutaneous, intramuscular, intravenous, or intradermal. Formulations suitable for parenteral administration include aqueous and non-aqueous sterile injection solutions which may contain antioxidanits, buffers, and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampules and vials and may be stored in a freeze-dried condition requiring only the addition of the sterile liquid carrier immediately prior to use. The vaccine formulation may also include adjuvant systems for enhancing the immunogenicity of the formulation. Adjuvants are substances that can be used to augment a specific immune response. Normally, the adjuvant and the composition are mixed prior to presentation to the immune system, or presented separately, but into the same site of the mammal being immunized. Examples of materials suitable for use in vaccine compositions are provided in Osol, A., ed., Remington's Pharmaceutical Sciences, Mack Publishing Co, Easton, Pa. (1980), pp. 1324-1341, which reference is entirely incorporated herein by reference.

Compositions comprising immunogenic minicells are injected into a human or animal at a dosage of 1-1000 ug per kg body weight. Antibody titers against growth factor are determined by ELISA, using the recombinant protein and horseradish peroxidase-conjugated goat anti-human or animal immunoglobulins or other serologic techniques (e.g., sandwich ELISA). Booster injections are administered as needed to achieve the desired levels of protective antibodies and/or T cells.

Routes and frequency of administration, as well as dosage, will vary from individual to individual. Between 1 and 10 doses may be administered for a 52-week period. Preferably, 6 doses are administered, at intervals of 1 month, and booster vaccinations may be given periodically thereafter. Alternate protocols may be appropriate for individual patients. In immunotherapy of hyperproliferative disorders, a suitable dose is an amount of a compound that, when administered as described above, is capable of promoting an anti-tumor immune response. Such response can be monitored by measuring the anti-tumor antibodies in a patient or by vaccine-dependent generation of cytolytic effector cells capable of killing the patient's tumor cells in vitro. Such vaccines should also be capable of causing an immune response that leads to an improved clinical outcome (e.g., more frequent remissions, complete or partial or longer disease-free survival) in vaccinated patients as compared to non-vaccinated patients.

The vaccine according to the invention may contain a single species of immunogenic minicells according to the invention or a variety of immunogenic minicells, each of which displays a different immunogen. Additionally or alternatively, immunogenic minicells may each display and/or express more than one iummunogen.

The summary of the invention described above is non-limiting and other features and advantages of the invention will be apparent from the following detailed description of the invention, and from the claims.

### EXAMPLES

### EXAMPLE 1: CREATION OF A MINICELL-PRODUCING BACTERIAL CELL LINE (MC-T7) THAT EXPRESSES AN EXOGENOUS RNA POLYMERASE

In order to maximize the amount of RNA transcription from episomal elements in minicells, a minicell-producing cell line that expresses an RNA polymerase specific for certain episomal expression elements was created. This E. coli strain, designated MC-T7, was created as follows.

The P678-54 E. coli strain contains mutations that influence cell division and induce the production of minicells (Adler et al., Proc. Natl. Acad. Sci. 57:321-326 (1967), Allen et al., Biochem. Biophys. Res. Communi. 47:1074-1079 (1972), Hollenberg et al., Gene 1:33-47 (1976)). The P678-54 strain is resistant to Lambda phage due to a mutation in the malT gene (Gottesman, Bacteriophage Lambda: The Untold Story. J. Mol. Biol. 293:177-180, 1999; Friedman, Interactions Between Bacteriophage Lambda and its Escherichia Coli Host. Curr. Opin. Genet. Dev. 2:727-738, 1992). Thus, as an initial step, the P678-54 strain was altered so as to be sensitive to Lambda phage so that it could form lysogens of Lambda-DE3 (see below). Wildtype MaIT-encoding sequences were restored via a HFR (high frequency recombination) conjugation protocol using the G43 E. coli strain (CGSC stain 4928).

Recipient (P678-54) and donor (G43:BW6169) strains were grown overnight in 10 mL of LB media (10 g g NaCl, 10 g select peptone 140, and 5 g yeast extract in one liter ddH₂0). The samples were centrifuged and then concentrated in about 0.2 mL of LB media. The concentrated samples were combined and incubated with slow rotation for 30 minutes at 30°C, and were then plated on LB agar plates that contained streptomycin (50 µg/mL) and tetracycline (50 µg/mL). (Ampicillin, streptomycin, tetracycline, and all other chemicals were purchased from Sigma Chemical (St. Louis, MO) unless otherwise indicated.) Recipient cells were resistant to streptomycin and donor cells were resistant to tetracycline; only conjugates, which contained both resistance genes, were able to grow on the LB agar plates that contained streptomycin (50 µg/mL) and tetracycline (50 µg/mL).

Putative conjugates were screened for Lambda phage sensitivity using a cross streak technique, in which putative colonies were cross-streaked on an LB agarose plate (streptomycin, 50 µg/mL, and tetracycline, 50 µg/mL) that had been streaked with live Lambda phage. The streaked conjugate colonies were streaked perpendicular to the Lambda phage streak; if a conjugate was sensitive to Lambda phage infection then, upon contact with the Lambda phage streak, there was cell lysis and thus less or no bacterial growth. Thus, in the case of conjugates that were sensitive to Lambda phage, there was deceased bacterial growth "downstreak" from the phage streak.

The conjugate E. coli that were found to be sensitive to Lambda phage infection were then used to create Lambda lysogens. Lysogenization is a process during which Lambda phage incorporates its genome, including exogenous genes added thereto, into a specific site on the chromosome of its E. coli host cell.

The DE3 gene, which is present in the genome of the Lamda phage used to create lysogens, encodes RNA polymerase from bacteriophage T7. Lysogenation was carried out using the DE3-Lysogenation kit (Novagen, Madison, WI) essentially according to the manufacturer's instructions. A T7 polymerase dependent tester phage was used to confirm the presence and expression of the DE3 gene on the bacterial chromosome. The T7-dependent tester phage can only form plaques on a baterial known in the presence of T7 polymerase. The phage uses a T7 promoter for expression of its essential genes. Therefore in a plaque-forming assay only cells which express T7 polymerase can be lysed by the tester phage and only these cells will allow for the formation of plaques. As is described in more detail herein, episomal expression elements that are used in minicells may be designed such that transcription and translation of a cloned gene is driven by T7 RNA polymerase by utilizing expression sequences specific for the T7 RNA polymerase.

### EXAMPLE 2: CLONING OF RAT EDG-1 INTO THE PCAL-C EXPRESSION VECTOR

### Materials

Taq Polymerase, PCR Buffers, and PCR reagents were purchased from Roche Molecular Biochemicals (Indianapolis, IN). All restriction enzymes were purchased from Gibco BRL (Grand Island, NY) and Stratagene (La Jolla, CA). QIAprep mini and maxi kits, PCR purification Kits, RNeasy miniprep kits, and the One Step RT-PCR Kit were purchased from QIAGEN (Valencia, CA). The Geneclean Kit was purchased from BIO 101 (Carlsbad, CA). IPTG (isopropy-beta-D-thiogalactopyranoside), T4 DNA Ligase, LB Media components and agarose were purchased from Gibco BRL. The pCAL-c prokaryote expression vector and competent cells were purchased from Stratagene.

The pCAL-c expression vector has a structure in which an ORF may be operably linked to a high-level (but T7 RNA polymerase dependent) promoter, sequences that bind the E. coli Lac repressor, and the strong T7 gene 10 ribosome-binding site (RBS). The LacI repressor is also encoded by an expressed from te pCAL-c vector. As long as it is bound to its recognition sequences in the pCAL-c expression element, the lac repressor blocks transcription from the T7 promoter. When an inducing agent, such as IPTG is added, the lac repressor is released from its binding sites and transcription proceeds from the T7 protmoter, provided the T7 RNA polymerase is present. After induction, the cloned and expressed protein may constitute the majority of newley expressed cellular proteins due to the efficient transcription and translation processes of the system.

### Amplification

The first step in cloning rat Edg-1 (rEDG-1) into an expression vector was to design primers for amplification via PCR (polymerase chain reaction). PCR primers were designed using the rat Edg-1 sequence (Nakajima et al., Biophy, J. 78:319A, 2000) in such a manner that they contained either sites for Nhel (GCTAGC) or BamHI (GGATCC) on their five prime ends. The upstream primer had the sequence of SEQ ID NO:31. The three prime downstream primer (SEQ ID NO:32) also contained a stop codon, as the pCAL-c vector contains a Calmodulin Binding Protein (CBP) "tag" at its carboxyl terminus which was not intended to be incorporated into the rat Edg-1 polypeptide in this expression construct. The primer and resulting PCR products were designed so that the five prime end of the rat Edg-1 ORF was in frame with the methionine start codon found in the pCAL-c vector.

### OLIGONUCLEOTIDE PRIMER SEQUENCES FOR CLONING INTO PCAL-C:

**Edg1/pCAL-c construct primers:**
   Upstream primer (SEQ ID NO:31)
      5'-AATTGCTAGCTCCACCAGCATCCCAGTGGTTA-3'
   Downstream primer (SEQ ID NO:32)
      5'-AATTGGATCCTTAAGAAGAAGAATTGACGTTT-3'
**Edg1/CBP fusion construct primers:**
   Upstream primer (SEQ ID NO:31)
      5'-AATTGCTAGCTCCACCAGCATCCCAGTGGTTA-3'
   Downstream primer (SEQ ID NO:33)
      5'-AATTGGATCCAGAAGAAGAATTGACGTTTCCA-3'
**Edg1/His6 construct primers:**
   Upstream primer (SEQ ID NO:31)
      5'-AATTGCTAGCTCCACCAGCATCCCAGTGGTTA-3'
   Downstream primer (SEQ ID NO:34)
      5'-AATTGGATCCTTAATGATGATGATGATGATGAGAAGAAGAATTGACGTTTCC-3'
**Edg3/rtPCR primers:**
   Upstream primer (SEQ ID NO:35)
      5'-TTATGGCAACCACGCACGCGCAGG-3'
   Downstream primer (SEQ ID NO:36)
      5'-AGACCGTCACTTGCAGAGGAC-3'
**Edg3/pCAL-c construct primers:**
   Upstream primer (SEQ ID NO:37)
      5'-AATTGCTAGCACGCACGCGCAGGGGCACCCGC-3'
   Downstream primer (SEQ ID NO:38)
      5'-AATTGGTACCTCACTTGCAGAGGACCCCATTCTG-3'
**Edg3/His6 construct primers:**
   Upstream primer (SEQ ID NO:39)
      5'-AATTGCTAGCACGCACGCGCAGGGGCACCCGC-3'
   Downstream primer (SEQ ID NO:16)
      5'-AATTGGTACCTCAATGATGATGATGATGATGCTTGCAGAGGACCCCATTCTG-3'
**GFP/pCAL-c construct primers:**
   Upstream primer (SEQ ID NO:40)
      5'-GGTCGCCACCATGGTGAGCAA-3'
   Downstream primer (SEQ ID NO:41)
      5'-TTAAGGATCCTTACTTGTACAGCTCGTCCAT-3'
**GFP/CBP construct primers:**
   Upstream primer (SEQ ID NO:42)
      5'-GGTCGCCACCATGGTGAGCAA-3'
   Downstream primer (SEQ ID NO:43)
      5'-TTAAGGATCCCTTGTACAGCTCGTCCATGCC-3'

### Notes:

Restriction endonuclease sites are underlined

Stop codons are double underlined

The primers were used to amplify the rEdg-1 DNA ORF using the polymerase chain reaction (PCR). The template used for amplification was mRNA isolated from rat muscle tissue using the RNeasy Miniprep Kit (Qiagen) and was carried out essentially according to the manufacturer's protocol. Both the rtPCR and PCR amplification steps were carried out in a single reaction using the One Step RT-PCR Kit (Qiagen). The resulting rat Edg-1 PCR fragment was purified using the PCR Purification Kit (Qiagen). The amplified double stranded rEdg-1 DNA sequence contained the Nhel site at the 5-prime end and the BamHI site at the 3-prime end. This amplified rEdg-1 fragment was used for cloning into the pCAL-c expression vector.

The pCAL-c expression vector contains Ncol, Nhel, and BamHI restriction sites in its multiple cloning site. In order to insert rEdg-1-encoding sequence into the expression vector, the rEdg-1 PCR fragment and the pCAL-c expression vector were digested with Nhel and BamHI restriction enzymes for one hour at 37°C. The reaction mixture for the digestion step consisted of 1 µg of DNA, 1x restriction buffer, and 1 µL of each enzyme. The reaction mixture was brought to a final volume of 20 µL with ddH₂O (dd, double distilled). After 45 minutes, 1 µL of Calf Intestine Alkaline Phosphatase (CIAP) was added to the pCAL-c reaction mixture in order to remove the terminal phosphates from the digested plasmid DNA. The reactions were incubated for an additional 15 minutes at 37°C. The digested DNA samples were then run on a 1% TAE (Tris-acetate/EDTA electrophoresis buffer) agarose gel at 130 volts for 45 minutes. The bands were visualized with UV light after the gel was stained with ethidium bromide.

The appropriate bands were cut out of the gel for purification using the Geneclean Kit (BIO101). The Purified DNA fragments were then quantified on a 1% TAE agarose gel. For the ligation reaction, ratios of insert to vector of 6:1 and 3:1 were used. A negative control comprising vector only was also included in the ligation reactions. The reaction mixtures contained insert and vector DNA, 4 µL Ligase buffer, and 2 µL Ligase. The reaction was brought up to a final volume of 20 µL with ddH₂O. The ligation was carried out at room temperature for about 2 hours. Ten (10) µL of the ligation reaction mixture was used for subsequent transformation steps.

Ligated DNA was introduced into Epicurian Coli XL1-Blue competent cells using the heat shock transformation technique as follows. The ligation mixture was added to 100 µL of competent cells, placed on ice, and was incubated for about 30 minutes. The cells were then heat shocked at 37°C for 1 minute and put back on ice for 2 minutes. Following heat shock, 950 µL of room temperature LB media was added to the cells and the cells were shaken at 37°C for 1 hour. Following the 1-hour agitation the cells were pelleted for one minute at 12000 rpm in a Eppendorf 5417C microcentrifuge. The supernatant was carefully poured off so that about 200 µL remained. The cells were then resuspended in the remaining LB media and spread on 100x15 mm LB agarose plates containing 50 µg/mL ampicilin. The plates were incubated overnight at 37°C. Colonies were counted the following day, and the ratio of colonies between the negative control and the ligated samples was determined. A high ratio of the number of colonies when the ligation mixture was used to transform cells, as contrasted to the number of negative control colonies indicated that the cloning was successful. Transformed colonies were identified, isolated, and grown overnight in LB media in the presence of ampicillin. The resulting bacterial populations were screened for the presence of the Edg-1-pCAL-c expression construct.

Plasmid DNA was isolated from the cells using the QIAprep Spin Miniprep Kit (Qiagen). Isolated Edg-1-pCAL-c constructs were screened using the restriction enzyme ApaI, which digests the Edg-1-pCAL-c construct at two different sites: one in the Edg-1 coding sequence and one in the pCAL-c vector itself. The plasmid preparations were digested with ApaI electrophoresed on a 1% TAE agarose gel and visualized using uv light and ethidium bromide staining. The predicted sizes of the expected DNA fragments were 2065 bp and 4913 bp. As shown in Figure 3, bands of the predicted size were present on the gel. The entire Edg-1-pCAL-c construct was sequenced in order to confirm its structure. This expression construct, a pCAL-c derivative that contains the rat Edg-1 ORF operably linked to a T7 promoter and lac repressor binding sites, is designated "prEDG-1" herein.

### EXAMPLE 3: CONSTRUCTION OF RAT EDG-1-CBP FUSION PROTEIN

In order to detect rat Edg-1 protein expression, rEdg-1 coding sequences were cloned into the pCAL-c vector in frame with a CBP fusion tag. The cloning strategy for the rEdg-1-CBP construct was performed essentially as described for the Edg-1-pCAL-c construct with the following differences. The PCR primers (SEQ ID NOS:3 and 5) were as described for the Edg-1-pCAL-c cloning except for the omission of the stop codon in the downstream primer (SEQ ID NO:33). The removal of the stop codon is required for the construction of the Edg-1-CBP fusion protein. The pCAL-c vector is designed so that, when the BamHI site is used for insertional cloning, and no stop codon is present in an ORF inserted into the pCAL-c expression vector the cloned ORF will be in-frame with the CBP fusion tag. Because the three prime downstream primer did not contain a stop codon, a CBP fusion tag could be cloned in-frame with the Edg-1 ORF. Other cloning steps were performed essentially as described before. The resulting plasmid, a pCAL-c derivative that comprises an ORF encoding a rat Edg-1-CBP fusion protein operably linked to a T7 promoter and lac repressor binding sites, is designated "prEDG-1-CBP" herein.

### EXAMPLE 4: CLONING OF A HIS-TAGGED RAT EDG-1 INTO PCAL-C EXPRESSION VECTOR

The rEdg-1 protein was manipulated to generate a fusion protein having a 6xHis tag at its carboxyl terminus. A "6xHis tag" or "His tag" is an amino acid sequence consisting of six contiguous histidine residues that can be used as an epitope for the binding of anti-6xHis antibodies, or as ligand for binding nickel atoms. The His-tagged rEdg-1 fusion protein is used to detect rEdg-1 protein expression in the minicell expression system environment.

The rEdg-1-6xHis construct was cloned using the strategy described above for the construction of the rEdg-1-pCAL-c expression construct (prEDG-1), with the upstream primer having the sequence of SEQ ID NO:3, but with the exception that the three prime downstream primer (SEQ ID NO:34) was designed to contain six histidine codons followed by a stop codon. The 18 base pair 6xHis tag was incorporated into the carboxyl terminus of the Edg-1 protein as expressed from the pCAL-c vector. Subsequent cloning procedures (PCR, restriction digest, gel purification, ligation, transformation, etc.) were performed as described previously for the Edg-1-pCAL-c construct (prEDG-1). The resulting plasmid, a pCAL-c derivative that comprises an ORF encoding a carboxy-terminal His-tagged rat Edg-1-CBP fusion protein operably linked to a T7 promoter and lac repressor binding sites, is designated "prEDG-1-6xHis" herein.

### EXAMPLE 5: AMPLIFICATION AND CLONING OF RAT EDG-3 SEQUENCES

The Edg-3 full length coding sequence was amplified via PCR from rat skeletal muscle mRNA using primers (SEQ ID NOS:35 and 36) designed from the known mouse sequence (Genbank accession NM_010101). The mRNA used as a template for the amplification reaction was isolated using the RNeasy Miniprep Kit (Qiagen). Both the rtPCR and PCR amplification steps were carried out in a single reaction using the One Step RT-PCR Kit (Qiagen). The rEdg-3 PCR products were visualized with UV after electrophoresis in 1% TAE agarose gels and ethidium bromide staining.

The predicted size of the amplified PCR products is 1145 base pairs. An appropriately-sized DNA band was isolated from the TAE gel and purified using the Geneclean Kit (BIO101). The purified band was ligated to the pCR3.1 vector using the TA-cloning kit (Invitrogen). Other cloning steps were carried out as described previously for the cloning of the rEdg-1-pCAL-c construct (prEDG-1) with the exception that the samples were screened using the EcoRI restriction enzyme. The expected sizes of the digested bands were 1145 base pairs and 5060 base pairs. Positive clones were analyzed by automated sequencing. The nucleotide sequences were analyzed using BLAST searches from the NCBI web site (www.ncbi.nlm.nih.gov/). The predicted full length rat Edg-3 amino acid sequence was assembled from the nucleotide sequencing data using in silico translation. The pCR3.1 vector comprising the rat Edg-3 ORF is designated "pCR-rEDG-3" herein.

### EXAMPLE 6: CLONING OF RAT EDG-3 CODING SEQUENCES INTO THE PCAL-C EXPRESSION VECTOR

In order to express it in the minicell expression system, the rat Edg-3 ORF was cloned into the pCAL-c expression vector. The cloning strategy used was as described above for the cloning of the rat Edg-1 gene into the pCAL-c vector with the following exceptions. The primers used for PCR amplification were designed from the rat Edg-3 sequence and contained sites for the restriction enzymes Nhel and KpnI (GGTACC). The Nhel site was added to the five prime upstream primer (SEQ ID NO:37) and the Kpnl site was added to the three prime downstream primer; SEQ ID NO:38). The NheI and KpnI restriction enzymes were used for the digestion reaction. The reaction mixture for the digestion step consisted of 1 µg of DNA, 1x restriction buffer (provided with the enzyme), and 1 µL of each enzyme. Plasmid preparations were screened by digestion with Nhel and KpnI. The digested plasmid DNA was electrophosesed on a TAE agarose gel and visualized by UV after staining with ethidium bromide. The resultant band sizes were predicted to be 1145 base pairs and 5782 base pairs. The positive plasmid clones were analyzed with automated sequencing. The resulting plasmid, a pCAL-c derivative that comprises an ORF encoding a rat Edg-3 protein operably linked to a T7 promoter and lac repressor binding sites, is designated "pEDG-3" herein.

### EXAMPLE 7: CLONING OF A HIS-TAGGED RAT EDG-3 INTO THE PCAL-C EXPRESSION VECTOR

In order to detect expression of the rat Edg-3 protein in the minicell expression system, the rat Edg-3 coding sequence was manipulated so as to contain a 6xHis tag at the carboxyl terminus of the protein. The cloning strategy used to create this construct was essentially the same as described above for the rEdg-3-pCAL-c (prEDG-3) construct cloning, with the upstream primer having the sequence of SEQ ID NO:37, with the exception that the three-prime downstream primer (SEQ ID NO: 18) was designed to contain a 6xHis coding sequence followed by a stop codon, which allowed for the incorporation of the 6xHis amino acid sequence onto the carboxyl terminus of the Edg-3 receptor protein. Other cloning and screening steps were performed as described above. The resulting plasmid, a pCAL-c derivative that comprises an ORF encoding a carboxy-terminal His-tagged rat Edg-3 fusion protein operably linked to a T7 promoter and lac repressor binding sites, is designated "prEDG-3-6xHis" herein.

### EXAMPLE 8: GFP CLONING INTO PCAL-C EXPRESSION CONSTRUCT

Cloning of GFP-encoding nucleotide sequences into the pCAL-c vector was performed in order to produce an expression construct having a reporter gene that can be used to detect protein expression (GFP, green flourescent protein). The cloning strategy used was essentially the same as the cloning strategy described above with the following exceptions. The template used for PCR amplification was the peGFP plasmid "construct" (GFP construct sold by Clontech). The primers used for amplification were designed from the GFP coding sequence and contained sites for the restriction enzymes Ncol and BamHI. The Ncol site was added to the five prime upstream primer (SEQ ID NO:40) and the BamHI site was added to the three prime downstream primer; see SEQ ID NO:41) The NcoI and BamHI restriction enzymes were used for the digestion reaction. The reaction mixture for the digestion step consisted of 1 µg of DNA, 1x restriction buffer (provided with the enzyme), and 1 µL of each enzyme. The screening of the plasmid preparations was carried out using Ncol and BamHI. Digested plasmid preparations were electrophoresed and visualized on TAE agarose gels with UV after staining with ethidium bromide. Restriction products having the predicted sizes of 797 and 5782 base pairs were seen. Positive plasmid clones were sequenced using an automated sequencer. The resulting plasmid, a pCAL-c derivative that comprises an ORF encoding a rEdg-3-GFP fusion protein operably linked to a T7 promoter and lac repressor binding sites, is designated "prEDG-3-GFP" herein.

### EXAMPLE 9: DESIGN CONSTRUCTION OF CONTROL EXPRESSION ELEMENTS

Control expression elements used to detect and quantify expression of proteins in minicells were preposed. These controls direct the expression of detectable proteins. An expression element used as positive control is pPTC12, which is supplied with the pCAL-c expression vector from Stratagene. This construct contains an ORF encoding a fusion protein comprising beta-galactosidase linked to CBP. Induction of expression of pTC12 should result in the production of a protein of about 120 kD, and this protein is detected via its enzymatic activity or by using antibodies directed to epitopes on the beta-galactosidase or CBP polypeptide.

A GFP fusion construct was created and used as a positive control for the CBP detection kit. This construct was a positive control for induction of protein expression in the minicell expression system. The cloning strategy used to create the construct was essentially the same as that used for the cloning of the GFP into the pCAL-c expression vector, with the exception that the three prime downstream primer did not contain a stop codon; this allowed for the in frame incorporation of the CBP fusion tag to the GFP protein. The upstream primer had the sequence of SEQ ID NO:42, and the downstream primer had the sequence SEQ ID NO:43. The nucleotide sequence of the expression element was confirmed using an automated sequencer. The resulting plasmid, a pCAL-c derivative that comprises an ORF encoding GFP operably linked to a T7 promoter and lac repressor binding sites, is designated "pGFP-CBP" herein.

### EXAMPLE 10: INTRODUCTION OF PCAL-C EXPRESSION CONSTRUCTS INTO THE MC-T7 ESCHERICHIA COLI STRAIN

The MC-T7 E. coli strain was made competent using the CaCl₂ technique. In brief, cells were grown in 40 mL LB medium to an OD₆₀₀ of 0.6 to 0.8, and then centrifuged at 8000 rpm (7,700 g) for 5 min at 4°C. The pellet was resuspended in 20 mL of cold CaCl₂ and left on ice for five minutes. The cells were then centrifuged at 8000 rpm (7,700 g) for 5 min at 4°C. The cell pellet was resuspended in 1 mL of cold CaCl₂ and incubated on ice for 30 min. Following this incubation 1 mL of 25% glycerol was added to the cells and they were distributed and frozen in 200 µL aliquots. Liquid nitrogen was used to freeze the cells. These cells subsequently then used for the transformation of expression constructs.

### EXAMPLE 11: PREPARATION OF MINICELLS

To some degree, the preparation of minicells varied according to the type of expression approach that is used. In general, there are two such approaches, although it should be noted from the outset that these approaches are neither limiting nor mutually exclusive. One approach is designed to isolate minicells that already contain an expressed therapeutic protein or nucleic acid. Another approach is designed to isolate minicells that will express the protein or nucleic acid in the minicell following isolation.

E. coli are inoculated into bacterial growth media (e.g., Luria broth) and grown overnight. After this, the overall protocol varies with regards to methods of induction of expression. The minicell producing cultures used to express protein post isolation are diluted and grown to the desired OD₆₀₀ or OD450, typically in the log growth phase of bacterial cultures. The cultures are then induced with IPTG and then isolated. The IPTG concentration and exposure depended on which construct was being used, but was usually about 500 µM final for a short time, typically about 4 hours. This treatment results in the production of the T7 polymerase, which is under control of the LacUVR5 promoter, which is repressed by the Lacl repressor protein. IPTG relieves the Lacl repression and thus induces expression from the LacUVR5 promoter which controls expression of the T7 polymerase from the chromosome. This promoter is "leaky" that is, there is always a basal level of T7 polymerase which can be selected for or against so that the induction before isolation is not required. (This induction step is not required if a non-T7 expression system is used, as the reason for this step is to express the T7 RNA polymerase in the minicell-producing cells so that the polymerase and molecules segregate with the minicell.)

The E. coli cultures that produce minicells containing a therapeutic protein or nucleic acid have different induction protocols. The overnight cultures are diluted as described above; however, in the case of proteins that are not toxic to the parent cells, this time the media used for dilution already contains IPTG. The cultures are then grown to mid-log growth and minicells are isolated. These cultures produce the therapeutic protein or nucleic acid as they grow, and the minicells derived therefrom contain the therapeutic protein or nucleic acid.

Altenatively or additionally, IPTG is added and expression is induced after the isolation of minicells. In the case of non-toxic proteins or nucleic acids that are expressed from expression elements in minicells, this treatment enhances production of the eposimally encoded gene product. In the case of toxic gene products induction post-isolation is preferred.

### EXAMPLE 12: MINICELL ISOLATION

Minicells were isolated from the minicell producing MC-T7 strain of E. coli using centrifugation techniques. The protocol that was used is essentially that of Jannatipour et al. (Translocation of Vibrio Harveyi N,N'-Diacetylchitobiase to the Outer Membrane of Escherichia Coli, J. Bacteriol. 169: 3785-3791, 1987) and Matsumura et al. (Synthesis of Mot and Che Products of Escherichia coli Programmed by Hybrid ColE1 Plasmids in Minicells, J. Bacteriol. 132:996-1002, 1977).

In brief, MC-T7 cells were grown overnight at 37°C in 2 to 3 mL of LB media containing ampicillin (50 µg/mL), streptomycin (50 µg/mL), and tetracycline (50 µg/mL) (ampicillin was used only when growing MC-T7 cells containing a pCAL-c expression construct). The cells were diluted 1:100 in a total volume of 100 to 200 mL LB media with antibiotics, and grown at 37°C until they reached an OD₆₀₀ of 0.4 to 0.6, which is roughly beginning of the log growth phase for the MC-T7 E. coli. During this incubation the remainder of the overnight culture was screened for the presence of the correct expression construct using the techniques described above. When the cultures reached the appropriate OD₆₀₀ they were transferred to 250 mL GS3 centrifuge bottles and centrifuged (Beckman centrifuge) at 4500 rpm (3,500 g) for 5 min. At this point the supernatant contains mostly minicells, although a few relatively small whole cells may be present.

The supernatant was transferred to a clean 250 mL GS3 centrifuge bottle and centrifuged at 8000 rpm (11,300 g) for 10 min. The pellet was resuspended in 2 mL of 1x BSG (10x BSG: 85 g NaCl, 3 g KH₂PO₄, 6 g Na₂HPO₄, and 1 g gelatin in 1 L ddH₂O) and layered onto a 32 mL 5 to 20% continuous sucrose gradient. The sucrose gradient was made with sucrose dissolved in 1x BSG.

The sucrose gradient was then loaded in a Beckman SW24 rotor and centrifuged in a Beckman Ultracentrifuge at 4500 rpm (9,000 g) for 14 min. Following ultracentrifugation a single diffuse band of minicells was present. The top two thirds of this band was aspirated using a 10 mL pipette and transferred to a 30 mL Oakridge tube containing 10 mL of 1x BSG. The sample was then centrifuged at 13,000 rpm (20,400 g) for 8 min. Following centrifugation, the pellet was resuspended in 2 mL 1x BSG, and the resuspended cells were loaded onto another 5 to 20% sucrose gradient. This sucrose gradient was centrifuged and the minicells were collected as described above. The sucrose gradient procedure was repeated a total of three times.

Following the final sucrose gradient step the entire minicell band was collected from the sucrose gradient and added to a 30 mL Oakridge tube that contained 10 mL of MMM buffer (200 mL 1x M9 salts, 2 mL 20% glucose, and 2.4 mL DIFCO Methionine Assay Medium). This minicell solution was centrifuged at 13,000 rpm (20,400 g) for 8 min. The pellet was resuspended in 1 mL of MMM Buffer.

The concentration of minicells was determined using a spectrophotometer. The OD₄₅₀ was obtained by reading a sample of minicells that was diluted 1:100.

### EXAMPLE 13: OTHER METHODS TO PREPARE AND ISOLATE MINICELLS

By way of non-limiting example, induction of E. coli parental cells to form minicells may occur by overexpression of the E. coli ftsZ gene. To accomplish this both plasmid-based and chromosomal overexpression constructs were created that place the ftsZ gene under the control of various regulatory elements (Table 5).

**Table 5. Regulatory constructs controlling ftsZ expression.**

| Regulatory region | inducer | [inducer] | SEQ ID NO.: |
|---|---|---|---|
| Para::ftsZ | Arabinose | 10mM | 1,3 |
| Prha::ftsZ | Rhamnose | 1 mM | 2,4 |
| Ptac::ftsZ | IPTG | 30 µM | 5, Garrido et al.^{a} |

| | | | |
|---|---|---|---|
| a. Garrido, T. et al. 1993. Transcription of ftsZ oscillates during the cell cycle of Escherichia coli. | | | |

### Oligonucleotide names and PCR reactions use the following format:

- "gene-1" is N-terminal, 100% homology oligo for chromosomal or cDNA amplification
- "gene-2" is C-terminal, 100% homology oligo for chromosomal or cDNA amplification
- "gene-1-RE site" is same sequence as gene-1 with additional residues for remainder of sequence, RE sites, and/or chimeric fusions.
- "gene-2-RE site" is same sequence as gene-1 with additional residues for remainder of sequence, RE sites, and/or chimeric fusions.
Use "gene-1, 2" combo for chromosomal/cDNA amplification and "gene-1 RE site, gene-2-RE site" to amplify the mature sequence from the "gene-1, 2" gel-purified product.

**TABLE 6: OLIGONUCLEOTIDE PRIMER SEQUENCES FOR TABLE 5 CONSTRUCTS**

| SEQ ID NO.: | Primer name | 5' to 3' sequence |
|---|---|---|
| 44 | FtsZ-1 | CCAATGGAACTTACCAATGACGCGG |
| 45 | FtsZ-2 | GCTTGCTTACGCAGGAATGCTGGG |
| 46 | FtsZ-1-Pstl | CGCGGCTGCAGATGTTTGAACCAATGGAACTTACCAATGACGCGG |
| 47 | FtsZ-2-Xbal | GCGCCTCTAGATTATTAATCAGCTTGCTTACGCAGGAATGCTGGG |

Table 7 oligonucleotide sequences are for use in cloning ftsZ into SEQ ID NO.:1 and 2 (insertions of ftsZ behind the arabinose promotor (SEQ ID NO.: 1) and the rhamnose promotor (SEQ ID NO.: 2).

**TABLE7: OLIGONUCLEOTIDE PRIMER SEQUENCES FOR FTSZ CHROMOSOMAL DUPLICATION CONSTRUCTS**

| SEQ ID NO.: | Primer name | 5' to 3' sequence |
|---|---|---|
| 48 | Kan-1 | 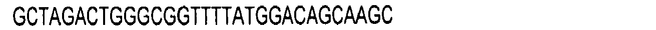 |
| 49 | Kan-2 | 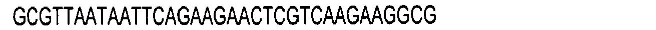 |
| 50 | Kan-1-X-frt | 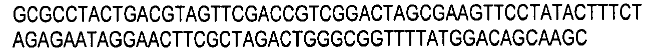 |
| 51 | Kan-2-intD-frt | 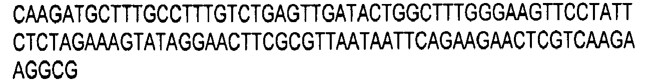 |
| 52 | AraC-1 | 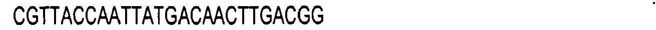 |
| 53 | RhaR-1 | 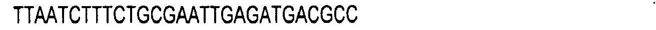 |
| 54 | Lacl^{q}-1 | 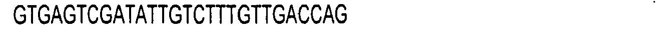 |
| 55 | Ara-1-intD | 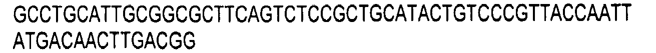 |
| 56 | RhaR-1-intD | 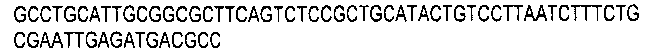 |
| 57 | Lacl^{q}-1-intD | 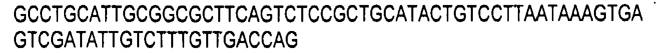 |
| 58 | FtsZ-1-X | 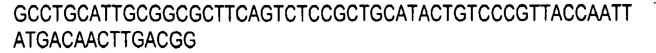 |

In like fashion, the ftsZ gene was amplified from SEQ ID NO.: 1, 2 and Ptac::ftsZ (Garrido, T. et al. 1993. Transcription of ftsZ oscillates during the cell cycle of Escherichia coli. EMBO J. 12:3957-3965) plasmid and chromosomal constructs, respectively using the following oligonucleotides:
For amplification of araC through ftsZ of SEQ ID NO.: 1 use oligonucleotides:
   AraC-1
   FtsZ-2
For amplification of rhaR through ftsZ of SEQ ID NO.: 2 use oligonucleotides:
   RhaR-1
   FtsZ-2
For amplification of lacI^{q} through ftsZ of Ptac::ftsZ (Garrido, T., et al.) use oligonucleotides:
   lacI^{q}-1
   ftsZ-2

The above amplified DNA regions were gel-purified and used as template for the second round of PCR using oligonucleotides containing homology with the E. coli chromosomal gene intD and on the other end with random sequence termed "X". Oligonucleotides used in this round of PCR are shown below:
For amplification of araC through ftsZ from SEQ ID NO.: 1 to contain homology to intD and the random X use oligonucleotides:
   AraC-1-intD
   FtsZ-1-X
For amplification of rhaR through ftsZ from SEQ ID NO.: 2 to contain homology to intD and the random X use oligonucleotides:
   RhaR-1-intD
   FtsZ-1-X
For amplification of lacIq through ftsZ from Ptac::ftsZ to contain homology to intD and the random X use oligonucleotides:
   LacIq-1-intD
   FtsZ-1-X
The PCR products from these PCR reactions are as shown below:
   intD - araC - Ara promotor - ftsZ - "X"
   intD - rhaRS - Rha promotor - ftsZ - "X"
   intD - lacI^{q} - Ptac promotor - ftsZ - "X"
To amplify the mature complexes, the following regions were mixed and amplified with the coupled oligonucleotide sequence primers:
SEQ ID NO.: 3 was produced using: 
intD - araC - Ara promotor - ftsZ - "X"
"X" - frt - Kan - frt - intD
AraC-1-intD
Kan-2-intD-frt
intD - araC - Ara promotor - ftsZ - "X" - frt - Kan - frt - intD
SEQ ID NO.: 4 was produced using: 
intD - rhaRS - Rha promotor - ftsZ - "X"
"X" - frt - Kan - frt - intD
RhaR-1-intD
Kan-2-intD-frt
intD - rhaRS - Ara promotor - ftsZ - "X" - frt - Kan - frt - intD
SEQ ID NO.: 5 was produced using:

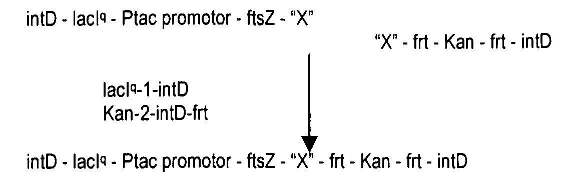
intD - lacIq - Ptac promotor - ftsZ - "X"
"X" - frt - Kan - frt - intD
lacIq-1-intD
Kan-2-intD-frt
intD - lacIq - Ptac promotor - ftsZ - "X" - frt - Kan - frt - intD

These expression constructs may be expressed from the plasmid, placed in single copy, replacing the native ftsZ copy on the E. coli chromosome (Garrido, T., et al. 1993. Transcription of ftsZ oscillates during the cell cycle of Escherichia coli. EMBO J. 12:3957-3965), or in duplicate copy retaining the native ftsZ copy while inserting one of the expression constructs in Table 4 into the intD gene on the same chromosome. Chromosomal duplications were constructed using the RED recombinase system (Katsenko, K. A., and B. L. Wanner. One-Step Inactivation of Chromosomal Genes in Escherichia coli K-12 Using PCR Products. Proc. Natl. Acad. Sci. 97:6640-6645. 2000) and are shown in SEQ ID NO 3-5. The later constructs allow native replication during non-minicell producing conditions, thus avoiding selective pressure during strain construction and maintenance. Furthermore, these strains provide defined points of minicell induction that improve minicell purification while creating conditions that allow strain manipulation prior to, during, and following minicell production. By way of non-limiting example these manipulations may be protein production that the cytoplasmic redox state, modify plasmid copy number, and/or produce chaperone proteins.

For minicell production, a minicell producing strain described in the previous section is grown overnight in Luria broth (LB) supplemented with 0.1 % dextrose, 100 µg/ml ampicillin, and when using the single-copy ftsZ construct, 15 µM IPTG. All incubations were performed at 37°C. For minicell induction only, overnight strains are subcultured 1/1000 into the same media. If minicell induction is to be coupled with co-expression of other proteins that are controlled by a catabolite repression-sensitive regulator, dextrose was excluded. Minicell induction is sensitive to aeration and mechanical forces. Therefore, flask size, media volume and shake speed is critical for optimal yields. Likewise, bioreactor conditions must be properly regulated to optimize these production conditions.

In shake-flask cultures, strains are grown to early exponential (log) phase as monitored by optical density (OD) at 600 nm (OD₆₀₀ 0.05-0.20). (Bioreactor conditions may differ significantly depending on the application and yield desired). For minicell induction alone, early log phase cultures are induced with the appropriate inducer concentration shown in Table 5. For coupled co-expression, these cultures are induced as shown in Table 5 for the appropriate minicell regulator, while the coupled protein(s) is induced with the inducer appropriate for the regulator controlling the synthesis of that protein. Cultures are grown under the appropriate conditions and harvested during late log (OD₆₀₀ 0.8-1.2). Depending on the application, minicell induced cultures may be immediately chilled on ice prior to purification, or maintained at room temperature during the harvesting process.

To separate minicells from viable, parental cells, cultures are subjected to differential centrifugation (Voros, J., and R. N. Goodman. 1965. Filamentous forms of Erwinia amylovora. Phytopathol. 55:876-879). Briefly, cultures are centrifuged at 4,500 rpm in a GSA rotor for 5 min. Supernatants are removed to a fresh bottle and centrifuged at 8,000 rpm for an additional 10 min to pellet minicells. Pelleted minicells (containing contaminating parental cells) are resuspended in 2 ml LB, LBD (LB supplemented with 0.1 % dextrose), Min (minimal M63 salt media) (Roozen, K. J., et al. 1971. Synthesis of ribonucleic acid and protein in plasmid-containing minicells of Escherichia coli K-12. J. Bacteriol. 107:21-23), supplemented with 0.5% casamino acids) or MDT (minimal M63 salt media, supplemented with 0.5% casamino acids, 0.1% dextrose, and thiamine). Resuspended minicells are next separated using linear density gradients. By way of non-limiting example, these gradients may contain sucrose (Cohen A., et al. 1968. The properties of DNA transferred to minicells during conjugation. Cold Spring Harb. Symp. Quant. Biol. 33:635-641), ficol, or glycerol. For example, linear sucrose gradients range from 5-20% and are poured in LB, LBD , Minor MDT. Using a SW28 swinging bucket rotor, gradients are centrifuged at 4,500 rpm for 14 min. Banded minicells are removed, mixed with LB, LBD , Minor MDT, and using a JA-20 rotor are centrifuged at 13,000 rpm for 12 min. Following centrifugation, pellets are resuspended in 2 ml LB, LBD , Minor MDT and subjected to a second density gradient. Following the second density separation, banded minicells are removed from the gradient, pelleted as described, and resuspended in LB, LBD , Minor MDT for use and/or storage.

Purified minicells are quantitated using an OD₆₀₀ measurement as compared to a standard curve incorporating LPS quantity, minicell size, and minicell volume. Quantitated minicells mixtures are analyzed for contaminating, viable parental cells by plating on the appropriate growth media (Table 8).

**TABLE 8: MINICELL PURIFICATION AND PARENTAL CELL QUANTITATION**

| Purification | Total cells | Total parental cells | MC / PC ratio | Fold-purification |
|---|---|---|---|---|
| Before | 4.76 X 10¹¹ | 3.14 X 10¹¹ | 0.25/1 | - |
| After | 1.49 X 10¹¹ | 6.01 X 10⁴ | 2.48 X 10⁶/1 | 5.23 X 10⁶ |

### EXAMPLE 15: T7-DEPENDENT INDUCTION OF EXPRESSION

Expression from the pCAL-c expression vector is driven from a T7 bacteriophage promoter that is repressed by the LacI gene product. Transcription of the DNA into mRNA, and subsequent translation of mRNA into proteins, does not occur as long as the LacI repressor is bound to the T7 promoter. However, in the presence of IPTG, the LacI repressor does not bind the T7 promoter. Thus, induction of expression from pCAL-c sequences is dependent on the presence of IPTG. Slightly different protocols were used for the induction of Escherichia coli whole and for the induction of minicells. Slight differences are also present in the protocols for induction of minicells for ³⁵S-methionine labeling of proteins in contrast to those for the induction of minicells for Western blot analysis. These induction protocols are described bellow.

For expression in E. coli whole cells, the cells were first grown overnight in 3 mL of LB and antibiotics. The cultures were screened for the presence of the desired expression element as previously described. Cultures containing the desired expression elements were diluted 1:100 and grown to an OD₆₀₀ of between 0.4 to 0.6. The culture size varied depending on the intended use of the cells. IPTG was then added to a final concentration of 200 µg/mL, and the cells were shaken at 30°C for 4 hours. Following the induction, cells were harvested for analysis.

The induction of minicells was carried out as follows. The minicells were diluted in MMM buffer to 1 mL total volume according to the concentration obtained from the isolation procedure (OD₄₅₀ of about 0.5). The cells were then treated with 50 µg/mL of cycloserine for 30 minutes at 37°C to stop whole cell growth. Following the cycloserine treatment the cells were provided with an amino acid, methionine, which the MMM buffer does not contain. For ³⁵S-labeled protein induction ³⁵S-methionine was added to the minicell sample whereas, for unlabeled protein induction unlabeled methionine was added. Fifteen (15) µCi of ³⁵S-methionine (Amersham Pharmacia Biotech, Piscataway, NJ) was added to the samples for radiolabeling and 5 µmol of methionine was added to the non-labeled minicell samples. Two hundred (200) µg/mL IPTG was also added to the minicell samples, which were then shaken at 30°C for about 4 hours. Following induction, the minicells were harvested for further preparation or analysis.

### EXAMPLE 17: METHODS TO INDUCE EXPRESSION

Expression in minicells may proceed following purification of minicells and/or minicell protoplasts from parental cells and LPS constituents, repectively. However, for some applications it is suitable to co-express proteins of interest with minicell induction. For these approaches, one may use the protocol described in EXAMPLE 13 for expression of the phoA constructs. By way of non-limiting example, either of these approaches may be accomplished using one or more of the following expression constructs (Table 9).

**TABLE 9: EXPRESSION CONSTRUCTS**

| Plasmid | Regulatory element(s) | inducer | Plasmid | SEQ ID NO.: |
|---|---|---|---|---|
| pMPX-5 | rhaRS | Rhamnose | pUC-18 | 6 |
| pMPX-7 | uidR | β-glucuronate | pUC-18 | 10 |
| pMPX-8 | meIR | Melibiose | pUC-18 | 11 |
| pMPX-18 | araC | Arabinose | pUC-18 | 12 |
| pMPX-6 | araC | Arabinose | pUC-18 | 13 |

**TABLE 10: OLIGONUCLEOTIDE PRIMER SEQUENCES FOR TABLE 9 CONSTRUCTS**

| SEQ ID NO.: | Primer name | 5' to 3' sequence |
|---|---|---|
| 69 | Rha-1 | 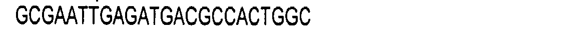 |
| 70 | Rha-2 | 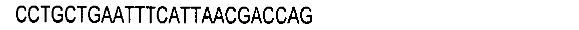 |
| 71 | Rha-1-HindIII | 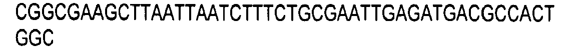 |
| 72 | Rha-2-PstI | 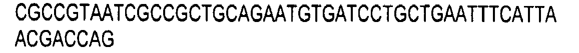 |
| 73 | Uid-1 | 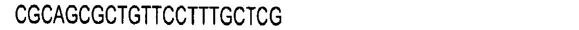 |
| 74 | Uid-2 | 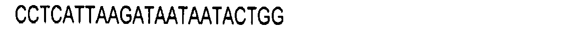 |
| 75 | Uid-1-HindIII | 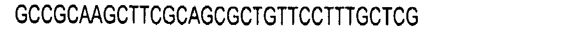 |
| 76 | Uid-2-PstI | 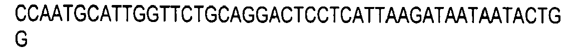 |
| 77 | MeI-1 | 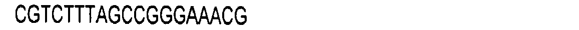 |
| 78 | MeI-2 | 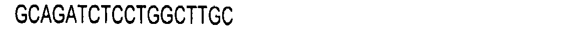 |
| 79 | MeI-1-HindIII | 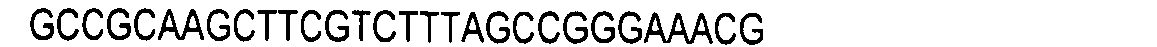 |
| 80 | MeI-2-SalI | 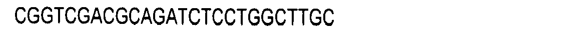 |
| 81 | Ara-1 | 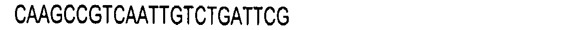 |
| 82 | Ara-2 | GGTGAATTCCTCCTGCTAGCCC |
| 83 | Ara-1-HindIII | GCGCCAAGCTTCAAGCCGTCAATTGTCTGATTCG |
| 84 | Ara-2-Pstl | CTGCAGGGTGAATTCCTCCTGCTAGCCC |
| 85 | Ara-1-XhoI | GCTTAACTCGAGCTTAATAACAAGCCGTCAATTGTCTGATTC |
| 86 | Ara-2-Sstl | GCTTAACCGCGGGCCAAGCTTGCATGCCTGCTCC |

Oligonucleotides SEQ ID NOS.:69, 70, 71 and 72 were used to amplify the rhaRS genes and their divergent control region from the E. coli chromosome. Once amplified, this region was inserted into pUC18 using Hindlll and Pstl to create SEQ ID NO.: 6.

Oligonucleotides SEQ ID NOS.:73, 74, 75 and 76 were used to amplify the uidR control region, the uidR gene and the control region for expression from the E. coli chromosome. Once amplified, this region was inserted into pUC18 using HindIII and Pstl to create SEQ ID NO.: 10.

Oligonucleotides SEQ ID NOS.:77, 78, 79 and 80 were used to amplify the meIR gene and its divergent control region from the E. coli chromosome. Once amplified, this region was inserted into pUC18 using HindIII and SalI to create SEQ ID NO.: 11.

Oligonucleotides SEQ ID NOS.:81, 82, 83 and 84 were used to amplify the araC gene and its divergent control region from the E. coli chromosome. Once amplified, this region was inserted into pUC18 using Hindlll and PstI to create SEQ ID NO.: 12.

Oligonucleotides SEQ ID NOS.:81, 82, 85 and 86 were used to amplify the araC gene and its divergent control region was PCR amplified from pBAD-24. Once amplified, this region was inserted into pEGFP (Clontech) using Xhol and Sstl to create SEQ ID NO.: 13.

Except of pMPX-6, these expression constructs contain the same multiple cloning site. Therefore, any protein of interested may be inserted in each modular expression construct for simple expression screening and optimization.

By way of non-limiting example, other proteins that may be expressed are listed in Table 11.

**TABLE 11: OTHER EXPRESSED PROTEINS**

| Protein | Origin | Construct | Purpose | SEQ ID NO.: |
|---|---|---|---|---|
| Edg3 | Rat | native | GPCR | 14 |
| β2AR | Human | native | GPCR | 15 |
| TNFR-1α (human) | Human | residues 29-455 | Receptor | 18 |
| TNFR-1b (human) | Human | residues 41-455 | Receptor | 17 |
| TNF (human) | Human | native | Gene transfer | 19 |
| T-EGF | Human | chimera | Gene transfer | 20 |
| T-Invasin | Y. pseudotuberculosis | chimera | Gene transfer | 21 |

**TABLE 12: OLIGONUCLEOTIDE PRIMER SEQUENCES FOR TABLE 11**

| SEQ ID NO.: | Primer name | 5' to 3' sequence |
|---|---|---|
| 87 | Edg-1 | 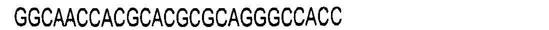 |
| 88 | Edq-2 | 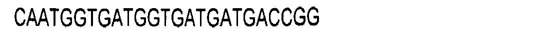 |
| 89 | Edg-1-SalI | 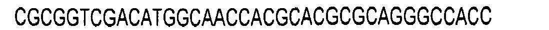 |
| 90 | Edg-2-KpnI | 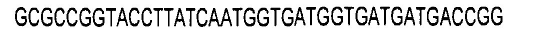 |
| 91 | β2AR-1 | 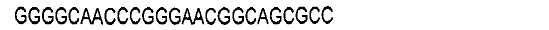 |
| 92 | β2AR-2 | 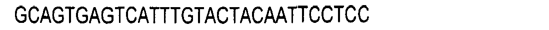 |
| 93 | β2AR-1-SalI | 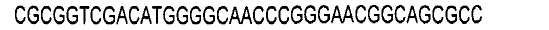 |
| 94 | β2AR-2-BamHI | 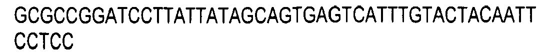 |
| 95 | TNFR(29)-1 | 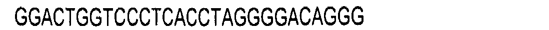 |
| 96 | TNFR(29)-2 | 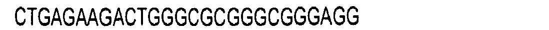 |
| 97 | TNFR(29)-1-SalI | 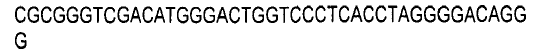 |
| 98 | TNFR(29)-2-KpnI | 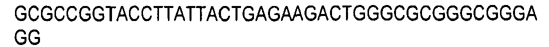 |
| 99 | TNFR(41)-1 | 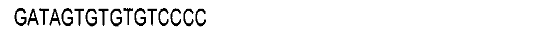 |
| 100 | TNFR(41)-2 | 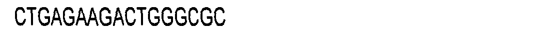 |
| 101 | TNFR(41)-1-NcoI | 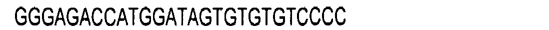 |
| 102 | TNFR(41)-2-XbaI | 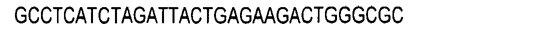 |
| 103 | TNF-1 | 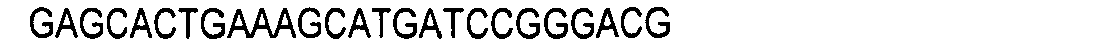 |
| 104 | TNF-2 | 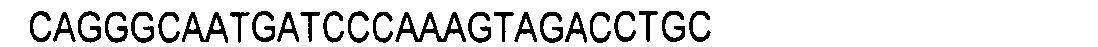 |
| 105 | TNF-1-EcoRI | 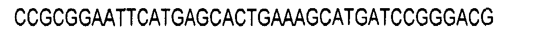 |
| 106 | TNF-2-HindIII | 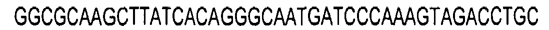 |
| 107 | T-EGF-1 | 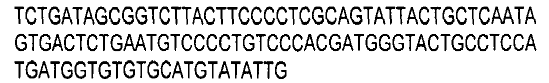 |
| 108 | T-EGF-2 | 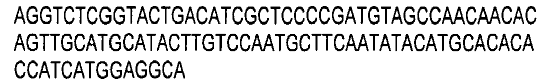 |
| 109 | T-EGF-3 | 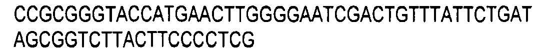 |
| 110 | T-EGF-4 | 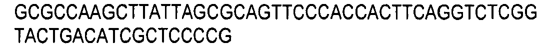 |
| 111 | Inv-1 | 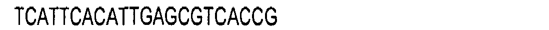 |
| 112 | Inv-2 | 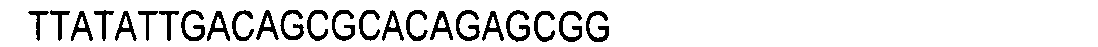 |
| 113 | Inv-1-ToxR-EcoRI | 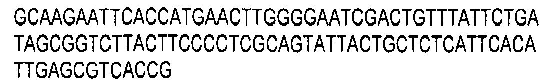 |
| 114 | Inv-2-PstI | 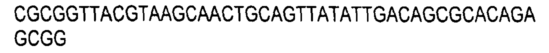 |

Oligonucleotides SEQ ID NOS.:87, 88, 89 and 90 were used to amplify rat Edg3 from rat cDNA. Once amplified, this region was inserted into SEQ ID NO.: 6 (pMPX-5) using SalI and KpnI to create SEQ ID NO.:14.

Oligonucleotides SEQ ID NOS.:91, 92, 93 and 94 were used to amplify human β2 adrenergic receptor (β2AR) from human heart cDNA. Once amplified, this region was inserted into SEQ ID NO.: 6 (pMPX-5) using Sall and BamHI to create SEQ ID NO.:15.

Oligonucleotides SEQ ID NOS.:95, 96, 97 and 98 were used to amplify human tumor necrosis factor receptor (TNFR residues 29-455) from human Jurkat CL71 cDNA. Once amplified, this region was inserted into SEQ ID NO.: 12 (pMPX-18) using Sall and Kpnl to create SEQ ID NO.:18.

Oligonucleotides SEQ ID NOS.:99, 100, 101 and 102 were used to amplify human tumor necrosis factor receptor (TNFR residues 41-455) from human Jurkat CL71 cDNA. Once amplified, this region was inserted into pBAD24 using Ncol and Xbal to create SEQ ID NO.:17.

Oligonucleotides SEQ ID NOS.:103, 104, 105 and 106 were used to amplify human tumor necrosis factor (TNF) from human Jurkat CL71 cDNA. Once amplified, this region was inserted into SEQ ID NO.: 13 (pMPX-6) using EcoRI and HindIII to create SEQ ID NO.:19.

**TABLE 13: PROGRAM TO ANNEAL GRADIENT PCR WITH PFX POLYMERASE**

| Step | Temp (°C) | Time (min) |
|---|---|---|
| 1 | 95 | 2.0 |
| 2 | 95 | 0.5 |
| 3 | 64 | 0.5 |
| 4 | 68 | 2.5 |
| 5 | Goto 2, 2X | |
| 6 | 95 | 0.5 |
| 7 | 62 | 0.5 |
| 8 | 68 | 2.5 |
| 9 | Goto 6, 4X | |
| 10 | 95 | 0.5 |
| 11 | 60 | 0.5 |
| 12 | 68 | 2.5 |
| 13 | Goto 10, 6X | |
| 14 | 95 | 0.5 |
| 15 | 58 | 0.5 |
| 16 | 68 | 2.5 |
| 17 | Goto 14, 24X | |
| 18 | 4 | hold |
| 19 | end | |

Oligonucleotides SEQ ID NOS.:107, 108, 109 and 110 were mixed and PCR amplified using anneal gradient PCR (Table 13) to form mature human epidermal growth factor (EGF) (residues 971-1023) translationally fused to the transmembrane domain of toxR from Vibrio cholerae. Once amplified, this region was inserted into SEQ ID NO.: 13 (pMPX-6) using Kpnl and HindIII to create SEQ ID NO.:20.

Using PFX polymerase (Invitrogen) oligonucleotide SEQ ID NO.:111, 112, 113 and 114 were used to amplify invasin residues 490-986 (inv) from Yersinia pseudotuberculosis chromosomal DNA and form a translational fusion between the transmembrane domain of toxR from Vibrio cholerae. Once amplified, this region was inserted into SEQ ID NO.:13 (pMPX-6) using EcoRI and PstI to create SEQ ID NO.:21.

These proteins were proof-of-principle constructs used to evaluate the minicell platform. For purposes of this initial evaluation, all proteins except TNF, T-EGF and T-Invasin were cloned into pMPX-5, with these later proteins cloned into pMPX-6 for gene transfer experiments.

Whether the approach for protein expression is co-expression with minicell induction or expression following minicell and/or protoplast isolation, the procedure to transform the expression constructs is the same. To accomplish this, protein constructs were initially cloned into E. coli MG1655 and then into the minicell producing strain of interest. Transformation events were selected prior to minicell induction. For co-induction of protein and minicells, see the protocol for phoA expression above. For post-minicell and/or protoplast purification induction experiments, following minicell purification and/or protoplast preparation and purification, these cellular bodies were induced for protein production in either LBD or MDT at a minicell or protoplast / volume ratio of 1 X 10⁹ minicells or protoplasts / 1ml media. Media was supplemented with the appropriate inducer concentration (see Table 4). Protein induction is sensitive to a variety of factors including, but not limited to aeration and temperature, thus reaction volume to surface area ratio is important, as is the method of shaking and temperature of induction. Therefore, each protein must be treated as required to optimize expression. In addition to expression parameters, protoplasted minicells are sensitive to osmotic and mechanical forces. Therefore, protoplast protein induction reactions must also contain 10% sucrose with greater volume to surface area ratios than required for intact minicells to achieve similar aeration at lower revolutions.

Using the T-PhoA as a non-limiting example, protein expression was performed during and following minicell isolation. To accomplish this task, t-phoA co-expressed with minicell induction was compared to t-phoA expressed after minicell isolation. In both cases, overnight minicell-producing parental strains containing pMPX-5::t-phoA were subcultured into LBD supplemented with the appropriate antibiotic. Cultures were grown to OD₆₀₀ 0.1 and induced for minicell production alone or for both minicell and protein production. Both cultures were harvested at OD₆₀₀ 1.0 and minicells produced were harvested as described above. Minicells to be induced for T-phoA production following purification were induced by introducing 1 X 10⁹ purified minicells into a 15 ml culture tube containing 1 ml MDT with 1 mM L-rhamnose. Minicell protein induction was allowed to proceed for up to 14 hours and compared to protein production obtained using the co-expression approach. For each approach, minicells were fractionated and analyzed for membrane association, total protein, and membrane association-dependent enzymatic activity. These observations were compared to post-induction, pre-isolation parental cell/minicell (PC/MC) mixtures from the co-expressed reactions. The first observation was that co-expression of minicell and protein induction was superior to post-minicell purification induction (Table 14). However, although the kinetics are slower for the post-minicell purification induction protocol, the end result is equivalent.

**TABLE 14. COMPARATIVE EXPRESSION: CO-EXPRESSION VERSUS POST MINICELL PURIFICATION INDUCTION**

| Time of induction | Purified minicell induction ^{a} | Co-expression induction ^{a} |
|---|---|---|
| 1.0 | 8.0 | - |
| 2.0 | - | 812.2 |
| 4.0 | 70.0 | - |
| 14.0 | 445.0 | - |

| | | |
|---|---|---|
| a. Nanogram expressed T-PhoA per 1 X 10⁹ minicells. | | |

Using the co-expression induction procedure, the amount of membrane-associated T-PhoA was measured and compared for both parental cells and minicells. Briefly, following co-expression induction of T-PhoA and minicells, minicells were purified and their membranes isolated. For membrane isolation, minicells containing expressed T-PhoA were subjected to three rounds of freeze-thaw lysis in the presence of 10 µg/ml lysozyme. Following freeze-thaw cycling, the reaction was subjected to sonication. Sonicated material was centrifuged at 6,000 rpm in a microcentrifuge for 5 min at room temperature. Supernatants were transferred to a fresh 1.5 ml Eppendorf tube and centrifuged at 70,000 rpm using a TLA-100 rotor. Following centrifugation, the pellet was resuspended in buffer and analyzed for total T-PhoA protein (Table 15) and T-PhoA enzyme activity (Table 16).

**TABLE 15: MEMBRANE ASSOCIATED T-PHOA: PARENTAL CELLS VERSUS MINICELLS**

| Cell type ^{a} | Protein total ^{a} | T-PhoA total ^{b} | T-PhoA % total | | Protein membrane associated ^{a} | T-PhoA membrane associated ^{b} | T-PhoA % membrane protein total |
|---|---|---|---|---|---|---|---|
| Parental cells | 107.5 | 5.3 | 4.9 | | 10.7 | 3.1 | 29.0 |
| Minicells | 4.6 | 0.8 | 17.5 | | 1.0 | 0.5 | 50.0 |
| Minicells EQ^{b} | 25.2 | 4.4 | - | | 5.5 | 2.7 | - |

| | | | | | | | |
|---|---|---|---|---|---|---|---|
| a. Total protein as determined by BCA assay (Pierce) b. Microgram expressed T-PhoA per 1 X 10⁹ minicells as determined via Western using an anti-PhoA antibody (Sigma) versus a PhoA standard curve (BCA determined). c. Equivalent membrane lipid to parental cell | | | | | | | |

**TABLE 16: PHOA ENZYMATIC ACTIVITY^{a} (RELATIVE UNITS): PARENTAL CELLS VERSUS MINICELLS.**

| Cell type ^{b} | Unlysed | Lysed, total | Lysed, membrane |
|---|---|---|---|
| Parent cell | - | 358 | 240 |
| Minicell | 275 | 265 | 211 |
| Minicell EQ ^{c} | 1,504 | 1,447 | 1,154 |

| | | | |
|---|---|---|---|
| a. Activity determined colorimetrically using PNPP measuring optical density at 405 nm b. Based on 1X 10⁹ parental cells or minicells per reaction c. Equivalent membrane lipid to parental cell | | | |

These results suggest that co-expression induction of T-PhoA and minicells together results in minicells containing an equivalent amount of T-PhoA produced in both parental cells and minicells. However, the percent of T-PhoA compared to total protein is 3.5X greater in minicells than in parental cells. Furthermore, of the protein made, T-PhoA constitutes 50% of the total membrane protein in minicells, whereas it is only 29% in parental cells. It should be noted that the T-PhoA protein associated with the membrane can be easily removed by treatment with mild, non-ionic detergent suggesting that the T-PhoA present in the membrane pellet is indeed associated with the membrane and not an insoluble, co-sedimenting precipitate (data not shown). Finally, PhoA is a periplasmic enzyme that requires export to the periplasmic space for proper folding and disulfide bond formation. Both of which are required for enzymatic activity. In the time course of this experiment, expression of ΔPhoA lacking a leader sequence does not demonstrate enzymatic activity. Furthermore, there is no difference between unlysed and lysed minicells containing expressed T-PhoA (Table 16) also demonstrating that the PhoA enzyme domain of the T-PhoA chimera must be present in the periplasmic space. Therefore, the T-PhoA construct must membrane associate and the PhoA domain must orient into the periplasmic space for enzymatic activity. Thus, when comparing equivalent amounts of membrane lipid between parental cells and minicells in Table 16, membrane association-dependent T-PhoA activity is almost 5X greater than in parental cells. Taking into account the data in Table 15 where 50% of T-PhoA is in the membrane compared to 29% in parental cells, the difference in T-PhoA membrane association is not sufficient to explain the almost 5X increase in minicell activity. These observations suggest that minicells contain a capacity to support more expressed membrane protein than parental cells and that the protein that associates with the membrane is more active. This activity may be simply result from minicells allowing greater efficiency of folding and disulfide bond formation for this particular protein. However, do to the fact that minicells do not contain chromosome, it is also possible that the overexpression of this protein is readily finding membrane-binding sites in the absence of chromosomally produced competitors present in parental cells. Furthermore, overexpression of proteins often leads to increased protease expression. Because minicells do not contain chromosome, these otherwise degraded surplus T-PhoA is allowed the continued opportunity to insert and properly fold in the membrane, an attribute that could lend favor to overexpression of more complex membrane proteins.

### EXAMPLE 18: EXEMPLARY METHODS TO INDUCE AND STUDY COMPLEX MEMBRANE PROTEINS

Expression of non-native (exogenous) complex membrane proteins in bacterial systems can be difficult. Using the minicell system, we are able to eliminate toxicity issues. However, issues still remain with proper translation, compartmentalization at the membrane, insertion in the membrane and proper folding for native activity. To account for these potential problems we have constructed a modular chimeric system that incorporates leader sequences and chaperone-recognized soluble domains that are native to our bacterial minicell system. In addition, we created modular constructs that overexpress the native chaperones groESL and trigger factor (tig). Finally, we have constructed minicell-producing strains that contain mutations that effect protein export and disulfide bond formation. For non-limiting examples of these constructs see Table 17.

**TABLE 17: NON-LIMITING TOOLS FOR EXOGENOUS COMPLEX PROTEIN SYNTHESIS AND FUNCTION**

| Tool | Ref. | Residues of sequence | Purpose | SEQ ID NO |
|---|---|---|---|---|
| pMPX-5::phoA leader | - | 1-48 | Membrane targeting | 22 |
| pMPX-5::phoA leader | - | 1-494 | Membrane targeting | 23 |
| pMPX-5::malE leader | 1 | 1-28 | Membrane targeting | 24 |
| pMPX-5::malE leader | 1 | 1-370 | Membrane targeting | 25 |
| pMPX-17 (groESL, tig) | - | - | Chaperone | 26 |
| pMPX-5::trxA::FLAG | 2 | 2-109 ^{a} | Solubility | 27 |

| | | | | |
|---|---|---|---|---|
| a. Residues do not include FLAG sequence. | | | | |

References to Table 17.
1. Grisshammer, R., et al. 1993. Expression of a rat neurotensin receptor in Escherichia coli. Biochem. J. 295:571-576.
2. Tucker, J., and R. Grisshammer. 1996. Purification of a rat neurotensin receptor expressed in Escherichia coli. Biochem. J. 317:891-899.

**TABLE 18: OLIGONUCLEOTIDE PRIMER SEQUENCES FOR TABLE 17 CONSTRUCTS**

| SEQ ID NO.: | Primer name | 5' to 3' sequence |
|---|---|---|
| 115 | PhoA lead-1 | GTCACGGCCGAGACTTATAGTCGC |
| 116 | PhoA lead-2 | GGTGTCCGGGCTTTTGTCACAGG |
| 117 | PhoA lead-1-PstI | 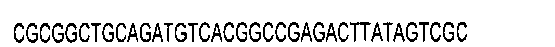 |
| 118 | PhoA lead-2-XbaI | 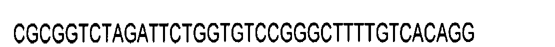 |
| 119 | PhoA complete | 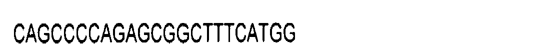 |
| 120 | PhoA complete-2-XbaI | 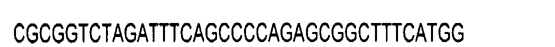 |
| 121 | MalE lead-1 | 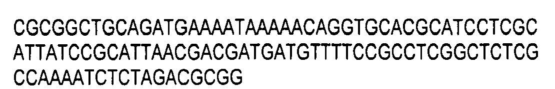 |
| 122 | MalE lead-2 | 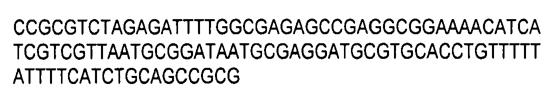 |
| 123 | MaIE-1 | 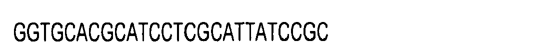 |
| 124 | MaIE-2 | 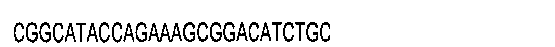 |
| 125 | MaIE-1-Pstl | 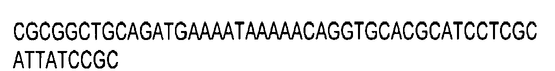 |
| 126 | MaIE-2-XbaI | 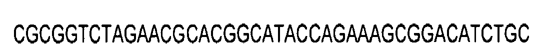 |
| 127 | Tiq-1 | 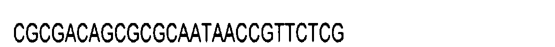 |
| 128 | Tiq-2 | 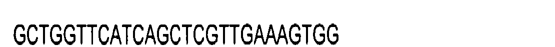 |
| 129 | Tig-1-NarI | 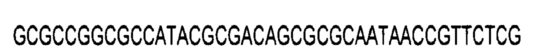 |
| 130 | Tig-2-Xbal | 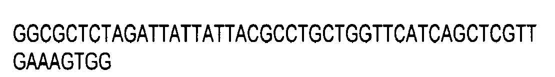 |
| 131 | Gro-1 | 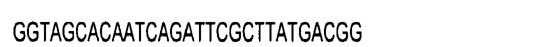 |
| 132 | Gro-2 | 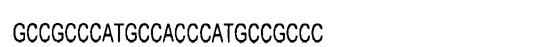 |
| 133 | Gro-1-XbaI | 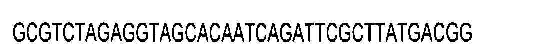 |
| 134 | Gro-2-HindIII | 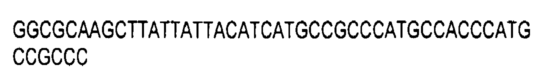 |
| 135 | TrxA-1 | 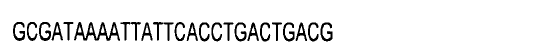 |
| 136 | TrxA-2 | 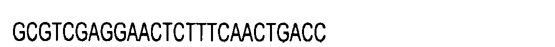 |
| 137 | TrxA-1-Fxa-Pstl | 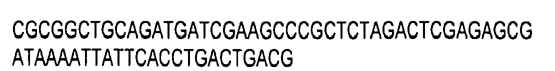 |
| 138 | TrxA-2-FLAG-BamHI | 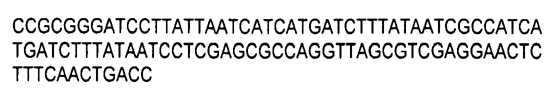 |

Oligonucleotides SEQ ID NOS.:115, 116, 117 and 118 were used to amplify the phoA leader (residues 1-49) from E. coli chromosomal DNA. Once amplified, this region was inserted into SEQ ID NO.: 6 (pMPX-5) using Pstl and Xbal to create SEQ ID NO.:22.

Oligonucleotides SEQ ID NOS.: 115, 117, 119 and 120 were used to amplify the complete phoA gene from E. coli chromosomal DNA. Once amplified, this region was inserted into SEQ ID NO.: 6 (pMPX-5) using Pstl and Xbal to create SEQ ID NO.23.

Oligonucleotides SEQ ID NOS.:121 and 122 were used to construct the malE leader (residues 1-28) sequence. Once annealed, this construct was inserted into SEQ ID NO.: 6 (pMPX-5) using Pstl and Xbal to create SEQ ID NO.:24.

Oligonucleotides SEQ ID NOS.:123, 124, 125 and 126 were used to amplify the malE expanded leader (residues 1-370) from E. coli chromosomal DNA. Once amplified, this region was inserted into SEQ ID NO.: 6 (pMPX-5) using Pstl and Xbal to create SEQ ID NO.:25.

Oligonucleotides SEQ ID NOS.:127, 128, 129 and 130 were used to amplify the tig control and gene region from E. coli chromosomal DNA. Once amplified, this region was ligated to the groESL amplified region below using Xbal prior to insertion into SEQ ID NO.: 6 (pMPX-5) using Narl (from the tig region) and HindIII (from the groESL region) to create SEQ ID NO.:26.

Oligonucleotides SEQ ID NOS.:131, 132, 133 and 134 were used to amplify the groESL control and gene region from E. coli chromosomal DNA. Once amplified, this region was ligated to the tig amplified region above using Xbal prior to insertion into SEQ ID NO.: 6 (pMPX-5) using Narl (from the tig region) and HindIII (from the groESL region) to create SEQ ID NO.:26.

Oligonucleotides SEQ ID NOS.:135, 136, 137 and 138 were used to amplify trxA (residues 2-109) from E. coli chromosomal DNA and insert FLAG and Factor Xa sequences. Once amplified, this region was inserted into SEQ ID NO.: 6 (pMPX-5) using Pstl and BamHI to create SEQ ID NO.:27.

By way of non-limiting example, the pMPX-5::phoA leader (residues 1-48), pMPX-5::phoA leader (residues 1-494), pMPX-5::maIE leader (residues 1-28), and pMPX-5::maIE leader (residues 1-370) constructs are designed to direct expressed exogenous membrane proteins to the minicell cytoplasmic membrane. In addition to these constructs, By way of non-limiting example, mutations in E. coli genes secA and secY, specifically mutation prIA4 (Strader, J., et al. 1986. Kinetic analysis of lamb mutants suggests the signal sequence plays multiple roles in protein export. J. Biol. Chem. 261:15075-15080), permit promiscuous targeting to the membrane. These mutations, like the above constructs are integrated into the minicell expression system. To complement these mutations, the chaperone complex groESL and trigger factor have also been incorporated into the expression system. By way of non-limiting example, pMPX-5::trxA::FLAG will be used to create a carboxy-terminal fusion to the protein of interest to increase the membrane insertion efficiency of the membrane protein of interest (Tucker, J., and R. Grisshammer. 1996. Purification of a rat neurotensin receptor expressed in Escherichia coli. Biochem. J. 317:891-899). Also By way of non-limiting example, pMPX-5::FLAG::toxR and pMPX-5::FLAG::λcl constructs will be prepared to create a carboxy-terminal fusion to the protein of interest for use in a reporter-based assay for protein-protein interactions. By way of non-limiting example, the protein of interest for this system is a GPCR. Also By way of non-limiting example, this GPCR may be the neurotensin receptor from rat (Grisshammer, R., et al. 1993. Expression of a rat neurotensin receptor in Escherichia coli. Biochem. J. 295:571-576.), or the β2 adrenergic receptor from humans (Freissmuth, M., et al. 1991. Expression of two β-adrenergic receptors in Escherichia coli: functional interaction with two forms of the stimulatory G protein. Proc. Natl. Acad. Sci. 88:8548-8552). Insertion of a GPCR into one of these reporter constructs creates a carboxy-terminal fusion between the GPCR of interest and the DNA-binding regulatory domain of the ToxR positive activator, the λcl repressor, or the AraC positive activator. To complete this reporter system, By way of non-limiting example pMPX-5::(X)::toxR or pMPX-5::(X)::λcl will be used to create a carboxy-terminal fusion to the protein of interest for use in a reporter-based assay for protein-protein interactions, where (X) may be any protein or molecule involved in an intermolecular or intramolecular interaction. By way of non-limiting example, this molecule of interest may be a G-protein. This G-protein may be the Gαᵢ₁-protein from rat (Grisshammer, R., and E. Hermans. 2001. Functional coupling with Gαq and Gαi1 protein subunits promotes high-affinity agonist binding to the neurotensin receptor NTS-1 expressed in Escherichia coli. FEBS Lett. 493:101-105), or the G_{sα}-protein from human (Freissmuth, M., et al. 1991. Expression of two β-adrenergic receptors in Escherichia coli: functional interaction with two forms of the stimulatory G protein. Proc. Natl. Acad. Sci. 88:8548-8552). Like the GPCR, insertion of a G-protein into one of these reporter constructs creates a carboxy-terminal fusion between the G-protein of interest and the DNA-binding regulatory domain of the ToxR positive activator, the λcl repressor, or other regulatory protein. Finally, these plasmid constructs contain the DNA-binding domain of each regulator; the ctx regulatory region from Vibrio cholerae (Russ, W. P., and D. M. Engelman. 1999. TOXCAT: a measure of transmembrane helix association in a biological membrane. 96:863-868), or the P_{R}1O_{R}1 region of bacteriophage lambda (Hu, J. C., et al. 1990. Sequence requirements for coiled-coils: analysis with lambda repressor-GCN4 leucine zipper fusions. Science. 250:1400-1403), respectively. By way of non-limiting example, each binding domain is coupled to a reporter sequence encoding luciferase (Dunlap, P. V., and E. P. Greenberg. 1988. Control of Vibrio fischeri lux gene transcription by a cyclic AMP receptor protein-luxR protein regulatory circuit. J. Bacteriol. 170:4040-4046), green fluorescent protein (GFP) (Yang, T. T., et al. 1996. Dual color microscopic imagery of cells expressing the green fluorescent protein and a red-shifted variant. Gene. 173:19-23; Matthysse, A. G., et al. 1996. Construction of GFP vectors for use in gram-negative bacteria other than Escherichia coli. FEMS Microbiol. Lett. 145:87-94), or other reporter. Co-expression of these GPCR and G-protein chimeras will create a system measuring the interaction between a GPCR and G-protein within an intact minicell. This system is designed to be used as a positive or negative read-out assay and may be used to detect loss or gain of GPCR function. Although the GPCR-G-protein interaction is provided as an example, this modular system may be employed with any soluble or membrane protein system measuring protein-protein or other intermolecular interaction.

### EXAMPLE 19: EXEMPLARY METHODS FOR GENE TRANSFER USING MINICELLS OR MINICELL PROTOPLASTS

Included in the design of the invention is the use of minicells to transfer genetic information to a recipient cell. By way of non-limiting example, this gene transfer may occur between a minicell and a mammalian cell in vitro, or in vivo, and this gene transfer may occur through cell-specific interactions, through general interactions, or a combination of each. To accomplish this task three basic constructs were created. Each of these constructs is created in pMPX-6 which contains a CMV promotor controlling the synthesis of GFP. The plasmid pMPX-6 was constructed by cloning the araC through the multiple cloning site of pBAD24 into pEGFP (Clontech). This construct provided a bacterial regulator as well as a method to monitor the success of gene transfer using GFP expression form the CMV promotor. In design, the protein expressed using the bacterial promotor will drive the cell-cell interaction, while the successful transfer of DNA from the minicell to the recipient cell will initiate the production of GFP. By way of non-limiting example, proteins that will drive the cell-cell interaction may be the invasin protein from Yersinia pseudotuberculosis, which stimulates β1 integrin-dependent endocytic events. To properly display the invasin protein on the surface of minicells, the domain of invasin that stimulates these events (residues 490-986) (Dersch, P., and R. R. Isberg. 1999. A region of the Yersinia pseudotuberculosis invasin protein enhances integrin-mediated uptake into mammalian cells and promotes self-association. EMBO J. 18:1199-1213) was fused to the transmembrane domain of ToxR. Expression of this construct from pMPX-6 will display T-Inv on the surface of the minicell and stimulate endocytosis with any cell displaying a β1 integrin. Thus, T-Inv display will provide a general mechanism of gene transfer from minicells. To provide specificity, By way of non-limiting example, the ligand portion of epidermal growth factor (EGF) may be fused to the transmembrane domain of ToxR, thus creating a protein that will interact with cells displaying the EGF receptor (EGFR). Likewise, tumor nucrosis factor (TNF) may also serve this purpose by stimulating cell-cell interactions between minicells displaying TNF and cells displaying TNF receptor (TNFR). Although EGF-EGFR and TNF-TNFR interactions may stimulate cell-cell fusion between minicells and recipient cells, or minicell uptake, this alone may not be sufficient to efficiently transfer genetic information from minicells. Therefore, a genetic approach to increasing the cell-cell genetic transfer may be the development of a genetic switch that senses the specificity interaction, e.g. EGF-EGFR interaction, and turns on the production of a second gene product, e.g. invasin, that stimulates the endocytic event. By way of non-limiting example, this genetic switch may be similar to the GPCR-G-protein interaction reporter system above, in that an extracellular event stimulates the dimerization of a transcriptional active regulator, thus turning on the production of invasin or invasin-like protein. In either approach, the display system to stimulate transfer of genetic information from minicells to recipient cells may also be applicable to the transfer of substances other than genetic information, e.g. pre-synthesized therapeutic drugs.

To test this targeting methodology, different pMPX-6 constructs containing each of these general or specific cell-cell interaction proteins will be transformed into a minicell producing strain and either by co-expression induction of minicells, by post-minicell purification induction, or by post-protoplasting induction, minicells displaying the targeting protein of interest will be produced. When using the co-expression induction and post-minicell purification induction of the targeting protein approaches, it is necessary to protoplast the purified minicells after protein induction. Once the targeting protein has been displayed on the surface of a minicell protoplast, these protoplasts are ready to be exposed to target cells. For preliminary experiments these interactions will be monitored using cell culture of Cos cells in comparison to lipofectamine (Invitrogen), electroporation, and other transfection techniques. Initial experiments will expose protoplasts displaying T-Inv to Cos cells and compare the transfection efficiency to protoplast containing pMPX-6::t-inv in the absence of t-inv expression, naked pMPX-6::t-inv alone, and naked pMPX-6::t-inv with lipofectamine. Each of these events will be monitored using fluorescent microscopy and/or flow cytometry. From these results the specific targeting apparatus proteins will be tested. Using A-431 (display EGFR) and K-562 (no EGFR) cell lines, the pMPX-6::t-egf constructs will be tested. Using the same approaches as for the t-inv study, the level of transfection between A-431 and K-562 cell lines will be measured and compared to those achieved using lipofectamine. Similarly, the ability of TNF to stimulate gene transfer will be studied using L-929 cells. In all cases, the ability of these general and specific targeting protein constructs will be compared to standard transfection techniques. Upon positive results, these methodologies will be tested on difficult to transfect cell lines, e.g. adult cardiomyocytes. The basis of these results will create a foundation for which applications into in vivo gene transfer may occur.

### EXAMPLE 20: ADDITIONAL AND OPTIMIZED METHODS FOR GENETIC EXPRESSION

Expression in minicells may occur following purification of minicells and/or minicell protoplasts from parental cells and LPS constituents, respectively. However, for some applications it is preferred to co-express proteins of interest with minicell induction. For these approaches, one may use the protocol described in Example 13 for expression of the phoA constructs. Either of these approaches may be accomplished using one or more of the following expression constructs (Table 19) and/or optimized expression constructs (Table 21).

Expression plasmid pCGV1 contains a temperature sensitive lambda cl repressor (cl857) and both lambda PR and PL promoters (Guzman, C. A., et al. 1994. A novel Escherichia coli expression-export vector containing alkaline phosphatase as an insertional inactivation screening system. Gene. 148:171-172) with an atpE initiation region (Schauder, B., et al. 1987. Inducible expression vectors incorporating the Escherichia coli atpE translational initiation region. Gene. 52:279-283). Included in the design of the invention is the modification of this expression vector to best align the required Shine-Delgarno ribosomal binding site with cloning sites. In addition, the pCGVI expression vector was modified to incorporate a stem-loop structure at the 3-prime end of the transcript in order to provide a strong transcriptional stop sequence (Table 23).

Expression plasmid pCL478 contains a temperature sensitive lambda cl repressor (cl857) and both lambda PR and PL promoters (Love, C. A., et al. 1996. Stable high-copy bacteriophage promoter vectors for overproduction of proteins in Escherichia coli. Gene. 176:49-53). Included in the design of the invention is the modification of this expression vector to best align the required Shine-Delgarno ribosomal binding site with cloning sites. In addition, the pCL478 expression vector was modified to incorporate a stem-loop structure at the 3-prime end of the transcript in order to provide a strong transcriptional stop sequence (Table 19).

**TABLE 19. LAMBDA CI857 EXPRESSION VECTOR MODIFICATIONS**

| New Plasmid | Parent plasmid | Region removed | Region added ^{a} | SEQ ID NO |
|---|---|---|---|---|
| pMPX-84 | pCGV1 | NdeI - BamHI | NdeI, SD - Pstl, Xbal, Kpnl, Stem-loop, BamHl | 139 |
| pMPX-85 | pCGV1 | NdeI - BamHI | NdeI, SD - Sall, Xbal, Kpnl, Stem-loop, BamHl | 140 |
| pMPX-86 | pCL478 | BamHI - XhoI | BamHI, SD - Pstl, Xbal, Kpnl, Stem-loop, Xhol | 141 |
| pMPX-87 | pCL478 | BamHI - XhoI | BamHI, SD - Sall, Xbal, Kpnl, Stem-loop, Xhol | 142 |

| | | | | |
|---|---|---|---|---|
| a. "SD" refers to a Shine-Delgarno ribosome-binding sequence; "Stem-loop" refers to a stem-loop structure that functions as a transcriptional stop site. | | | | |

**TABLE 20. OLIGONUCLEOTIDE PRIMER SEQUENCES FOR TABLE 19**

| SEQ ID NO | Primer name | 5' to 3' sequence |
|---|---|---|
| 143 | CGV1-1-SalI | TATGTAAGGAGGTTGTCGACCGGCTCAGTCTAGAGGTACCCGCCCTCA TCCGAAAGGGCGTATTG |
| 144 | CGV1-2-SalI | GATCCAATACGCCCTTTCGGATGAGGGCGGGTACCTCTAGACTGAGCC GGTCGACAACCTCCTTACA |
| 145 | CGV1-1-PstI | TATGTAAGGAGGTTCTGCAGCGGCTCAGTCTAGAGGTACCCGCCCTCA TCCGAAAGGGCGTATTG |
| 146 | CGV1-2-PstI | GATCCAATACGCCCTTTCGGATGAGGGCGGGTACCTCTAGACTGAGCC GCTGCAGAACCTCCTTACA |
| 147 | CL478-1-Sall | GATCCTAAGGAGGTTGTCGACCGGCTCAGTCTAGAGGTACCCGCCCTC ATCCGAAAGGGCGTATTC |
| 148 | CL478-2-SalI | TCGAGAATACGCCCTTTCGGATGAGGGCGGGTACCTCTAGACTGAGCC GGTCGACAACCTCCTTAG |
| 149 | CL478-1-PstI | GATCCTAAGGAGGTTCTGCAGCGGCTCAGTCTAGAGGTACCCGCCCTC ATCCGAAAGGGCGTATTC |
| 150 | CL478-2-Pstl | TCGAGAATACGCCCTTTCGGATGAGGGCGGGTACCTCTAGACTGAGCC GCTGCAGAACCTCCTTAG |

Oligonucleoides SEQ ID NOS.: 143 and 144 were annealed to each other to generate a DNA molecule with a 5' overhang at both ends. The overhangs are designed so that the DNA can be directly cloned into pCGVI cut with NdeI (5' overhang is TA) and BamHI (5' overhang is GATC). Insertion of the annealed DNA into pCGVI creates SEQ ID NO.: 139, pMPX-84.

Oligonucleoides SEQ ID NOS.: 145 and 146 were annealed to each other to generate a DNA molecule with a 5' overhang at both ends. The overhangs are designed so that the DNA can be directly cloned into pCGVI cut with NdeI (5' overhang is TA) and BamHI (5' overhang is GATC). Insertion of the annealed DNA into pCGVI creates SEQ ID NO.: 140, pMPX-85.

Oligonucleoides SEQ ID NOS.: 147 and 148 were annealed to each other to generate a DNA molecule with a 5' overhang at both ends. The overhangs are designed so that the DNA can be directly cloned into pCL478 cut with BamHI (5' overlap is GATC) and Xhol (overhang is TCGA). Insertion of the annealed DNA into pCL578 cut with BamHI and Xhol creates SEQ ID NO.: 141, pMPX-86.

Oligonucleoides SEQ ID NOS.: 149 and 150 were annealed to were annealed to each other to generate a DNA molecule with a 5' overhang at both ends. The overhangs are designed so that the DNA can be directly cloned into pCL578 cut with BamHI (5' overlap is GATC) and Xhol (overhang is TCGA). Insertion of the annealed DNA into pCL478 cut with BamHI and Xhol creates SEQ ID NO.: 142, pMPX-87.

The optimized expression constructs in Table 21 were created from SEQ ID NOS.: 6, 11, and 12 (see Table 9). Modifications were made to optimize the alignment of the SalI or PstI cloning sites with the Shine-Delgarno ribosome-binding site. In addition, stem-loop transcriptional termination sequences were added on the 3' end of the cloning region.

**TABLE 21: EXPRESSION CONSTRUCTS**

| Plasmid | Regulatory element(s) | inducer | Plasmid | SEQ ID NO.: |
|---|---|---|---|---|
| pMPX-67 | RhaRS | Rhamnose | PUC-18 | 151 |
| pMPX-72 | RhaRS | Rhamnose | PUC-18 | 152 |
| pMPX-66 | AraC | Arabinose | PUC-18 | 153 |
| pMPX-71 | AraC | Arabinose | PUC-18 | 154 |
| pMPX-68 | MeIR | Melibiose | PUC-18 | 155 |

**TABLE 22. OLIGONUCLEOTIDE PRIMER SEQUENCES FOR TABLE 21 CONSTRUCTS**

| SEQ ID NO.: | Primer name | 5' to 3' sequence |
|---|---|---|
| 69 | Rha-1 | GCGAATTGAGATGACGCCACTGGC |
| 156 | Rha-SD | GCAGAACCTCCTGAATTTCATTACGACC |
| 71 | Rha-1-HindIII | CGGCGAAGCTTAATTAATCTTTCTGCGAATTGAGATGACGCCACT GGC |
| 157 | Rha-SD SalI Kpnl | 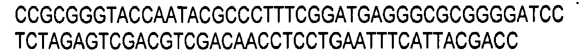 |
| 158 | Rha-SD Kpnl Kpnl | 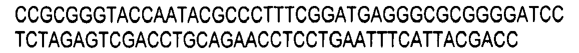 |
| 81 | Ara-1 | 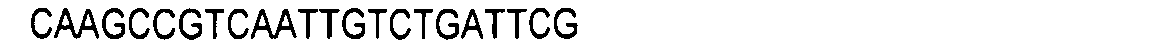 |
| 159 | Ara-SD | 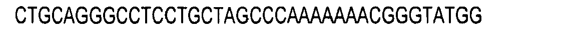 |
| 83 | Ara-1-HindIII | 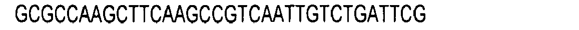 |
| 160 | Ara-SD SalI Kpnl | 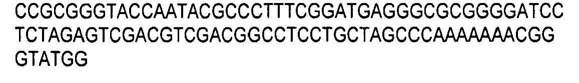 |
| 161 | Ara-SD Pstl KpnI | 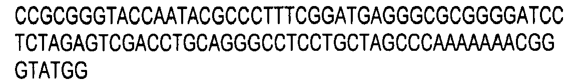 |
| 77 | Mel-1 | 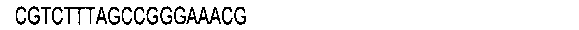 |
| 162 | Mel-SD | 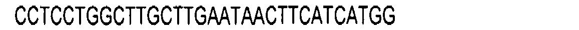 |
| 79 | MeI-1-HindIII | 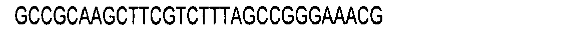 |
| 163 | Mel-SD-Sall Kpnl | 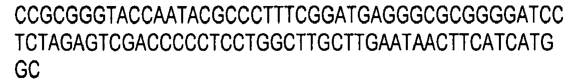 |

Oligonucleotides SEQ ID NOS.: 69, 156, 72, and 157 were used to amplify the rhaRS genes and their divergent control region from the E. coli chromosome and insertion of an optimized Sall-Shine-Delgarno ribosome-binding alignment and a stem-loop transcriptional termination sequence. Once amplified, this region was inserted into pUC18 using HindIII and Kpnl to create pMPX67, SEQ ID NO.: 151.

Oligonucleotides SEQ ID NOS.: 69, 156, 72, and 158 were used to amplify the rhaRS genes and their divergent control region from the E. coli chromosome and insertion of an optimized PstI-Shine-Delgarno ribosome-binding alignment and a stem-loop transcriptional termination sequence. Once amplified, this region was inserted into pUC18 using HindIII and Kpnl to create, pMPX-72, SEQ ID NO.: 152.

Oligonucleotides SEQ ID NOS.: 81, 159, 81, 160 were used to amplify the araC genes and their divergent control region from the E. coli chromosome and insertion of an optimized SalI-Shine-Delgarno ribosome-binding alignment and a stem-loop transcriptional termination sequence. Once amplified, this region was inserted into pUC18 using HindIII and Kpnl to create, pMPX-66, SEQ ID NO.: 153.

Oligonucleotides SEQ ID NOS.: 81, 159, 81, 161 were used to amplify the araC genes and their divergent control region from the E. coli chromosome and insertion of an optimized PstI-Shine-Delgarno ribosome-binding alignment and a stem-loop transcriptional termination sequence. Once amplified, this region was inserted into pUC18 using HindIII and Kpnl to createm pMPX-71, SEQ ID NO.: 154.

Oligonucleotides SEQ ID NOS.: 77, 162, 79, 163 were used to amplify the meIR genes and their divergent control region from the E. coli chromosome and insertion of an optimized SalI-Shine-Delgarno ribosome-binding alignment and a stem-loop transcriptional termination sequence. Once amplified, this region was inserted into pUC18 using HindIII and Kpnl to create, pMPX-68, SEQ ID NO.: 155.

### EXAMPLE 21: OPTIMIZATION OF RAT NEUROTENSIN RECEPTOR (NTR) EXPRESSION

Expression of specific GPCR proteins in minicells may require chimeric domain fusions_to stabilize the expressed protein and/or direct the synthesized protein to the membrane. The NTR protein from rat was cloned into several chimeric combinations to assist in NTR expression and membrane association (Grisshammer, R., et al. 1993. Expression of a rat neurotensin receptor in Escherichia coli. Biochem. J. 295:571-576; Tucker, J., and Grisshammer, R. 1996. Purification of a rat neurotensin receptor expressed in Escherichia coli. Biochem. J. 317:891-899). Methods for construction are shown the Tables below.

**TABLE 23. NEUROTENSIN RECEPTOR EXPRESSION FACILITATING CONSTRUCTS**

| Protein ^{a} | Construct ^{b} | SEQ ID NO |
|---|---|---|
| MaIE (L) | SalI-MaIE (1-370)-Factor Xa-NTR homology | 164 |
| NTR | Factor Xa-NTR (43-424)-NotI-FLAG-KpnI | 165 |
| MaIE(L)-NTR | SalI-MaIE(1-370)-Factor Xa-NTR(43-424)-NotI-FLAG-KpnI | 166 |
| MaIE(S)-NTR | SalI-MaIE(1-28)-Factor Xa-NTR(43-424)-NotI-FLAG-KpnI | 167 |
| TrxA | NotI-TrxA(2-109)-NotI | 168 |
| MaIE(L)-NTR-TrxA | Sall-MaIE(1-370)-Factor Xa-NTR(43-424)-NotI-TrxA(2-109)-FLAG-KpnI | 169 |
| MaIE(S)-NTR-TrxA | SalI-MaIE(1-28)-Factor Xa-NTR(43-424)-NotI-TrxA(2-109)-FLAG-KpnI | 170 |

| | | |
|---|---|---|
| a. (L) refers to MalE residues 1-370, and (S) refers to MalE residues 1-28. b. All mature constructs were cloned into SalI and Kpnl sites of SEQ ID NOS.: 140, 142, 151 and 153. | | |

**TABLE 24. OLIGONUCLEOTIDE PRIMER SEQUENCES FOR TABLE 23**

| SEQ ID NO.: | Primer name | 5' to 3'sequence |
|---|---|---|
| 171 | MaIE-1 | 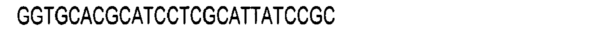 |
| 172 | MaIE-2 | 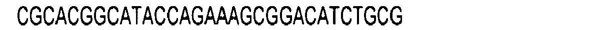 |
| 173 | MaIE-1-SalI | 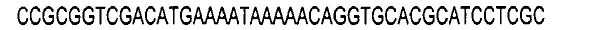 |
| 174 | MaIE-2-XaNTR | 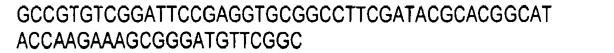 |
| 175 | NTR-1 | 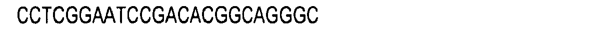 |
| 176 | NTR-2 | 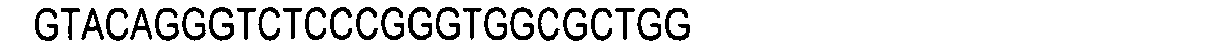 |
| 177 | NTR-1-Xa | 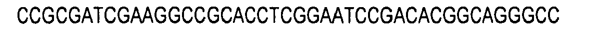 |
| 178 | NTR-2-Flag | 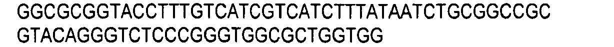 |
| 179 | NTR-2-Stop Kpnl | 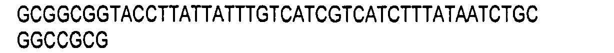 |
| 180 | NTR-1-Xa Lead | 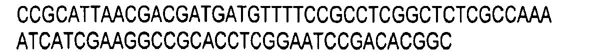 |
| 181 | NTR-2-Lead2 SalI | 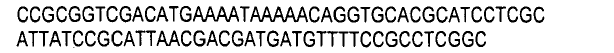 |
| 182 | TrxA-1 | 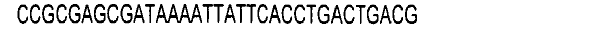 |
| 183 | TrxA-2 | 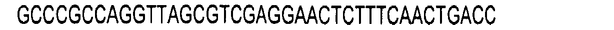 |
| 184 | TrxA-1-NotI | 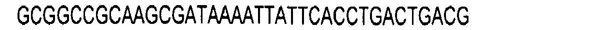 |
| 185 | TrxA-2-NotI | 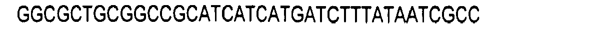 |

Oligonucleotides SEQ ID NOS.: 171, 172, 173 and 174 were used to amplify malE residues 1-370 from the E. coli chromosome to create SEQ ID NO.: 164. Using overlap PCR with the extended NTR homology, a chimeric translational fusion was made between MalE (1-370) and NTR residues 43-424 (SEQ ID NO.: 165) to create a SEQ ID NO.: 166. SEQ ID NO.: 166 was cloned into plasmids pMPX-85, pMPX-87, pMPX-66 and pMPX-67 (respectively, SEQ ID NOS.: 140, 142, 151 and 153) using Sall and Kpnl.

Three-step PCR with oligonucleotides, SEQ ID NOS.: 175 and 176 as primers was used to amplify NTR residues 43-424 from rat brain cDNA. SEQ ID NOS.: 177 and 178 were then used with the NTR (43-424) template to add factor Xa and FLAG sequence. Finally, SEQ ID NOS.: 177 and 179 were used to add a Kpnl site to create SEQ ID NO.: 165. Using overlap PCR with maIE(1-370) containing extended NTR homology, a chimeric translational fusion was made between NTR (43-424) and MalE (1-370) (SEQ ID NO.: 164) to create a SEQ ID NO.: 166. SEQ ID NO.: 166 was cloned into SEQ ID NOS.: 140, 142, 151 and 153 using Sall and Kpnl.

Using three-step PCR oligonucleotides SEQ ID NOS.: 175 and 176 were first used to amplify NTR residues 43-424 from rat brain cDNA. SEQ ID NOS.: 178 and 180 were then used with the NTR (43-424) template to add factor Xa and FLAG sequence. Finally, SEQ ID NOS.: 179 and 181 were used to add Kpnl to create SEQ ID NO.: 167. SEQ ID NO.: 167 was cloned into SEQ ID NOS.: 140, 142, 151 and 153 using Sall and Kpnl.

Oligonucleotides SEQ ID NOS.: 182, 183, 184 and 185 were used to amplify TrxA residues 2-109 from the E. coli chromosome to create SEQ ID NO.: 168. Using Notl, TrxA residues 2-109 was cloned into SEQ ID NOS.: 166 and 167 to create SEQ ID NOS.: 169 and 170, respectively. SEQ ID NO.: 169 and 170 were cloned into SEQ ID NOS.: 140, 142, 151 and 153 using Sall and Kpnl.

### EXAMPLE 22: METHODS FOR FUNCTIONAL GPCR ASSAY

Functional G-protein-coupled receptor (GPCR) binding assays in minicells requires expression of a GPCR of interest into the minicell membrane bilayer and cytoplasmic expression of the required G-protein. For these purposes, constructs were created to co-express both a GPCR and a G-protein. To regulate the ratio of GPCR to G-protein, transcriptional fusions were created. In these constructs, the GPCR and G-protein are co-transcribed as a bi-cistronic mRNA. To measure the GPCR-G-protein interaction in the intact minicell, each protein was created as a chimera with a transactivation domain. For these studies the N-terminal DNA-binding, activation domain of the ToxR protein from V. cholerae was fused to the C-terminus of both the GPCR and G-protein. Finally, to measure the interaction GPCR-G-protein interaction, the ToxR-activated ctx promoter region was cloned in front of lacZ. Dimerization of the ToxR DNA-binding region will bind and activate the ctx promoter. In this construct, heterodimerization of the GPCR and G-protein will promote dimerization of ToxR that will be monitored by LacZ expression. Details of these constructs are shown in Table 25.

**TABLE 25. FUNCTIONAL HUMAN GPCR CONSTRUCTS**

| Protein ^{a,b} | Construct ^{a,b} | SEQ ID NO.: |
|---|---|---|
| □2AR | Sall-□2AR-PstI, XhoI | 186 |
| GS1□ | XhoI-GS1□□XbaI | 187 |
| □2AR-GS1□ fusion | SalI-□2AR-PstI, XhoI-GS1□□XbaI | 188 |
| □2AR-stop | SalI-□2AR-PstI-Stop-SD-XhoI | 189 |
| □2AR-stop-GS1□ | SalI-□2AR-PstI-Stop-SD-XhoI-GS1□□XbaI | 190 |
| ToxR | ClaI-ToxR-XbaI | 191 |
| GS1□ | XhoI-GS1□-ClaI | 192 |
| GS2□ | XhoI-GS2□-ClaI | 193 |
| G□q | XhoI-Gq□-ClaI | 194 |
| Gi□ | XhoI-Gi□-ClaI | 195 |
| G□12/13 | XhoI-G□12/13-ClaI | 196 |
| GS1□-ToxR | XhoI-GS1□-ClaI-ToxR-XbaI | 197 |
| GS2□-ToxR | XhoI-GS2□-ClaI-ToxR-XbaI | 198 |
| G□q-ToxR | Xhol- G□q -Clal-ToxR-Xbal | 199 |
| Gi□-ToxR | XhoI-Gi□-ClaI-ToxR-XbaI | 200 |
| G□12/13-ToxR | Xhol- G□12/13-ClaI-ToxR-XbaI | 201 |
| ToxR | PstI-ToxR-XhoI | 202 |
| □2AR | SalI-□2AR-PstI | 203 |
| □2AR-ToxR | SalI-□2AR-PstI-ToxR-Stop-SD-XhoI | 204 |
| □2AR-ToxR-stop-GS1□-ToxR | SalI-□2AR-PstI-ToxR-Stop-SD-XhoI-GS1□-ClaI-ToxR-XbaI | 205 |
| Pctx | XbaI-Pctx-lacZ homology | 206 |
| lacZ | Pctx homology-lacZ-XbaI | 207 |
| Pctx::lacZ | XbaI-Pctx-lacZ-XbaI | 208 |

| | | |
|---|---|---|
| a. "SD" refers to the Shine-Delgarno ribosome-binding sequence and "ToxR" refers to the transactivation, DNA-binding domain of the ToxR protein (residues 5-141). b. All mature constructs were cloned into SalI and Xbal sites of SEQ ID NOS.: 140, 142, 151 and 153. | | |

**TABLE 26. OLIGONUCLEOTIDE PRIMER SEQUENCES FOR TABLE 25.**

| SEQ ID NO.: | Primer name | 5' to 3' sequence |
|---|---|---|
| 209 | □2AR-1 | 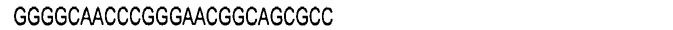 |
| 210 | □2AR-2 | 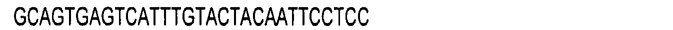 |
| 211 | □2AR-1-SalI | 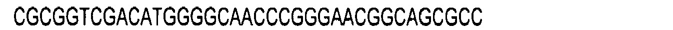 |
| 212 | □2AR-2-Link-Xhol | 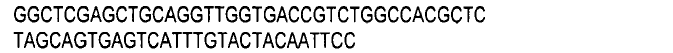 |
| 213 | GS1□-1 | 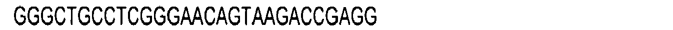 |
| 214 | GS1□2 | 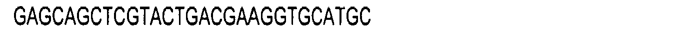 |
| 215 | GS1□-1-XhoI | 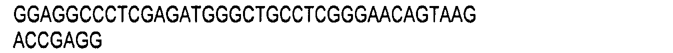 |
| 216 | GS1□-2-XbaI | 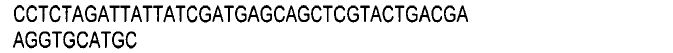 |
| 217 | GS1□-2-ClaI | 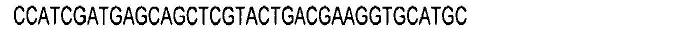 |
| 218 | G□12-1 | 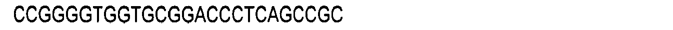 |
| 219 | G□12-2 | 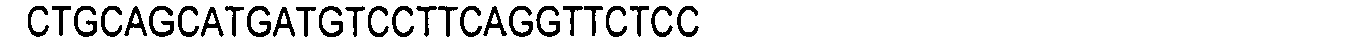 |
| 220 | G□12-1-XhoI | 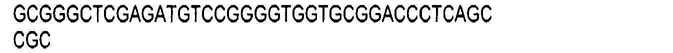 |
| 221 | G□12-2-ClaI | 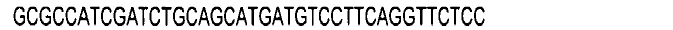 |
| 222 | G□q-1 | 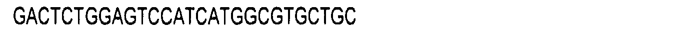 |
| 223 | G□q-2 | 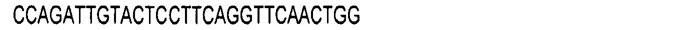 |
| 224 | G□q-1-XhoI | 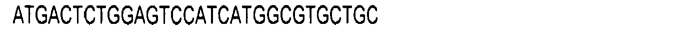 |
| 225 | G□q-2-ClaI | 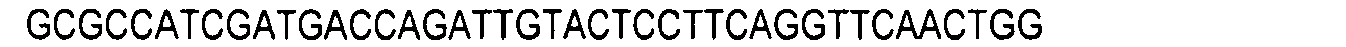 |
| 226 | Gi□-1 | 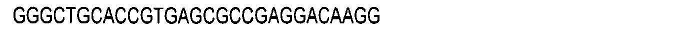 |
| 227 | Gi□-2 | 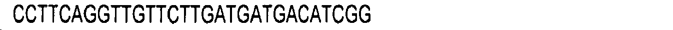 |
| 228 | Gi□-1-XhoI | 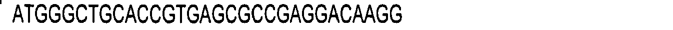 |
| 229 | Gi□-2-ClaI | 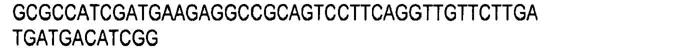 |
| 230 | GS2□-1 | 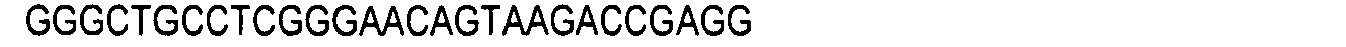 |
| 231 | GS2□-2 | 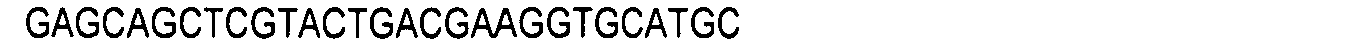 |
| 232 | GS2□-1-XhoI | 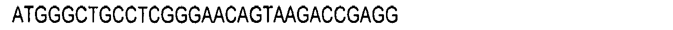 |
| 233 | GS2□-2-ClaI | 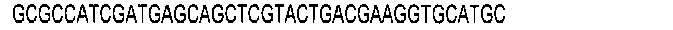 |
| 234 | □2AR-2-Link-Stop-Xhol | 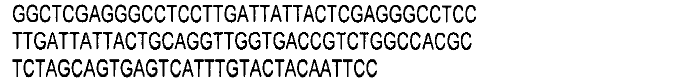 |
| 235 | □2AR-2-Link | 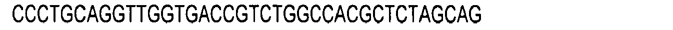 |
| | | 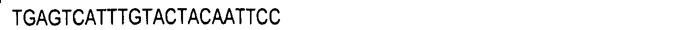 |
| 236 | Tox (5-141)-1B | 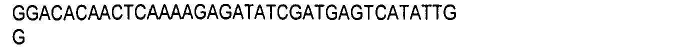 |
| 237 | Tox (5-141)-2 | 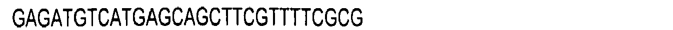 |
| 238 | Tox (5-141)-1-Link | 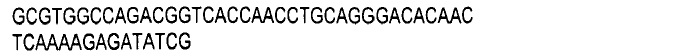 |
| 239 | Tox (5-141)-2-Xhol | 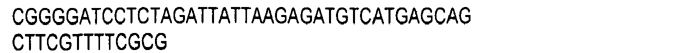 |
| 240 | Ctx-1 | 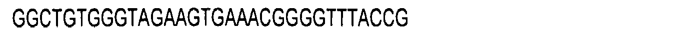 |
| 241 | Ctx-2 | 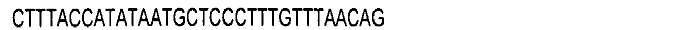 |
| 242 | Ctx-2-Xbal | 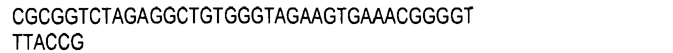 |
| 243 | Ctx-2-LacZ | 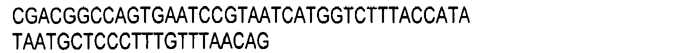 |
| 244 | LacZ-1 | 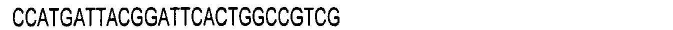 |
| 245 | LacZ-2 | 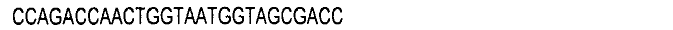 |
| 246 | LacZ-1-Ctx | 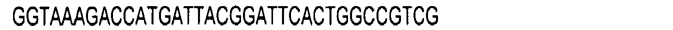 |
| 247 | LacZ-2-XbaI | 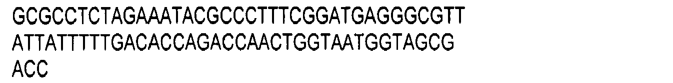 |

Oligonucleotides SEQ ID NOS.: 209, 210, 211 and 212 were used to amplify human □2AR from human cDNA to create SEQ ID NO.: 186. Using Sall and Xhol a translational fusion was made between □2AR and human GS10 (SEQ ID NO.: 187) to create a SEQ ID NO.: 188. SEQ ID NO.: 188 was cloned into SEQ ID NOS.: 140, 142, 151 and 153 using Sall and Xbal.

Oligonucleotides SEQ ID NOS.: 213, 214, 215 and 216 were used to amplify human GS1□ from human cDNA to create SEQ ID NO.: 187. Using Xhol and Xbal a translational fusion was made between GS10 and human □2AR (SEQ ID NO.: 186) create SEQ ID NO.: 188. SEQ ID NO.: 188 was cloned into SEQ ID NOS.: 140, 142, 151and 153 using Sall and Xbal.

Oligonucleotides SEQ ID NOS.: 213, 214, 215 and 217 were used to amplify human GS1□ from human cDNA to create SEQ ID NO.: 192. Using Xhol and Xbal a translational fusion was made with ToxR residues 5-141 from Vibrio cholerae (SEQ ID NO.: 191) to create SEQ ID NO.: 197. To be used to create a transcriptional fusion with □2AR-ToxR chimeras as shown in SEQ ID NO.: 205 and future GPCR-ToxR chimeras.

Oligonucleotides SEQ ID NOS.: 218, 219, 220 and 221 were used to amplify human G□12/13 from human cDNA to create SEQ ID NO.: 196. Using Xhol and Xbal a translational fusion was made with ToxR residues 5-141 from Vibrio cholerae (SEQ ID NO.: 191) to create SEQ ID NO.: 201. To be used to create future transcriptional fusions with GPCR-ToxR chimeras as shown in SEQ ID NO.: 205.

Oligonucleotides SEQ ID NOS.: 222, 223, 224 and 225 were used to amplify human G□q from human cDNA to create SEQ ID NO.: 194. Using Xhol and Xbal a translational fusion was made with ToxR residues 5-141 from Vibrio cholerae (SEQ ID NO.: 191) to create SEQ ID NO.: 199. To be used to create future transcriptional fusions with GPCR-ToxR chimeras as shown in SEQ ID NO.: 205.

Oligonucleotides SEQ ID NOS.: 226, 227, 228 and 229 were used to amplify human Gi□ from human cDNA to create SEQ ID NO.: 195. Using Xhol and Xbal a translational fusion was made with ToxR residues 5-141 from Vibrio cholerae (SEQ ID NO.: 191) to create SEQ ID NO.: 200. To be used to create future transcriptional fusions with GPCR-ToxR chimeras as shown in SEQ ID NO.: 205.

Oligonucleotides SEQ ID NOS.: 230, 231, 232 and 233 were used to amplify human GS2□ from human cDNA to create SEQ ID NO.: 193. Using Xhol and Xbal a translational fusion was made with ToxR residues 5-141 from Vibrio cholerae (SEQ ID NO.: 191) to create SEQ ID NO.: 198. To be used to create future transcriptional fusions with GPCR-ToxR chimeras as shown in SEQ ID NO.: 205.

Oligonucleotides SEQ ID NOS.: 209, 210, 211 and 234 were used to amplify human □2AR from human cDNA to create SEQ ID NO.: 189. Using SalI and Xhol a transcriptional fusion was made between □2AR and human GS1□ (SEQ ID NO.: 187) to create a SEQ ID NO.: 190. SEQ ID NO.: 190 was cloned into SEQ ID NOS.: 140, 142, 151 and 153 using SalI and Xbal.

Oligonucleotides SEQ ID NOS.: 236, 237, 238 and 239 were used to amplify bases coinciding with ToxR residues 5-141 from Vibrio Cholerae to create SEQ ID NO.: 202. Using Pstl and Xhol a translational fusion was made between ToxR and human □2AR (SEQ ID NO.: 203) to create SEQ ID NO.: 204.

Oligonucleotides SEQ ID NOS.: 209, 210, 211 and 235 were used to amplify human □2AR from human cDNA to create SEQ ID NO.: 203. Using SalI and Pstl a translational fusion was made between □2AR and ToxR (SEQ ID NO.: 202) to create SEQ ID NO.: 204.

Using oligonucleotides SEQ ID NOS.: 197 and 204 transcriptional fusions were created between the □2AR-ToxR translational fusion (SEQ ID NO.: 204) and the GS1□-ToxR translational fusion (SEQ ID NO.: 197) to create SEQ ID NO.: 205.

Oligonucleotides SEQ ID NOS.: 240, 241, 242 and 243 were used to amplify the ctx promoter region (Pctx) from Vibrio cholerae to create SEQ ID NO.: 206. Combining this PCR product in combination with the SEQ ID NO.: 207 PCR product and amplifying in the presence of SEQ ID NOS.: 242, 247, SEQ ID NO.: 208 was created. Using Xbal, the SEQ ID NO.: 208 reporter construct was subsequently cloned into pACYC184 for co-transformation with the GPCR-G-protein fusions constructs above.

Oligonucleotides SEQ ID NOS.: 244, 245, 246 and 247 were used to amplify the lacZ from E. coli to create SEQ ID NO.: 207. Combining this PCR product in combination with the SEQ ID NO.: 206 PCR product and amplifying in the presence of SEQ ID NOS.: 242 and 247, SEQ ID NO.: 208 was created. Using Xbal, the 208 reporter construct was subsequently cloned into pACYC184 for co-transformation with the GPCR-G-protein fusions constructs above.

### EXAMPLE 23. MODULAR MEMBRANE-TARGETING AND SOLUBILIZATION EXPRESSION CONSTRUCTS

To produce membrane proteins efficiently in minicells it may be necessary to create chimeric fusions with the membrane protein of interest. In this Example various regions of the MalE protein have been cloned into a modular expression system designed to create chimeric fusions with direct difficult to target membrane proteins to produce leader domains that will direct the proteins to the cytoplasmic membrane (Miller, K., W., et al. 1998. Production of active chimeric pediocin AcH in Escherichia coli in the absence of processing and secretion genes from the Pediococcus pap operon. Appl. Environ. Microbiol. 64:14-20). Similarly, a modified version of the TrxA protein has also been cloned into this modular expression system to create chimeric fusions with proteins that are difficult to maintain in a soluble conformation (LaVallie, E. R., et al. 1993. A thioredoxin gene fusion expression system that circumvents inclusion body formation in the E. coli cytoplasm. Biotechnology (N. Y.) 11:187-193). Table 27 describes each of these modular constructs.

**TABLE 27. MODULAR MEMBRANE-TARGETING AND SOLUBILIZATION EXPRESSION CONSTRUCTS**

| Protein ^{a} | Construct ^{a} | SEQ ID NO |
|---|---|---|
| MalE (1-28) | Nsil-MaIE(1-28)-Factor Xa-Pstl, SalI, Xbal-FLAG, Nhel | 248 |
| MalE (1-370, del 354-364) | Nsil-MaIE(1-370, del 354-364)-Factor Xa-Pstl, SalI, Xbal-FLAG, Nhel | 249 |
| TrxA (2-109, del 103-107) | Pstl, SalI, Xbal-TrxA(2-109, del 103-107)-FLAG-Nhel | 250 |
| MalE (1-28)-TrxA (2-109, del 103-107) | NsiI-MaIE(1-28)-Factor Xa-PstI, SalI, XbaI-TrxA (2-109 del 103-107)-FLAG, Nhel | 251 |
| MalE (1-370, del 354-364)-TrxA (2-109, del 103-107) | NsiI-MaIE(1-370, del 354-364)-Factor Xa-Pstl, SalI, XbaI-TrxA (2-109 del 103-107)-FLAG, Nhel | 252 |

| | | |
|---|---|---|
| a. The term "del" refers to a deletion in which amino acid residues following the term "del" are removed from the sequence. | | |

**TABLE 28. OLIGONUCLEOTIDE PRIMER SEQUENCES FOR TABLE 27.**

| SEQ ID NO.: | Primer name | 5' to 3' sequence |
|---|---|---|
| 253 | MaIE-1-NsiI | 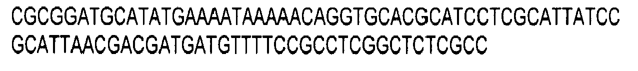 |
| 254 | MaIE-2-middle | 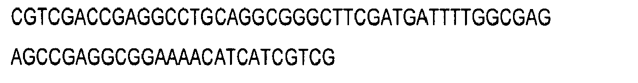 |
| 255 | MaIE-3s-NheI | 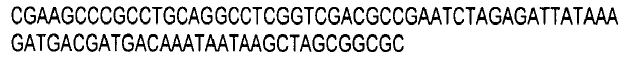 |
| 256 | MaIE-4-NheI | 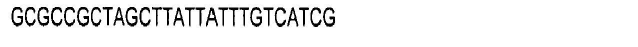 |
| 257 | MaIE-1a | 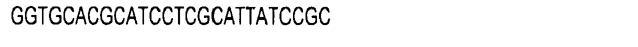 |
| 258 | MaIE-2a | 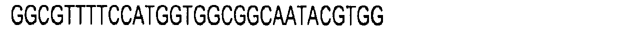 |
| 259 | MaIE-1-NsiI | 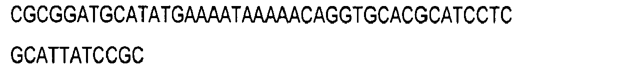 |
| 260 | MaIE-2-NheI | 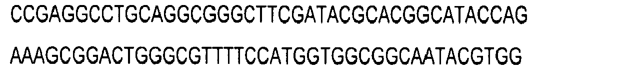 |
| 261 | MaIE-3L-NheI | 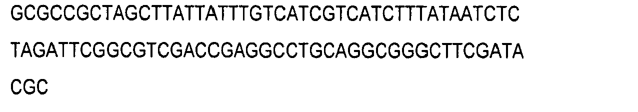 |
| 262 | TrxA-1a | 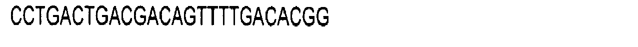 |
| 263 | TrxA-2a | 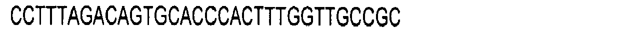 |
| 264 | TrxA-1a-PstI | 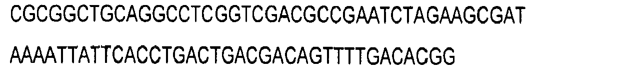 |
| 265 | TrxA-2-Nhel | 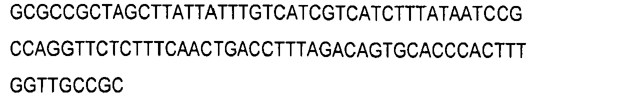 |

Oligonucleotides SEQ ID NOS.: 253, 254, 255 and 256 overlap with each other to form a scaffold template to PCR amplify malE (1-28) to create a SEQ ID NO.: 248. Following PCR amplification, SEQ ID NO.: 248 was digested with Nsil and Nhel and cloned into SEQ ID NOS.: 152, 154, 139 and 141 digested with Pstl and Xbal. The resultant products create SEQ ID NOS.: 266, 267, 268 and 269, respectively, that lose both the 5-prime Pstl and 3-prime Xbal restriction site and retain the Pstl, SalI, and Xbal restriction sites between MalE (1-28) and the FLAG sequence. Insertion of a protein in alignment with these sites results in a chimeric protein containing amino-terminal MalE (1-28) and carboxy-terminal FLAG.

Oligonucleotides SEQ ID NOS.: 257, 258, 259 and 260 were used to amplify maIE (1-370 with a deletion removing residues 354-364) to create SEQ ID NO.: 249. Following PCR amplification, SEQ ID NO.: 249 was digested with Nsil and Nhel and cloned into SEQ ID NOS.: 152, 154, 139 and 141 digested with Pstl and Xbal. The resultant products create SEQ ID NOS.: 270, 271, 272 and 273, respectively, that lose both the 5-prime Pstl and 3-prime Xbal restriction site and retain the Pstl, SalI, and Xbal restriction sites between MalE (1-370, deI 354-364) and the FLAG sequence. Insertion of a protein in alignment with these sites results in a chimeric protein containing amino-terminal MalE (1-370, del 354-364) and carboxy-terminal FLAG.

Oligonucleotides SEQ ID NOS.: 262, 263, 264 and 265 were used to amplify trxA (2-109 with a deletion removing residues 103-107) to create SEQ ID NO.: 250. Following PCR amplification, SEQ ID NO.: 250 was digested with Pstl and Nhel and cloned into SEQ ID NOS.: 152, 154, 139 and 141 digested with Pstl and Xbal. to create SEQ ID NOS.: 274, 275, 276 and 277, respectively. Using these restriction digestion combinations results in loss of the Xbal SEQ ID NO.: 249 insertion site.

The resultant products create SEQ ID NOS.: 274, 275, 276 and 277, respectively, that lose the 3-prime Xbal restriction site and retain the Pstl, SalI, and Xbal restriction sites on the 3-prime end of the TrxA (1-109, del 103-107) sequence. Insertion of a protein in alignment with these sites results in a chimeric protein containing Carboxy-terminal TrxA (1-109, del 103-107)-FLAG.

SEQ ID NO.: 248 was digested with Nsil and Xbal and cloned into SEQ ID NOS.: 274, 275, 276 and 277 that were digested with Pstl and Xbal. The resultant products create SEQ ID NOS.: 278, 279, 280 and 281, respectively, that lose the 5 prime PstI restriction site and retain the Pstl, SalI, and Xbal restriction sites between MalE (1-28) and TrxA (1-109, del 103-107). Insertion of a protein in alignment with these sites results in a chimeric protein containing amino-terminal MalE (1-28) and carboxy-terminal TrxA (1-109, del 103-107)-FLAG.

SEQ ID NO.: 249 was digested with Nsil and Xbal and cloned into SEQ ID NOS.: 274, 275, 276 and 277 that were digested with Pstl and Xbal. The resultant products create SEQ ID NOS.: 282, 283, 284 and 285, respectively, that lose the 5 prime PstI restriction site and retain the Pstl, SalI, and Xbal restriction sites between MalE (1-370, del 354-364) and TrxA (1-109, del 103-107). Insertion of a protein in alignment with these sites results in a chimeric protein containing amino-terminal MalE (1-370, del 354-364) and carboxy-terminal TrxA (1-109, del 103-107)-FLAG.

The contents of the articles, patents, and patent applications, and all other documents and electronically available information mentioned or cited herein, are hereby incorporated by reference in their entirety to the same extent as if each individual publication was specifically and individually indicated to be incorporated by reference. Applicants reserve the right to physically incorporate into this application any and all materials and information from any such articles, patents, patent applications, or other documents.

The inventions illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.
The aspects of the invention are
1. A minicell comprising a membrane protein selected from the group consisting of a eukaryotic membrane protein, an archeabacterial membrane protein and an organellar membrane protein.
2. The minicell according to aspect 1, wherein said minicell is selected from the group consisting of a eubacterial minicell, a poroplast, a spheroplast and a protoplast.
3. The minicell according to aspect 1, wherein said minicell comprises a biologically active compound.
4. The minicell according to aspect 1, wherein said minicell comprises a expression construct, wherein said first expression construct comprises expression sequences operably linked to an ORF that encodes a protein.
5. The minicell according to aspect 4, wherein said ORF encodes said membrane protein.
6. The minicell according to aspect 4, wherein said expression sequences that are operably linked to an ORF are inducible and/or repressible.
7. The minicell according to aspect 4, wherein said minicell comprises a second expression construct, wherein said second expression construct comprises expression sequences operably linked to a gene.
8. The minicell according to aspect 7, wherein said expression sequences that are operably linked to a gene are inducible and/or repressible.
9. The minicell according to aspect 7, wherein the gene product of said gene regulates the expression of the ORF that encodes said protein.
10. The minicell according to aspect 7, wherein the gene product of said gene is a nucleic acid.
11. The minicell according to aspect 7, wherein the gene product of said gene is a polypeptide.
12. The minicell according to aspect 11, wherein said polypeptide is a membrane protein, a soluble protein or a secreted protein.
13. The minicell according to aspect 12, wherein said membrane protein is a membrane fusion protein, said membrane fusion protein comprising a first polypeptide, said first polypeptide comprising at least one transmembrane domain or at least one membrane anchoring domain; and a second polypeptide.
14. A minicell comprising a membrane fusion protein, said fusion protein comprising a first polypeptide, said first polypeptide comprising at least one transmembrane domain or at least one membrane anchoring domain; and a second polypeptide, wherein said second polypeptide is not derived from a eubacterial protein and is neither a His tag nor a glutathione-S-transferase polypeptide.
15. The minicell according to aspect 14, wherein said minicell is selected from the group consisting of a eubacterial minicell, a poroplast, a spheroplast and a protoplast.
16. The minicell according to aspect 14, wherein said minicell comprises a biologically active compound.
17. A minicell comprising a membrane conjugate, wherein said membrane conjugate comprises a membrane protein chemically linked to a conjugated compound.
18. The minicell according to aspect 17, wherein said minicell is selected from the group consisting of a eubacterial minicell, a poroplast, a spheroplast and a protoplast.
19. The minicell according to aspect 17, wherein said minicell comprises a biologically active compound.
20. The minicell according to aspect 17, wherein said conjugated compound is selected from the group consisting of a nucleic acid, a polypeptide, a lipid and a small molecule.
21. A method for making minicells, comprising (a) culturing a minicell-producing parent cell, wherein said parent cell comprises an expression construct, wherein said expression construct comprises a gene operably linked to expression sequences that are inducible and/or repressible, and wherein induction or repression of said gene causes or enhances the production of minicells ; and (b) separating said minicells from said parent cell, thereby generating a composition comprising minicells, wherein an inducer or repressor is present within said parent cells during one or more steps and/or between two or more steps of said method.
22. The method according to aspect 21, further comprising (c) purifying said minicells from said composition.
23. The method according to aspect 21, wherein said minicell is selected from the group consisting of a eubacterial minicell, a poroplast, a spheroplast and a protoplast.
24. The method according to aspect 21, wherein said gene expresses a gene product that is a factor that is involved in or modulates DNA replication, cellular division, cellular partitioning, septation, transcription, translation, or protein folding.
25. The method according to aspect 21, wherein said minicells are separated from said parent cells by a process selected from the group consisting of centrifugation, ultracentrifugation, density gradation, immunoaffinity and immunoprecipitation.
26. The method according to aspect 22, wherein said minicell is a poroplast, said method further comprising (d) treating said minicells with an agent, or incubating said minicells under a set of conditions, that degrades the outer membrane of said minicell.
27. The method according to aspect 26, wherein said outer membrane is degraded by treatment with an agent selected from the group consisting of EDTA, EGTA, lactic acid, citric acid, gluconic acid, tartaric acid, polyethyleneimine, polycationic peptides, cationic leukocyte peptides, aminoglycosides, aminoglycosides, protamine, insect cecropins, reptilian magainins, polymers of basic amino acids, polymixin B, chloroform, nitrilotriacetic acid and sodium hexametaphosphate and/or by exposure to conditions selected from the group consisting of osmotic shock and insonation.
28. The method according to aspect 26, further comprising removing one or more contaminants from said composition.
29. The method according to aspect 28, wherein said contaminant is LPS or peptidoglycan.
30. The method according to aspect 29, wherein said LPS is removed by contacting said composition to an agent that binds or degrades LPS.
31. The method according to aspect 21, wherein said minicell-producing parent cell comprises a mutation in a gene required for lipopolysaccharide synthesis.
32. The method according to aspect 22, wherein said minicell is a spheroplast, said method further comprising (d) treating said minicells with an agent, or incubating said minicells under a set of conditions, that disrupts or degrades the outer membrane; and (e) treating said minicells with an agent, or incubating said minicells under a set of conditions, that disrupts or degrades the cell wall.
33. The method according to aspect 32, wherein said agent that disrupts or degrades the cell wall is a lysozyme, and said set of conditions that disrupts or degrades the cell wall is incubation in a hypertonic solution.
34. The method according to aspect 22, wherein said minicell is a protoplast, said method further comprising (d) treating said minicells with an agent, or incubating said minicells under a set of conditions, that disrupt or degrade the outer membrane; (e) treating said minicells with an agent, or incubating said minicells under a set of conditions, that disrupts or degrades the cell wall, in order to generate a composition that comprises protoplasts; and purifying protoplasts from said composition.
35. The method according to aspect 22, further comprising preparing a denuded minicell from said minicell.
36. The method according to aspect 22, further comprising covalently or non-covalently linking one or more components of said minicell to a conjugated moiety.
37. A method of preparing a L-form minicell comprising: (a) culturing an L-form eubacterium, wherein said eubacterium comprises one or more of the following: (i) an expression element that comprises a gene operably linked to expression sequences that are inducible and/or repressible, wherein induction or repression of said gene regulates the copy number of an episomal expression construct; (ii) a mutation in an endogenous gene, wherein said mutation regulates the copy number of an episomal expression construct; (iii) an expression element that comprises a gene operably linked to expression sequences that are inducible and/or repressible, wherein induction or repression of said gene causes or enhances the production of minicells; and (iv) a mutation in an endogenous gene, wherein said mutation causes or enhances minicell production.
   (b) culturing said L-form minicell-producing parent cell in media under conditions wherein minicells are produced; and
   (c) separating said minicells from said parent cell, thereby generating a composition comprising L-form minicells,
   wherein an inducer or repressor is present within said minicells during one or more steps and/or between two or more steps of said method.
38. The method according to aspect 37, further comprising (d) purifying said L-form minicells from said composition.
39. A method of producing a protein, comprising: (a) transforming a minicell-producing parent cell with an expression element that comprises expression sequences operably linked to a nucleic acid having an ORF that encodes said protein; (b) culturing said minicell-producing parent cell under conditions wherein minicells are produced; and (c) purifying minicells from said parent cell, (d) purifying said protein from said minicells. wherein said ORF is expressed during step (b), between steps (b) and (c), and during step (c).
40. The method according to aspect 39, wherein said expression elements segregate into said minicells, and said ORF is expressed between steps (c) and (d).
41. The method according to aspect 39, wherein said protein is a membrane protein.
42. The method according to aspect 39, wherein said protein is a soluble protein contained within said minicells, further comprising: (e) at least partially lysing said minicells.
43. The method according to aspect 39, wherein said protein is a secreted protein, wherein said method further comprises (e) collecting a composition in which said minicells are suspended or with which said minicells are in contact.
44. The method according to aspect 39, wherein the expression sequences to which said ORF is operably linked are inducible, wherein said method further comprises adding an inducing agent between steps (a) and (b); during step (b); and between steps (b) and (c).
45. The method according to aspect 39, wherein the expression sequences to which said ORF is operably linked are inducible, wherein said expression elements segregate into said minicells, said method further comprises adding an inducing agent after step (c).
46. The method according to aspect 39, further comprising: (e) preparing poroplasts from said minicells, wherein said ORF is expressed during step (b); between steps (b) and (c); during step (c); between step (c) and step (d) when said expression elements segregate into said minicells; and/or after step (d) when said expression elements segregate into said minicells.
47. The method according to aspect 46, further comprising: purifying said protein from said poroplasts.
48. The method according to aspect 39, further comprising (e) preparing spheroplasts from said minicells, wherein said ORF is expressed during step (b), between steps (b) and (c), during step (c), between steps (c) and (d) and/or after step (d).
49. The method according to aspect 48, further comprising: purifying said protein from said spheroplasts.
50. The method according to aspect 39, further comprising (e) preparing protoplasts from said minicells, wherein said ORF is expressed during step (b), between steps (b) and (c), during step (c), between steps (c) and (d) and/or after step (d).
51. The method according to aspect 50, further comprising: (f) purifying said protein from said protoplasts.
52. The method according to aspect 39, further comprising (e) preparing membrane preparations from said minicells, wherein said ORF is expressed during step (b), between steps (b) and (c), during step (c), between steps (c) and (d) and/or after step (d).
53. The method according to aspect 48, further comprising: purifying said protein from said membrane preparations.
54. The method according to aspect 39, wherein said minicell-producing parent cell is an L-form bacterium.
55. A method of producing a protein, comprising: (a) transforming a minicell with an expression element. that comprises expression sequences operably linked to a nucleic acid having an ORF that encodes said protein; and (b) incubating said minicells under conditions wherein said ORF is expressed.
56. The method according to aspect 55, further comprising: (c) purifying said protein from said minicells.
57. The method according to aspect 55, wherein said minicell is selected from the group consisting of a eubacterial minicell, a poroplast, a spheroplast and a protoplast.
58. A method of producing a protein, comprising: (a) transforming a minicell-producing parent cell with an expression element that comprises expression sequences operably linked to a nucleic acid having an ORF that encodes a fusion protein comprising said protein and a polypeptide, wherein a protease-sensitive amino acid sequence is positioned between said protein and said polypeptide; (b) culturing said minicell-producing parent cell under conditions wherein minicells are produced; (c) purifying minicells from said parent cell, wherein said ORF is expressed during step (b); between steps (b) and (c); and/or after step (c) when said expression elements segregate into said minicells; and (d) treating said minicells with a protease that cleaves said sensitive amino acid sequence, thereby separating said protein from said polypeptide.
59. A poroplast, said poroplast comprising a vesicle, bonded by a membrane, wherein said membrane is an eubacterial inner membrane, wherein said vesicle is surrounded by a eubacterial cell wall, and wherein said eubacterial inner membrane is accessible to a compound in solution with said poroplast.
60. The poroplast according to aspect 59, wherein said poroplast is a cellular poroplast.
61. The poroplast according to aspect 59, wherein said compound has a molecular weight of at least 1 kD.
62. The poroplast according to aspect 59, wherein said poroplast comprises an exogenous nucleic acid.
63. The poroplast according to aspect 62, wherein said exogenous nucleic acid is an expression construct.
64. The poroplast according to aspect 63, wherein said expression construct comprises an ORF that encodes an exogenous protein, wherein said ORF is operably linked to expression sequences.
65. The poroplast according to aspect 64, wherein said poroplast comprises an exogenous protein.
66. The poroplast according to aspect 59, wherein said poroplast comprises an exogenous protein.
67. The poroplast according to aspect 66, wherein said exogenous protein is a fusion protein, a soluble protein or a secreted protein.
68. The poroplast according to aspect 66, wherein said exogenous protein is a membrane protein.
69. The poroplast according to aspect 68, wherein said membrane protein is accessible to compounds in solution with said poroplast.
70. The poroplast according to aspect 68, wherein said membrane protein is selected from the group consisting of a eukaryotic membrane protein, an archeabacterial membrane protein, and an organellar membrane protein.
71. The poroplast according to aspect 68, wherein said membrane protein is a fusion protein, said fusion protein comprising a first polypeptide, said first polypeptide comprising at least one transmembrane domain or at least one membrane anchoring domain; and a second polypeptide, wherein said second polypeptide is displayed by said poroplast.
72. The poroplast according to aspect 71, wherein said second polypeptide is displayed on the external side of said eubacterial inner membrane.
73. The poroplast according to aspect 59, wherein said poroplast comprises a membrane component that is chemically linked to a conjugated compound.
74. The poroplast according to aspect 64, wherein said expression construct comprises one or more DNA fragments from a genome or cDNA.
75. The poroplast according to aspect 64, wherein said exogenous protein has a primary amino acid sequence that is predicted from in silico translation of a nucleic acid sequence.
76. A method of making poroplasts or cellular poroplasts, comprising treating eubacterial minicells or cells with an agent, or incubating said minicells or cells under a set of conditions, that degrades the outer membrane of said minicells or cells.
77. The method according to aspect 76, further comprising purifying said poroplasts or cellular poroplasts in order to remove contaminants.
78. The method according to aspect 76, further comprising placing said poroplasts in a hypertonic solution, wherein 90 % or more of said cells or minicells used to prepare said poroplasts would lyse in said solution under the same conditions.
79. A solid support comprising a minicell.
80. The solid support according to aspect 79, wherein said minicell is selected from the group consisting of a eubacterial minicell, a poroplast, a spheroplast and a protoplast.
81. The solid support according to aspect 79, wherein said solid support is a dipstick.
82. The solid support according to aspect 79, wherein said solid support is a bead.
83. The solid support according to aspect 79, wherein said solid support is a mictrotiter multiwell plate.
84. The solid support according to aspect 79, wherein said minicell comprises a detectable compound.
85. The solid support according to aspect 84, wherein said detectable compound is a fluorescent compound.
86. The solid support according to aspect 79, wherein said minicell displays a membrane component.
87. The solid support according to aspect 86, wherein said membrane component is selected from the group consisting of (i) a eukaryotic membrane protein, (ii) an archeabacterial membrane protein, (iii) an organellar membrane protein, (iv) a fusion protein comprising at least one transmembrane domain or at least one membrane anchoring domain, and (v) a membrane conjugate comprising a membrane component chemically linked to a conjugated compound.
88. The solid support according to aspect 86, wherein said membrane component is a receptor.
89. The solid support according to aspect 87, wherein said solid support further comprises a co- receptor.
90. The solid support according to aspect 79, wherein said minicell displays a binding moiety.
91. A solid support comprising a minicell, wherein said minicell displays a fusion protein, said fusion protein comprising a first polypeptide that comprises at least one transmembrane domain or at least one membrane anchoring domain, and a second polypeptide.
92. The solid support according to aspect 91, wherein said minicell is selected from the group consisting of a eubacterial minicell, a poroplast, a spheroplast and a protoplast.
93. The solid support according to aspect 91, wherein said second polypeptide comprises a binding moiety.
94. The solid support according to aspect 91, wherein said second polypeptide comprises an enzyme moiety.
95. A solid support comprising a minicell, wherein said minicell comprises a membrane conjugate comprising a membrane component chemically linked to a conjugated compound.
96. The solid support according to aspect 95, wherein said conjugated compound is a spacer.
97. The solid support according to aspect 96, wherein said spacer is covalently linked to said solid support.
98. The solid support according to aspect 95, wherein said conjugated compound is covalently linked to said solid support.
99. A minicell comprising a biologically active compound, wherein said minicell displays a binding moiety, wherein said binding moiety is part of a fusion protein comprising a first polypeptide that comprises at least one transmembrane domain or at least one membrane anchoring domain and a second polypeptide that comprises a binding moiety, and said minicell is a poroplast, spheroplast or protoplast.
100. A eubacterial minicell comprising a biologically active compound, wherein said minicell displays a binding moiety, wherein said binding moiety is selected from the group consisting of (a) a eukaryotic membrane protein; (b) an archeabacterial membrane protein; (c) an organellar membrane protein; and (d) a fusion protein, said fusion protein comprising a first polypeptide, said first polypeptide comprising at least one transmembrane domain or at least one membrane anchoring domain; and a second polypeptide, wherein said second polypeptide is not derived from a eubacterial protein and is neither a His tag nor a glutathione-S-transferase polypeptide, and wherein said polypeptide comprises a binding moiety.
101. The minicell according to aspect 99, wherein said binding moiety is selected from the group consisting of an antibody, an antibody derivative, a receptor and an active site of a non-catalytic derivative of an enzyme.
102. The minicell according to aspect 99, wherein said binding moiety is a single-chain antibody.
103. The minicell according to aspect 99, wherein said binding moiety is directed to a ligand selected from the group consisting of an epitope displayed on a pathogen, an epitope displayed on an infected cell and an epitope displayed on a hyperproliferative cell.
104. The minicell according to aspect 99, wherein said biologically active compound is selected from the group consisting of a radioisotope, a polypeptide, a nucleic acid and a small molecule.
105. The minicell according to aspect 99, further comprising a first and second nucleic acid, wherein said first nucleic acid comprises eukaryotic expression sequences operably linked to a first ORF, and a second nucleic acid, wherein said second nucleic acid comprises eubacterial expression sequences operably linked to a second ORF.
106. The minicell according to aspect 105, wherein one of said ORFs encodes a protein that comprises said binding moiety.
107. The minicell according to aspect 105, wherein said eubacterial expression sequences are induced and/or derepressed when said binding moiety is in contact with a target cell.
108. The minicell according to aspect 105, wherein said eukaryotic expression sequences are induced and/or derepressed when said nucleic acid is in the cytoplasm of a eukaryotic cell.
109. The minicell according to aspect 105, wherein the protein encoded by said first ORF comprises eukaryotic secretion sequences and/or the protein encoded by said second ORF comprises eubacterial secretion sequences.
110. A method of associating a radioactive compound with a cell, wherein said cell displays a ligand specifically recognized by a binding moiety, comprising contacting said cell with a minicell that comprises said radioactive compound and displays said binding moiety.
111. The method according to aspect 110, wherein said minicell is selected from the group consisting of a eubacterial minicell, a poroplast, a spheroplast and a protoplast.
112. The method according to aspect 110, wherein the amount of radiation emitted by said radioactive isotope is sufficient to be detectable.
113. The method according to aspect 110, wherein the amount of radiation emitted by said radioactive isotope is sufficient to be cytotoxic.
114. The method according to aspect 110, wherein said ligand displayed by said cell is selected from the group consisting of an epitope displayed on a pathogen, an epitope displayed on an infected cell and an epitope displayed on a hyperproliferative cell.
115. The method according to aspect 110, wherein said binding moiety is selected from the group consisting of an antibody, an antibody derivative, a channel protein protein and a receptor.
116. The method according to aspect 110, wherein said binding moiety is a single-chain antibody.
117. The method according to aspect 110, wherein said binding moiety is selected from the group consisting of an aptamer and a small molecule.
118. A method of delivering a biologically active compound to a cell, wherein said cell displays a ligand specifically recognized by a binding moiety, comprising contacting said cell with a minicell that displays said binding moiety, wherein said minicell comprises said biologically active compound, and wherein the contents of said minicell are delivered into said cell from a minicell bound to said cell.
119. The method according to aspect 118, wherein said minicell is selected from the group consisting of a eubacterial minicell, a poroplast, a spheroplast and a protoplast.
120. The method according to aspect 118, wherein said biologically active compound is selected from the group consisting of a nucleic acid, a lipid, a polypeptide, a radioactive compound, an ion and a small molecule.
121. The method according to aspect 118, wherein the membrane of said minicell comprises a system for transferring a molecule from the interior of a minicell into the cytoplasm of said cell.
122. The method according to aspect 121, wherein said system for transferring a molecule from the interior of a minicell into the cytoplasm of said cell is a Type III secretion system.
123. The method according to aspect 118, wherein said minicell further comprises a first and second nucleic acid, wherein said first nucleic acid comprises eukaryotic expression sequences operably linked to a first ORF, and a second nucleic acid, wherein said second nucleic acid comprises eubacterial expression sequences operably linked to a second ORF.
124. The method according to aspect 123, wherein one of said ORFs encodes a protein that comprises said binding moiety.
125. The method according to aspect 123, wherein said eubacterial expression sequences are induced and/or derepressed when said binding moiety is in contact with a target cell.
126. The method according to aspect 123, wherein said eukaryotic expression sequences are induced and/or derepressed when said nucleic acid is in the cytoplasm of a eukaryotic cell.
127. The method according to aspect 123, wherein the protein encoded by said first ORF comprises eukaryotic secretion sequences and/or the protein encoded by said second ORF comprises eubacterial secretion sequences.
128. A minicell displaying a synthetic linking moiety, wherein said synthetic linking moiety is covalenty or non-covalently attached to a membrane component of said mincell.
129. The minicell according to aspect 128, wherein said minicell is selected from the group consisting of a eubacterial minicell, a poroplast, a spheroplast and a protoplast.
130. A sterically stabilized minicell comprising a displayed moiety that has a longer half- life in vivo than a wild-type minicell, wherein said displayed moiety is a hydrophilic polymer that comprises a PEG moiety, a carboxylic group of a polyalkylene glycol or PEG stearate.
131. A minicell having a membrane comprising an exogenous lipid, wherein a minicell comprising said exogenous lipid has a longer half-life in vivo than a minicell lacking said exogenous lipid, and wherein said minicell is selected from the group consisting of a eubacterial minicell, a poroplast, a spheroplast and a protoplast.
132. The minicell according to aspect 131, wherein said exogenous lipid is a derivitized lipid.
133. The minicell according to aspect 132, wherein said derivitized lipid is selected from the group consisting of phosphatidylethanolamine derivatized with PEG, DSPE-PEG, PEG stearate; PEG-derivatized phospholipids, and PEG ceramides is DSPE-PEG.
134. The minicell according to aspect 131, wherein said exogenous lipid is not present in a wild-type membrane, or is present in a different proportion than is found in minicells comprising a wild-type membrane,
135. The minicell according to aspect 134, wherein said exogenous lipid is selected from the group consisting of ganglioside, sphingomyelin, monosialoganglioside GM1, galactocerebroside sulfate, 1, 2-sn-dimyristoylphosphatidylcholine, phosphatidylinositol and cardiolipin.
136. The minicell according to aspect 128, wherein said linking moiety is non-covalently attached to said minicell.
137. The minicell according to aspect 136, wherein one of said linking moiety and said membrane component comprises biotin, and the other comprises avidin or streptavidin.
138. The minicell according to aspect 128, wherein said synthetic linking moiety is a cross-linker.
139. The minicell according to aspect 128, wherein said cross-linker is a bifunctional cross-linker.
140. A method of transferring a membrane protein from a minicell membrane to a biological membrane comprising contacting a minicell to said biological membrane, wherein said minicell membrane comprises said membrane protein, and allowing said mincell and said biological membrane to remain in contact for a period of time sufficient for said transfer to occur.
141. The method according to aspect 140, wherein said minicell is selected from the group consisting of a eubacterial minicell, a poroplast, a spheroplast and a protoplast.
142. The method according to aspect 140, wherein biological membrane is a cytoplasmic membrane or an organellar membrane.
143. The method according to aspect 140, wherein said biological membrane is a membrane selected from the group consisting of a membrane of a pathogen, a membrane of an infected cell and a membrane of a hyperproliferative cell.
144. The method according to aspect 140, wherein said biological membrane is the cytoplasmic membrane of a recipient cell.
145. The method according to aspect 144, wherein said recipient cell is selected from the group consisting of a cultured cell and a cell within an organism.
146. The method according to aspect 140, wherein biological membrane is present on a cell that has been removed from an animal, said contacting occurs in vitro, after which said cell is returned to said organism.
147. The method according to aspect 144, wherein said membrane protein is an enzyme.
148. The method according to aspect 147, wherein said membrane protein having enzymatic activity is selected from the group consisting of a cytochrome P450 and a fusion protein, said fusion protein comprising a first polypeptide, said first polypeptide comprising at least one polypeptide, wherein said second polypeptide has enzymatic acitivity.
149. The method according to aspect 140, wherein said membrane protein is a membrane fusion protein, said membrane fusion protein comprising a first polypeptide, said first polypeptide comprising at least one transmembrane domain or at least one membrane anchoring domain; and a second polypeptide.
150. The method according to aspect 149, wherein said second polypeptide is a biologically active polypeptide.
151. The method according to aspect 140, wherein said minicell displays a binding moiety.
152. A minicell that comprises an expression construct comprising an ORF encoding a membrane protein operably linked to expression sequences, wherein said expression sequences are induced and/or derepressed when said minicell is in contact with a target cell.
153. The minicell according to aspect 152, wherein said minicell is selected from the group consisting of a eubacterial minicell, a poroplast, a spheroplast and a protoplast.
154. The minicell according to aspect 152, wherein biological membrane is a cytoplasmic membrane or an organellar membrane.
155. The minicell according to aspect 152, wherein said biological membrane is a membrane selected from the group consisting of a membrane of a pathogen, a membrane of an infected cell and a membrane of a hyperproliferative cell.
156. The minicell according to aspect 152, wherein said minicell displays a binding moiety.
157. The minicell according to aspect 156, wherein said binding moiety is selected from the group consisting of an antibody, an antibody derivative, an aptamer and a small molecule.
158. The minicell according to aspect 152, wherein said membrane protein is a membrane fusion protein, said membrane fusion protein comprising a first polypeptide, said first polypeptide comprising at least one transmembrane domain or at least one membrane anchoring domain; and a second polypeptide.
159. The minicell according to aspect. 152, wherein said membrane protein having enzymatic activity is selected from the group consisting of a cytochrome P450 and a fusion protein, said fusion protein comprising a first polypeptide, said first polypeptide comprising at least one polypeptide, wherein said second polypeptide has enzymatic activity.
160. A pharmaceutical composition comprising a minicell, wherein said minicell displays a membrane protein, wherein said membrane protein is selected from the group consisting of a eukaryotic membrane protein, an archeabacterial membrane protein and an organellar membrane protein.
161. The pharmaceutical composition according to aspect 160, wherein said minicell is selected from the group consisting of a eubacterial minicell, a poroplast, a spheroplast and a protoplast.
162. The pharmaceutical composition according to aspect 160, wherein said membrane protein is selected from the group consisting of a receptor, a channel protein, a cellular adhesion factor and an integrin.
163. The pharmaceutical formulation according to aspect 162, wherein said pharmaceutical formulation further comprises an adjuvant.
164. The pharmaceutical formulation according to aspect 162, wherein said membrane protein comprises a polypeptide epitope displayed by a hyperproliferative cell.
165. The pharmaceutical formulation according to aspect 162, wherein said membrane protein comprises an epitope displayed by a eukaryotic pathogen, an archeabacterial pathogen, a virus or an infected cell.
166. A pharmaceutical composition comprising a minicell, wherein said minicell displays a membrane protein that is a fusion protein, said fusion protein comprising (i) a first polypeptide, said first polypeptide comprising at least one transmembrane domain or at least one membrane anchoring domain ; and (ii) a second polypeptide, wherein said second polypeptide is not derived from'a eubacterial protein.
167. The pharmaceutical composition according to aspect 166, wherein said minicell is selected from the group consisting of a eubacterial minicell, a poroplast, a spheroplast and a protoplast.
168. The pharmaceutical formulation according to aspect 167, wherein said pharmaceutical formulation further comprises an adjuvant.
169. The pharmaceutical formulation according to aspect 167, wherein said second polypeptide comprises a polypeptide epitope displayed by a hyperproliferative cell.
170. The pharmaceutical formulation according to aspect 169, wherein said membrane protein comprises an epitope displayed by a eukaryotic pathogen, an archeabacterial pathogen, a virus or an infected cell.
171. A pharmaceutical composition comprising a minicell, wherein said minicell displays a membrane conjugate, wherein said membrane conjugate comprises a membrane component chemically linked to a conjugated compound.
172. The pharmaceutical composition according to aspect 171, wherein said minicell is selected from the group consisting of a eubacterial minicell, a poroplast, a spheroplast and a protoplast.
173. The pharmaceutical composition according to aspect 171, wherein said membrane protein is selected from the group consisting of a receptor, a channel protein, a cellular adhesion factor and an integrin.
174. The pharmaceutical composition according to aspect 171, wherein said pharmaceutical further comprises an adjuvant.
175. The pharmaceutical composition according to aspect 171, wherein said membrane component is a polypeptide comprising at least one transmembrane domain or at least one membrane anchoring domain, or a lipid that is part of a membrane.
176. The pharmaceutical composition according to aspect 171, wherein said conjugated compound is a polypeptide, and the chemical linkage between said membrane compound and said conjugated compound is not a peptide bond.
177. The pharmaceutical composition according to aspect 171, wherein said conjugated compound is a nucleic acid.
178. The pharmaceutical composition according to aspect 171, wherein said conjugated compound is an organic compound.
179. The pharmaceutical composition according to aspect 176, wherein said organic compound is selected from the group consisting of a narcotic, a toxin, a venom, a sphingolipid and a soluble protein.
180. A method of making a pharmaceutical composition comprising a minicell, wherein said minicell displays a membrane protein, wherein said membrane protein is selected from the group consisting of a eukaryotic membrane protein, an archeabacterial membrane protein and an organellar membrane protein.
181. The method according to aspect 1, wherein said minicell is selected from the group consisting of a eubacterial minicell, a poroplast, a spheroplast and a protoplast.
182. The method according to aspect 180, wherein said method further comprises adding an adjuvant to said pharmaceutical formulation.
183. The method according to aspect 180, wherein said method further comprises desiccating said formulation.
184. The method according to aspect 183, wherein said method further comprises adding a suspension buffer to said formulation.
185. The method according to aspect 180, wherein said method further comprises making a chemical modification of said membrane protein.
186. The method according to aspect 185, wherein said chemical modification is selected from the group consisting of glycosylation, deglycosylation, phosphorylation, dephosphorylation and proteolysis.
187. A method of making a pharmaceutical composition comprising a minicell, wherein said minicell displays a membrane protein that is a fusion protein, said fusion protein comprising (i) a first polypeptide, said first polypeptide comprising at least one transmembrane domain or at least one membrane anchoring domain; and (ii) a second polypeptide, wherein said second polypeptide is not derived from a eubacterial protein.
188. The method according to aspect 187, wherein said method further comprises adding an adjuvant to said pharmaceutical formulation.
189. The method according to aspect 187, wherein said method further comprises desiccating said pharmaceutical formulation.
190. The method according to aspect 189 wherein said method further comprises adding a suspension buffer to said pharmaceutical formulation.
191. The method according to aspect 187, wherein said method further comprises making a chemical modification of said membrane protein.
192. The method according to aspect 191, wherein said chemical modification is selected from the group consisting of glycosylation, deglycosylation, phosphorylation, dephosphorylation and proteolysis.
193. A method of making a pharmaceutical formulation comprising a minicell, wherein said minicell displays a membrane conjugate, wherein said membrane conjugate comprises a membrane component chemically linked to a conjugated compound.
194. The method according to aspect 193, wherein said method further comprises adding an adjuvant to said pharmaceutical formulation.
195. The method according to aspect 193, wherein said membrane component is a polypeptide comprising at least one transmembrane domain or at least one membrane anchoring domain, or a lipid that is part of a membrane.
196. The method according to aspect 193, wherein said conjugated compound is a polypeptide, and the chemical linkage between said membrane compound and said conjugated compound is not a peptide bond.
197. The method according to aspect 193, wherein said conjugated compound is a nucleic acid.
198. The method according to aspect 193, wherein said conjugated compound is an organic compound.
199. The method according to aspect 186, wherein said organic compound is selected from the group consisting of a narcotic, a toxin, a venom, and a sphingolipid.
200. A method of detecting an agent that is specifically bound by a binding moiety, comprising contacting a minicell displaying said binding moiety with a composition known or suspected to contain said agent, and detecting a signal that is modulated by the binding of said agent to said binding moiety.
201. The method according to aspect 200, wherein said minicell is a eubacterial minicell, a poroplast, a spheroplast or a protoplast.
202. The method according to aspect 200, wherein said agent is associated with a disease.
203. The method according to aspect 200, wherein said minicell comprises a detectable compound.
204. The method according to aspect 200, wherein said binding moiety is antibody or antibody derivative.
205. The method according to aspect 200, wherein said composition is an environmental sample.
206. The method according to aspect 200, wherein said composition is a biological sample.
207. The method according to aspect 206, wherein said biological sample is selected from the group consisting of blood, serum, plasma, urine, saliva, a biopsy sample, feces and a skin patch.
208. A method of in situ imaging of a tissue or organ, comprising administering to an organism a minicell comprising an imaging agent and a binding moiety and detecting said imaging agent in said organism.
209. The method according to aspect 208, wherein said minicell is a eubacterial minicell, a poroplast, a spheroplast or a protoplast.
210. The method according to aspect 208, wherein said binding moiety is an antibody or antibody derivative.
211. The method according to aspect 208, wherein said binding moiety specifically binds a cell surface antigen.
212. The method according to aspect 211, wherein said cell surface antigen is an antigen displayed by a tumorigenic cell, a cancer cell, and an infected cell.
213. The method according to aspect 211, wherein said cell surface antigen is a tissue-specific antigen.
214. The method according to aspect 208, wherein said method of imaging is selected from the group consisting of magnetic resonance imaging, ultrasound imaging; and computer axial tomography (CAT).
215. A device comprising a microchip operatively associated with a biosensor comprising a minicell, wherein said microchip comprises or contacts said minicell, and wherein said minicell displays a binding moiety.
216. The device according to aspect 215, wherein said minicell is a eubacterial minicell, a poroplast, a spheroplast or a protoplast.
217. A method of detecting a substance that is specifically bound by a binding moiety, comprising contacting the device according to aspect 215 with a composition known or suspected to contain said substance, and detecting a signal from said device, wherein said signal changes as a function of the amount of said substance present in said composition.
218. The method according to aspect 217, wherein said composition is a biological sample or an environmental sample.
219. A method of identifying an agent that specifically binds a target compound, comprising contacting a minicell displaying said target compound with a library of compounds, and identifying an agent in said library that binds said target compound.
220. The method according to aspect 219, wherein said minicell is a eubacterial minicell, a poroplast, a spheroplast or a protoplast.
221. The method according to aspect 219, wherein said library of compounds is a protein library.
222. The method according to aspect 221, wherein said protein library is selected from the group consisting of a phage display library, a phagemid display library, a baculovirus library, a yeast display library, and a ribosomal display library.
223. The method according to aspect 219, wherein said library of compounds is selected from the group consisting of a library of aptamers, a library of synthetic peptides and a library of small molecules.
224. The method according to aspect 219, wherein said target compound is a target polypeptide.
225. The method according to aspect 224, wherein said minicell comprises an expression construct comprising expression sequences operably linked to an ORF encoding said target polypeptide.
226. The method according to aspect 224, wherein said target polypeptide is a membrane protein.
227. The method according to aspect 226, wherein said membrane protein is a receptor or a channel protein.
228. The method according to aspect 226, wherein said membrane protein is an enzyme.
229. The method according to aspect 219, wherein said target compound is a membrane fusion protein, said membrane fusion protein comprising a first polypeptide, wherein said first polypeptide comprises at least one transmembrane domain or at least one membrane anchoring domain; and a second polypeptide, wherein said second polypeptide comprises amino acid sequences derived from a target polypeptide.
230. The method according to aspect 219, wherein said method further comprises comparing the activity of said target compound in the presence of said agent to the activity of said target compound in the absence of said agent.
231. The method according to aspect 230, wherein said activity of said target compound is an enzyme activity.
232. The method according to aspect 231, wherein said enzyme is selected from the group consisting of an oxidoreductase, a transferase, a hydrolase, a lyase, an isomerase, a ligase and a synthetase.
233. The method according to aspect 230, wherein said activity of said target compound is a binding activity.
234. The method according to aspect 233, further comprising comparing the binding of said agent to said target compound to the binding of a known ligand of said target compound.
235. The method according to aspect 234, wherein a competition assay is used for said comparing.
236. A device comprising microchips operatively associated with a biosensor comprising a set of minicells in a prearranged pattern, wherein said each of said microchips comprise or contact a minicell, wherein each of said minicell displays a different target compound, and wherein binding of a ligand to a target compound results in an increased or decreased signal.
237. A method of identifying an agent that specifically binds a target compound, comprising contacting the device according to aspect 236 with a library of compounds, and detecting a signal from said device, wherein said signal changes as a function of the binding of an agent to said target compound.
238. A method of identifying an agent that specifically blocks the binding of a target compound to its ligand, comprising contacting the device according to aspect 236 with a library of compounds, and detecting a signal from said device, wherein said signal changes as a function of the binding of an agent to said target compound.
239. A method of making a antibody that specifically binds a protein domain, wherein said domain is in its native conformation, wherein said domain is contained within a protein displayed on a minicell, comprising contacting said minicell with a cell, wherein said cell is competent for producing antibodies to an antigen contacted with said cell, in order to generate an immunogenic response in which said cell produces said antibody.
240. The method according to aspect 239, wherein said minicell is a eubacterial minicell, a poroplast, a spheroplast or a protoplast.
241. The method according to aspect 239, wherein said protein displayed on a minicell is a membrane protein.
242. The method according to aspect 241, wherein said membrane protein is a receptor or a channel protein.
243. The method according to aspect 239, wherein said domain is found within the second polypeptide of a membrane fusion protein, wherein said membrane, fusion protein comprises a first polypeptide, wherein said first polypeptide comprises at least one transmembrane domain or at least one membrane anchoring domain.
244. The method according to aspect 239, wherein said contacting occurs in vivo.
245. The method according to aspect 244, wherein said antibody is a polyclonal antibody or a monoclonal antibody.
246. The method according to aspect 244, wherein said contacting occurs in an animal that comprises an adjuvant.
247. The method of making an antibody derivative that specifically binds a protein domain, wherein said domain is in its native conformation, wherein said domain is displayed on a minicell, comprising contacting said minicell with a protein library, and identifying an antibody derivative from said protein library that specifically binds said protein domain.
248. The method according to aspect 247, wherein said protein library is selected from the group consisting of a phage display library, a phagemid display library, and a ribosomal display library.
249. The method according to aspect 247 wherein said antibody derivative is a single-chain antibody.
250. A method of making an antibody or antibody derivative that specifically binds an epitope, wherein said epitope is selected from the group consisting of (i) an epitope composed of amino acids found within a membrane protein, (ii) an epitope present in an interface between a membrane protein and a membrane component, (iii) an epitope present in an interface between a membrane protein and one or more other proteins and (iv) an epitope in a fusion protein, said fusion protein comprising a first polypeptide, said first polypeptide comprising at least one transmembrane domain or at least one membrane anchoring domain, and a second polypeptide, said second polypeptide comprising said epitope; comprising contacting a minicell displaying said epitope with a protein library, or to a cell, wherein said cell is competent for producing antibodies to an antigen contacted with said cell, in order to generate an immunogenic response in which said cell produces said antibody.
251. The method according to aspect 250, wherein said minicell is a eubacterial minicell, a poroplast, a spheroplast or a protoplast.
252. The method according to aspect 250, wherein said cell is contacted in vivo.
253. The method according to aspect 252, wherein said antibody is a polyclonal antibody.
254. The method according to aspect 252, wherein said antibody is a monoclonal antibody.
255. The method according to aspect 250, wherein said protein library is contacted in vitro.
256. The method according to aspect 255, wherein said protein library is selected from the group consisting of a phage display library, a phagemid display library, and a ribosomal display library.
257. The method according to aspect 256, wherein said antibody derivative is a single-chain antibody.
258. A method of determining the rate of transfer of nucleic acid from a minicell to a cell, comprising (a) contacting said cell to said minicell, wherein said minicell comprises said nucleic acid, for a set period of time; (b) separating minicells from said cells; (c) measuring the amount of nucleic acid in said cells, wherein the amount of nucleic acid in said cells over said set period of time is the rate of transfer of a nucleic acid from a minicell.
259. A method of determining the amount of a nucleic acid transferred to a cell from a minicell, comprising (a) contacting said cell to said minicell, wherein said minicell comprises an expression element having eukaryotic expression sequences operably linked to an ORF encoding a detectable polypeptide, wherein said minicell displays a binding moiety, and wherein said binding moiety binds an epitope of said cell; and (b) detecting a signal from said detectable polypeptide, wherein a change in said signal corresponds to an increase in the amount of a nucleic acid transferred to a cell.
260. The method according to aspect 258, wherein said minicell is selected from the group consisting of a eubacterial minicell, a poroplast, a spheroplast and a protoplast.
261. The method according to aspect 258, wherein said cell is a eukaryotic cell.
262. The method according to aspect 258, wherein said binding moiety is an antibody or antibody derivative.
263. The method according to aspect 258, wherein said binding moiety is a single-chain antibody.
264. The method according to aspect 258, wherein said binding moiety is an aptamer.
265. The method according to aspect 258, wherein said binding moiety is an organic compound.
266. The method according to aspect 258, wherein said detectable polypeptide is a fluorescent polypeptide.
267. A method of detecting the expression of an expression element in a cell, comprising (a) contacting said cell to a minicell, wherein said minicell comprises an expression element having cellular expression sequences operably linked to an ORF encoding a detectable polypeptide, wherein said minicell displays a binding moiety, and wherein said binding moiety binds an epitope of said cell; (b) incubating said cell and said minicell for a period of time effective for transfer of nucleic acid from said minicell to said cell; and (c) detecting a signal from said detectable polypeptide, wherein an increase in said signal corresponds to an increase in the expression of said expression element.
268. The method according to aspect 267, wherein said minicell is selected from the group consisting of a eubacterial minicell, a poroplast, a spheroplast and a protoplast.
269. The method according to aspect 267, wherein said cell is a eukaryotic cell and said expression sequences are eukaryotic expression sequences.
270. The method according to aspect 269, wherein said eukaryotic cell is a mammalian cell.
271. The method according to aspect267, wherein said binding moiety is an antibody or antibody derivative.
272. The method according to aspect 267, wherein said binding moiety is a single-chain antibody.
273. The method according to aspect 267, wherein said binding moiety is an aptamer.
274. The method according to aspect 267, wherein said binding moiety is an organic compound.
275. The method according to aspect 267, wherein said detectable polypeptide is a fluorescent polypeptide.
276. A method for detecting the transfer of a fusion protein from the cytosol to an organelle of a eukaryotic cell, comprising (a) contacting said cell to a minicell, wherein (i) said minicell comprises an expression element having eukaryotic expression sequences operably linked to an ORF encoding a fusion protein, wherein said fusion protein comprises a first polypeptide that comprises organellar delivery sequences, and a second polypeptide that comprises a detectable polypeptide; and (ii) said minicell displays a binding moiety that binds an epitope of said cell, or an epitope of an organelle; (b) incubating said cell and said minicell for a period of time effective for transfer of nucleic acid from said minicell to said cell and production of said fusion protein ; and (c) detecting a signal from the detectable polypeptide, wherein a change in the signal corresponds to an increase in the amount of the fusion protein transferred to said organelle.
277. The method according to aspect 276, wherein said organelle is a mitochondrion, a chloroplast or a kinetoplast.
278. A minicell comprising at least one nucleic acid, wherein said minicell displays a binding moiety directed to a target compound, wherein said binding moiety is selected from the group consisting of (i) a eukaryotic membrane protein; (ii) an archeabacterial membrane protein; (iii) an organellar membrane protein; and (iv) a fusion protein, said fusion protein comprising a first polypeptide, said first polypeptide comprising at least one transmembrane domain or at least one membrane anchoring domain; and a second polypeptide, wherein said second polypeptide is not derived from a eubacterial protein and is neither a His tag nor a glutathione-S- transferase polypeptide, and wherein said polypeptide comprises a binding moiety.
279. The minicell according to aspect 278, wherein said minicell is selected from the group consisting of a eubacterial minicell, a poroplast, a spheroplast and a protoplast.
280. The minicell according to aspect 278, wherein said nucleic acid comprises an expression construct comprising expression sequences operably linked to an ORF encoding a protein selected from the group consisting of (i) said eukaryotic membrane protein, (ii) said archeabacterial membrane protein, (iii) said organellar membrane protein; and (iv) said fusion protein.
281. The minicell according to aspect 280, wherein said nucleic acid comprises an expression construct comprising expression sequences operably linked to an ORF, wherein said
   ORF encodes a therapeutic polypeptide.
282. The minicell according to aspect 281, wherein said therapeutic polypeptide is a membrane polypeptide.
283. The minicell according to aspect 281, wherein said therapeutic polypeptide is a soluble polypeptide.
284. The minicell according to aspect 283, wherein said soluble polypeptide comprises a cellular secretion sequence.
285. The minicell according to aspect 281, wherein said expression sequences are inducible and/or repressible.
286. The minicell according to aspect2858, wherein said expression sequences are induced and/or derepressed when the binding moiety displayed by said minicell binds to its target compound.
287. The minicell according to aspect 1278herein said nucleic acid comprises an expression construct comprising expression sequences operably linked to an ORF, wherein said ORF encodes a polypeptide having an amino acid sequence that facilitates cellular transfer of a biologically active compound contained within or displayed by said minicell.
288. The minicell according to aspect 278 wherein the membrane of said minicell comprises a system for transferring a molecule from the interior of a minicell into the cytoplasm of said cell.
289. The minicell according to aspect 288 wherein said system for transferring a molecule from the interior of a minicell into the cytoplasm of said cell is a Type III secretion system.
290. A method of introducing a nucleic acid into a cell, comprising contacting said cell with a minicell that comprises said nucleic acid, wherein said minicell displays a binding moiety, wherein said binding moiety is selected from the group consisting of (i) a eukaryotic membrane protein; (ii) an archeabacterial membrane protein; (iii) an organellar membrane protein; and (iv) a fusion protein, said fusion protein comprising a first polypeptide, said first polypeptide comprising at least one transmembrane domain or at least one membrane anchoring domain; and a second polypeptide, wherein said second polypeptide is not derived from a eubacterial protein and is neither a His tag nor a glutathione-S-transferase polypeptide, and wherein said polypeptide comprises a binding moiety; and wherein said binding moiety binds an epitope of said cell.
291. The method according to aspect 290, wherein said minicell is selected from the group consisting of a eubacterial minicell, a poroplast, a spheroplast and a protoplast.
292. The method according to aspect 290, wherein said nucleic acid comprises an expression construct comprising expression sequences operably linked to an ORF encoding a protein selected from the group consisting of (i) said eukaryotic membrane protein, (ii) said archeabacterial membrane protein, (iii) said organellar membrane protein; and (iv) said fusion protein.
293. The method according to aspect 290, wherein said nucleic acid comprises an expression construct comprising expression sequences operably linked to an ORF, wherein said
   ORF encodes a therapeutic polypeptide.
294. The method according to aspect 293, wherein said expression sequences are inducible and/or derepressible.
295. The method according to aspect 294, wherein said expression sequences are induced or derepressed when the binding moiety displayed by said minicell binds its target compound.
296. The method according to aspect 294, wherein said expression sequences are induced or derepressed by a transactivation or transrepression event.
297. The method according to aspect 292, wherein said nucleic acid comprises an expression construct comprising expression sequences operably linked to an ORF, wherein said ORF encodes a polypeptide having an amino acid sequence that facilitates cellular transfer of a biologically active compound contained within or displayed by said minicell.
298. A minicell comprising a nucleic acid, wherein said nucleic acid comprises eukaryotic expression sequences and eubacterial expression sequences, each of which is independently operably linked to an ORF.
299. The minicell according to aspect 298, wherein said minicell is selected from the group consisting of a eubacterial minicell, a poroplast, a spheroplast and a protoplast.
300. The minicell according to aspect 298, wherein said minicell displays a binding moiety.
301. The minicell according to aspect 300, wherein said eubacterial expression sequences are induced and/or derepressed when said binding moiety is in contact with a target cell.
302. The minicell according to aspect 300, wherein said eukaryotic expression sequences are induced and/or derepressed when said nucleic acid is in the cytoplasm of a eukaryotic cell.
303. The minicell according to aspect 301, wherein the protein encoded by said ORF comprises eubacterial or eukaryotic secretion sequences.
304. A minicell comprising a first and second nucleic acid, wherein said first nucleic acid comprises eukaryotic expression sequences operably linked to a first ORF, and a second nucleic acid, wherein said second nucleic acid comprises eubacterial expression sequences operably linked to a second ORF.
305. The minicell according to aspect 304, wherein said minicell is selected from the group consisting of a eubacterial minicell, a poroplast, a spheroplast and a protoplast.
306. The minicell according to aspect 304, wherein said minicell displays a binding moiety.
307. The minicell according to aspect 306, wherein said eubacterial expression sequences are induced and/or derepressed when said binding moiety is in contact with a target cell.
308. The minicell according to aspect 306, wherein said eukaryotic expression sequences are induced and/or derepressed when said nucleic acid is in the cytoplasm of a eukaryotic cell.
309. The minicell according to aspect 304, wherein the protein encoded by said first ORF comprises eukaryotic secretion sequences and/or the protein encoded by said second ORF comprises eubacterial secretion sequences.
310. A method of introducing into and expressing a nucleic acid in an organism, comprising contacting a minicell to a cell of said organism, wherein said minicell comprises said nucleic acid.
311. The method according to aspect 310, wherein said minicell is selected from the group consisting of a eubacterial minicell, a poroplast, a spheroplast and a protoplast.
312. The method according to aspect 310, wherein said minicell displays a binding moiety.
313. The method according to aspect 310, wherein said nucleic acid comprises a eukaryotic expression construct, wherein said eukaryotic expression construct comprises eukaryotic expression sequences operably linked to an ORF.
314. The method according to aspect 310, wherein said ORF encodes a protein selected from the group consisting of a membrane protein, a soluble protein and a protein comprising eukaryotic secretion signal sequences.
315. The method according to aspect 310, wherein said nucleic acid comprises a eubacterial expression construct, wherein said eubacterial expression construct comprises eubacterial expression sequences operably linked to an ORF.
316. The method according to aspect 315, wherein said minicell displays a binding moiety, wherein said eubacterial expression sequences are induced and/or derepressed when said binding moiety is in contact with a target cell.
317. The method according to aspect 316, wherein the protein encoded by said ORF comprises eubacterial secretion sequences.
318. A minicell comprising a crystal of a membrane protein.
319. The minicell according to aspect 318, wherein said minicell is a eubacterial minicell, a poroplast, a spheroplast or a protoplast.
320. The minicell according to aspect 318, wherein said membrane protein is a receptor.
321. The minicell according to aspect 320, wherein said receptor is a G-protein coupled receptor.
322. The minicell according to aspect 318, wherein said crystal is displayed.
323. The minicell according to aspect 318, wherein said membrane protein is a fusion protein, said fusion protein comprising a first polypeptide, said first polypeptide comprising at least one transmembrane domain or at least one membrane anchoring domain, and a second polypeptide.
324. The minicell according to aspect 323, wherein said crystal is a crystal of said second polypeptide.
325. The minicell according to aspect 323, wherein said crystal is displayed.
326. A method of determining the three-dimensional structure of a membrane protein, comprising preparing a crystal of said membrane protein in a minicell, and determining the three-dimensional structure of said crystal.
327. A method for identifying ligand-interacting atoms in a defined three-dimensional structure of a target protein, comprising (a) preparing one or more variant proteins of a target protein having a known or predicted three-dimensional structure, wherein said target protein binds a preselected ligand; (b) expressing and displaying a variant protein in a minicell; and (c) determining if a minicell displaying said variant protein binds said preselected ligand with increased or decreased affinity as compared to the binding of said preselected ligand to said target protein.
328. The method according to aspect 327, wherein said ligand is a protein that forms a multimer with said target protein, and said ligand interacting atoms are atoms in said-defined three- dimensional structure are atoms that are involved in protein-protein interactions.
329. The method according to aspect 327, wherein said ligand is a compound that induces a conformational change in said target protein, and said defined three-dimensional structure is the site of said conformational change.
330. The method according to aspect 327, adopted to a method, said method for identifying ligands of a target protein, further comprising identifying the chemical differences in said variant proteins as compared to said target protein.
331. The method according to aspect 330, further comprising mapping said chemical differences onto said defined three-dimensional structure, and correlating the effect of said chemical differences on said defined three-dimensional structure.
332. The method according to aspect 331, wherein said target protein is a wild-type protein.
333. A minicell library, comprising two or more minicells, wherein each minicell comprises a different exogenous protein.
334. The minicell library according to aspect 333, wherein said minicell is a eubacterial minicell, a poroplast, a spheroplast or a protoplast.
335. The minicell library according to aspect 333, wherein said exogenous protein is a displayed protein.
336. The minicell library according to aspect 333, wherein said exogenous protein is a membrane protein.
337. The minicell library according to aspect 336, wherein said membrane protein is a receptor.
338. The minicell library according to aspect 333, wherein said protein is a soluble protein that is contained within or secreted from said minicell.
339. The minicell library according to aspect 333, wherein minicells within said library comprise an expression element that comprises expression sequences operably linked to a nucleic acid having an ORF that encodes said exogenous protein.
340. The minicell library according to aspect 339, wherein said nucleic acid has been mutagenized.
341. The minicell library according to aspect 339, wherein an active site of said exogenous protein has a known or predicted three-dimensional structure, and said a portion of said ORF encoding said active site has been mutagenized.
342. The minicell library according to aspect 333, wherein each of said minicells comprises an exogenous protein that is a variant of a protein having a known or predicted three- dimensional structure.
343. A minicell library, comprising two or more minicells, wherein each minicell comprises a different fusion protein, each of said fusion protein comprising a first polypeptide that is a constant polypeptide, wherein said constant polypeptide comprises at least one transmembrane domain or at least one membrane anchoring domain, and a second polypeptide, wherein said second polypeptide is a variable amino acid sequence that is different in each fusion proteins.
344. The minicell library according to aspect 343, wherein minicells within said library comprise an expression element that comprises expression sequences operably linked to a nucleic acid having an ORF that encodes said fusion protein.
345. The minicell library according to aspect 344, wherein said second polypeptide of said fusion protein is encoded by a nucleic acid that has been cloned.
346. The minicell library according to aspect 344, wherein each of said second polypeptide of each of said fusion proteins comprises a variant of an amino acid sequence from a protein having a known or predicted three-dimensional structure.
347. A minicell library, comprising two or more minicells, wherein each minicell comprises a constant protein that is present in each minicell and a variable protein that differs from minicell to minicell.
348. The minicell library according to aspect 347, wherein one of said constant and variable proteins is a receptor, and the other of said constant and variable proteins is a co-receptor.
349. The minicell library according to aspect 347, wherein each of said constant and variable proteins is different from each other and is a factor in a signal transduction pathway.
350. The minicell library according to aspect 347, wherein one of said constant and variable proteins is a G-protein, and the other of said constant and variable proteins is a G-protein coupled receptor.
351. The minicell library according to aspect 347, wherein one of said constant and variable proteins comprises a first transrepression domain, and the other of said constant and variable comprises a second transrepression domain, wherein said transrepression domains limit or block expression of a reporter gene when said constant and variable proteins associate with each other.
352. The minicell library according to aspect 347, wherein one of said constant and variable proteins comprises a first transactivation domain, and the other of said constant and variable comprises a second transactivation domain, wherein said transactivation domains stimulate expression of a reporter gene when said constant and variable proteins associate with each other.
353. A method of identifying a nucleic acid that encodes a protein that binds to or chemically alters a preselected ligand, comprising: (a) separately contacting said ligand with individual members of a minicell library, wherein minicells in said library comprise expression elements, wherein said expression elements comprise DNA inserts, wherein an ORF in said DNA insert is operably linked to expression sequences, in order to generate a series of reaction mixes, each reaction mix comprising a different member of said minicell library; (b) incubating said reaction mixes, thereby allowing a protein that binds to or chemically alters said preselected ligand to bind or chemically alter said preselected ligand; (c) detecting a change in a signal from reaction mixes in which said ligand has been bound or chemically altered; (d) preparing DNA from reaction mixes in which said ligand has been bound or chemically altered; wherein said DNA is a nucleic acid that encodes a protein that binds to or chemically alters said preselected ligand.
354. The method according to aspect 353, wherein said minicell is a eubacterial minicell, a poroplast, a spheroplast or a protoplast.
355. The method according to aspect 353, wherein said preselected ligand is a biologically active compound.
356. The method according to aspect 353, wherein said preselected ligand is a therapeutic drug.
357. The method according to aspect 353, wherein a protein that binds or chemically alters said preselected ligand is a target protein for compounds that are therapeutic for a disease that is treated by administering said drug to an organism in need thereof.
358. The method according to aspect 353, wherein said preselected ligand is detectably labeled, said mincell comprises a detectable compound, and/or a chemically altered derivative of said protein is detectably labeled.
359. A method of determining the amino acid sequence of a protein that binds or chemically alters a preselected ligand, comprising:
   (a) contacting said ligand with a minicell library, wherein minicells in said library comprise expression elements, wherein said expression elements comprise DNA inserts, wherein an ORF in said DNA insert is operably linked to expression sequences; (b) incubating said mixture of ligand and minicells, under conditions which allow complexes comprising ligands and minicells to form and/or chemical reactions to occur; (c) isolating or identifying said complexes from said ligand and said mixture of ligand and minicells; (d) preparing DNA from an expression element found in one or more of said complexes, or in a minicell thereof; (e) determining the nucleotide sequence of said ORF in said DNA; and (f) generating an amino sequence by in silico translation, wherein said amino acid sequence is or is derived from a protein that binds or chemically alters a preselected ligand.
360. The method according to aspect 359, wherein said minicell is a eubacterial minicell, a poroplast, a spheroplast or a protoplast.
361. The method according to aspect 359, wherein said DNA is prepared by isolating DNA from said complexes, or in a minicell thereof.
362. The method according to aspect 359, wherein said DNA is prepared by amplifying DNA from said complexes, or in a minicell thereof.
363. The method according to aspect 359, wherein said protein is a fusion protein.
364. The method according to aspect 359, wherein said protein is a membrane or a soluble protein.
365. The method according to aspect 364, wherein said protein comprises secretion sequences.
366. The method according to aspect 359, wherein said preselected ligand is a biologically active compound.
367. The method according to aspect 359, wherein said preselected ligand is a therapeutic drug.
368. The method according to aspect 359, wherein said preselected ligand is a therapeutic drug, and said protein that binds said preselected ligand is a target protein for compounds that are therapeutic for a disease that is treated by administering said drug to an organism in need thereof.
369. A method of identifying a nucleic acid that encodes a protein that inhibits or blocks an agent from binding to or chemically altering a preselected ligand, comprising: (a) separately contacting said ligand with individual members of a minicell library, wherein minicells in said library comprise expression elements, wherein said expression elements comprise DNA inserts, wherein an ORF in said DNA insert is operably linked to expression sequences, in order to generate a series of reaction mixes, each reaction mix comprising a different member of said minicell library; (b) incubating said reaction mixes, thereby allowing a protein that binds to or chemically alters said preselected ligand to bind or chemically alter said preselected ligand; (c) detecting a change in a signal from reaction mixes in which said ligand has been bound or chemically altered; (d) preparing DNA from reaction mixes in which said change in signal ligand has been bound or chemically altered; wherein said DNA is a nucleic acid that encodes a protein that inhibits or blocks said agent from binding to or chemically altering said preselected ligand
370. The method according to aspect 369, wherein said minicell is a eubacterial minicell, a poroplast, a spheroplast or a protoplast.
371. The method according to aspect 369, wherein said DNA has a nucleotide sequence that encodes the amino acid sequence of said protein that inhibits or blocks said agent from binding to or chemically altering said preselected ligand.
372. The method according to aspect 369, wherein a protein that binds or chemically alters said preselected ligand is a target protein for compounds that are therapeutic for a disease that is treated by administering said drug to an organism in need thereof.
373. A method of identifying an agent that effects the activity of a protein, comprising contacting a library of two or more candidate agents with a minicell comprising said protein or a polypeptide derived from said protein, assaying the effect of candidate agents on the activity of said protein, and identifying agents that effect the activity of said protein.
374. The method according to aspect 373, wherein said minicell is selected from the group consisting of a eubacterial minicell, a poroplast, a spheroplast and a protoplast.
375. The method according to aspect 373, wherein said protein or said polypeptide derived from said protein is displayed on the surface of said minicell.
376. The method according to aspect 373, wherein said protein is a membrane protein.
377. The method according to aspect 376, wherein said membrane protein is selected from the group consisting of a receptor, a channel protein and an enzyme.
378. The method according to aspect 373, wherein said activity of a protein is a binding activity or an enzymatic activity.
379. The method according to aspect 373, wherein said library of compounds is a protein library.
380. The method according to aspect 379, wherein said protein library is selected from the group consisting of a phage display library, a phagemid display library, and a ribosomal display library.
381. The method according to aspect 373, wherein said library of compounds is a library of aptamers.
382. The method according to aspect 373, wherein said library of compounds is a library of small molecules.
383. A method of identifying an agent that effects the activity of a protein domain containing a library of two or more candidate agents with a minicell displaying a membrane fusion protein, said fusion protein comprising a first polypeptide, said first polypeptide comprising at least one transmembrane domain or at least one membrane anchoring domain, and a second polypeptide, wherein said second polypeptide comprises said protein domain.
384. A method of identifying undesirable side-effects of a biologically active compound that occur as a result of binding of said compound to a protein, wherein binding a compound to said protein is known to result in undesirable side effects, comprising contacting a minicell that comprises said protein to said biologically active compound.
385. The method according to aspect 384, wherein said minicell is selected from the group consisting of a eubacterial minicell, a poroplast, a spheroplast and a protoplast.
386. The method according to aspect 384, further comprising characterizing the binding of said biologically active compound to said protein.
387. The method according to aspect 384, further comprising characterizing the effect of said biologically active compound on the activity of said protein.
388. A method for identifying an agent that effects the interaction of a first signaling protein with a second signaling protein, comprising (a) contacting a library of compounds with a minicell, wherein said minicell comprises: (i) a first protein comprising said first signaling protein and a first trans- acting regulatory domain; (ii) a second protein comprising said second signaling protein and a second trans-acting regulatory domain ; and (iii) a reporter gene, the expression of which is modulated by the interaction between said first trans-acting regulatory domain and said second trans-acting regulatory domain; and (b) detecting the gene product of said reporter gene.
389. The method according to aspect 388, wherein said minicell is selected from the group consisting of a eubacterial minicell, a poroplast, a spheroplast and a protoplast.
390. The method according to aspect 388, wherein said trans-acting regulatory domains are transactivation domains.
391. The method according to aspect 388, wherein said trans-acting regulatory domains are transrepression domains.
392. The method according to aspect 388, wherein said reporter gene is induced by the interaction of said first trans-acting regulatory domain and said second trans-acting regulatory domain.
393. The method according to aspect 388, wherein said agent that effects the interaction of said first signalling protein with said second signalling protein is an agent that causes or promotes said interaction.
394. The method according to aspect 388, wherein said reporter gene is repressed by the interaction of said first trans-acting regulatory domain and said second trans-acting regulatory domain.
395. The method according to aspect 394, wherein said agent that effects the interaction of said first signaling protein with said second signaling protein is an agent that inhibits or blocks said interaction.
396. The method according to aspect 388, wherein said first signaling protein is a GPCR.
397. The method according to aspect 396, wherein said GPCR is an Edg receptor or a ScAMPER.
398. The method according to aspect 396, wherein said second signalling protein is a G-protein..
399. The method according to aspect 398, wherein said G-protein is selected from the group consisting of G-alpha-i, G-alpha-s, G-alpha-q, G-alpha-12/13 and Go.
400. The method according to aspect 388, wherein said library of compounds is a protein library.
401. The method according to aspect 400, wherein said protein library is selected from the group consisting of a phage display library, a phagemid display library, and a ribosomal display library.
402. The method according to aspect 388, wherein said library of compounds is a library of aptamers.
403. The method according to aspect 388, wherein said library of compounds is a library of small molecules.
404. A method for identifying an agent that effects the interaction of a first signaling protein with a second signaling protein, comprising contacting a library of two or more candidate agents with a minicell, wherein said minicell comprises:
   (a) a first fusion protein comprising said first signaling protein and a first detectable domain; and (b) a second fusion protein comprising said second signaling protein and a second detectable domain, wherein a signal is generated when said first and second signaling proteins are in close proximity to each other, and detecting said signal.
405. The method according to aspect 404, wherein said signal is fluorescence.
406. The method according to aspect 404, wherein said first detectable domain and said second detectable domain are fluorescent and said signal is generated by FRET.
407. The method according to aspect 406, wherein said first and second detectable domains are independently selected from the group consisting of a green fluorescent protein, a blue-shifted green fluorescent protein, a cyan-shifted green fluorescent protein; a red- shifted green fluorescent protein; a yellow-shifted green fluorescent protein, and a red fluorescent protein, wherein said first fluorescent domain and said second fluorescent domain are not identical.
408. A method of bioremediation, said method comprising contacting a composition that comprises an undesirable substance with a minicell, wherein said minicell alters the chemical structure and/or binds said undesirable substance.
409. A method of bioremediation, said method comprising contacting a composition that comprises an undesirable substance with a minicell, wherein said mincell comprises an agent that alters the chemical structure of said undesirable substance.
410. The method according to aspect 409, wherein said agent that alters the chemical structure of said undesirable substance is an inorganic catalyst.
411. The method according to aspect 409, wherein said agent that alters the chemical structure of said undesirable substance is an enzyme.
412. The method according to aspect 411, wherein said enzyme is a soluble protein contained within said minicell.
413. The method according to aspect 412, wherein said soluble protein is selected from the group consisting of an oxidoreductase, a transferase, a hydrolase, a lyase, an isomerase, a ligase and a synthetase.
414. The method according to aspect 411, wherein said enzyme is a secreted protein.
415. The method according to aspect 414, wherein said secreted protein is selected from the group consisting of an oxidoreductase, a transferase, a hydrolase, a lyase, an isomerase, a ligase and a synthetase.
416. The method according to aspect 411, wherein said enzyme is a membrane protein.
417. The method according to aspect 416, wherein said membrane enzyme is selected from the group consisting of a cytochrome P450, an oxidoreductase, a transferase, a hydrolase, a lyase, an isomerase, a ligase and a synthetase.
418. The method according to aspect 409, wherein said agent that alters the chemical structure of said undesirable substance is a fusion protein comprising a first polypeptide that comprises a transmembrane domain or at least one membrane-anchoring domain, and a second polypeptide, wherein said second polypeptide is an enzyme moiety.
419. The method according to aspect 418, wherein said second polypeptide is a polypeptide derived from a protein selected from the group consisting of an oxidoreductase, a transferase, a hydrolase, a lyase, an isomerase, a ligase and a synthetase.
420. A method of bioremediation, said method comprising contacting a composition that comprises an undesirable, substance with a minicell, wherein said mincell comprises an agent that binds an undesirable substance.
421. The method according to aspect 420, wherein said undesirable substance binds to and is internalized by said minicell or is otherwise inactivated by selective absorption.
422. The method according to aspect 420, wherein said agent that binds said undesirable substance is a secreted soluble protein.
423. The method according to aspect 422, wherein said secreted protein is a transport accessory protein.
424. The method according to aspect 420, wherein said agent that binds said undesirable substance is a membrane protein.
425. The method according to aspect 420, wherein said undesirable substance is selected from the group consisting of a toxin, a pollutant and a pathogen.
426. The method according to aspect 420, wherein said agent that binds said undesirable substance is a fusion protein comprising a first polypeptide that comprises a transmembrane domain or at least one membrane-anchoring domain, and a second polypeptide, wherein said second polypeptide is a binding moiety.
427. The method according to aspect 426, wherein said binding moiety is selected from the group consisting of an antibody, an antibody derivative, the active site of a non- enzymatically active mutant enzyme, a single-chain antibody and an aptamer.
428. A minicell-producing parent cell, wherein said parent cell comprises one or more of the following : (a) an expression element that comprises a gene operably linked to expression sequences that are inducible and/or repressible, wherein induction or repression of said gene regulates the copy number of an episomal expression construct; (b) a mutation in an endogenous gene, wherein said mutation regulates the copy number of an episomal expression construct; (c) an expression element that comprises a gene operably linked to expression sequences that are inducible and/or repressible, wherein induction or repression of said gene causes or enhances the production of minicells; and (d) a mutation in an endogenous gene, wherein said mutation causes or enhances minicell production.
429. The minicell-producing parent cell according to aspect 428, further comprising an episomal expression construct.
430. The minicell-producing parent cell according to aspect 428, further comprising a chromosomal expression construct.
431. The minicell-producing parent cell according to aspect 429, wherein said expression sequences of said expression construct are inducible and/or repressible.
432. The minicell-producing parent cell according to aspect 428, wherein said minicell-producing parent cell comprises a biologically active compound.
433. The minicell according to aspect 428 wherein said gene that causes or enhances the production of minicells has a gene product that is involved in or regulates DNA replication, cellular division, cellular partitioning, septation, transcription, translation, or protein folding.
434. A minicell-producing parent cell, wherein said parent cell comprises an expression construct, wherein said expression construct comprises expression sequences operably linked to an ORF that encodes a protein, and a regulatory expression element, wherein said regulatory expression element comprises expression sequences operably linked to a regulatory gene that encodes a factor that regulates the expression of said
   ORF.
435. The minicell-producing parent cell according to aspect 434, wherein said expression sequences of said expression construct are inducible and/or repressible.
436. The minicell-producing parent cell according to aspect 434, wherein said expression sequences of said regulatory expression construct are inducible and/or repressible.
437. The minicell-producing parent cell according to aspect 434, wherein one or more of said expression element or said regulatory expression element is located on a chromosome of said parent cell.
438. The minicell-producing parent cell according to aspect 434, wherein one or more of said expression element or said regulatory expression element is located on an episomal expression construct.
439. The minicell-producing parent cell according to aspect 438, wherein both of said expression element and said regulatory expression element are located on an episomal expression construct, and one or both of said expression element and said regulatory expression element segregates into minicells produced from said parent cell.
440. The minicell-producing parent cell according to aspect 434, wherein said minicell-producing parent cell comprises a biologically active compound.
441. The minicell-producing parent cell according to aspect 440, wherein said biologically active compound segregates into minicells produced from said parent cell.
442. The minicell-producing parent cell according to aspect 434, wherein said ORF encodes a membrane protein or a soluble protein.
443. The minicell-producing parent cell according to aspect 434, wherein said protein comprises secretion sequences.
444. The minicell-producing parent cell according to aspect 434, wherein the gene product of said gene regulates the expression of said ORF.
445. The minicell-producing parent cell according to aspect 444, wherein said gene product is a transcription factor.
446. The minicell-producing parent cell according to aspect 440, wherein said gene product is a RNA polymerase.
447. The minicell-producing parent cell according to aspect 446, wherein said parent cell is MC-T7.
448. A minicell comprising a biologically active compound, wherein said minicell displays a binding moiety, wherein said minicell selectively absorbs and/or internalizes an undesirable compound, and said minicell is a poroplast, spheroplast or protoplast.
449. The minicell according to aspect 448, wherein said binding moiety is selected from the group consisting of an antibody, an antibody derivative, a receptor and an active site of a non-catalytic derivative of an enzyme.
450. The minicell according to aspect 458, wherein said binding moiety is a single-chain antibody.
451. The minicell according to aspect 458, wherein said binding moiety is directed to a ligand selected from the group consisting of an epitope displayed on a pathogen, an epitope displayed on an infected cell and an epitope displayed on a hyperproliferative cell.
452. The minicell according to aspect 458, wherein said biologically active compound is selected from the group consisting of a radioisotope, a polypeptide, a nucleic acid and a small molecule.
453. The minicell according to aspect 448, wherein a ligand binds to and is internalized by said minicell or is otherwise inactivated by selective absorption.
454. A pharmaceutical composition comprising the minicell according to aspect 448.
455. A method of reducing the free concentration of a substance in a composition, wherein said substance displays a ligand specifically recognized by a binding moiety, comprising contacting said composition with a minicell that displays said binding moiety, wherein said binding moiety binds said substance, thereby reducing the free concentration of said substance in said composition.
456. The method according to aspect 455, wherein said minicell is selected from the group consisting of a eubacterial minicell, a poroplast, a spheroplast and a protoplast.
457. The method according to aspect 455, wherein said substance is selected from the group consisting of a nucleic acid, a lipid, a polypeptide, a radioactive compound, an ion and a small molecule.
458. The method according to aspect 455, wherein said binding moiety is selected from the group consisting of an antibody, an antibody derivative, a channel protein and a receptor.
459. The method according to aspect 455, wherein said composition is present in an environment.
460. The method according to aspect 459, wherein said environment is water, air or soil.
461. The method according to aspect 455, wherein said composition is a biological sample from an organism.
462. The method according to aspect 461, wherein said biological sample is selected from the group consisting of blood, serum, plasma, urine, saliva, a biopsy sample, feces, tissue and a skin patch.
463. The method according to aspect 461, wherein said substance binds to and is internalized by said minicell or is otherwise inactivated by selective absorption.
464. The method according to aspect 463, wherein said biological sample is returned to said organism after being contacting to said minicell.

### SEQUENCE LISTING

### SEQ ID NO 1

pMPX-23 (complete *ftsZ* cloned into pMPX-18 using PCR-introduced PstI and XbaI)

Shine-Delgarno PstI
1621 CCATACCCGTTTTTTTGGGCTAGCAGGAGGAATTCACCCTGCAGATGTTTGAACCAATGG
1 M F E P M

1681 AACTTACCAATGACGCGGTGATTAAAGTCATCGGCGTCGGCGGCGGCGGTAATGCTG
6 E L T N D A V I K V I G V G G G G G N A

1741 TTGAACACATGGTGCGCGAGCGCATTGAAGGTGTTGAATTCTTCGCGGTAAATACCGATG
26 V E H M V R E R I E G V E F F A V N T D

1801 CACAAGCGCTGCGTAAAACAGCGGTTGGACAGACGATTCAAATCGGTAGCGGTATCACCA
46 A Q A L R K T A V G Q T I Q I G S G I T

1861 AAGGACTGGGCGCTGGCGCTAATCCAGAAGTTGGCCGCAATGCGGCTGATGAGGATCGCG
66 K G L G A G A N P E V G R N A A D E D R

1921 ATGCATTGCGTGCGGCGCTGGAAGGTGCAGACATGGTCTTTATTGCTGCGGGTATGGGTG
86 D A L R A A L E G A D M V F I A A G M G

1981 GTGGTACCGGTACAGGTGCAGCACCAGTCGTCGCTGAAGTGGCAAAAGATTTGGGTATCC
106 G G T G T G A A P V V A E V A K D L G I

2041 TGACCGTTGCTGTCGTCACTAAGCCTTTCAACTTTGAAGGCAAGAAGCGTATGGCATTCG
126 L T V A V V T K P F N F E G K K R M A F

2101 CGGAGCAGGGGATCACTGAACTGTCCAAGCATGTGGACTCTCTGATCACTATCCCGAACG
146 A E Q G I T E L S K H V D S L I T I P N

2161 ACAAACTGCTGAAAGTTCTGGGCCGCGGTATCTCCCTGCTGGATGCGTTTGGCGCAGCGA
166 D K L L K V L G R G I S L L D A F G A A

2221 ACGATGTACTGAAAGGCGCTGTGCAAGGTATCGCTGAACTGATTACTCGTCCGGGTTTGA
186 N D V L K G A V Q G I A E L I T R P G L

2281 TGAACGTGGACTTTGCAGACGTACGCACCGTAATGTCTGAGATGGGCTACGCAATGATGG
206 M N V D F A D V R T V M S E M G Y A M M

2341 GTTCTGGCGTGGCGAGCGGTGAAGACCGTGCGGAAGAAGCTGCTGAAATGGCTATCTCTT
226 G S G V A S G E D R A E E A A E M A I S

2401 CTCCGCTGCTGGAAGATATCGACCTGTCTGGCGCGCGCGGCGTGCTGGTTAACATCACGG
246 S P L L E D I D L S G A R G V L V N I T

2461 CGGGCTTCGACCTGCGTCTGGATGAGTTCGAAACGGTAGGTAACACCATCCGTGCATTTG
266 A G F D L R L D E F E T V G N T I R A F

2521 CTTCCGACAACGCGACTGTGGTTATCGGTACTTCTCTTGACCCGGATATGAATGACGAGC
286 A S D N A T V V I G T S L D P D M N D E

2581 TGCGCGTAACCGTTGTTGCGACAGGTATCGGCATGGACAAACGTCCTGAAATCACTCTGG
306 L R V T V V A T G I G M D K R P E I T L

2641 TGACCAATAAGCAGGTTCAGCAGCCAGTGATGGATCGCTACCAGCAGCATGGGATGGCTC

326 V T N K Q V Q Q P V M D R Y Q Q H G M A

2701 CGCTGACCCAGGAGCAGAAGCCGGTTGCTAAAGTCGTGAATGACAATGCGCCGCAAACTG
346 P L T Q E Q K P V A K V V N D N A P Q T

2761 CGAAAGAGCCGGATTATCTGGATATCCCAGCATTCCTGCGTAAGCAAGCTGATTAATAAT
366 A K E P D Y L D I P A F L R K Q A D

XbaI
2821 CTAGAGGATCCCCGGGTACCGAGCTCGAATTCGTAATCATGGTCATAGCTGTTTCCTGTG

Sequence contains full-length *ftsZ* PCR amplified from *E. coli* MG1655 using oligos containing Pstl and XbaI restriction sites.

### SEQ ID NO 2

pMPX-47 (complete *ftsZ* cloned into pMPX-5 using PCR-introduced PstI and XbaI)

Shine-Delgarno PstI
2401 GAATTCAGGCGCTTTTTAGACTGGTCGTAATGAAATTCAGCAGGATCACATTCTGCAGAT
M

2461 GTTTGAACCAATGGAACTTACCAATGACGCGGTGATTAAAGTCATCGGCGTCGGCGGCGG
2 F E P M E L T N D A V I K V I G V G G G

2521 CGGCGGTAATGCTGTTGAACACATGGTGCGCGAGCGCATTGAAGGTGTTGAATTCTTCGC
22 G G N A V E H M V R E R I E G V E F F A

2581 GGTAAATACCGATGCACAAGCGCTGCGTAAAACAGCGGTTGGACAGACGATTCAAATCGG
42 V N T D A Q A L R K T A V G Q T I Q I G

2641 TAGCGGTATCACCAAAGGACTGGGCGCTGGCGCTAATCCAGAAGTTGGCCGCAATGCGGC
62 S G I T K G L G A G A N P E V G R N A A

2701 TGATGAGGATCGCGATGCATTGCGTGCGGCGCTGGAAGGTGCAGACATGGTCTTTATTGC
82 D E D R D A L R A A L E G A D M V F I A

2761 TGCGGGTATGGGTGGTGGTACCGGTACAGGTGCAGCACCAGTCGTCGCTGAAGTGGCAAA
102 A G M G G G T G T G A A P V V A E V A K

2821 AGATTTGGGTATCCTGACCGTTGCTGTCGTCACTAAGCCTTTCAACTTTGAAGGCAAGAA
122 D L G I L T V A V V T K P F N F E G K K

2881 GCGTATGGCATTCGCGGAGCAGGGGATCACTGAACTGTCCAAGCATGTGGACTCTCTGAT
142 R M A F A E Q G I T E L S K H V D S L I

2941 CACTATCCCGAACGACAAACTGCTGAAAGTTCTGGGCCGCGGTATCTCCCTGCTGGATGC
162 T I P N D K L L K V L G R G I S L L D A

3001 GTTTGGCGCAGCGAACGATGTACTGAAAGGCGCTGTGCAAGGTATCGCTGAACTGATTAC
182 F G A A N D V L K G A V Q G I A E L I T

3061 TCGTCCGGGTTTGATGAACGTGGACTTTGCAGACGTACGCACCGTAATGTCTGAGATGGG
202 R P G L M N V D F A D V R T V M S E M G

3121 CTACGCAATGATGGGTTCTGGCGTGGCGAGCGGTGAAGACCGTGCGGAAGAAGCTGCTGA

222 Y A M M G S G V A S G E D R A E E A A E

3181 AATGGCTATCTCTTCTCCGCTGCTGGAAGATATCGACCTGTCTGGCGCGCGCGGCGTGCT
242 M A I S S P L L E D I D L S G A R G V L

3241 GGTTAACATCACGGCGGGCTTCGACCTGCGTCTGGATGAGTTCGAAACGGTAGGTAACAC
262 V N I T A G F D L R L D E F E T V G N T

3301 CATCCGTGCATTTGCTTCCGACAACGCGACTGTGGTTATCGGTACTTCTCTTGACCCGGA
282 I R A F A S D N A T V V I G T S L D P D

3361 TATGAATGACGAGCTGCGCGTAACCGTTGTTGCGACAGGTATCGGCATGGACAAACGTCC
302 M N D E L R V T V V A T G I G M D K R P

3421 TGAAATCACTCTGGTGACCAATAAGCAGGTTCAGCAGCCAGTGATGGATCGCTACCAGCA
322 E I T L V T N K Q V Q Q P V M D R Y Q Q

3481 GCATGGGATGGCTCCGCTGACCCAGGAGCAGAAGCCGGTTGCTAAAGTCGTGAATGACAA
342 H G M A P L T Q E Q K P V A K V V N D N

3541 TGCGCCGCAAACTGCGAAAGAGCCGGATTATCTGGATATCCCAGCATTCCTGCGTAAGCA
362 A P Q T A K E P D Y L D I P A F L R K Q

XbaI
3601 AGCTGATTAATAATCTAGAGGATCCCCGGGTACCGAGCTCGAATTCGTAATCATGGTCAT
382 A D

Sequence contains full-length *ftsZ* PCR amplified from *E*. *coli* MG1655 using oligos containing PstI and XbaI restriction sites.

### SEQ ID NO 3

*araC*::Para::*ftsZ* inserted by RED recombination into *E. coli* MG1655 *intD*

intD homology for recombination                                    Stop araC
181   AAGCCTGCAT TGCGGCGCTT CAGTCTCCGC TGCATACTGT CCCGTTACCA ATTATGACAA

241   CTTGACGGCT ACATCATTCA CTTTTTCTTC ACAACCGGCA CGGAACTCGC TCGGGCTGGC
301   CCCGGTGCAT TTTTTAAATA CCCGCGAGAA ATAGAGTTGA TCGTCAAAAC CAACATTGCG
361   ACCGACGGTG GCGATAGGCA TCCGGGTGGT GCTCAAAAGC AGCTTCGCCT GGCTGATACG
421   TTGGTCCTCG CGCCAGCTTA AGACGCTAAT CCCTAACTGC TGGCGGAAAA GATGTGACAG
481   ACGCGACGGC GACAAGCAAA CATGCTGTGC GACGCTGGCG ATATCAAAAT TGCTGTCTGC
541   CAGGTGATCG CTGATGTACT GACAAGCCTC GCGTACCCGA TTATCCATCG GTGGATGGAG
601   CGACTCGTTA ATCGCTTCCA TGCGCCGCAG TAACAATTGC TCAAGCAGAT TTATCGCCAG
661   CAGCTCCGAA TAGCGCCCTT CCCCTTGCCC GGCGTTAATG ATTTGCCCAA ACAGGTCGCT
721   GAAATGCGGC TGGTGCGCTT CATCCGGGCG AAAGAACCCC GTATTGGCAA ATATTGACGG
781   CCAGTTAAGC CATTCATGCC AGTAGGCGCG CGGACGAAAG TAAACCCACT GGTGATACCA
841   TTCGCGAGCC TCCGGATGAC GACCGTAGTG ATGAATCTCT CCTGGCGGGA ACAGCAAAAT
901   ATCACCCGGT CGGCAAACAA ATTCTCGTCC CTGATTTTTC ACCACCCCCT GACCGCGAAT
961   GGTGAGATTG AGAATATAAC CTTTCATTCC CAGCGGTCGG TCGATAAAAA AATCGAGATA
1021  ACCGTTGGCC TCAATCGGCG TTAAACCCGC CACCAGATGG GCATTAAACG AGTATCCCGG
1081  CAGCAGGGGA TCATTTTGCG CTTCAGCCAT ACTTTTCATA CTCCCGCCAT TCAGAGAAGA

Start araC

1141 AACCAATTGT CCATATTGCA TCAGACATTG CCGTCACTGC GTCTTTTACT GGCTCTTCTC
←

1201 GCTAACCAAA CCGGTAACCC CGCTTATTAA AAGCATTCTG TAACAAAGCG GGACCAAAGC
1261 CATGACAAAA ACGCGTAACA AAAGTGTCTA TAATCACGGC AGAAAAGTCC ACATTGATTA
1321 TTTGCACGGC GTCACACTTT GCTATGCCAT AGCATTTTTA TCCATAAGAT TAGCGGATCC
1381 TACCTGACGC TTTTTATCGC AACTCTCTAC TGTTTCTCCA TACCCGTTTT TTTGGGCTAG

Shine-Delgarno Start ftsZ
1441 CAGGAGGAAT TCACCCTGCA GATGTTTGAA CCAATGGAAC TTACCAATGA CGCGGTGATT
→

1501 AAAGTCATCG GCGTCGGCGG CGGCGGCGGT AATGCTGTTG AACACATGGT GCGCGAGCGC
1561 ATTGAAGGTG TTGAATTCTT CGCGGTAAAT ACCGATGCAC AAGCGCTGCG TAAAACAGCG
1621 GTTGGACAGA CGATTCAAAT CGGTAGCGGT ATCACCAAAG GACTGGGCGC TGGCGCTAAT
1681 CCAGAAGTTG GCCGCAATGC GGCTGATGAG GATCGCGATG CATTGCGTGC GGCGCTGGAA
1741 GGTGCAGACA TGGTCTTTAT TGCTGCGGGT ATGGGTGGTG GTACCGGTAC AGGTGCAGCA
1801 CCAGTCGTCG CTGAAGTGGC AAAAGATTTG GGTATCCTGA CCGTTGCTGT CGTCACTAAG
1861 CCTTTCAACT TTGAAGGCAA GAAGCGTATG GCATTCGCGG AGCAGGGGAT CACTGAACTG
1921 TCCAAGCATG TGGACTCTCT GATCACTATC CCGAACGACA AACTGCTGAA AGTTCTGGGC
1981 CGCGGTATCT CCCTGCTGGA TGCGTTTGGC GCAGCGAACG ATGTACTGAA AGGCGCTGTG
2041 CAAGGTATCG CTGAACTGAT TACTCGTCCG GGTTTGATGA ACGTGGACTT TGCAGACGTA
2101 CGCACCGTAA TGTCTGAGAT GGGCTACGCA ATGATGGGTT CTGGCGTGGC GAGCGGTGAA
2161 GACCGTGCGG AAGAAGCTGC TGAAATGGCT ATCTCTTCTC CGCTGCTGGA AGATATCGAC
2221 CTGTCTGGCG CGCGCGGCGT GCTGGTTAAC ATCACGGCGG GCTTCGACCT GCGTCTGGAT
2281 GAGTTCGAAA CGGTAGGTAA CACCATCCGT GCATTTGCTT CCGACAACGC GACTGTGGTT
2341 ATCGGTACTT CTCTTGACCC GGATATGAAT GACGAGCTGC GCGTAACCGT TGTTGCGACA
2401 GGTATCGGCA TGGACAAACG TCCTGAAATC ACTCTGGTGA CCAATAAGCA GGTTCAGCAG
2461 CCAGTGATGG ATCGCTACCA GCAGCATGGG ATGGCTCCGC TGACCCAGGA GCAGAAGCCG
2521 GTTGCTAAAG TCGTGAATGA CAATGCGCCG CAAACTGCGA AAGAGCCGGA TTATCTGGAT

Stop ftsZ
2581 ATCCCAGCAT TCCTGCGTAA GCAAGCTGAT TAATAATCTA GAGGCGTTAC CAATTATGAC

FRT scar intD homology
2641 AACTTGACGG GAAGTTCCTA TACTTTCTAG AGAATAGGAA CTTCCC AAAG CCAGTATCAA

for recombination
3721 CTCAGACAAA GGCAAAGCAT CTTG

Bold, italicized represents homology between the PCR product shown below and *intD.*
*araC*::Para::*ftsZ*::FRT::*kan*::Frt

Following RED recombination into *intD,* the kanamycin cassette was removed with *flp* recombinase resulting in a single FRT scar as depicted above. Bold alone represents FRT scar after the *flp* reaction.

### SEO ID NO 4

*rhaRS*::Prha::*ftsZ* inserted by RED recombination into *E. coli* MG1655 *intD*

intD homology for recombination Stop rhaR
181 AAGCCTGCAT TGCGGCGCTT CAGTCTCCGC TGCATACTGT CCTTAATCTT TCTGCGAATT
241 GAGATGACGC CACTGGCTGG GCGTCATCCC GGTTTCCCGG GTAAACACCA CCGAAAAATA

301 GTTACTATCT TCAAAGCCAC ATTCGGTCGA AATATCACTG ATTAACAGGC GGCTATGCTG
361 GAGAAGATAT TGCGCATGAC ACACTCTGAC CTGTCGCAGA TATTGATTGA TGGTCATTCC
421 AGTCTGCTGG CGAAATTGCT GACGCAAAAC GCGCTCACTG CACGATGCCT CATCACAAAA
481 TTTATCCAGC GCAAAGGGAC TTTTCAGGCT AGCCGCCAGC CGGGTAATCA GCTTATCCAG
541 CAACGTTTCG CTGGATGTTG GCGGCAACGA ATCACTGGTG TAACGATGGC GATTCAGCAA
601 CATCACCAAC TGCCCGAACA GCAACTCAGC CATTTCGTTA GCAAACGGCA CATGCTGACT
661 ACTTTCATGC TCAAGCTGAC CGATAACCTG CCGCGCCTGC GCCATCCCCA TGCTACCTAA
721 GCGCCAGTGT GGTTGCCCTG CGCTGGCGTT AAATCCCGGA ATCGCCCCCT GCCAGTCAAG
781 ATTCAGCTTC AGACGCTCCG GGCAATAAAT AATATTCTGC AAAACCAGAT CGTTAACGGA
841 AGCGTAGGAG TGTTTATCGT CAGCATGAAT GTAAAAGAGA TCGCCACGGG TAATGCGATA
901 AGGGCGATCG TTGAGTACAT GCAGGCCATT ACCGCGCCAG ACAATCACCA GCTCACAAAA
961 ATCATGTGTA TGTTCAGCAA AGACATCTTG CGGATAACGG TCAGCCACAG CGACTGCCTG
1021 CTGGTCGCTG GCAAAAAAAT CATCTTTGAG AAGTTTTAAC TGATGCGCCA CCGTGGCTAC
1081 CTCGGCCAGA GAACGAAGTT GATTATTCGC AATATGGCGT ACAAATACGT TGAGAAGATT

Stop rhaS Start rhaR
1141 CGCGTTATTG CAGAAAGCCA TCCCGTCCCT GGCGAATATC ACGCGGTGAC CAGTTAAACT
←

1201 CTCGGCGAAA AAGCGTCGAA AAGTGGTTAC TGTCGCTGAA TCCACAGCGA TAGGCGATGT
1261 CAGTAACGCT GGCCTCGCTG TGGCGTAGCA GATGTCGGGC TTTCATCAGT CGCAGGCGGT
1321 TCAGGTATCG CTGAGGCGTC AGTCCCGTTT GCTGCTTAAG CTGCCGATGT AGCGTACGCA
1381 GTGAAAGAGA AAATTGATCC GCCACGGCAT CCCAATTCAC CTCATCGGCA AAATGGTCCT
1441 CCAGCCAGGC CAGAAGCAAG TTGAGACGTG ATGCGCTGTT TTCCAGGTTC TCCTGCAAAC
1501 TGCTTTTACG CAGCAAGAGC AGTAATTGCA TAAACAAGAT CTCGCGACTG GCGGTCGAGG
1561 GTAAATCATT TTCCCCTTCC TGCTGTTCCA TCTGTGCAAC CAGCTGTCGC ACCTGCTGCA
1621 ATACGCTGTG GTTAACGCGC CAGTGAGACG GATACTGCCC ATCCAGCTCT TGTGGCAGCA
1681 ACTGATTCAG CCCGGCGAGA AACTGAAATC GATCCGGCGA GCGATACAGC ACATTGGTCA
1741 GACACAGATT ATCGGTATGT TCATACAGAT GCCGATCATG ATCGCGTACG AAACAGACCG
1801 TGCCACCGGT GATGGTATAG GGCTGCCCAT TAAACACATG AATACCCGTG CCATGTTCGA
1861 CAATCACAAT TTCATGAAAA TCATGATGAT GTTCAGGAAA ATCCGCCTGC GGGAGCCGGG

Start
rhaS
1921 GTTCTATCGC CACGGACGCG TTACCAGACG GAAAAAAATC CACACTATGT AATACGGTCA
←

1981 TACTGGCCTC CTGATGTCGT CAACACGGCG AAATAGTAAT CACGAGGTCA GGTTCTTACC

2041 TTAAATTTTC GACGGAAAAC CACGTAAAAA ACGTCGATTT TTCAAGATAC AGCGTGAATT
2101 TTCAGGAAAT GCGGTGAGCA TCACATCACC ACAATTCAGC AAATTGTGAA CATCATCACG
2161 TTCATCTTTC CCTGGTTGCC AATGGCCCAT TTTCCTGTCA GTAACGAGAA GGTCGCGAAT

Shine-Delgarno Start ftsZ
2221 TCAGGCGCTT TTTAGACTGG TCGTAATGAA ATTCAGCAGG ATCACATATG TTTGAACCAA
→

2581 TGGAACTTAC CAATGACGCG GTGATTAAAG TCATCGGCGT CGGCGGCGGC GGCGGTAATG
2641 CTGTTGAACA CATGGTGCGC GAGCGCATTG AAGGTGTTGA ATTCTTCGCG GTAAATACCG
2701 ATGCACAAGC GCTGCGTAAA ACAGCGGTTG GACAGACGAT TCAAATCGGT AGCGGTATCA
2761 CCAAAGGACT GGGCGCTGGC GCTAATCCAG AAGTTGGCCG CAATGCGGCT GATGAGGATC
2821 GCGATGCATT GCGTGCGGCG CTGGAAGGTG CAGACATGGT CTTTATTGCT GCGGGTATGG
2881 GTGGTGGTAC CGGTACAGGT GCAGCACCAG TCGTCGCTGA AGTGGCAAAA GATTTGGGTA
2941 TCCTGACCGT TGCTGTCGTC ACTAAGCCTT TCAACTTTGA AGGCAAGAAG CGTATGGCAT
3001 TCGCGGAGCA GGGGATCACT GAACTGTCCA AGCATGTGGA CTCTCTGATC ACTATCCCGA
3061 ACGACAAACT GCTGAAAGTT CTGGGCCGCG GTATCTCCCT GCTGGATGCG TTTGGCGCAG
3121 CGAACGATGT ACTGAAAGGC GCTGTGCAAG GTATCGCTGA ACTGATTACT CGTCCGGGTT
3181 TGATGAACGT GGACTTTGCA GACGTACGCA CCGTAATGTC TGAGATGGGC TACGCAATGA
3241 TGGGTTCTGG CGTGGCGAGC GGTGAAGACC GTGCGGAAGA AGCTGCTGAA ATGGCTATCT

3301 CTTCTCCGCT GCTGGAAGAT ATCGACCTGT CTGGCGCGCG CGGCGTGCTG GTTAACATCA
3361 CGGCGGGCTT CGACCTGCGT CTGGATGAGT TCGAAACGGT AGGTAACACC ATCCGTGCAT
3421 TTGCTTCCGA CAACGCGACT GTGGTTATCG GTACTTCTCT TGACCCGGAT ATGAATGACG
3481 AGCTGCGCGT AACCGTTGTT GCGACAGGTA TCGGCATGGA CAAACGTCCT GAAATCACTC
3541 TGGTGACCAA TAAGCAGGTT CAGCAGCCAG TGATGGATCG CTACCAGCAG CATGGGATGG
3601 CTCCGCTGAC CCAGGAGCAG AAGCCGGTTG CTAAAGTCGT GAATGACAAT GCGCCGCAAA

Stop
ftsZ
3661 CTGCGAAAGA GCCGGATTAT CTGGATATCC CAGCATTCCT GCGTAAGCAA GCTGATTAAT

FRT scar
3721 AATCTAGAGG CGTTACCAAT TATGACAACT TGACGGGAAG TTCCTATACT TTCTAGAGAA

intD homology for recombination
3781 TAGGAACTTC CCAAAGCCAG TATCAACTCA GACAAAGGCA AAGCATCTTG

Bold, italicized represents homology between the PCR product shown below and *intD.*
*rhaRS*::Prha::*ftsZ*::FRT::*kan*::Frt

Following RED recombination into *intD,* the kanamycin cassette was removed with *flp* recombinase resulting in a single FRT scar as depicted above. Bold alone represents FRT scar after the *flp* reaction.

### SEQ ID NO 5

*lac*I::Ptac::*ftsZ* inserted by RED recombination into *E. coli* MG1655 *intD*

intD homology for recombination                    Stop lacI
181   AAGCCTGCAT TGCGGCGCTT CAGTCTCCGC TGCATACTGT CCTTAATAAA GTGAGTCGAT

241   ATTGTCTTTG TTGACCAGTA ATACCTTATG GAAACGGATA ATTCGCTTAT CCATATCTAC
301   GTCGGCCTTA CCCAGATTCT GCATTTCTAA TCCAGGCTTG ATCTCTTCAC CCTTCAGCAA
361   CGTGCTGGCG ACGGCTGCGA GTGCGTAACC TGCAGAGGCC GGATCGTAAG TAATCCCTTC
421   GGTGATATCA CCACTTTTAA TCAGTGATGC CGCCTGTGAA GGGATCATCA TGCCATAGAC
481   TGCGACTTTA TTTTTCGCCC GTTTCTCTTT CACCGCACGT CCCGCGCCAA TCGGACCGTT
541   TGAACCAAAG GAGACAACCG CTTTCAAGTC AGGATAGGTT TTCATCAGGT CCAGTGTAGT
601   ACGACGTGAG ACATCCACAC TCTCGGCAAC CGGCATGCGG CGGGTAACTT CATGCATATC
661   CGGGTAATGC TCTTTCTGGT ATTTCACCAG CAAGTCAGCC CATAAGTTAT GCTGCGGCAC
721   GGTCAAACTA CCCACGTAAA TCACATAGCC GCCCTTGCCA CCCATGCGTT TCGCCATATG
781   CTCAACATAT TCAGCGGCAA ATTTTTCGTT ATCAATGATT TCGATATCCC AGTTAGCACT
841   TGGCTGACCG GGGGATTCGT TGGTCAGAAC CACAATTCCG GCATCTCGCG CTTTTTTGAA
901   TACCGGTTCC AGCACGTTGG CATCGTTTGG CACGATAGTA ATTGCATTAA CCTTACGGGC
961   GATTAAATCC TCAATAATTT TAACTTGTTG CGGAGCATCA GTACTTGAAG GCCCCACCTG
1021  TGAGGCATTA ACACCAAAGG CTTTACCCGC CTCAACCACA CCTTCGCCCA TGCGATTAAA
1081  CCACGGCATA CCATCGACTT TAGAAATATT CACCACGACT TTTTCCGCTG CCTGGAGCGG
1141  CGCAGAAATT AGCGCAGCGC CTAATAACAG CGAAGACACC ATATTGATAA CAAAACGTTT

      Start lacI                                          Start ftsZ
1201  ATTCATCAT    Ptac sequence (see reference below)   A TGGAACTTAC

12    CAATGACGCG GTGATTAAAG TCATCGGCGT CGGCGGCGGC GGCGGTAATG CTGTTGAACA
72    CATGGTGCGC GAGCGCATTG AAGGTGTTGA ATTCTTCGCG GTAAATACCG ATGCACAAGC
132   GCTGCGTAAA ACAGCGGTTG GACAGACGAT TCAAATCGGT AGCGGTATCA CCAAAGGACT

192 GGGCGCTGGC GCTAATCCAG AAGTTGGCCG CAATGCGGCT GATGAGGATC GCGATGCATT
252 GCGTGCGGCG CTGGAAGGTG CAGACATGGT CTTTATTGCT GCGGGTATGG GTGGTGGTAC
312 CGGTACAGGT GCAGCACCAG TCGTCGCTGA AGTGGCAAAA GATTTGGGTA TCCTGACCGT
372 TGCTGTCGTC ACTAAGCCTT TCAACTTTGA AGGCAAGAAG CGTATGGCAT TCGCGGAGCA
432 GGGGATCACT GAACTGTCCA AGCATGTGGA CTCTCTGATC ACTATCCCGA ACGACAAACT
492 GCTGAAAGTT CTGGGCCGCG GTATCTCCCT GCTGGATGCG TTTGGCGCAG CGAACGATGT
552 ACTGAAAGGC GCTGTGCAAG GTATCGCTGA ACTGATTACT CGTCCGGGTT TGATGAACGT
612 GGACTTTGCA GACGTACGCA CCGTAATGTC TGAGATGGGC TACGCAATGA TGGGTTCTGG
672 CGTGGCGAGC GGTGAAGACC GTGCGGAAGA AGCTGCTGAA ATGGCTATCT CTTCTCCGCT
732 GCTGGAAGAT ATCGACCTGT CTGGCGCGCG CGGCGTGCTG GTTAACATCA CGGCGGGCTT
792 CGACCTGCGT CTGGATGAGT TCGAAACGGT AGGTAACACC ATCCGTGCAT TTGCTTCCGA
852 CAACGCGACT GTGGTTATCG GTACTTCTCT TGACCCGGAT ATGAATGACG AGCTGCGCGT
912 AACCGTTGTT GCGACAGGTA TCGGCATGGA CAAACGTCCT GAAATCACTC TGGTGACCAA
972 TAAGCAGGTT CAGCAGCCAG TGATGGATCG CTACCAGCAG CATGGGATGG CTCCGCTGAC
1032 CCAGGAGCAG AAGCCGGTTG CTAAAGTCGT GAATGACAAT GCGCCGCAAA CTGCGAAAGA
1092 GCCGGATTAT CTGGATATCC CAGCATTCCT GCGTAAGCAA GCTGATTAAT AATCTAGAGG
1152 CGTTACCAAT TATGACAACT TGACGGGAAG TTCCTATTCT CTAGAAAGTA TAGGAACTTC
1212 CCAAAGCCAG TATCAACTCA GACAAAGGCA AAGCATCTTG

Bold, italicized represents homology between the PCR product shown below and *intD.*
*lacI*::Ptac::*ftsZ*::FRT::*kan*::Frt

Following RED recombination into *intD,* the kanamycin cassette was removed with *flp* recombinase resulting in a single FRT scar as depicted above.
Garrido, T., et al. 1993. Transcription of ftsZ oscillates during the cell cycle of Escherichia coli. EMBO J. 12:3957-3965

### SEQ ID NO 6

### pMPX-5 expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

Stop rhaR
361 TTTCCCAGTC ACGACGTTGT AAAACGACGG CCAGTGCCAA GCTTAATTAA TCTTTCTGCG

421 AATTGAGATG ACGCCACTGG CTGGGCGTCA TCCCGGTTTC CCGGGTAAAC ACCACCGAAA
481 AATAGTTACT ATCTTCAAAG CCACATTCGG TCGAAATATC ACTGATTAAC AGGCGGCTAT
541 GCTGGAGAAG ATATTGCGCA TGACACACTC TGACCTGTCG CAGATATTGA TTGATGGTCA
601 TTCCAGTCTG CTGGCGAAAT TGCTGACGCA AAACGCGCTC ACTGCACGAT GCCTCATCAC
661 AAAATTTATC CAGCGCAAAG GGACTTTTCA GGCTAGCCGC CAGCCGGGTA ATCAGCTTAT
721 CCAGCAACGT TTCGCTGGAT GTTGGCGGCA ACGAATCACT GGTGTAACGA TGGCGATTCA
781 GCAACATCAC CAACTGCCCG AACAGCAACT CAGCCATTTC GTTAGCAAAC GGCACATGCT
841 GACTACTTTC ATGCTCAAGC TGACCGATAA CCTGCCGCGC CTGCGCCATC CCCATGCTAC
901 CTAAGCGCCA GTGTGGTTGC CCTGCGCTGG CGTTAAATCC CGGAATCGCC CCCTGCCAGT
961 CAAGATTCAG CTTCAGACGC TCCGGGCAAT AAATAATATT CTGCAAAACC AGATCGTTAA
1021 CGGAAGCGTA GGAGTGTTTA TCGTCAGCAT GAATGTAAAA GAGATCGCCA CGGGTAATGC
1081 GATAAGGGCG ATCGTTGAGT ACATGCAGGC CATTACCGCG CCAGACAATC ACCAGCTCAC

1141 AAAAATCATG TGTATGTTCA GCAAAGACAT CTTGCGGATA ACGGTCAGCC ACAGCGACTG
1201 CCTGCTGGTC GCTGGCAAAA AAATCATCTT TGAGAAGTTT TAACTGATGC GCCACCGTGG
1261 CTACCTCGGC CAGAGAACGA AGTTGATTAT TCGCAATATG GCGTACAAAT ACGTTGAGAA

Stop rhaS Start rhaR
1321 GATTCGCGTT ATTGCAGAAA GCCATCCCGT CCCTGGCGAA TATCACGCGG TGACCAGTTA
←

1381 AACTCTCGGC GAAAAAGCGT CGAAAAGTGG TTACTGTCGC TGAATCCACA GCGATAGGCG
1441 ATGTCAGTAA CGCTGGCCTC GCTGTGGCGT AGCAGATGTC GGGCTTTCAT CAGTCGCAGG
1501 CGGTTCAGGT ATCGCTGAGG CGTCAGTCCC GTTTGCTGCT TAAGCTGCCG ATGTAGCGTA
1561 CGCAGTGAAA GAGAAAATTG ATCCGCCACG GCATCCCAAT TCACCTCATC GGCAAAATGG
1621 TCCTCCAGCC AGGCCAGAAG CAAGTTGAGA CGTGATGCGC TGTTTTCCAG GTTCTCCTGC
1681 AAACTGCTTT TACGCAGCAA GAGCAGTAAT TGCATAAACA AGATCTCGCG ACTGGCGGTC
1741 GAGGGTAAAT CATTTTCCCC TTCCTGCTGT TCCATCTGTG CAACCAGCTG TCGCACCTGC
1801 TGCAATACGC TGTGGTTAAC GCGCCAGTGA GACGGATACT GCCCATCCAG CTCTTGTGGC
1861 AGCAACTGAT TCAGCCCGGC GAGAAACTGA AATCGATCCG GCGAGCGATA CAGCACATTG
1921 GTCAGACACA GATTATCGGT ATGTTCATAC AGATGCCGAT CATGATCGCG TACGAAACAG
1981 ACCGTGCCAC CGGTGATGGT ATAGGGCTGC CCATTAAACA CATGAATACC CGTGCCATGT
2041 TCGACAATCA CAATTTCATG AAAATCATGA TGATGTTCAG GAAAATCCGC CTGCGGGAGC
2101 CGGGGTTCTA TCGCCACGGA CGCGTTACCA GACGGAAAAA AATCCACACT ATGTAATACG

Start rhaS
2161 GTCATACTGG CCTCCTGATG TCGTCAACAC GGCGAAATAG TAATCACGAG GTCAGGTTCT
←

2221 TACCTTAAAT TTTCGACGGA AAACCACGTA AAAAACGTCG ATTTTTCAAG ATACAGCGTG
2281 AATTTTCAGG AAATGCGGTG AGCATCACAT CACCACAATT CAGCAAATTG TGAACATCAT
2341 CACGTTCATC TTTCCCTGGT TGCCAATGGC CCATTTTCCT GTCAGTAACG AGAAGGTCGC

a. Shine-Delgarno PstI

2401 GAATTCAGGC GCTTTTTAGA CTGGTCGTAA TGAAATTCAG CAGGATCACA TTCTGCAGGT
→

SalI XbaI BamHI KpnI
2461 CGACTCTAGA GGATCCCCGG GTACCGAGCT CGAATTCGTA ATCATGGTCA TAGCTGTTTC
2521 CTGTGTGAAA TTGTTATCCG CTCACAATTC CACACAACAT ACGAGCCGGA AGCATAAAGT
2581 GTAAAGCCTG GGGTGCCTAA TGAGTGAGCT AACTCACATT AATTGCGTTG CGCTCACTGC
2641 CCGCTTTCCA GTCGGGAAAC CTGTCGTGCC AGCTGCATTA ATGAATCGGC CAACGCGCGG
2701 GGAGAGGCGG TTTGCGTATT GGGCGCTCTT CCGCTTCCTC GCTCACTGAC TCGCTGCGCT
2761 CGGTCGTTCG GCTGCGGCGA GCGGTATCAG CTCACTCAAA GGCGGTAATA CGGTTATCCA
2821 CAGAATCAGG GGATAACGCA GGAAAGAACA TGTGAGCAAA AGGCCAGCAA AAGGCCAGGA
2881 ACCGTAAAAA GGCCGCGTTG CTGGCGTTTT TCCATAGGCT CCGCCCCCCT GACGAGCATC
2941 ACAAAAATCG ACGCTCAAGT CAGAGGTGGC GAAACCCGAC AGGACTATAA AGATACCAGG
3001 CGTTTCCCCC TGGAAGCTCC CTCGTGCGCT CTCCTGTTCC GACCCTGCCG CTTACCGGAT
3061 ACCTGTCCGC CTTTCTCCCT TCGGGAAGCG TGGCGCTTTC TCATAGCTCA CGCTGTAGGT
3121 ATCTCAGTTC GGTGTAGGTC GTTCGCTCCA AGCTGGGCTG TGTGCACGAA CCCCCCGTTC
3181 AGCCCGACCG CTGCGCCTTA TCCGGTAACT ATCGTCTTGA GTCCAACCCG GTAAGACACG
3241 ACTTATCGCC ACTGGCAGCA GCCACTGGTA ACAGGATTAG CAGAGCGAGG TATGTAGGCG
3301 GTGCTACAGA GTTCTTGAAG TGGTGGCCTA ACTACGGCTA CACTAGAAGG ACAGTATTTG
3361 GTATCTGCGC TCTGCTGAAG CCAGTTACCT TCGGAAAAAG AGTTGGTAGC TCTTGATCCG
3421 GCAAACAAAC CACCGCTGGT AGCGGTGGTT TTTTTGTTTG CAAGCAGCAG ATTACGCGCA
3481 GAAAAAAAGG ATCTCAAGAA GATCCTTTGA TCTTTTCTAC GGGGTCTGAC GCTCAGTGGA
3541 ACGAAAACTC ACGTTAAGGG ATTTTGGTCA TGAGATTATC AAAAAGGATC TTCACCTAGA
3601 TCCTTTTAAA TTAAAAATGA AGTTTTAAAT CAATCTAAAG TATATATGAG TAAACTTGGT

Stop bla
3661 CTGACAGTTA CCAATGCTTA ATCAGTGAGG CACCTATCTC AGCGATCTGT CTATTTCGTT

3721 CATCCATAGT TGCCTGACTC CCCGTCGTGT AGATAACTAC GATACGGGAG GGCTTACCAT
3781 CTGGCCCCAG TGCTGCAATG ATACCGCGAG ACCCACGCTC ACCGGCTCCA GATTTATCAG
3841 CAATAAACCA GCCAGCCGGA AGGGCCGAGC GCAGAAGTGG TCCTGCAACT TTATCCGCCT
3901 CCATCCAGTC TATTAATTGT TGCCGGGAAG CTAGAGTAAG TAGTTCGCCA GTTAATAGTT
3961 TGCGCAACGT TGTTGCCATT GCTACAGGCA TCGTGGTGTC ACGCTCGTCG TTTGGTATGG
4021 CTTCATTCAG CTCCGGTTCC CAACGATCAA GGCGAGTTAC ATGATCCCCC ATGTTGTGCA
4081 AAAAAGCGGT TAGCTCCTTC GGTCCTCCGA TCGTTGTCAG AAGTAAGTTG GCCGCAGTGT
4141 TATCACTCAT GGTTATGGCA GCACTGCATA ATTCTCTTAC TGTCATGCCA TCCGTAAGAT
4201 GCTTTTCTGT GACTGGTGAG TACTCAACCA AGTCATTCTG AGAATAGTGT ATGCGGCGAC
4261 CGAGTTGCTC TTGCCCGGCG TCAATACGGG ATAATACCGC GCCACATAGC AGAACTTTAA
4321 AAGTGCTCAT CATTGGAAAA CGTTCTTCGG GGCGAAAACT CTCAAGGATC TTACCGCTGT
4381 TGAGATCCAG TTCGATGTAA CCCACTCGTG CACCCAACTG ATCTTCAGCA TCTTTTACTT
4441 TCACCAGCGT TTCTGGGTGA GCAAAAACAG GAAGGCAAAA TGCCGCAAAA AAGGGAATAA

Start bla
4501 GGGCGACACG GAAATGTTGA ATACTCATAC TCTTCCTTTT TCAATATTAT TGAAGCATTT
←

4561 ATCAGGGTTA TTGTCTCATG AGCGGATACA TATTTGAATG TATTTAGAAA AATAAACAAA
4621 TAGGGGTTCC GCGCACATTT CCCCGAAAAG TGCCACCTGA CGTCTAAGAA ACCATTATTA
4681 TCATGACATT AACCTATAAA AATAGGCGTA TCACGAGGCC CTTTCGTC

The segment *rhaR* through the Prha control region was taken from the *E. coli* MG1655 chromosome using PCR-added *Hind*III and *Pst*I restriciton sites. This fragment was cut with *Hin*dlll and *Pst*I and cloned into pUC-18 cut with the same enzymes. Italicized sequence constitutes both *rhaSR* and protein to be expressed promotor region.

### SEQ ID NO 10

### pMPX-7 expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

HindIII
361 TTTCCCAGTC ACGACGTTGT AAAACGACGG CCAGTGCCAA GCTTCGCAGC GCTGTTCCTT

421 TGCTCGCCTG CTGCGAGCTG GGTAAGCGGA CAAATTCTCA CCGTCTCCGG TGGTGGGGTA
481 CAGGAGCTCA ATTAATACAC TAACGGACCG GTAAACAACC GTGCGTGTTG TTTACCGGGA
541 TAAACTCATC AACGTCTCTG CTAAATAACT GGCAGCCAAA TCACGGCTAT TGGTTAACCA
601 ATTTCAGAGT GAAAAGTATA CGAATAGAGT GTGCCTTCGC ACTATTCAAC AGCAATGATA

Start uidR
661 GGCGCTCACC TGACAACGCG GTAAACTAGT TATTCACGCT AACTATAATG GTTTAATGAT
→

721 GGATAACATG CAGACTGAAG CACAACCGAC ACGGACCCGG ATCCTCAATG CTGCCAGAGA
781 GATTTTTTCA GAAAATGGAT TTCACAGTGC CTCGATGAAA GCCATCTGTA AATCTTGCGC
841 CATTAGTCCC GGGACGCTCT ATCACCATTT CATCTCCAAA GAAGCCTTGA TTCAGGCGAT
901 TATCTTACAG GACCAGGAGA GGGCGCTGGC CCGTTTCCGG GAACCGATTG AAGGGATTCA
961 TTTCGTTGAC TATATGGTCG AGTCCATTGT CTCTCTCACC CATGAAGCCT TTGGACAACG
1021 GGCGCTGGTG GTTGAAATTA TGGCGGAAGG GATGCGTAAC CCACAGGTCG CCGCCATGCT
1081 TAAAAATAAG CATATGACGA TCACGGAATT TGTTGCCCAG CGGATGCGTG ATGCCCAGCA
1141 AAAAGGCGAG ATAAGCCCAG ACATCAACAC GGCAATGACT TCACGTTTAC TGCTGGATCT

1201 GACCTACGGT GTACTGGCCG ATATCGAAGC GGAAGACCTG GCGCGTGAAG CGTCGTTTGC

Stop uidR
1261 TCAGGGATTA CGCGCGATGA TTGGCGGTAT CTTAACCGCA TCCTGATTCT CTCTCTTTTT

1321 CGGCGGGCTG GTGATAACTG TGCCCGCGTT TCATATCGTA ATTTCTCTGT GCAAAAATTA
1381 TCCTTCCCGG CTTCGGAGAA TTCCCCCCAA AATATTCACT GTAGCCATAT GTCATGAGAG
1441 TTTATCGTTC CCAATACGCT CGAACGAACG TTCGGTTGCT TATTTTATGG CTTCTGTCAA
1501 CGCTGTTTTA AAGATTAATG CGATCTATAT CACGCTGTGG GTATTGCAGT TTTTGGTTTT
1561 TTGATCGCGG TGTCAGTTCT TTTTATTTCC ATTTCTCTTC CATGGGTTTC TCACAGATAA
1621 CTGTGTGCAA CACAGAATTG GTTAACTAAT CAGATTAAAG GTTGACCAGT ATTATTATCT

Shine-Delgarno PstI SalI XbaI KpnI
1681 TAATGAGGAG TCCTGCAGGT CGACTCTAGA GGATCCCCGG GTACCGAGCT CGAATTCGTA
→

1741 ATCATGGTCA TAGCTGTTTC CTGTGTGAAA TTGTTATCCG CTCACAATTC CACACAACAT
1801 ACGAGCCGGA AGCATAAAGT GTAAAGCCTG GGGTGCCTAA TGAGTGAGCT AACTCACATT
1861 AATTGCGTTG CGCTCACTGC CCGCTTTCCA GTCGGGAAAC CTGTCGTGCC AGCTGCATTA
1921 ATGAATCGGC CAACGCGCGG GGAGAGGCGG TTTGCGTATT GGGCGCTCTT CCGCTTCCTC
1981 GCTCACTGAC TCGCTGCGCT CGGTCGTTCG GCTGCGGCGA GCGGTATCAG CTCACTCAAA
2041 GGCGGTAATA CGGTTATCCA CAGAATCAGG GGATAACGCA GGAAAGAACA TGTGAGCAAA
2101 AGGCCAGCAA AAGGCCAGGA ACCGTAAAAA GGCCGCGTTG CTGGCGTTTT TCCATAGGCT
2161 CCGCCCCCCT GACGAGCATC ACAAAAATCG ACGCTCAAGT CAGAGGTGGC GAAACCCGAC
2221 AGGACTATAA AGATACCAGG CGTTTCCCCC TGGAAGCTCC CTCGTGCGCT CTCCTGTTCC
2281 GACCCTGCCG CTTACCGGAT ACCTGTCCGC CTTTCTCCCT TCGGGAAGCG TGGCGCTTTC
2341 TCATAGCTCA CGCTGTAGGT ATCTCAGTTC GGTGTAGGTC GTTCGCTCCA AGCTGGGCTG
2401 TGTGCACGAA CCCCCCGTTC AGCCCGACCG CTGCGCCTTA TCCGGTAACT ATCGTCTTGA
2461 GTCCAACCCG GTAAGACACG ACTTATCGCC ACTGGCAGCA GCCACTGGTA ACAGGATTAG
2521 CAGAGCGAGG TATGTAGGCG GTGCTACAGA GTTCTTGAAG TGGTGGCCTA ACTACGGCTA
2581 CACTAGAAGG ACAGTATTTG GTATCTGCGC TCTGCTGAAG CCAGTTACCT TCGGAAAAAG
2641 AGTTGGTAGC TCTTGATCCG GCAAACAAAC CACCGCTGGT AGCGGTGGTT TTTTTGTTTG
2701 CAAGCAGCAG ATTACGCGCA GAAAAAAAGG ATCTCAAGAA GATCCTTTGA TCTTTTCTAC
2761 GGGGTCTGAC GCTCAGTGGA ACGAAAACTC ACGTTAAGGG ATTTTGGTCA TGAGATTATC
2821 AAAAAGGATC TTCACCTAGA TCCTTTTAAA TTAAAAATGA AGTTTTAAAT CAATCTAAAG

Stop bla
2881 TATATATGAG TAAACTTGGT CTGACAGTTA CCAATGCTTA ATCAGTGAGG CACCTATCTC

2941 AGCGATCTGT CTATTTCGTT CATCCATAGT TGCCTGACTC CCCGTCGTGT AGATAACTAC
3001 GATACGGGAG GGCTTACCAT CTGGCCCCAG TGCTGCAATG ATACCGCGAG ACCCACGCTC
3061 ACCGGCTCCA GATTTATCAG CAATAAACCA GCCAGCCGGA AGGGCCGAGC GCAGAAGTGG
3121 TCCTGCAACT TTATCCGCCT CCATCCAGTC TATTAATTGT TGCCGGGAAG CTAGAGTAAG
3181 TAGTTCGCCA GTTAATAGTT TGCGCAACGT TGTTGCCATT GCTACAGGCA TCGTGGTGTC
3241 ACGCTCGTCG TTTGGTATGG CTTCATTCAG CTCCGGTTCC CAACGATCAA GGCGAGTTAC
3301 ATGATCCCCC ATGTTGTGCA AAAAAGCGGT TAGCTCCTTC GGTCCTCCGA TCGTTGTCAG
3361 AAGTAAGTTG GCCGCAGTGT TATCACTCAT GGTTATGGCA GCACTGCATA ATTCTCTTAC
3421 TGTCATGCCA TCCGTAAGAT GCTTTTCTGT GACTGGTGAG TACTCAACCA AGTCATTCTG
3481 AGAATAGTGT ATGCGGCGAC CGAGTTGCTC TTGCCCGGCG TCAATACGGG ATAATACCGC
3541 GCCACATAGC AGAACTTTAA AAGTGCTCAT CATTGGAAAA CGTTCTTCGG GGCGAAAACT
3601 CTCAAGGATC TTACCGCTGT TGAGATCCAG TTCGATGTAA CCCACTCGTG CACCCAACTG
3661 ATCTTCAGCA TCTTTTACTT TCACCAGCGT TTCTGGGTGA GCAAAAACAG GAAGGCAAAA

Start bla
3721 TGCCGCAAAA AAGGGAATAA GGGCGACACG GAAATGTTGA ATACTCATAC TCTTCCTTTT
←

3781 TCAATATTAT TGAAGCATTT ATCAGGGTTA TTGTCTCATG AGCGGATACA TATTTGAATG
3841 TATTTAGAAA AATAAACAAA TAGGGGTTCC GCGCACATTT CCCCGAAAAG TGCCACCTGA

3901 CGTCTAAGAA ACCATTATTA TCATGACATT AACCTATAAA AATAGGCGTA TCACGAGGCC
3961 CTTTCGTC

The segment *uidR control region* through the Puid promotor region was taken from the *E*. *coli* MG1655 chromosome using PCR-added *Hin*dIII and *Pst*I restriciton sites. This fragment was cut with *Hin*dIII and *Pst*I and cloned into pUC-18 cut with the same enzymes. Underlined sequence constitutes the *uidR* regulatory region while the italicized sequence constitutes the protein to be expressed promotor region under the control of *uidR.*

### SEQ ID NO 11

### pMPX-8 expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

Stop melR
361 TTTCCCAGTC ACGACGTTGT AAAACGACGG CCAGTGCCAA GCTTTTAGCC GGGAAACGTC

421 TGGCGGCGCT GTTGGCTAAG TTTGCGGTAT TGTTGCGGCG ACATGCCGAC ATATTTGCCG
481 AACGTGCTGT AAAAACGACT ACTTGAACGA AAGCCTGCCG TCAGGGCAAT ATCGAGAATA
541 CTTTTATCGG TATCGCTCAG TAACGCGCGA ACGTGGTTGA TGCGCATCGC GGTAATGTAC
601 TGTTTCATCG TCAATTGCAT GACCCGCTGG AATATCCCCA TTGCATAGTT GGCGTTAAGT
661 TTGACGTGCT CAGCCACATC GTTGATGGTC AGCGCCTGAT CATAGTTTTC GGCAATAAAG
721 CCCAGCATCT GGCTAACATA AAATTGCGCA TGGCGCGAGA CGCTGTTTTT GTGTGTGCGC
781 GAGGTTTTAT TGACCAGAAT CGGTTCCCAG CCAGAGAGGC TAAATCGCTT GAGCATCAGG
841 CCAATTTCAT CAATGGCGAG CTGGCGAATT TGCTCGTTCG GACTGTTTAA TTCCTGCTGC
901 CAGCGGCGCA CTTCAAACGG GCTAAGTTGC TGTGTGGCCA GTGATTTGAT CACCATGCCG
961 TGAGTGACGT GGTTAATCAG GTCTTTATCC AGCGGCCAGG AGAGAAACAG ATGCATCGGC
1021 AGATTAAAAA TCGCCATGCT CTGACAGGTT CCGGTATCTG TTAGTTGGTG CGGTGTACAG
1081 GCCCAGAACA GCGTGATATG ACCCTGATTG ATATTCACTT TTTCATTGTT GATCAGGTAT
1141 TCCACATCGC CATCGAAAGG CACATTCACT TCGACCTGAC CATGCCAGTG GCTGGTGGGC
1201 ATGATATGCG GTGCGCGAAA CTCAATCTCC ATCCGCTGGT ATTCCGAATA CAGCGACAGC

Start melR
1261 GGGCTGCGGG TCTGTTTTTC GTCGCTGCTG CACATAAACG TATCTGTATT CATGGATGGC
←

1321 TCTCTTTCCT GGAATATCAG AATTATGGCA GGAGTGAGGG AGGATGACTG CGAGTGGGAG
1381 CACGGTTTTC ACCCTCTTCC CAGAGGGGCG AGGGGACTCT CCGAGTATCA TGAGGCCGAA
1441 AACTCTGCTT TTCAGGTAAT TTATTCCCAT AAACTCAGAT TTACTGCTGC TTCACGCAGG

Shine-Delgarno PstI
1501 ATCTGAGTTT ATGGGAATGC TCAACCTGGA AGCCGGAGGT TTTCTGCAGA TTCGCCTGCC

SalI XbaI BamHI
1561 ATGATGAAGT TATTCAAGCA AGCCAGGAGA TCTGGTACCC GGGTCGACTC TAGAGGATCC

KpnI
1621 CCGGGTACCG AGCTCGAATT CGTAATCATG GTCATAGCTG TTTCCTGTGT GAAATTGTTA
1681 TCCGCTCACA ATTCCACACA ACATACGAGC CGGAAGCATA AAGTGTAAAG CCTGGGGTGC
1741 CTAATGAGTG AGCTAACTCA CATTAATTGC GTTGCGCTCA CTGCCCGCTT TCCAGTCGGG
1801 AAACCTGTCG TGCCAGCTGC ATTAATGAAT CGGCCAACGC GCGGGGAGAG GCGGTTTGCG
1861 TATTGGGCGC TCTTCCGCTT CCTCGCTCAC TGACTCGCTG CGCTCGGTCG TTCGGCTGCG

1921 GCGAGCGGTA TCAGCTCACT CAAAGGCGGT AATACGGTTA TCCACAGAAT CAGGGGATAA
1981 CGCAGGAAAG AACATGTGAG CAAAAGGCCA GCAAAAGGCC AGGAACCGTA AAAAGGCCGC
2041 GTTGCTGGCG TTTTTCCATA GGCTCCGCCC CCCTGACGAG CATCACAAAA ATCGACGCTC
2101 AAGTCAGAGG TGGCGAAACC CGACAGGACT ATAAAGATAC CAGGCGTTTC CCCCTGGAAG
2161 CTCCCTCGTG CGCTCTCCTG TTCCGACCCT GCCGCTTACC GGATACCTGT CCGCCTTTCT
2221 CCCTTCGGGA AGCGTGGCGC TTTCTCATAG CTCACGCTGT AGGTATCTCA GTTCGGTGTA
2281 GGTCGTTCGC TCCAAGCTGG GCTGTGTGCA CGAACCCCCC GTTCAGCCCG ACCGCTGCGC
2341 CTTATCCGGT AACTATCGTC TTGAGTCCAA CCCGGTAAGA CACGACTTAT CGCCACTGGC
2401 AGCAGCCACT GGTAACAGGA TTAGCAGAGC GAGGTATGTA GGCGGTGCTA CAGAGTTCTT
2461 GAAGTGGTGG CCTAACTACG GCTACACTAG AAGGACAGTA TTTGGTATCT GCGCTCTGCT
2521 GAAGCCAGTT ACCTTCGGAA AAAGAGTTGG TAGCTCTTGA TCCGGCAAAC AAACCACCGC
2581 TGGTAGCGGT GGTTTTTTTG TTTGCAAGCA GCAGATTACG CGCAGAAAAA AAGGATCTCA
2641 AGAAGATCCT TTGATCTTTT CTACGGGGTC TGACGCTCAG TGGAACGAAA ACTCACGTTA
2701 AGGGATTTTG GTCATGAGAT TATCAAAAAG GATCTTCACC TAGATCCTTT TAAATTAAAA

Stop bla
2761 ATGAAGTTTT AAATCAATCT AAAGTATATA TGAGTAAACT TGGTCTGACA GTTACCAATG

2821 CTTAATCAGT GAGGCACCTA TCTCAGCGAT CTGTCTATTT CGTTCATCCA TAGTTGCCTG
2881 ACTCCCCGTC GTGTAGATAA CTACGATACG GGAGGGCTTA CCATCTGGCC CCAGTGCTGC
2941 AATGATACCG CGAGACCCAC GCTCACCGGC TCCAGATTTA TCAGCAATAA ACCAGCCAGC
3001 CGGAAGGGCC GAGCGCAGAA GTGGTCCTGC AACTTTATCC GCCTCCATCC AGTCTATTAA
3061 TTGTTGCCGG GAAGCTAGAG TAAGTAGTTC GCCAGTTAAT AGTTTGCGCA ACGTTGTTGC
3121 CATTGCTACA GGCATCGTGG TGTCACGCTC GTCGTTTGGT ATGGCTTCAT TCAGCTCCGG
3181 TTCCCAACGA TCAAGGCGAG TTACATGATC CCCCATGTTG TGCAAAAAAG CGGTTAGCTC
3241 CTTCGGTCCT CCGATCGTTG TCAGAAGTAA GTTGGCCGCA GTGTTATCAC TCATGGTTAT
3301 GGCAGCACTG CATAATTCTC TTACTGTCAT GCCATCCGTA AGATGCTTTT CTGTGACTGG
3361 TGAGTACTCA ACCAAGTCAT TCTGAGAATA GTGTATGCGG CGACCGAGTT GCTCTTGCCC
3421 GGCGTCAATA CGGGATAATA CCGCGCCACA TAGCAGAACT TTAAAAGTGC TCATCATTGG
3481 AAAACGTTCT TCGGGGCGAA AACTCTCAAG GATCTTACCG CTGTTGAGAT CCAGTTCGAT
3541 GTAACCCACT CGTGCACCCA ACTGATCTTC AGCATCTTTT ACTTTCACCA GCGTTTCTGG
3601 GTGAGCAAAA ACAGGAAGGC AAAATGCCGC AAAAAAGGGA ATAAGGGCGA CACGGAAATG

Start bla
3661 TTGAATACTC ATACTCTTCC TTTTTCAATA TTATTGAAGC ATTTATCAGG GTTATTGTCT
←

3721 CATGAGCGGA TACATATTTG AATGTATTTA GAAAAATAAA CAAATAGGGG TTCCGCGCAC
3781 ATTTCCCCGA AAAGTGCCAC CTGACGTCTA AGAAACCATT ATTATCATGA CATTAACCTA
3841 TAAAAATAGG CGTATCACGA GGCCCTTTCG TC

The segment *melR* through the PmeI control region was taken from the *E*. *coli* MG1655 chromosome using PCR-added *Hin*dIII and *Pst*I restriciton sites. This fragment was cut with *Hind*III and *Pst*I and cloned into pUC-18 cut with the same enzymes. Italicized sequence constitutes both *melR* and protein to be expressed promotor region.

### SEQ ID NO 12

### pMPX-18 expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

HindIII

361 TTTCCCAGTC ACGACGTTGT AAAACGACGG CCAGTGCCAA GCTTCAAGCC GTCAATTGTC

Stop araC
421 TGATTCGTTA CCAATTATGA CAACTTGACG GCTACATCAT TCACTTTTTC TTCACAACCG

481 GCACGGAACT CGCTCGGGCT GGCCCCGGTG CATTTTTTAA ATACCCGCGA GAAATAGAGT
541 TGATCGTCAA AACCAACATT GCGACCGACG GTGGCGATAG GCATCCGGGT GGTGCTCAAA
601 AGCAGCTTCG CCTGGCTGAT ACGTTGGTCC TCGCGCCAGC TTAAGACGCT AATCCCTAAC
661 TGCTGGCGGA AAAGATGTGA CAGACGCGAC GGCGACAAGC AAACATGCTG TGCGACGCTG
721 GCGATATCAA AATTGCTGTC TGCCAGGTGA TCGCTGATGT ACTGACAAGC CTCGCGTACC
781 CGATTATCCA TCGGTGGATG GAGCGACTCG TTAATCGCTT CCATGCGCCG CAGTAACAAT
841 TGCTCAAGCA GATTTATCGC CAGCAGCTCC GAATAGCGCC CTTCCCCTTG CCCGGCGTTA
901 ATGATTTGCC CAAACAGGTC GCTGAAATGC GGCTGGTGCG CTTCATCCGG GCGAAAGAAC
961 CCCGTATTGG CAAATATTGA CGGCCAGTTA AGCCATTCAT GCCAGTAGGC GCGCGGACGA
1021 AAGTAAACCC ACTGGTGATA CCATTCGCGA GCCTCCGGAT GACGACCGTA GTGATGAATC
1081 TCTCCTGGCG GGAACAGCAA AATATCACCC GGTCGGCAAA CAAATTCTCG TCCCTGATTT
1141 TTCACCACCC CCTGACCGCG AATGGTGAGA TTGAGAATAT AACCTTTCAT TCCCAGCGGT
1201 CGGTCGATAA AAAAATCGAG ATAACCGTTG GCCTCAATCG GCGTTAAACC CGCCACCAGA
1261 TGGGCATTAA ACGAGTATCC CGGCAGCAGG GGATCATTTT GCGCTTCAGC CATACTTTTC

Start araC
1321 ATACTCCCGC CATTCAGAGA AGAAACCAAT TGTCCATATT GCATCAGACA TTGCCGTCAC
←

1381 TGCGTCTTTT ACTGGCTCTT CTCGCTAACC AAACCGGTAA CCCCGCTTAT TAAAAGCATT
1441 CTGTAACAAA GCGGGACCAA AGCCATGACA AAAACGCGTA ACAAAAGTGT CTATAATCAC
1501 GGCAGAAAAG TCCACATTGA TTATTTGCAC GGCGTCACAC TTTGCTATGC CATAGCATTT
1561 TTATCCATAA GATTAGCGGA TCCTACCTGA CGCTTTTTAT CGCAACTCTC TACTGTTTCT

Shine-Delgarno PstI SalI XbaI
1621 CCATACCCGT TTTTTTGGGC TAGCAGGAGG AATTCACCCT GCAGGTCGAC TCTAGAGGAT
→

XmaI KpnI
1681 CCCCGGGTAC CGAGCTCGAA TTCGTAATCA TGGTCATAGC TGTTTCCTGT GTGAAATTGT
1741 TATCCGCTCA CAATTCCACA CAACATACGA GCCGGAAGCA TAAAGTGTAA AGCCTGGGGT
1801 GCCTAATGAG TGAGCTAACT CACATTAATT GCGTTGCGCT CACTGCCCGC TTTCCAGTCG
1861 GGAAACCTGT CGTGCCAGCT GCATTAATGA ATCGGCCAAC GCGCGGGGAG AGGCGGTTTG
1921 CGTATTGGGC GCTCTTCCGC TTCCTCGCTC ACTGACTCGC TGCGCTCGGT CGTTCGGCTG
1981 CGGCGAGCGG TATCAGCTCA CTCAAAGGCG GTAATACGGT TATCCACAGA ATCAGGGGAT
2041 AACGCAGGAA AGAACATGTG AGCAAAAGGC CAGCAAAAGG CCAGGAACCG TAAAAAGGCC
2101 GCGTTGCTGG CGTTTTTCCA TAGGCTCCGC CCCCCTGACG AGCATCACAA AAATCGACGC
2161 TCAAGTCAGA GGTGGCGAAA CCCGACAGGA CTATAAAGAT ACCAGGCGTT TCCCCCTGGA
2221 AGCTCCCTCG TGCGCTCTCC TGTTCCGACC CTGCCGCTTA CCGGATACCT GTCCGCCTTT
2281 CTCCCTTCGG GAAGCGTGGC GCTTTCTCAT AGCTCACGCT GTAGGTATCT CAGTTCGGTG
2341 TAGGTCGTTC GCTCCAAGCT GGGCTGTGTG CACGAACCCC CCGTTCAGCC CGACCGCTGC
2401 GCCTTATCCG GTAACTATCG TCTTGAGTCC AACCCGGTAA GACACGACTT ATCGCCACTG
2461 GCAGCAGCCA CTGGTAACAG GATTAGCAGA GCGAGGTATG TAGGCGGTGC TACAGAGTTC
2521 TTGAAGTGGT GGCCTAACTA CGGCTACACT AGAAGGACAG TATTTGGTAT CTGCGCTCTG
2581 CTGAAGCCAG TTACCTTCGG AAAAAGAGTT GGTAGCTCTT GATCCGGCAA ACAAACCACC
2641 GCTGGTAGCG GTGGTTTTTT TGTTTGCAAG CAGCAGATTA CGCGCAGAAA AAAAGGATCT
2701 CAAGAAGATC CTTTGATCTT TTCTACGGGG TCTGACGCTC AGTGGAACGA AAACTCACGT
2761 TAAGGGATTT TGGTCATGAG ATTATCAAAA AGGATCTTCA CCTAGATCCT TTTAAATTAA

Start bla
2821 AAATGAAGTT TTAAATCAAT CTAAAGTATA TATGAGTAAA CTTGGTCTGA CAGTTACCAA

2881 TGCTTAATCA GTGAGGCACC TATCTCAGCG ATCTGTCTAT TTCGTTCATC CATAGTTGCC
2941 TGACTCCCCG TCGTGTAGAT AACTACGATA CGGGAGGGCT TACCATCTGG CCCCAGTGCT
3001 GCAATGATAC CGCGAGACCC ACGCTCACCG GCTCCAGATT TATCAGCAAT AAACCAGCCA

3061 GCCGGAAGGG CCGAGCGCAG AAGTGGTCCT GCAACTTTAT CCGCCTCCAT CCAGTCTATT
3121 AATTGTTGCC GGGAAGCTAG AGTAAGTAGT TCGCCAGTTA ATAGTTTGCG CAACGTTGTT
3181 GCCATTGCTA CAGGCATCGT GGTGTCACGC TCGTCGTTTG GTATGGCTTC ATTCAGCTCC
3241 GGTTCCCAAC GATCAAGGCG AGTTACATGA TCCCCCATGT TGTGCAAAAA AGCGGTTAGC
3301 TCCTTCGGTC CTCCGATCGT TGTCAGAAGT AAGTTGGCCG CAGTGTTATC ACTCATGGTT
3361 ATGGCAGCAC TGCATAATTC TCTTACTGTC ATGCCATCCG TAAGATGCTT TTCTGTGACT
3421 GGTGAGTACT CAACCAAGTC ATTCTGAGAA TAGTGTATGC GGCGACCGAG TTGCTCTTGC
3481 CCGGCGTCAA TACGGGATAA TACCGCGCCA CATAGCAGAA CTTTAAAAGT GCTCATCATT
3541 GGAAAACGTT CTTCGGGGCG AAAACTCTCA AGGATCTTAC CGCTGTTGAG ATCCAGTTCG
3601 ATGTAACCCA CTCGTGCACC CAACTGATCT TCAGCATCTT TTACTTTCAC CAGCGTTTCT
3661 GGGTGAGCAA AAACAGGAAG GCAAAATGCC GCAAAAAAGG GAATAAGGGC GACACGGAAA

Start bla
3721 TGTTGAATAC TCATACTCTT CCTTTTTCAA TATTATTGAA GCATTTATCA GGGTTATTGT
←

3781 CTCATGAGCG GATACATATT TGAATGTATT TAGAAAAATA AACAAATAGG GGTTCCGCGC
3841 ACATTTCCCC GAAAAGTGCC ACCTGACGTC TAAGAAACCA TTATTATCAT GACATTAACC
3901 TATAAAAATA GGCGTATCAC GAGGCCCTTT CGTC

The segment *araC* through the Para control region was taken from pBAD24 using PCR-added *Hin*dIII and *Pst*I restriciton sites. This fragment was cut with *Hin*dIII and *Pst*I and cloned into pUC-18 cut with the same enzymes. Italicized sequence constitutes both *araC* and protein to be expressed promotor region.

### SEO ID NO 13

### pMPX-6 expression vector

1 TAGTTATTAA TAGTAATCAA TTACGGGGTC ATTAGTTCAT AGCCCATATA TGGAGTTCCG
61 CGTTACATAA CTTACGGTAA ATGGCCCGCC TGGCTGACCG CCCAACGACC CCCGCCCATT
121 GACGTCAATA ATGACGTATG TTCCCATAGT AACGCCAATA GGGACTTTCC ATTGACGTCA
181 ATGGGTGGAG TATTTACGGT AAACTGCCCA CTTGGCAGTA CATCAAGTGT ATCATATGCC
241 AAGTACGCCC CCTATTGACG TCAATGACGG TAAATGGCCC GCCTGGCATT ATGCCCAGTA
301 CATGACCTTA TGGGACTTTC CTACTTGGCA GTACATCTAC GTATTAGTCA TCGCTATTAC
361 CATGGTGATG CGGTTTTGGC AGTACATCAA TGGGCGTGGA TAGCGGTTTG ACTCACGGGG
421 ATTTCCAAGT CTCCACCCCA TTGACGTCAA TGGGAGTTTG TTTTGGCACC AAAATCAACG
481 GGACTTTCCA AAATGTCGTA ACAACTCCGC CCCATTGACG CAAATGGGCG GTAGGCGTGT
541 ACGGTGGGAG GTCTATATAA GCAGAGCTGG TTTAGTGAAC CGTCAGATCC GCTAGCGCTA

Start GFP
601 CCGGTCGCCA CCATGGTGAG CAAGGGCGAG GAGCTGTTCA CCGGGGTGGT GCCCATCCTG
→

661 GTCGAGCTGG ACGGCGACGT AAACGGCCAC AAGTTCAGCG TGTCCGGCGA GGGCGAGGGC
721 GATGCCACCT ACGGCAAGCT GACCCTGAAG TTCATCTGCA CCACCGGCAA GCTGCCCGTG
781 CCCTGGCCCA CCCTCGTGAC CACCCTGACC TACGGCGTGC AGTGCTTCAG CCGCTACCCC
841 GACCACATGA AGCAGCACGA CTTCTTCAAG TCCGCCATGC CCGAAGGCTA CGTCCAGGAG
901 CGCACCATCT TCTTCAAGGA CGACGGCAAC TACAAGACCC GCGCCGAGGT GAAGTTCGAG
961 GGCGACACCC TGGTGAACCG CATCGAGCTG AAGGGCATCG ACTTCAAGGA GGACGGCAAC
1021 ATCCTGGGGC ACAAGCTGGA GTACAACTAC AACAGCCACA ACGTCTATAT CATGGCCGAC
1081 AAGCAGAAGA ACGGCATCAA GGTGAACTTC AAGATCCGCC ACAACATCGA GGACGGCAGC
1141 GTGCAGCTCG CCGACCACTA CCAGCAGAAC ACCCCCATCG GCGACGGCCC CGTGCTGCTG
1201 CCCGACAACC ACTACCTGAG CACCCAGTCC GCCCTGAGCA AAGACCCCAA CGAGAAGCGC
1261 GATCACATGG TCCTGCTGGA GTTCGTGACC GCCGCCGGGA TCACTCTCGG CATGGACGAG

XhoI Stop GFP

1321 CTGTACAAGT CCGGACTCAG ATCTCGAGCT TAATAACAAG CCGTCAATTG TCTGATTCGT

Stop araC
1381 TACCAATTAT GACAACTTGA CGGCTACATC ATTCACTTTT TCTTCACAAC CGGCACGGAA

1441 CTCGCTCGGG CTGGCCCCGG TGCATTTTTT AAATACCCGC GAGAAATAGA GTTGATCGTC
1501 AAAACCAACA TTGCGACCGA CGGTGGCGAT AGGCATCCGG GTGGTGCTCA AAAGCAGCTT
1561 CGCCTGGCTG ATACGTTGGT CCTCGCGCCA GCTTAAGACG CTAATCCCTA ACTGCTGGCG
1621 GAAAAGATGT GACAGACGCG ACGGCGACAA GCAAACATGC TGTGCGACGC TGGCGATATC
1681 AAAATTGCTG TCTGCCAGGT GATCGCTGAT GTACTGACAA GCCTCGCGTA CCCGATTATC
1741 CATCGGTGGA TGGAGCGACT CGTTAATCGC TTCCATGCGC CGCAGTAACA ATTGCTCAAG
1801 CAGATTTATC GCCAGCAGCT CCGAATAGCG CCCTTCCCCT TGCCCGGCGT TAATGATTTG
1861 CCCAAACAGG TCGCTGAAAT GCGGCTGGTG CGCTTCATCC GGGCGAAAGA ACCCCGTATT
1921 GGCAAATATT GACGGCCAGT TAAGCCATTC ATGCCAGTAG GCGCGCGGAC GAAAGTAAAC
1981 CCACTGGTGA TACCATTCGC GAGCCTCCGG ATGACGACCG TAGTGATGAA TCTCTCCTGG
2041 CGGGAACAGC AAAATATCAC CCGGTCGGCA AACAAATTCT CGTCCCTGAT TTTTCACCAC
2101 CCCCTGACCG CGAATGGTGA GATTGAGAAT ATAACCTTTC ATTCCCAGCG GTCGGTCGAT
2161 AAAAAAATCG AGATAACCGT TGGCCTCAAT CGGCGTTAAA CCCGCCACCA GATGGGCATT
2221 AAACGAGTAT CCCGGCAGCA GGGGATCATT TTGCGCTTCA GCCATACTTT TCATACTCCC

Start araC
2281 GCCATTCAGA GAAGAAACCA ATTGTCCATA TTGCATCAGA CATTGCCGTC ACTGCGTCTT
←
2341 TTACTGGCTC TTCTCGCTAA CCAAACCGGT AACCCCGCTT ATTAAAAGCA TTCTGTAACA
2401 AAGCGGGACC AAAGCCATGA CAAAAACGCG TAACAAAAGT GTCTATAATC ACGGCAGAAA
2461 AGTCCACATT GATTATTTGC ACGGCGTCAC ACTTTGCTAT GCCATAGCAT TTTTATCCAT
2521 AAGATTAGCG GATCCTACCT GACGCTTTTT ATCGCAACTC TCTACTGTTT CTCCATACCC

→ EcoRI KpnI SalI
2581 GTTTTTTTGG GCTAGCAGGA GGAATTCACC ATGGTACCCG GGGATCCTCT AGAGTCGACC
Shine-Delgarno

PstI HindIII SstII
2641 TGCAGGCATG CAAGCTTGGC CCGCGGGCCC GGGATCCACC GGATCTAGAT AACTGATCAT

2701 AATCAGCCAT ACCACATTTG TAGAGGTTTT ACTTGCTTTA AAAAACCTCC CACACCTCCC
2761 CCTGAACCTG AAACATAAAA TGAATGCAAT TGTTGTTGTT AACTTGTTTA TTGCAGCTTA
2821 TAATGGTTAC AAATAAAGCA ATAGCATCAC AAATTTCACA AATAAAGCAT TTTTTTCACT
2881 GCATTCTAGT TGTGGTTTGT CCAAACTCAT CAATGTATCT TAACGCGTAA ATTGTAAGCG
2941 TTAATATTTT GTTAAAATTC GCGTTAAATT TTTGTTAAAT CAGCTCATTT TTTAACCAAT
3001 AGGCCGAAAT CGGCAAAATC CCTTATAAAT CAAAAGAATA GACCGAGATA GGGTTGAGTG
3061 TTGTTCCAGT TTGGAACAAG AGTCCACTAT TAAAGAACGT GGACTCCAAC GTCAAAGGGC
3121 GAAAAACCGT CTATCAGGGC GATGGCCCAC TACGTGAACC ATCACCCTAA TCAAGTTTTT
3181 TGGGGTCGAG GTGCCGTAAA GCACTAAATC GGAACCCTAA AGGGAGCCCC CGATTTAGAG
3241 CTTGACGGGG AAAGCCGGCG AACGTGGCGA GAAAGGAAGG GAAGAAAGCG AAAGGAGCGG
3301 GCGCTAGGGC GCTGGCAAGT GTAGCGGTCA CGCTGCGCGT AACCACCACA CCCGCCGCGC
3361 TTAATGCGCC GCTACAGGGC GCGTCAGGTG GCACTTTTCG GGGAAATGTG CGCGGAACCC
3421 CTATTTGTTT ATTTTTCTAA ATACATTCAA ATATGTATCC GCTCATGAGA CAATAACCCT
3481 GATAAATGCT TCAATAATAT TGAAAAAGGA AGAGTCCTGA GGCGGAAAGA ACCAGCTGTG
3541 GAATGTGTGT CAGTTAGGGT GTGGAAAGTC CCCAGGCTCC CCAGCAGGCA GAAGTATGCA
3601 AAGCATGCAT CTCAATTAGT CAGCAACCAG GTGTGGAAAG TCCCCAGGCT CCCCAGCAGG
3661 CAGAAGTATG CAAAGCATGC ATCTCAATTA GTCAGCAACC ATAGTCCCGC CCCTAACTCC
3721 GCCCATCCCG CCCCTAACTC CGCCCAGTTC CGCCCATTCT CCGCCCCATG GCTGACTAAT
3781 TTTTTTTATT TATGCAGAGG CCGAGGCCGC CTCGGCCTCT GAGCTATTCC AGAAGTAGTG
3841 AGGAGGCTTT TTTGGAGGCC TAGGCTTTTG CAAAGATCGA TCAAGAGACA GGATGAGGAT

Start Kan
3901 CGTTTCGCAT GATTGAACAA GATGGATTGC ACGCAGGTTC TCCGGCCGCT TGGGTGGAGA
→

3961 GGCTATTCGG CTATGACTGG GCACAACAGA CAATCGGCTG CTCTGATGCC GCCGTGTTCC
4021 GGCTGTCAGC GCAGGGGCGC CCGGTTCTTT TTGTCAAGAC CGACCTGTCC GGTGCCCTGA
4081 ATGAACTGCA AGACGAGGCA GCGCGGCTAT CGTGGCTGGC CACGACGGGC GTTCCTTGCG
4141 CAGCTGTGCT CGACGTTGTC ACTGAAGCGG GAAGGGACTG GCTGCTATTG GGCGAAGTGC
4201 CGGGGCAGGA TCTCCTGTCA TCTCACCTTG CTCCTGCCGA GAAAGTATCC ATCATGGCTG
4261 ATGCAATGCG GCGGCTGCAT ACGCTTGATC CGGCTACCTG CCCATTCGAC CACCAAGCGA
4321 AACATCGCAT CGAGCGAGCA CGTACTCGGA TGGAAGCCGG TCTTGTCGAT CAGGATGATC
4381 TGGACGAAGA GCATCAGGGG CTCGCGCCAG CCGAACTGTT CGCCAGGCTC AAGGCGAGCA
4441 TGCCCGACGG CGAGGATCTC GTCGTGACCC ATGGCGATGC CTGCTTGCCG AATATCATGG
4501 TGGAAAATGG CCGCTTTTCT GGATTCATCG ACTGTGGCCG GCTGGGTGTG GCGGACCGCT
4561 ATCAGGACAT AGCGTTGGCT ACCCGTGATA TTGCTGAAGA GCTTGGCGGC GAATGGGCTG
4621 ACCGCTTCCT CGTGCTTTAC GGTATCGCCG CTCCCGATTC GCAGCGCATC GCCTTCTATC

Stop Kan
4681 GCCTTCTTGA CGAGTTCTTC TGAGCGGGAC TCTGGGGTTC GAAATGACCG ACCAAGCGAC

4741 GCCCAACCTG CCATCACGAG ATTTCGATTC CACCGCCGCC TTCTATGAAA GGTTGGGCTT
4801 CGGAATCGTT TTCCGGGACG CCGGCTGGAT GATCCTCCAG CGCGGGGATC TCATGCTGGA
4861 GTTCTTCGCC CACCCTAGGG GGAGGCTAAC TGAAACACGG AAGGAGACAA TACCGGAAGG
4921 AACCCGCGCT ATGACGGCAA TAAAAAGACA GAATAAAACG CACGGTGTTG GGTCGTTTGT
4981 TCATAAACGC GGGGTTCGGT CCCAGGGCTG GCACTCTGTC GATACCCCAC CGAGACCCCA
5041 TTGGGGCCAA TACGCCCGCG TTTCTTCCTT TTCCCCACCC CACCCCCCAA GTTCGGGTGA
5101 AGGCCCAGGG CTCGCAGCCA ACGTCGGGGC GGCAGGCCCT GCCATAGCCT CAGGTTACTC
5161 ATATATACTT TAGATTGATT TAAAACTTCA TTTTTAATTT AAAAGGATCT AGGTGAAGAT
5221 CCTTTTTGAT AATCTCATGA CCAAAATCCC TTAACGTGAG TTTTCGTTCC ACTGAGCGTC
5281 AGACCCCGTA GAAAAGATCA AAGGATCTTC TTGAGATCCT TTTTTTCTGC GCGTAATCTG
5341 CTGCTTGCAA ACAAAAAAAC CACCGCTACC AGCGGTGGTT TGTTTGCCGG ATCAAGAGCT
5401 ACCAACTCTT TTTCCGAAGG TAACTGGCTT CAGCAGAGCG CAGATACCAA ATACTGTCCT
5461 TCTAGTGTAG CCGTAGTTAG GCCACCACTT CAAGAACTCT GTAGCACCGC CTACATACCT
5521 CGCTCTGCTA ATCCTGTTAC CAGTGGCTGC TGCCAGTGGC GATAAGTCGT GTCTTACCGG
5581 GTTGGACTCA AGACGATAGT TACCGGATAA GGCGCAGCGG TCGGGCTGAA CGGGGGGTTC
5641 GTGCACACAG CCCAGCTTGG AGCGAACGAC CTACACCGAA CTGAGATACC TACAGCGTGA
5701 GCTATGAGAA AGCGCCACGC TTCCCGAAGG GAGAAAGGCG GACAGGTATC CGGTAAGCGG
5761 CAGGGTCGGA ACAGGAGAGC GCACGAGGGA GCTTCCAGGG GGAAACGCCT GGTATCTTTA
5821 TAGTCCTGTC GGGTTTCGCC ACCTCTGACT TGAGCGTCGA TTTTTGTGAT GCTCGTCAGG
5881 GGGGCGGAGC CTATGGAAAA ACGCCAGCAA CGCGGCCTTT TTACGGTTCC TGGCCTTTTG
5941 CTGGCCTTTT GCTCACATGT TCTTTCCTGC GTTATCCCCT GATTCTGTGG ATAACCGTAT
6001 TACCGCCATG CAT

The segment araC through *Sstl*I following the Para control region was taken from pBAD24 using a PCR-added *Xho*I restriciton site. This fragment was cut with *Xho*I and *Sstl*I and cloned into pEGFP-C1 (Clontech) cut with the same enzymes. Italicized and underlined sequence constitutes the CMV promotor region while the italicized alone region constitutes both the *araC* and protein to be expressed promotor region.

### SEQ ID NO 14

pMPX-56 (rat Edg3 cloned into pMPX-5 using PCR-introduced SalI and KpnI)

Shine-Delgarno
2401 GAATTCAGGCGCTTTTTAGACTGGTCGTAATGAAATTCAGCAGGATCACATTCTGCAGGT
SalI

2461 CGACATGGCAACCACGCACGCGCAGGGCCACCCGCCAGTCTTGGGGAATGATACTCTCCG
1 M A T T H A Q G H P P V L G N D T L R

2521 GGAACATTATGATTACGTGGGGAAGCTGGCAGGCAGGCTGCGGGATCCCCCTGAGGGTAG
20 E H Y D Y V G K L A G R L R D P P E G S

2581 CACCCTCATCACCACCATCCTCTTCTTGGTCACCTGTAGCTTCATCGTCTTGGAGAACCT
40 T L I T T I L F L V T C S F I V L E N L

2641 GATGGTTTTGATTGCCATCTGGAAAAACAATAAATTTCATAACCGCATGTACTTTTTCAT
60 M V L I A I W K N N K F H N R M Y F F I

2701 CGGCAACTTGGCTCTCTGCGACCTGCTGGCCGGCATAGCCTACAAGGTCAACATTCTGAT
80 G N L A L C D L L A G I A Y K V N I L M

2761 GTCCGGTAGGAAGACGTTCAGCCTGTCTCCAACAGTGTGGTTCCTCAGGGAGGGCAGTAT
100 S G R K T F S L S P T V W F L R E G S M

2821 GTTCGTAGCCCTGGGCGCATCCACATGCAGCTTATTGGCCATTGCCATTGAGCGGCACCT
120 F V A L G A S T C S L L A I A I E R H L

2881 GACCATGATCAAGATGAGGCCGTACGACGCCAACAAGAAGCACCGCGTGTTCCTTCTGAT
140 T M I K M R P Y D A N K K H R V F L L I

2941 TGGGATGTGCTGGCTAATTGCCTTCTCGCTGGGTGCCCTGCCCATCCTGGGCTGGAACTG
160 G M C W L I A F S L G A L P I L G W N C

3001 CCTGGAAAACTTTCCCGACTGCTCTACCATCTTGCCCCTCTACTCCAAGAAATACATTGC
180 L E N F P D C S T I L P L Y S K K Y I A

3061 CTTTCTCATCAGCATCTTCATAGCCATTCTGGTGACCATCGTCATCTTGTACGCGCGCAT
200 F L I S I F I A I L V T I V I L Y A R I

3121 CTACTTCCTGGTCAAGTCCAGCAGCCGCAGGGTGGCCAACCACAACTCCGAGAGATCCAT
220 Y F L V K S S S R R V A N H N S E R S M

3181 GGCCCTTCTGCGGACCGTAGTGATCGTGGTGAGCGTGTTCATCGCCTGTTGGTCCCCCCT
240 A L L R T V V I V V S V F I A C W S P L

3241 TTTCATCCTCTTCCTCATCGATGTGGCCTGCAGGGCGAAGGAGTGCTCCATCCTCTTCAA
260 F I L F L I D V A C R A K E C S I L F K

3301 GAGTCAGTGGTTCATCATGCTGGCTGTCCTCAACTCGGCCATGAACCCTGTCATCTACAC
280 S Q W F I M L A V L N S A M N P V I Y T

3361 GCTGGCCAGCAAAGAGATGCGGCGTGCTTTCTTCCGGTTGGTGTGCGGCTGTCTGGTCAA
300 L A S K E M R R A F F R L V C G C L V K

3421 GGGCAAGGGGACCCAGGCCTCCCCGATGCAGCCTGCTCTTGACCCGAGCAGAAGTAAATC
320 G K G T Q A S P M Q P A L D P S R S K S

3481 AAGCTCCAGTAACAACAGCAGCAGCCACTCTCCAAAGGTCAAGGAAGACCTGCCCCATGT
340 S S S N N S S S H S P K V K E D L P H V

3541 GGCTACCTCTTCCTGCGTTACTGACAAAACGAGGTCGCTTCAGAATGGGGTCCTCTGCAA
360 A T S S C V T D K T R S L Q N G V L C K

3601 GAAGGGCAATTCTGCAGATATCCAGCACAGTGGCGGCCGCTCGAGTCTAGAGGGCCCGCG
380 K G N S A D I Q H S G G R S S L E G P R

3661 GTTCGAAGGTAAGCCTATCCCTAACCCTCTCCTCGGTCTCGATTCTACGCGTACCGGTCA
400 F E G K P I P N P L L G L D S T R T G H

KpnI
3721 TCATCACCATCACCATTGATAAGGTACCGAGCTCGAATTCGTAATCATGGTCATAGCTGT
420 H H H H H

### SEQ ID NO 15

pMPX-57 (β2 Adrenergic receptor (β2AR) cloned into pMPX-5 using PCR-introduced SalI and BamHI)

Shine-Delgarno
2401 GAATTCAGGCGCTTTTTAGACTGGTCGTAATGAAATTCAGCAGGATCACATTCTGCAGGT

SalI
2461 CGACATGGGGCAACCCGGGAACGGCAGCGCCTTCTTGCTGGCACCCAATGGAAGCCATGC
1 M G Q P G N G S A F L L A P N G S H A

2521 GCCGGACCACGACGTCACGCAGCAAAGGGACGAGGTGTGGGTGGTGGGCATGGGCATCGT
20 P D H D V T Q Q R D E V W V V G M G I V

2581 CATGTCTCTCATCGTCCTGGCCATCGTGTTTGGCAATGTGCTGGTCATCACAGCCATTGC
40 M S L I V L A I V F G N V L V I T A I A

2641 CAAGTTCGAGCGTCTGCAGACGGTCACCAACTACTTCATCACTTCACTGGCCTGTGCTGA
60 K F E R L Q T V T N Y F I T S L A C A D

2701 TCTGGTCATGGGCCTAGCAGTGGTGCCCTTTGGGGCCGCCCATATTCTTATGAAAATGTG
80 L V M G L A V V P F G A A H I L M K M W

2761 GACTTTTGGCAACTTCTGGTGCGAGTTTTGGACTTCCATTGATGTGCTGTGCGTCACGGC
100 T F G N F W C E F W T S I D V L C V T A

2821 CAGCATTGAGACCCTGTGCGTGATCGCAGTGGATCGCTACTTTGCCATTACTTCACCTTT
120 S I E T L C V I A V D R Y F A I T S P F

2881 CAAGTACCAGAGCCTGCTGACCAAGAATAAGGCCCGGGTGATCATTCTGATGGTGTGGAT
140 K Y Q S L L T K N K A R V I I L M V W I

2941 TGTGTCAGGCCTTAYCTCCTTCTTGCCCATTCAGATGCACTGGTACAGGGCCACCCACCA
160 V S G L X S F L P I Q M H W Y R A T H Q

3001 GGAAGCCATCAACTGCTATGCCAATGAGACCTGCTGTGACTTCTTCACGAACCAAGCCTA
180 E A I N C Y A N E T C C D F F T N Q A Y

3061 TGCCATTGCCTCTTCCATCGTGTCCTTCTACGTTCCCCTGGTGATCATGGTCTTCGTCTA
200 A I A S S I V S F Y V P L V I M V F V Y

3121 CTCCAGGGTCTTTCAGGAGGCCAAAAGGCAGCTCCAGAAGATTGACAAATCTGAGGGCCG
220 S R V F Q E A K R Q L Q K I D K S E G R

3181 CTTCCATGTCCAGAACCTTAGCCAGGTGGAGCAGGATGGGCGGACGGGGCATGGACTCCG
240 F H V Q N L S Q V E Q D G R T G H G L R

3241 CAGATCTTCCAAGTTCTGCTTGAAGGAGCACAAAGCCCTCAAGACGTTAGGCATCATCAT
260 R S S K F C L K E H K A L K T L G I I M

3301 GGGCACTTTCACCCTCTGCTGGCTGCCCTTCTTCATCGTTAACATTGTGCATGTGATCCA
280 G T F T L C W L P F F I V N I V H V I Q

3361 GGATAACCTCATCCGTAAGGAAGTTTACATCCTCCTAAATTGGATAGGCTATGTCAATTC
300 D N L I R K E V Y I L L N W I G Y V N S

3421 TGGTTTCAATCCCCTTATCTACTGCCGGAGCCCAGATTTCAGGATTGCCTTCCAGGAGCT
320 G F N P L I Y C R S P D F R I A F Q E L

3481 TCTGTGCCTGCGCAGGTCTTCTTTGAAGGCCTATGGCAATGGCTACTCCAGCAACGGCAA
340 L C L R R S S L K A Y G N G Y S S N G N

3541 CACAGGGGAGCAGAGTGGATATCACGTGGAACAGGAGAAAGAAAATAAACTGCTGTGTGA
360 T G E Q S G Y H V E Q E K E N K L L C E

3601 AGACCTCCCAGGCACGGAAGACTTTGTGGGCCATCAAGGTACTGTGCCTAGCGATAACAT
380 D L P G T E D F V G H Q G T V P S D N I

BamHI
3661 TGATTCACAAGGGAGGAATTGTAGTACAAATGACTCACTGCTATAATAAGGATCCCCGGG
400 D S Q G R N C S T N D S L L

### SEQ ID NO 16

AATTGGTACC TCAATGATGA TGATGATGAT GCTTGCAGAG GACCCCATTC TG

### SEQ ID NO 17

pMPX-1 (Human tumer necrosis factor receptor (TNFR-1) residues 41-455 cloned into pBAD-24 using PCR-introduced NcoI and XbaI)

Shine-Delgarno
1261 TCGCAACTCTCTACTGTTTCTCCATACCCGTTTTTTTGGGCTAGCAGGAGGAATTCACCA

NcoI
1321 TGGATAGTGTGTGTCCCCAAGGAAAATATATCCACCCTCAAAATAATTCGATTTGCTGTA
1 M D S V C P Q G K Y I H P Q N N S I C C

1381 CCAAGTGCCACAAAGGAACCTACTTGTACAATGACTGTCCAGGCCCGGGGCAGGATACGG
21 T K C H K G T Y L Y N D C P G P G Q D T

1441 ACTGCAGGGAGTGTGAGAGCGGCTCCTTCACCGCTTCAGAAAACCACCTCAGACACTGCC
41 D C R E C E S G S F T A S E N H L R H C

1501 TCAGCTGCTCCAAATGCCGAAAGGAAATGGGTCAGGTGGAGATCTCTTCTTGCACAGTGG
61 L S C S K C R K E M G Q V E I S S C T V

1561 ACCGGGACACCGTGTGTGGCTGCAGGAAGAACCAGTACCGGCATTATTGGAGTGAAAACC
81 D R D T V C G C R K N Q Y R H Y W S E N

1621 TTTTCCAGTGCTTCAATTGCAGCCTCTGCCTCAATGGGACCGTGCACCTCTCCTGCCAGG
101 L F Q C F N C S L C L N G T V H L S C Q

1681 AGAAACAGAACACCGTGTGCACCTGCCATGCAGGTTTCTTTCTAAGAGAAAACGAGTGTG
121 E K Q N T V C T C H A G F F L R E N E C

1741 TCTCCTGTAGTAACTGTAAGAAAAGCCTGGAGTGCACGAAGTTGTGCCTACCCCAGATTG
141 V S C S N C K K S L E C T K L C L P Q I

1801 AGAATGTTAAGGGCACTGAGGACTCAGGCACCACAGTGCTGTTGCCCCTGGTCATTTTCT
161 E N V K G T E D S G T T V L L P L V I F

1861 TTGGTCTTTGCCTTTTATCCCTCCTCTTCATTGGTTTAATGTATCGCTACCAACGGTGGA
181 F G L C L L S L L F I G L M Y R Y Q R W

1921 AGTCCAAGCTCTACTCCATTGTTTGTGGGAAATCGACACCTGAAAAAGAGGGGGAGCTTG
201 K S K L Y S I V C G K S T P E K E G E L

1981 AAGGAACTACTACTAAGCCCCTGGCCCCAAACCCAAGCTTCAGTCCCACTCCAGGCTTCA
221 E G T T T K P L A P N P S F S P T P G F

2041 CCCCCACCCTGGGCTTCAGTCCCGTGCCCAGTTCCACCTTCACCTCCAGCTCCACCTATA
241 T P T L G F S P V P S S T F T S S S T Y

2101 CCCCCGGTGACTGTCCCAACTTTGCGGCTCCCCGCAGAGAGGTGGCACCACCCTATCAGG
261 T P G D C P N F A A P R R E V A P P Y Q

2161 GGGCTGACCCCATCCTTGCGACAGCCCTCGCCTCCGACCCCATCCCCAACCCCCTTCAGA
281 G A D P I L A T A L A S D P I P N P L Q

2221 AGTGGGAGGACAGCGCCCACAAGCCACAGAGCCTAGACACTGATGACCCCGCGACGCTGT
301 K W E D S A H K P Q S L D T D D P A T L

2281 ACGCCGTGGTGGAGAACGTGCCCCCGTTGCGCTGGAAGGAATTCGTGCGGCGCCTAGGGC
321 Y A V V E N V P P L R W K E F V R R L G

2341 TGAGCGACCACGAGATCGATCGGCTGGAGCTGCAGAACGGGCGCTGCCTGCGCGAGGCGC
341 L S D H E I D R L E L Q N G R C L R E A

2401 AATACAGCATGCTGGCGACCTGGAGGCGGCGCACGCCGCGGCGCGAGGCCACGCTGGAGC
361 Q Y S M L A T W R R R T P R R E A T L E

2461 TGCTGGGACGCGTGCTCCGCGACATGGACCTGCTGGGCTGCCTGGAGGACATCGAGGAGG
381 L L G R V L R D M D L L G C L E D I E E

Xbal
2521 CGCTTTGCGGCCCCGCCGCCCTCCCGCCCGCGCCCAGTCTTCTCAGATGATCTAGAGTCG
401 A L C G P A A L P P A P S L L R

### SEQ ID NO 18

pMPX-22 (Human tumer necrosis factor receptor (TNFR-1) residues 29-455 cloned into pMPX-18 using PCR-introduced SalI and KpnI)

1621 CCATACCCGTTTTTTTGGGCTAGCAGGAGGAATTCACCCTGCAGGTCGACATGGACTGG
1 M G L

1681 TCCCTCACCTAGGGGACAGGGAGAAGAGAGATAGTGTGTGTCCCCAAGGAAAATATATCC
4 V P H L G D R E K R D S V C P Q G K Y I

1741 ACCCTCAAAATAATTCGATTTGCTGTACCAAGTGCCACAAAGGAACCTACTTGTACAATG

24 H P Q N N S I C C T K C H K G T Y L Y N

1801 ACTGTCCAGGCCCGGGGCAGGATACGGACTGCAGGGAGTGTGAGAGCGGCTCCTTCACCG
44 D C P G P G Q D T D C R E C E S G S F T

1861 CTTCAGAAAACCACCTCAGACACTGCCTCAGCTGCTCCAAATGCCGAAAGGAAATGGGTC
64 A S E N H L R H C L S C S K C R K E M G

1921 AGGTGGAGATCTCTTCTTGCACAGTGGACCGGGACACCGTGTGTGGCTGCAGGAAGAACC
84 Q V E I S S C T V D R D T V C G C R K N

1981 AGTACCGGCATTATTGGAGTGAAAACCTTTTCCAGTGCTTCAATTGCAGCCTCTGCCTCA
104 Q Y R H Y W S E N L F Q C F N C S L C L

2041 ATGGGACCGTGCACCTCTCCTGCCAGGAGAAACAGAACACCGTGTGCACCTGCCATGCAG
124 N G T V H L S C Q E K Q N T V C T C H A

2101 GTTTCTTTCTAAGAGAAAACGAGTGTGTCTCCTGTAGTAACTGTAAGAAAAGCCTGGAGT
144 G F F L R E N E C V S C S N C K K S L E

2161 GCACGAAGTTGTGCCTACCCCAGATTGAGAATGTTAAGGGCACTGAGGACTCAGGCACCA
164 C T K L C L P Q I E N V K G T E D S G T

2221 CAGTGCTGTTGCCCCTGGTCATTTTCTTTGGTCTTTGCCTTTTATCCCTCCTCTTCATTG
184 T V L L P L V I F F G L C L L S L L F I

2281 GTTTAATGTATCGCTACCAACGGTGGAAGTCCAAGCTCTACTCCATTGTTTGTGGGAAAT
204 G L M Y R Y Q R W K S K L Y S I V C G K

2341 CGACACCTGAAAAAGAGGGGGAGCTTGAAGGAACTACTACTAAGCCCCTGGCCCCAAACC
224 S T P E K E G E L E G T T T K P L A P N

2401 CAAGCTTCAGTCCCACTCCAGGCTTCACCCCCACCCTGGGCTTCAGTCCCGTGCCCAGTT
244 P S F S P T P G F T P T L G F S P V P S

2461 CCACCTTCACCTCCAGCTCCACCTATACCCCCGGTGACTGTCCCAACTTTGCGGCTCCCC
264 S T F T S S S T Y T P G D C P N F A A P

2521 GCAGAGAGGTGGCACCACCCTATCAGGGGGCTGACCCCATCCTTGCGACAGCCCTCGCCT
284 R R E V A P P Y Q G A D P I L A T A L A

2581 CCGACCCCATCCCCAACCCCCTTCAGAAGTGGGAGGACAGCGCCCACAAGCCACAGAGCC
304 S D P I P N P L Q K W E D S A H K P Q S

2641 TAGACACTGATGACCCCGCGACGCTGTACGCCGTGGTGGAGAACGTGCCCCCGTTGCGCT
324 L D T D D P A T L Y A V V E N V P P L R

2701 GGAAGGAATTCGTGCGGCGCCTAGGGCTGAGCGACCACGAGATCGATCGGCTGGAGCTGC
344 W K E F V R R L G L S D H E I D R L E L

2761 AGAACGGGCGCTGCCTGCGCGAGGCGCAATACAGCATGCTGGCGACCTGGAGGCGGCGCA
364 Q N G R C L R E A Q Y S M L A T W R R R

2821 CGCCGCGGCGCGAGGCCACGCTGGAGCTGCTGGGACGCGTGCTCCGCGACATGGACCTGC
384 T P R R E A T L E L L G R V L R D M D L

2881 TGGGCTGCCTGGAGGACATCGAGGAGGCGCTTTGCGGCCCCGCCGCCCTCCCGCCCGCGC
404 L G C L E D I E E A L C G P A A L P P A

KpnI
2941 CCAGTCTTCTCAGATAATAAGGTACCGAGCTCGAATTCGTAATCATGGTCATAGCTGTTT
424 P S L L R

### SEQ ID NO 19

pMPX-40 (Human tumer necrosis factor (TNF) cloned into pMPX-6 using PCR-introduced EcoRI and Hindlll)

EcoRI
Shine-Delgarno
2581 GTTTTTTTGGGCTAGCAGGAGGAATTCATGAGCACTGAAAGCATGATCCGGGACGTGGAG
1 M S T E S M I R D V E

2641 CTGGCCGAGGAGGCGCTCCCCAAGAAGACAGGGGGGCCCCAGGGCTCCAGGCGGTGCTTG
12 L A E E A L P K K T G G P Q G S R R C L

2701 TTCCTCAGCCTCTTCTCCTTCCTGATCGTGGCAGGCGCCACCACGCTCTTCTGCCTGCTG
32 F L S L F S F L I V A G A T T L F C L L

2761 CACTTTGGAGTGATCGGCCCCCAGAGGGAAGAGTTCCCCAGGGACCTCTCTCTAATCAGC
52 H F G V I G P Q R E E F P R D L S L I S

2821 CCTCTGGCCCAGGCAGTCAGATCATCTTCTCGAACCCCGAGTGACAAGCCTGTAGCCCAT
72 P L A Q A V R S S S R T P S D K P V A H

2881 GTTGTAGCAAACCCTCAAGCTGAGGGGCAGCTCCAGTGGCTGAACCGCCGGGCCAATGCC
92 V V A N P Q A E G Q L Q W L N R R A N A

2941 CTCCTGGCCAATGGCGTGGAGCTGAGAGATAACCAGCTGGTGGTGCCATCAGAGGGCCTG
112 L L A N G V E L R D N Q L V V P S E G L

3001 TACCTCATCTACTCCCAGGTCCTCTTCAAGGGCCAAGGCTGCCCCTCCACCCATGTGCTC
132 Y L I Y S Q V L F K G Q G C P S T H V L

3061 CTCACCCACACCATCAGCCGCATCGCCGTCTCCTACCAGACCAAGGTCAACCTCCTCTCT
152 L T H T I S R I A V S Y Q T K V N L L S

3121 GCCATCAAGAGCCCCTGCCAGAGGGAGACCCCAGAGGGGGCTGAGGCCAAGCCCTGGTAT
172 A I K S P C Q R E T P E G A E A K P W Y

3181 GAGCCCATCTATCTGGGAGGGGTCTTCCAGCTGGAGAAGGGTGACCGACTCAGCGCTGAG
192 E P I Y L G G V F Q L E K G D R L S A E

3241 ATCAATCGGCCCGACTATCTCGACTTTGCCGAGTCTGGGCAGGTCTACTTTGGGATCATT
212 I N R P D Y L D F A E S G Q V Y F G I I

HindIII
3301 GCCCTGTGATAAGCTTGGCCCGCGGGCCCGGGATCCACCGGATCTAGATAACTGATCATA
232 A L

### SEQ ID NO 20

pMPX-52 (*toxR*-EGF cloned into pMPX-6 using PCR-introduced Kpnl and Hindlll)

Shine-Delgarno                    KpnI
2581 GTTTTTTTGGGCTAGCAGGAGGAATTCACCATGGTACCATGAACTTGGGGAATCGACTGT
1 M N L G N R L

2641 TTATTCTGATAGCGGTCTTACTTCCCCTCGCAGTATTACTGCTCAATAGTGACTCTGAAT
8 F I L I A V L L P L A V L L L N S D S E

2701 GTCCCCTGTCCCACGATGGGTACTGCCTCCATGATGGTGTGTGCATGTATATTGAAGCAT
28 C P L S H D G Y C L H D G V C M Y I E A

2761 TGGACAAGTATGCATGCAACTGTGTTGTTGGCTACATCGGGGAGCGATGTCAGTACCGAG
48 L D K Y A C N C V V G Y I G E R C Q Y R

HindIII
2821 ACCTGAAGTGGTGGGAACTGCGCTAATAAGCTTGGCCCGCGGGCCCGGGATCCACCGGAT
68 D L K W W E L R

Non-bold, underlined sequence is *toxR* transmembrane domain segment that constitutes *toxR* residues 178-198. The remaining sequence is from human EGF constituting EGF residues 971-1023.

### SEQ ID NO 21

pMPX-27 (*toxR*-invasin cloned into pMPX-6 using PCR-introduced EcoRI and PstI)

EcoRI
Shine-Delgarno
2581 GTTTTTTTGGGCTAGCAGGAGGAATTCACCATGAACTTGGGGAATCGACTGTTTATTCTG
1 M N L G N R L F I L

2641 ATAGCGGTCTTACTTCCCCTCGCAGTATTACTGCTCTCATTCACATTGAGCGTCACCGTT
11 I A V L L P L A V L L L S F T L S V T V

2701 CAGCAGCCTCAGTTGACATTAACGGCGGCCGTCATTGGTGATGGCGCACCGGCTAATGGG
31 Q Q P Q L T L T A A V I G D G A P A N G

2761 AAAACTGCAATCACCGTTGAGTTCACCGTTGCTGATTTTGAGGGGAAACCCTTAGCCGGG
51 K T A I T V E F T V A D F E G K P L A G

2821 CAGGAGGTGGTGATAACCACCAATAATGGTGCGCTACCGAATAAAATCACGGAAAAGACA
71 Q E V V I T T N N G A L P N K I T E K T

2881 GATGCAAATGGCGTCGCGCGCATTGCATTAACCAATACGACAGATGGCGTGACGGTAGTC
91 D A N G V A R I A L T N T T D G V T V V

2941 ACAGCAGAAGTGGAGGGGCAACGGCAAAGTGTTGATACCCACTTTGTTAAGGGTACTATC
111 T A E V E G Q R Q S V D T H F V K G T I

3001 GCGGCGGATAAATCCACTCTGGCTGCGGTACCGACATCTATCATCGCTGATGGTCTAATG
131 A A D K S T L A A V P T S I I A D G L M

3061 GCTTCAACCATCACGTTGGAGTTGAAGGATACCTATGGGGACCCGCAGGCTGGCGCGAAT
151 A S T I T L E L K D T Y G D P Q A G A N

3121 GTGGCTTTTGACACAACCTTAGGCAATATGGGCGTTATCACGGATCACAATGACGGCACT
171 V A F D T T L G N M G V I T D H N D G T

3181 TATAGCGCACCATTGACCAGTACCACGTTGGGGGTAGCAACAGTAACGGTGAAAGTGGAT
191 Y S A P L T S T T L G V A T V T V K V D

3241 GGGGCTGCGTTCAGTGTGCCGAGTGTGACGGTTAATTTCACGGCAGATCCTATTCCAGAT

211 G A A F S V P S V T V N F T A D P I P D

3301 GCTGGCCGCTCCAGTTTCACCGTCTCCACACCGGATATCTTGGCTGATGGCACGATGAGT
231 A G R S S F T V S T P D I L A D G T M S

3361 TCCACATTATCCTTTGTCCCTGTCGATAAGAATGGCCATTTTATCAGTGGGATGCAGGGC
251 S T L S F V P V D K N G H F I S G M Q G

3421 TTGAGTTTTACTCAAAACGGTGTGCCGGTGAGTATTAGCCCCATTACCGAGCAGCCAGAT
271 L S F T Q N G V P V S I S P I T E Q P D

3481 AGCTATACCGCGACGGTGGTTGGGAATAGTGTCGGTGATGTCACAATCACGCCGCAGGTT
291 S Y T A T V V G N S V G D V T I T P Q V

3541 GATACCCTGATACTGAGTACATTGCAGAAAAAAATATCCCTATTCCCGGTACCTACGCTG
311 D T L I L S T L Q K K I S L F P V P T L

3601 ACCGGTATTCTGGTTAACGGGCAAAATTTCGCTACGGATAAAGGGTTCCCGAAAACGATC
331 T G I L V N G Q N F A T D K G F P K T I

3661 TTTAAAAACGCCACATTCCAGTTACAGATGGATAACGATGTTGCTAATAATACTCAGTAT
351 F K N A T F Q L Q M D N D V A N N T Q Y

3721 GAGTGGTCGTCGTCATTCACACCCAATGTATCGGTTAACGATCAGGGTCAGGTGACGATT
371 E W S S S F T P N V S V N D Q G Q V T I

3781 ACCTACCAAACCTATAGCGAAGTGGCTGTGACGGCGAAAAGTAAAAAATTCCCAAGTTAT
391 T Y Q T Y S E V A V T A K S K K F P S Y

3841 TCGGTGAGTTATCGGTTCTACCCAAATCGGTGGATATACGATGGCGGCAGATCGCTGGTA
411 S V S Y R F Y P N R W I Y D G G R S L V

3901 TCCAGTCTCGAGGCCAGCAGACAATGCCAAGGTTCAGATATGTCTGCGGTTCTTGAATCC
431 S S L E A S R Q C Q G S D M S A V L E S

3961 TCACGTGCAACCAACGGAACGCGTGCGCCTGACGGGACATTGTGGGGCGAGTGGGGGAGC
451 S R A T N G T R A P D G T L W G E W G S

4021 TTGACCGCGTATAGTTCTGATTGGCAATCTGGTGAATATTGGGTCAAAAAGACCAGCACG
471 L T A Y S S D W Q S G E Y W V K K T S T

4081 GATTTTGAAACCATGAATATGGACACAGGCGCACTGCAACCAGGGCCTGCATACTTGGCG
491 D F E T M N M D T G A L Q P G P A Y L A

PstI
4141 TTCCCGCTCTGTGCGCTGTCAATATAACTGCAGGCATGCAAGCTTGGCCCGCGGGCCCGG
511 F P L C A L S I

Non-bold, underlined sequence is *toxR* transmembrane domain segment that constitutes *toxR* residues 178-198. The remaining sequence is from *Yersinia pseudotuberculosis* invasin constituting *inv* residues 490-986.

### SEQ ID NO 22

pMPX-59 (*phoA* leader cloned into pMPX-5 using PCR-introduced PstI and XbaI)

Shine-Delgarno PstI
2401 GAATTCAGGCGCTTTTTAGACTGGTCGTAATGAAATTCAGCAGGATCACATTCTGCAGAT
M

2461 GTCACGGCCGAGACTTATAGTCGCTTTGTTTTTATTTTTAATGTATTTGTACATGGAGA
2 S R P R L I V A L F L F F N V F V H G E

2521 AAATAAAGTGAAACAAAGCACTATTGCACTGGCACTCTTACCGTTACTGTTTACCCCTGT
22 N K V K Q S T I A L A L L P L L F T P V

XbaI
2581 GACAAAAGCCCGGACACCAGAATCTAGA
42 T K A R T P E S R

PhoA leader (residues 1-48) from *E. coli* MG1655 cloned into pMPX-5. Create chimeric fusions with the *phoA* leader by cloning into Xbal and introducing a stop sequence.

### SEQ ID NO 23

pMPX-60 (complete *phoA* cloned into pMPX-5 using PCR-introduced PstI and XbaI)

Shine-Delgarno PstI
2401 GAATTCAGGCGCTTTTTAGACTGGTCGTAATGAAATTCAGCAGGATCACATTCTGCAGAT
1 M

2461 GTCACGGCCGAGACTTATAGTCGCTTTGTTTTTATTTTTAATGTATTTGTACATGGAGA
2 S R P R L I V A L F L F F N V F V H G E

2521 AAATAAAGTGAAACAAAGCACTATTGCACTGGCACTCTTACCGTTACTGTTTACCCCTGT
22 N K V K Q S T I A L A L L P L L F T P V

2581 GACAAAAGCCCGGACACCAGAAATGCCTGTTCTGGAAAACCGGGCTGCTCAGGGCGATAT
42 T K A R T P E M P V L E N R A A Q G D I

2641 TACTGCACCCGGCGGTGCTCGCCGTTTAACGGGTGATCAGACTGCCGCTCTGCGTGATTC
62 T A P G G A R R L T G D Q T A A L R D S

2701 TCTTAGCGATAAACCTGCAAAAAATATTATTTTGCTGATTGGCGATGGGATGGGGGACTC
82 L S D K P A K N I I L L I G D G M G D S

2761 GGAAATTACTGCCGCACGTAATTATGCCGAAGGTGCGGGCGGCTTTTTTAAAGGTATAGA
102 E I T A A R N Y A E G A G G F F K G I D

2821 TGCCTTACCGCTTACCGGGCAATACACTCACTATGCGCTGAATAAAAAAACCGGCAAACC
122 A L P L T G Q Y T H Y A L N K K T G K P

2881 GGACTACGTCACCGACTCGGCTGCATCAGCAACCGCCTGGTCAACCGGTGTCAAAACCTA
142 D Y V T D S A A S A T A W S T G V K T Y

2941 TAACGGCGCGCTGGGCGTCGATATTCACGAAAAAGATCACCCAACGATTCTGGAAATGGC
162 N G A L G V D I H E K D H P T I L E M A

3001 AAAAGCCGCAGGTCTGGCGACCGGTAACGTTTCTACCGCAGAGTTGCAGGATGCCACGCC
182 K A A G L A T G N V S T A E L Q D A T P

3061 CGCTGCGCTGGTGGCACATGTGACCTCGCGCAAATGCTACGGTCCGAGCGCGACCAGTGA
202 A A L V A H V T S R K C Y G P S A T S E

3121 AAAATGTCCGGGTAACGCTCTGGAAAAAGGCGGAAAAGGATCGATTACCGAACAGCTGCT
222 K C P G N A L E K G G K G S I T E Q L L

3181 TAACGCTCGTGCCGACGTTACGCTTGGCGGCGGCGCAAAAACCTTTGCTGAAACGGCAAC
242 N A R A D V T L G G G A K T F A E T A T

3241 CGCTGGTGAATGGCAGGGAAAAACGCTGCGTGAACAGGCACAGGCGCGTGGTTATCAGTT
262 A G E W Q G K T L R E Q A Q A R G Y Q L

3301 GGTGAGCGATGCTGCCTCACTGAATTCGGTGACGGAAGCGAATCAGCAAAAACCCCTGCT
282 V S D A A S L N S V T E A N Q Q K P L L

3361 TGGCCTGTTTGCTGACGGCAATATGCCAGTGCGCTGGCTAGGACCGAAAGCAACGTACCA
302 G L F A D G N M P V R W L G P K A T Y H

3421 TGGCAATATCGATAAGCCCGCAGTCACCTGTACGCCAAATCCGCAACGTAATGACAGTGT
322 G N I D K P A V T C T P N P Q R N D S V

3481 ACCAACCCTGGCGCAGATGACCGACAAAGCCATTGAATTGTTGAGTAAAAATGAGAAAGG
342 P T L A Q M T D K A I E L L S K N E K G

3541 CTTTTTCCTGCAAGTTGAAGGTGCGTCAATCGATAAACAGGATCATGCTGCGAATCCTTG
362 F F L Q V E G A S I D K Q D H A A N P C

3601 TGGGCAAATTGGCGAGACGGTCGATCTCGATGAAGCCGTACAACGGGCGCTGGAATTCGC
382 G Q I G E T V D L D E A V Q R A L E F A

3661 TAAAAAGGAGGGTAACACGCTGGTCATAGTCACCGCTGATCACGCCCACGCCAGCCAGAT
402 K K E G N T L V I V T A D H A H A S Q I

3721 TGTTGCGCCGGATACCAAAGCTCCGGGCCTCACCCAGGCGCTAAATACCAAAGATGGCGC
422 V A P D T K A P G L T Q A L N T K D G A

3781 AGTGATGGTGATGAGTTACGGGAACTCCGAAGAGGATTCACAAGAACATACCGGCAGTCA
442 V M V M S Y G N S E E D S Q E H T G S Q

3841 GTTGCGTATTGCGGCGTATGGCCCGCATGCCGCCAATGTTGTTGGACTGACCGACCAGAC
462 L R I A A Y G P H A A N V V G L T D Q T

XbaI
3901 CGATCTCTTCTACACCATGAAAGCCGCTCTGGGGCTGAAATCTAGA
482 D L F Y T M K A A L G L K S R

Complete PhoA from *E. coli* MG1655 cloned into pMPX-5. Create chimeric fusions with the *phoA* by cloning into XbaI and introducing a stop sequence.

### SEQ ID NO 24

pMPX-62 (MalE residues 1-28 cloned into pMPX-5 using PCR-introduced PstI and XbaI)

Shine-Delgarno PstI
2401 GAATTCAGGCGCTTTTTAGACTGGTCGTAATGAAATTCAGCAGGATCACATTCTGCAGAT
1 M

2461 GAAAATAAAAACAGGTGCACGCATCCTCGCATTATCCGCATTAACGACGATGATGTTTTC
2 K I K T G A R I L A L S A L T T M M F S

XbaI
2521 CGCCTCGGCTCTCGCCAAAATCTCTAGA
22 A S A L A K I S R

MalE residues 1-28 from *E. coli* MG1655 cloned into pMPX-5. Create chimeric fusions with the *malE* by cloning into XbaI and introducing a stop sequence.

### SEQ ID NO 25

pMPX-61 (MalE residues 1-370 cloned into pMPX-5 using PCR-introduced PstI and XbaI)

Shine-Delgarno PstI
2401 GAATTCAGGCGCTTTTTAGACTGGTCGTAATGAAATTCAGCAGGATCACATTCTGCAGAT
1 M

2461 GAAAATAAAAACAGGTGCACGCATCCTCGCATTATCCGCATTAACGACGATGATGTTTTC
2 K I K T G A R I L A L S A L T T M M F S

2521 CGCCTCGGCTCTCGCCAAAATCGAAGAAGGTAAACTGGTAATCTGGATTAACGGCGATAA
22 A S A L A K I E E G K L V I W I N G D K

2581 AGGCTATAACGGTCTCGCTGAAGTCGGTAAGAAATTCGAGAAAGATACCGGAATTAAAGT
42 G Y N G L A E V G K K F E K D T G I K V

2641 CACCGTTGAGCATCCGGATAAACTGGAAGAGAAATTCCCACAGGTTGCGGCAACTGGCGA
62 T V E H P D K L E E K F P Q V A A T G D

2701 TGGCCCTGACATTATCTTCTGGGCACACGACCGCTTTGGTGGCTACGCTCAATCTGGCCT
82 G P D I I F W A H D R F G G Y A Q S G L

2761 GTTGGCTGAAATCACCCCGGACAAAGCGTTCCAGGACAAGCTGTATCCGTTTACCTGGGA
102 L A E I T P D K A F Q D K L Y P F T W D

2821 TGCCGTACGTTACAACGGCAAGCTGATTGCTTACCCGATCGCTGTTGAAGCGTTATCGCT
122 A V R Y N G K L I A Y P I A V E A L S L

2881 GATTTATAACAAAGATCTGCTGCCGAACCCGCCAAAAACCTGGGAAGAGATCCCGGCGCT
142 I Y N K D L L P N P P K T W E E I P A L

2941 GGATAAAGAACTGAAAGCGAAAGGTAAGAGCGCGCTGATGTTCAACCTGCAAGAACCGTA
162 D K E L K A K G K S A L M F N L Q E P Y

3001 CTTCACCTGGCCGCTGATTGCTGCTGACGGGGGTTATGCGTTCAAGTATGAAAACGGCAA
182 F T W P L I A A D G G Y A F K Y E N G K

3061 GTACGACATTAAAGACGTGGGCGTGGATAACGCTGGCGCGAAAGCGGGTCTGACCTTCCT
202 Y D I K D V G V D N A G A K A G L T F L

3121 GGTTGACCTGATTAAAAACAAACACATGAATGCAGACACCGATTACTCCATCGCAGAAGC
222 V D L I K N K H M N A D T D Y S I A E A

3181 TGCCTTTAATAAAGGCGAAACAGCGATGACCATCAACGGCCCGTGGGCATGGTCCAACAT
242 A F N K G E T A M T I N G P W A W S N I

3241 CGACACCAGCAAAGTGAATTATGGTGTAACGGTACTGCCGACCTTCAAGGGTCAACCATC
262 D T S K V N Y G V T V L P T F K G Q P S

3301 CAAACCGTTCGTTGGCGTGCTGAGCGCAGGTATTAACGCCGCCAGTCCGAACAAAGAGCT
282 K P F V G V L S A G I N A A S P N K E L

3361 GGCGAAAGAGTTCCTCGAAAACTATCTGCTGACTGATGAAGGTCTGGAAGCGGTTAATAA

302 A K E F L E N Y L L T D E G L E A V N K

3421 AGACAAACCGCTGGGTGCCGTAGCGCTGAAGTCTTACGAGGAAGAGTTGGCGAAAGATCC
322 D K P L G A V A L K S Y E E E L A K D P

3481 ACGTATTGCCGCCACCATGGAAAACGCCCAGAAAGGTGAAATCATGCCGAACATCCCGCA
342 R I A A T M E N A Q K G E I M P N I P Q

XbaI
3541 GATGTCCGCTTTCTGGTATGCCGTGCGTTCTAGA
362 M S A F W Y A V R S R

MalE residues 1-370 from *E. coli* MG1655 cloned into pMPX-5. Create chimeric fusions with the *malE* by cloning into XbaI and introducing a stop sequence.

### SEQ ID NO 26

pMPX-17 (complete *tig* and groESL, both with complete native control region cloned into pMPX-5 using PCR-introduced Narl and HindIII. The *tig* and *groESL* regions are joined using XbaI). Construct to be used on same vector as protein to be expressed or as a template for insertion into pACYC184.

NarI
181 ACCATATGCG GTGTGAAATA CCGCACAGAT GCGTAAGGAG AAAATACCGC ATCAGGCGCC

241 ATACGCGACA GCGCGCAATA ACCGTTCTCG ACTCATAAAA GTGATGCCGC TATAATGCCG
301 CGTCCTATTT GAATGCTTTC GGGATGATTC TGGTAACAGG GAATGTGATT GATTATAAGA
361 ACATCCCGGT TCCGCGAAGC CAACAACCTG TGCTTGCGGG GTAAGAGTTG ACCGAGCACT

+1 tig
421 GTGATTTTTT GAGGTAACAA GATGCAAGTT TCAGTTGAAA CCACTCAAGG CCTTGGCCGC
→

481 CGTGTAACGA TTACTATCGC TGCTGACAGC ATCGAGACCG CTGTTAAAAG CGAGCTGGTC
541 AACGTTGCGA AAAAAGTACG TATTGACGGC TTCCGCAAAG GCAAAGTGCC AATGAATATC
601 GTTGCTCAGC GTTATGGCGC GTCTGTACGC CAGGACGTTC TGGGTGACCT GATGAGCCGT
661 AACTTCATTG ACGCCATCAT TAAAGAAAAA ATCAATCCGG CTGGCGCACC GACTTATGTT
721 CCGGGCGAAT ACAAGCTGGG TGAAGACTTC ACTTACTCTG TAGAGTTTGA AGTTTATCCG
781 GAAGTTGAAC TGCAGGGTCT GGAAGCGATC GAAGTTGAAA AACCGATCGT TGAAGTGACC
841 GACGCTGACG TTGACGGCAT GCTGGATACT CTGCGTAAAC AGCAGGCGAC CTGGAAAGAA
901 AAAGACGGCG CTGTTGAAGC AGAAGACCGC GTAACCATCG ACTTCACCGG TTCTGTAGAC
961 GGCGAAGAGT TCGAAGGCGG TAAAGCGTCT GATTTCGTAC TGGCGATGGG CCAGGGTCGT
1021 ATGATCCCGG GCTTTGAAGA CGGTATCAAA GGCCACAAAG CTGGCGAAGA GTTCACCATC
1081 GACGTGACCT TCCCGGAAGA ATACCACGCA GAAAACCTGA AAGGTAAAGC AGCGAAATTC
1141 GCTATCAACC TGAAGAAAGT TGAAGAGCGT GAACTGCCGG AACTGACTGC AGAATTCATC
1201 AAACGTTTCG GCGTTGAAGA TGGTTCCGTA GAAGGTCTGC GCGCTGAAGT GCGTAAAAAC
1261 ATGGAGCGCG AGCTGAAGAG CGCCATCCGT AACCGCGTTA AGTCTCAGGC GATCGAAGGT
1321 CTGGTAAAAG CTAACGACAT CGACGTACCG GCTGCGCTGA TCGACAGCGA AATCGACGTT
1381 CTGCGTCGCC AGGCTGCACA GCGTTTCGGT GGCAACGAAA AACAAGCTCT GGAACTGCCG
1441 CGCGAACTGT TCGAAGAACA GGCTAAACGC CGCGTAGTTG TTGGCCTGCT GCTGGGCGAA
1501 GTTATCCGCA CCAACGAGCT GAAAGCTGAC GAAGAGCGCG TGAAAGGCCT GATCGAAGAG
1561 ATGGCTTCTG CGTACGAAGA TCCGAAAGAA GTTATCGAGT TCTACAGCAA AAACAAAGAA
1621 CTGATGGACA ACATGCGCAA TGTTGCTCTG GAAGAACAGG CTGTTGAAGC TGTACTGGCG

Stop
tig
1681 AAAGCGAAAG TGACTGAAAA AGAAACCACT TTCAACGAGC TGATGAACCA GCAGGCGTAA

1741 TAATAATCTA GAGGTAGCAC AATCAGATTC GCTTATGACG GCGATGAAGA AATTGCGATG
1801 AAATGTGAGG TGAATCAGGG TTTTCACCCG ATTTTGTGCT GATCAGAATT TTTTTTCTTT
1861 TTCCCCCTTG AAGGGGCGAA GCCTCATCCC CATTTCTCTG GTCACCAGCC GGGAAACCAC

+1 groES
1921 GTAAGCTCCG GCGTCACCCA TAACAGATAC GGACTTTCTC AAAGGAGAGT TATCAATGAA
→

1981 TATTCGTCCA TTGCATGATC GCGTGATCGT CAAGCGTAAA GAAGTTGAAA CTAAATCTGC
2041 TGGCGGCATC GTTCTGACCG GCTCTGCAGC GGCTAAATCC ACCCGCGGCG AAGTGCTGGC
2101 TGTCGGCAAT GGCCGTATCC TTGAAAATGG CGAAGTGAAG CCGCTGGATG TGAAAGTTGG
2161 CGACATCGTT ATTTTCAACG ATGGCTACGG TGTGAAATCT GAGAAGATCG ACAATGAAGA

Stop groES
2221 AGTGTTGATC ATGTCCGAAA GCGACATTCT GGCAATTGTT GAAGCGTAAT CCGCGCACGA
+1 groEL
2281 CACTGAACAT ACGAATTTAA GGAATAAAGA TAATGGCAGC TAAAGACGTA AAATTCGGTA
→

2341 ACGACGCTCG TGTGAAAATG CTGCGCGGCG TAAACGTACT GGCAGATGCA GTGAAAGTTA
2401 CCCTCGGTCC AAAAGGCCGT AACGTAGTTC TGGATAAATC TTTCGGTGCA CCGACCATCA
2461 CCAAAGATGG TGTTTCCGTT GCTCGTGAAA TCGAACTGGA AGACAAGTTC GAAAATATGG
2521 GTGCGCAGAT GGTGAAAGAA GTTGCCTCTA AAGCAAACGA CGCTGCAGGC GACGGTACCA
2581 CCACTGCAAC CGTACTGGCT CAGGCTATCA TCACTGAAGG TCTGAAAGCT GTTGCTGCGG
2641 GCATGAACCC GATGGACCTG AAACGTGGTA TCGACAAAGC GGTTACCGCT GCAGTTGAAG
2701 AACTGAAAGC GCTGTCCGTA CCATGCTCTG ACTCTAAAGC GATTGCTCAG GTTGGTACCA
2761 TCTCCGCTAA CTCCGACGAA ACCGTAGGTA AACTGATCGC TGAAGCGATG GACAAAGTCG
2821 GTAAAGAAGG CGTTATCACC GTTGAAGACG GTACCGGTCT GCAGGACGAA CTGGACGTGG
2881 TTGAAGGTAT GCAGTTCGAC CGTGGCTACC TGTCTCCTTA CTTCATCAAC AAGCCGGAAA
2941 CTGGCGCAGT AGAACTGGAA AGCCCGTTCA TCCTGCTGGC TGACAAGAAA ATCTCCAACA
3001 TCCGCGAAAT GCTGCCGGTT CTGGAAGCTG TTGCCAAAGC AGGCAAACCG CTGCTGATCA
3061 TCGCTGAAGA TGTAGAAGGC GAAGCGCTGG CAACTCTGGT TGTTAACACC ATGCGTGGCA
3121 TCGTGAAAGT CGCTGCGGTT AAAGCACCGG GCTTCGGCGA TCGTCGTAAA GCTATGCTGC
3181 AGGATATCGC AACCCTGACT GGCGGTACCG TGATCTCTGA AGAGATCGGT ATGGAGCTGG
3241 AAAAAGCAAC CCTGGAAGAC CTGGGTCAGG CTAAACGTGT TGTGATCAAC AAAGACACCA
3301 CCACTATCAT CGATGGCGTG GGTGAAGAAG CTGCAATCCA GGGCCGTGTT GCTCAGATCC
3361 GTCAGCAGAT TGAAGAAGCA ACTTCTGACT ACGACCGTGA AAAACTGCAG GAACGCGTAG
3421 CGAAACTGGC AGGCGGCGTT GCAGTTATCA AAGTGGGTGC TGCTACCGAA GTTGAAATGA
3481 AAGAGAAAAA AGCACGCGTT GAAGATGCCC TGCACGCGAC CCGTGCTGCG GTAGAAGAAG
3541 GCGTGGTTGC TGGTGGTGGT GTTGCGCTGA TCCGCGTAGC GTCTAAACTG GCTGACCTGC
3601 GTGGTCAGAA CGAAGACCAG AACGTGGGTA TCAAAGTTGC ACTGCGTGCA ATGGAAGCTC
3661 CGCTGCGTCA GATCGTATTG AACTGCGGCG AAGAACCGTC TGTTGTTGCT AACACCGTTA
3721 AAGGCGGCGA CGGCAACTAC GGTTACAACG CAGCAACCGA AGAATACGGC AACATGATCG
3781 ACATGGGTAT CCTGGATCCA ACCAAAGTAA CTCGTTCTGC TCTGCAGTAC GCAGCTTCTG
3841 TGGCTGGCCT GATGATCACC ACCGAATGCA TGGTTACCGA CCTGCCGAAA AACGATGCAG

Stop groEL
3901 CTGACTTAGG CGCTGCTGGC GGTATGGGCG GCATGGGTGG CATGGGCGGC ATGATGTAAT

HindIII
3961 AATAAGCTTG CATGCCTGCA GGTCGACTCT AGAGGATCCC CGGGTACCGA GCTCGAATTC

### SEQ ID NO 27

pMPX-63 (C-terminal fusion with Factor Xa TrxA residues 2-109 FLAG cloned into pMPX-5 using PCR-introduced Pstl and BamHI)

2401 GAATTCAGGCGCTTTTTAGACTGGTCGTAATGAAATTCAGCAGGATCACATTCTGCAGAT
1 M

Factor Xa XbaI XhoI
2461 GATCGAAGCCCGCTCTAGACTCGAGAGCGATAAAATTATTCACCTGACTGACGACAGTTT
2 I E A R S R L E S D K I I H L T D D S F

2521 TGACACGGATGTACTCAAAGCGGACGGGGCGATCCTCGTCGATTTCTGGGCAGAGTGGTG
22 D T D V L K A D G A I L V D F W A E W C

2581 CGGTCCGTGCAAAATGATCGCCCCGATTCTGGATGAAATCGCTGACGAATATCAGGGCAA
42 G P C K M I A P I L D E I A D E Y Q G K

2641 ACTGACCGTTGCAAAACTGAACATCGATCAAAACCCTGGCACTGCGCCGAAATATGGCAT
62 L T V A K L N I D Q N P G T A P K Y G I

2701 CCGTGGTATCCCGACTCTGCTGCTGTTCAAAAACGGTGAAGTGGCGGCAACCAAAGTGGG
82 R G I P T L L L F K N G E V A A T K V G

XhoI
2761 TGCACTGTCTAAAGGTCAGTTGAAAGAGTTCCTCGACGCTAACCTGGCGCTCGAGGATTA
102 A L S K G Q L K E F L D A N L A L E D Y

BamHI
2821 TAAAGATCATGATGGCGATTATAAAGATCATGATGATTAATAAGGATCCCCGGGTACCGA
122 K D H D G D Y K D H D D

Gene *trxA* (2-109) from *E. coli* MG1655 cloned into pMPX-5. Create chimeric fusions with the *trxA* by cloning into PstI and XbaI. May remove *trxA* using XhoI. FLAG sequence shown in italics only.

### SEQ ID NO:28

### Rat Edg-3 nucleotide sequence

ATGGCAACCACGCACGCGCAGGGGCACCCGCCAGTCTTGGGGAATGATACTCTCCGGGAA
CATTATGATTACGTGGGGAAGCTGGCAGGCAGGCTGCGGATCCCCCTGAGGGTAGCACCCTCATCACCACC
ATCCTCTTCTTGGTCACCTGTAGCTTCATCGTCTTGGAGAACCTGATGGTTTTGATTGCCATCTGGAAAAACAAT
AAATTTCATAACCGCATGTACTTTTTCATCGGCAACTTGGCTCTCTGCGACCTGCTGGCCGGCATAGCCTACAA
GGTCAACATTCTGATGTCCGGTAGGAAGACGTTCAGCCTGTCTCCAACAGTGTGGTTCCTCAGGGAGGGCAGT
ATGTTCGTAGCCCTGGGCGCATCCACATGCAGCTTATTGGCCATTGCCATTGAGCGGCACCTGACCATGATCA
AGATGAGGCCGTACGACGCCAACAAGAAGCACCGCGTGTTCCTTCTGATTGGGATGTGCTGGCTAATTGCCTT
CTCGCTGGGTGCCCTGCCCATCCTGGGCTGGAACTGCCTGGAGAACTTTCCCGACTGCTCTACCATCTTGCCC
CTCTACTCCAAGAAATACATTGCCTTTCTCATCAGCATCTTCACAGCCATTCTGGTGACCATCGTCATCTTGTAC
GCGCGCATCTACTTCCTGGTCAAGTCCAGCAGCCGCAGGGTGGCCAACCACAACTCCGAGAGATCCATGGCC
CTTCTGCGGACCGTAGTGATCGTGGTGAGCGTGTTCATCGCCTGTTGGTCCCCCCTTTTCATCCTCTTCCTCAT
CGATGTGGCCTGCAGGGCGAAGGAGTGCTCCATCCTCTTCAAGAGTCAGTGGTTCATCATGCTGGCTGTCCTC
AACTCGGCCATGAACCCTGTCATCTACACGCTGGCCAGCAAAGAGATGCGGCGTGCTTTCTTCCGGTTGGTGT
GCGGCTGTCTGGTCAAGGGCAAGGGGACCCAGGCCTCCCCGATGCAGCCTGCTCTTGACCCGAGCAGAAGTA
AATCAAGCTCCAGTAACAACAGCAGCAGCCACTCTCCAAAGGTCAAGGAAGACCTGCCCCATGTGGCTACCTC
TTCCTGCGTCACTGACAAAACGAGGTCGCTTCAGAATGGGGTCCTCTGCAAGTGA -1145

### SEQ ID NO:29

### Rat Edg-3 amino acid sequence

MATTHAQGHPPVLGNDTLREHYDYVGKLAGRLRDPPEGSTLITTILFLVTCSFIVL
ENLMVLIAIWKNNKFHNRMYFFIGNLALCDLLAGIAYKVNILMSGRKTFSLSPTVW
FLREGSMFVALGASTCSLLAIAIERHLTMIKMRPYDANKKHRVFLLIGMCWLIAFS
LGALPILGWNCLENFPDCSTILPLYSKKYIAFLISIFTAILVTIVILYARIYFLVKSSS
RRVANHNSERSMALLRTVVIVVSVFIACWSPLFILFLIDVACRAKECSILFKSQWF
IMLAVLNSAMNPVIYTLASKEMRRAFFRLVCGCLVKGKGTQASPMQPALDPSRS
KSSSSNNSSSHSPKVKEDLPHVATSSCVTDKTRSLQNGVLCK

### SEQ ID NO.: 153

### pMPX-66 arabinose-inducible expression vector

1 TCGCGCGTTT CGGTGATGAC GGTGAAAACC TCTGACACAT GCAGCTCCCG GAGACGGTCA

61 CAGCTTGTCT GTAAGCGGAT GCCGGGAGCA GACAAGCCCG TCAGGGCGCG TCAGCGGGTG

121 TTGGCGGGTG TCGGGGCTGG CTTAACTATG CGGCATCAGA GCAGATTGTA CTGAGAGTGC

181 ACCATATGCG GTGTGAAATA CCGCACAGAT GCGTAAGGAG AAAATACCGC ATCAGGCGCC

241 ATTCGCCATT CAGGCTGCGC AACTGTTGGG AAGGGCGATC GGTGCGGGCC TCTTCGCTAT

301 TACGCCAGCT GGCGAAAGGG GGATGTGCTG CAAGGCGATT AAGTTGGGTA ACGCCAGGGT

HindIII

361 TTTCCCAGTC ACGACGTTGT AAAACGACGG CCAGTGCCAA GCTTCAAGCC GTCAATTGTC

Stop araC

421 TGATTCGTTA CCAATTATGA CAACTTGACG GCTACATCAT TCACTTTTTC TTCACAACCG

481 GCACGGAACT CGCTCGGGCT GGCCCCGGTG CATTTTTTAA ATACCCGCGA GAAATAGAGT

541 TGATCGTCAA AACCAACATT GCGACCGACG GTGGCGATAG GCATCCGGGT GGTGCTCAAA

601 AGCAGCTTCG CCTGGCTGAT ACGTTGGTCC TCGCGCCAGC TTAAGACGCT AATCCCTAAC

661 TGCTGGCGGA AAAGATGTGA CAGACGCGAC GGCGACAAGC AAACATGCTG TGCGACGCTG

721 GCGATATCAA AATTGCTGTC TGCCAGGTGA TCGCTGATGT ACTGACAAGC CTCGCGTACC

781 CGATTATCCA TCGGTGGATG GAGCGACTCG TTAATCGCTT CCATGCGCCG CAGTAACAAT

841 TGCTCAAGCA GATTTATCGC CAGCAGCTCC GAATAGCGCC CTTCCCCTTG CCCGGCGTTA

901 ATGATTTGCC CAAACAGGTC GCTGAAATGC GGCTGGTGCG CTTCATCCGG GCGAAAGAAC

961 CCCGTATTGG CAAATATTGA CGGCCAGTTA AGCCATTCAT GCCAGTAGGC GCGCGGACGA

1021 AAGTAAACCC ACTGGTGATA CCATTCGCGA GCCTCCGGAT GACGACCGTA GTGATGAATC

1081 TCTCCTGGCG GGAACAGCAA AATATCACCC GGTCGGCAAA CAAATTCTCG TCCCTGATTT

1141 TTCACCACCC CCTGACCGCG AATGGTGAGA TTGAGAATAT AACCTTTCAT TCCCAGCGGT

1201 CGGTCGATAA AAAAATCGAG ATAACCGTTG GCCTCAATCG GCGTTAAACC CGCCACCAGA

1261 TGGGCATTAA ACGAGTATCC CGGCAGCAGG GGATCATTTT GCGCTTCAGC CATACTTTTC

Start araC

1321 ATACTCCCGC CATTCAGAGA AGAAACCAAT TGTCCATATT GCATCAGACA TTGCCGTCAC

<--

1381 TGCGTCTTTT ACTGGCTCTT CTCGCTAACC AAACCGGTAA CCCCGCTTAT TAAAAGCATT

1441 CTGTAACAAA GCGGGACCAA AGCCATGACA AAAACGCGTA ACAAAAGTGT CTATAATCAC

1501 GGCAGAAAAG TCCACATTGA TTATTTGCAC GGCGTCACAC TTTGCTATGC CATAGCATTT

1561 TTATCCATAA GATTAGCGGA TCCTACCTGA CGCTTTTTAT CGCAACTCTC TACTGTTTCT

SD SalI XbaI

1621 CCATACCCGT TTTTTTGGGC TAGCAGGAGG CCGTCGACTC TAGAGGATCC CCGCGCCCTC

Stem-loop KpnI

1681 ATCCGAAAGG GCGTATTGGT ACCGAGCTCG AATTCGTAAT CATGGTCATA GCTGTTTCCT

1741 GTGTGAAATT GTTATCCGCT CACAATTCCA CACAACATAC GAGCCGGAAG CATAAAGTGT

1801 AAAGCCTGGG GTGCCTAATG AGTGAGCTAA CTCACATTAA TTGCGTTGCG CTCACTGCCC

1861 GCTTTCCAGT CGGGAAACCT GTCGTGCCAG CTGCATTAAT GAATCGGCCA ACGCGCGGGG

1921 AGAGGCGGTT TGCGTATTGG GCGCTCTTCC GCTTCCTCGC TCACTGACTC GCTGCGCTCG

1981 GTCGTTCGGC TGCGGCGAGC GGTATCAGCT CACTCAAAGG CGGTAATACG GTTATCCACA

2041 GAATCAGGGG ATAACGCAGG AAAGAACATG TGAGCAAAAG GCCAGCAAAA GGCCAGGAAC

2101 CGTAAAAAGG CCGCGTTGCT GGCGTTTTTC CATAGGCTCC GCCCCCCTGA CGAGCATCAC

2161 AAAAATCGAC GCTCAAGTCA GAGGTGGCGA AACCCGACAG GACTATAAAG ATACCAGGCG

2221 TTTCCCCCTG GAAGCTCCCT CGTGCGCTCT CCTGTTCCGA CCCTGCCGCT TACCGGATAC

2281 CTGTCCGCCT TTCTCCCTTC GGGAAGCGTG GCGCTTTCTC ATAGCTCACG CTGTAGGTAT

2341 CTCAGTTCGG TGTAGGTCGT TCGCTCCAAG CTGGGCTGTG TGCACGAACC CCCCGTTCAG

2401 CCCGACCGCT GCGCCTTATC CGGTAACTAT CGTCTTGAGT CCAACCCGGT AAGACACGAC

2461 TTATCGCCAC TGGCAGCAGC CACTGGTAAC AGGATTAGCA GAGCGAGGTA TGTAGGCGGT

2521 GCTACAGAGT TCTTGAAGTG GTGGCCTAAC TACGGCTACA CTAGAAGGAC AGTATTTGGT

2581 ATCTGCGCTC TGCTGAAGCC AGTTACCTTC GGAAAAAGAG TTGGTAGCTC TTGATCCGGC

2641 AAACAAACCA CCGCTGGTAG CGGTGGTTTT TTTGTTTGCA AGCAGCAGAT TACGCGCAGA

2701 AAAAAAGGAT CTCAAGAAGA TCCTTTGATC TTTTCTACGG GGTCTGACGC TCAGTGGAAC

2761 GAAAACTCAC GTTAAGGGAT TTTGGTCATG AGATTATCAA AAAGGATCTT CACCTAGATC

2821 CTTTTAAATT AAAAATGAAG TTTTAAATCA ATCTAAAGTA TATATGAGTA AACTTGGTCT

2881 GACAGTTACC AATGCTTAAT CAGTGAGGCA CCTATCTCAG CGATCTGTCT ATTTCGTTCA

2941 TCCATAGTTG CCTGACTCCC CGTCGTGTAG ATAACTACGA TACGGGAGGG CTTACCATCT

3001 GGCCCCAGTG CTGCAATGAT ACCGCGAGAC CCACGCTCAC CGGCTCCAGA TTTATCAGCA

3061 ATAAACCAGC CAGCCGGAAG GGCCGAGCGC AGAAGTGGTC CTGCAACTTT ATCCGCCTCC

3121 ATCCAGTCTA TTAATTGTTG CCGGGAAGCT AGAGTAAGTA GTTCGCCAGT TAATAGTTTG

3181 CGCAACGTTG TTGCCATTGC TACAGGCATC GTGGTGTCAC GCTCGTCGTT TGGTATGGCT

3241 TCATTCAGCT CCGGTTCCCA ACGATCAAGG CGAGTTACAT GATCCCCCAT GTTGTGCAAA

3301 AAAGCGGTTA GCTCCTTCGG TCCTCCGATC GTTGTCAGAA GTAAGTTGGC CGCAGTGTTA

3361 TCACTCATGG TTATGGCAGC ACTGCATAAT TCTCTTACTG TCATGCCATC CGTAAGATGC

3421 TTTTCTGTGA CTGGTGAGTA CTCAACCAAG TCATTCTGAG AATAGTGTAT GCGGCGACCG

3481 AGTTGCTCTT GCCCGGCGTC AATACGGGAT AATACCGCGC CACATAGCAG AACTTTAAAA

3541 GTGCTCATCA TTGGAAAACG TTCTTCGGGG CGAAAACTCT CAAGGATCTT ACCGCTGTTG

3601 AGATCCAGTT CGATGTAACC CACTCGTGCA CCCAACTGAT CTTCAGCATC TTTTACTTTC

3661 ACCAGCGTTT CTGGGTGAGC AAAAACAGGA AGGCAAAATG CCGCAAAAAA GGGAATAAGG

3721 GCGACACGGA AATGTTGAAT ACTCATACTC TTCCTTTTTC AATATTATTG AAGCATTTAT

3781 CAGGGTTATT GTCTCATGAG CGGATACATA TTTGAATGTA TTTAGAAAAA TAAACAAATA

3841 GGGGTTCCGC GCACATTTCC CCGAAAAGTG CCACCTGACG TCTAAGAAAC CATTATTATC

3901 ATGACATTAA CCTATAAAAA TAGGCGTATC ACGAGGCCCT TTCGTC

The segment araC through Para was taken from pBAD24 using PCR added HindIII and modified aligned Shine-Delgarno (SD) sequence with SalI followed by Xbal, a stem-loop transcriptional stop sequence, and KpnI. The PCR product was cloned into pUC18 using Hindlll and Kpnl.

### SEQ ID NO.: 152

### pMPX-72 rhamnose-inducible expression vector

1 TCGCGCGTTT CGGTGATGAC GGTGAAAACC TCTGACACAT GCAGCTCCCG GAGACGGTCA

61 CAGCTTGTCT GTAAGCGGAT GCCGGGAGCA GACAAGCCCG TCAGGGCGCG TCAGCGGGTG

121 TTGGCGGGTG TCGGGGCTGG CTTAACTATG CGGCATCAGA GCAGATTGTA CTGAGAGTGC

181 ACCATATGCG GTGTGAAATA CCGCACAGAT GCGTAAGGAG AAAATACCGC ATCAGGCGCC

241 ATTCGCCATT CAGGCTGCGC AACTGTTGGG AAGGGCGATC GGTGCGGGCC TCTTCGCTAT

301 TACGCCAGCT GGCGAAAGGG GGATGTGCTG CAAGGCGATT AAGTTGGGTA ACGCCAGGGT

Stop rhaR

361 TTTCCCAGTC ACGACGTTGT AAAACGACGG CCAGTGCCAA GCTTAATTAA TCTTTCTGCG

HindIII

421 AATTGAGATG ACGCCACTGG CTGGGCGTCA TCCCGGTTTC CCGGGTAAAC ACCACCGAAA

481 AATAGTTACT ATCTTCAAAG CCACATTCGG TCGAAATATC ACTGATTAAC AGGCGGCTAT

541 GCTGGAGAAG ATATTGCGCA TGACACACTC TGACCTGTCG CAGATATTGA TTGATGGTCA

601 TTCCAGTCTG CTGGCGAAAT TGCTGACGCA AAACGCGCTC ACTGCACGAT GCCTCATCAC

661 AAAATTTATC CAGCGCAAAG GGACTTTTCA GGCTAGCCGC CAGCCGGGTA ATCAGCTTAT

721 CCAGCAACGT TTCGCTGGAT GTTGGCGGCA ACGAATCACT GGTGTAACGA TGGCGATTCA

781 GCAACATCAC CAACTGCCCG AACAGCAACT CAGCCATTTC GTTAGCAAAC GGCACATGCT

841 GACTACTTTC ATGCTCAAGC TGACCGATAA CCTGCCGCGC CTGCGCCATC CCCATGCTAC

901 CTAAGCGCCA GTGTGGTTGC CCTGCGCTGG CGTTAAATCC CGGAATCGCC CCCTGCCAGT

961 CAAGATTCAG CTTCAGACGC TCCGGGCAAT AAATAATATT CTGCAAAACC AGATCGTTAA

1021 CGGAAGCGTA GGAGTGTTTA TCGTCAGCAT GAATGTAAAA GAGATCGCCA CGGGTAATGC

1081 GATAAGGGCG ATCGTTGAGT ACATGCAGGC CATTACCGCG CCAGACAATC ACCAGCTCAC

1141 AAAAATCATG TGTATGTTCA GCAAAGACAT CTTGCGGATA ACGGTCAGCC ACAGCGACTG

1201 CCTGCTGGTC GCTGGCAAAA AAATCATCTT TGAGAAGTTT TAACTGATGC GCCACCGTGG

1261 CTACCTCGGC CAGAGAACGA AGTTGATTAT TCGCAATATG GCGTACAAAT ACGTTGAGAA

Stop rhaS Start rhaR

1321 GATTCGCGTT ATTGCAGAAA GCCATCCCGT CCCTGGCGAA TATCACGCGG TGACCAGTTA

<--

1381 AACTCTCGGC GAAAAAGCGT CGAAAAGTGG TTACTGTCGC TGAATCCACA GCGATAGGCG

1441 ATGTCAGTAA CGCTGGCCTC GCTGTGGCGT AGCAGATGTC GGGCTTTCAT CAGTCGCAGG

1501 CGGTTCAGGT ATCGCTGAGG CGTCAGTCCC GTTTGCTGCT TAAGCTGCCG ATGTAGCGTA

1561 CGCAGTGAAA GAGAAAATTG ATCCGCCACG GCATCCCAAT TCACCTCATC GGCAAAATGG

1621 TCCTCCAGCC AGGCCAGAAG CAAGTTGAGA CGTGATGCGC TGTTTTCCAG GTTCTCCTGC

1681 AAACTGCTTT TACGCAGCAA GAGCAGTAAT TGCATAAACA AGATCTCGCG ACTGGCGGTC

1741 GAGGGTAAAT CATTTTCCCC TTCCTGCTGT TCCATCTGTG CAACCAGCTG TCGCACCTGC

1801 TGCAATACGC TGTGGTTAAC GCGCCAGTGA GACGGATACT GCCCATCCAG CTCTTGTGGC

1861 AGCAACTGAT TCAGCCCGGC GAGAAACTGA AATCGATCCG GCGAGCGATA CAGCACATTG

1921 GTCAGACACA GATTATCGGT ATGTTCATAC AGATGCCGAT CATGATCGCG TACGAAACAG

1981 ACCGTGCCAC CGGTGATGGT ATAGGGCTGC CCATTAAACA CATGAATACC CGTGCCATGT

2041 TCGACAATCA CAATTTCATG AAAATCATGA TGATGTTCAG GAAAATCCGC CTGCGGGAGC

2101 CGGGGTTCTA TCGCCACGGA CGCGTTACCA GACGGAAAAA AATCCACACT ATGTAATACG

Start rhaS

2161 GTCATACTGG CCTCCTGATG TCGTCAACAC GGCGAAATAG TAATCACGAG GTCAGGTTCT

<--

2221 TACCTTAAAT TTTCGACGGA AAACCACGTA AAAAACGTCG ATTTTTCAAG ATACAGCGTG

2281 AATTTTCAGG AAATGCGGTG AGCATCACAT CACCACAATT CAGCAAATTG TGAACATCAT

2341 CACGTTCATC TTTCCCTGGT TGCCAATGGC CCATTTTCCT GTCAGTAACG AGAAGGTCGC

SD PstI SalI

2401 GAATTCAGGC GCTTTTTAGA CTGGTCGTAA TGAAATTCAG GAGGTTCTGC AGGTCGACTC

XbaI Stem-loop KpnI

2461 TAGAGGATCC CCGCGCCCTC ATCCGAAAGG GCGTATTGGT ACCGAGCTCG AATTCGTAAT

2521 CATGGTCATA GCTGTTTCCT GTGTGAAATT GTTATCCGCT CACAATTCCA CACAACATAC

2581 GAGCCGGAAG CATAAAGTGT AAAGCCTGGG GTGCCTAATG AGTGAGCTAA CTCACATTAA

2641 TTGCGTTGCG CTCACTGCCC GCTTTCCAGT CGGGAAACCT GTCGTGCCAG CTGCATTAAT

2701 GAATCGGCCA ACGCGCGGGG AGAGGCGGTT TGCGTATTGG GCGCTCTTCC
GCTTCCTCGC

2761 TCACTGACTC GCTGCGCTCG GTCGTTCGGC TGCGGCGAGC GGTATCAGCT CACTCAAAGG

2821 CGGTAATACG GTTATCCACA GAATCAGGGG ATAACGCAGG AAAGAACATG TGAGCAAAAG

2881 GCCAGCAAAA GGCCAGGAAC CGTAAAAAGG CCGCGTTGCT GGCGTTTTTC CATAGGCTCC

2941 GCCCCCCTGA CGAGCATCAC AAAAATCGAC GCTCAAGTCA GAGGTGGCGA AACCCGACAG

3001 GACTATAAAG ATACCAGGCG TTTCCCCCTG GAAGCTCCCT CGTGCGCTCT CCTGTTCCGA

3061 CCCTGCCGCT TACCGGATAC CTGTCCGCCT TTCTCCCTTC GGGAAGCGTG GCGCTTTCTC

3121 ATAGCTCACG CTGTAGGTAT CTCAGTTCGG TGTAGGTCGT TCGCTCCAAG CTGGGCTGTG

3181 TGCACGAACC CCCCGTTCAG CCCGACCGCT GCGCCTTATC CGGTAACTAT CGTCTTGAGT

3241 CCAACCCGGT AAGACACGAC TTATCGCCAC TGGCAGCAGC CACTGGTAAC AGGATTAGCA

3301 GAGCGAGGTA TGTAGGCGGT GCTACAGAGT TCTTGAAGTG GTGGCCTAAC TACGGCTACA

3361 CTAGAAGGAC AGTATTTGGT ATCTGCGCTC TGCTGAAGCC AGTTACCTTC GGAAAAAGAG

3421 TTGGTAGCTC TTGATCCGGC AAACAAACCA CCGCTGGTAG CGGTGGTTTT TTTGTTTGCA

3481 AGCAGCAGAT TACGCGCAGA AAAAAAGGAT CTCAAGAAGA TCCTTTGATC TTTTCTACGG

3541 GGTCTGACGC TCAGTGGAAC GAAAACTCAC GTTAAGGGAT TTTGGTCATG AGATTATCAA

3601 AAAGGATCTT CACCTAGATC CTTTTAAATT AAAAATGAAG TTTTAAATCA ATCTAAAGTA

3661 TATATGAGTA AACTTGGTCT GACAGTTACC AATGCTTAAT CAGTGAGGCA CCTATCTCAG

3721 CGATCTGTCT ATTTCGTTCA TCCATAGTTG CCTGACTCCC CGTCGTGTAG ATAACTACGA

3781 TACGGGAGGG CTTACCATCT GGCCCCAGTG CTGCAATGAT ACCGCGAGAC CCACGCTCAC

3841 CGGCTCCAGA TTTATCAGCA ATAAACCAGC CAGCCGGAAG GGCCGAGCGC AGAAGTGGTC

3901 CTGCAACTTT ATCCGCCTCC ATCCAGTCTA TTAATTGTTG CCGGGAAGCT AGAGTAAGTA

3961 GTTCGCCAGT TAATAGTTTG CGCAACGTTG TTGCCATTGC TACAGGCATC GTGGTGTCAC

4021 GCTCGTCGTT TGGTATGGCT TCATTCAGCT CCGGTTCCCA ACGATCAAGG CGAGTTACAT

4081 GATCCCCCAT GTTGTGCAAA AAAGCGGTTA GCTCCTTCGG TCCTCCGATC GTTGTCAGAA

4141 GTAAGTTGGC CGCAGTGTTA TCACTCATGG TTATGGCAGC ACTGCATAAT TCTCTTACTG

4201 TCATGCCATC CGTAAGATGC TTTTCTGTGA CTGGTGAGTA CTCAACCAAG TCATTCTGAG

4261 AATAGTGTAT GCGGCGACCG AGTTGCTCTT GCCCGGCGTC AATACGGGAT AATACCGCGC

4321 CACATAGCAG AACTTTAAAA GTGCTCATCA TTGGAAAACG TTCTTCGGGG CGAAAACTCT

4381 CAAGGATCTT ACCGCTGTTG AGATCCAGTT CGATGTAACC CACTCGTGCA CCCAACTGAT

4441 CTTCAGCATC TTTTACTTTC ACCAGCGTTT CTGGGTGAGC AAAAACAGGA AGGCAAAATG

4501 CCGCAAAAAA GGGAATAAGG GCGACACGGA AATGTTGAAT ACTCATACTC TTCCTTTTTC

4561 AATATTATTG AAGCATTTAT CAGGGTTATT GTCTCATGAG CGGATACATA TTTGAATGTA

4621 TTTAGAAAAA TAAACAAATA GGGGTTCCGC GCACATTTCC CCGAAAAGTG CCACCTGACG

4681 TCTAAGAAAC CATTATTATC ATGACATTAA CCTATAAAAA TAGGCGTATC ACGAGGCCCT

4741 TTCGTC

The segment rhaR through Prha was taken from the E. coli chromosome using PCR added HindIII and modified aligned Shine-Delgarno (SD) sequence with Pstl followed by SalI, XbaI, a stem-loop transcriptional stop sequence, and KpnI. The PCR product was cloned into pUC18 using HindIII and Kpnl.

### SEO ID NO.: 151

### pMPX-67 rhamnose-inducible expression vector

1 TCGCGCGTTT CGGTGATGAC GGTGAAAACC TCTGACACAT GCAGCTCCCG GAGACGGTCA

61 CAGCTTGTCT GTAAGCGGAT GCCGGGAGCA GACAAGCCCG TCAGGGCGCG TCAGCGGGTG

121 TTGGCGGGTG TCGGGGCTGG CTTAACTATG CGGCATCAGA GCAGATTGTA CTGAGAGTGC

181 ACCATATGCG GTGTGAAATA CCGCACAGAT GCGTAAGGAG AAAATACCGC ATCAGGCGCC

241 ATTCGCCATT CAGGCTGCGC AACTGTTGGG AAGGGCGATC GGTGCGGGCC TCTTCGCTAT

301 TACGCCAGCT GGCGAAAGGG GGATGTGCTG CAAGGCGATT AAGTTGGGTA ACGCCAGGGT

Stop rhaR

361 TTTCCCAGTC ACGACGTTGT AAAACGACGG CCAGTGCCAA GCTTAATTAA TCTTTCTGCG

HindIII

421 AATTGAGATG ACGCCACTGG CTGGGCGTCA TCCCGGTTTC CCGGGTAAAC ACCACCGAAA

481 AATAGTTACT ATCTTCAAAG CCACATTCGG TCGAAATATC ACTGATTAAC AGGCGGCTAT

541 GCTGGAGAAG ATATTGCGCA TGACACACTC TGACCTGTCG CAGATATTGA TTGATGGTCA

601 TTCCAGTCTG CTGGCGAAAT TGCTGACGCA AAACGCGCTC ACTGCACGAT GCCTCATCAC

661 AAAATTTATC CAGCGCAAAG GGACTTTTCA GGCTAGCCGC CAGCCGGGTA ATCAGCTTAT

721 CCAGCAACGT TTCGCTGGAT GTTGGCGGCA ACGAATCACT GGTGTAACGA TGGCGATTCA

781 GCAACATCAC CAACTGCCCG AACAGCAACT CAGCCATTTC GTTAGCAAAC GGCACATGCT

841 GACTACTTTC ATGCTCAAGC TGACCGATAA CCTGCCGCGC CTGCGCCATC CCCATGCTAC

901 CTAAGCGCCA GTGTGGTTGC CCTGCGCTGG CGTTAAATCC CGGAATCGCC CCCTGCCAGT

961 CAAGATTCAG CTTCAGACGC TCCGGGCAAT AAATAATATT CTGCAAAACC AGATCGTTAA

1021 CGGAAGCGTA GGAGTGTTTA TCGTCAGCAT GAATGTAAAA GAGATCGCCA CGGGTAATGC

1081 GATAAGGGCG ATCGTTGAGT ACATGCAGGC CATTACCGCG CCAGACAATC ACCAGCTCAC

1141 AAAAATCATG TGTATGTTCA GCAAAGACAT CTTGCGGATA ACGGTCAGCC ACAGCGACTG

1201 CCTGCTGGTC GCTGGCAAAA AAATCATCTT TGAGAAGTTT TAACTGATGC GCCACCGTGG

1261 CTACCTCGGC CAGAGAACGA AGTTGATTAT TCGCAATATG GCGTACAAAT ACGTTGAGAA

Stop rhaS Start rhaR

1321 GATTCGCGTT ATTGCAGAAA GCCATCCCGT CCCTGGCGAA TATCACGCGG TGACCAGTTA

<--

1381 AACTCTCGGC GAAAAAGCGT CGAAAAGTGG TTACTGTCGC TGAATCCACA GCGATAGGCG

1441 ATGTCAGTAA CGCTGGCCTC GCTGTGGCGT AGCAGATGTC GGGCTTTCAT CAGTCGCAGG

1501 CGGTTCAGGT ATCGCTGAGG CGTCAGTCCC GTTTGCTGCT TAAGCTGCCG ATGTAGCGTA

1561 CGCAGTGAAA GAGAAAATTG ATCCGCCACG GCATCCCAAT TCACCTCATC GGCAAAATGG

1621 TCCTCCAGCC AGGCCAGAAG CAAGTTGAGA CGTGATGCGC TGTTTTCCAG GTTCTCCTGC

1681 AAACTGCTTT TACGCAGCAA GAGCAGTAAT TGCATAAACA AGATCTCGCG ACTGGCGGTC

1741 GAGGGTAAAT CATTTTCCCC TTCCTGCTGT TCCATCTGTG CAACCAGCTG TCGCACCTGC

1801 TGCAATACGC TGTGGTTAAC GCGCCAGTGA GACGGATACT GCCCATCCAG CTCTTGTGGC

1861 AGCAACTGAT TCAGCCCGGC GAGAAACTGA AATCGATCCG GCGAGCGATA CAGCACATTG

1921 GTCAGACACA GATTATCGGT ATGTTCATAC AGATGCCGAT CATGATCGCG TACGAAACAG

1981 ACCGTGCCAC CGGTGATGGT ATAGGGCTGC CCATTAAACA CATGAATACC CGTGCCATGT

2041 TCGACAATCA CAATTTCATG AAAATCATGA TGATGTTCAG GAAAATCCGC CTGCGGGAGC

2101 CGGGGTTCTA TCGCCACGGA CGCGTTACCA GACGGAAAAA AATCCACACT ATGTAATACG

Start rhaS

2161 GTCATACTGG CCTCCTGATG TCGTCAACAC GGCGAAATAG TAATCACGAG GTCAGGTTCT

<--

2221 TACCTTAAAT TTTCGACGGA AAACCACGTA AAAAACGTCG ATTTTTCAAG ATACAGCGTG

2281 AATTTTCAGG AAATGCGGTG AGCATCACAT CACCACAATT CAGCAAATTG TGAACATCAT

2341 CACGTTCATC TTTCCCTGGT TGCCAATGGC CCATTTTCCT GTCAGTAACG AGAAGGTCGC

SD SalI XbaI

2401 GAATTCAGGC GCTTTTTAGA CTGGTCGTAA TGAAATTCAG GAGGTTGTCG ACTCTAGAGG

Stem-loop KpnI

2461 ATCCCCGCGC CCTCATCCGA AAGGGCGTAT TGGTACCGAG CTCGAATTCG TAATCATGGT

2521 CATAGCTGTT TCCTGTGTGA AATTGTTATC CGCTCACAAT TCCACACAAC ATACGAGCCG

2581 GAAGCATAAA GTGTAAAGCC TGGGGTGCCT AATGAGTGAG CTAACTCACA TTAATTGCGT

2641 TGCGCTCACT GCCCGCTTTC CAGTCGGGAA ACCTGTCGTG CCAGCTGCAT TAATGAATCG

2701 GCCAACGCGC GGGGAGAGGC GGTTTGCGTA TTGGGCGCTC TTCCGCTTCC TCGCTCACTG

2761 ACTCGCTGCG CTCGGTCGTT CGGCTGCGGC GAGCGGTATC AGCTCACTCA AAGGCGGTAA

2821 TACGGTTATC CACAGAATCA GGGGATAACG CAGGAAAGAA CATGTGAGCA AAAGGCCAGC

2881 AAAAGGCCAG GAACCGTAAA AAGGCCGCGT TGCTGGCGTT TTTCCATAGG CTCCGCCCCC

2941 CTGACGAGCA TCACAAAAAT CGACGCTCAA GTCAGAGGTG GCGAAACCCG ACAGGACTAT

3001 AAAGATACCA GGCGTTTCCC CCTGGAAGCT CCCTCGTGCG CTCTCCTGTT CCGACCCTGC

3061 CGCTTACCGG ATACCTGTCC GCCTTTCTCC CTTCGGGAAG CGTGGCGCTT TCTCATAGCT

3121 CACGCTGTAG GTATCTCAGT TCGGTGTAGG TCGTTCGCTC CAAGCTGGGC TGTGTGCACG

3181 AACCCCCCGT TCAGCCCGAC CGCTGCGCCT TATCCGGTAA CTATCGTCTT GAGTCCAACC

3241 CGGTAAGACA CGACTTATCG CCACTGGCAG CAGCCACTGG TAACAGGATT AGCAGAGCGA

3301 GGTATGTAGG CGGTGCTACA GAGTTCTTGA AGTGGTGGCC TAACTACGGC TACACTAGAA

3361 GGACAGTATT TGGTATCTGC GCTCTGCTGA AGCCAGTTAC CTTCGGAAAA AGAGTTGGTA

3421 GCTCTTGATC CGGCAAACAA ACCACCGCTG GTAGCGGTGG TTTTTTTGTT TGCAAGCAGC

3481 AGATTACGCG CAGAAAAAAA GGATCTCAAG AAGATCCTTT GATCTTTTCT ACGGGGTCTG

3541 ACGCTCAGTG GAACGAAAAC TCACGTTAAG GGATTTTGGT CATGAGATTA TCAAAAAGGA

3601 TCTTCACCTA GATCCTTTTA AATTAAAAAT GAAGTTTTAA ATCAATCTAA AGTATATATG

3661 AGTAAACTTG GTCTGACAGT TACCAATGCT TAATCAGTGA GGCACCTATC TCAGCGATCT

3721 GTCTATTTCG TTCATCCATA GTTGCCTGAC TCCCCGTCGT GTAGATAACT ACGATACGGG

3781 AGGGCTTACC ATCTGGCCCC AGTGCTGCAA TGATACCGCG AGACCCACGC TCACCGGCTC

3841 CAGATTTATC AGCAATAAAC CAGCCAGCCG GAAGGGCCGA GCGCAGAAGT GGTCCTGCAA

3901 CTTTATCCGC CTCCATCCAG TCTATTAATT GTTGCCGGGA AGCTAGAGTA AGTAGTTCGC

3961 CAGTTAATAG TTTGCGCAAC GTTGTTGCCA TTGCTACAGG CATCGTGGTG TCACGCTCGT

4021 CGTTTGGTAT GGCTTCATTC AGCTCCGGTT CCCAACGATC AAGGCGAGTT ACATGATCCC

4081 CCATGTTGTG CAAAAAAGCG GTTAGCTCCT TCGGTCCTCC GATCGTTGTC AGAAGTAAGT

4141 TGGCCGCAGT GTTATCACTC ATGGTTATGG CAGCACTGCA TAATTCTCTT ACTGTCATGC

4201 CATCCGTAAG ATGCTTTTCT GTGACTGGTG AGTACTCAAC CAAGTCATTC TGAGAATAGT

4261 GTATGCGGCG ACCGAGTTGC TCTTGCCCGG CGTCAATACG GGATAATACC GCGCCACATA

4321 GCAGAACTTT AAAAGTGCTC ATCATTGGAA AACGTTCTTC GGGGCGAAAA CTCTCAAGGA

4381 TCTTACCGCT GTTGAGATCC AGTTCGATGT AACCCACTCG TGCACCCAAC TGATCTTCAG

4441 CATCTTTTAC TTTCACCAGC GTTTCTGGGT GAGCAAAAAC AGGAAGGCAA AATGCCGCAA

4501 AAAAGGGAAT AAGGGCGACA CGGAAATGTT GAATACTCAT ACTCTTCCTT TTTCAATATT

4561 ATTGAAGCAT TTATCAGGGT TATTGTCTCA TGAGCGGATA CATATTTGAA TGTATTTAGA

4621 AAAATAAACA AATAGGGGTT CCGCGCACAT TTCCCCGAAA AGTGCCACCT GACGTCTAAG

4681 AAACCATTAT TATCATGACA TTAACCTATA AAAATAGGCG TATCACGAGG CCCTTTCGTC

The segment rhaR through Prha was taken from the E. coli chromosome using PCR added Hindlll and modified aligned Shine-Delgarno (SD) sequence with SalI followed by XbaI, a stem-loop transcriptional stop sequence, and KpnI. The PCR product was cloned into pUC18 using HindIII and KpnI.

### SEQ ID NO.: 154

### pMPX-71 arabinose-inducible expression vector

1 TCGCGCGTTT CGGTGATGAC GGTGAAAACC TCTGACACAT GCAGCTCCCG GAGACGGTCA

61 CAGCTTGTCT GTAAGCGGAT GCCGGGAGCA GACAAGCCCG TCAGGGCGCG TCAGCGGGTG

121 TTGGCGGGTG TCGGGGCTGG CTTAACTATG CGGCATCAGA GCAGATTGTA CTGAGAGTGC

181 ACCATATGCG GTGTGAAATA CCGCACAGAT GCGTAAGGAG AAAATACCGC ATCAGGCGCC

241 ATTCGCCATT CAGGCTGCGC AACTGTTGGG AAGGGCGATC GGTGCGGGCC TCTTCGCTAT

301 TACGCCAGCT GGCGAAAGGG GGATGTGCTG CAAGGCGATT AAGTTGGGTA ACGCCAGGGT

HindIII

361 TTTCCCAGTC ACGACGTTGT AAAACGACGG CCAGTGCCAA GCTTCAAGCC GTCAATTGTC

Stop araC

421 TGATTCGTTA CCAATTATGA CAACTTGACG GCTACATCAT TCACTTTTTC TTCACAACCG

481 GCACGGAACT CGCTCGGGCT GGCCCCGGTG CATTTTTTAA ATACCCGCGA GAAATAGAGT

541 TGATCGTCAA AACCAACATT GCGACCGACG GTGGCGATAG GCATCCGGGT GGTGCTCAAA

601 AGCAGCTTCG CCTGGCTGAT ACGTTGGTCC TCGCGCCAGC TTAAGACGCT AATCCCTAAC

661 TGCTGGCGGA AAAGATGTGA CAGACGCGAC GGCGACAAGC AAACATGCTG TGCGACGCTG

721 GCGATATCAA AATTGCTGTC TGCCAGGTGA TCGCTGATGT ACTGACAAGC CTCGCGTACC

781 CGATTATCCA TCGGTGGATG GAGCGACTCG TTAATCGCTT CCATGCGCCG CAGTAACAAT

841 TGCTCAAGCA GATTTATCGC CAGCAGCTCC GAATAGCGCC CTTCCCCTTG CCCGGCGTTA

901 ATGATTTGCC CAAACAGGTC GCTGAAATGC GGCTGGTGCG CTTCATCCGG GCGAAAGAAC

961 CCCGTATTGG CAAATATTGA CGGCCAGTTA AGCCATTCAT GCCAGTAGGC GCGCGGACGA

1021 AAGTAAACCC ACTGGTGATA CCATTCGCGA GCCTCCGGAT GACGACCGTA GTGATGAATC

1081 TCTCCTGGCG GGAACAGCAA AATATCACCC GGTCGGCAAA CAAATTCTCG TCCCTGATTT

1141 TTCACCACCC CCTGACCGCG AATGGTGAGA TTGAGAATAT AACCTTTCAT TCCCAGCGGT

1201 CGGTCGATAA AAAAATCGAG ATAACCGTTG GCCTCAATCG GCGTTAAACC CGCCACCAGA

1261 TGGGCATTAA ACGAGTATCC CGGCAGCAGG GGATCATTTT GCGCTTCAGC CATACTTTTC

Start araC

1321 ATACTCCCGC CATTCAGAGA AGAAACCAAT TGTCCATATT GCATCAGACA TTGCCGTCAC

<--

1381 TGCGTCTTTT ACTGGCTCTT CTCGCTAACC AAACCGGTAA CCCCGCTTAT TAAAAGCATT

1441 CTGTAACAAA GCGGGACCAA AGCCATGACA AAAACGCGTA ACAAAAGTGT CTATAATCAC

1501 GGCAGAAAAG TCCACATTGA TTATTTGCAC GGCGTCACAC TTTGCTATGC CATAGCATTT

1561 TTATCCATAA GATTAGCGGA TCCTACCTGA CGCTTTTTAT CGCAACTCTC TACTGTTTCT

SD PstI SalI XbaI

1621 CCATACCCGT TTTTTTGGGC TAGCAGGAGG CCCTGCAGGT CGACTCTAGA GGATCCCCGC

Stem-loop KpnI

1681 GCCCTCATCC GAAAGGGCGT ATTGGTACCG AGCTCGAATT CGTAATCATG GTCATAGCTG

1741 TTTCCTGTGT GAAATTGTTA TCCGCTCACA ATTCCACACA ACATACGAGC CGGAAGCATA

1801 AAGTGTAAAG CCTGGGGTGC CTAATGAGTG AGCTAACTCA CATTAATTGC GTTGCGCTCA

1861 CTGCCCGCTT TCCAGTCGGG AAACCTGTCG TGCCAGCTGC ATTAATGAAT CGGCCAACGC

1921 GCGGGGAGAG GCGGTTTGCG TATTGGGCGC TCTTCCGCTT CCTCGCTCAC TGACTCGCTG

1981 CGCTCGGTCG TTCGGCTGCG GCGAGCGGTA TCAGCTCACT CAAAGGCGGT AATACGGTTA

2041 TCCACAGAAT CAGGGGATAA CGCAGGAAAG AACATGTGAG CAAAAGGCCA GCAAAAGGCC

2101 AGGAACCGTA AAAAGGCCGC GTTGCTGGCG TTTTTCCATA GGCTCCGCCC CCCTGACGAG

2161 CATCACAAAA ATCGACGCTC AAGTCAGAGG TGGCGAAACC CGACAGGACT ATAAAGATAC

2221 CAGGCGTTTC CCCCTGGAAG CTCCCTCGTG CGCTCTCCTG TTCCGACCCT GCCGCTTACC

2281 GGATACCTGT CCGCCTTTCT CCCTTCGGGA AGCGTGGCGC TTTCTCATAG CTCACGCTGT

2341 AGGTATCTCA GTTCGGTGTA GGTCGTTCGC TCCAAGCTGG GCTGTGTGCA CGAACCCCCC

2401 GTTCAGCCCG ACCGCTGCGC CTTATCCGGT AACTATCGTC TTGAGTCCAA CCCGGTAAGA

2461 CACGACTTAT CGCCACTGGC AGCAGCCACT GGTAACAGGA TTAGCAGAGC GAGGTATGTA

2521 GGCGGTGCTA CAGAGTTCTT GAAGTGGTGG CCTAACTACG GCTACACTAG AAGGACAGTA

2581 TTTGGTATCT GCGCTCTGCT GAAGCCAGTT ACCTTCGGAA AAAGAGTTGG TAGCTCTTGA

2641 TCCGGCAAAC AAACCACCGC TGGTAGCGGT GGTTTTTTTG TTTGCAAGCA GCAGATTACG

2701 CGCAGAAAAA AAGGATCTCA AGAAGATCCT TTGATCTTTT CTACGGGGTC TGACGCTCAG

2761 TGGAACGAAA ACTCACGTTA AGGGATTTTG GTCATGAGAT TATCAAAAAG GATCTTCACC

2821 TAGATCCTTT TAAATTAAAA ATGAAGTTTT AAATCAATCT AAAGTATATA TGAGTAAACT

2881 TGGTCTGACA GTTACCAATG CTTAATCAGT GAGGCACCTA TCTCAGCGAT CTGTCTATTT

2941 CGTTCATCCA TAGTTGCCTG ACTCCCCGTC GTGTAGATAA CTACGATACG GGAGGGCTTA

3001 CCATCTGGCC CCAGTGCTGC AATGATACCG CGAGACCCAC GCTCACCGGC TCCAGATTTA

3061 TCAGCAATAA ACCAGCCAGC CGGAAGGGCC GAGCGCAGAA GTGGTCCTGC AACTTTATCC

3121 GCCTCCATCC AGTCTATTAA TTGTTGCCGG GAAGCTAGAG TAAGTAGTTC GCCAGTTAAT

3181 AGTTTGCGCA ACGTTGTTGC CATTGCTACA GGCATCGTGG TGTCACGCTC GTCGTTTGGT

3241 ATGGCTTCAT TCAGCTCCGG TTCCCAACGA TCAAGGCGAG TTACATGATC CCCCATGTTG

3301 TGCAAAAAAG CGGTTAGCTC CTTCGGTCCT CCGATCGTTG TCAGAAGTAA GTTGGCCGCA

3361 GTGTTATCAC TCATGGTTAT GGCAGCACTG CATAATTCTC TTACTGTCAT GCCATCCGTA

3421 AGATGCTTTT CTGTGACTGG TGAGTACTCA ACCAAGTCAT TCTGAGAATA GTGTATGCGG

3481 CGACCGAGTT GCTCTTGCCC GGCGTCAATA CGGGATAATA CCGCGCCACA TAGCAGAACT

3541 TTAAAAGTGC TCATCATTGG AAAACGTTCT TCGGGGCGAA AACTCTCAAG GATCTTACCG

3601 CTGTTGAGAT CCAGTTCGAT GTAACCCACT CGTGCACCCA ACTGATCTTC AGCATCTTTT

3661 ACTTTCACCA GCGTTTCTGG GTGAGCAAAA ACAGGAAGGC AAAATGCCGC AAAAAAGGGA

3721 ATAAGGGCGA CACGGAAATG TTGAATACTC ATACTCTTCC TTTTTCAATA TTATTGAAGC

3781 ATTTATCAGG GTTATTGTCT CATGAGCGGA TACATATTTG AATGTATTTA GAAAAATAAA

3841 CAAATAGGGG TTCCGCGCAC ATTTCCCCGA AAAGTGCCAC CTGACGTCTA AGAAACCATT

3901 ATTATCATGA CATTAACCTA TAAAAATAGG CGTATCACGA GGCCCTTTCG TC

The segment araC through Para was taken from pBAD24 using PCR added HindIII and modified aligned Shine-Delgarno (SD) sequence with Pstl followed by SalI, XbaI, a stem-loop transcriptional stop sequence, and KpnI. The PCR product was cloned into pUC18 using HindIII and KpnI.

### SEO ID NO.: 155

### pMPX-68 melibiose-inducible expression vector

1 TCGCGCGTTT CGGTGATGAC GGTGAAAACC TCTGACACAT GCAGCTCCCG GAGACGGTCA

61 CAGCTTGTCT GTAAGCGGAT GCCGGGAGCA GACAAGCCCG TCAGGGCGCG TCAGCGGGTG

121 TTGGCGGGTG TCGGGGCTGG CTTAACTATG CGGCATCAGA GCAGATTGTA CTGAGAGTGC

181 ACCATATGCG GTGTGAAATA CCGCACAGAT GCGTAAGGAG AAAATACCGC ATCAGGCGCC

241 ATTCGCCATT CAGGCTGCGC AACTGTTGGG AAGGGCGATC GGTGCGGGCC TCTTCGCTAT

301 TACGCCAGCT GGCGAAAGGG GGATGTGCTG CAAGGCGATT AAGTTGGGTA ACGCCAGGGT

HindIII

361 TTTCCCAGTC ACGACGTTGT AAAACGACGG CCAGTGCCAA GCTTTTAGCC GGGAAACGTC

Stop MelR

421 TGGCGGCGCT GTTGGCTAAG TTTGCGGTAT TGTTGCGGCG ACATGCCGAC ATATTTGCCG

481 AACGTGCTGT AAAAACGACT ACTTGAACGA AAGCCTGCCG TCAGGGCAAT ATCGAGAATA

541 CTTTTATCGG TATCGCTCAG TAACGCGCGA ACGTGGTTGA TGCGCATCGC GGTAATGTAC

601 TGTTTCATCG TCAATTGCAT GACCCGCTGG AATATCCCCA TTGCATAGTT GGCGTTAAGT

661 TTGACGTGCT CAGCCACATC GTTGATGGTC AGCGCCTGAT CATAGTTTTC GGCAATAAAG

721 CCCAGCATCT GGCTAACATA AAATTGCGCA TGGCGCGAGA CGCTGTTTTT GTGTGTGCGC

781 GAGGTTTTAT TGACCAGAAT CGGTTCCCAG CCAGAGAGGC TAAATCGCTT GAGCATCAGG

841 CCAATTTCAT CAATGGCGAG CTGGCGAATT TGCTCGTTCG GACTGTTTAA TTCCTGCTGC

901 CAGCGGCGCA CTTCAAACGG GCTAAGTTGC TGTGTGGCCA GTGATTTGAT CACCATGCCG

961 TGAGTGACGT GGTTAATCAG GTCTTTATCC AGCGGCCAGG AGAGAAACAG ATGCATCGGC

1021 AGATTAAAAA TCGCCATGCT CTGACAGGTT CCGGTATCTG TTAGTTGGTG CGGTGTACAG

1081 GCCCAGAACA GCGTGATATG ACCCTGATTG ATATTCACTT TTTCATTGTT GATCAGGTAT

1141 TCCACATCGC CATCGAAAGG CACATTCACT TCGACCTGAC CATGCCAGTG GCTGGTGGGC

1201 ATGATATGCG GTGCGCGAAA CTCAATCTCC ATCCGCTGGT ATTCCGAATA CAGCGACAGC

+1

MelR

1261 GGGCTGCGGG TCTGTTTTTC GTCGCTGCTG CACATAAACG TATCTGTATT CATGGATGGC

1321 TCTCTTTCCT GGAATATCAG AATTATGGCA GGAGTGAGGG AGGATGACTG CGAGTGGGAG

1381 CACGGTTTTC ACCCTCTTCC CAGAGGGGCG AGGGGACTCT CCGAGTATCA TGAGGCCGAA

1441 AACTCTGCTT TTCAGGTAAT TTATTCCCAT AAACTCAGAT TTACTGCTGC TTCACGCAGG

1501 ATCTGAGTTT ATGGGAATGC TCAACCTGGA AGCCGGAGGT TTTCTGCAGA TTCGCCTGCC

SD SalI XbaI

1561 ATGATGAAGT TATTCAAGCA AGCCAGGAGG TCGTCGACTC TAGAGGATCC CCGCGCCCTC

Stem-loop KpnI

1621 ATCCGAAAGG GCGTATTGGT ACCGAGCTCG AATTCGTAAT CATGGTCATA GCTGTTTCCT

1681 GTGTGAAATT GTTATCCGCT CACAATTCCA CACAACATAC GAGCCGGAAG CATAAAGTGT

1741 AAAGCCTGGG GTGCCTAATG AGTGAGCTAA CTCACATTAA TTGCGTTGCG CTCACTGCCC

1801 GCTTTCCAGT CGGGAAACCT GTCGTGCCAG CTGCATTAAT GAATCGGCCA ACGCGCGGGG

1861 AGAGGCGGTT TGCGTATTGG GCGCTCTTCC GCTTCCTCGC TCACTGACTC GCTGCGCTCG

1921 GTCGTTCGGC TGCGGCGAGC GGTATCAGCT CACTCAAAGG CGGTAATACG GTTATCCACA

1981 GAATCAGGGG ATAACGCAGG AAAGAACATG TGAGCAAAAG GCCAGCAAAA GGCCAGGAAC

2041 CGTAAAAAGG CCGCGTTGCT GGCGTTTTTC CATAGGCTCC GCCCCCCTGA CGAGCATCAC

2101 AAAAATCGAC GCTCAAGTCA GAGGTGGCGA AACCCGACAG GACTATAAAG ATACCAGGCG

2161 TTTCCCCCTG GAAGCTCCCT CGTGCGCTCT CCTGTTCCGA CCCTGCCGCT TACCGGATAC

2221 CTGTCCGCCT TTCTCCCTTC GGGAAGCGTG GCGCTTTCTC ATAGCTCACG CTGTAGGTAT

2281 CTCAGTTCGG TGTAGGTCGT TCGCTCCAAG CTGGGCTGTG TGCACGAACC CCCCGTTCAG

2341 CCCGACCGCT GCGCCTTATC CGGTAACTAT CGTCTTGAGT CCAACCCGGT AAGACACGAC

2401 TTATCGCCAC TGGCAGCAGC CACTGGTAAC AGGATTAGCA GAGCGAGGTA TGTAGGCGGT

2461 GCTACAGAGT TCTTGAAGTG GTGGCCTAAC TACGGCTACA CTAGAAGGAC AGTATTTGGT

2521 ATCTGCGCTC TGCTGAAGCC AGTTACCTTC GGAAAAAGAG TTGGTAGCTC TTGATCCGGC

2581 AAACAAACCA CCGCTGGTAG CGGTGGTTTT TTTGTTTGCA AGCAGCAGAT TACGCGCAGA

2641 AAAAAAGGAT CTCAAGAAGA TCCTTTGATC TTTTCTACGG GGTCTGACGC TCAGTGGAAC

2701 GAAAACTCAC GTTAAGGGAT TTTGGTCATG AGATTATCAA AAAGGATCTT CACCTAGATC

2761 CTTTTAAATT AAAAATGAAG TTTTAAATCA ATCTAAAGTA TATATGAGTA AACTTGGTCT

2821 GACAGTTACC AATGCTTAAT CAGTGAGGCA CCTATCTCAG CGATCTGTCT ATTTCGTTCA

2881 TCCATAGTTG CCTGACTCCC CGTCGTGTAG ATAACTACGA TACGGGAGGG CTTACCATCT

2941 GGCCCCAGTG CTGCAATGAT ACCGCGAGAC CCACGCTCAC CGGCTCCAGA TTTATCAGCA

3001 ATAAACCAGC CAGCCGGAAG GGCCGAGCGC AGAAGTGGTC CTGCAACTTT ATCCGCCTCC

3061 ATCCAGTCTA TTAATTGTTG CCGGGAAGCT AGAGTAAGTA GTTCGCCAGT TAATAGTTTG

3121 CGCAACGTTG TTGCCATTGC TACAGGCATC GTGGTGTCAC GCTCGTCGTT TGGTATGGCT

3181 TCATTCAGCT CCGGTTCCCA ACGATCAAGG CGAGTTACAT GATCCCCCAT GTTGTGCAAA

3241 AAAGCGGTTA GCTCCTTCGG TCCTCCGATC GTTGTCAGAA GTAAGTTGGC CGCAGTGTTA

3301 TCACTCATGG TTATGGCAGC ACTGCATAAT TCTCTTACTG TCATGCCATC CGTAAGATGC

3361 TTTTCTGTGA CTGGTGAGTA CTCAACCAAG TCATTCTGAG AATAGTGTAT GCGGCGACCG

3421 AGTTGCTCTT GCCCGGCGTC AATACGGGAT AATACCGCGC CACATAGCAG AACTTTAAAA

3481 GTGCTCATCA TTGGAAAACG TTCTTCGGGG CGAAAACTCT CAAGGATCTT ACCGCTGTTG

3541 AGATCCAGTT CGATGTAACC CACTCGTGCA CCCAACTGAT CTTCAGCATC TTTTACTTTC

3601 ACCAGCGTTT CTGGGTGAGC AAAAACAGGA AGGCAAAATG CCGCAAAAAA GGGAATAAGG

3661 GCGACACGGA AATGTTGAAT ACTCATACTC TTCCTTTTTC AATATTATTG AAGCATTTAT

3721 CAGGGTTATT GTCTCATGAG CGGATACATA TTTGAATGTA TTTAGAAAAA TAAACAAATA

3781 GGGGTTCCGC GCACATTTCC CCGAAAAGTG CCACCTGACG TCTAAGAAAC CATTATTATC

3841 ATGACATTAA CCTATAAAAA TAGGCGTATC ACGAGGCCCT TTCGTC

### SEQ ID NO.: 166

### MalE (1-370) Factor Xa NTR (43-424) FLAG

### SalI +1 MalE (1-370)

1 GTCGACATGAAAATAAAAACAGGTGCACGCATCCTCGCATTATCCGCATTAACGACGATGATGTTT

1 M K I K T G A R I L A L S A L T T M M F

61 TCCGCCTCGGCTCTCGCCAAAATCGAAGAAGGTAAACTGGTAATCTGGATTAACGGCGAT

21 S A S A L A K I E E G K L V I W I N G D

121 AAAGGCTATAACGGTCTCGCTGAAGTCGGTAAGAAATTCGAGAAAGATACCGGAATTAAA

41 K G Y N G L A E V G K K F E K D T G I K

181 GTCACCGTTGAGCATCCGGATAAACTGGAAGAGAAATTCCCACAGGTTGCGGCAACTGGC

61 V T V E H P D K L E E K F P Q V A A T G

241 GATGGCCCTGACATTATCTTCTGGGCACACGACCGCTTTGGTGGCTACGCTCAATCTGGC

81 D G P D I I F W A H D R F G G Y A Q S G

301 CTGTTGGCTGAAATCACCCCGGACAAAGCGTTCCAGGACAAGCTGTATCCGTTTACCTGG

101 L L A E I T P D K A F Q D K L Y P F T W

361 GATGCCGTACGTTACAACGGCAAGCTGATTGCTTACCCGATCGCTGTTGAAGCGTTATCG

121 D A V R Y N G K L I A Y P I A V E A L S

421 CTGATTTATAACAAAGATCTGCTGCCGAACCCGCCAAAAACCTGGGAAGAGATCCCGGCG

141 L I Y N K D L L P N P P K T W E E I P A

481 CTGGATAAAGAACTGAAAGCGAAAGGTAAGAGCGCGCTGATGTTCAACCTGCAAGAACCG

161 L D K E L K A K G K S A L M F N L Q E P

541 TACTTCACCTGGCCGCTGATTGCTGCTGACGGGGGTTATGCGTTCAAGTATGAAAACGGC

181 Y F T W P L I A A D G G Y A F K Y E N G

601 AAGTACGACATTAAAGACGTGGGCGTGGATAACGCTGGCGCGAAAGCGGGTCTGACCTTC

201 K Y D I K D V G V D N A G A K A G L T F

661 CTGGTTGACCTGATTAAAAACAAACACATGAATGCAGACACCGATTACTCCATCGCAGAA

221 L V D L I K N K H M N A D T D Y S I A E

721 GCTGCCTTTAATAAAGGCGAAACAGCGATGACCATCAACGGCCCGTGGGCATGGTCCAAC

241 A A F N K G E T A M T I N G P W A W S N

781 ATCGACACCAGCAAAGTGAATTATGGTGTAACGGTACTGCCGACCTTCAAGGGTCAACCA

261 I D T S K V N Y G V T V L P T F K G Q P

841 TCCAAACCGTTCGTTGGCGTGCTGAGCGCAGGTATTAACGCCGCCAGTCCGAACAAAGAG

281 S K P F V G V L S A G I N A A S P N K E

901 CTGGCGAAAGAGTTCCTCGAAAACTATCTGCTGACTGATGAAGGTCTGGAAGCGGTTAAT

301 L A K E F L E N Y L L T D E G L E A V N

961 AAAGACAAACCGCTGGGTGCCGTAGCGCTGAAGTCTTACGAGGAAGAGTTGGCGAAAGAT

321 K D K P L G A V A L K S Y E E E L A K D

1021 CCACGTATTGCCGCCACCATGGAAAACGCCCAGAAAGGTGAAATCATGCCGAACATCCCG

341 P R I A A T M E N A Q K G E I M P N I P

Factor Xa +43 NTR

1081 CAGATGTCCGCTTTCTGGTATGCCGTGCTGATCGAAGCCCGCACCTCGGAATCCGACACG

361 Q M S A F W Y A V L I E A R T S E S D T

1141 GCAGGGCCCAACAGCGACCTGGACGTGAACACTGACATTTATTCCAAGGTGCTGGTGACT

381 A G P N S D L D V N T D I Y S K V L V T

1201 GCTATATACCTGGCACTCTTCGTGGTGGGCACTGTGGGCAACTCCGTGACAGCCTTCACT

401 A I Y L A L F V V G T V G N S V T A F T

1261 CTAGCGCGGAAGAAGTCACTGCAGAGCCTGCAGAGCACTGTGCATTACCACCTGGGCAGC

421 L A R K K S L Q S L Q S T V H Y H L G S

1321 CTGGCACTGTCGGACCTGCTTATCCTTCTGCTGGCCATGCCCGTGGAGCTATACAACTTC

441 L A L S D L L I L L L A M P V E L Y N F

1381 ATCTGGGTACACCATCCCTGGGCCTTTGGGGACGCTGGCTGCCGTGGCTACTATTTCCTG

461 I W V H H P W A F G D A G C R G Y Y F L

1441 CGTGATGCCTGCACCTATGCCACAGCCCTCAATGTAGCCAGCCTGAGTGTGGAGCGCTAC

481 R D A C T Y A T A L N V A S L S V E R Y

1501 TTGGCCATCTGCCATCCCTTCAAGGCCAAGACCCTCATGTCCCGCAGCCGCACCAAGAAA

501 L A I C H P F K A K T L M S R S R T K K

1561 TTCATCAGTGCCATATGGCTAGCTTCGGCGCTGCTGGCTATACCCATGCTTTTCACCATG

521 F I S A I W L A S A L L A I P M L F T M

1621 GGCCTGCAGAACCGCAGTGGTGACGGCACGCACCCTGGCGGCCTGGTGTGCACACCCATT

541 G L Q N R S G D G T H P G G L V C T P I

1681 GTGGACACAGCCACTGTCAAGGTCGTCATCCAGGTTAACACCTTCATGTCCTTCCTGTTT

561 V D T A T V K V V I Q V N T F M S F L F

1741 CCCATGTTGGTCATCTCCATCCTAAACACCGTGATTGCCAACAAACTGACAGTCATGGTG

581 P M L V I S I L N T V I A N K L T V M V

1801 CACCAGGCCGCCGAGCAGGGCCGAGTGTGCACCGTGGGCACACACAACGGTTTAGAGCAC

601 H Q A A E Q G R V C T V G T H N G L E H1861
AGCACGTTCAACATGACCATCGAGCCGGGTCGTGTCCAGGCCCTGCGCCACGGAGTCCTC

621 S T F N M T I E P G R V Q A L R H G V L

1921 GTCTTACGTGCTGTGGTCATTGCCTTTGTGGTCTGCTGGCTGCCCTACCACGTGCGACGC

641 V L R A V V I A F V V C W L P Y H V R R

1981 CTGATGTTCTGCTATATCTCGGATGAACAGTGGACTACGTTCCTCTTCGATTTCTACCAC

661 L M F C Y I S D E Q W T T F L F D F Y H

2041 TATTTCTACATGCTAACCAACGCTCTCTTCTACGTCAGCTCCGCCATCAATCCCATCCTC

681 Y F Y M L T N A L F Y V S S A I N P I L

2101 TACAACCTGGTCTCCGCCAACTTCCGCCAGGTCTTTCTGTCCACGCTGGCCTGCCTTTGT

701 Y N L V S A N F R Q V F L S T L A C L C

2161 CCTGGGTGGCGCCACCGCCGAAAGAAGAGGCCAACGTTCTCCAGGAAGCCCAACAGCATG

721 P G W R H R R K K R P T F S R K P N S M

NotI

2221 TCCAGCAACCATGCCTTTTCCACCAGCGCCACCCGGGAGACCCTGTACgcggccgca

741 S S N H A F S T S A T R E T L Y A A A

Flag stop KpnI

GATTATAAAGATGACGATGACAAATAATAAGGTACC

D Y K D D D D K * *

### SEO ID NO.: 167

### MalE (1-28) Factor Xa NTR (43-424) FLAG

### SalI +1 MalE leader (1-28)

1 gtcgacATGAAAATAAAAACAGGTGCACGCATCCTCGCATTATCCGCATTAACGACGATGATGTTT

1 M K I K T G A R I L A L S A L T T M M F

Factor Xa +43 NTR

61 TCCGCCTCGGCTCTCGCCAAAATCATCGAAGCCCGCACCTCGGAATCCGACACGGCAGGG

21 S A S A L A K I I E A R T S E S D T A G

121 CCCAACAGCGACCTGGACGTGAACACTGACATTTATTCCAAGGTGCTGGTGACTGCTATA

41 P N S D L D V N T D I Y S K V L V T A I

181 TACCTGGCACTCTTCGTGGTGGGCACTGTGGGCAACTCCGTGACAGCCTTCACTCTAGCG

61 Y L A L F V V G T V G N S V T A F T L A

241 CGGAAGAAGTCACTGCAGAGCCTGCAGAGCACTGTGCATTACCACCTGGGCAGCCTGGCA

81 R K K S L Q S L Q S T V H Y H L G S L A

301 CTGTCGGACCTGCTTATCCTTCTGCTGGCCATGCCCGTGGAGCTATACAACTTCATCTGG

101 L S D L L I L L L A M P V E L Y N F I W

361 GTACACCATCCCTGGGCCTTTGGGGACGCTGGCTGCCGTGGCTACTATTTCCTGCGTGAT

121 V H H P W A F G D A G C R G Y Y F L R D

421 GCCTGCACCTATGCCACAGCCCTCAATGTAGCCAGCCTGAGTGTGGAGCGCTACTTGGCC

141 A C T Y A T A L N V A S L S V E R Y L A

481 ATCTGCCATCCCTTCAAGGCCAAGACCCTCATGTCCCGCAGCCGCACCAAGAAATTCATC

161 I C H P F K A K T L M S R S R T K K F I

541 AGTGCCATATGGCTAGCTTCGGCGCTGCTGGCTATACCCATGCTTTTCACCATGGGCCTG

181 S A I W L A S A L L A I P M L F T M G L

601 CAGAACCGCAGTGGTGACGGCACGCACCCTGGCGGCCTGGTGTGCACACCCATTGTGGAC

201 Q N R S G D G T H P G G L V C T P I V D

661 ACAGCCACTGTCAAGGTCGTCATCCAGGTTAACACCTTCATGTCCTTCCTGTTTCCCATG

221 T A T V K V V I Q V N T F M S F L F P M

721 TTGGTCATCTCCATCCTAAACACCGTGATTGCCAACAAACTGACAGTCATGGTGCACCAG

241 L V I S I L N T V I A N K L T V M V H Q

781 GCCGCCGAGCAGGGCCGAGTGTGCACCGTGGGCACACACAACGGTTTAGAGCACAGCACG

261 A A E Q G R V C T V G T H N G L E H S T

841 TTCAACATGACCATCGAGCCGGGTCGTGTCCAGGCCCTGCGCCACGGAGTCCTCGTCTTA

281 F N M T I E P G R V Q A L R H G V L V L

901 CGTGCTGTGGTCATTGCCTTTGTGGTCTGCTGGCTGCCCTACCACGTGCGACGCCTGATG

301 R A V V I A F V V C W L P Y H V R R L M

961 TTCTGCTATATCTCGGATGAACAGTGGACTACGTTCCTCTTCGATTTCTACCACTATTTC

321 F C Y I S D E Q W T T F L F D F Y H Y F

1021 TACATGCTAACCAACGCTCTCTTCTACGTCAGCTCCGCCATCAATCCCATCCTCTACAAC

341 Y M L T N A L F Y V S S A I N P I L Y N

1081 CTGGTCTCCGCCAACTTCCGCCAGGTCTTTCTGTCCACGCTGGCCTGCCTTTGTCCTGGG

361 L V S A N F R Q V F L S T L A C L C P G

1141 TGGCGCCACCGCCGAAAGAAGAGGCCAACGTTCTCCAGGAAGCCCAACAGCATGTCCAGC

381 W R H R R K K R P T F S R K P N S M S S

NotI Flag

1201 AACCATGCCTTTTCCACCAGCGCCACCCGGGAGACCCTGTACgcggccgcaGATTATAAA

401 N H A F S T S A T R E T L Y A A A D Y K

stop KpnI

GATGACGATGACAAATAATAAGGTACC

D D D D K

### SEQ ID NO.: 169

### MalE (1-370) Factor Xa NTR (43-424) TrxA (2-109) FLAG

### SalI +1 MalE (1-370)

1 GTCGACATGAAAATAAAAACAGGTGCACGCATCCTCGCATTATCCGCATTAACGACGATGATGTTT

1 M K I K T G A R I L A L S A L T T M M F

61 TCCGCCTCGGCTCTCGCCAAAATCGAAGAAGGTAAACTGGTAATCTGGATTAACGGCGAT

21 S A S A L A K I E E G K L V I W I N G D

121 AAAGGCTATAACGGTCTCGCTGAAGTCGGTAAGAAATTCGAGAAAGATACCGGAATTAAA

41 K G Y N G L A E V G K K F E K D T G I K

181 GTCACCGTTGAGCATCCGGATAAACTGGAAGAGAAATTCCCACAGGTTGCGGCAACTGGC

61 V T V E H P D K L E E K F P Q V A A T G

241 GATGGCCCTGACATTATCTTCTGGGCACACGACCGCTTTGGTGGCTACGCTCAATCTGGC

81 D G P D I I F W A H D R F G G Y A Q S G

301 CTGTTGGCTGAAATCACCCCGGACAAAGCGTTCCAGGACAAGCTGTATCCGTTTACCTGG

101 L L A E I T P D K A F Q D K L Y P F T W

361 GATGCCGTACGTTACAACGGCAAGCTGATTGCTTACCCGATCGCTGTTGAAGCGTTATCG

121 D A V R Y N G K L I A Y P I A V E A L S

421 CTGATTTATAACAAAGATCTGCTGCCGAACCCGCCAAAAACCTGGGAAGAGATCCCGGCG

141 L I Y N K D L L P N P P K T W E E I P A

481 CTGGATAAAGAACTGAAAGCGAAAGGTAAGAGCGCGCTGATGTTCAACCTGCAAGAACCG

161 L D K E L K A K G K S A L M F N L Q E P

541 TACTTCACCTGGCCGCTGATTGCTGCTGACGGGGGTTATGCGTTCAAGTATGAAAACGGC

181 Y F T W P L I A A D G G Y A F K Y E N G

601 AAGTACGACATTAAAGACGTGGGCGTGGATAACGCTGGCGCGAAAGCGGGTCTGACCTTC

201 K Y D I K D V G V D N A G A K A G L T F

661 CTGGTTGACCTGATTAAAAACAAACACATGAATGCAGACACCGATTACTCCATCGCAGAA

221 L V D L I K N K H M N A D T D Y S I A E

721 GCTGCCTTTAATAAAGGCGAAACAGCGATGACCATCAACGGCCCGTGGGCATGGTCCAAC

241 A A F N K G E T A M T I N G P W A W S N

781 ATCGACACCAGCAAAGTGAATTATGGTGTAACGGTACTGCCGACCTTCAAGGGTCAACCA

261 I D T S K V N Y G V T V L P T F K G Q P

841 TCCAAACCGTTCGTTGGCGTGCTGAGCGCAGGTATTAACGCCGCCAGTCCGAACAAAGAG

281 S K P F V G V L S A G I N A A S P N K E

901 CTGGCGAAAGAGTTCCTCGAAAACTATCTGCTGACTGATGAAGGTCTGGAAGCGGTTAAT

301 L A K E F L E N Y L L T D E G L E A V N

961 AAAGACAAACCGCTGGGTGCCGTAGCGCTGAAGTCTTACGAGGAAGAGTTGGCGAAAGAT

321 K D K P L G A V A L K S Y E E E L A K D

1021 CCACGTATTGCCGCCACCATGGAAAACGCCCAGAAAGGTGAAATCATGCCGAACATCCCG

341 P R I A A T M E N A Q K G E I M P N I P

Factor Xa +43 NTR

1081 CAGATGTCCGCTTTCTGGTATGCCGTGCTGATCGAAGCCCGCACCTCGGAATCCGACACG

361 Q M S A F W Y A V L I E A R T S E S D T

1141 GCAGGGCCCAACAGCGACCTGGACGTGAACACTGACATTTATTCCAAGGTGCTGGTGACT

381 A G P N S D L D V N T D I Y S K V L V T

1201 GCTATATACCTGGCACTCTTCGTGGTGGGCACTGTGGGCAACTCCGTGACAGCCTTCACT

401 A I Y L A L F V V G T V G N S V T A F T

1261 CTAGCGCGGAAGAAGTCACTGCAGAGCCTGCAGAGCACTGTGCATTACCACCTGGGCAGC

421 L A R K K S L Q S L Q S T V H Y H L G S

1321 CTGGCACTGTCGGACCTGCTTATCCTTCTGCTGGCCATGCCCGTGGAGCTATACAACTTC

441 L A L S D L L I L L L A M P V E L Y N F

1381 ATCTGGGTACACCATCCCTGGGCCTTTGGGGACGCTGGCTGCCGTGGCTACTATTTCCTG

461 I W V H H P W A F G D A G C R G Y Y F L

1441 CGTGATGCCTGCACCTATGCCACAGCCCTCAATGTAGCCAGCCTGAGTGTGGAGCGCTAC

481 R D A C T Y A T A L N V A S L S V E R Y

1501 TTGGCCATCTGCCATCCCTTCAAGGCCAAGACCCTCATGTCCCGCAGCCGCACCAAGAAA

501 L A I C H P F K A K T L M S R S R T K K

1561 TTCATCAGTGCCATATGGCTAGCTTCGGCGCTGCTGGCTATACCCATGCTTTTCACCATG

521 F I S A I W L A S A L L A I P M L F T M

1621 GGCCTGCAGAACCGCAGTGGTGACGGCACGCACCCTGGCGGCCTGGTGTGCACACCCATT

541 G L Q N R S G D G T H P G G L V C T P I

1681 GTGGACACAGCCACTGTCAAGGTCGTCATCCAGGTTAACACCTTCATGTCCTTCCTGTTT

561 V D T A T V K V V I Q V N T F M S F L F

1741 CCCATGTTGGTCATCTCCATCCTAAACACCGTGATTGCCAACAAACTGACAGTCATGGTG

581 P M L V I S I L N T V I A N K L T V M V

1801 CACCAGGCCGCCGAGCAGGGCCGAGTGTGCACCGTGGGCACACACAACGGTTTAGAGCAC

601 H Q A A E Q G R V C T V G T H N G L E H

1861 AGCACGTTCAACATGACCATCGAGCCGGGTCGTGTCCAGGCCCTGCGCCACGGAGTCCTC

621 S T F N M T I E P G R V Q A L R H G V L

1921 GTCTTACGTGCTGTGGTCATTGCCTTTGTGGTCTGCTGGCTGCCCTACCACGTGCGACGC

641 V L R A V V I A F V V C W L P Y H V R R

1981 CTGATGTTCTGCTATATCTCGGATGAACAGTGGACTACGTTCCTCTTCGATTTCTACCAC

661 L M F C Y I S D E Q W T T F L F D F Y H

2041 TATTTCTACATGCTAACCAACGCTCTCTTCTACGTCAGCTCCGCCATCAATCCCATCCTC

681 Y F Y M L T N A L F Y V S S A I N P I L

2101 TACAACCTGGTCTCCGCCAACTTCCGCCAGGTCTTTCTGTCCACGCTGGCCTGCCTTTGT

701 Y N L V S A N F R Q V F L S T L A C L C

2161 CCTGGGTGGCGCCACCGCCGAAAGAAGAGGCCAACGTTCTCCAGGAAGCCCAACAGCATG

721 P G W R H R R K K R P T F S R K P N S M

NotI +2 TrxA

2221 TCCAGCAACCATGCCTTTTCCACCAGCGCCACCCGGGAGACCCTGTACgcggccgcaAGC

741 S S N H A F S T S A T R E T L Y A A A S

2281 GATAAAATTATTCACCTGACTGACGACAGTTTTGACACGGATGTACTCAAAGCGGACGGG

761 D K I I H L T D D S F D T D V L K A D G

2341 GCGATCCTCGTCGATTTCTGGGCAGAGTGGTGCGGTCCGTGCAAAATGATCGCCCCGATT

781 A I L V D F W A E W C G P C K M I A P I

2401 CTGGATGAAATCGCTGACGAATATCAGGGCAAACTGACCGTTGCAAAACTGAACATCGAT

801 L D E I A D E Y Q G K L T V A K L N I D

2461 CAAAACCCTGGCACTGCGCCGAAATATGGCATCCGTGGTATCCCGACTCTGCTGCTGTTC

821 Q N P G T A P K Y G I R G I P T L L L F

2521 AAAAACGGTGAAGTGGCGGCAACCAAAGTGGGTGCACTGTCTAAAGGTCAGTTGAAAGAG

841 K N G E V A A T K V G A L S K G Q L K E

NotI +2 Flag stop

2581 TTCCTCGACGCTAACCTGGCGgcggccgcaGATTATAAAGATGACGATGACAAATAATAA

861 F L D A N L A A A A D Y K D D D D K * *

KpnI

2641 GGTACC

### SEO ID NO.: 170

### MalE (1-28) Factor Xa NTR (43-424) TrxA (2-109) FLAG

### SalI +1 MalE leader (1-28)

1 gtcgacATGAAAATAAAAACAGGTGCACGCATCCTCGCATTATCCGCATTAACGACGATGATGTTT

1 M K I K T G A R I L A L S A L T T M M F

Factor Xa +43 NTR

61 TCCGCCTCGGCTCTCGCCAAAATCATCGAAGCCCGCACCTCGGAATCCGACACGGCAGGG

21 S A S A L A K I I E A R T S E S D T A G

121 CCCAACAGCGACCTGGACGTGAACACTGACATTTATTCCAAGGTGCTGGTGACTGCTATA

41 P N S D L D V N T D I Y S K V L V T A I

181 TACCTGGCACTCTTCGTGGTGGGCACTGTGGGCAACTCCGTGACAGCCTTCACTCTAGCG

61 Y L A L F V V G T V G N S V T A F T L A

241 CGGAAGAAGTCACTGCAGAGCCTGCAGAGCACTGTGCATTACCACCTGGGCAGCCTGGCA

81 R K K S L Q S L Q S T V H Y H L G S L A

301 CTGTCGGACCTGCTTATCCTTCTGCTGGCCATGCCCGTGGAGCTATACAACTTCATCTGG

101 L S D L L I L L L A M P V E L Y N F I W

361 GTACACCATCCCTGGGCCTTTGGGGACGCTGGCTGCCGTGGCTACTATTTCCTGCGTGAT

121 V H H P W A F G D A G C R G Y Y F L R D

421 GCCTGCACCTATGCCACAGCCCTCAATGTAGCCAGCCTGAGTGTGGAGCGCTACTTGGCC

141 A C T Y A T A L N V A S L S V E R Y L A

481 ATCTGCCATCCCTTCAAGGCCAAGACCCTCATGTCCCGCAGCCGCACCAAGAAATTCATC

161 I C H P F K A K T L M S R S R T K K F I

541 AGTGCCATATGGCTAGCTTCGGCGCTGCTGGCTATACCCATGCTTTTCACCATGGGCCTG

181 S A I W L A S A L L A I P M L F T M G L

601 CAGAACCGCAGTGGTGACGGCACGCACCCTGGCGGCCTGGTGTGCACACCCATTGTGGAC

201 Q N R S G D G T H P G G L V C T P I V D

661 ACAGCCACTGTCAAGGTCGTCATCCAGGTTAACACCTTCATGTCCTTCCTGTTTCCCATG

221 T A T V K V V I Q V N T F M S F L F P M

721 TTGGTCATCTCCATCCTAAACACCGTGATTGCCAACAAACTGACAGTCATGGTGCACCAG

241 L V I S I L N T V I A N K L T V M V H Q

781 GCCGCCGAGCAGGGCCGAGTGTGCACCGTGGGCACACACAACGGTTTAGAGCACAGCACG

261 A A E Q G R V C T V G T H N G L E H S T

841 TTCAACATGACCATCGAGCCGGGTCGTGTCCAGGCCCTGCGCCACGGAGTCCTCGTCTTA

281 F N M T I E P G R V Q A L R H G V L V L

901 CGTGCTGTGGTCATTGCCTTTGTGGTCTGCTGGCTGCCCTACCACGTGCGACGCCTGATG

301 R A V V I A F V V C W L P Y H V R R L M

961 TTCTGCTATATCTCGGATGAACAGTGGACTACGTTCCTCTTCGATTTCTACCACTATTTC

321 F C Y I S D E Q W T T F L F D F Y H Y F

1021 TACATGCTAACCAACGCTCTCTTCTACGTCAGCTCCGCCATCAATCCCATCCTCTACAAC

341 Y M L T N A L F Y V S S A I N P I L Y N1081
CTGGTCTCCGCCAACTTCCGCCAGGTCTTTCTGTCCACGCTGGCCTGCCTTTGTCCTGGG

361 L V S A N F R Q V F L S T L A C L C P G

1141 TGGCGCCACCGCCGAAAGAAGAGGCCAACGTTCTCCAGGAAGCCCAACAGCATGTCCAGC

381 W R H R R K K R P T F S R K P N S M S S

NotI +2 TrxA

1201 AACCATGCCTTTTCCACCAGCGCCACCCGGGAGACCCTGTACgcggccgcaAGCGATAAA

401 N H A F S T S A T R E T L Y A A A S D K

1261 ATTATTCACCTGACTGACGACAGTTTTGACACGGATGTACTCAAAGCGGACGGGGCGATC

421 I I H L T D D S F D T D V L K A D G A I

1321 CTCGTCGATTTCTGGGCAGAGTGGTGCGGTCCGTGCAAAATGATCGCCCCGATTCTGGAT

441 L V D F W A E W C G P C K M I A P I L D

1381 GAAATCGCTGACGAATATCAGGGCAAACTGACCGTTGCAAAACTGAACATCGATCAAAAC

461 E I A D E Y Q G K L T V A K L N I D Q N

1441 CCTGGCACTGCGCCGAAATATGGCATCCGTGGTATCCCGACTCTGCTGCTGTTCAAAAAC

481 P G T A P K Y G I R G I P T L L L F K N

1501 GGTGAAGTGGCGGCAACCAAAGTGGGTGCACTGTCTAAAGGTCAGTTGAAAGAGTTCCTC

501 G E V A A T K V G A L S K G Q L K E F L

NotI Flag stop KpnI

1561 GACGCTAACCTGGCAgcggccgcaGATTATAAAGATGACGATGACAAATAATAAGGTACC

521 D A N L A A A D Y K D D D D K

### SEQ ID NO.: 188

### Human □2AR GS1□ chimeric fusion

### SalI +1 B2AR

1 GTCGACATGG GGCAACCCGG GAACGGCAGC GCCTTCTTGC TGGCACCCAA TGGAAGCCAT

61 GCGCCGGACC ACGACGTCAC GCAGCAAAGG GACGAGGTGT GGGTGGTGGG
CATGGGCATC

121 GTCATGTCTC TCATCGTCCT GGCCATCGTG TTTGGCAATG TGCTGGTCAT CACAGCCATT

181 GCCAAGTTCG AGCGTCTGCA GACGGTCACC AACTACTTCA TCACTTCACT GGCCTGTGCT

241 GATCTGGTCA TGGGCCTAGC AGTGGTGCCC TTTGGGGCCG CCCATATTCT TATGAAAATG

301 TGGACTTTTG GCAACTTCTG GTGCGAGTTT TGGACTTCCA TTGATGTGCT GTGCGTCACG

361 GCCAGCATTG AGACCCTGTG CGTGATCGCA GTGGATCGCT ACTTTGCCAT TACTTCACCT

421 TTCAAGTACC AGAGCCTGCT GACCAAGAAT AAGGCCCGGG TGATCATTCT GATGGTGTGG

481 ATTGTGTCAG GCCTTAYCTC CTTCTTGCCC ATTCAGATGC ACTGGTACAG GGCCACCCAC

541 CAGGAAGCCA TCAACTGCTA TGCCAATGAG ACCTGCTGTG ACTTCTTCAC GAACCAAGCC

601 TATGCCATTG CCTCTTCCAT CGTGTCCTTC TACGTTCCCC TGGTGATCAT GGTCTTCGTC

661 TACTCCAGGG TCTTTCAGGA GGCCAAAAGG CAGCTCCAGA AGATTGACAA ATCTGAGGGC

721 CGCTTCCATG TCCAGAACCT TAGCCAGGTG GAGCAGGATG GGCGGACGGG GCATGGACTC

781 CGCAGATCTT CCAAGTTCTG CTTGAAGGAG CACAAAGCCC TCAAGACGTT AGGCATCATC

841 ATGGGCACTT TCACCCTCTG CTGGCTGCCC TTCTTCATCG TTAACATTGT GCATGTGATC

901 CAGGATAACC TCATCCGTAA GGAAGTTTAC ATCCTCCTAA ATTGGATAGG CTATGTCAAT

961 TCTGGTTTCA ATCCCCTTAT CTACTGCCGG AGCCCAGATT TCAGGATTGC CTTCCAGGAG

1021 CTTCTGTGCC TGCGCAGGTC TTCTTTGAAG GCCTATGGCA ATGGCTACTC CAGCAACGGC

1081 AACACAGGGG AGCAGAGTGG ATATCACGTG GAACAGGAGA AAGAAAATAA ACTGCTGTGT

1141 GAAGACCTCC CAGGCACGGA AGACTTTGTG GGCCATCAAG GTACTGTGCC TAGCGATAAC

Last B2AR Linker

sequence

1201 ATTGATTCAC AAGGGAGGAA TTGTAGTACA AATGACTCAC TGCTAGAGCG TGGCCAGACG

PstI XhoI +2 GS1 alpha

1261 GTCACCAACC TGCAGCTCGA GGGCTGCCTC GGGAACAGTA AGACCGAGGA

CCAGCGCAAC

1321 GAGGAGAAGG CGCAGCGTGA GGCCAACAAA AAGATCGAGA AGCAGCTGCA

GAAGGACAAG

1381 CAGGTCTACC GGGCCACGCA CCGCCTGCTG CTGCTGGGTG CTGGAGAATC TGGTAAAAGC

1441 ACCATTGTGA AGCAGATGAG GATCCTGCAT GTTAATGGGT TTAATGGAGA CAGTGAGAAG

1501 GCAACCAAAG TGCAGGACAT CAAAAACAAC CTGAAAGAGG CGATTGAAAC CATTGTGGCC

1561 GCCATGAGCA ACCTGGTGCC CCCCGTGGAG CTGGCCAACC CCGAGAACCA

GTTCAGAGTG

1621 GACTACATCC TGAGTGTGAT GAACGTGCCT GACTTTGACT TCCCTCCCGA ATTCTATGAG

1681 CATGCCAAGG CTCTGTGGGA GGATGAAGGA GTGCGTGCCT GCTACGAACG CTCCAACGAG

1741 TACCAGCTGA TTGACTGTGC CCAGTACTTC CTGGACAAGA TCGACGTGAT CAAGCAGGCT

1801 GACTATGTGC CGAGCGATCA GGACCTGCTT CGCTGCCGTG TCCTGACTTC TGGAATCTTT

1861 GAGACCAAGT TCCAGGTGGA CAAAGTCAAC TTCCACATGT TTGACGTGGG TGGCCAGCGC

1921 GATGAACGCC GCAAGTGGAT CCAGTGCTTC AACGATGTGA CTGCCATCAT CTTCGTGGTG

1981 GCCAGCAGCA GCTACAACAT GGTCATCCGG GAGGACAACC AGACCAACCG

CCTGCAGGAG

2041 GCTCTGAACC TCTTCAAGAG CATCTGGAAC AACAGATGGC TGCGCACCAT CTCTGTGATC

2101 CTGTTCCTCA ACAAGCAAGA TCTGCTCGCT GAGAAAGTCC TTGCTGGGAA ATCGAAGATT

2161 GAGGACTACT TTCCAGAATT TGCTCGCTAC ACTACTCCTG AGGATGCTAC TCCCGAGCCC

2221 GGAGAGGACC CACGCGTGAC CCGGGCCAAG TACTTCATTC GAGATGAGTT TCTGAGGATC

2281 AGCACTGCCA GTGGAGATGG GCGTCACTAC TGCTACCCTC ATTTCACCTG CGCTGTGGAC

2341 ACTGAGAACA TCCGCCGTGT GTTCAACGAC TGCCGTGACA TCATTCAGCG CATGCACCTT

ClaI Stop XbaI Stem-loop

2401 CGTCAGTACG AGCTGCTCAT CGATTAATAA TCTAGAGGAT CCCCGCGCCC TCATCCGAAA

2461 GGGCG

SEQ ID NO.: 190

Human [?]2AR stop GS1[?] transcriptional fusion

PstI +1 B2AR

1 GTCGACATGG GGCAACCCGG GAACGGCAGC GCCTTCTTGC TGGCACCCAA TGGAAGCCAT

61 GCGCCGGACC ACGACGTCAC GCAGCAAAGG GACGAGGTGT GGGTGGTGGG
CATGGGCATC

121 GTCATGTCTC TCATCGTCCT GGCCATCGTG TTTGGCAATG TGCTGGTCAT CACAGCCATT

181 GCCAAGTTCG AGCGTCTGCA GACGGTCACC AACTACTTCA TCACTTCACT GGCCTGTGCT

241 GATCTGGTCA TGGGCCTAGC AGTGGTGCCC TTTGGGGCCG CCCATATTCT TATGAAAATG

301 TGGACTTTTG GCAACTTCTG GTGCGAGTTT TGGACTTCCA TTGATGTGCT GTGCGTCACG

361 GCCAGCATTG AGACCCTGTG CGTGATCGCA GTGGATCGCT ACTTTGCCAT TACTTCACCT

421 TTCAAGTACC AGAGCCTGCT GACCAAGAAT AAGGCCCGGG TGATCATTCT GATGGTGTGG

481 ATTGTGTCAG GCCTTAYCTC CTTCTTGCCC ATTCAGATGC ACTGGTACAG GGCCACCCAC

541 CAGGAAGCCA TCAACTGCTA TGCCAATGAG ACCTGCTGTG ACTTCTTCAC GAACCAAGCC

601 TATGCCATTG CCTCTTCCAT CGTGTCCTTC TACGTTCCCC TGGTGATCAT GGTCTTCGTC

661 TACTCCAGGG TCTTTCAGGA GGCCAAAAGG CAGCTCCAGA AGATTGACAA ATCTGAGGGC

721 CGCTTCCATG TCCAGAACCT TAGCCAGGTG GAGCAGGATG GGCGGACGGG GCATGGACTC

781 CGCAGATCTT CCAAGTTCTG CTTGAAGGAG CACAAAGCCC TCAAGACGTT AGGCATCATC

841 ATGGGCACTT TCACCCTCTG CTGGCTGCCC TTCTTCATCG TTAACATTGT GCATGTGATC

901 CAGGATAACC TCATCCGTAA GGAAGTTTAC ATCCTCCTAA ATTGGATAGG CTATGTCAAT

961 TCTGGTTTCA ATCCCCTTAT CTACTGCCGG AGCCCAGATT TCAGGATTGC CTTCCAGGAG

1021 CTTCTGTGCC TGCGCAGGTC TTCTTTGAAG GCCTATGGCA ATGGCTACTC CAGCAACGGC

1081 AACACAGGGG AGCAGAGTGG ATATCACGTG GAACAGGAGA AAGAAAATAA ACTGCTGTGT

1141 GAAGACCTCC CAGGCACGGA AGACTTTGTG GGCCATCAAG GTACTGTGCC TAGCGATAAC

Last B2AR Linker

sequence

1201 ATTGATTCAC AAGGGAGGAA TTGTAGTACA AATGACTCAC TGCTAGAGCG TGGCCAGACG

PstI Stop SD XhoI +2 GS1 alpha

1261 GTCACCAACC TGCAGTAATA ATCAAGGAGG CCCTCGAGAT GGGCTGCCTC GGGAACAGTA

1321 AGACCGAGGA CCAGCGCAAC GAGGAGAAGG CGCAGCGTGA GGCCAACAAA

AAGATCGAGA

1381 AGCAGCTGCA GAAGGACAAG CAGGTCTACC GGGCCACGCA CCGCCTGCTG

CTGCTGGGTG

1441 CTGGAGAATC TGGTAAAAGC ACCATTGTGA AGCAGATGAG GATCCTGCAT GTTAATGGGT

1501 TTAATGGAGA CAGTGAGAAG GCAACCAAAG TGCAGGACAT CAAAAACAAC CTGAAAGAGG

1561 CGATTGAAAC CATTGTGGCC GCCATGAGCA ACCTGGTGCC CCCCGTGGAG CTGGCCAACC

1621 CCGAGAACCA GTTCAGAGTG GACTACATCC TGAGTGTGAT GAACGTGCCT GACTTTGACT

1681 TCCCTCCCGA ATTCTATGAG CATGCCAAGG CTCTGTGGGA GGATGAAGGA GTGCGTGCCT

1741 GCTACGAACG CTCCAACGAG TACCAGCTGA TTGACTGTGC CCAGTACTTC CTGGACAAGA

1801 TCGACGTGAT CAAGCAGGCT GACTATGTGC CGAGCGATCA GGACCTGCTT CGCTGCCGTG

1861 TCCTGACTTC TGGAATCTTT GAGACCAAGT TCCAGGTGGA CAAAGTCAAC TTCCACATGT

1921 TTGACGTGGG TGGCCAGCGC GATGAACGCC GCAAGTGGAT CCAGTGCTTC AACGATGTGA

1981 CTGCCATCAT CTTCGTGGTG GCCAGCAGCA GCTACAACAT GGTCATCCGG GAGGACAACC

2041 AGACCAACCG CCTGCAGGAG GCTCTGAACC TCTTCAAGAG CATCTGGAAC AACAGATGGC

2101 TGCGCACCAT CTCTGTGATC CTGTTCCTCA ACAAGCAAGA TCTGCTCGCT GAGAAAGTCC

2161 TTGCTGGGAA ATCGAAGATT GAGGACTACT TTCCAGAATT TGCTCGCTAC ACTACTCCTG

2221 AGGATGCTAC TCCCGAGCCC GGAGAGGACC CACGCGTGAC CCGGGCCAAG TACTTCATTC

2281 GAGATGAGTT TCTGAGGATC AGCACTGCCA GTGGAGATGG GCGTCACTAC TGCTACCCTC

2341 ATTTCACCTG CGCTGTGGAC ACTGAGAACA TCCGCCGTGT GTTCAACGAC TGCCGTGACA

ClaI Stop XbaI

2401 TCATTCAGCG CATGCACCTT CGTCAGTACG AGCTGCTCAT CGATTAATAA TCTAGAGGAT

Stem-loop

2461 CCCCGCGCCC TCATCCGAAA GGGCG

SEQ ID NO.: 192

Human GS1[?]

XhoI

1
CTCGAGATGGGCTGCCTCGGGAACAGTAAGACCGAGGACCAGCGCAACGAGGAGAAGGCGCAGCGT

1 M G C L G N S K T E D Q R N E E K A Q R

61 GAGGCCAACAAAAAGATCGAGAAGCAGCTGCAGAAGGACAAGCAGGTCTACCGGGCCACG

21 E A N K K I E K Q L Q K D K Q V Y R A T

121 CACCGCCTGCTGCTGCTGGGTGCTGGAGAATCTGGTAAAAGCACCATTGTGAAGCAGATG

41 H R L L L L G A G E S G K S T I V K Q M

181 AGGATCCTGCATGTTAATGGGTTTAATGGAGACAGTGAGAAGGCAACCAAAGTGCAGGAC

61 R I L H V N G F N G D S E K A T K V Q D

241 ATCAAAAACAACCTGAAAGAGGCGATTGAAACCATTGTGGCCGCCATGAGCAACCTGGTG

81 I K N N L K E A I E T I V A A M S N L V

301 CCCCCCGTGGAGCTGGCCAACCCCGAGAACCAGTTCAGAGTGGACTACATCCTGAGTGTG

101 P P V E L A N P E N Q F R V D Y I L S V

361 ATGAACGTGCCTGACTTTGACTTCCCTCCCGAATTCTATGAGCATGCCAAGGCTCTGTGG

121 M N V P D F D F P P E F Y E H A K A L W

421 GAGGATGAAGGAGTGCGTGCCTGCTACGAACGCTCCAACGAGTACCAGCTGATTGACTGT

141 E D E G V R A C Y E R S N E Y Q L I D C

481 GCCCAGTACTTCCTGGACAAGATCGACGTGATCAAGCAGGCTGACTATGTGCCGAGCGAT

161 A Q Y F L D K I D V I K Q A D Y V P S D

541 CAGGACCTGCTTCGCTGCCGTGTCCTGACTTCTGGAATCTTTGAGACCAAGTTCCAGGTG

181 Q D L L R C R V L T S G I F E T K F Q V

601 GACAAAGTCAACTTCCACATGTTTGACGTGGGTGGCCAGCGCGATGAACGCCGCAAGTGG

201 D K V N F H M F D V G G Q R D E R R K W

661 ATCCAGTGCTTCAACGATGTGACTGCCATCATCTTCGTGGTGGCCAGCAGCAGCTACAAC

221 I Q C F N D V T A I I F V V A S S S Y N

721 ATGGTCATCCGGGAGGACAACCAGACCAACCGCCTGCAGGAGGCTCTGAACCTCTTCAAG

241 M V I R E D N Q T N R L Q E A L N L F K

781 AGCATCTGGAACAACAGATGGCTGCGCACCATCTCTGTGATCCTGTTCCTCAACAAGCAA

261 S I W N N R W L R T I S V I L F L N K Q

841 GATCTGCTCGCTGAGAAAGTCCTTGCTGGGAAATCGAAGATTGAGGACTACTTTCCAGAA

281 D L L A E K V L A G K S K I E D Y F P E

901 TTTGCTCGCTACACTACTCCTGAGGATGCTACTCCCGAGCCCGGAGAGGACCCACGCGTG

301 F A R Y T T P E D A T P E P G E D P R V

961 ACCCGGGCCAAGTACTTCATTCGAGATGAGTTTCTGAGGATCAGCACTGCCAGTGGAGAT

321 T R A K Y F I R D E F L R I S T A S G D

1021 GGGCGTCACTACTGCTACCCTCATTTCACCTGCGCTGTGGACACTGAGAACATCCGCCGT

341 G R H Y C Y P H F T C A V D T E N I R R

1081 GTGTTCAACGACTGCCGTGACATCATTCAGCGCATGCACCTTCGTCAGTACGAGCTGCTC

361 V F N D C R D I I Q R M H L R Q Y E L L

ClaI

ATCGAT

### SEQ ID NO.: 193

### Human GS2□

XhoI

1
CTCGAGATGGGCTGCCTCGGGAACAGTAAGACCGAGGACCAGCGCAACGAGGAGAAGGCGCAGCGT

1 M G C L G N S K T E D Q R N E E K A Q R

61 GAGGCCAACAAAAAGATCGAGAAGCAGCTGCAGAAGGACAAGCAGGTCTACCGGGCCACG

21 E A N K K I E K Q L Q K D K Q V Y R A T

121 CACCGCCTGCTGCTGCTGGGTGCTGGAGAATCTGGTAAAAGCACCATTGTGAAGCAGATG

41 H R L L L L G A G E S G K S T I V K Q M

181 AGGATCCTGCATGTTAATGGGTTTAATGGAGAGGGCGGCGAAGAGGACCCGCAGGCTGCA

61 R I L H V N G F N G E G G E E D P Q A A

241 AGGAGCAACAGCGATGGTGAGAAGGCAACCAAAGTGCAGGACATCAAAAACAACCTGAAA

81 R S N S D G E K A T K V Q D I K N N L K

301 GAGGCGATTGAAACCATTGTGGCCGCCATGAGCAACCTGGTGCCCCCCGTGGAGCTGGCC

101 E A I E T I V A A M S N L V P P V E L A

361 AACCCCGAGAACCAGTTCAGAGTGGACTACATCCTGAGTGTGATGAACGTGCCTGACTTT

121 N P E N Q F R V D Y I L S V M N V P D F

421 GACTTCCCTCCCGAATTCTATGAGCATGCCAAGGCTCTGTGGGAGGATGAAGGAGTGCGT

141 D F P P E F Y E H A K A L W E D E G V R

481 GCCTGCTACGAACGCTCCAACGAGTACCAGCTGATTGACTGTGCCCAGTACTTCCTGGAC

161 A C Y E R S N E Y Q L I D C A Q Y F L D

541 AAGATCGACGTGATCAAGCAGGCTGACTATGTGCCGAGCGATCAGGACCTGCTTCGCTGC

181 K I D V I K Q A D Y V P S D Q D L L R C

601 CGTGTCCTGACTTCTGGAATCTTTGAGACCAAGTTCCAGGTGGACAAAGTCAACTTCCAC

201 R V L T S G I F E T K F Q V D K V N F H

661 ATGTTTGACGTGGGTGGCCAGCGCGATGAACGCCGCAAGTGGATCCAGTGCTTCAACGAT

221 M F D V G G Q R D E R R K W I Q C F N D

721 GTGACTGCCATCATCTTCGTGGTGGCCAGCAGCAGCTACAACATGGTCATCCGGGAGGAC

241 V T A I I F V V A S S S Y N M V I R E D

781 AACCAGACCAACCGCCTGCAGGAGGCTCTGAACCTCTTCAAGAGCATCTGGAACAACAGA

261 N Q T N R L Q E A L N L F K S I W N N R

841 TGGCTGCGCACCATCTCTGTGATCCTGTTCCTCAACAAGCAAGATCTGCTCGCTGAGAAA

281 W L R T I S V I L F L N K Q D L L A E K

901 GTCCTTGCTGGGAAATCGAAGATTGAGGACTACTTTCCAGAATTTGCTCGCTACACTACT

301 V L A G K S K I E D Y F P E F A R Y T T

961 CCTGAGGATGCTACTCCCGAGCCCGGAGAGGACCCACGCGTGACCCGGGCCAAGTACTTC

321 P E D A T P E P G E D P R V T R A K Y F

1021 ATTCGAGATGAGTTTCTGAGGATCAGCACTGCCAGTGGAGATGGGCGTCACTACTGCTAC

341 I R D E F L R I S T A S G D G R H Y C Y

1081 CCTCATTTCACCTGCGCTGTGGACACTGAGAACATCCGCCGTGTGTTCAACGACTGCCGT

361 P H F T C A V D T E N I R R V F N D C R

ClaI

1141 GACATCATTCAGCGCATGCACCTTCGTCAGTACGAGCTGCTCATCGAT

381 D I I Q R M H L R Q Y E L L

### SEQ ID NO.: 194

### Human G□q

XhoI

1
CTCGAGATGACTCTGGAGTCCATCATGGCGTGCTGCCTGAGCGAGGAGGCCAAGGAAGCCCGGCGG

1 

61 ATCAACGACGAGATCGAGCGGCAGCTCCGCAGGGACAAGCGGGACGCCCGCCGGGAGCTC

21 

121 AAGCTGCTGCTGCTCGGGACAGGAGAGAGTGGCAAGAGTACGTTTATCAAGCAGATGAGA

41 

181 ATCATCCATGGGTCAGGATACTCTGATGAAGATAAAAGGGGCTTCACCAAGCTGGTGTAT

61 

241 CAGAACATCTTCACGGCCATGCAGGCCATGATCAGAGCCATGGACACACTCAAGATCCCA

81 

301 TACAAGTATGAGCACAATAAGGCTCATGCACAATTAGTTCGAGAAGTTGATGTGGAGAAG

101 

361 GTGTCTGCTTTTGAGAATCCATATGTAGATGCAATAAAGAGTTTATGGAATGATCCTGGA

121 

421 ATCCAGGAATGCTATGATAGACGACGAGAATATCAATTATCTGACTCTACCAAATACTAT

141 

481 CTTAATGACTTGGACCGCGTAGCTGACCCTGCCTACCTGCCTACGCAACAAGATGTGCTT

161 

541 AGAGTTCGAGTCCCCACCACAGGGATCATCGAATACCCCTTTGACTTACAAAGTGTCATT

181 

601 TTCAGAATGGTCGATGTAGGGGGCCAAAGGTCAGAGAGAAGAAAATGGATACACTGCTTT

201 

661 GAAAATGTCACCTCTATCATGTTTCTAGTAGCGCTTAGTGAATATGATCAAGTTCTCGTG

221 

721 GAGTCAGACAATGAGAACCGAATGGAGGAAAGCAAGGCTCTCTTTAGAACAATTATCACA

241 

781 TACCCCTGGTTCCAGAACTCCTCGGTTATTCTGTTCTTAAACAAGAAAGATCTTCTAGAG

261 

841 GAGAAAATCATGTATTCCCATCTAGTCGACTACTTCCCAGAATATGATGGACCCCAGAGA

281 

901 GATGCCCAGGCAGCCCGAGAATTCATTCTGAAGATGTTCGTGGACCTGAACCCAGACAGT

301 

961 GACAAAATTATCTACTCCCACTTCACGTGCGCCACAGACACCGAGAATATCCGCTTTGTC

321 

ClaI

1021
TTTGCTGCCGTCAAGGACACCATCCTCCAGTTGAACCTGAAGGAGTACAATCTGGTCATCGAT

341 

### SEQ ID NO.: 195

### Human Gi□

XhoI

1
CTCGAGATGGGCTGCACCGTGAGCGCCGAGGACAAGGCGGCGGCCGAGCGCTCTAAGATGATCGAC

1 M G C T V S A E D K A A A E R S K M I D

61 AAGAACCTGCGGGAGGACGGAGAGAAGGCGGCGCGGGAGGTGAAGTTGCTGCTGTTGGGT

21 K N L R E D G E K A A R E V K L L L L G

121 GCTGGGGAGTCAGGGAAGAGCACCATCGTCAAGCAGATGAAGATCATCCACGAGGATGGC

41 A G E S G K S T I V K Q M K I I H E D G

181 TACTCCGAGGAGGAATGCCGGCAGTACCGGGCGGTTGTCTACAGCAACACCATCCAGTCC

61 Y S E E E C R Q Y R A V V Y S N T I Q S

241 ATCATGGCCATTGTCAAAGCCATGGGAAACCTGCAGATCGACTTTGCCGACCCCTCCAGA

81 I M A I V K A M G N L Q I D F A D P S R

301 GCGGACGACGCCAGGCAGCTATTTGCACTGTCCTGCACCGCCGAGGAGCAAGGCGTGCTC

101 A D D A R Q L F A L S C T A E E Q G V L

361 CCTGATGACCTGTCCGGCGTCATCCGGAGGCTCTGGGCTGACCATGGTGTGCAGGCCTGC

121 P D D L S G V I R R L W A D H G V Q A C

421 TTTGGCCGCTCAAGGGAATACCAGCTCAACGACTCAGCTGCCTACTACCTGAACGACCTG

141 F G R S R E Y Q L N D S A A Y Y L N D L

481 GAGCGTATTGCACAGAGTGACTACATCCCCACACAGCAAGATGTGCTACGGACCCGCGTA

161 E R I A Q S D Y I P T Q Q D V L R T R V

541 AAGACCACGGGGATCGTGGAGACACACTTCACCTTCAAGGACCTACACTTCAAGATGTTT

181 K T T G I V E T H F T F K D L H F K M F

601 GATGTGGGTGGTCAGCGGTCTGAGCGGAAGAAGTGGATCCACTGCTTTGAGGGCGTCACA

201 D V G G Q R S E R K K W I H C F E G V T

661 GCCATCATCTTCTGCGTAGCCTTGAGCGCCTATGACTTGGTGCTAGCTGAGGACGAGGAG

221 A I I F C V A L S A Y D L V L A E D E E

721 ATGAACCGCATGCATGAGAGCATGAAGCTATTCGATAGCATCTGCAACAACAAGTGGTTC

241 M N R M H E S M K L F D S I C N N K W F

781 ACAGACACGTCCATCATCCTCTTCCTCAACAAGAAGGACCTGTTTGAGGAGAAGATCACA

261 T D T S I I L F L N K K D L F E E K I T

841 CACAGTCCCCTGACCATCTGCTTCCCTGAGTACACAGGGGCCAACAAATATGATGAGGCA

281 H S P L T I C F P E Y T G A N K Y D E A

901 GCCAGCTACATCCAGAGTAAGTTTGAGGACCTGAATAAGCGCAAAGACACCAAGGAGATC

301 A S Y I Q S K F E D L N K R K D T K E I

961 TACACGCACTTCACGTGCGCCACCGACACCAAGAACGTGCAGTTCGTGTTTGACGCCGTC

321 Y T H F T C A T D T K N V Q F V F D A V

ClaI

1021 ACCGATGTCATCATCAAGAACAACCTGAAGGACTGCGGCCTCTTCATGCAT

341 T D V I I K N N L K D C G L F

### SEQ ID NO.: 196

### Human G□12/13

XhoI

1
CTCGAGATGTCCGGGGTGGTGCGGACCCTCAGCCGCTGCCTGCTGCCGGCCGAGGCCGGCGGGGCC

1 M S G V V R T L S R C L L P A E A G G A

61
CGCGAGCGCAGGGCGGGCAGCGGCGCGCGCGACGCGGAGCGCGAGGCCCGGAGGCGTAGC

21 R E R R A G S G A R D A E R E A R R R S

121 CGCGACATCGACGCGCTGCTGGCCCGCGAGCGGCGCGCGGTCCGGCGCCTGGTGAAGATC

41 R D I D A L L A R E R R A V R R L V K I

181 CTGCTGCTGGGCGCGGGCGAGAGCGGCAAGTCCACGTTCCTCAAGCAGATGCGCATCATC

61 L L L G A G E S G K S T F L K Q M R I I

241 CACGGCCGCGAGTTCGACCAGAAGGCGCTGCTGGAGTTCCGCGACACCATCTTCGACAAC

81 H G R E F D Q K A L L E F R D T I F D N

301 ATCCTCAAGGGCTCAAGGGTTCTTGTTGATGCACGAGATAAGCTTGGCATTCCTTGGCAG

101 I L K G S R V L V D A R D K L G I P W Q

361 TATTCTGAAAATGAGAAGCATGGGATGTTCCTGATGGCCTTCGAGAACAAGGCGGGGCTG

121 Y S E N E K H G M F L M A F E N K A G L

421 CCTGTGGAGCCGGCCACCTTCCAGCTGTACGTCCCGGCCCTGAGCGCACTCTGGAGGGAT

141 P V E P A T F Q L Y V P A L S A L W R D

481 TCTGGCATCAGGGAGGCTTTCAGCCGGAGAAGCGAGTTTCAGCTGGGGGAGTCGGTGAAG

161 S G I R E A F S R R S E F Q L G E S V K

541 TACTTCCTGGACAACTTGGACCGGATCGGCCAGCTGAATTACTTTCCTAGTAAGCAAGAT

181 Y F L D N L D R I G Q L N Y F P S K Q D

601 ATCCTGCTGGCTAGGAAAGCCACCAAGGGAATTGTGGAGCATGACTTCGTTATTAAGAAG

201 I L L A R K A T K G I V E H D F V I K K

661 ATCCCCTTTAAGATGGTGGATGTGGGCGGCCAGCGGTCCCAGCGCCAGAAGTGGTTCCAG

221 I P F K M V D V G G Q R S Q R Q K W F Q

721 TGCTTCGACGGGATCACGTCCATCCTGTTCATGGTCTCCTCCAGCGAGTACGACCAGGTC

241 C F D G I T S I L F M V S S S E Y D Q V

781 CTCATGGAGGACAGGCGCACCAACCGGCTGGTGGAGTCCATGAACATCTTCGAGACCATC

261 L M E D R R T N R L V E S M N I F E T I

841 GTCAACAACAAGCTCTTCTTCAACGTCTCCATCATTCTCTTCCTCAACAAGATGGACCTC

281 V N N K L F F N V S I I L F L N K M D L

901 CTGGTGGAGAAGGTGAAGACCGTGAGCATCAAGAAGCACTTCCCGGACTTCAGGGGCGAC

301 L V E K V K T V S I K K H F P D F R G D

961 CCGCACCAGCTGGAGGACGTCCAGCGCTACCTGGTCCAGTGCTTCGACAGGAAGAGACGG

321 P H Q L E D V Q R Y L V Q C F D R K R R

1021 AACCGCAGCAAGCCACTCTTCCACCACTTCACCACCGCCATCGACACCGAGAACGTCCGC

341 N R S K P L F H H F T T A I D T E N V R

1081 TTCGTGTTCCATGCTGTGAAAGACACCATCCTGCAGGAGAACCTGAAGGACATCATGCTG

361 F V F H A V K D T I L Q E N L K D I M L

ClaI

1141 CAGATCGAT

381 Q

### SEQ ID NO.: 205

### Human □2AR-ToxR (5-141) chimera stop GS1□-ToxR (5-141) chimera transcriptional fusion

SalI +1 B2AR

1 GTCGACATGG GGCAACCCGG GAACGGCAGC GCCTTCTTGC TGGCACCCAA TGGAAGCCAT

61 GCGCCGGACC ACGACGTCAC GCAGCAAAGG GACGAGGTGT GGGTGGTGGG
CATGGGCATC

121 GTCATGTCTC TCATCGTCCT GGCCATCGTG TTTGGCAATG TGCTGGTCAT CACAGCCATT

181 GCCAAGTTCG AGCGTCTGCA GACGGTCACC AACTACTTCA TCACTTCACT GGCCTGTGCT

241 GATCTGGTCA TGGGCCTAGC AGTGGTGCCC TTTGGGGCCG CCCATATTCT TATGAAAATG

301 TGGACTTTTG GCAACTTCTG GTGCGAGTTT TGGACTTCCA TTGATGTGCT GTGCGTCACG

361 GCCAGCATTG AGACCCTGTG CGTGATCGCA GTGGATCGCT ACTTTGCCAT TACTTCACCT

421 TTCAAGTACC AGAGCCTGCT GACCAAGAAT AAGGCCCGGG TGATCATTCT GATGGTGTGG

481 ATTGTGTCAG GCCTTAYCTC CTTCTTGCCC ATTCAGATGC ACTGGTACAG GGCCACCCAC

541 CAGGAAGCCA TCAACTGCTA TGCCAATGAG ACCTGCTGTG ACTTCTTCAC GAACCAAGCC

601 TATGCCATTG CCTCTTCCAT CGTGTCCTTC TACGTTCCCC TGGTGATCAT GGTCTTCGTC

661 TACTCCAGGG TCTTTCAGGA GGCCAAAAGG CAGCTCCAGA AGATTGACAA ATCTGAGGGC

721 CGCTTCCATG TCCAGAACCT TAGCCAGGTG GAGCAGGATG GGCGGACGGG GCATGGACTC

781 CGCAGATCTT CCAAGTTCTG CTTGAAGGAG CACAAAGCCC TCAAGACGTT AGGCATCATC

841 ATGGGCACTT TCACCCTCTG CTGGCTGCCC TTCTTCATCG TTAACATTGT GCATGTGATC

901 CAGGATAACC TCATCCGTAA GGAAGTTTAC ATCCTCCTAA ATTGGATAGG CTATGTCAAT

961 TCTGGTTTCA ATCCCCTTAT CTACTGCCGG AGCCCAGATT TCAGGATTGC CTTCCAGGAG

1021 CTTCTGTGCC TGCGCAGGTC TTCTTTGAAG GCCTATGGCA ATGGCTACTC CAGCAACGGC

1081 AACACAGGGG AGCAGAGTGG ATATCACGTG GAACAGGAGA AAGAAAATAA ACTGCTGTGT

1141 GAAGACCTCC CAGGCACGGA AGACTTTGTG GGCCATCAAG GTACTGTGCC TAGCGATAAC

last B2AR linker sequence

1201 ATTGATTCAC AAGGGAGGAA TTGTAGTACA AATGACTCAC TGCTAGAGCG TGGCCAGACG

PstI +5 toxR (5-141)

1261 GTCACCAACC TGCAGGGACA CAACTCAAAA GAGATATCGA TGAGTCATAT TGGTACTAAA

1321 TTCATTCTTG CTGAAAAATT TACCTTCGAT CCCCTAAGCA ATACTCTGAT TGACAAAGAA

1381 GATAGTGAAG AGATCATTCG ATTAGGCAGC AACGAAAGCC GAATTCTTTG GCTGCTGGCC

1441 CAACGTCCAA ACGAGGTAAT TTCTCGCAAT GATTTGCATG ACTTTGTTTG GCGAGAGCAA

1501 GGTTTTGAAG TCGATGATTC CAGCTTAACC CAAGCCATTT CGACTCTGCG CAAAATGCTC

1561 AAAGATTCGA CAAAGTCCCC ACAATACGTC AAAACGGTTC CGAAGCGCGG TTACCAATTG

1621 ATCGCCCGAG TGGAAACGGT TGAAGAAGAG ATGGCTCGCG AAAACGAAGC TGCTCATGAC

stop SD XhoI +1 GS1 alpha

1681 ATCTCTTAAT AATCAAGGAG GCCCTCGAGA TGGGCTGCCT CGGGAACAGT AAGACCGAGG

1741 ACCAGCGCAA CGAGGAGAAG GCGCAGCGTG AGGCCAACAA AAAGATCGAG
AAGCAGCTGC

1801 AGAAGGACAA GCAGGTCTAC CGGGCCACGC ACCGCCTGCT GCTGCTGGGT
GCTGGAGAAT

1861 CTGGTAAAAG CACCATTGTG AAGCAGATGA GGATCCTGCA TGTTAATGGG TTTAATGGAG

1921 ACAGTGAGAA GGCAACCAAA GTGCAGGACA TCAAAAACAA CCTGAAAGAG GCGATTGAAA

1981 CCATTGTGGC CGCCATGAGC AACCTGGTGC CCCCCGTGGA GCTGGCCAAC
CCCGAGAACC

2041 AGTTCAGAGT GGACTACATC CTGAGTGTGA TGAACGTGCC TGACTTTGAC TTCCCTCCCG

2101 AATTCTATGA GCATGCCAAG GCTCTGTGGG AGGATGAAGG AGTGCGTGCC TGCTACGAAC

2161 GCTCCAACGA GTACCAGCTG ATTGACTGTG CCCAGTACTT CCTGGACAAG ATCGACGTGA

2221 TCAAGCAGGC TGACTATGTG CCGAGCGATC AGGACCTGCT TCGCTGCCGT GTCCTGACTT

2281 CTGGAATCTT TGAGACCAAG TTCCAGGTGG ACAAAGTCAA CTTCCACATG TTTGACGTGG

2341 GTGGCCAGCG CGATGAACGC CGCAAGTGGA TCCAGTGCTT CAACGATGTG ACTGCCATCA

2401 TCTTCGTGGT GGCCAGCAGC AGCTACAACA TGGTCATCCG GGAGGACAAC CAGACCAACC

2461 GCCTGCAGGA GGCTCTGAAC CTCTTCAAGA GCATCTGGAA CAACAGATGG CTGCGCACCA

2521 TCTCTGTGAT CCTGTTCCTC AACAAGCAAG ATCTGCTCGC TGAGAAAGTC CTTGCTGGGA

2581 AATCGAAGAT TGAGGACTAC TTTCCAGAAT TTGCTCGCTA CACTACTCCT GAGGATGCTA

2641 CTCCCGAGCC CGGAGAGGAC CCACGCGTGA CCCGGGCCAA GTACTTCATT CGAGATGAGT

2701 TTCTGAGGAT CAGCACTGCC AGTGGAGATG GGCGTCACTA CTGCTACCCT CATTTCACCT

2761 GCGCTGTGGA CACTGAGAAC ATCCGCCGTG TGTTCAACGA CTGCCGTGAC ATCATTCAGC

ClaI +5 toxR (5-141)

2821 GCATGCACCT TCGTCAGTAC GAGCTGCTCA TCGATGGACA CAACTCAAAA GAGATATCGA

2881 TGAGTCATAT TGGTACTAAA TTCATTCTTG CTGAAAAATT TACCTTCGAT CCCCTAAGCA

2941 ATACTCTGAT TGACAAAGAA GATAGTGAAG AGATCATTCG ATTAGGCAGC AACGAAAGCC

3001 GAATTCTTTG GCTGCTGGCC CAACGTCCAA ACGAGGTAAT TTCTCGCAAT GATTTGCATG

3061 ACTTTGTTTG GCGAGAGCAA GGTTTTGAAG TCGATGATTC CAGCTTAACC CAAGCCATTT

3121 CGACTCTGCG CAAAATGCTC AAAGATTCGA CAAAGTCCCC ACAATACGTC AAAACGGTTC

3181 CGAAGCGCGG TTACCAATTG ATCGCCCGAG TGGAAACGGT TGAAGAAGAG ATGGCTCGCG

Stop XbaI

Stem-loop

3241 AAAACGAAGC TGCTCATGAC ATCTCTTAAT AATCTAGAGG ATCCCCGCGC CCTCATCCGA

3301 AAGGGCG

SEQ ID NO.: 208

Vibrio cholerae Pctx::lacZ reporter fusion constuct

XbaI

1 TCTAGAGGCT GTGGGTAGAA GTGAAACGGG GTTTACCGAT AAAAACAGAA AATGATAAAA

3 ToxR binding repeats

61 AAGGACTAAA TAGTATATTT TGATTTTTGA TTTTTGATTT CAAATAATAC AAATTTATTT

+1 lacZ

121 ACTTATTTAA TTGTTTTGAT CAATTATTTT TCTGTTAAAC AAAGGGAGCA TTATATGGTA

181 AAGACCATGA TTACGGATTC ACTGGCCGTC GTTTTACAAC GTCGTGACTG GGAAAACCCT

241 GGCGTTACCC AACTTAATCG CCTTGCAGCA CATCCCCCTT TCGCCAGCTG GCGTAATAGC

301 GAAGAGGCCC GCACCGATCG CCCTTCCCAA CAGTTGCGCA GCCTGAATGG CGAATGGCGC

361 TTTGCCTGGT TTCCGGCACC AGAAGCGGTG CCGGAAAGCT GGCTGGAGTG CGATCTTCCT

421 GAGGCCGATA CTGTCGTCGT CCCCTCAAAC TGGCAGATGC ACGGTTACGA TGCGCCCATC

481 TACACCAACG TGACCTATCC CATTACGGTC AATCCGCCGT TTGTTCCCAC GGAGAATCCG

541 ACGGGTTGTT ACTCGCTCAC ATTTAATGTT GATGAAAGCT GGCTACAGGA AGGCCAGACG

601 CGAATTATTT TTGATGGCGT TAACTCGGCG TTTCATCTGT GGTGCAACGG GCGCTGGGTC

661 GGTTACGGCC AGGACAGTCG TTTGCCGTCT GAATTTGACC TGAGCGCATT TTTACGCGCC

721 GGAGAAAACC GCCTCGCGGT GATGGTGCTG CGCTGGAGTG ACGGCAGTTA TCTGGAAGAT

781 CAGGATATGT GGCGGATGAG CGGCATTTTC CGTGACGTCT CGTTGCTGCA TAAACCGACT

841 ACACAAATCA GCGATTTCCA TGTTGCCACT CGCTTTAATG ATGATTTCAG CCGCGCTGTA

901 CTGGAGGCTG AAGTTCAGAT GTGCGGCGAG TTGCGTGACT ACCTACGGGT AACAGTTTCT

961 TTATGGCAGG GTGAAACGCA GGTCGCCAGC GGCACCGCGC CTTTCGGCGG TGAAATTATC

1021 GATGAGCGTG GTGGTTATGC CGATCGCGTC ACACTACGTC TGAACGTCGA AAACCCGAAA

1081 CTGTGGAGCG CCGAAATCCC GAATCTCTAT CGTGCGGTGG TTGAACTGCA CACCGCCGAC

1141 GGCACGCTGA TTGAAGCAGA AGCCTGCGAT GTCGGTTTCC GCGAGGTGCG GATTGAAAAT

1201 GGTCTGCTGC TGCTGAACGG CAAGCCGTTG CTGATTCGAG GCGTTAACCG TCACGAGCAT

1261 CATCCTCTGC ATGGTCAGGT CATGGATGAG CAGACGATGG TGCAGGATAT CCTGCTGATG

1321 AAGCAGAACA ACTTTAACGC CGTGCGCTGT TCGCATTATC CGAACCATCC GCTGTGGTAC

1381 ACGCTGTGCG ACCGCTACGG CCTGTATGTG GTGGATGAAG CCAATATTGA AACCCACGGC

1441 ATGGTGCCAA TGAATCGTCT GACCGATGAT CCGCGCTGGC TACCGGCGAT GAGCGAACGC

1501 GTAACGCGAA TGGTGCAGCG CGATCGTAAT CACCCGAGTG TGATCATCTG GTCGCTGGGG

1561 AATGAATCAG GCCACGGCGC TAATCACGAC GCGCTGTATC GCTGGATCAA ATCTGTCGAT

1621 CCTTCCCGCC CGGTGCAGTA TGAAGGCGGC GGAGCCGACA CCACGGCCAC CGATATTATT

1681 TGCCCGATGT ACGCGCGCGT GGATGAAGAC CAGCCCTTCC CGGCTGTGCC GAAATGGTCC

1741 ATCAAAAAAT GGCTTTCGCT ACCTGGAGAG ACGCGCCCGC TGATCCTTTG CGAATACGCC

1801 CACGCGATGG GTAACAGTCT TGGCGGTTTC GCTAAATACT GGCAGGCGTT TCGTCAGTAT

1861 CCCCGTTTAC AGGGCGGCTT CGTCTGGGAC TGGGTGGATC AGTCGCTGAT TAAATATGAT

1921 GAAAACGGCA ACCCGTGGTC GGCTTACGGC GGTGATTTTG GCGATACGCC GAACGATCGC

1981 CAGTTCTGTA TGAACGGTCT GGTCTTTGCC GACCGCACGC CGCATCCAGC GCTGACGGAA

2041 GCAAAACACC AGCAGCAGTT TTTCCAGTTC CGTTTATCCG GGCAAACCAT CGAAGTGACC

2101 AGCGAATACC TGTTCCGTCA TAGCGATAAC GAGCTCCTGC ACTGGATGGT GGCGCTGGAT

2161 GGTAAGCCGC TGGCAAGCGG TGAAGTGCCT CTGGATGTCG CTCCACAAGG TAAACAGTTG

2221 ATTGAACTGC CTGAACTACC GCAGCCGGAG AGCGCCGGGC AACTCTGGCT CACAGTACGC

2281 GTAGTGCAAC CGAACGCGAC CGCATGGTCA GAAGCCGGGC ACATCAGCGC
CTGGCAGCAG

2341 TGGCGTCTGG CGGAAAACCT CAGTGTGACG CTCCCCGCCG CGTCCCACGC CATCCCGCAT

2401 CTGACCACCA GCGAAATGGA TTTTTGCATC GAGCTGGGTA ATAAGCGTTG GCAATTTAAC

2461 CGCCAGTCAG GCTTTCTTTC ACAGATGTGG ATTGGCGATA AAAAACAACT GCTGACGCCG

2521 CTGCGCGATC AGTTCACCCG TGCACCGCTG GATAACGACA TTGGCGTAAG TGAAGCGACC

2581 CGCATTGACC CTAACGCCTG GGTCGAACGC TGGAAGGCGG CGGGCCATTA
CCAGGCCGAA

2641 GCAGCGTTGT TGCAGTGCAC GGCAGATACA CTTGCTGATG CGGTGCTGAT TACGACCGCT

2701 CACGCGTGGC AGCATCAGGG GAAAACCTTA TTTATCAGCC GGAAAACCTA CCGGATTGAT

2761 GGTAGTGGTC AAATGGCGAT TACCGTTGAT GTTGAAGTGG CGAGCGATAC ACCGCATCCG

2821 GCGCGGATTG GCCTGAACTG CCAGCTGGCG CAGGTAGCAG AGCGGGTAAA
CTGGCTCGGA

2881 TTAGGGCCGC AAGAAAACTA TCCCGACCGC CTTACTGCCG CCTGTTTTGA CCGCTGGGAT

2941 CTGCCATTGT CAGACATGTA TACCCCGTAC GTCTTCCCGA GCGAAAACGG TCTGCGCTGC

3001 GGGACGCGCG AATTGAATTA TGGCCCACAC CAGTGGCGCG GCGACTTCCA GTTCAACATC

3061 AGCCGCTACA GTCAACAGCA ACTGATGGAA ACCAGCCATC GCCATCTGCT GCACGCGGAA

3121 GAAGGCACAT GGCTGAATAT CGACGGTTTC CATATGGGGA TTGGTGGCGA CGACTCCTGG

3181 AGCCCGTCAG TATCGGCGGA ATTCCAGCTG AGCGCCGGTC GCTACCATTA CCAGTTGGTC

Stop Stem-loop XbaI

3241 TGGTGTCAAA AATAATAACGCCCTCAT CCGAAAGGGC GTCTAGA

### SEQ ID NO.: 266

### pMPX-74 MalE (1-28) fusion vector

SD old PstI +1
2401 GAATTCAGGCGCTTTTTAGACTGGTCGTAATGAAATTCAGGAGGTTCTGCATATGAAAAT
1 M K I
2461 AAAAACAGGTGCACGCATCCTCGCATTATCCGCATTAACGACGATGATGTTTTCCGCCTC
4 K T G A R I L A L S A L T T M M F S A S
Factor Xa PstI SalI XbaI

2521 GGCTCTCGCCAAAATCATCGAAGCCCGCCTGCAGGCCTCGGTCGACGCCGAATCTAGAGA

24 A L A K I I E A R L Q A S V D A E S R D

FLAG lost XbaI

2581 TTATAAAGATGACGATGACAAATAATAAGCTAGAGG (transcriptional stop)

44 Y K D D D D K

### pMPX-72::malE(1-28)::FXa::Pstl, Sall, Xbal::FLAG

Rhamnose inducible, clone into Pstl, Sall, Xbal Made by cutting TOPO Nsil-malE (1-28)::FXa::Pstl, Sall, Xbal::FLAG-Nhel insertion with Nsil & Nhel and cloning into pMPX-72 cut with Pstl & Xbal.

### SEQ ID NO.: 267

### pMPX-75 MalE (1-28) fusion vector

SD old PstI +1

1621 CCATACCCGTTTTTTTGGGCTAGCAGGAGGCCCTGCATATGAAAATAAAAACAGGTGCAC

1 M K I K T G A

1681 GCATCCTCGCATTATCCGCATTAACGACGATGATGTTTTCCGCCTCGGCTCTCGCCAAAA

8 R I L A L S A L T T M M F S A S A L A K

Factor Xa PstI SalI XbaI FLAG

1741 TCATCGAAGCCCGCCTGCAGGCCTCGGTCGACGCCGAATCTAGAGATTATAAAGATGACG

Lost XbaI

1801 ATGACAAATAATAAGCTAGAGG (Transcriptional stop)

### pMPX-71::malE(1-28)::FXa::Pstl, Sall, Xbal::FLAG

### Arabinose inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-71 cut with Pstl & Xbal.

### SEQ ID NO.: 268

### pMPX-88 MalE (1-28) fusion vector

SD old PstI +1
AGGAGGTTCTGCATATGAAAAT
1 M K I
AAAAACAGGTGCACGCATCCTCGCATTATCCGCATTAACGACGATGATGTTTTCCGCCTC
4 K T G A R I L A L S A L T T M M F S A S
Factor Xa PstI SalI XbaI
GGCTCTCGCCAAAATCATCGAAGCCCGCCTGCAGGCCTCGGTCGACGCCGAATCTAGAGA
24 A L A K I I E A R L Q A S V D A E S R D
FLAG lost XbaI
TTATAAAGATGACGATGACAAATAATAAGCTAGAGGTACC (transcriptional stop)
44 Y K D D D D K

### pMPX-84::malE(1-28)::FXa::Pstl, Sall, XbaI::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-84 cut with Pstl & Xbal.

### SEQ ID NO.: 269

### pMPX-93 MalE (1-28) fusion vector

SD old PstI +1
AGGAGGTTCTGCATATGAAAAT
1 M K I
AAAAACAGGTGCACGCATCCTCGCATTATCCGCATTAACGACGATGATGTTTTCCGCCTC
4 K T G A R I L A L S A L T T M M F S A S
Factor Xa PstI SalI XbaI
GGCTCTCGCCAAAATCATCGAAGCCCGCCTGCAGGCCTCGGTCGACGCCGAATCTAGAGA
24 A L A K I I E A R L Q A S V D A E S R D
FLAG lost XbaI
TTATAAAGATGACGATGACAAATAATAAGCTAGAGGTACC (transcriptional stop)
44 Y K D D D D K

### pMPX-86::malE(1-28)::FXa::Pstl, Sall, XbaI::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-86 cut with Pstl & Xbal.

### SEQ ID NO.: 270

### pMPX-77 MalE (1-370 del 354-364) fusion vector

SD old PstI +1
2401 GAATTCAGGCGCTTTTTAGACTGGTCGTAATGAAATTCAGGAGGTTCTGCATATGAAAAT
1 M K I
2461 AAAAACAGGTGCACGCATCCTCGCATTATCCGCATTAACGACGATGATGTTTTCCGCCTC

4 K T G A R I L A L S A L T T M M F S A S

2521 GGCTCTCGCCAAAATCGAAGAAGGTAAACTGGTAATCTGGATTAACGGCGATAAAGGCTA

24 A L A K I E E G K L V I W I N G D K G Y

2581 TAACGGTCTCGCTGAAGTCGGTAAGAAATTCGAGAAAGATACCGGAATTAAAGTCACCGT

44 N G L A E V G K K F E K D T G I K V T V

2641 TGAGCATCCGGATAAACTGGAAGAGAAATTCCCACAGGTTGCGGCAACTGGCGATGGCCC

64 E H P D K L E E K F P Q V A A T G D G P

2701 TGACATTATCTTCTGGGCACACGACCGCTTTGGTGGCTACGCTCAATCTGGCCTGTTGGC

84 D I I F W A H D R F G G Y A Q S G L L A

2761 TGAAATCACCCCGGACAAAGCGTTCCAGGACAAGCTGTATCCGTTTACCTGGGATGCCGT

104 E I T P D K A F Q D K L Y P F T W D A V

2821 ACGTTACAACGGCAAGCTGATTGCTTACCCGATCGCTGTTGAAGCGTTATCGCTGATTTA

124 R Y N G K L I A Y P I A V E A L S L I Y

2881 TAACAAAGATCTGCTGCCGAACCCGCCAAAAACCTGGGAAGAGATCCCGGCGCTGGATAA

144 N K D L L P N P P K T W E E I P A L D K

2941 AGAACTGAAAGCGAAAGGTAAGAGCGCGCTGATGTTCAACCTGCAAGAACCGTACTTCAC

164 E L K A K G K S A L M F N L Q E P Y F T

3001 CTGGCCGCTGATTGCTGCTGACGGGGGTTATGCGTTCAAGTATGAAAACGGCAAGTACGA

184 W P L I A A D G G Y A F K Y E N G K Y D

3061 CATTAAAGACGTGGGCGTGGATAACGCTGGCGCGAAAGCGGGTCTGACCTTCCTGGTTGA

204 I K D V G V D N A G A K A G L T F L V D

3121 CCTGATTAAAAACAAACACATGAATGCAGACACCGATTACTCCATCGCAGAAGCTGCCTT

224 L I K N K H M N A D T D Y S I A E A A F

3181 TAATAAAGGCGAAACAGCGATGACCATCAACGGCCCGTGGGCATGGTCCAACATCGACAC

244 N K G E T A M T I N G P W A W S N I D T

3241 CAGCAAAGTGAATTATGGTGTAACGGTACTGCCGACCTTCAAGGGTCAACCATCCAAACC

264 S K V N Y G V T V L P T F K G Q P S K P

3301 GTTCGTTGGCGTGCTGAGCGCAGGTATTAACGCCGCCAGTCCGAACAAAGAGCTGGCGAA

284 F V G V L S A G I N A A S P N K E L A K

3361 AGAGTTCCTCGAAAACTATCTGCTGACTGATGAAGGTCTGGAAGCGGTTAATAAAGACAA

304 E F L E N Y L L T D E G L E A V N K D K

3421 ACCGCTGGGTGCCGTAGCGCTGAAGTCTTACGAGGAAGAGTTGGCGAAAGATCCACGTAT

324 P L G A V A L K S Y E E E L A K D P R I

### pMPX-72::malE(1-370 del 354-364)::FXa::Pstl, Sall, XbaI::FLAG

### Rhamnose inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-72 cut with Pstl & Xbal.

### SEQ ID NO.: 271

### pMPX-76 MalE (1-370 del 354-364) fusion vector

SD old PstI +1

1621 CCATACCCGTTTTTTTGGGCTAGCAGGAGGCCCTGCATATGAAAATAAAAACAGGTGCAC

1 M K I K T G A

1681 GCATCCTCGCATTATCCGCATTAACGACGATGATGTTTTCCGCCTCGGCTCTCGCCAAAA

8 R I L A L S A L T T M M F S A S A L A K

1741 TCGAAGAAGGTAAACTGGTAATCTGGATTAACGGCGATAAAGGCTATAACGGTCTCGCTG

28 I E E G K L V I W I N G D K G Y N G L A

1801 AAGTCGGTAAGAAATTCGAGAAAGATACCGGAATTAAAGTCACCGTTGAGCATCCGGATA

48 E V G K K F E K D T G I K V T V E H P D

1861 AACTGGAAGAGAAATTCCCACAGGTTGCGGCAACTGGCGATGGCCCTGACATTATCTTCT

68 K L E E K F P Q V A A T G D G P D I I F

1921 GGGCACACGACCGCTTTGGTGGCTACGCTCAATCTGGCCTGTTGGCTGAAATCACCCCGG

88 W A H D R F G G Y A Q S G L L A E I T P

1981 ACAAAGCGTTCCAGGACAAGCTGTATCCGTTTACCTGGGATGCCGTACGTTACAACGGCA

108 D K A F Q D K L Y P F T W D A V R Y N G

2041 AGCTGATTGCTTACCCGATCGCTGTTGAAGCGTTATCGCTGATTTATAACAAAGATCTGC

128 K L I A Y P I A V E A L S L I Y N K D L

2101 TGCCGAACCCGCCAAAAACCTGGGAAGAGATCCCGGCGCTGGATAAAGAACTGAAAGCGA

148 L P N P P K T W E E I P A L D K E L K A

2161 AAGGTAAGAGCGCGCTGATGTTCAACCTGCAAGAACCGTACTTCACCTGGCCGCTGATTG

168 K G K S A L M F N L Q E P Y F T W P L I

2221 CTGCTGACGGGGGTTATGCGTTCAAGTATGAAAACGGCAAGTACGACATTAAAGACGTGG

188 A A D G G Y A F K Y E N G K Y D I K D V

2281 GCGTGGATAACGCTGGCGCGAAAGCGGGTCTGACCTTCCTGGTTGACCTGATTAAAAACA

208 G V D N A G A K A G L T F L V D L I K N

2341 AACACATGAATGCAGACACCGATTACTCCATCGCAGAAGCTGCCTTTAATAAAGGCGAAA

228 K H M N A D T D Y S I A E A A F N K G E

2401 CAGCGATGACCATCAACGGCCCGTGGGCATGGTCCAACATCGACACCAGCAAAGTGAATT

248 T A M T I N G P W A W S N I D T S K V N

2461 ATGGTGTAACGGTACTGCCGACCTTCAAGGGTCAACCATCCAAACCGTTCGTTGGCGTGC

268 Y G V T V L P T F K G Q P S K P F V G V

2521 TGAGCGCAGGTATTAACGCCGCCAGTCCGAACAAAGAGCTGGCGAAAGAGTTCCTCGAAA

288 L S A G I N A A S P N K E L A K E F L E

2581 ACTATCTGCTGACTGATGAAGGTCTGGAAGCGGTTAATAAAGACAAACCGCTGGGTGCCG

308 N Y L L T D E G L E A V N K D K P L G A

2641 TAGCGCTGAAGTCTTACGAGGAAGAGTTGGCGAAAGATCCACGTATTGCCGCCACCATGG

328 V A L K S Y E E E L A K D P R I A A T M

Factor Xa PstI

2701 AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI FLAG Lost XbaI

2761 TCGACGCCGAATCTAGAGATTATAAAGATGACGATGACAAATAATAAGCTAGAGGA(trxn stop)

368 V D A E S R D Y K D D D D K

### pMPX-71::malE(1-370 del 354-364)::FXa::Pstl, Sall, Xbal::FLAG

Arabinose inducible, clone into Pstl, Sall, Xbal Made by cutting TOPO Nsil-malE (1-370 del 354-364)::FXa::Pstl, Sall, Xbal::FLAG-Nhel insertion with Nsil & Nhel and cloning into pMPX-71 cut with Pstl & Xbal.

### SEQ ID NO.: 272

### pMPX-89 MalE (1-370 del 354-364) fusion vector

SD old PstI +1

AGGAGGTTCTGCATATGAAAATAAAAACAGGTGCAC

1 M K I K T G A

GCATCCTCGCATTATCCGCATTAACGACGATGATGTTTTCCGCCTCGGCTCTCGCCAAAA
8 R I L A L S A L T T M M F S A S A L A K
TCGAAGAAGGTAAACTGGTAATCTGGATTAACGGCGATAAAGGCTATAACGGTCTCGCTG
28 I E E G K L V I W I N G D K G Y N G L A
AAGTCGGTAAGAAATTCGAGAAAGATACCGGAATTAAAGTCACCGTTGAGCATCCGGATA
48 E V G K K F E K D T G I K V T V E H P D
AACTGGAAGAGAAATTCCCACAGGTTGCGGCAACTGGCGATGGCCCTGACATTATCTTCT
68 K L E E K F P Q V A A T G D G P D I I F
GGGCACACGACCGCTTTGGTGGCTACGCTCAATCTGGCCTGTTGGCTGAAATCACCCCGG
88 W A H D R F G G Y A Q S G L L A E I T P
ACAAAGCGTTCCAGGACAAGCTGTATCCGTTTACCTGGGATGCCGTACGTTACAACGGCA
108 D K A F Q D K L Y P F T W D A V R Y N G
AGCTGATTGCTTACCCGATCGCTGTTGAAGCGTTATCGCTGATTTATAACAAAGATCTGC
128 K L I A Y P I A V E A L S L I Y N K D L
TGCCGAACCCGCCAAAAACCTGGGAAGAGATCCCGGCGCTGGATAAAGAACTGAAAGCGA
148 L P N P P K T W E E I P A L D K E L K A
AAGGTAAGAGCGCGCTGATGTTCAACCTGCAAGAACCGTACTTCACCTGGCCGCTGATTG
168 K G K S A L M F N L Q E P Y F T W P L I
CTGCTGACGGGGGTTATGCGTTCAAGTATGAAAACGGCAAGTACGACATTAAAGACGTGG
188 A A D G G Y A F K Y E N G K Y D I K D V
GCGTGGATAACGCTGGCGCGAAAGCGGGTCTGACCTTCCTGGTTGACCTGATTAAAAACA
208 G V D N A G A K A G L T F L V D L I K N
AACACATGAATGCAGACACCGATTACTCCATCGCAGAAGCTGCCTTTAATAAAGGCGAAA

228 K H M N A D T D Y S I A E A A F N K G E

CAGCGATGACCATCAACGGCCCGTGGGCATGGTCCAACATCGACACCAGCAAAGTGAATT

248 T A M T I N G P W A W S N I D T S K V N

ATGGTGTAACGGTACTGCCGACCTTCAAGGGTCAACCATCCAAACCGTTCGTTGGCGTGC

268 Y G V T V L P T F K G Q P S K P F V G V

TGAGCGCAGGTATTAACGCCGCCAGTCCGAACAAAGAGCTGGCGAAAGAGTTCCTCGAAA

288 L S A G I N A A S P N K E L A K E F L E

ACTATCTGCTGACTGATGAAGGTCTGGAAGCGGTTAATAAAGACAAACCGCTGGGTGCCG

308 N Y L L T D E G L E A V N K D K P L G A

TAGCGCTGAAGTCTTACGAGGAAGAGTTGGCGAAAGATCCACGTATTGCCGCCACCATGG

328 V A L K S Y E E E L A K D P R I A A T M

Factor Xa PstI

AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI FLAG Lost XbaI

TCGACGCCGAATCTAGAGATTATAAAGATGACGATGACAAATAATAAGCTAGAGG (trxn stop)

368 V D A E S R D Y K D D D D K

### pMPX-84::malE(1-370 del 354-364)::FXa::PstI, Sall, Xbal::FLAG

### Temperature inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-84 cut with Pstl & Xbal.

### SEQ ID NO.: 273

### pMPX-94 MalE (1-370 del 354-364) fusion vector

SD old PstI +1

AGGAGGTTCTGCATATGAAAATAAAAACAGGTGCAC

1 M K I K T G A

GCATCCTCGCATTATCCGCATTAACGACGATGATGTTTTCCGCCTCGGCTCTCGCCAAAA

8 R I L A L S A L T T M M F S A S A L A K

TCGAAGAAGGTAAACTGGTAATCTGGATTAACGGCGATAAAGGCTATAACGGTCTCGCTG

28 I E E G K L V I W I N G D K G Y N G L A

AAGTCGGTAAGAAATTCGAGAAAGATACCGGAATTAAAGTCACCGTTGAGCATCCGGATA

48 E V G K K F E K D T G I K V T V E H P D

AACTGGAAGAGAAATTCCCACAGGTTGCGGCAACTGGCGATGGCCCTGACATTATCTTCT

68 K L E E K F P Q V A A T G D G P D I I F

GGGCACACGACCGCTTTGGTGGCTACGCTCAATCTGGCCTGTTGGCTGAAATCACCCCGG

88 W A H D R F G G Y A Q S G L L A E I T P

ACAAAGCGTTCCAGGACAAGCTGTATCCGTTTACCTGGGATGCCGTACGTTACAACGGCA

108 D K A F Q D K L Y P F T W D A V R Y N G

AGCTGATTGCTTACCCGATCGCTGTTGAAGCGTTATCGCTGATTTATAACAAAGATCTGC

128 K L I A Y P I A V E A L S L I Y N K D L

TGCCGAACCCGCCAAAAACCTGGGAAGAGATCCCGGCGCTGGATAAAGAACTGAAAGCGA

148 L P N P P K T W E E I P A L D K E L K A

AAGGTAAGAGCGCGCTGATGTTCAACCTGCAAGAACCGTACTTCACCTGGCCGCTGATTG

168 K G K S A L M F N L Q E P Y F T W P L I

CTGCTGACGGGGGTTATGCGTTCAAGTATGAAAACGGCAAGTACGACATTAAAGACGTGG

188 A A D G G Y A F K Y E N G K Y D I K D V

GCGTGGATAACGCTGGCGCGAAAGCGGGTCTGACCTTCCTGGTTGACCTGATTAAAAACA

208 G V D N A G A K A G L T F L V D L I K N

AACACATGAATGCAGACACCGATTACTCCATCGCAGAAGCTGCCTTTAATAAAGGCGAAA

228 K H M N A D T D Y S I A E A A F N K G E

CAGCGATGACCATCAACGGCCCGTGGGCATGGTCCAACATCGACACCAGCAAAGTGAATT

248 T A M T I N G P W A W S N I D T S K V N

ATGGTGTAACGGTACTGCCGACCTTCAAGGGTCAACCATCCAAACCGTTCGTTGGCGTGC

268 Y G V T V L P T F K G Q P S K P F V G V

TGAGCGCAGGTATTAACGCCGCCAGTCCGAACAAAGAGCTGGCGAAAGAGTTCCTCGAAA

288 L S A G I N A A S P N K E L A K E F L E

ACTATCTGCTGACTGATGAAGGTCTGGAAGCGGTTAATAAAGACAAACCGCTGGGTGCCG

308 N Y L L T D E G L E A V N K D K P L G A

TAGCGCTGAAGTCTTACGAGGAAGAGTTGGCGAAAGATCCACGTATTGCCGCCACCATGG

328 V A L K S Y E E E L A K D P R I A A T M

Factor Xa PstI

AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI FLAG Lost XbaI

TCGACGCCGAATCTAGAGATTATAAAGATGACGATGACAAATAATAAGCTAGAGG (trxn stop)

368 V D A E S R D Y K D D D D K

### pMPX-86::malE(1-370 del 354-364)::FXa::Pstl, Sall, Xbal::FLAG

### Temperature inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-86 cut with Pstl & Xbal.

### SEQ ID NO.: 274

### pMPX-79 TrxA (2-109 del 103-107) fusion vector

SD PstI SalI XbaI +2 trxA(del 103-107)

1 TAGCAGGAGGCCCTGCAGGCCTCGGTCGACGCCGAATCTAGAAGCGATAAAATTATT

1 A S V D A E S R S D K I I

61 CACCTGACTGACGACAGTTTTGACACGGATGTACTCAAAGCGGACGGGGCGATCCTCGTC

17 H L T D D S F D T D V L K A D G A I L V

121 GATTTCTGGGCAGAGTGGTGCGGTCCGTGCAAAATGATCGCCCCGATTCTGGATGAAATC

37 D F W A E W C G P C K M I A P I L D E I

181 GCTGACGAATATCAGGGCAAACTGACCGTTGCAAAACTGAACATCGATCAAAACCCTGGC

57 A D E Y Q G K L T V A K L N I D Q N P G

241 ACTGCGCCGAAATATGGCATCCGTGGTATCCCGACTCTGCTGCTGTTCAAAAACGGTGAA

77 T A P K Y G I R G I P T L L L F K N G E

301 GTGGCGGCAACCAAAGTGGGTGCACTGTCTAAAGGTCAGTTGAAAGAGAACCTGGCGGAT

97 V A A T K V G A L S K G Q L K E N L A D

FLAG Lost XbaI

361 TATAAAGATGACGATGACAAATAATAAGCTAGAGG (transcriptional stop)

117 Y K D D D D K

### pMPX-71::Pstl, Sall, Xbal::trxA (2-109 del 103-107)::FLAG

### Arabinose inducible, clone into Pstl, Sall, Xbal

### +1 Met required for protein to be fused

Made by cutting TOPO Pstl, Sall, Xbal::trxA (2-109 del 103-107)::FLAG-Nhel insertion with Pstl & Nhel and cloning into pMPX-71 cut with Pstl & Xbal.

### SEQ ID NO.: 275

### pMPX-78 TrxA (2-109 del 103-107) fusion vector

SD PstI

1 GAATTCAGGCGCTTTTTAGACTGGTCGTAATGAAATTCAGGAGGTTCTGCAGGCCTC

1 A S

SalI XbaI +2 trxA(del 103-107)

61 GGTCGACGCCGAATCTAGAAGCGATAAAATTATTCACCTGACTGACGACAGTTTTGACAC

6 V D A E S R S D K I I H L T D D S F D T

121 GGATGTACTCAAAGCGGACGGGGCGATCCTCGTCGATTTCTGGGCAGAGTGGTGCGGTCC

26 D V L K A D G A I L V D F W A E W C G P

181 GTGCAAAATGATCGCCCCGATTCTGGATGAAATCGCTGACGAATATCAGGGCAAACTGAC

46 C K M I A P I L D E I A D E Y Q G K L T

241 CGTTGCAAAACTGAACATCGATCAAAACCCTGGCACTGCGCCGAAATATGGCATCCGTGG

66 V A K L N I D Q N P G T A P K Y G I R G

301 TATCCCGACTCTGCTGCTGTTCAAAAACGGTGAAGTGGCGGCAACCAAAGTGGGTGCACT

86 I P T L L L F K N G E V A A T K V G A L

FLAG

361 GTCTAAAGGTCAGTTGAAAGAGAACCTGGCGGATTATAAAGATGACGATGACAAATAATAA

106 S K G Q L K E N L A D Y K D D D D K

### pMPX-72::Pstl, Sall, Xbal::trxA (2-109 del 103-107)::FLAG

### Rhamnose inducible, clone into Pstl, Sall, Xbal

+1 Met required for protein to be fused

Made by cutting TOPO Pstl, Sall, Xbal::trxA (2-109 del 103-107)::FLAG-Nhel insertion with Pstl & Nhel and cloning into pMPX-72 cut with Pstl & Xbal.

### SEQ ID NO.: 276

### pMPX-90 TrxA (2-109 del 103-107) fusion vector

lost XbaI

GCTAGAGG (transcriptional stop)

SD PstI SalI XbaI +2 trxA(del 103-107)

AGGAGGTTCTGCAGGCCTCGGTCGACGCCGAATCTAGAAGCGATAAAATTATT

1 A S V D A E S R S D K I I

CACCTGACTGACGACAGTTTTGACACGGATGTACTCAAAGCGGACGGGGCGATCCTCGTC

17 H L T D D S F D T D V L K A D G A I L V

GATTTCTGGGCAGAGTGGTGCGGTCCGTGCAAAATGATCGCCCCGATTCTGGATGAAATC

37 D F W A E W C G P C K M I A P I L D E I

GCTGACGAATATCAGGGCAAACTGACCGTTGCAAAACTGAACATCGATCAAAACCCTGGC

57 A D E Y Q G K L T V A K L N I D Q N P G

ACTGCGCCGAAATATGGCATCCGTGGTATCCCGACTCTGCTGCTGTTCAAAAACGGTGAA

77 T A P K Y G I R G I P T L L L F K N G E

GTGGCGGCAACCAAAGTGGGTGCACTGTCTAAAGGTCAGTTGAAAGAGAACCTGGCGGAT

97 V A A T K V G A L S K G Q L K E N L A D

FLAG Lost XbaI

TATAAAGATGACGATGACAAATAATAAGCTAGAGGTACC (transcriptional stop)

117 Y K D D D D K

### pMPX-84::Pstl, Sall, Xbal::trxA (2-109 del 103-107)::FLAG

### Temperature inducible, clone into Pstl, Sall, Xbal

+1 Met required for protein to be fused

Made by cutting TOPO Pstl, Sall, Xbal::trxA (2-109 del 103-107)::FLAG-Nhel insertion with Pstl & Nhel and cloning into pMPX-84 cut with Pstl & Xbal.

### SEQ ID NO.: 277

### pMPX-95 TrxA (2-109 del 103-107) fusion vector

SD PstI SalI XbaI +2 trxA(del 103-107)

AGGAGGTTCTGCAGGCCTCGGTCGACGCCGAATCTAGAAGCGATAAAATTATT

1 A S V D A E S R S D K I I

CACCTGACTGACGACAGTTTTGACACGGATGTACTCAAAGCGGACGGGGCGATCCTCGTC

17 H L T D D S F D T D V L K A D G A I L V

GATTTCTGGGCAGAGTGGTGCGGTCCGTGCAAAATGATCGCCCCGATTCTGGATGAAATC

37 D F W A E W C G P C K M I A P I L D E I

GCTGACGAATATCAGGGCAAACTGACCGTTGCAAAACTGAACATCGATCAAAACCCTGGC

57 A D E Y Q G K L T V A K L N I D Q N P G

ACTGCGCCGAAATATGGCATCCGTGGTATCCCGACTCTGCTGCTGTTCAAAAACGGTGAA

77 T A P K Y G I R G I P T L L L F K N G E

GTGGCGGCAACCAAAGTGGGTGCACTGTCTAAAGGTCAGTTGAAAGAGAACCTGGCGGAT

97 V A A T K V G A L S K G Q L K E N L A D

FLAG Lost XbaI

TATAAAGATGACGATGACAAATAATAAGCTAGAGGTACC (transcriptional stop)

117 Y K D D D D K

### pMPX-86::Pstl, Sall, Xbal::trxA (2-109 del 103-107)::FLAG

### Temperature inducible, clone into Pstl, Sall, Xbal

+1 Met required for protein to be fused

Made by cutting TOPO Pstl, Sall, Xbal::trxA (2-109 del 103-107)::FLAG-Nhel insertion with Pstl & Nhel and cloning into pMPX-86 cut with Pstl & Xbal.

### SEQ ID NO.: 278

### pMPX-80 MalE (1-28) MCS TrxA (2-109 del 103-107) fusion vector

SD  Lost PstI +1 malE(1-
28)

2401    GAATTCAGGCGCTTTTTAGACTGGTCGTAATGAAATTCAGGAGGTTCTGCATATGAAAAT

1                                    M K I

2461    AAAAACAGGTGCACGCATCCTCGCATTATCCGCATTAACGACGATGATGTTTTCCGCCTC

4       K T G A R I L A L S A L T T M M F S A S

                                     Factor Xa   PstI      SalI       XbaI

2521    GGCTCTCGCCAAAATCATCGAAGCCCGCCTGCAGGCCTCGGTCGACGCCGAATCTAGAAG

24      A L A K I I E A R L Q A S V D A E S R S

        +2 trxA (2-109 del 103-107)

2581    CGATAAAATTATTCACCTGACTGACGACAGTTTTGACACGGATGTACTCAAAGCGGACGG

44      D K I I H L T D D S F D T D V L K A D G

2641    GGCGATCCTCGTCGATTTCTGGGCAGAGTGGTGCGGTCCGTGCAAAATGATCGCCCCGAT

64 A I L V D F W A E W C G P C K M I A P I

2701 TCTGGATGAAATCGCTGACGAATATCAGGGCAAACTGACCGTTGCAAAACTGAACATCGA

84 L D E I A D E Y Q G K L T V A K L N I D

2761 TCAAAACCCTGGCACTGCGCCGAAATATGGCATCCGTGGTATCCCGACTCTGCTGCTGTT

104 Q N P G T A P K Y G I R G I P T L L L F

2821 CAAAAACGGTGAAGTGGCGGCAACCAAAGTGGGTGCACTGTCTAAAGGTCAGTTGAAAGA

124 K N G E V A A T K V G A L S K G Q L K E

FLAG Lost XbaI

2881 GAACCTGGCGGATTATAAAGATGACGATGACAAATAATAAGCTAGAGG (trxn stop)

144 N L A D Y K D D D D K

### pMPX-72::malE(1-28)::FXa::Pstl, Sall, Xbal::TrxA(1-109 del 103-107)::FLAG

### Rhamnose inducible, clone into Pstl, Sall, Xbal

Made by cutting TOPO Nsil-malE (1-28)::FXa::Pstl, Sall, Xbal::FLAG-Nhel insertion with Nsil & Xbal and cloning into pMPX-78 cut with Pstl & Xbal.

### SEQ ID NO.: 279

### pMPX-81 MalE (1-28) MCS TrxA (2-109 del 103-107) fusion vector

SD Lost PstI +1 malE (1-28)

1621 CCATACCCGTTTTTTTGGGCTAGCAGGAGGCCCTGCATATGAAAATAAAAACAGGTGCAC

1 M K I K T G A

1681 GCATCCTCGCATTATCCGCATTAACGACGATGATGTTTTCCGCCTCGGCTCTCGCCAAAA

8 R I L A L S A L T T M M F S A S A L A K

+2 trxA(2-109 del Factor Xa PstI SalI XbaI 103-107)

1741 TCATCGAAGCCCGCCTGCAGGCCTCGGTCGACGCCGAATCTAGAAGCGATAAAATTATTC

28 I I E A R L Q A S V D A E S R S D K I I

1801 ACCTGACTGACGACAGTTTTGACACGGATGTACTCAAAGCGGACGGGGCGATCCTCGTCG

48 H L T D D S F D T D V L K A D G A I L V

1861 ATTTCTGGGCAGAGTGGTGCGGTCCGTGCAAAATGATCGCCCCGATTCTGGATGAAATCG

68 D F W A E W C G P C K M I A P I L D E I

1921 CTGACGAATATCAGGGCAAACTGACCGTTGCAAAACTGAACATCGATCAAAACCCTGGCA

88 A D E Y Q G K L T V A K L N I D Q N P G

1981 CTGCGCCGAAATATGGCATCCGTGGTATCCCGACTCTGCTGCTGTTCAAAAACGGTGAAG

108 T A P K Y G I R G I P T L L L F K N G E

2041 TGGCGGCAACCAAAGTGGGTGCACTGTCTAAAGGTCAGTTGAAAGAGAACCTGGCGGATT

128 V A A T K V G A L S K G Q L K E N L A D

FLAG

2101 ATAAAGATGACGATGACAAATAATAAGCTAGAGG (transcriptional stop)

148 Y K D D D D K

### pMPX-71::malE(1-28)::FXa::Pstl, Sall, XbaI::TrxA(1-109 del 103-107)::FLAG

### Arabinose inducible, clone into Pstl, Sall, Xbal

Made by cutting TOPO Nsil-malE (1-28)::FXa::Pstl, Sall, Xbal::FLAG-Nhel insertion with Nsil & Xbal and cloning into pMPX-79 cut with Pstl & Xbal.

### SEQ ID NO.: 280

### pMPX-91 MalE (1-28) MCS TrxA (2-109 del 103-107) fusion vector

SD  Lost PstI +1 malE(1-
28)

AGGAGGTTCTGCATATGAAAAT

1 M K I

AAAAACAGGTGCACGCATCCTCGCATTATCCGCATTAACGACGATGATGTTTTCCGCCTC

4 K T G A R I L A L S A L T T M M F S A S

Factor Xa PstI SalI XbaI

GGCTCTCGCCAAAATCATCGAAGCCCGCCTGCAGGCCTCGGTCGACGCCGAATCTAGAAG

24 A L A K I I E A R L Q A S V D A E S R S

+2 trxA (2-109 del 103-107)

CGATAAAATTATTCACCTGACTGACGACAGTTTTGACACGGATGTACTCAAAGCGGACGG

44 D K I I H L T D D S F D T D V L K A D G

GGCGATCCTCGTCGATTTCTGGGCAGAGTGGTGCGGTCCGTGCAAAATGATCGCCCCGAT

64 A I L V D F W A E W C G P C K M I A P I

TCTGGATGAAATCGCTGACGAATATCAGGGCAAACTGACCGTTGCAAAACTGAACATCGA

84 L D E I A D E Y Q G K L T V A K L N I D

TCAAAACCCTGGCACTGCGCCGAAATATGGCATCCGTGGTATCCCGACTCTGCTGCTGTT

104 Q N P G T A P K Y G I R G I P T L L L F

CAAAAACGGTGAAGTGGCGGCAACCAAAGTGGGTGCACTGTCTAAAGGTCAGTTGAAAGA

124 K N G E V A A T K V G A L S K G Q L K E

FLAG Lost XbaI

GAACCTGGCGGATTATAAAGATGACGATGACAAATAATAAGCTAGAGGTACC (trxn stop)

144 N L A D Y K D D D D K

### pMPX-84::malE(1-28)::FXa::Pstl, Sall, XbaI::TrxA(1-109 del 103-107)::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Xbal and cloning into pMPX-90 cut with Pstl & Xbal.

### SEQ ID NO.: 281

### pMPX-96 MalE (1-28) MCS TrxA (2-109 del 103-107) fusion vector

SD Lost PstI +1 malE(1-
28)

AGGAGGTTCTGCATATGAAAAT

1 M K I

AAAAACAGGTGCACGCATCCTCGCATTATCCGCATTAACGACGATGATGTTTTCCGCCTC

4 K T G A R I L A L S A L T T M M F S A S

Factor Xa PstI SalI XbaI

GGCTCTCGCCAAAATCATCGAAGCCCGCCTGCAGGCCTCGGTCGACGCCGAATCTAGAAG

24 A L A K I I E A R L Q A S V D A E S R S

+2 trxA (2-109 del 103-107)

CGATAAAATTATTCACCTGACTGACGACAGTTTTGACACGGATGTACTCAAAGCGGACGG

44 D K I I H L T D D S F D T D V L K A D
GGCGATCCTCGTCGATTTCTGGGCAGAGTGGTGCGGTCCGTGCAAAATGATCGCCCCGAT

64 A I L V D F W A E W C G P C K M I A P I

TCTGGATGAAATCGCTGACGAATATCAGGGCAAACTGACCGTTGCAAAACTGAACATCGA

84 L D E I A D E Y Q G K L T V A K L N I D

TCAAAACCCTGGCACTGCGCCGAAATATGGCATCCGTGGTATCCCGACTCTGCTGCTGTT

104 Q N P G T A P K Y G I R G I P T L L L F

CAAAAACGGTGAAGTGGCGGCAACCAAAGTGGGTGCACTGTCTAAAGGTCAGTTGAAAGA

124 K N G E V A A T K V G A L S K G Q L K E

FLAG Lost XbaI

GAACCTGGCGGATTATAAAGATGACGATGACAAATAATAAGCTAGAGGTACC (trxn stop)

144 N L A D Y K D D D D K

### pMPX-86::malE(1-28)::FXa::Pstl, Sall, Xbal::TrxA(1-109 del 103-107)::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Xbal and cloning into pMPX-95 cut with Pstl & Xbal.

### SEQ ID NO.: 282

### pMPX-83 MalE (1-370 del 354-364) MCS TrxA (2-109 del 103-107) fusion vector

SD Lost PstI +1 malE(1-
28)
2401 GAATTCAGGCGCTTTTTAGACTGGTCGTAATGAAATTCAGGAGGTTCTGCATATGAAAAT
1 M K I
2461 AAAAACAGGTGCACGCATCCTCGCATTATCCGCATTAACGACGATGATGTTTTCCGCCTC
4 K T G A R I L A L S A L T T M M F S A S
2521 GGCTCTCGCCAAAATCGAAGAAGGTAAACTGGTAATCTGGATTAACGGCGATAAAGGCTA
24 A L A K I E E G K L V I W I N G D K G Y
2581 TAACGGTCTCGCTGAAGTCGGTAAGAAATTCGAGAAAGATACCGGAATTAAAGTCACCGT
44 N G L A E V G K K F E K D T G I K V T V
2641 TGAGCATCCGGATAAACTGGAAGAGAAATTCCCACAGGTTGCGGCAACTGGCGATGGCCC
64 E H P D K L E E K F P Q V A A T G D G P
2701 TGACATTATCTTCTGGGCACACGACCGCTTTGGTGGCTACGCTCAATCTGGCCTGTTGGC

84 D I I F W A H D R F G G Y A Q S G L L A

2761 TGAAATCACCCCGGACAAAGCGTTCCAGGACAAGCTGTATCCGTTTACCTGGGATGCCGT

104 E I T P D K A F Q D K L Y P F T W D A V

2821 ACGTTACAACGGCAAGCTGATTGCTTACCCGATCGCTGTTGAAGCGTTATCGCTGATTTA

124 R Y N G K L I A Y P I A V E A L S L I Y

2881 TAACAAAGATCTGCTGCCGAACCCGCCAAAAACCTGGGAAGAGATCCCGGCGCTGGATAA

144 N K D L L P N P P K T W E E I P A L D K

2941 AGAACTGAAAGCGAAAGGTAAGAGCGCGCTGATGTTCAACCTGCAAGAACCGTACTTCAC

164 E L K A K G K S A L M F N L Q E P Y F T

3001 CTGGCCGCTGATTGCTGCTGACGGGGGTTATGCGTTCAAGTATGAAAACGGCAAGTACGA

184 W P L I A A D G G Y A F K Y E N G K Y D

3061 CATTAAAGACGTGGGCGTGGATAACGCTGGCGCGAAAGCGGGTCTGACCTTCCTGGTTGA

204 I K D V G V D N A G A K A G L T F L V D

3121 CCTGATTAAAAACAAACACATGAATGCAGACACCGATTACTCCATCGCAGAAGCTGCCTT

224 L I K N K H M N A D T D Y S I A E A A F

3181 TAATAAAGGCGAAACAGCGATGACCATCAACGGCCCGTGGGCATGGTCCAACATCGACAC

244 N K G E T A M T I N G P W A W S N I D T

3241 CAGCAAAGTGAATTATGGTGTAACGGTACTGCCGACCTTCAAGGGTCAACCATCCAAACC

264 S K V N Y G V T V L P T F K G Q P S K P

3301 GTTCGTTGGCGTGCTGAGCGCAGGTATTAACGCCGCCAGTCCGAACAAAGAGCTGGCGAA

284 F V G V L S A G I N A A S P N K E L A K

3361 AGAGTTCCTCGAAAACTATCTGCTGACTGATGAAGGTCTGGAAGCGGTTAATAAAGACAA

304 E F L E N Y L L T D E G L E A V N K D K

3421 ACCGCTGGGTGCCGTAGCGCTGAAGTCTTACGAGGAAGAGTTGGCGAAAGATCCACGTAT

324 P L G A V A L K S Y E E E L A K D P R I

Factor

Xa

3481 TGCCGCCACCATGGAAAACGCCCAGTCCGCTTTCTGGTATGCCGTGCGTATCGAAGCCCG

344 A A T M E N A Q S A F W Y A V R I E A R

PstI SalI XbaI +2 trxA (2-109 del 103-107)

3541 CCTGCAGGCCTCGGTCGACGCCGAATCTAGAAGCGATAAAATTATTCACCTGACTGACGA

364 L Q A S V D A E S R S D K I I H L T D D

3601 CAGTTTTGACACGGATGTACTCAAAGCGGACGGGGCGATCCTCGTCGATTTCTGGGCAGA

384 S F D T D V L K A D G A I L V D F W A E

3661 GTGGTGCGGTCCGTGCAAAATGATCGCCCCGATTCTGGATGAAATCGCTGACGAATATCA

404 W C G P C K M I A P I L D E I A D E Y Q

3721 GGGCAAACTGACCGTTGCAAAACTGAACATCGATCAAAACCCTGGCACTGCGCCGAAATA

424 G K L T V A K L N I D Q N P G T A P K Y

3781 TGGCATCCGTGGTATCCCGACTCTGCTGCTGTTCAAAAACGGTGAAGTGGCGGCAACCAA

444 G I R G I P T L L L F K N G E V A A T K

FLAG

3841 AGTGGGTGCACTGTCTAAAGGTCAGTTGAAAGAGAACCTGGCGGATTATAAAGATGACGA

464 V G A L S K G Q L K E N L A D Y K D D D

3901 TGACAAATAATAAGCTAGAGG (transcriptional stop)

484 D K

### pMPX-72::malE(1-320 del 354-364)::FXa::Pstl, Sall, XbaI::TrxA(1-109 del 103-107)::FLAG

### Rhamnose inducible, clone into Pstl, Sall, Xbal

Made by cutting TOPO Nsil-malE (1-370 del 354-364)::FXa::Pstl, Sall, Xbal::FLAG-Nhel insertion with Nsil & Xbal and cloning into pMPX-78 cut with Pstl & Xbal.

### SEQ ID NO.: 283

### pMPX-82 MalE (1-370 del 354-364) MCS TrxA (2-109 del 103-107) fusion vector

SD Lost PstI +1 malE (1-370 del 352-362)

1621 CCATACCCGTTTTTTTGGGCTAGCAGGAGGCCCTGCATATGAAAATAAAAACAGGTGCAC

1 M K I K T G A

1681 GCATCCTCGCATTATCCGCATTAACGACGATGATGTTTTCCGCCTCGGCTCTCGCCAAAA

8 R I L A L S A L T T M M F S A S A L A K

1741 TCGAAGAAGGTAAACTGGTAATCTGGATTAACGGCGATAAAGGCTATAACGGTCTCGCTG

28 I E E G K L V I W I N G D K G Y N G L A

1801 AAGTCGGTAAGAAATTCGAGAAAGATACCGGAATTAAAGTCACCGTTGAGCATCCGGATA

48 E V G K K F E K D T G I K V T V E H P D

1861 AACTGGAAGAGAAATTCCCACAGGTTGCGGCAACTGGCGATGGCCCTGACATTATCTTCT

68 K L E E K F P Q V A A T G D G P D I I F

1921 GGGCACACGACCGCTTTGGTGGCTACGCTCAATCTGGCCTGTTGGCTGAAATCACCCCGG

88 W A H D R F G G Y A Q S G L L A E I T P

1981 ACAAAGCGTTCCAGGACAAGCTGTATCCGTTTACCTGGGATGCCGTACGTTACAACGGCA

108 D K A F Q D K L Y P F T W D A V R Y N G

2041 AGCTGATTGCTTACCCGATCGCTGTTGAAGCGTTATCGCTGATTTATAACAAAGATCTGC

128 K L I A Y P I A V E A L S L I Y N K D L

2101 TGCCGAACCCGCCAAAAACCTGGGAAGAGATCCCGGCGCTGGATAAAGAACTGAAAGCGA

148 L P N P P K T W E E I P A L D K E L K A

2161 AAGGTAAGAGCGCGCTGATGTTCAACCTGCAAGAACCGTACTTCACCTGGCCGCTGATTG

168 K G K S A L M F N L Q E P Y F T W P L I

2221 CTGCTGACGGGGGTTATGCGTTCAAGTATGAAAACGGCAAGTACGACATTAAAGACGTGG

188 A A D G G Y A F K Y E N G K Y D I K D V

2281 GCGTGGATAACGCTGGCGCGAAAGCGGGTCTGACCTTCCTGGTTGACCTGATTAAAAACA

208 G V D N A G A K A G L T F L V D L I K N

2341 AACACATGAATGCAGACACCGATTACTCCATCGCAGAAGCTGCCTTTAATAAAGGCGAAA

228 K H M N A D T D Y S I A E A A F N K G E

2401 CAGCGATGACCATCAACGGCCCGTGGGCATGGTCCAACATCGACACCAGCAAAGTGAATT

248 T A M T I N G P W A W S N I D T S K V N

2461 ATGGTGTAACGGTACTGCCGACCTTCAAGGGTCAACCATCCAAACCGTTCGTTGGCGTGC

268 Y G V T V L P T F K G Q P S K P F V G V

2521 TGAGCGCAGGTATTAACGCCGCCAGTCCGAACAAAGAGCTGGCGAAAGAGTTCCTCGAAA

288 L S A G I N A A S P N K E L A K E F L E

2581 ACTATCTGCTGACTGATGAAGGTCTGGAAGCGGTTAATAAAGACAAACCGCTGGGTGCCG

308 N Y L L T D E G L E A V N K D K P L G A

2641 TAGCGCTGAAGTCTTACGAGGAAGAGTTGGCGAAAGATCCACGTATTGCCGCCACCATGG

328 V A L K S Y E E E L A K D P R I A A T M

Factor Xa PstI

2701 AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI +2 trxA (2-109 del 103-107)

2761 TCGACGCCGAATCTAGAAGCGATAAAATTATTCACCTGACTGACGACAGTTTTGACACGG

368 V D A E S R S D K I I H L T D D S F D T

2821 ATGTACTCAAAGCGGACGGGGCGATCCTCGTCGATTTCTGGGCAGAGTGGTGCGGTCCGT

388 D V L K A D G A I L V D F W A E W C G P

2881 GCAAAATGATCGCCCCGATTCTGGATGAAATCGCTGACGAATATCAGGGCAAACTGACCG

408 C K M I A P I L D E I A D E Y Q G K L T

2941 TTGCAAAACTGAACATCGATCAAAACCCTGGCACTGCGCCGAAATATGGCATCCGTGGTA

428 V A K L N I D Q N P G T A P K Y G I R G

3001 TCCCGACTCTGCTGCTGTTCAAAAACGGTGAAGTGGCGGCAACCAAAGTGGGTGCACTGT

448 I P T L L L F K N G E V A A T K V G A L

FLAG

3061 CTAAAGGTCAGTTGAAAGAGAACCTGGCGGATTATAAAGATGACGATGACAAATAATAAG

468 S K G Q L K E N L A D Y K D D D D K

Lost XbaI

CTAGAGG (transcriptional stop)

### pMPX-71::malE(1-370 del 354-364)::FXa::PstI, Sall, XbaI::TrxA(1-109 del 103-107)::FLAG

### Arabinose inducible, clone into Pstl, Sall, Xbal

Made by cutting TOPO Nsil-malE (1-370 del 354-364)::FXa::Pstl, Sall, Xbal::FLAG-Nhel insertion with NsiI & Xbal and cloning into pMPX-79 cut with Pstl & Xbal.

### SEQ ID NO.: 284

### pMPX-92 MalE (1-370 del 354-364) MCS TrxA (2-109 del 103-107) fusion vector

SD  Lost PstI +1 malE (1-370 del 354-364)

AGGAGGTTCTGCATATGAAAATAAAAACAGGTGCAC

1 M K I K T G A

GCATCCTCGCATTATCCGCATTAACGACGATGATGTTTTCCGCCTCGGCTCTCGCCAAAA

8 R I L A L S A L T T M M F S A S A L A K

TCGAAGAAGGTAAACTGGTAATCTGGATTAACGGCGATAAAGGCTATAACGGTCTCGCTG

28 I E E G K L V I W I N G D K G Y N G L A

AAGTCGGTAAGAAATTCGAGAAAGATACCGGAATTAAAGTCACCGTTGAGCATCCGGATA

48 E V G K K F E K D T G I K V T V E H P D

AACTGGAAGAGAAATTCCCACAGGTTGCGGCAACTGGCGATGGCCCTGACATTATCTTCT

68 K L E E K F P Q V A A T G D G P D I I F

GGGCACACGACCGCTTTGGTGGCTACGCTCAATCTGGCCTGTTGGCTGAAATCACCCCGG

88 W A H D R F G G Y A Q S G L L A E I T P

ACAAAGCGTTCCAGGACAAGCTGTATCCGTTTACCTGGGATGCCGTACGTTACAACGGCA

108 D K A F Q D K L Y P F T W D A V R Y N G

AGCTGATTGCTTACCCGATCGCTGTTGAAGCGTTATCGCTGATTTATAACAAAGATCTGC

128 K L I A Y P I A V E A L S L I Y N K D L

TGCCGAACCCGCCAAAAACCTGGGAAGAGATCCCGGCGCTGGATAAAGAACTGAAAGCGA

148 L P N P P K T W E E I P A L D K E L K A

AAGGTAAGAGCGCGCTGATGTTCAACCTGCAAGAACCGTACTTCACCTGGCCGCTGATTG

168 K G K S A L M F N L Q E P Y F T W P L I

CTGCTGACGGGGGTTATGCGTTCAAGTATGAAAACGGCAAGTACGACATTAAAGACGTGG

188 A A D G G Y A F K Y E N G K Y D I K D V

GCGTGGATAACGCTGGCGCGAAAGCGGGTCTGACCTTCCTGGTTGACCTGATTAAAAACA

208 G V D N A G A K A G L T F L V D L I K N

2341 AACACATGAATGCAGACACCGATTACTCCATCGCAGAAGCTGCCTTTAATAAAGGCGAAA

228 K H M N A D T D Y S I A E A A F N K G E

2401 CAGCGATGACCATCAACGGCCCGTGGGCATGGTCCAACATCGACACCAGCAAAGTGAATT

248 T A M T I N G P W A W S N I D T S K V N

2461 ATGGTGTAACGGTACTGCCGACCTTCAAGGGTCAACCATCCAAACCGTTCGTTGGCGTGC

268 Y G V T V L P T F K G Q P S K P F V G V

2521 TGAGCGCAGGTATTAACGCCGCCAGTCCGAACAAAGAGCTGGCGAAAGAGTTCCTCGAAA

288 L S A G I N A A S P N K E L A K E F L E

2581 ACTATCTGCTGACTGATGAAGGTCTGGAAGCGGTTAATAAAGACAAACCGCTGGGTGCCG

308 N Y L L T D E G L E A V N K D K P L G A

2641 TAGCGCTGAAGTCTTACGAGGAAGAGTTGGCGAAAGATCCACGTATTGCCGCCACCATGG

328 V A L K S Y E E E L A K D P R I A A T M

Factor Xa PstI

2701 AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI +2 trxA (2-109 del 103-107)

2761 TCGACGCCGAATCTAGAAGCGATAAAATTATTCACCTGACTGACGACAGTTTTGACACGG

368 V D A E S R S D K I I H L T D D S F D T

2821 ATGTACTCAAAGCGGACGGGGCGATCCTCGTCGATTTCTGGGCAGAGTGGTGCGGTCCGT

388 D V L K A D G A I L V D F W A E W C G P

2881 GCAAAATGATCGCCCCGATTCTGGATGAAATCGCTGACGAATATCAGGGCAAACTGACCG

408 C K M I A P I L D E I A D E Y Q G K L T

2941 TTGCAAAACTGAACATCGATCAAAACCCTGGCACTGCGCCGAAATATGGCATCCGTGGTA

428 V A K L N I D Q N P G T A P K Y G I R G

3001 TCCCGACTCTGCTGCTGTTCAAAAACGGTGAAGTGGCGGCAACCAAAGTGGGTGCACTGT

448 I P T L L L F K N G E V A A T K V G A L

FLAG

3061 CTAAAGGTCAGTTGAAAGAGAACCTGGCGGATTATAAAGATGACGATGACAAATAATAAG

468 S K G Q L K E N L A D Y K D D D D K

Lost XbaI

CTAGAGGTACC (transcriptional stop)

### pMPX-84::malE(1-370 del 354-364)::FXa::Pstl, Sall, Xbal::TrxA(1-109 del 103-107)::FLAG

### Temperature inducible, clone into Pstl, Sall, Xbal

Made by cutting TOPO Nsil-malE (1-370 del 354-364)::FXa::Pstl, Sall, Xbal::FLAG-Nhel insertion with Nsil & Xbal and cloning into pMPX-90 cut with Pstl & Xbal.

### SEQ ID NO.: 285

### pMPX-97 MalE (1-370 del 354-364) MCS TrxA (2-109 del 103-107) fusion vector

SD Lost PstI +1 malE (1-370 del 354-364)

AGGAGGTTCTGCATATGAAAATAAAAACAGGTGCAC

1 M K I K T G A

GCATCCTCGCATTATCCGCATTAACGACGATGATGTTTTCCGCCTCGGCTCTCGCCAAAA

8 R I L A L S A L T T M M F S A S A L A K

TCGAAGAAGGTAAACTGGTAATCTGGATTAACGGCGATAAAGGCTATAACGGTCTCGCTG

28 I E E G K L V I W I N G D K G Y N G L A

AAGTCGGTAAGAAATTCGAGAAAGATACCGGAATTAAAGTCACCGTTGAGCATCCGGATA

48 E V G K K F E K D T G I K V T V E H P D

AACTGGAAGAGAAATTCCCACAGGTTGCGGCAACTGGCGATGGCCCTGACATTATCTTCT

68 K L E E K F P Q V A A T G D G P D I I F

GGGCACACGACCGCTTTGGTGGCTACGCTCAATCTGGCCTGTTGGCTGAAATCACCCCGG

88 W A H D R F G G Y A Q S G L L A E I T P

ACAAAGCGTTCCAGGACAAGCTGTATCCGTTTACCTGGGATGCCGTACGTTACAACGGCA

108 D K A F Q D K L Y P F T W D A V R Y N G

AGCTGATTGCTTACCCGATCGCTGTTGAAGCGTTATCGCTGATTTATAACAAAGATCTGC

128 K L I A Y P I A V E A L S L I Y N K D L

TGCCGAACCCGCCAAAAACCTGGGAAGAGATCCCGGCGCTGGATAAAGAACTGAAAGCGA

148 L P N P P K T W E E I P A L D K E L K A

AAGGTAAGAGCGCGCTGATGTTCAACCTGCAAGAACCGTACTTCACCTGGCCGCTGATTG

168 K G K S A L M F N L Q E P Y F T W P L I

CTGCTGACGGGGGTTATGCGTTCAAGTATGAAAACGGCAAGTACGACATTAAAGACGTGG

188 A A D G G Y A F K Y E N G K Y D I K D V

GCGTGGATAACGCTGGCGCGAAAGCGGGTCTGACCTTCCTGGTTGACCTGATTAAAAACA

208 G V D N A G A K A G L T F L V D L I K N

2341 AACACATGAATGCAGACACCGATTACTCCATCGCAGAAGCTGCCTTTAATAAAGGCGAAA

228 K H M N A D T D Y S I A E A A F N K G E

2401 CAGCGATGACCATCAACGGCCCGTGGGCATGGTCCAACATCGACACCAGCAAAGTGAATT

248 T A M T I N G P W A W S N I D T S K V N

2461 ATGGTGTAACGGTACTGCCGACCTTCAAGGGTCAACCATCCAAACCGTTCGTTGGCGTGC

268 Y G V T V L P T F K G Q P S K P F V G V

2521 TGAGCGCAGGTATTAACGCCGCCAGTCCGAACAAAGAGCTGGCGAAAGAGTTCCTCGAAA

288 L S A G I N A A S P N K E L A K E F L E

2581 ACTATCTGCTGACTGATGAAGGTCTGGAAGCGGTTAATAAAGACAAACCGCTGGGTGCCG

308 N Y L L T D E G L E A V N K D K P L G A

2641 TAGCGCTGAAGTCTTACGAGGAAGAGTTGGCGAAAGATCCACGTATTGCCGCCACCATGG

328 V A L K S Y E E E L A K D P R I A A T M

Factor Xa PstI

2701 AAAACGCCCAGTCCGCTTTCTGGTATGCCGTGCGTATCGAAGCCCGCCTGCAGGCCTCGG

348 E N A Q S A F W Y A V R I E A R L Q A S

SalI XbaI +2 trxA (2-109 del 103-107)

2761 TCGACGCCGAATCTAGAAGCGATAAAATTATTCACCTGACTGACGACAGTTTTGACACGG

368 V D A E S R S D K I I H L T D D S F D T

2821 ATGTACTCAAAGCGGACGGGGCGATCCTCGTCGATTTCTGGGCAGAGTGGTGCGGTCCGT

388 D V L K A D G A I L V D F W A E W C G P

2881 GCAAAATGATCGCCCCGATTCTGGATGAAATCGCTGACGAATATCAGGGCAAACTGACCG

408 C K M I A P I L D E I A D E Y Q G K L T

2941 TTGCAAAACTGAACATCGATCAAAACCCTGGCACTGCGCCGAAATATGGCATCCGTGGTA

428 V A K L N I D Q N P G T A P K Y G I R G

3001 TCCCGACTCTGCTGCTGTTCAAAAACGGTGAAGTGGCGGCAACCAAAGTGGGTGCACTGT

448 I P T L L L F K N G E V A A T K V G A L

FLAG

3061 CTAAAGGTCAGTTGAAAGAGAACCTGGCGGATTATAAAGATGACGATGACAAATAATAAG

468 S K G Q L K E N L A D Y K D D D D K

Lost XbaI

CTAGAGGTACC (transcriptional stop)

### pMPX-86::maIE(1-370 del 354-364)::FXa::Pstl, Sall, XbaI::TrxA(1-109 del 103-107)::FLAG

### Temperature inducible, clone into Pstl, Sall, Xbal

Made by cutting TOPO Nsil-malE (1-370 del 354-364)::FXa::Pstl, Sall, Xbal::FLAG-Nhel insertion with Nsil & Xbal and cloning into pMPX-95 cut with Pstl & Xbal.

### SEQ ID NO.: 151

### pMPX-66 arabinose-inducible expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

HindIII
361 TTTCCCAGTC ACGACGTTGT AAAACGACGG CCAGTGCCAA GCTTCAAGCC GTCAATTGTC

Stop araC
421 TGATTCGTTA CCAATTATGA CAACTTGACG GCTACATCAT TCACTTTTTC TTCACAACCG

481 GCACGGAACT CGCTCGGGCT GGCCCCGGTG CATTTTTTAA ATACCCGCGA GAAATAGAGT
541 TGATCGTCAA AACCAACATT GCGACCGACG GTGGCGATAG GCATCCGGGT GGTGCTCAAA
601 AGCAGCTTCG CCTGGCTGAT ACGTTGGTCC TCGCGCCAGC TTAAGACGCT AATCCCTAAC
661 TGCTGGCGGA AAAGATGTGA CAGACGCGAC GGCGACAAGC AAACATGCTG TGCGACGCTG
721 GCGATATCAA AATTGCTGTC TGCCAGGTGA TCGCTGATGT ACTGACAAGC CTCGCGTACC
781 CGATTATCCA TCGGTGGATG GAGCGACTCG TTAATCGCTT CCATGCGCCG CAGTAACAAT
841 TGCTCAAGCA GATTTATCGC CAGCAGCTCC GAATAGCGCC CTTCCCCTTG CCCGGCGTTA
901 ATGATTTGCC CAAACAGGTC GCTGAAATGC GGCTGGTGCG CTTCATCCGG GCGAAAGAAC
961 CCCGTATTGG CAAATATTGA CGGCCAGTTA AGCCATTCAT GCCAGTAGGC GCGCGGACGA
1021 AAGTAAACCC ACTGGTGATA CCATTCGCGA GCCTCCGGAT GACGACCGTA GTGATGAATC
1081 TCTCCTGGCG GGAACAGCAA AATATCACCC GGTCGGCAAA CAAATTCTCG TCCCTGATTT
1141 TTCACCACCC CCTGACCGCG AATGGTGAGA TTGAGAATAT AACCTTTCAT TCCCAGCGGT
1201 CGGTCGATAA AAAAATCGAG ATAACCGTTG GCCTCAATCG GCGTTAAACC CGCCACCAGA
1261 TGGGCATTAA ACGAGTATCC CGGCAGCAGG GGATCATTTT GCGCTTCAGC CATACTTTTC

Start araC
1321 ATACTCCCGC CATTCAGAGA AGAAACCAAT TGTCCATATT GCATCAGACA TTGCCGTCAC

<--

1381 TGCGTCTTTT ACTGGCTCTT CTCGCTAACC AAACCGGTAA CCCCGCTTAT TAAAAGCATT
1441 CTGTAACAAA GCGGGACCAA AGCCATGACA AAAACGCGTA ACAAAAGTGT CTATAATCAC
1501 GGCAGAAAAG TCCACATTGA TTATTTGCAC GGCGTCACAC TTTGCTATGC CATAGCATTT
1561 TTATCCATAA GATTAGCGGA TCCTACCTGA CGCTTTTTAT CGCAACTCTC TACTGTTTCT

SD Sall XbaI
1621 CCATACCCGT TTTTTTGGGC TAGCAGGAGG CCGTCGACTC TAGAGGATCC CCGCGCCCTC

Stem-loop KpnI
1681 ATCCGAAAGG GCGTATTGGT ACCGAGCTCG AATTCGTAAT CATGGTCATA GCTGTTTCCT

1741 GTGTGAAATT GTTATCCGCT CACAATTCCA CACAACATAC GAGCCGGAAG CATAAAGTGT
1801 AAAGCCTGGG GTGCCTAATG AGTGAGCTAA CTCACATTAA TTGCGTTGCG CTCACTGCCC
1861 GCTTTCCAGT CGGGAAACCT GTCGTGCCAG CTGCATTAAT GAATCGGCCA ACGCGCGGGG
1921 AGAGGCGGTT TGCGTATTGG GCGCTCTTCC GCTTCCTCGC TCACTGACTC GCTGCGCTCG
1981 GTCGTTCGGC TGCGGCGAGC GGTATCAGCT CACTCAAAGG CGGTAATACG GTTATCCACA
2041 GAATCAGGGG ATAACGCAGG AAAGAACATG TGAGCAAAAG GCCAGCAAAA GGCCAGGAAC
2101 CGTAAAAAGG CCGCGTTGCT GGCGTTTTTC CATAGGCTCC GCCCCCCTGA CGAGCATCAC
2161 AAAAATCGAC GCTCAAGTCA GAGGTGGCGA AACCCGACAG GACTATAAAG ATACCAGGCG
2221 TTTCCCCCTG GAAGCTCCCT CGTGCGCTCT CCTGTTCCGA CCCTGCCGCT TACCGGATAC
2281 CTGTCCGCCT TTCTCCCTTC GGGAAGCGTG GCGCTTTCTC ATAGCTCACG CTGTAGGTAT
2341 CTCAGTTCGG TGTAGGTCGT TCGCTCCAAG CTGGGCTGTG TGCACGAACC CCCCGTTCAG
2401 CCCGACCGCT GCGCCTTATC CGGTAACTAT CGTCTTGAGT CCAACCCGGT AAGACACGAC
2461 TTATCGCCAC TGGCAGCAGC CACTGGTAAC AGGATTAGCA GAGCGAGGTA TGTAGGCGGT
2521 GCTACAGAGT TCTTGAAGTG GTGGCCTAAC TACGGCTACA CTAGAAGGAC AGTATTTGGT
2581 ATCTGCGCTC TGCTGAAGCC AGTTACCTTC GGAAAAAGAG TTGGTAGCTC TTGATCCGGC
2641 AAACAAACCA CCGCTGGTAG CGGTGGTTTT TTTGTTTGCA AGCAGCAGAT TACGCGCAGA
2701 AAAAAAGGAT CTCAAGAAGA TCCTTTGATC TTTTCTACGG GGTCTGACGC TCAGTGGAAC
2761 GAAAACTCAC GTTAAGGGAT TTTGGTCATG AGATTATCAA AAAGGATCTT CACCTAGATC
2821 CTTTTAAATT AAAAATGAAG TTTTAAATCA ATCTAAAGTA TATATGAGTA AACTTGGTCT
2881 GACAGTTACC AATGCTTAAT CAGTGAGGCA CCTATCTCAG CGATCTGTCT ATTTCGTTCA
2941 TCCATAGTTG CCTGACTCCC CGTCGTGTAG ATAACTACGA TACGGGAGGG CTTACCATCT
3001 GGCCCCAGTG CTGCAATGAT ACCGCGAGAC CCACGCTCAC CGGCTCCAGA TTTATCAGCA
3061 ATAAACCAGC CAGCCGGAAG GGCCGAGCGC AGAAGTGGTC CTGCAACTTT ATCCGCCTCC
3121 ATCCAGTCTA TTAATTGTTG CCGGGAAGCT AGAGTAAGTA GTTCGCCAGT TAATAGTTTG
3181 CGCAACGTTG TTGCCATTGC TACAGGCATC GTGGTGTCAC GCTCGTCGTT TGGTATGGCT
3241 TCATTCAGCT CCGGTTCCCA ACGATCAAGG CGAGTTACAT GATCCCCCAT GTTGTGCAAA
3301 AAAGCGGTTA GCTCCTTCGG TCCTCCGATC GTTGTCAGAA GTAAGTTGGC CGCAGTGTTA
3361 TCACTCATGG TTATGGCAGC ACTGCATAAT TCTCTTACTG TCATGCCATC CGTAAGATGC
3421 TTTTCTGTGA CTGGTGAGTA CTCAACCAAG TCATTCTGAG AATAGTGTAT GCGGCGACCG
3481 AGTTGCTCTT GCCCGGCGTC AATACGGGAT AATACCGCGC CACATAGCAG AACTTTAAAA
3541 GTGCTCATCA TTGGAAAACG TTCTTCGGGG CGAAAACTCT CAAGGATCTT ACCGCTGTTG
3601 AGATCCAGTT CGATGTAACC CACTCGTGCA CCCAACTGAT CTTCAGCATC TTTTACTTTC
3661 ACCAGCGTTT CTGGGTGAGC AAAAACAGGA AGGCAAAATG CCGCAAAAAA GGGAATAAGG
3721 GCGACACGGA AATGTTGAAT ACTCATACTC TTCCTTTTTC AATATTATTG AAGCATTTAT
3781 CAGGGTTATT GTCTCATGAG CGGATACATA TTTGAATGTA TTTAGAAAAA TAAACAAATA
3841 GGGGTTCCGC GCACATTTCC CCGAAAAGTG CCACCTGACG TCTAAGAAAC CATTATTATC
3901 ATGACATTAA CCTATAAAAA TAGGCGTATC ACGAGGCCCT TTCGTC

The segment araC through Para was taken from pBAD24 using PCR added Hindlll and modified aligned Shine-Delgarno (SD) sequence with Sall followed by Xbal, a stem-loop transcriptional stop sequence, and Kpnl. The PCR product was cloned into pUC18 using Hindlll and Kpnl.

### SEO ID NO.: 152

### pMPX-72 rhamnose-inducible expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

Stop rhaR
361 TTTCCCAGTC ACGACGTTGT AAAACGACGG CCAGTGCCAA GCTTAATTAA TCTTTCTGCG
HindIII

421 AATTGAGATG ACGCCACTGG CTGGGCGTCA TCCCGGTTTC CCGGGTAAAC ACCACCGAAA
481 AATAGTTACT ATCTTCAAAG CCACATTCGG TCGAAATATC ACTGATTAAC AGGCGGCTAT
541 GCTGGAGAAG ATATTGCGCA TGACACACTC TGACCTGTCG CAGATATTGA TTGATGGTCA
601 TTCCAGTCTG CTGGCGAAAT TGCTGACGCA AAACGCGCTC ACTGCACGAT GCCTCATCAC
661 AAAATTTATC CAGCGCAAAG GGACTTTTCA GGCTAGCCGC CAGCCGGGTA ATCAGCTTAT
721 CCAGCAACGT TTCGCTGGAT GTTGGCGGCA ACGAATCACT GGTGTAACGA TGGCGATTCA
781 GCAACATCAC CAACTGCCCG AACAGCAACT CAGCCATTTC GTTAGCAAAC GGCACATGCT
841 GACTACTTTC ATGCTCAAGC TGACCGATAA CCTGCCGCGC CTGCGCCATC CCCATGCTAC
901 CTAAGCGCCA GTGTGGTTGC CCTGCGCTGG CGTTAAATCC CGGAATCGCC CCCTGCCAGT
961 CAAGATTCAG CTTCAGACGC TCCGGGCAAT AAATAATATT CTGCAAAACC AGATCGTTAA
1021 CGGAAGCGTA GGAGTGTTTA TCGTCAGCAT GAATGTAAAA GAGATCGCCA CGGGTAATGC
1081 GATAAGGGCG ATCGTTGAGT ACATGCAGGC CATTACCGCG CCAGACAATC ACCAGCTCAC
1141 AAAAATCATG TGTATGTTCA GCAAAGACAT CTTGCGGATA ACGGTCAGCC ACAGCGACTG
1201 CCTGCTGGTC GCTGGCAAAA AAATCATCTT TGAGAAGTTT TAACTGATGC GCCACCGTGG
1261 CTACCTCGGC CAGAGAACGA AGTTGATTAT TCGCAATATG GCGTACAAAT ACGTTGAGAA

Stop rhaS Start rhaR
1321 GATTCGCGTT ATTGCAGAAA GCCATCCCGT CCCTGGCGAA TATCACGCGG TGACCAGTTA
<--

1381 AACTCTCGGC GAAAAAGCGT CGAAAAGTGG TTACTGTCGC TGAATCCACA GCGATAGGCG
1441 ATGTCAGTAA CGCTGGCCTC GCTGTGGCGT AGCAGATGTC GGGCTTTCAT CAGTCGCAGG
1501 CGGTTCAGGT ATCGCTGAGG CGTCAGTCCC GTTTGCTGCT TAAGCTGCCG ATGTAGCGTA
1561 CGCAGTGAAA GAGAAAATTG ATCCGCCACG GCATCCCAAT TCACCTCATC GGCAAAATGG
1621 TCCTCCAGCC AGGCCAGAAG CAAGTTGAGA CGTGATGCGC TGTTTTCCAG GTTCTCCTGC
1681 AAACTGCTTT TACGCAGCAA GAGCAGTAAT TGCATAAACA AGATCTCGCG ACTGGCGGTC
1741 GAGGGTAAAT CATTTTCCCC TTCCTGCTGT TCCATCTGTG CAACCAGCTG TCGCACCTGC
1801 TGCAATACGC TGTGGTTAAC GCGCCAGTGA GACGGATACT GCCCATCCAG CTCTTGTGGC
1861 AGCAACTGAT TCAGCCCGGC GAGAAACTGA AATCGATCCG GCGAGCGATA CAGCACATTG
1921 GTCAGACACA GATTATCGGT ATGTTCATAC AGATGCCGAT CATGATCGCG TACGAAACAG
1981 ACCGTGCCAC CGGTGATGGT ATAGGGCTGC CCATTAAACA CATGAATACC CGTGCCATGT
2041 TCGACAATCA CAATTTCATG AAAATCATGA TGATGTTCAG GAAAATCCGC CTGCGGGAGC
2101 CGGGGTTCTA TCGCCACGGA CGCGTTACCA GACGGAAAAA AATCCACACT ATGTAATACG

Start rhaS
2161 GTCATACTGG CCTCCTGATG TCGTCAACAC GGCGAAATAG TAATCACGAG GTCAGGTTCT
<--

2221 TACCTTAAAT TTTCGACGGA AAACCACGTA AAAAACGTCG ATTTTTCAAG ATACAGCGTG
2281 AATTTTCAGG AAATGCGGTG AGCATCACAT CACCACAATT CAGCAAATTG TGAACATCAT
2341 CACGTTCATC TTTCCCTGGT TGCCAATGGC CCATTTTCCT GTCAGTAACG AGAAGGTCGC

SD PstI SalI
2401 GAATTCAGGC GCTTTTTAGA CTGGTCGTAA TGAAATTCAG GAGGTTCTGC AGGTCGACTC

XbaI Stem-loop KpnI

2461 TAGAGGATCC CCGCGCCCTC ATCCGAAAGG GCGTATTGGT ACCGAGCTCG AATTCGTAAT

2521 CATGGTCATA GCTGTTTCCT GTGTGAAATT GTTATCCGCT CACAATTCCA CACAACATAC
2581 GAGCCGGAAG CATAAAGTGT AAAGCCTGGG GTGCCTAATG AGTGAGCTAA CTCACATTAA
2641 TTGCGTTGCG CTCACTGCCC GCTTTCCAGT CGGGAAACCT GTCGTGCCAG CTGCATTAAT
2701 GAATCGGCCA ACGCGCGGGG AGAGGCGGTT TGCGTATTGG GCGCTCTTCC GCTTCCTCGC
2761 TCACTGACTC GCTGCGCTCG GTCGTTCGGC TGCGGCGAGC GGTATCAGCT CACTCAAAGG
2821 CGGTAATACG GTTATCCACA GAATCAGGGG ATAACGCAGG AAAGAACATG TGAGCAAAAG
2881 GCCAGCAAAA GGCCAGGAAC CGTAAAAAGG CCGCGTTGCT GGCGTTTTTC CATAGGCTCC
2941 GCCCCCCTGA CGAGCATCAC AAAAATCGAC GCTCAAGTCA GAGGTGGCGA AACCCGACAG
3001 GACTATAAAG ATACCAGGCG TTTCCCCCTG GAAGCTCCCT CGTGCGCTCT CCTGTTCCGA
3061 CCCTGCCGCT TACCGGATAC CTGTCCGCCT TTCTCCCTTC GGGAAGCGTG GCGCTTTCTC
3121 ATAGCTCACG CTGTAGGTAT CTCAGTTCGG TGTAGGTCGT TCGCTCCAAG CTGGGCTGTG
3181 TGCACGAACC CCCCGTTCAG CCCGACCGCT GCGCCTTATC CGGTAACTAT CGTCTTGAGT
3241 CCAACCCGGT AAGACACGAC TTATCGCCAC TGGCAGCAGC CACTGGTAAC AGGATTAGCA
3301 GAGCGAGGTA TGTAGGCGGT GCTACAGAGT TCTTGAAGTG GTGGCCTAAC TACGGCTACA
3361 CTAGAAGGAC AGTATTTGGT ATCTGCGCTC TGCTGAAGCC AGTTACCTTC GGAAAAAGAG
3421 TTGGTAGCTC TTGATCCGGC AAACAAACCA CCGCTGGTAG CGGTGGTTTT TTTGTTTGCA
3481 AGCAGCAGAT TACGCGCAGA AAAAAAGGAT CTCAAGAAGA TCCTTTGATC TTTTCTACGG
3541 GGTCTGACGC TCAGTGGAAC GAAAACTCAC GTTAAGGGAT TTTGGTCATG AGATTATCAA
3601 AAAGGATCTT CACCTAGATC CTTTTAAATT AAAAATGAAG TTTTAAATCA ATCTAAAGTA
3661 TATATGAGTA AACTTGGTCT GACAGTTACC AATGCTTAAT CAGTGAGGCA CCTATCTCAG
3721 CGATCTGTCT ATTTCGTTCA TCCATAGTTG CCTGACTCCC CGTCGTGTAG ATAACTACGA
3781 TACGGGAGGG CTTACCATCT GGCCCCAGTG CTGCAATGAT ACCGCGAGAC CCACGCTCAC
3841 CGGCTCCAGA TTTATCAGCA ATAAACCAGC CAGCCGGAAG GGCCGAGCGC AGAAGTGGTC
3901 CTGCAACTTT ATCCGCCTCC ATCCAGTCTA TTAATTGTTG CCGGGAAGCT AGAGTAAGTA
3961 GTTCGCCAGT TAATAGTTTG CGCAACGTTG TTGCCATTGC TACAGGCATC GTGGTGTCAC
4021 GCTCGTCGTT TGGTATGGCT TCATTCAGCT CCGGTTCCCA ACGATCAAGG CGAGTTACAT
4081 GATCCCCCAT GTTGTGCAAA AAAGCGGTTA GCTCCTTCGG TCCTCCGATC GTTGTCAGAA
4141 GTAAGTTGGC CGCAGTGTTA TCACTCATGG TTATGGCAGC ACTGCATAAT TCTCTTACTG
4201 TCATGCCATC CGTAAGATGC TTTTCTGTGA CTGGTGAGTA CTCAACCAAG TCATTCTGAG
4261 AATAGTGTAT GCGGCGACCG AGTTGCTCTT GCCCGGCGTC AATACGGGAT AATACCGCGC
4321 CACATAGCAG AACTTTAAAA GTGCTCATCA TTGGAAAACG TTCTTCGGGG CGAAAACTCT
4381 CAAGGATCTT ACCGCTGTTG AGATCCAGTT CGATGTAACC CACTCGTGCA CCCAACTGAT
4441 CTTCAGCATC TTTTACTTTC ACCAGCGTTT CTGGGTGAGC AAAAACAGGA AGGCAAAATG
4501 CCGCAAAAAA GGGAATAAGG GCGACACGGA AATGTTGAAT ACTCATACTC TTCCTTTTTC
4561 AATATTATTG AAGCATTTAT CAGGGTTATT GTCTCATGAG CGGATACATA TTTGAATGTA
4621 TTTAGAAAAA TAAACAAATA GGGGTTCCGC GCACATTTCC CCGAAAAGTG CCACCTGACG
4681 TCTAAGAAAC CATTATTATC ATGACATTAA CCTATAAAAA TAGGCGTATC ACGAGGCCCT
4741 TTCGTC

The segment rhaR through Prha was taken from the E. coli chromosome using PCR added Hindlll and modified aligned Shine-Delgarno (SD) sequence with Pstl followed by Sall, Xbal, a stem-loop transcriptional stop sequence, and KpnI. The PCR product was cloned into pUC18 using Hindlll and Kpnl.

### SEQ ID NO.: 153

### pMPX-67 rhamnose-inducible expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

Stop rhaR
361 TTTCCCAGTC ACGACGTTGT AAAACGACGG CCAGTGCCAA GCTTAATTAA TCTTTCTGCG

HindIII

421 AATTGAGATG ACGCCACTGG CTGGGCGTCA TCCCGGTTTC CCGGGTAAAC ACCACCGAAA
481 AATAGTTACT ATCTTCAAAG CCACATTCGG TCGAAATATC ACTGATTAAC AGGCGGCTAT
541 GCTGGAGAAG ATATTGCGCA TGACACACTC TGACCTGTCG CAGATATTGA TTGATGGTCA
601 TTCCAGTCTG CTGGCGAAAT TGCTGACGCA AAACGCGCTC ACTGCACGAT GCCTCATCAC
661 AAAATTTATC CAGCGCAAAG GGACTTTTCA GGCTAGCCGC CAGCCGGGTA ATCAGCTTAT
721 CCAGCAACGT TTCGCTGGAT GTTGGCGGCA ACGAATCACT GGTGTAACGA TGGCGATTCA
781 GCAACATCAC CAACTGCCCG AACAGCAACT CAGCCATTTC GTTAGCAAAC GGCACATGCT
841 GACTACTTTC ATGCTCAAGC TGACCGATAA CCTGCCGCGC CTGCGCCATC CCCATGCTAC
901 CTAAGCGCCA GTGTGGTTGC CCTGCGCTGG CGTTAAATCC CGGAATCGCC CCCTGCCAGT
961 CAAGATTCAG CTTCAGACGC TCCGGGCAAT AAATAATATT CTGCAAAACC AGATCGTTAA
1021 CGGAAGCGTA GGAGTGTTTA TCGTCAGCAT GAATGTAAAA GAGATCGCCA CGGGTAATGC
1081 GATAAGGGCG ATCGTTGAGT ACATGCAGGC CATTACCGCG CCAGACAATC ACCAGCTCAC
1141 AAAAATCATG TGTATGTTCA GCAAAGACAT CTTGCGGATA ACGGTCAGCC ACAGCGACTG
1201 CCTGCTGGTC GCTGGCAAAA AAATCATCTT TGAGAAGTTT TAACTGATGC GCCACCGTGG
1261 CTACCTCGGC CAGAGAACGA AGTTGATTAT TCGCAATATG GCGTACAAAT ACGTTGAGAA

Stop rhaS Start rhaR
1321 GATTCGCGTT ATTGCAGAAA GCCATCCCGT CCCTGGCGAA TATCACGCGG TGACCAGTTA
<--

1381 AACTCTCGGC GAAAAAGCGT CGAAAAGTGG TTACTGTCGC TGAATCCACA GCGATAGGCG
1441 ATGTCAGTAA CGCTGGCCTC GCTGTGGCGT AGCAGATGTC GGGCTTTCAT CAGTCGCAGG
1501 CGGTTCAGGT ATCGCTGAGG CGTCAGTCCC GTTTGCTGCT TAAGCTGCCG ATGTAGCGTA
1561 CGCAGTGAAA GAGAAAATTG ATCCGCCACG GCATCCCAAT TCACCTCATC GGCAAAATGG
1621 TCCTCCAGCC AGGCCAGAAG CAAGTTGAGA CGTGATGCGC TGTTTTCCAG GTTCTCCTGC
1681 AAACTGCTTT TACGCAGCAA GAGCAGTAAT TGCATAAACA AGATCTCGCG ACTGGCGGTC
1741 GAGGGTAAAT CATTTTCCCC TTCCTGCTGT TCCATCTGTG CAACCAGCTG TCGCACCTGC
1801 TGCAATACGC TGTGGTTAAC GCGCCAGTGA GACGGATACT GCCCATCCAG CTCTTGTGGC
1861 AGCAACTGAT TCAGCCCGGC GAGAAACTGA AATCGATCCG GCGAGCGATA CAGCACATTG
1921 GTCAGACACA GATTATCGGT ATGTTCATAC AGATGCCGAT CATGATCGCG TACGAAACAG
1981 ACCGTGCCAC CGGTGATGGT ATAGGGCTGC CCATTAAACA CATGAATACC CGTGCCATGT
2041 TCGACAATCA CAATTTCATG AAAATCATGA TGATGTTCAG GAAAATCCGC CTGCGGGAGC
2101 CGGGGTTCTA TCGCCACGGA CGCGTTACCA GACGGAAAAA AATCCACACT ATGTAATACG

Start rhaS
2161 GTCATACTGG CCTCCTGATG TCGTCAACAC GGCGAAATAG TAATCACGAG GTCAGGTTCT
<--

2221 TACCTTAAAT TTTCGACGGA AAACCACGTA AAAAACGTCG ATTTTTCAAG ATACAGCGTG
2281 AATTTTCAGG AAATGCGGTG AGCATCACAT CACCACAATT CAGCAAATTG TGAACATCAT
2341 CACGTTCATC TTTCCCTGGT TGCCAATGGC CCATTTTCCT GTCAGTAACG AGAAGGTCGC

SD SalI XbaI
2401 GAATTCAGGC GCTTTTTAGA CTGGTCGTAA TGAAATTCAG GAGGTTGTCG ACTCTAGAGG

Stem-loop KpnI
2461 ATCCCCGCGC CCTCATCCGA AAGGGCGTAT TGGTACCGAG CTCGAATTCG TAATCATGGT

2521 CATAGCTGTT TCCTGTGTGA AATTGTTATC CGCTCACAAT TCCACACAAC ATACGAGCCG
2581 GAAGCATAAA GTGTAAAGCC TGGGGTGCCT AATGAGTGAG CTAACTCACA TTAATTGCGT
2641 TGCGCTCACT GCCCGCTTTC CAGTCGGGAA ACCTGTCGTG CCAGCTGCAT TAATGAATCG
2701 GCCAACGCGC GGGGAGAGGC GGTTTGCGTA TTGGGCGCTC TTCCGCTTCC TCGCTCACTG
2761 ACTCGCTGCG CTCGGTCGTT CGGCTGCGGC GAGCGGTATC AGCTCACTCA AAGGCGGTAA
2821 TACGGTTATC CACAGAATCA GGGGATAACG CAGGAAAGAA CATGTGAGCA AAAGGCCAGC
2881 AAAAGGCCAG GAACCGTAAA AAGGCCGCGT TGCTGGCGTT TTTCCATAGG CTCCGCCCCC
2941 CTGACGAGCA TCACAAAAAT CGACGCTCAA GTCAGAGGTG GCGAAACCCG ACAGGACTAT

3001 AAAGATACCA GGCGTTTCCC CCTGGAAGCT CCCTCGTGCG CTCTCCTGTT CCGACCCTGC
3061 CGCTTACCGG ATACCTGTCC GCCTTTCTCC CTTCGGGAAG CGTGGCGCTT TCTCATAGCT
3121 CACGCTGTAG GTATCTCAGT TCGGTGTAGG TCGTTCGCTC CAAGCTGGGC TGTGTGCACG
3181 AACCCCCCGT TCAGCCCGAC CGCTGCGCCT TATCCGGTAA CTATCGTCTT GAGTCCAACC
3241 CGGTAAGACA CGACTTATCG CCACTGGCAG CAGCCACTGG TAACAGGATT AGCAGAGCGA
3301 GGTATGTAGG CGGTGCTACA GAGTTCTTGA AGTGGTGGCC TAACTACGGC TACACTAGAA
3361 GGACAGTATT TGGTATCTGC GCTCTGCTGA AGCCAGTTAC CTTCGGAAAA AGAGTTGGTA
3421 GCTCTTGATC CGGCAAACAA ACCACCGCTG GTAGCGGTGG TTTTTTTGTT TGCAAGCAGC
3481 AGATTACGCG CAGAAAAAAA GGATCTCAAG AAGATCCTTT GATCTTTTCT ACGGGGTCTG
3541 ACGCTCAGTG GAACGAAAAC TCACGTTAAG GGATTTTGGT CATGAGATTA TCAAAAAGGA
3601 TCTTCACCTA GATCCTTTTA AATTAAAAAT GAAGTTTTAA ATCAATCTAA AGTATATATG
3661 AGTAAACTTG GTCTGACAGT TACCAATGCT TAATCAGTGA GGCACCTATC TCAGCGATCT
3721 GTCTATTTCG TTCATCCATA GTTGCCTGAC TCCCCGTCGT GTAGATAACT ACGATACGGG
3781 AGGGCTTACC ATCTGGCCCC AGTGCTGCAA TGATACCGCG AGACCCACGC TCACCGGCTC
3841 CAGATTTATC AGCAATAAAC CAGCCAGCCG GAAGGGCCGA GCGCAGAAGT GGTCCTGCAA
3901 CTTTATCCGC CTCCATCCAG TCTATTAATT GTTGCCGGGA AGCTAGAGTA AGTAGTTCGC
3961 CAGTTAATAG TTTGCGCAAC GTTGTTGCCA TTGCTACAGG CATCGTGGTG TCACGCTCGT
4021 CGTTTGGTAT GGCTTCATTC AGCTCCGGTT CCCAACGATC AAGGCGAGTT ACATGATCCC
4081 CCATGTTGTG CAAAAAAGCG GTTAGCTCCT TCGGTCCTCC GATCGTTGTC AGAAGTAAGT
4141 TGGCCGCAGT GTTATCACTC ATGGTTATGG CAGCACTGCA TAATTCTCTT ACTGTCATGC
4201 CATCCGTAAG ATGCTTTTCT GTGACTGGTG AGTACTCAAC CAAGTCATTC TGAGAATAGT
4261 GTATGCGGCG ACCGAGTTGC TCTTGCCCGG CGTCAATACG GGATAATACC GCGCCACATA
4321 GCAGAACTTT AAAAGTGCTC ATCATTGGAA AACGTTCTTC GGGGCGAAAA CTCTCAAGGA
4381 TCTTACCGCT GTTGAGATCC AGTTCGATGT AACCCACTCG TGCACCCAAC TGATCTTCAG
4441 CATCTTTTAC TTTCACCAGC GTTTCTGGGT GAGCAAAAAC AGGAAGGCAA AATGCCGCAA
4501 AAAAGGGAAT AAGGGCGACA CGGAAATGTT GAATACTCAT ACTCTTCCTT TTTCAATATT
4561 ATTGAAGCAT TTATCAGGGT TATTGTCTCA TGAGCGGATA CATATTTGAA TGTATTTAGA
4621 AAAATAAACA AATAGGGGTT CCGCGCACAT TTCCCCGAAA AGTGCCACCT GACGTCTAAG
4681 AAACCATTAT TATCATGACA TTAACCTATA AAAATAGGCG TATCACGAGG CCCTTTCGTC

The segment rhaR through Prha was taken from the E. coli chromosome using PCR added Hindlll and modified aligned Shine-Delgarno (SD) sequence with Sall followed by Xbal, a stem-loop transcriptional stop sequence, and Kpnl. The PCR product was cloned into pUC18 using Hindlll and Kpnl.

### SEQ ID NO.: 154

### pMPX-71 arabinose-inducible expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT
HindIII
361 TTTCCCAGTC ACGACGTTGT AAAACGACGG CCAGTGCCAA GCTTCAAGCC GTCAATTGTC
Stop araC
421 TGATTCGTTA CCAATTATGA CAACTTGACG GCTACATCAT TCACTTTTTC TTCACAACCG
481 GCACGGAACT CGCTCGGGCT GGCCCCGGTG CATTTTTTAA ATACCCGCGA GAAATAGAGT
541 TGATCGTCAA AACCAACATT GCGACCGACG GTGGCGATAG GCATCCGGGT GGTGCTCAAA
601 AGCAGCTTCG CCTGGCTGAT ACGTTGGTCC TCGCGCCAGC TTAAGACGCT AATCCCTAAC
661 TGCTGGCGGA AAAGATGTGA CAGACGCGAC GGCGACAAGC AAACATGCTG TGCGACGCTG
721 GCGATATCAA AATTGCTGTC TGCCAGGTGA TCGCTGATGT ACTGACAAGC CTCGCGTACC
781 CGATTATCCA TCGGTGGATG GAGCGACTCG TTAATCGCTT CCATGCGCCG CAGTAACAAT
841 TGCTCAAGCA GATTTATCGC CAGCAGCTCC GAATAGCGCC CTTCCCCTTG CCCGGCGTTA
901 ATGATTTGCC CAAACAGGTC GCTGAAATGC GGCTGGTGCG CTTCATCCGG GCGAAAGAAC
961 CCCGTATTGG CAAATATTGA CGGCCAGTTA AGCCATTCAT GCCAGTAGGC GCGCGGACGA

1021 AAGTAAACCC ACTGGTGATA CCATTCGCGA GCCTCCGGAT GACGACCGTA GTGATGAATC
1081 TCTCCTGGCG GGAACAGCAA AATATCACCC GGTCGGCAAA CAAATTCTCG TCCCTGATTT
1141 TTCACCACCC CCTGACCGCG AATGGTGAGA TTGAGAATAT AACCTTTCAT TCCCAGCGGT
1201 CGGTCGATAA AAAAATCGAG ATAACCGTTG GCCTCAATCG GCGTTAAACC CGCCACCAGA
1261 TGGGCATTAA ACGAGTATCC CGGCAGCAGG GGATCATTTT GCGCTTCAGC CATACTTTTC

Start araC
1321 ATACTCCCGC CATTCAGAGA AGAAACCAAT TGTCCATATT GCATCAGACA TTGCCGTCAC
<--

1381 TGCGTCTTTT ACTGGCTCTT CTCGCTAACC AAACCGGTAA CCCCGCTTAT TAAAAGCATT
1441 CTGTAACAAA GCGGGACCAA AGCCATGACA AAAACGCGTA ACAAAAGTGT CTATAATCAC
1501 GGCAGAAAAG TCCACATTGA TTATTTGCAC GGCGTCACAC TTTGCTATGC CATAGCATTT
1561 TTATCCATAA GATTAGCGGA TCCTACCTGA CGCTTTTTAT CGCAACTCTC TACTGTTTCT

SD PstI SalI XbaI
1621 CCATACCCGT TTTTTTGGGC TAGCAGGAGG CCCTGCAGGT CGACTCTAGA GGATCCCCGC

Stem-loop KpnI
1681 GCCCTCATCC GAAAGGGCGT ATTGGTACCG AGCTCGAATT CGTAATCATG GTCATAGCTG

1741 TTTCCTGTGT GAAATTGTTA TCCGCTCACA ATTCCACACA ACATACGAGC CGGAAGCATA
1801 AAGTGTAAAG CCTGGGGTGC CTAATGAGTG AGCTAACTCA CATTAATTGC GTTGCGCTCA
1861 CTGCCCGCTT TCCAGTCGGG AAACCTGTCG TGCCAGCTGC ATTAATGAAT CGGCCAACGC
1921 GCGGGGAGAG GCGGTTTGCG TATTGGGCGC TCTTCCGCTT CCTCGCTCAC TGACTCGCTG
1981 CGCTCGGTCG TTCGGCTGCG GCGAGCGGTA TCAGCTCACT CAAAGGCGGT AATACGGTTA
2041 TCCACAGAAT CAGGGGATAA CGCAGGAAAG AACATGTGAG CAAAAGGCCA GCAAAAGGCC
2101 AGGAACCGTA AAAAGGCCGC GTTGCTGGCG TTTTTCCATA GGCTCCGCCC CCCTGACGAG
2161 CATCACAAAA ATCGACGCTC AAGTCAGAGG TGGCGAAACC CGACAGGACT ATAAAGATAC
2221 CAGGCGTTTC CCCCTGGAAG CTCCCTCGTG CGCTCTCCTG TTCCGACCCT GCCGCTTACC
2281 GGATACCTGT CCGCCTTTCT CCCTTCGGGA AGCGTGGCGC TTTCTCATAG CTCACGCTGT
2341 AGGTATCTCA GTTCGGTGTA GGTCGTTCGC TCCAAGCTGG GCTGTGTGCA CGAACCCCCC
2401 GTTCAGCCCG ACCGCTGCGC CTTATCCGGT AACTATCGTC TTGAGTCCAA CCCGGTAAGA
2461 CACGACTTAT CGCCACTGGC AGCAGCCACT GGTAACAGGA TTAGCAGAGC GAGGTATGTA
2521 GGCGGTGCTA CAGAGTTCTT GAAGTGGTGG CCTAACTACG GCTACACTAG AAGGACAGTA
2581 TTTGGTATCT GCGCTCTGCT GAAGCCAGTT ACCTTCGGAA AAAGAGTTGG TAGCTCTTGA
2641 TCCGGCAAAC AAACCACCGC TGGTAGCGGT GGTTTTTTTG TTTGCAAGCA GCAGATTACG
2701 CGCAGAAAAA AAGGATCTCA AGAAGATCCT TTGATCTTTT CTACGGGGTC TGACGCTCAG
2761 TGGAACGAAA ACTCACGTTA AGGGATTTTG GTCATGAGAT TATCAAAAAG GATCTTCACC
2821 TAGATCCTTT TAAATTAAAA ATGAAGTTTT AAATCAATCT AAAGTATATA TGAGTAAACT
2881 TGGTCTGACA GTTACCAATG CTTAATCAGT GAGGCACCTA TCTCAGCGAT CTGTCTATTT
2941 CGTTCATCCA TAGTTGCCTG ACTCCCCGTC GTGTAGATAA CTACGATACG GGAGGGCTTA
3001 CCATCTGGCC CCAGTGCTGC AATGATACCG CGAGACCCAC GCTCACCGGC TCCAGATTTA
3061 TCAGCAATAA ACCAGCCAGC CGGAAGGGCC GAGCGCAGAA GTGGTCCTGC AACTTTATCC
3121 GCCTCCATCC AGTCTATTAA TTGTTGCCGG GAAGCTAGAG TAAGTAGTTC GCCAGTTAAT
3181 AGTTTGCGCA ACGTTGTTGC CATTGCTACA GGCATCGTGG TGTCACGCTC GTCGTTTGGT
3241 ATGGCTTCAT TCAGCTCCGG TTCCCAACGA TCAAGGCGAG TTACATGATC CCCCATGTTG
3301 TGCAAAAAAG CGGTTAGCTC CTTCGGTCCT CCGATCGTTG TCAGAAGTAA GTTGGCCGCA
3361 GTGTTATCAC TCATGGTTAT GGCAGCACTG CATAATTCTC TTACTGTCAT GCCATCCGTA
3421 AGATGCTTTT CTGTGACTGG TGAGTACTCA ACCAAGTCAT TCTGAGAATA GTGTATGCGG
3481 CGACCGAGTT GCTCTTGCCC GGCGTCAATA CGGGATAATA CCGCGCCACA TAGCAGAACT
3541 TTAAAAGTGC TCATCATTGG AAAACGTTCT TCGGGGCGAA AACTCTCAAG GATCTTACCG
3601 CTGTTGAGAT CCAGTTCGAT GTAACCCACT CGTGCACCCA ACTGATCTTC AGCATCTTTT
3661 ACTTTCACCA GCGTTTCTGG GTGAGCAAAA ACAGGAAGGC AAAATGCCGC AAAAAAGGGA
3721 ATAAGGGCGA CACGGAAATG TTGAATACTC ATACTCTTCC TTTTTCAATA TTATTGAAGC
3781 ATTTATCAGG GTTATTGTCT CATGAGCGGA TACATATTTG AATGTATTTA GAAAAATAAA
3841 CAAATAGGGG TTCCGCGCAC ATTTCCCCGA AAAGTGCCAC CTGACGTCTA AGAAACCATT
3901 ATTATCATGA CATTAACCTA TAAAAATAGG CGTATCACGA GGCCCTTTCG TC

The segment araC through Para was taken from pBAD24 using PCR added Hindlll and modified aligned Shine-Delgarno (SD) sequence with Pstl followed by Sall, Xbal, a stem-loop transcriptional stop sequence, and Kpnl. The PCR product was cloned into pUC18 using Hindlll and Kpnl.

### SEQ ID NO.: 155

### pMPX-68 melibiose-inducible expression vector

1 TCGCGCGTTT CGGTGATGAC GGTGAAAACC TCTGACACAT GCAGCTCCCG GAGACGGTCA
61 CAGCTTGTCT GTAAGCGGAT GCCGGGAGCA GACAAGCCCG TCAGGGCGCG TCAGCGGGTG
121 TTGGCGGGTG TCGGGGCTGG CTTAACTATG CGGCATCAGA GCAGATTGTA CTGAGAGTGC
181 ACCATATGCG GTGTGAAATA CCGCACAGAT GCGTAAGGAG AAAATACCGC ATCAGGCGCC
241 ATTCGCCATT CAGGCTGCGC AACTGTTGGG AAGGGCGATC GGTGCGGGCC TCTTCGCTAT
301 TACGCCAGCT GGCGAAAGGG GGATGTGCTG CAAGGCGATT AAGTTGGGTA ACGCCAGGGT

HindIII
361 TTTCCCAGTC ACGACGTTGT AAAACGACGG CCAGTGCCAA GCTTTTAGCC GGGAAACGTC
Stop MelR

421 TGGCGGCGCT GTTGGCTAAG TTTGCGGTAT TGTTGCGGCG ACATGCCGAC ATATTTGCCG
481 AACGTGCTGT AAAAACGACT ACTTGAACGA AAGCCTGCCG TCAGGGCAAT ATCGAGAATA
541 CTTTTATCGG TATCGCTCAG TAACGCGCGA ACGTGGTTGA TGCGCATCGC GGTAATGTAC
601 TGTTTCATCG TCAATTGCAT GACCCGCTGG AATATCCCCA TTGCATAGTT GGCGTTAAGT
661 TTGACGTGCT CAGCCACATC GTTGATGGTC AGCGCCTGAT CATAGTTTTC GGCAATAAAG
721 CCCAGCATCT GGCTAACATA AAATTGCGCA TGGCGCGAGA CGCTGTTTTT GTGTGTGCGC
781 GAGGTTTTAT TGACCAGAAT CGGTTCCCAG CCAGAGAGGC TAAATCGCTT GAGCATCAGG
841 CCAATTTCAT CAATGGCGAG CTGGCGAATT TGCTCGTTCG GACTGTTTAA TTCCTGCTGC
901 CAGCGGCGCA CTTCAAACGG GCTAAGTTGC TGTGTGGCCA GTGATTTGAT CACCATGCCG
961 TGAGTGACGT GGTTAATCAG GTCTTTATCC AGCGGCCAGG AGAGAAACAG ATGCATCGGC
1021 AGATTAAAAA TCGCCATGCT CTGACAGGTT CCGGTATCTG TTAGTTGGTG CGGTGTACAG
1081 GCCCAGAACA GCGTGATATG ACCCTGATTG ATATTCACTT TTTCATTGTT GATCAGGTAT
1141 TCCACATCGC CATCGAAAGG CACATTCACT TCGACCTGAC CATGCCAGTG GCTGGTGGGC
1201 ATGATATGCG GTGCGCGAAA CTCAATCTCC ATCCGCTGGT ATTCCGAATA CAGCGACAGC

+1 MelR
1261 GGGCTGCGGG TCTGTTTTTC GTCGCTGCTG CACATAAACG TATCTGTATT CATGGATGGC

1321 TCTCTTTCCT GGAATATCAG AATTATGGCA GGAGTGAGGG AGGATGACTG CGAGTGGGAG
1381 CACGGTTTTC ACCCTCTTCC CAGAGGGGCG AGGGGACTCT CCGAGTATCA TGAGGCCGAA
1441 AACTCTGCTT TTCAGGTAAT TTATTCCCAT AAACTCAGAT TTACTGCTGC TTCACGCAGG
1501 ATCTGAGTTT ATGGGAATGC TCAACCTGGA AGCCGGAGGT TTTCTGCAGA TTCGCCTGCC

SD SalI XbaI
1561 ATGATGAAGT TATTCAAGCA AGCCAGGAGG TCGTCGACTC TAGAGGATCC CCGCGCCCTC

Stem-loop KpnI
1621 ATCCGAAAGG GCGTATTGGT ACCGAGCTCG AATTCGTAAT CATGGTCATA GCTGTTTCCT

1681 GTGTGAAATT GTTATCCGCT CACAATTCCA CACAACATAC GAGCCGGAAG CATAAAGTGT
1741 AAAGCCTGGG GTGCCTAATG AGTGAGCTAA CTCACATTAA TTGCGTTGCG CTCACTGCCC
1801 GCTTTCCAGT CGGGAAACCT GTCGTGCCAG CTGCATTAAT GAATCGGCCA ACGCGCGGGG
1861 AGAGGCGGTT TGCGTATTGG GCGCTCTTCC GCTTCCTCGC TCACTGACTC GCTGCGCTCG
1921 GTCGTTCGGC TGCGGCGAGC GGTATCAGCT CACTCAAAGG CGGTAATACG GTTATCCACA
1981 GAATCAGGGG ATAACGCAGG AAAGAACATG TGAGCAAAAG GCCAGCAAAA GGCCAGGAAC
2041 CGTAAAAAGG CCGCGTTGCT GGCGTTTTTC CATAGGCTCC GCCCCCCTGA CGAGCATCAC
2101 AAAAATCGAC GCTCAAGTCA GAGGTGGCGA AACCCGACAG GACTATAAAG ATACCAGGCG

2161 TTTCCCCCTG GAAGCTCCCT CGTGCGCTCT CCTGTTCCGA CCCTGCCGCT TACCGGATAC
2221 CTGTCCGCCT TTCTCCCTTC GGGAAGCGTG GCGCTTTCTC ATAGCTCACG CTGTAGGTAT
2281 CTCAGTTCGG TGTAGGTCGT TCGCTCCAAG CTGGGCTGTG TGCACGAACC CCCCGTTCAG
2341 CCCGACCGCT GCGCCTTATC CGGTAACTAT CGTCTTGAGT CCAACCCGGT AAGACACGAC
2401 TTATCGCCAC TGGCAGCAGC CACTGGTAAC AGGATTAGCA GAGCGAGGTA TGTAGGCGGT
2461 GCTACAGAGT TCTTGAAGTG GTGGCCTAAC TACGGCTACA CTAGAAGGAC AGTATTTGGT
2521 ATCTGCGCTC TGCTGAAGCC AGTTACCTTC GGAAAAAGAG TTGGTAGCTC TTGATCCGGC
2581 AAACAAACCA CCGCTGGTAG CGGTGGTTTT TTTGTTTGCA AGCAGCAGAT TACGCGCAGA
2641 AAAAAAGGAT CTCAAGAAGA TCCTTTGATC TTTTCTACGG GGTCTGACGC TCAGTGGAAC
2701 GAAAACTCAC GTTAAGGGAT TTTGGTCATG AGATTATCAA AAAGGATCTT CACCTAGATC
2761 CTTTTAAATT AAAAATGAAG TTTTAAATCA ATCTAAAGTA TATATGAGTA AACTTGGTCT
2821 GACAGTTACC AATGCTTAAT CAGTGAGGCA CCTATCTCAG CGATCTGTCT ATTTCGTTCA
2881 TCCATAGTTG CCTGACTCCC CGTCGTGTAG ATAACTACGA TACGGGAGGG CTTACCATCT
2941 GGCCCCAGTG CTGCAATGAT ACCGCGAGAC CCACGCTCAC CGGCTCCAGA TTTATCAGCA
3001 ATAAACCAGC CAGCCGGAAG GGCCGAGCGC AGAAGTGGTC CTGCAACTTT ATCCGCCTCC
3061 ATCCAGTCTA TTAATTGTTG CCGGGAAGCT AGAGTAAGTA GTTCGCCAGT TAATAGTTTG
3121 CGCAACGTTG TTGCCATTGC TACAGGCATC GTGGTGTCAC GCTCGTCGTT TGGTATGGCT
3181 TCATTCAGCT CCGGTTCCCA ACGATCAAGG CGAGTTACAT GATCCCCCAT GTTGTGCAAA
3241 AAAGCGGTTA GCTCCTTCGG TCCTCCGATC GTTGTCAGAA GTAAGTTGGC CGCAGTGTTA
3301 TCACTCATGG TTATGGCAGC ACTGCATAAT TCTCTTACTG TCATGCCATC CGTAAGATGC
3361 TTTTCTGTGA CTGGTGAGTA CTCAACCAAG TCATTCTGAG AATAGTGTAT GCGGCGACCG
3421 AGTTGCTCTT GCCCGGCGTC AATACGGGAT AATACCGCGC CACATAGCAG AACTTTAAAA
3481 GTGCTCATCA TTGGAAAACG TTCTTCGGGG CGAAAACTCT CAAGGATCTT ACCGCTGTTG
3541 AGATCCAGTT CGATGTAACC CACTCGTGCA CCCAACTGAT CTTCAGCATC TTTTACTTTC
3601 ACCAGCGTTT CTGGGTGAGC AAAAACAGGA AGGCAAAATG CCGCAAAAAA GGGAATAAGG
3661 GCGACACGGA AATGTTGAAT ACTCATACTC TTCCTTTTTC AATATTATTG AAGCATTTAT
3721 CAGGGTTATT GTCTCATGAG CGGATACATA TTTGAATGTA TTTAGAAAAA TAAACAAATA
3781 GGGGTTCCGC GCACATTTCC CCGAAAAGTG CCACCTGACG TCTAAGAAAC CATTATTATC
3841 ATGACATTAA CCTATAAAAA TAGGCGTATC ACGAGGCCCT TTCGTC

### SEO ID NO.: 166

### MalE (1-370) Factor Xa NTR (43-424) FLAG

Sall +1 MalE (1-370)
1 GTCGACATGAAAATAAAAACAGGTGCACGCATCCTCGCATTATCCGCATTAACGACGATGATGTTT
1 M K I K T G A R I L A L S A L T T M M F

61 TCCGCCTCGGCTCTCGCCAAAATCGAAGAAGGTAAACTGGTAATCTGGATTAACGGCGAT
21 S A S A L A K I E E G K L V I W I N G D

121 AAAGGCTATAACGGTCTCGCTGAAGTCGGTAAGAAATTCGAGAAAGATACCGGAATTAAA
41 K G Y N G L A E V G K K F E K D T G I K

181 GTCACCGTTGAGCATCCGGATAAACTGGAAGAGAAATTCCCACAGGTTGCGGCAACTGGC
61 V T V E H P D K L E E K F P Q V A A T G

241 GATGGCCCTGACATTATCTTCTGGGCACACGACCGCTTTGGTGGCTACGCTCAATCTGGC
81 D G P D I I F W A H D R F G G Y A Q S G

301 CTGTTGGCTGAAATCACCCCGGACAAAGCGTTCCAGGACAAGCTGTATCCGTTTACCTGG
101 L L A E I T P D K A F Q D K L Y P F T W

361 GATGCCGTACGTTACAACGGCAAGCTGATTGCTTACCCGATCGCTGTTGAAGCGTTATCG
121 D A V R Y N G K L I A Y P I A V E A L S

421 CTGATTTATAACAAAGATCTGCTGCCGAACCCGCCAAAAACCTGGGAAGAGATCCCGGCG
141 L I Y N K D L L P N P P K T W E E I P A

481 CTGGATAAAGAACTGAAAGCGAAAGGTAAGAGCGCGCTGATGTTCAACCTGCAAGAACCG
161 L D K E L K A K G K S A L M F N L Q E P

541 TACTTCACCTGGCCGCTGATTGCTGCTGACGGGGGTTATGCGTTCAAGTATGAAAACGGC
181 Y F T W P L I A A D G G Y A F K Y E N G

601 AAGTACGACATTAAAGACGTGGGCGTGGATAACGCTGGCGCGAAAGCGGGTCTGACCTTC
201 K Y D I K D V G V D N A G A K A G L T F

661 CTGGTTGACCTGATTAAAAACAAACACATGAATGCAGACACCGATTACTCCATCGCAGAA
221 L V D L I K N K H M N A D T D Y S I A E

721 GCTGCCTTTAATAAAGGCGAAACAGCGATGACCATCAACGGCCCGTGGGCATGGTCCAAC
241 A A F N K G E T A M T I N G P W A W S N

781 ATCGACACCAGCAAAGTGAATTATGGTGTAACGGTACTGCCGACCTTCAAGGGTCAACCA
261 I D T S K V N Y G V T V L P T F K G Q P

841 TCCAAACCGTTCGTTGGCGTGCTGAGCGCAGGTATTAACGCCGCCAGTCCGAACAAAGAG
281 S K P F V G V L S A G I N A A S P N K E

901 CTGGCGAAAGAGTTCCTCGAAAACTATCTGCTGACTGATGAAGGTCTGGAAGCGGTTAAT
301 L A K E F L E N Y L L T D E G L E A V N

961 AAAGACAAACCGCTGGGTGCCGTAGCGCTGAAGTCTTACGAGGAAGAGTTGGCGAAAGAT
321 K D K P L G A V A L K S Y E E E L A K D

1021 CCACGTATTGCCGCCACCATGGAAAACGCCCAGAAAGGTGAAATCATGCCGAACATCCCG
341 P R I A A T M E N A Q K G E I M P N I P

Factor Xa +43 NTR
1081 CAGATGTCCGCTTTCTGGTATGCCGTGCTGATCGAAGCCCGCACCTCGGAATCCGACACG
361 Q M S A F W Y A V L I E A R T S E S D T

1141 GCAGGGCCCAACAGCGACCTGGACGTGAACACTGACATTTATTCCAAGGTGCTGGTGACT
381 A G P N S D L D V N T D I Y S K V L V T

1201 GCTATATACCTGGCACTCTTCGTGGTGGGCACTGTGGGCAACTCCGTGACAGCCTTCACT
401 A I Y L A L F V V G T V G N S V T A F T

1261 CTAGCGCGGAAGAAGTCACTGCAGAGCCTGCAGAGCACTGTGCATTACCACCTGGGCAGC
421 L A R K K S L Q S L Q S T V H Y H L G S

1321 CTGGCACTGTCGGACCTGCTTATCCTTCTGCTGGCCATGCCCGTGGAGCTATACAACTTC
441 L A L S D L L I L L L A M P V E L Y N F

1381 ATCTGGGTACACCATCCCTGGGCCTTTGGGGACGCTGGCTGCCGTGGCTACTATTTCCTG
461 I W V H H P W A F G D A G C R G Y Y F L

1441 CGTGATGCCTGCACCTATGCCACAGCCCTCAATGTAGCCAGCCTGAGTGTGGAGCGCTAC
481 R D A C T Y A T A L N V A S L S V E R Y

1501 TTGGCCATCTGCCATCCCTTCAAGGCCAAGACCCTCATGTCCCGCAGCCGCACCAAGAAA
501 L A I C H P F K A K T L M S R S R T K K

1561 TTCATCAGTGCCATATGGCTAGCTTCGGCGCTGCTGGCTATACCCATGCTTTTCACCATG
521 F I S A I W L A S A L L A I P M L F T M

1621 GGCCTGCAGAACCGCAGTGGTGACGGCACGCACCCTGGCGGCCTGGTGTGCACACCCATT
541 G L Q N R S G D G T H P G G L V C T P I

1681 GTGGACACAGCCACTGTCAAGGTCGTCATCCAGGTTAACACCTTCATGTCCTTCCTGTTT
561 V D T A T V K V V I Q V N T F M S F L F

1741 CCCATGTTGGTCATCTCCATCCTAAACACCGTGATTGCCAACAAACTGACAGTCATGGTG
581 P M L V I S I L N T V I A N K L T V M V

1801 CACCAGGCCGCCGAGCAGGGCCGAGTGTGCACCGTGGGCACACACAACGGTTTAGAGCAC
601 H Q A A E Q G R V C T V G T H N G L E H

1861 AGCACGTTCAACATGACCATCGAGCCGGGTCGTGTCCAGGCCCTGCGCCACGGAGTCCTC
621 S T F N M T I E P G R V Q A L R H G V L

1921 GTCTTACGTGCTGTGGTCATTGCCTTTGTGGTCTGCTGGCTGCCCTACCACGTGCGACGC
641 V L R A V V I A F V V C W L P Y H V R R

1981 CTGATGTTCTGCTATATCTCGGATGAACAGTGGACTACGTTCCTCTTCGATTTCTACCAC
661 L M F C Y I S D E Q W T T F L F D F Y H

2041 TATTTCTACATGCTAACCAACGCTCTCTTCTACGTCAGCTCCGCCATCAATCCCATCCTC
681 Y F Y M L T N A L F Y V S S A I N P I L

2101 TACAACCTGGTCTCCGCCAACTTCCGCCAGGTCTTTCTGTCCACGCTGGCCTGCCTTTGT
701 Y N L V S A N F R Q V F L S T L A C L C

2161 CCTGGGTGGCGCCACCGCCGAAAGAAGAGGCCAACGTTCTCCAGGAAGCCCAACAGCATG
721 P G W R H R R K K R P T F S R K P N S M

NotI
2221 TCCAGCAACCATGCCTTTTCCACCAGCGCCACCCGGGAGACCCTGTACgcggccgca
741 S S N H A F S T S A T R E T L Y A A A

Flag stop KpnI
GATTATAAAGATGACGATGACAAATAATAAGGTACC
D Y K D D D D K * *

### SEQ ID NO.: 167

### MalE (1-28) Factor Xa NTR (43-424) FLAG

Sall +1 MalE leader (1-28)
1 gtcgacATGAAAATAAAAACAGGTGCACGCATCCTCGCATTATCCGCATTAACGACGATGATGTTT
1 M K I K T G A R I L A L S A L T T M M F

Factor Xa +43 NTR
61 TCCGCCTCGGCTCTCGCCAAAATCATCGAAGCCCGCACCTCGGAATCCGACACGGCAGGG
21 S A S A L A K I I E A R T S E S D T A G

121 CCCAACAGCGACCTGGACGTGAACACTGACATTTATTCCAAGGTGCTGGTGACTGCTATA
41 P N S D L D V N T D I Y S K V L V T A I

181 TACCTGGCACTCTTCGTGGTGGGCACTGTGGGCAACTCCGTGACAGCCTTCACTCTAGCG
61 Y L A L F V V G T V G N S V T A F T L A

241 CGGAAGAAGTCACTGCAGAGCCTGCAGAGCACTGTGCATTACCACCTGGGCAGCCTGGCA

81 R K K S L Q S L Q S T V H Y H L G S L A

301 CTGTCGGACCTGCTTATCCTTCTGCTGGCCATGCCCGTGGAGCTATACAACTTCATCTGG
101 L S D L L I L L L A M P V E L Y N F I W

361 GTACACCATCCCTGGGCCTTTGGGGACGCTGGCTGCCGTGGCTACTATTTCCTGCGTGAT
121 V H H P W A F G D A G C R G Y Y F L R D

421 GCCTGCACCTATGCCACAGCCCTCAATGTAGCCAGCCTGAGTGTGGAGCGCTACTTGGCC
141 A C T Y A T A L N V A S L S V E R Y L A

481 ATCTGCCATCCCTTCAAGGCCAAGACCCTCATGTCCCGCAGCCGCACCAAGAAATTCATC
161 I C H P F K A K T L M S R S R T K K F I

541 AGTGCCATATGGCTAGCTTCGGCGCTGCTGGCTATACCCATGCTTTTCACCATGGGCCTG
181 S A I W L A S A L L A I P M L F T M G L

601 CAGAACCGCAGTGGTGACGGCACGCACCCTGGCGGCCTGGTGTGCACACCCATTGTGGAC
201 Q N R S G D G T H P G G L V C T P I V D

661 ACAGCCACTGTCAAGGTCGTCATCCAGGTTAACACCTTCATGTCCTTCCTGTTTCCCATG
221 T A T V K V V I Q V N T F M S F L F P M

721 TTGGTCATCTCCATCCTAAACACCGTGATTGCCAACAAACTGACAGTCATGGTGCACCAG
241 L V I S I L N T V I A N K L T V M V H Q

781 GCCGCCGAGCAGGGCCGAGTGTGCACCGTGGGCACACACAACGGTTTAGAGCACAGCACG
261 A A E Q G R V C T V G T H N G L E H S T

841 TTCAACATGACCATCGAGCCGGGTCGTGTCCAGGCCCTGCGCCACGGAGTCCTCGTCTTA
281 F N M T I E P G R V Q A L R H G V L V L

901 CGTGCTGTGGTCATTGCCTTTGTGGTCTGCTGGCTGCCCTACCACGTGCGACGCCTGATG
301 R A V V I A F V V C W L P Y H V R R L M

961 TTCTGCTATATCTCGGATGAACAGTGGACTACGTTCCTCTTCGATTTCTACCACTATTTC
321 F C Y I S D E Q W T T F L F D F Y H Y F

1021 TACATGCTAACCAACGCTCTCTTCTACGTCAGCTCCGCCATCAATCCCATCCTCTACAAC
341 Y M L T N A L F Y V S S A I N P I L Y N

1081 CTGGTCTCCGCCAACTTCCGCCAGGTCTTTCTGTCCACGCTGGCCTGCCTTTGTCCTGGG
361 L V S A N F R Q V F L S T L A C L C P G

1141 TGGCGCCACCGCCGAAAGAAGAGGCCAACGTTCTCCAGGAAGCCCAACAGCATGTCCAGC
381 W R H R R K K R P T F S R K P N S M S S

NotI Flag
1201 AACCATGCCTTTTCCACCAGCGCCACCCGGGAGACCCTGTACgcggccgcaGATTATAAA
401 N H A F S T S A T R E T L Y A A A D Y K

stop KpnI
GATGACGATGACAAATAATAAGGTACC
D D D D K

### SEQ ID NO.: 169

### MalE (1-370) Factor Xa NTR (43-424) TrxA (2-109) FLAG

Sall +1 MalE (1-370)
1 GTCGACATGAAAATAAAAACAGGTGCACGCATCCTCGCATTATCCGCATTAACGACGATGATGTTT
1 M K I K T G A R I L A L S A L T T M M F

61 TCCGCCTCGGCTCTCGCCAAAATCGAAGAAGGTAAACTGGTAATCTGGATTAACGGCGAT
21 S A S A L A K I E E G K L V I W I N G D

121 AAAGGCTATAACGGTCTCGCTGAAGTCGGTAAGAAATTCGAGAAAGATACCGGAATTAAA
41 K G Y N G L A E V G K K F E K D T G I K

181 GTCACCGTTGAGCATCCGGATAAACTGGAAGAGAAATTCCCACAGGTTGCGGCAACTGGC
61 V T V E H P D K L E E K F P Q V A A T G

241 GATGGCCCTGACATTATCTTCTGGGCACACGACCGCTTTGGTGGCTACGCTCAATCTGGC
81 D G P D I I F W A H D R F G G Y A Q S G

301 CTGTTGGCTGAAATCACCCCGGACAAAGCGTTCCAGGACAAGCTGTATCCGTTTACCTGG
101 L L A E I T P D K A F Q D K L Y P F T W

361 GATGCCGTACGTTACAACGGCAAGCTGATTGCTTACCCGATCGCTGTTGAAGCGTTATCG
121 D A V R Y N G K L I A Y P I A V E A L S

421 CTGATTTATAACAAAGATCTGCTGCCGAACCCGCCAAAAACCTGGGAAGAGATCCCGGCG
141 L I Y N K D L L P N P P K T W E E I P A

481 CTGGATAAAGAACTGAAAGCGAAAGGTAAGAGCGCGCTGATGTTCAACCTGCAAGAACCG
161 L D K E L K A K G K S A L M F N L Q E P

541 TACTTCACCTGGCCGCTGATTGCTGCTGACGGGGGTTATGCGTTCAAGTATGAAAACGGC
181 Y F T W P L I A A D G G Y A F K Y E N G

601 AAGTACGACATTAAAGACGTGGGCGTGGATAACGCTGGCGCGAAAGCGGGTCTGACCTTC
201 K Y D I K D V G V D N A G A K A G L T F

661 CTGGTTGACCTGATTAAAAACAAACACATGAATGCAGACACCGATTACTCCATCGCAGAA
221 L V D L I K N K H M N A D T D Y S I A E

721 GCTGCCTTTAATAAAGGCGAAACAGCGATGACCATCAACGGCCCGTGGGCATGGTCCAAC
241 A A F N K G E T A M T I N G P W A W S N

781 ATCGACACCAGCAAAGTGAATTATGGTGTAACGGTACTGCCGACCTTCAAGGGTCAACCA
261 I D T S K V N Y G V T V L P T F K G Q P

841 TCCAAACCGTTCGTTGGCGTGCTGAGCGCAGGTATTAACGCCGCCAGTCCGAACAAAGAG
281 S K P F V G V L S A G I N A A S P N K E

901 CTGGCGAAAGAGTTCCTCGAAAACTATCTGCTGACTGATGAAGGTCTGGAAGCGGTTAAT
301 L A K E F L E N Y L L T D E G L E A V N

961 AAAGACAAACCGCTGGGTGCCGTAGCGCTGAAGTCTTACGAGGAAGAGTTGGCGAAAGAT
321 K D K P L G A V A L K S Y E E E L A K D

1021 CCACGTATTGCCGCCACCATGGAAAACGCCCAGAAAGGTGAAATCATGCCGAACATCCCG
341 P R I A A T M E N A Q K G E I M P N I P

Factor Xa +43 NTR
1081 CAGATGTCCGCTTTCTGGTATGCCGTGCTGATCGAAGCCCGCACCTCGGAATCCGACACG
361 Q M S A F W Y A V L I E A R T S E S D T

1141 GCAGGGCCCAACAGCGACCTGGACGTGAACACTGACATTTATTCCAAGGTGCTGGTGACT
381 A G P N S D L D V N T D I Y S K V L V T

1201 GCTATATACCTGGCACTCTTCGTGGTGGGCACTGTGGGCAACTCCGTGACAGCCTTCACT
401 A I Y L A L F V V G T V G N S V T A F T

1261 CTAGCGCGGAAGAAGTCACTGCAGAGCCTGCAGAGCACTGTGCATTACCACCTGGGCAGC
421 L A R K K S L Q S L Q S T V H Y H L G S

1321 CTGGCACTGTCGGACCTGCTTATCCTTCTGCTGGCCATGCCCGTGGAGCTATACAACTTC
441 L A L S D L L I L L L A M P V E L Y N F

1381 ATCTGGGTACACCATCCCTGGGCCTTTGGGGACGCTGGCTGCCGTGGCTACTATTTCCTG
461 I W V H H P W A F G D A G C R G Y Y F L

1441 CGTGATGCCTGCACCTATGCCACAGCCCTCAATGTAGCCAGCCTGAGTGTGGAGCGCTAC
481 R D A C T Y A T A L N V A S L S V E R Y

1501 TTGGCCATCTGCCATCCCTTCAAGGCCAAGACCCTCATGTCCCGCAGCCGCACCAAGAAA
501 L A I C H P F K A K T L M S R S R T K K

1561 TTCATCAGTGCCATATGGCTAGCTTCGGCGCTGCTGGCTATACCCATGCTTTTCACCATG
521 F I S A I W L A S A L L A I P M L F T M

1621 GGCCTGCAGAACCGCAGTGGTGACGGCACGCACCCTGGCGGCCTGGTGTGCACACCCATT
541 G L Q N R S G D G T H P G G L V C T P I

1681 GTGGACACAGCCACTGTCAAGGTCGTCATCCAGGTTAACACCTTCATGTCCTTCCTGTTT
561 V D T A T V K V V I Q V N T F M S F L F

1741 CCCATGTTGGTCATCTCCATCCTAAACACCGTGATTGCCAACAAACTGACAGTCATGGTG
581 P M L V I S I L N T V I A N K L T V M V

1801 CACCAGGCCGCCGAGCAGGGCCGAGTGTGCACCGTGGGCACACACAACGGTTTAGAGCAC
601 H Q A A E Q G R V C T V G T H N G L E H

1861 AGCACGTTCAACATGACCATCGAGCCGGGTCGTGTCCAGGCCCTGCGCCACGGAGTCCTC
621 S T F N M T I E P G R V Q A L R H G V L

1921 GTCTTACGTGCTGTGGTCATTGCCTTTGTGGTCTGCTGGCTGCCCTACCACGTGCGACGC
641 V L R A V V I A F V V C W L P Y H V R R

1981 CTGATGTTCTGCTATATCTCGGATGAACAGTGGACTACGTTCCTCTTCGATTTCTACCAC
661 L M F C Y I S D E Q W T T F L F D F Y H

2041 TATTTCTACATGCTAACCAACGCTCTCTTCTACGTCAGCTCCGCCATCAATCCCATCCTC
681 Y F Y M L T N A L F Y V S S A I N P I L

2101 TACAACCTGGTCTCCGCCAACTTCCGCCAGGTCTTTCTGTCCACGCTGGCCTGCCTTTGT
701 Y N L V S A N F R Q V F L S T L A C L C

2161 CCTGGGTGGCGCCACCGCCGAAAGAAGAGGCCAACGTTCTCCAGGAAGCCCAACAGCATG
721 P G W R H R R K K R P T F S R K P N S M

NotI +2 TrxA
2221 TCCAGCAACCATGCCTTTTCCACCAGCGCCACCCGGGAGACCCTGTACgcggccgcaAGC
741 S S N H A F S T S A T R E T L Y A A A S

2281 GATAAAATTATTCACCTGACTGACGACAGTTTTGACACGGATGTACTCAAAGCGGACGGG
761 D K I I H L T D D S F D T D V L K A D G

2341 GCGATCCTCGTCGATTTCTGGGCAGAGTGGTGCGGTCCGTGCAAAATGATCGCCCCGATT
781 A I L V D F W A E W C G P C K M I A P I

2401 CTGGATGAAATCGCTGACGAATATCAGGGCAAACTGACCGTTGCAAAACTGAACATCGAT
801 L D E I A D E Y Q G K L T V A K L N I D

2461 CAAAACCCTGGCACTGCGCCGAAATATGGCATCCGTGGTATCCCGACTCTGCTGCTGTTC
821 Q N P G T A P K Y G I R G I P T L L L F

2521 AAAAACGGTGAAGTGGCGGCAACCAAAGTGGGTGCACTGTCTAAAGGTCAGTTGAAAGAG
841 K N G E V A A T K V G A L S K G Q L K E

NotI +2 Flag stop
2581 TTCCTCGACGCTAACCTGGCGgcggccgcaGATTATAAAGATGACGATGACAAATAATAA
861 F L D A N L A A A A D Y K D D D D K * *

KpnI
2641 GGTACC

### SEQ ID NO.: 170

### MalE (1-28) Factor Xa NTR (43-424) TrxA (2-109) FLAG

Sall  +1 MalE leader (1-28)
1  gtcgacATGAAAATAAAAACAGGTGCACGCATCCTCGCATTATCCGCATTAACGACGATGATGTTT
1       M  K  I  K  T  G  A  R  I  L  A  L  S  A  L  T  T  M  M  F

                                        Factor Xa  +43 NTR
61      TCCGCCTCGGCTCTCGCCAAAATCATCGAAGCCCGCACCTCGGAATCCGACACGGCAGGG
21      S  A  S  A  L  A  K  I  I  E  A  R  T  S  E  S  D  T  A  G

121     CCCAACAGCGACCTGGACGTGAACACTGACATTTATTCCAAGGTGCTGGTGACTGCTATA
41      P  N  S  D  L  D  V  N  T  D  I  Y  S  K  V  L  V  T  A  I

181     TACCTGGCACTCTTCGTGGTGGGCACTGTGGGCAACTCCGTGACAGCCTTCACTCTAGCG
61      Y  L  A  L  F  V  V  G  T  V  G  N  S  V  T  A  F  T  L  A

241     CGGAAGAAGTCACTGCAGAGCCTGCAGAGCACTGTGCATTACCACCTGGGCAGCCTGGCA
81      R  K  K  S  L  Q  S  L  Q  S  T  V  H  Y  H  L  G  S  L  A

301     CTGTCGGACCTGCTTATCCTTCTGCTGGCCATGCCCGTGGAGCTATACAACTTCATCTGG
101     L  S  D  L  L  I  L  L  L  A  M  P  V  E  L  Y  N  F  I  W

361     GTACACCATCCCTGGGCCTTTGGGGACGCTGGCTGCCGTGGCTACTATTTCCTGCGTGAT
121     V  H  H  P  W  A  F  G  D  A  G  C  R  G  Y  Y  F  L  R  D

421     GCCTGCACCTATGCCACAGCCCTCAATGTAGCCAGCCTGAGTGTGGAGCGCTACTTGGCC
141     A  C  T  Y  A  T  A  L  N  V  A  S  L  S  V  E  R  Y  L  A

481     ATCTGCCATCCCTTCAAGGCCAAGACCCTCATGTCCCGCAGCCGCACCAAGAAATTCATC
161     I  C  H  P  F  K  A  K  T  L  M  S  R  S  R  T  K  K  F  I

541     AGTGCCATATGGCTAGCTTCGGCGCTGCTGGCTATACCCATGCTTTTCACCATGGGCCTG
181     S  A  I  W  L  A  S  A  L  L  A  I  P  M  L  F  T  M  G  L

601 CAGAACCGCAGTGGTGACGGCACGCACCCTGGCGGCCTGGTGTGCACACCCATTGTGGAC
201 Q N R S G D G T H P G G L V C T P I V D

661 ACAGCCACTGTCAAGGTCGTCATCCAGGTTAACACCTTCATGTCCTTCCTGTTTCCCATG
221 T A T V K V V I Q V N T F M S F L F P M

721 TTGGTCATCTCCATCCTAAACACCGTGATTGCCAACAAACTGACAGTCATGGTGCACCAG
241 L V I S I L N T V I A N K L T V M V H Q

781 GCCGCCGAGCAGGGCCGAGTGTGCACCGTGGGCACACACAACGGTTTAGAGCACAGCACG
261 A A E Q G R V C T V G T H N G L E H S T

841 TTCAACATGACCATCGAGCCGGGTCGTGTCCAGGCCCTGCGCCACGGAGTCCTCGTCTTA
281 F N M T I E P G R V Q A L R H G V L V L

901 CGTGCTGTGGTCATTGCCTTTGTGGTCTGCTGGCTGCCCTACCACGTGCGACGCCTGATG
301 R A V V I A F V V C W L P Y H V R R L M

961 TTCTGCTATATCTCGGATGAACAGTGGACTACGTTCCTCTTCGATTTCTACCACTATTTC
321 F C Y I S D E Q W T T F L F D F Y H Y F

1021 TACATGCTAACCAACGCTCTCTTCTACGTCAGCTCCGCCATCAATCCCATCCTCTACAAC
341 Y M L T N A L F Y V S S A I N P I L Y N

1081 CTGGTCTCCGCCAACTTCCGCCAGGTCTTTCTGTCCACGCTGGCCTGCCTTTGTCCTGGG
361 L V S A N F R Q V F L S T L A C L C P G

1141 TGGCGCCACCGCCGAAAGAAGAGGCCAACGTTCTCCAGGAAGCCCAACAGCATGTCCAGC
381 W R H R R K K R P T F S R K P N S M S S

NotI +2 TrxA
1201 AACCATGCCTTTTCCACCAGCGCCACCCGGGAGACCCTGTACgcggccgcaAGCGATAAA
401 N H A F S T S A T R E T L Y A A A S D K

1261 ATTATTCACCTGACTGACGACAGTTTTGACACGGATGTACTCAAAGCGGACGGGGCGATC
421 I I H L T D D S F D T D V L K A D G A I

1321 CTCGTCGATTTCTGGGCAGAGTGGTGCGGTCCGTGCAAAATGATCGCCCCGATTCTGGAT
441 L V D F W A E W C G P C K M I A P I L D

1381 GAAATCGCTGACGAATATCAGGGCAAACTGACCGTTGCAAAACTGAACATCGATCAAAAC
461 E I A D E Y Q G K L T V A K L N I D Q N

1441 CCTGGCACTGCGCCGAAATATGGCATCCGTGGTATCCCGACTCTGCTGCTGTTCAAAAAC
481 P G T A P K Y G I R G I P T L L L F K N

1501 GGTGAAGTGGCGGCAACCAAAGTGGGTGCACTGTCTAAAGGTCAGTTGAAAGAGTTCCTC
501 G E V A A T K V G A L S K G Q L K E F L

NotI Flag stop KpnI
1561 GACGCTAACCTGGCAgcggccgcaGATTATAAAGATGACGATGACAAATAATAAGGTACC
521 D A N L A A A A D Y K D D D D K

### SEQ ID NO.: 188

### Human □2AR GS1□ chimeric fusion

Sall +1 B2AR
1 GTCGACATGG GGCAACCCGG GAACGGCAGC GCCTTCTTGC TGGCACCCAA TGGAAGCCAT

61 GCGCCGGACC ACGACGTCAC GCAGCAAAGG GACGAGGTGT GGGTGGTGGG CATGGGCATC
121 GTCATGTCTC TCATCGTCCT GGCCATCGTG TTTGGCAATG TGCTGGTCAT CACAGCCATT
181 GCCAAGTTCG AGCGTCTGCA GACGGTCACC AACTACTTCA TCACTTCACT GGCCTGTGCT
241 GATCTGGTCA TGGGCCTAGC AGTGGTGCCC TTTGGGGCCG CCCATATTCT TATGAAAATG
301 TGGACTTTTG GCAACTTCTG GTGCGAGTTT TGGACTTCCA TTGATGTGCT GTGCGTCACG
361 GCCAGCATTG AGACCCTGTG CGTGATCGCA GTGGATCGCT ACTTTGCCAT TACTTCACCT
421 TTCAAGTACC AGAGCCTGCT GACCAAGAAT AAGGCCCGGG TGATCATTCT GATGGTGTGG
481 ATTGTGTCAG GCCTTAYCTC CTTCTTGCCC ATTCAGATGC ACTGGTACAG GGCCACCCAC
541 CAGGAAGCCA TCAACTGCTA TGCCAATGAG ACCTGCTGTG ACTTCTTCAC GAACCAAGCC
601 TATGCCATTG CCTCTTCCAT CGTGTCCTTC TACGTTCCCC TGGTGATCAT GGTCTTCGTC
661 TACTCCAGGG TCTTTCAGGA GGCCAAAAGG CAGCTCCAGA AGATTGACAA ATCTGAGGGC
721 CGCTTCCATG TCCAGAACCT TAGCCAGGTG GAGCAGGATG GGCGGACGGG GCATGGACTC
781 CGCAGATCTT CCAAGTTCTG CTTGAAGGAG CACAAAGCCC TCAAGACGTT AGGCATCATC
841 ATGGGCACTT TCACCCTCTG CTGGCTGCCC TTCTTCATCG TTAACATTGT GCATGTGATC
901 CAGGATAACC TCATCCGTAA GGAAGTTTAC ATCCTCCTAA ATTGGATAGG CTATGTCAAT
961 TCTGGTTTCA ATCCCCTTAT CTACTGCCGG AGCCCAGATT TCAGGATTGC CTTCCAGGAG
1021 CTTCTGTGCC TGCGCAGGTC TTCTTTGAAG GCCTATGGCA ATGGCTACTC CAGCAACGGC
1081 AACACAGGGG AGCAGAGTGG ATATCACGTG GAACAGGAGA AAGAAAATAA ACTGCTGTGT
1141 GAAGACCTCC CAGGCACGGA AGACTTTGTG GGCCATCAAG GTACTGTGCC TAGCGATAAC

Last B2AR Linker sequence
1201 ATTGATTCAC AAGGGAGGAA TTGTAGTACA AATGACTCAC TGCTAGAGCG TGGCCAGACG

PstI XhoI +2 GS1 alpha
1261 GTCACCAACC TGCAGCTCGA GGGCTGCCTC GGGAACAGTA AGACCGAGGA CCAGCGCAAC

1321 GAGGAGAAGG CGCAGCGTGA GGCCAACAAA AAGATCGAGA AGCAGCTGCA GAAGGACAAG
1381 CAGGTCTACC GGGCCACGCA CCGCCTGCTG CTGCTGGGTG CTGGAGAATC TGGTAAAAGC
1441 ACCATTGTGA AGCAGATGAG GATCCTGCAT GTTAATGGGT TTAATGGAGA CAGTGAGAAG
1501 GCAACCAAAG TGCAGGACAT CAAAAACAAC CTGAAAGAGG CGATTGAAAC CATTGTGGCC
1561 GCCATGAGCA ACCTGGTGCC CCCCGTGGAG CTGGCCAACC CCGAGAACCA GTTCAGAGTG
1621 GACTACATCC TGAGTGTGAT GAACGTGCCT GACTTTGACT TCCCTCCCGA ATTCTATGAG
1681 CATGCCAAGG CTCTGTGGGA GGATGAAGGA GTGCGTGCCT GCTACGAACG CTCCAACGAG
1741 TACCAGCTGA TTGACTGTGC CCAGTACTTC CTGGACAAGA TCGACGTGAT CAAGCAGGCT
1801 GACTATGTGC CGAGCGATCA GGACCTGCTT CGCTGCCGTG TCCTGACTTC TGGAATCTTT
1861 GAGACCAAGT TCCAGGTGGA CAAAGTCAAC TTCCACATGT TTGACGTGGG TGGCCAGCGC
1921 GATGAACGCC GCAAGTGGAT CCAGTGCTTC AACGATGTGA CTGCCATCAT CTTCGTGGTG
1981 GCCAGCAGCA GCTACAACAT GGTCATCCGG GAGGACAACC AGACCAACCG CCTGCAGGAG
2041 GCTCTGAACC TCTTCAAGAG CATCTGGAAC AACAGATGGC TGCGCACCAT CTCTGTGATC
2101 CTGTTCCTCA ACAAGCAAGA TCTGCTCGCT GAGAAAGTCC TTGCTGGGAA ATCGAAGATT
2161 GAGGACTACT TTCCAGAATT TGCTCGCTAC ACTACTCCTG AGGATGCTAC TCCCGAGCCC
2221 GGAGAGGACC CACGCGTGAC CCGGGCCAAG TACTTCATTC GAGATGAGTT TCTGAGGATC
2281 AGCACTGCCA GTGGAGATGG GCGTCACTAC TGCTACCCTC ATTTCACCTG CGCTGTGGAC
2341 ACTGAGAACA TCCGCCGTGT GTTCAACGAC TGCCGTGACA TCATTCAGCG CATGCACCTT

ClaI Stop XbaI Stem-loop
2401 CGTCAGTACG AGCTGCTCAT CGATTAATAA TCTAGAGGAT CCCCGCGCCC TCATCCGAAA

2461 GGGCG

### SEQ ID NO.: 190

### Human □2AR stop GS1□ transcriptional fusion

PstI  +1 B2AR
1    GTCGACATGG GGCAACCCGG GAACGGCAGC GCCTTCTTGC TGGCACCCAA TGGAAGCCAT

61   GCGCCGGACC ACGACGTCAC GCAGCAAAGG GACGAGGTGT GGGTGGTGGG CATGGGCATC
121  GTCATGTCTC TCATCGTCCT GGCCATCGTG TTTGGCAATG TGCTGGTCAT CACAGCCATT
181  GCCAAGTTCG AGCGTCTGCA GACGGTCACC AACTACTTCA TCACTTCACT GGCCTGTGCT
241  GATCTGGTCA TGGGCCTAGC AGTGGTGCCC TTTGGGGCCG CCCATATTCT TATGAAAATG
301  TGGACTTTTG GCAACTTCTG GTGCGAGTTT TGGACTTCCA TTGATGTGCT GTGCGTCACG
361  GCCAGCATTG AGACCCTGTG CGTGATCGCA GTGGATCGCT ACTTTGCCAT TACTTCACCT
421  TTCAAGTACC AGAGCCTGCT GACCAAGAAT AAGGCCCGGG TGATCATTCT GATGGTGTGG
481  ATTGTGTCAG GCCTTAYCTC CTTCTTGCCC ATTCAGATGC ACTGGTACAG GGCCACCCAC
541  CAGGAAGCCA TCAACTGCTA TGCCAATGAG ACCTGCTGTG ACTTCTTCAC GAACCAAGCC
601  TATGCCATTG CCTCTTCCAT CGTGTCCTTC TACGTTCCCC TGGTGATCAT GGTCTTCGTC
661  TACTCCAGGG TCTTTCAGGA GGCCAAAAGG CAGCTCCAGA AGATTGACAA ATCTGAGGGC
721  CGCTTCCATG TCCAGAACCT TAGCCAGGTG GAGCAGGATG GGCGGACGGG GCATGGACTC
781  CGCAGATCTT CCAAGTTCTG CTTGAAGGAG CACAAAGCCC TCAAGACGTT AGGCATCATC
841  ATGGGCACTT TCACCCTCTG CTGGCTGCCC TTCTTCATCG TTAACATTGT GCATGTGATC
901  CAGGATAACC TCATCCGTAA GGAAGTTTAC ATCCTCCTAA ATTGGATAGG CTATGTCAAT
961  TCTGGTTTCA ATCCCCTTAT CTACTGCCGG AGCCCAGATT TCAGGATTGC CTTCCAGGAG
1021 CTTCTGTGCC TGCGCAGGTC TTCTTTGAAG GCCTATGGCA ATGGCTACTC CAGCAACGGC
1081 AACACAGGGG AGCAGAGTGG ATATCACGTG GAACAGGAGA AAGAAAATAA ACTGCTGTGT
1141 GAAGACCTCC CAGGCACGGA AGACTTTGTG GGCCATCAAG GTACTGTGCC TAGCGATAAC

Last B2AR  Linker sequence
1201 ATTGATTCAC AAGGGAGGAA TTGTAGTACA AATGACTCAC TGCTAGAGCG TGGCCAGACG

PstI  Stop  SD  XhoI  +2 GS1 alpha
1261 GTCACCAACC TGCAGTAATA ATCAAGGAGG CCCTCGAGAT GGGCTGCCTC GGGAACAGTA

1321 AGACCGAGGA CCAGCGCAAC GAGGAGAAGG CGCAGCGTGA GGCCAACAAA AAGATCGAGA
1381 AGCAGCTGCA GAAGGACAAG CAGGTCTACC GGGCCACGCA CCGCCTGCTG CTGCTGGGTG
1441 CTGGAGAATC TGGTAAAAGC ACCATTGTGA AGCAGATGAG GATCCTGCAT GTTAATGGGT
1501 TTAATGGAGA CAGTGAGAAG GCAACCAAAG TGCAGGACAT CAAAAACAAC CTGAAAGAGG
1561 CGATTGAAAC CATTGTGGCC GCCATGAGCA ACCTGGTGCC CCCCGTGGAG CTGGCCAACC
1621 CCGAGAACCA GTTCAGAGTG GACTACATCC TGAGTGTGAT GAACGTGCCT GACTTTGACT
1681 TCCCTCCCGA ATTCTATGAG CATGCCAAGG CTCTGTGGGA GGATGAAGGA GTGCGTGCCT
1741 GCTACGAACG CTCCAACGAG TACCAGCTGA TTGACTGTGC CCAGTACTTC CTGGACAAGA
1801 TCGACGTGAT CAAGCAGGCT GACTATGTGC CGAGCGATCA GGACCTGCTT CGCTGCCGTG
1861 TCCTGACTTC TGGAATCTTT GAGACCAAGT TCCAGGTGGA CAAAGTCAAC TTCCACATGT
1921 TTGACGTGGG TGGCCAGCGC GATGAACGCC GCAAGTGGAT CCAGTGCTTC AACGATGTGA
1981 CTGCCATCAT CTTCGTGGTG GCCAGCAGCA GCTACAACAT GGTCATCCGG GAGGACAACC
2041 AGACCAACCG CCTGCAGGAG GCTCTGAACC TCTTCAAGAG CATCTGGAAC AACAGATGGC
2101 TGCGCACCAT CTCTGTGATC CTGTTCCTCA ACAAGCAAGA TCTGCTCGCT GAGAAAGTCC
2161 TTGCTGGGAA ATCGAAGATT GAGGACTACT TTCCAGAATT TGCTCGCTAC ACTACTCCTG
2221 AGGATGCTAC TCCCGAGCCC GGAGAGGACC CACGCGTGAC CCGGGCCAAG TACTTCATTC
2281 GAGATGAGTT TCTGAGGATC AGCACTGCCA GTGGAGATGG GCGTCACTAC TGCTACCCTC
2341 ATTTCACCTG CGCTGTGGAC ACTGAGAACA TCCGCCGTGT GTTCAACGAC TGCCGTGACA

ClaI  Stop  XbaI
2401 TCATTCAGCG CATGCACCTT CGTCAGTACG AGCTGCTCAT CGATTAATAA TCTAGAGGAT

Stem-loop
2461 CCCCGCGCCC TCATCCGAAA GGGCG

### SEQ ID NO.: 192

### Human GS1□

XhoI
1 CTCGAGATGGGCTGCCTCGGGAACAGTAAGACCGAGGACCAGCGCAACGAGGAGAAGGCGCAGCGT
1 M G C L G N S K T E D Q R N E E K A Q R

61 GAGGCCAACAAAAAGATCGAGAAGCAGCTGCAGAAGGACAAGCAGGTCTACCGGGCCACG
21 E A N K K I E K Q L Q K D K Q V Y R A T

121 CACCGCCTGCTGCTGCTGGGTGCTGGAGAATCTGGTAAAAGCACCATTGTGAAGCAGATG
41 H R L L L L G A G E S G K S T I V K Q M

181 AGGATCCTGCATGTTAATGGGTTTAATGGAGACAGTGAGAAGGCAACCAAAGTGCAGGAC
61 R I L H V N G F N G D S E K A T K V Q D

241 ATCAAAAACAACCTGAAAGAGGCGATTGAAACCATTGTGGCCGCCATGAGCAACCTGGTG
81 I K N N L K E A I E T I V A A M S N L V

301 CCCCCCGTGGAGCTGGCCAACCCCGAGAACCAGTTCAGAGTGGACTACATCCTGAGTGTG
101 P P V E L A N P E N Q F R V D Y I L S V

361 ATGAACGTGCCTGACTTTGACTTCCCTCCCGAATTCTATGAGCATGCCAAGGCTCTGTGG
121 M N V P D F D F P P E F Y E H A K A L W

421 GAGGATGAAGGAGTGCGTGCCTGCTACGAACGCTCCAACGAGTACCAGCTGATTGACTGT
141 E D E G V R A C Y E R S N E Y Q L I D C

481 GCCCAGTACTTCCTGGACAAGATCGACGTGATCAAGCAGGCTGACTATGTGCCGAGCGAT
161 A Q Y F L D K I D V I K Q A D Y V P S D

541 CAGGACCTGCTTCGCTGCCGTGTCCTGACTTCTGGAATCTTTGAGACCAAGTTCCAGGTG
181 Q D L L R C R V L T S G I F E T K F Q V

601 GACAAAGTCAACTTCCACATGTTTGACGTGGGTGGCCAGCGCGATGAACGCCGCAAGTGG
201 D K V N F H M F D V G G Q R D E R R K W

661 ATCCAGTGCTTCAACGATGTGACTGCCATCATCTTCGTGGTGGCCAGCAGCAGCTACAAC
221 I Q C F N D V T A I I F V V A S S S Y N

721 ATGGTCATCCGGGAGGACAACCAGACCAACCGCCTGCAGGAGGCTCTGAACCTCTTCAAG
241 M V I R E D N Q T N R L Q E A L N L F K

781 AGCATCTGGAACAACAGATGGCTGCGCACCATCTCTGTGATCCTGTTCCTCAACAAGCAA
261 S I W N N R W L R T I S V I L F L N K Q

841 GATCTGCTCGCTGAGAAAGTCCTTGCTGGGAAATCGAAGATTGAGGACTACTTTCCAGAA
281 D L L A E K V L A G K S K I E D Y F P E

901 TTTGCTCGCTACACTACTCCTGAGGATGCTACTCCCGAGCCCGGAGAGGACCCACGCGTG
301 F A R Y T T P E D A T P E P G E D P R V

961 ACCCGGGCCAAGTACTTCATTCGAGATGAGTTTCTGAGGATCAGCACTGCCAGTGGAGAT
321 T R A K Y F I R D E F L R I S T A S G D

1021 GGGCGTCACTACTGCTACCCTCATTTCACCTGCGCTGTGGACACTGAGAACATCCGCCGT
341 G R H Y C Y P H F T C A V D T E N I R R

1081 GTGTTCAACGACTGCCGTGACATCATTCAGCGCATGCACCTTCGTCAGTACGAGCTGCTC
361 V F N D C R D I I Q R M H L R Q Y E L L

ClaI
ATCGAT

### SEQ ID NO.: 193

### Human GS2□

XhoI
1 CTCGAGATGGGCTGCCTCGGGAACAGTAAGACCGAGGACCAGCGCAACGAGGAGAAGGCGCAGCGT
1 M G C L G N S K T E D Q R N E E K A Q R

61 GAGGCCAACAAAAAGATCGAGAAGCAGCTGCAGAAGGACAAGCAGGTCTACCGGGCCACG
21 E A N K K I E K Q L Q K D K Q V Y R A T

121 CACCGCCTGCTGCTGCTGGGTGCTGGAGAATCTGGTAAAAGCACCATTGTGAAGCAGATG
41 H R L L L L G A G E S G K S T I V K Q M

181 AGGATCCTGCATGTTAATGGGTTTAATGGAGAGGGCGGCGAAGAGGACCCGCAGGCTGCA
61 R I L H V N G F N G E G G E E D P Q A A

241 AGGAGCAACAGCGATGGTGAGAAGGCAACCAAAGTGCAGGACATCAAAAACAACCTGAAA
81 R S N S D G E K A T K V Q D I K N N L K

301 GAGGCGATTGAAACCATTGTGGCCGCCATGAGCAACCTGGTGCCCCCCGTGGAGCTGGCC
101 E A I E T I V A A M S N L V P P V E L A

361 AACCCCGAGAACCAGTTCAGAGTGGACTACATCCTGAGTGTGATGAACGTGCCTGACTTT
121 N P E N Q F R V D Y I L S V M N V P D F

421 GACTTCCCTCCCGAATTCTATGAGCATGCCAAGGCTCTGTGGGAGGATGAAGGAGTGCGT
141 D F P P E F Y E H A K A L W E D E G V R

481 GCCTGCTACGAACGCTCCAACGAGTACCAGCTGATTGACTGTGCCCAGTACTTCCTGGAC
161 A C Y E R S N E Y Q L I D C A Q Y F L D

541 AAGATCGACGTGATCAAGCAGGCTGACTATGTGCCGAGCGATCAGGACCTGCTTCGCTGC
181 K I D V I K Q A D Y V P S D Q D L L R C

601 CGTGTCCTGACTTCTGGAATCTTTGAGACCAAGTTCCAGGTGGACAAAGTCAACTTCCAC
201 R V L T S G I F E T K F Q V D K V N F H

661 ATGTTTGACGTGGGTGGCCAGCGCGATGAACGCCGCAAGTGGATCCAGTGCTTCAACGAT
221 M F D V G G Q R D E R R K W I Q C F N D

721 GTGACTGCCATCATCTTCGTGGTGGCCAGCAGCAGCTACAACATGGTCATCCGGGAGGAC
241 V T A I I F V V A S S S Y N M V I R E D

781 AACCAGACCAACCGCCTGCAGGAGGCTCTGAACCTCTTCAAGAGCATCTGGAACAACAGA
261 N Q T N R L Q E A L N L F K S I W N N R

841 TGGCTGCGCACCATCTCTGTGATCCTGTTCCTCAACAAGCAAGATCTGCTCGCTGAGAAA
281 W L R T I S V I L F L N K Q D L L A E K

901 GTCCTTGCTGGGAAATCGAAGATTGAGGACTACTTTCCAGAATTTGCTCGCTACACTACT

301 V L A G K S K I E D Y F P E F A R Y T T

961 CCTGAGGATGCTACTCCCGAGCCCGGAGAGGACCCACGCGTGACCCGGGCCAAGTACTTC
321 P E D A T P E P G E D P R V T R A K Y F

1021 ATTCGAGATGAGTTTCTGAGGATCAGCACTGCCAGTGGAGATGGGCGTCACTACTGCTAC
341 I R D E F L R I S T A S G D G R H Y C Y

1081 CCTCATTTCACCTGCGCTGTGGACACTGAGAACATCCGCCGTGTGTTCAACGACTGCCGT
361 P H F T C A V D T E N I R R V F N D C R

ClaI
1141 GACATCATTCAGCGCATGCACCTTCGTCAGTACGAGCTGCTCATCGAT
381 D I I Q R M H L R Q Y E L L

### SEQ ID NO.: 194

### Human G□q

XhoI
1 CTCGAGATGACTCTGGAGTCCATCATGGCGTGCTGCCTGAGCGAGGAGGCCAAGGAAGCCCGGCGG
1 M T L E S I M A C C L S E E A K E A R R

61 ATCAACGACGAGATCGAGCGGCAGCTCCGCAGGGACAAGCGGGACGCCCGCCGGGAGCTC
21 I N D E I E R Q L R R D K R D A R R E L

121 AAGCTGCTGCTGCTCGGGACAGGAGAGAGTGGCAAGAGTACGTTTATCAAGCAGATGAGA
41 K L L L L G T G E S G K S T F I K Q M R

181 ATCATCCATGGGTCAGGATACTCTGATGAAGATAAAAGGGGCTTCACCAAGCTGGTGTAT
61 I I H G S G Y S D E D K R G F T K L V Y

241 CAGAACATCTTCACGGCCATGCAGGCCATGATCAGAGCCATGGACACACTCAAGATCCCA
81 Q N I F T A M Q A M I R A M D T L K I P

301 TACAAGTATGAGCACAATAAGGCTCATGCACAATTAGTTCGAGAAGTTGATGTGGAGAAG
101 Y K Y E H N K A H A Q L V R E V D V E K

361 GTGTCTGCTTTTGAGAATCCATATGTAGATGCAATAAAGAGTTTATGGAATGATCCTGGA
121 V S A F E N P Y V D A I K S L W N D P G

421 ATCCAGGAATGCTATGATAGACGACGAGAATATCAATTATCTGACTCTACCAAATACTAT
141 I Q E C Y D R R R E Y Q L S D S T K Y Y

481 CTTAATGACTTGGACCGCGTAGCTGACCCTGCCTACCTGCCTACGCAACAAGATGTGCTT
161 L N D L D R V A D P A Y L P T Q Q D V L

541 AGAGTTCGAGTCCCCACCACAGGGATCATCGAATACCCCTTTGACTTACAAAGTGTCATT
181 R V R V P T T G I I E Y P F D L Q S V I

601 TTCAGAATGGTCGATGTAGGGGGCCAAAGGTCAGAGAGAAGAAAATGGATACACTGCTTT
201 F R M V D V G G Q R S E R R K W I H C F

661 GAAAATGTCACCTCTATCATGTTTCTAGTAGCGCTTAGTGAATATGATCAAGTTCTCGTG
221 E N V T S I M F L V A L S E Y D Q V L V

721 GAGTCAGACAATGAGAACCGAATGGAGGAAAGCAAGGCTCTCTTTAGAACAATTATCACA

241 E S D N E N R M E E S K A L F R T I I T

781 TACCCCTGGTTCCAGAACTCCTCGGTTATTCTGTTCTTAAACAAGAAAGATCTTCTAGAG
261 Y P W F Q N S S V I L F L N K K D L L E

841 GAGAAAATCATGTATTCCCATCTAGTCGACTACTTCCCAGAATATGATGGACCCCAGAGA
281 E K I M Y S H L V D Y F P E Y D G P Q R

901 GATGCCCAGGCAGCCCGAGAATTCATTCTGAAGATGTTCGTGGACCTGAACCCAGACAGT
301 D A Q A A R E F I L K M F V D L N P D S

961 GACAAAATTATCTACTCCCACTTCACGTGCGCCACAGACACCGAGAATATCCGCTTTGTC
321 D K I I Y S H F T C A T D T E N I R F V

ClaI
1021 TTTGCTGCCGTCAAGGACACCATCCTCCAGTTGAACCTGAAGGAGTACAATCTGGTCATCGAT
341 F A A V K D T I L Q L N L K E Y N L V

### SEQ ID NO.: 195

### Human Gi□

XhoI
1 CTCGAGATGGGCTGCACCGTGAGCGCCGAGGACAAGGCGGCGGCCGAGCGCTCTAAGATGATCGAC
1 M G C T V S A E D K A A A E R S K M I D

61 AAGAACCTGCGGGAGGACGGAGAGAAGGCGGCGCGGGAGGTGAAGTTGCTGCTGTTGGGT
21 K N L R E D G E K A A R E V K L L L L G

121 GCTGGGGAGTCAGGGAAGAGCACCATCGTCAAGCAGATGAAGATCATCCACGAGGATGGC
41 A G E S G K S T I V K Q M K I I H E D G

181 TACTCCGAGGAGGAATGCCGGCAGTACCGGGCGGTTGTCTACAGCAACACCATCCAGTCC
61 Y S E E E C R Q Y R A V V Y S N T I Q S

241 ATCATGGCCATTGTCAAAGCCATGGGAAACCTGCAGATCGACTTTGCCGACCCCTCCAGA
81 I M A I V K A M G N L Q I D F A D P S R

301 GCGGACGACGCCAGGCAGCTATTTGCACTGTCCTGCACCGCCGAGGAGCAAGGCGTGCTC
101 A D D A R Q L F A L S C T A E E Q G V L

361 CCTGATGACCTGTCCGGCGTCATCCGGAGGCTCTGGGCTGACCATGGTGTGCAGGCCTGC
121 P D D L S G V I R R L W A D H G V Q A C

421 TTTGGCCGCTCAAGGGAATACCAGCTCAACGACTCAGCTGCCTACTACCTGAACGACCTG
141 F G R S R E Y Q L N D S A A Y Y L N D L

481 GAGCGTATTGCACAGAGTGACTACATCCCCACACAGCAAGATGTGCTACGGACCCGCGTA
161 E R I A Q S D Y I P T Q Q D V L R T R V

541 AAGACCACGGGGATCGTGGAGACACACTTCACCTTCAAGGACCTACACTTCAAGATGTTT
181 K T T G I V E T H F T F K D L H F K M F

601 GATGTGGGTGGTCAGCGGTCTGAGCGGAAGAAGTGGATCCACTGCTTTGAGGGCGTCACA
201 D V G G Q R S E R K K W I H C F E G V T

661 GCCATCATCTTCTGCGTAGCCTTGAGCGCCTATGACTTGGTGCTAGCTGAGGACGAGGAG

221 A I I F C V A L S A Y D L V L A E D E E

721 ATGAACCGCATGCATGAGAGCATGAAGCTATTCGATAGCATCTGCAACAACAAGTGGTTC
241 M N R M H E S M K L F D S I C N N K W F

781 ACAGACACGTCCATCATCCTCTTCCTCAACAAGAAGGACCTGTTTGAGGAGAAGATCACA
261 T D T S I I L F L N K K D L F E E K I T

841 CACAGTCCCCTGACCATCTGCTTCCCTGAGTACACAGGGGCCAACAAATATGATGAGGCA
281 H S P L T I C F P E Y T G A N K Y D E A

901 GCCAGCTACATCCAGAGTAAGTTTGAGGACCTGAATAAGCGCAAAGACACCAAGGAGATC
301 A S Y I Q S K F E D L N K R K D T K E I

961 TACACGCACTTCACGTGCGCCACCGACACCAAGAACGTGCAGTTCGTGTTTGACGCCGTC
321 Y T H F T C A T D T K N V Q F V F D A V

ClaI
1021 ACCGATGTCATCATCAAGAACAACCTGAAGGACTGCGGCCTCTTCATGCAT
341 T D V I I K N N L K D C G L F

### SEO ID NO.: 196

### Human G□12/13

XhoI
1 CTCGAGATGTCCGGGGTGGTGCGGACCCTCAGCCGCTGCCTGCTGCCGGCCGAGGCCGGCGGGGCC
1 M S G V V R T L S R C L L P A E A G G A

61 CGCGAGCGCAGGGCGGGCAGCGGCGCGCGCGACGCGGAGCGCGAGGCCCGGAGGCGTAGC
21 R E R R A G S G A R D A E R E A R R R S

121 CGCGACATCGACGCGCTGCTGGCCCGCGAGCGGCGCGCGGTCCGGCGCCTGGTGAAGATC
41 R D I D A L L A R E R R A V R R L V K I

181 CTGCTGCTGGGCGCGGGCGAGAGCGGCAAGTCCACGTTCCTCAAGCAGATGCGCATCATC
61 L L L G A G E S G K S T F L K Q M R I I

241 CACGGCCGCGAGTTCGACCAGAAGGCGCTGCTGGAGTTCCGCGACACCATCTTCGACAAC
81 H G R E F D Q K A L L E F R D T I F D N

301 ATCCTCAAGGGCTCAAGGGTTCTTGTTGATGCACGAGATAAGCTTGGCATTCCTTGGCAG
101 I L K G S R V L V D A R D K L G I P W Q

361 TATTCTGAAAATGAGAAGCATGGGATGTTCCTGATGGCCTTCGAGAACAAGGCGGGGCTG
121 Y S E N E K H G M F L M A F E N K A G L

421 CCTGTGGAGCCGGCCACCTTCCAGCTGTACGTCCCGGCCCTGAGCGCACTCTGGAGGGAT
141 P V E P A T F Q L Y V P A L S A L W R D

481 TCTGGCATCAGGGAGGCTTTCAGCCGGAGAAGCGAGTTTCAGCTGGGGGAGTCGGTGAAG
161 S G I R E A F S R R S E F Q L G E S V K

541 TACTTCCTGGACAACTTGGACCGGATCGGCCAGCTGAATTACTTTCCTAGTAAGCAAGAT
181 Y F L D N L D R I G Q L N Y F P S K Q D

601 ATCCTGCTGGCTAGGAAAGCCACCAAGGGAATTGTGGAGCATGACTTCGTTATTAAGAAG

201 I L L A R K A T K G I V E H D F V I K K

661 ATCCCCTTTAAGATGGTGGATGTGGGCGGCCAGCGGTCCCAGCGCCAGAAGTGGTTCCAG
221 I P F K M V D V G G Q R S Q R Q K W F Q

721 TGCTTCGACGGGATCACGTCCATCCTGTTCATGGTCTCCTCCAGCGAGTACGACCAGGTC
241 C F D G I T S I L F M V S S S E Y D Q V

781 CTCATGGAGGACAGGCGCACCAACCGGCTGGTGGAGTCCATGAACATCTTCGAGACCATC
261 L M E D R R T N R L V E S M N I F E T I

841 GTCAACAACAAGCTCTTCTTCAACGTCTCCATCATTCTCTTCCTCAACAAGATGGACCTC
281 V N N K L F F N V S I I L F L N K M D L

901 CTGGTGGAGAAGGTGAAGACCGTGAGCATCAAGAAGCACTTCCCGGACTTCAGGGGCGAC
301 L V E K V K T V S I K K H F P D F R G D

961 CCGCACCAGCTGGAGGACGTCCAGCGCTACCTGGTCCAGTGCTTCGACAGGAAGAGACGG
321 P H Q L E D V Q R Y L V Q C F D R K R R

1021 AACCGCAGCAAGCCACTCTTCCACCACTTCACCACCGCCATCGACACCGAGAACGTCCGC
341 N R S K P L F H H F T T A I D T E N V R

1081 TTCGTGTTCCATGCTGTGAAAGACACCATCCTGCAGGAGAACCTGAAGGACATCATGCTG
361 F V F H A V K D T I L Q E N L K D I M L

ClaI
1141 CAGATCGAT
381 Q

### SEQ ID NO.: 205

### Human □2AR-ToxR (5-141) chimera stop GS1□-ToxR (5-141) chimera transcriptional fusion

Sall +1 B2AR
1 GTCGACATGG GGCAACCCGG GAACGGCAGC GCCTTCTTGC TGGCACCCAA TGGAAGCCAT

61 GCGCCGGACC ACGACGTCAC GCAGCAAAGG GACGAGGTGT GGGTGGTGGG CATGGGCATC
121 GTCATGTCTC TCATCGTCCT GGCCATCGTG TTTGGCAATG TGCTGGTCAT CACAGCCATT
181 GCCAAGTTCG AGCGTCTGCA GACGGTCACC AACTACTTCA TCACTTCACT GGCCTGTGCT
241 GATCTGGTCA TGGGCCTAGC AGTGGTGCCC TTTGGGGCCG CCCATATTCT TATGAAAATG
301 TGGACTTTTG GCAACTTCTG GTGCGAGTTT TGGACTTCCA TTGATGTGCT GTGCGTCACG
361 GCCAGCATTG AGACCCTGTG CGTGATCGCA GTGGATCGCT ACTTTGCCAT TACTTCACCT
421 TTCAAGTACC AGAGCCTGCT GACCAAGAAT AAGGCCCGGG TGATCATTCT GATGGTGTGG
481 ATTGTGTCAG GCCTTAYCTC CTTCTTGCCC ATTCAGATGC ACTGGTACAG GGCCACCCAC
541 CAGGAAGCCA TCAACTGCTA TGCCAATGAG ACCTGCTGTG ACTTCTTCAC GAACCAAGCC
601 TATGCCATTG CCTCTTCCAT CGTGTCCTTC TACGTTCCCC TGGTGATCAT GGTCTTCGTC
661 TACTCCAGGG TCTTTCAGGA GGCCAAAAGG CAGCTCCAGA AGATTGACAA ATCTGAGGGC
721 CGCTTCCATG TCCAGAACCT TAGCCAGGTG GAGCAGGATG GGCGGACGGG GCATGGACTC

781 CGCAGATCTT CCAAGTTCTG CTTGAAGGAG CACAAAGCCC TCAAGACGTT AGGCATCATC
841 ATGGGCACTT TCACCCTCTG CTGGCTGCCC TTCTTCATCG TTAACATTGT GCATGTGATC
901 CAGGATAACC TCATCCGTAA GGAAGTTTAC ATCCTCCTAA ATTGGATAGG CTATGTCAAT
961 TCTGGTTTCA ATCCCCTTAT CTACTGCCGG AGCCCAGATT TCAGGATTGC CTTCCAGGAG
1021 CTTCTGTGCC TGCGCAGGTC TTCTTTGAAG GCCTATGGCA ATGGCTACTC CAGCAACGGC
1081 AACACAGGGG AGCAGAGTGG ATATCACGTG GAACAGGAGA AAGAAAATAA ACTGCTGTGT
1141 GAAGACCTCC CAGGCACGGA AGACTTTGTG GGCCATCAAG GTACTGTGCC TAGCGATAAC

last B2AR linker sequence
1201 ATTGATTCAC AAGGGAGGAA TTGTAGTACA AATGACTCAC TGCTAGAGCG TGGCCAGACG

PstI +5 toxR (5-141)
1261 GTCACCAACC TGCAGGGACA CAACTCAAAA GAGATATCGA TGAGTCATAT TGGTACTAAA

1321 TTCATTCTTG CTGAAAAATT TACCTTCGAT CCCCTAAGCA ATACTCTGAT TGACAAAGAA
1381 GATAGTGAAG AGATCATTCG ATTAGGCAGC AACGAAAGCC GAATTCTTTG GCTGCTGGCC
1441 CAACGTCCAA ACGAGGTAAT TTCTCGCAAT GATTTGCATG ACTTTGTTTG GCGAGAGCAA
1501 GGTTTTGAAG TCGATGATTC CAGCTTAACC CAAGCCATTT CGACTCTGCG CAAAATGCTC
1561 AAAGATTCGA CAAAGTCCCC ACAATACGTC AAAACGGTTC CGAAGCGCGG TTACCAATTG
1621 ATCGCCCGAG TGGAAACGGT TGAAGAAGAG ATGGCTCGCG AAAACGAAGC TGCTCATGAC

stop SD XhoI +1 GS1 alpha
1681 ATCTCTTAAT AATCAAGGAG GCCCTCGAGA TGGGCTGCCT CGGGAACAGT AAGACCGAGG

1741 ACCAGCGCAA CGAGGAGAAG GCGCAGCGTG AGGCCAACAA AAAGATCGAG AAGCAGCTGC
1801 AGAAGGACAA GCAGGTCTAC CGGGCCACGC ACCGCCTGCT GCTGCTGGGT GCTGGAGAAT
1861 CTGGTAAAAG CACCATTGTG AAGCAGATGA GGATCCTGCA TGTTAATGGG TTTAATGGAG
1921 ACAGTGAGAA GGCAACCAAA GTGCAGGACA TCAAAAACAA CCTGAAAGAG GCGATTGAAA
1981 CCATTGTGGC CGCCATGAGC AACCTGGTGC CCCCCGTGGA GCTGGCCAAC CCCGAGAACC
2041 AGTTCAGAGT GGACTACATC CTGAGTGTGA TGAACGTGCC TGACTTTGAC TTCCCTCCCG
2101 AATTCTATGA GCATGCCAAG GCTCTGTGGG AGGATGAAGG AGTGCGTGCC TGCTACGAAC
2161 GCTCCAACGA GTACCAGCTG ATTGACTGTG CCCAGTACTT CCTGGACAAG ATCGACGTGA
2221 TCAAGCAGGC TGACTATGTG CCGAGCGATC AGGACCTGCT TCGCTGCCGT GTCCTGACTT
2281 CTGGAATCTT TGAGACCAAG TTCCAGGTGG ACAAAGTCAA CTTCCACATG TTTGACGTGG
2341 GTGGCCAGCG CGATGAACGC CGCAAGTGGA TCCAGTGCTT CAACGATGTG ACTGCCATCA
2401 TCTTCGTGGT GGCCAGCAGC AGCTACAACA TGGTCATCCG GGAGGACAAC CAGACCAACC
2461 GCCTGCAGGA GGCTCTGAAC CTCTTCAAGA GCATCTGGAA CAACAGATGG CTGCGCACCA
2521 TCTCTGTGAT CCTGTTCCTC AACAAGCAAG ATCTGCTCGC TGAGAAAGTC CTTGCTGGGA
2581 AATCGAAGAT TGAGGACTAC TTTCCAGAAT TTGCTCGCTA CACTACTCCT GAGGATGCTA
2641 CTCCCGAGCC CGGAGAGGAC CCACGCGTGA CCCGGGCCAA GTACTTCATT CGAGATGAGT
2701 TTCTGAGGAT CAGCACTGCC AGTGGAGATG GGCGTCACTA CTGCTACCCT CATTTCACCT
2761 GCGCTGTGGA CACTGAGAAC ATCCGCCGTG TGTTCAACGA CTGCCGTGAC ATCATTCAGC

ClaI +5 toxR (5-141)
2821 GCATGCACCT TCGTCAGTAC GAGCTGCTCA TCGATGGACA CAACTCAAAA GAGATATCGA

2881 TGAGTCATAT TGGTACTAAA TTCATTCTTG CTGAAAAATT TACCTTCGAT CCCCTAAGCA
2941 ATACTCTGAT TGACAAAGAA GATAGTGAAG AGATCATTCG ATTAGGCAGC AACGAAAGCC
3001 GAATTCTTTG GCTGCTGGCC CAACGTCCAA ACGAGGTAAT TTCTCGCAAT GATTTGCATG
3061 ACTTTGTTTG GCGAGAGCAA GGTTTTGAAG TCGATGATTC CAGCTTAACC CAAGCCATTT
3121 CGACTCTGCG CAAAATGCTC AAAGATTCGA CAAAGTCCCC ACAATACGTC AAAACGGTTC
3181 CGAAGCGCGG TTACCAATTG ATCGCCCGAG TGGAAACGGT TGAAGAAGAG ATGGCTCGCG

Stop XbaI Stem-loop
3241 AAAACGAAGC TGCTCATGAC ATCTCTTAAT AATCTAGAGG ATCCCCGCGC CCTCATCCGA

3301 AAGGGCG

### SEQ ID NO.: 208

### Vibrio cholerae Pctx::lacZ reporter fusion constuct

XbaI
1 TCTAGAGGCT GTGGGTAGAA GTGAAACGGG GTTTACCGAT AAAAACAGAA AATGATAAAA

3 ToxR binding repeats
61 AAGGACTAAA TAGTATATTT TGATTTTTGA TTTTTGATTT CAAATAATAC AAATTTATTT

+1
lacZ
121 ACTTATTTAA TTGTTTTGAT CAATTATTTT TCTGTTAAAC AAAGGGAGCA TTATATGGTA

181 AAGACCATGA TTACGGATTC ACTGGCCGTC GTTTTACAAC GTCGTGACTG GGAAAACCCT
241 GGCGTTACCC AACTTAATCG CCTTGCAGCA CATCCCCCTT TCGCCAGCTG GCGTAATAGC
301 GAAGAGGCCC GCACCGATCG CCCTTCCCAA CAGTTGCGCA GCCTGAATGG CGAATGGCGC
361 TTTGCCTGGT TTCCGGCACC AGAAGCGGTG CCGGAAAGCT GGCTGGAGTG CGATCTTCCT
421 GAGGCCGATA CTGTCGTCGT CCCCTCAAAC TGGCAGATGC ACGGTTACGA TGCGCCCATC
481 TACACCAACG TGACCTATCC CATTACGGTC AATCCGCCGT TTGTTCCCAC GGAGAATCCG
541 ACGGGTTGTT ACTCGCTCAC ATTTAATGTT GATGAAAGCT GGCTACAGGA AGGCCAGACG
601 CGAATTATTT TTGATGGCGT TAACTCGGCG TTTCATCTGT GGTGCAACGG GCGCTGGGTC
661 GGTTACGGCC AGGACAGTCG TTTGCCGTCT GAATTTGACC TGAGCGCATT TTTACGCGCC
721 GGAGAAAACC GCCTCGCGGT GATGGTGCTG CGCTGGAGTG ACGGCAGTTA TCTGGAAGAT
781 CAGGATATGT GGCGGATGAG CGGCATTTTC CGTGACGTCT CGTTGCTGCA TAAACCGACT
841 ACACAAATCA GCGATTTCCA TGTTGCCACT CGCTTTAATG ATGATTTCAG CCGCGCTGTA
901 CTGGAGGCTG AAGTTCAGAT GTGCGGCGAG TTGCGTGACT ACCTACGGGT AACAGTTTCT
961 TTATGGCAGG GTGAAACGCA GGTCGCCAGC GGCACCGCGC CTTTCGGCGG TGAAATTATC
1021 GATGAGCGTG GTGGTTATGC CGATCGCGTC ACACTACGTC TGAACGTCGA AAACCCGAAA
1081 CTGTGGAGCG CCGAAATCCC GAATCTCTAT CGTGCGGTGG TTGAACTGCA CACCGCCGAC
1141 GGCACGCTGA TTGAAGCAGA AGCCTGCGAT GTCGGTTTCC GCGAGGTGCG GATTGAAAAT
1201 GGTCTGCTGC TGCTGAACGG CAAGCCGTTG CTGATTCGAG GCGTTAACCG TCACGAGCAT
1261 CATCCTCTGC ATGGTCAGGT CATGGATGAG CAGACGATGG TGCAGGATAT CCTGCTGATG
1321 AAGCAGAACA ACTTTAACGC CGTGCGCTGT TCGCATTATC CGAACCATCC GCTGTGGTAC
1381 ACGCTGTGCG ACCGCTACGG CCTGTATGTG GTGGATGAAG CCAATATTGA AACCCACGGC
1441 ATGGTGCCAA TGAATCGTCT GACCGATGAT CCGCGCTGGC TACCGGCGAT GAGCGAACGC
1501 GTAACGCGAA TGGTGCAGCG CGATCGTAAT CACCCGAGTG TGATCATCTG GTCGCTGGGG
1561 AATGAATCAG GCCACGGCGC TAATCACGAC GCGCTGTATC GCTGGATCAA ATCTGTCGAT
1621 CCTTCCCGCC CGGTGCAGTA TGAAGGCGGC GGAGCCGACA CCACGGCCAC CGATATTATT
1681 TGCCCGATGT ACGCGCGCGT GGATGAAGAC CAGCCCTTCC CGGCTGTGCC GAAATGGTCC
1741 ATCAAAAAAT GGCTTTCGCT ACCTGGAGAG ACGCGCCCGC TGATCCTTTG CGAATACGCC
1801 CACGCGATGG GTAACAGTCT TGGCGGTTTC GCTAAATACT GGCAGGCGTT TCGTCAGTAT
1861 CCCCGTTTAC AGGGCGGCTT CGTCTGGGAC TGGGTGGATC AGTCGCTGAT TAAATATGAT
1921 GAAAACGGCA ACCCGTGGTC GGCTTACGGC GGTGATTTTG GCGATACGCC GAACGATCGC
1981 CAGTTCTGTA TGAACGGTCT GGTCTTTGCC GACCGCACGC CGCATCCAGC GCTGACGGAA
2041 GCAAAACACC AGCAGCAGTT TTTCCAGTTC CGTTTATCCG GGCAAACCAT CGAAGTGACC
2101 AGCGAATACC TGTTCCGTCA TAGCGATAAC GAGCTCCTGC ACTGGATGGT GGCGCTGGAT
2161 GGTAAGCCGC TGGCAAGCGG TGAAGTGCCT CTGGATGTCG CTCCACAAGG TAAACAGTTG
2221 ATTGAACTGC CTGAACTACC GCAGCCGGAG AGCGCCGGGC AACTCTGGCT CACAGTACGC
2281 GTAGTGCAAC CGAACGCGAC CGCATGGTCA GAAGCCGGGC ACATCAGCGC CTGGCAGCAG
2341 TGGCGTCTGG CGGAAAACCT CAGTGTGACG CTCCCCGCCG CGTCCCACGC CATCCCGCAT
2401 CTGACCACCA GCGAAATGGA TTTTTGCATC GAGCTGGGTA ATAAGCGTTG GCAATTTAAC
2461 CGCCAGTCAG GCTTTCTTTC ACAGATGTGG ATTGGCGATA AAAAACAACT GCTGACGCCG
2521 CTGCGCGATC AGTTCACCCG TGCACCGCTG GATAACGACA TTGGCGTAAG TGAAGCGACC

2581 CGCATTGACC CTAACGCCTG GGTCGAACGC TGGAAGGCGG CGGGCCATTA CCAGGCCGAA
2641 GCAGCGTTGT TGCAGTGCAC GGCAGATACA CTTGCTGATG CGGTGCTGAT TACGACCGCT
2701 CACGCGTGGC AGCATCAGGG GAAAACCTTA TTTATCAGCC GGAAAACCTA CCGGATTGAT
2761 GGTAGTGGTC AAATGGCGAT TACCGTTGAT GTTGAAGTGG CGAGCGATAC ACCGCATCCG
2821 GCGCGGATTG GCCTGAACTG CCAGCTGGCG CAGGTAGCAG AGCGGGTAAA CTGGCTCGGA
2881 TTAGGGCCGC AAGAAAACTA TCCCGACCGC CTTACTGCCG CCTGTTTTGA CCGCTGGGAT
2941 CTGCCATTGT CAGACATGTA TACCCCGTAC GTCTTCCCGA GCGAAAACGG TCTGCGCTGC
3001 GGGACGCGCG AATTGAATTA TGGCCCACAC CAGTGGCGCG GCGACTTCCA GTTCAACATC
3061 AGCCGCTACA GTCAACAGCA ACTGATGGAA ACCAGCCATC GCCATCTGCT GCACGCGGAA
3121 GAAGGCACAT GGCTGAATAT CGACGGTTTC CATATGGGGA TTGGTGGCGA CGACTCCTGG
3181 AGCCCGTCAG TATCGGCGGA ATTCCAGCTG AGCGCCGGTC GCTACCATTA CCAGTTGGTC

Stop Stem-loop XbaI
3241 TGGTGTCAAA AATAATAACGCCCTCAT CCGAAAGGGC GTCTAGA

### SEQ ID NO.: 266

### pMPX-74 MalE (1-28) fusion vector

SD old PstI +1
2401 GAATTCAGGCGCTTTTTAGACTGGTCGTAATGAAATTCAGGAGGTTCTGCATATGAAAAT
1 M K I

2461 AAAAACAGGTGCACGCATCCTCGCATTATCCGCATTAACGACGATGATGTTTTCCGCCTC
4 K T G A R I L A L S A L T T M M F S A S

Factor Xa PstI SalI XbaI
2521 GGCTCTCGCCAAAATCATCGAAGCCCGCCTGCAGGCCTCGGTCGACGCCGAATCTAGAGA
24 A L A K I I E A R L Q A S V D A E S R D

FLAG lost XbaI
2581 TTATAAAGATGACGATGACAAATAATAAGCTAGAGG (transcriptional stop)
44 Y K D D D D K

### pMPX-72::malE(1-28)::FXa::Pstl, Sall, Xbal::FLAG

### Rhamnose inducible, clone into Pstl, Sall, Xbal

Made by cutting TOPO Nsil-malE (1-28)::FXa::Pstl, Sall, Xbal::FLAG-Nhel insertion withNsil & Nhel and cloning into pMPX-72 cut with Pstl & Xbal.

### SEQ ID NO.: 267

### pMPX-75 MalE (1-28) fusion vector

SD  old PstI +1
1621   CCATACCCGTTTTTTGGGCTAGCAGGAGGCCCTGCATATGAAAATAAAAACAGGTGCAC
1                                    M  K  I  K  T  G  A

1681   GCATCCTCGCATTATCCGCATTAACGACGATGATGTTTTCCGCCTCGGCTCTCGCCAAAA
8      R  I  L  A  L  S  A  L  T  T  M  M  F  S  A  S  A  L  A  K

              Factor Xa   PstI              SalI              XbaI              FLAG
1741   TCATCGAAGCCCGCCTGCAGGCCTCGGTCGACGCCGAATCTAGAGATTATAAAGATGACG

                    Lost XbaI
1801   ATGACAAATAATAAGCTAGAGG (Transcriptional stop)

### pMPX-71::malE(1-28)::FXa::Pstl, Sall, Xbal::FLAG

### Arabinose inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-71 cut with Pstl & Xbal.

### SEQ ID NO.: 268

### pMPX-88 MalE (1-28) fusion vector

SD old PstI +1
AGGAGGTTCTGCATATGAAAAT
1 M K I
AAAAACAGGTGCACGCATCCTCGCATTATCCGCATTAACGACGATGATGTTTTCCGCCTC
4 K T G A R I L A L S A L T T M M F S A S
Factor Xa PstI SalI XbaI
GGCTCTCGCCAAAATCATCGAAGCCCGCCTGCAGGCCTCGGTCGACGCCGAATCTAGAGA
24 A L A K I I E A R L Q A S V D A E S R D
FLAG lost XbaI
TTATAAAGATGACGATGACAAATAATAAGCTAGAGGTACC (transcriptional stop)
44 Y K D D D D K

### pMPX-84::malE(1-28)::FXa::Pstl, Sall, Xbal::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-84 cut with Pstl & Xbal.

### SEQ ID NO.: 269

### pMPX-93 MalE (1-28) fusion vector

SD old PstI +1
AGGAGGTTCTGCATATGAAAAT
1 M K I
AAAAACAGGTGCACGCATCCTCGCATTATCCGCATTAACGACGATGATGTTTTCCGCCTC
4 K T G A R I L A L S A L T T M M F S A S
Factor Xa PstI SalI XbaI
GGCTCTCGCCAAAATCATCGAAGCCCGCCTGCAGGCCTCGGTCGACGCCGAATCTAGAGA
24 A L A K I I E A R L Q A S V D A E S R D
FLAG lost XbaI
TTATAAAGATGACGATGACAAATAATAAGCTAGAGGTACC (transcriptional stop)
44 Y K D D D D K

### pMPX-86::malE(1-28)::FXa::Pstl, Sall, Xbal::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Nhel and cloning into pMPX-86 cut with Pstl & Xbal.

### SEQ ID NO.: 270

### pMPX-77 MalE (1-370 del 354-364) fusion vector

SD        old PstI  +1
2401   GAATTCAGGCGCTTTTTAGACTGGTCGTAATGAAATTCAGGAGGTTCTGCATATGAAAAT
1                                                    M  K  I

2461   AAAAACAGGTGCACGCATCCTCGCATTATCCGCATTAACGACGATGATGTTTTCCGCCTC
4      K  T  G  A  R  I  L  A  L  S  A  L  T  T  M  M  F  S  A  S

2521   GGCTCTCGCCAAAATCGAAGAAGGTAAACTGGTAATCTGGATTAACGGCGATAAAGGCTA
24     A  L  A  K  I  E  E  G  K  L  V  I  W  I  N  G  D  K  G  Y

2581   TAACGGTCTCGCTGAAGTCGGTAAGAAATTCGAGAAAGATACCGGAATTAAAGTCACCGT
44     N  G  L  A  E  V  G  K  K  F  E  K  D  T  G  I  K  V  T  V

2641   TGAGCATCCGGATAAACTGGAAGAGAAATTCCCACAGGTTGCGGCAACTGGCGATGGCCC
64     E  H  P  D  K  L  E  E  K  F  P  Q  V  A  A  T  G  D  G  P

2701   TGACATTATCTTCTGGGCACACGACCGCTTTGGTGGCTACGCTCAATCTGGCCTGTTGGC
84     D  I  I  F  W  A  H  D  R  F  G  G  Y  A  Q  S  G  L  L  A

2761   TGAAATCACCCCGGACAAAGCGTTCCAGGACAAGCTGTATCCGTTTACCTGGGATGCCGT
104    E  I  T  P  D  K  A  F  Q  D  K  L  Y  P  F  T  W  D  A  V

2821   ACGTTACAACGGCAAGCTGATTGCTTACCCGATCGCTGTTGAAGCGTTATCGCTGATTTA
124    R  Y  N  G  K  L  I  A  Y  P  I  A  V  E  A  L  S  L  I  Y

2881   TAACAAAGATCTGCTGCCGAACCCGCCAAAAACCTGGGAAGAGATCCCGGCGCTGGATAA
144    N  K  D  L  L  P  N  P  P  K  T  W  E  E  I  P  A  L  D  K

2941   AGAACTGAAAGCGAAAGGTAAGAGCGCGCTGATGTTCAACCTGCAAGAACCGTACTTCAC
164    E  L  K  A  K  G  K  S  A  L  M  F  N  L  Q  E  P  Y  F  T

3001   CTGGCCGCTGATTGCTGCTGACGGGGGTTATGCGTTCAAGTATGAAAACGGCAAGTACGA
184    W  P  L  I  A  A  D  G  G  Y  A  F  K  Y  E  N  G  K  Y  D

3061   CATTAAAGACGTGGGCGTGGATAACGCTGGCGCGAAAGCGGGTCTGACCTTCCTGGTTGA
204    I  K  D  V  G  V  D  N  A  G  A  K  A  G  L  T  F  L  V  D

3121   CCTGATTAAAAACAAACACATGAATGCAGACACCGATTACTCCATCGCAGAAGCTGCCTT
224    L  I  K  N  K  H  M  N  A  D  T  D  Y  S  I  A  E  A  A  F

3181   TAATAAAGGCGAAACAGCGATGACCATCAACGGCCCGTGGGCATGGTCCAACATCGACAC
244    N  K  G  E  T  A  M  T  I  N  G  P  W  A  W  S  N  I  D  T

3241   CAGCAAAGTGAATTATGGTGTAACGGTACTGCCGACCTTCAAGGGTCAACCATCCAAACC
264    S  K  V  N  Y  G  V  T  V  L  P  T  F  K  G  Q  P  S  K  P

3301   GTTCGTTGGCGTGCTGAGCGCAGGTATTAACGCCGCCAGTCCGAACAAAGAGCTGGCGAA
284    F  V  G  V  L  S  A  G  I  N  A  A  S  P  N  K  E  L  A  K

3361 AGAGTTCCTCGAAAACTATCTGCTGACTGATGAAGGTCTGGAAGCGGTTAATAAAGACAA
304 E F L E N Y L L T D E G L E A V N K D K

3421 ACCGCTGGGTGCCGTAGCGCTGAAGTCTTACGAGGAAGAGTTGGCGAAAGATCCACGTAT
324 P L G A V A L K S Y E E E L A K D P R I

### pMPX-72::malE(1-370 del 354-364)::FXa::PstI, Sall, Xbal::FLAG

### Rhamnose inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-72 cut with Pstl & Xbal.

### SEO ID NO.: 271

### pMPX-76 MalE (1-370 del 354-364) fusion vector

SD  old PstI +1
1621   CCATACCCGTTTTTTTGGGCTAGCAGGAGGCCCTGCATATGAAAATAAAAACAGGTGCAC
1                                    M  K  I  K  T  G  A

1681   GCATCCTCGCATTATCCGCATTAACGACGATGATGTTTTCCGCCTCGGCTCTCGCCAAAA
8      R  I  L  A  L  S  A  L  T  T  M  M  F  S  A  S  A  L  A  K

1741   TCGAAGAAGGTAAACTGGTAATCTGGATTAACGGCGATAAAGGCTATAACGGTCTCGCTG
28     I  E  E  G  K  L  V  I  W  I  N  G  D  K  G  Y  N  G  L  A

1801   AAGTCGGTAAGAAATTCGAGAAAGATACCGGAATTAAAGTCACCGTTGAGCATCCGGATA
48     E  V  G  K  K  F  E  K  D  T  G  I  K  V  T  V  E  H  P  D

1861   AACTGGAAGAGAAATTCCCACAGGTTGCGGCAACTGGCGATGGCCCTGACATTATCTTCT
68     K  L  E  E  K  F  P  Q  V  A  A  T  G  D  G  P  D  I  I  F

1921   GGGCACACGACCGCTTTGGTGGCTACGCTCAATCTGGCCTGTTGGCTGAAATCACCCCGG
88     W  A  H  D  R  F  G  G  Y  A  Q  S  G  L  L  A  E  I  T  P

1981   ACAAAGCGTTCCAGGACAAGCTGTATCCGTTTACCTGGGATGCCGTACGTTACAACGGCA
108    D  K  A  F  Q  D  K  L  Y  P  F  T  W  D  A  V  R  Y  N  G

2041   AGCTGATTGCTTACCCGATCGCTGTTGAAGCGTTATCGCTGATTTATAACAAAGATCTGC
128    K  L  I  A  Y  P  I  A  V  E  A  L  S  L  I  Y  N  K  D  L

2101   TGCCGAACCCGCCAAAAACCTGGGAAGAGATCCCGGCGCTGGATAAAGAACTGAAAGCGA
148    L  P  N  P  P  K  T  W  E  E  I  P  A  L  D  K  E  L  K  A

2161   AAGGTAAGAGCGCGCTGATGTTCAACCTGCAAGAACCGTACTTCACCTGGCCGCTGATTG
168    K  G  K  S  A  L  M  F  N  L  Q  E  P  Y  F  T  W  P  L  I

2221   CTGCTGACGGGGGTTATGCGTTCAAGTATGAAAACGGCAAGTACGACATTAAAGACGTGG
188    A  A  D  G  G  Y  A  F  K  Y  E  N  G  K  Y  D  I  K  D  V

2281   GCGTGGATAACGCTGGCGCGAAAGCGGGTCTGACCTTCCTGGTTGACCTGATTAAAAACA
208    G  V  D  N  A  G  A  K  A  G  L  T  F  L  V  D  L  I  K  N

2341   AACACATGAATGCAGACACCGATTACTCCATCGCAGAAGCTGCCTTTAATAAAGGCGAAA
228    K  H  M  N  A  D  T  D  Y  S  I  A  E  A  A  F  N  K  G  E

2401   CAGCGATGACCATCAACGGCCCGTGGGCATGGTCCAACATCGACACCAGCAAAGTGAATT
248    T  A  M  T  I  N  G  P  W  A  W  S  N  I  D  T  S  K  V  N

2461 ATGGTGTAACGGTACTGCCGACCTTCAAGGGTCAACCATCCAAACCGTTCGTTGGCGTGC
268 Y G V T V L P T F K G Q P S K P F V G V

2521 TGAGCGCAGGTATTAACGCCGCCAGTCCGAACAAAGAGCTGGCGAAAGAGTTCCTCGAAA
288 L S A G I N A A S P N K E L A K E F L E

2581 ACTATCTGCTGACTGATGAAGGTCTGGAAGCGGTTAATAAAGACAAACCGCTGGGTGCCG
308 N Y L L T D E G L E A V N K D K P L G A

2641 TAGCGCTGAAGTCTTACGAGGAAGAGTTGGCGAAAGATCCACGTATTGCCGCCACCATGG
328 V A L K S Y E E E L A K D P R I A A T M

Factor Xa PstI
2701 AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI FLAG Lost XbaI
2761 TCGACGCCGAATCTAGAGATTATAAAGATGACGATGACAAATAATAAGCTAGAGGA(trxn stop)
368 V D A E S R D Y K D D D D K

### pMPX-71::malE(1-370 del 354-364)::FXa::Pstl, Sall, Xbal::FLAG

### Arabinose inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-71 cut with Pstl & Xbal.

### SEQ ID NO.: 272

### pMPX-89 MalE (1-370 del 354-364) fusion vector

SD old PstI +1
AGGAGGTTCTGCATATGAAAATAAAAACAGGTGCAC
1 M K I K T G A

GCATCCTCGCATTATCCGCATTAACGACGATGATGTTTTCCGCCTCGGCTCTCGCCAAAA
8 R I L A L S A L T T M M F S A S A L A K

TCGAAGAAGGTAAACTGGTAATCTGGATTAACGGCGATAAAGGCTATAACGGTCTCGCTG
28 I E E G K L V I W I N G D K G Y N G L A

AAGTCGGTAAGAAATTCGAGAAAGATACCGGAATTAAAGTCACCGTTGAGCATCCGGATA
48 E V G K K F E K D T G I K V T V E H P D

AACTGGAAGAGAAATTCCCACAGGTTGCGGCAACTGGCGATGGCCCTGACATTATCTTCT
68 K L E E K F P Q V A A T G D G P D I I F

GGGCACACGACCGCTTTGGTGGCTACGCTCAATCTGGCCTGTTGGCTGAAATCACCCCGG
88 W A H D R F G G Y A Q S G L L A E I T P

ACAAAGCGTTCCAGGACAAGCTGTATCCGTTTACCTGGGATGCCGTACGTTACAACGGCA
108 D K A F Q D K L Y P F T W D A V R Y N G

AGCTGATTGCTTACCCGATCGCTGTTGAAGCGTTATCGCTGATTTATAACAAAGATCTGC
128 K L I A Y P I A V E A L S L I Y N K D L

TGCCGAACCCGCCAAAAACCTGGGAAGAGATCCCGGCGCTGGATAAAGAACTGAAAGCGA
148 L P N P P K T W E E I P A L D K E L K A

AAGGTAAGAGCGCGCTGATGTTCAACCTGCAAGAACCGTACTTCACCTGGCCGCTGATTG
168 K G K S A L M F N L Q E P Y F T W P L I

CTGCTGACGGGGGTTATGCGTTCAAGTATGAAAACGGCAAGTACGACATTAAAGACGTGG
188 A A D G G Y A F K Y E N G K Y D I K D V

GCGTGGATAACGCTGGCGCGAAAGCGGGTCTGACCTTCCTGGTTGACCTGATTAAAAACA
208 G V D N A G A K A G L T F L V D L I K N

AACACATGAATGCAGACACCGATTACTCCATCGCAGAAGCTGCCTTTAATAAAGGCGAAA
228 K H M N A D T D Y S I A E A A F N K G E

CAGCGATGACCATCAACGGCCCGTGGGCATGGTCCAACATCGACACCAGCAAAGTGAATT
248 T A M T I N G P W A W S N I D T S K V N

ATGGTGTAACGGTACTGCCGACCTTCAAGGGTCAACCATCCAAACCGTTCGTTGGCGTGC
268 Y G V T V L P T F K G Q P S K P F V G V

TGAGCGCAGGTATTAACGCCGCCAGTCCGAACAAAGAGCTGGCGAAAGAGTTCCTCGAAA
288 L S A G I N A A S P N K E L A K E F L E

ACTATCTGCTGACTGATGAAGGTCTGGAAGCGGTTAATAAAGACAAACCGCTGGGTGCCG
308 N Y L L T D E G L E A V N K D K P L G A

TAGCGCTGAAGTCTTACGAGGAAGAGTTGGCGAAAGATCCACGTATTGCCGCCACCATGG
328 V A L K S Y E E E L A K D P R I A A T M

Factor Xa PstI
AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI FLAG Lost XbaI
TCGACGCCGAATCTAGAGATTATAAAGATGACGATGACAAATAATAAGCTAGAGG (trxn stop)
368 V D A E S R D Y K D D D D K

### pMPX-84::malE(1-370 del 354-364)::FXa::Pstl, Sall, Xbal::FLAG

### Temperature inducible, clone into Pstl, Sall, Xbal

Made by cutting TOPO Nsil-malE (1-370 del 354-364)::FXa::Pstl, Sall, Xbal::FLAG-Nhel insertion with Nsil & Nhel and cloning into pMPX-84 cut with Pstl & Xbal.

### SEQ ID NO.: 273

### pMPX-94 MalE (1-370 del 354-364) fusion vector

SD old PstI +1
AGGAGGTTCTGCATATGAAAATAAAAACAGGTGCAC
1 M K I K T G A
GCATCCTCGCATTATCCGCATTAACGACGATGATGTTTTCCGCCTCGGCTCTCGCCAAAA
8 R I L A L S A L T T M M F S A S A L A K
TCGAAGAAGGTAAACTGGTAATCTGGATTAACGGCGATAAAGGCTATAACGGTCTCGCTG
28 I E E G K L V I W I N G D K G Y N G L A
AAGTCGGTAAGAAATTCGAGAAAGATACCGGAATTAAAGTCACCGTTGAGCATCCGGATA
48 E V G K K F E K D T G I K V T V E H P D

AACTGGAAGAGAAATTCCCACAGGTTGCGGCAACTGGCGATGGCCCTGACATTATCTTCT
68 K L E E K F P Q V A A T G D G P D I I F

GGGCACACGACCGCTTTGGTGGCTACGCTCAATCTGGCCTGTTGGCTGAAATCACCCCGG
88 W A H D R F G G Y A Q S G L L A E I T P

ACAAAGCGTTCCAGGACAAGCTGTATCCGTTTACCTGGGATGCCGTACGTTACAACGGCA
108 D K A F Q D K L Y P F T W D A V R Y N G

AGCTGATTGCTTACCCGATCGCTGTTGAAGCGTTATCGCTGATTTATAACAAAGATCTGC
128 K L I A Y P I A V E A L S L I Y N K D L

TGCCGAACCCGCCAAAAACCTGGGAAGAGATCCCGGCGCTGGATAAAGAACTGAAAGCGA
148 L P N P P K T W E E I P A L D K E L K A

AAGGTAAGAGCGCGCTGATGTTCAACCTGCAAGAACCGTACTTCACCTGGCCGCTGATTG
168 K G K S A L M F N L Q E P Y F T W P L I

CTGCTGACGGGGGTTATGCGTTCAAGTATGAAAACGGCAAGTACGACATTAAAGACGTGG
188 A A D G G Y A F K Y E N G K Y D I K D V

GCGTGGATAACGCTGGCGCGAAAGCGGGTCTGACCTTCCTGGTTGACCTGATTAAAAACA
208 G V D N A G A K A G L T F L V D L I K N

AACACATGAATGCAGACACCGATTACTCCATCGCAGAAGCTGCCTTTAATAAAGGCGAAA
228 K H M N A D T D Y S I A E A A F N K G E

CAGCGATGACCATCAACGGCCCGTGGGCATGGTCCAACATCGACACCAGCAAAGTGAATT
248 T A M T I N G P W A W S N I D T S K V N

ATGGTGTAACGGTACTGCCGACCTTCAAGGGTCAACCATCCAAACCGTTCGTTGGCGTGC
268 Y G V T V L P T F K G Q P S K P F V G V

TGAGCGCAGGTATTAACGCCGCCAGTCCGAACAAAGAGCTGGCGAAAGAGTTCCTCGAAA
288 L S A G I N A A S P N K E L A K E F L E

ACTATCTGCTGACTGATGAAGGTCTGGAAGCGGTTAATAAAGACAAACCGCTGGGTGCCG
308 N Y L L T D E G L E A V N K D K P L G A

TAGCGCTGAAGTCTTACGAGGAAGAGTTGGCGAAAGATCCACGTATTGCCGCCACCATGG
328 V A L K S Y E E E L A K D P R I A A T M

Factor Xa PstI
AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI FLAG Lost XbaI
TCGACGCCGAATCTAGAGATTATAAAGATGACGATGACAAATAATAAGCTAGAGG (trxn stop)
368 V D A E S R D Y K D D D D K

### pMPX-86::malE(1-370 del 354-364)::FXa::Pstl, Sall, Xbal::FLAG

### Temperature inducible, clone into Pstl, Sall, Xbal

Made by cutting TOPO NsiI-malE (1-370 del 354-364)::FXa::Pstl, Sall, Xbal::FLAG-Nhel insertion with Nsil & Nhel and cloning into pMPX-86 cut with Pstl & Xbal.

### SEQ ID NO.: 274

### pMPX-79 TrxA (2-109 del 103-107) fusion vector

SD PstI SalI XbaI +2 trxA(del 103-107)
1 TAGCAGGAGGCCCTGCAGGCCTCGGTCGACGCCGAATCTAGAAGCGATAAAATTATT
1 A S V D A E S R S D K I I

61 CACCTGACTGACGACAGTTTTGACACGGATGTACTCAAAGCGGACGGGGCGATCCTCGTC
17 H L T D D S F D T D V L K A D G A I L V

121 GATTTCTGGGCAGAGTGGTGCGGTCCGTGCAAAATGATCGCCCCGATTCTGGATGAAATC
37 D F W A E W C G P C K M I A P I L D E I

181 GCTGACGAATATCAGGGCAAACTGACCGTTGCAAAACTGAACATCGATCAAAACCCTGGC
57 A D E Y Q G K L T V A K L N I D Q N P G

241 ACTGCGCCGAAATATGGCATCCGTGGTATCCCGACTCTGCTGCTGTTCAAAAACGGTGAA
77 T A P K Y G I R G I P T L L L F K N G E

301 GTGGCGGCAACCAAAGTGGGTGCACTGTCTAAAGGTCAGTTGAAAGAGAACCTGGCGGAT
97 V A A T K V G A L S K G Q L K E N L A D

FLAG Lost XbaI
361 TATAAAGATGACGATGACAAATAATAAGCTAGAGG (transcriptional stop)
117 Y K D D D D K

### pMPX-71::Pstl, Sall, Xbal::trxA (2-109 del 103-107)::FLAG

### Arabinose inducible, clone into Pstl, SalI, XbaI

+1 Met required for protein to be fused

Made by cutting TOPO Pstl, Sall, Xbal::trxA (2-109 del 103-107)::FLAG-Nhel insertion with Pstl & Nhel and cloning into pMPX-71 cut with Pstl & Xbal.

### SEQ ID NO.: 275

### pMPX-78 TrxA (2-109 del 103-107) fusion vector

SD PstI
1 GAATTCAGGCGCTTTTTAGACTGGTCGTAATGAAATTCAGGAGGTTCTGCAGGCCTC
1 A S

SalI XbaI +2 trxA(del 103-107)
61 GGTCGACGCCGAATCTAGAAGCGATAAAATTATTCACCTGACTGACGACAGTTTTGACAC
6 V D A E S R S D K I I H L T D D S F D T

121 GGATGTACTCAAAGCGGACGGGGCGATCCTCGTCGATTTCTGGGCAGAGTGGTGCGGTCC
26 D V L K A D G A I L V D F W A E W C G P

181 GTGCAAAATGATCGCCCCGATTCTGGATGAAATCGCTGACGAATATCAGGGCAAACTGAC
46 C K M I A P I L D E I A D E Y Q G K L T

241 CGTTGCAAAACTGAACATCGATCAAAACCCTGGCACTGCGCCGAAATATGGCATCCGTGG
66 V A K L N I D Q N P G T A P K Y G I R G

301 TATCCCGACTCTGCTGCTGTTCAAAAACGGTGAAGTGGCGGCAACCAAAGTGGGTGCACT
86 I P T L L L F K N G E V A A T K V G A L

FLAG
361 GTCTAAAGGTCAGTTGAAAGAGAACCTGGCGGATTATAAAGATGACGATGACAAATAATAA
106 S K G Q L K E N L A D Y K D D D D K

lost XbaI
GCTAGAGG (transcriptional stop)

### pMPX-72::Pstl, Sall, Xbal::trxA (2-109 del 103-107)::FLAG

### Rhamnose inducible, clone into PstI, Sall, Xbal

+1 Met required for protein to be fused

Made by cutting TOPO Pstl, Sall, Xbal::trxA (2-109 del 103-107)::FLAG-Nhel insertion with Pstl & Nhel and cloning into pMPX-72 cut with Pstl & Xbal.

### SEQ ID NO.: 276

### pMPX-90 TrxA (2-109 del 103-107) fusion vector

SD PstI SalI XbaI +2 trxA(del 103-107)
AGGAGGTTCTGCAGGCCTCGGTCGACGCCGAATCTAGAAGCGATAAAATTATT
1 A S V D A E S R S D K I I

CACCTGACTGACGACAGTTTTGACACGGATGTACTCAAAGCGGACGGGGCGATCCTCGTC
17 H L T D D S F D T D V L K A D G A I L V

GATTTCTGGGCAGAGTGGTGCGGTCCGTGCAAAATGATCGCCCCGATTCTGGATGAAATC
37 D F W A E W C G P C K M I A P I L D E I

GCTGACGAATATCAGGGCAAACTGACCGTTGCAAAACTGAACATCGATCAAAACCCTGGC
57 A D E Y Q G K L T V A K L N I D Q N P G

ACTGCGCCGAAATATGGCATCCGTGGTATCCCGACTCTGCTGCTGTTCAAAAACGGTGAA
77 T A P K Y G I R G I P T L L L F K N G E

GTGGCGGCAACCAAAGTGGGTGCACTGTCTAAAGGTCAGTTGAAAGAGAACCTGGCGGAT
97 V A A T K V G A L S K G Q L K E N L A D

FLAG Lost XbaI
TATAAAGATGACGATGACAAATAATAAGCTAGAGGTACC (transcriptional stop)
117 Y K D D D D K

### pMPX-84::Pstl, Sall, Xbal::trxA (2-109 del 103-107)::FLAG

### Temperature inducible, clone into Pstl, SalI, Xbal

+1 Met required for protein to be fused

Made by cutting TOPO Pstl, Sall, Xbal::trxA (2-109 del 103-107)::FLAG-Nhel insertion with Pstl & Nhel and cloning into pMPX-84 cut with Pstl & Xbal.

### SEQ ID NO.: 277

### pMPX-95 TrxA (2-109 del 103-107) fusion vector

SD      PstI          SalI          XbaI  +2 trxA(del 103-107)
AGGAGGTTCTGCAGGCCTCGGTCGACGCCGAATCTAGAAGCGATAAAATTATT
1          A S V D A E S R S D K I I

CACCTGACTGACGACAGTTTTGACACGGATGTACTCAAAGCGGACGGGGCGATCCTCGTC
17 H L T D D S F D T D V L K A D G A I L V

GATTTCTGGGCAGAGTGGTGCGGTCCGTGCAAAATGATCGCCCCGATTCTGGATGAAATC
37 D F W A E W C G P C K M I A P I L D E I

GCTGACGAATATCAGGGCAAACTGACCGTTGCAAAACTGAACATCGATCAAAACCCTGGC
57 A D E Y Q G K L T V A K L N I D Q N P G

ACTGCGCCGAAATATGGCATCCGTGGTATCCCGACTCTGCTGCTGTTCAAAAACGGTGAA
77 T A P K Y G I R G I P T L L L F K N G E

GTGGCGGCAACCAAAGTGGGTGCACTGTCTAAAGGTCAGTTGAAAGAGAACCTGGCGGAT
97 V A A T K V G A L S K G Q L K E N L A D

FLAG Lost XbaI
TATAAAGATGACGATGACAAATAATAAGCTAGAGGTACC (transcriptional stop)
117 Y K D D D D K

### pMPX-86::Pstl, Sall, Xbal::trxA (2-109 del 103-107)::FLAG

### Temperature inducible, clone into Pstl, Sall, Xbal

+1 Met required for protein to be fused

Made by cutting TOPO PstI, Sall, Xbal::trxA (2-109 del 103-107)::FLAG-Nhel insertion with Pstl & Nhel and cloning into pMPX-86 cut with Pstl & Xbal.

### SEQ ID NO.: 278

### pMPX-80 MalE (1-28) MCS TrxA (2-109 del 103-107) fusion vector

SD  Lost PstI +1 malE(1-28)
2401   GAATTCAGGCGCTTTTTAGACTGGTCGTAATGAAATTCAGGAGGTTCTGCATATGAAAAT
1                                                    M  K  I

2461   AAAAACAGGTGCACGCATCCTCGCATTATCCGCATTAACGACGATGATGTTTTCCGCCTC
4       K  T  G  A  R  I  L  A  L  S  A  L  T  T  M  M  F  S  A  S

                                    Factor Xa   PstI      SalI        XbaI
2521   GGCTCTCGCCAAAATCATCGAAGCCCGCCTGCAGGCCTCGGTCGACGCCGAATCTAGAAG
24      A  L  A  K  I  I  E  A  R  L  Q  A  S  V  D  A  E  S  R  S

       +2 trxA (2-109 del 103-107)
2581   CGATAAAATTATTCACCTGACTGACGACAGTTTTGACACGGATGTACTCAAAGCGGACGG
44      D  K  I  I  H  L  T  D  D  S  F  D  T  D  V  L  K  A  D  G

2641   GGCGATCCTCGTCGATTTCTGGGCAGAGTGGTGCGGTCCGTGCAAAATGATCGCCCCGAT
64      A  I  L  V  D  F  W  A  E  W  C  G  P  C  K  M  I  A  P  I

2701   TCTGGATGAAATCGCTGACGAATATCAGGGCAAACTGACCGTTGCAAAACTGAACATCGA
84      L  D  E  I  A  D  E  Y  Q  G  K  L  T  V  A  K  L  N  I  D

2761   TCAAAACCCTGGCACTGCGCCGAAATATGGCATCCGTGGTATCCCGACTCTGCTGCTGTT
104     Q  N  P  G  T  A  P  K  Y  G  I  R  G  I  P  T  L  L  L  F

2821   CAAAAACGGTGAAGTGGCGGCAACCAAAGTGGGTGCACTGTCTAAAGGTCAGTTGAAAGA
124     K  N  G  E  V  A  A  T  K  V  G  A  L  S  K  G  Q  L  K  E

FLAG                    Lost XbaI
2881 GAACCTGGCGGATTATAAAGATGACGATGACAAATAATAAGCTAGAGG (trxn stop)
144 N L A D Y K D D D D K

### pMPX-72::malE(1-28)::FXa::Pstl, Sall, Xbal::TrxA(1-109 del 103-107)::FLAG

### Rhamnose inducible, clone into Pstl, SalI, Xbal

Made by cutting TOPO NsiI-malE (1-28)::FXa::Pstl, Sall, Xbal::FLAG-Nhel insertion with Nsil & Xbal and cloning into pMPX-78 cut with Pstl & Xbal.

### SEQ ID NO.: 279

### pMPX-81 MalE (1-28) MCS TrxA (2-109 del 103-107) fusion vector

SD  Lost PstI +1 malE (1-28)
1621  CCATACCCGTTTTTTTGGGCTAGCAGGAGGCCCTGCATATGAAAATAAAAACAGGTGCAC
1  M K I K T G A

1681  GCATCCTCGCATTATCCGCATTAACGACGATGATGTTTTCCGCCTCGGCTCTCGCCAAAA
8  R I L A L S A L T T M M F S A S A L A K

+2 trxA(2-109 del
Factor Xa  PstI  SalI  XbaI  103-107)
1741  TCATCGAAGCCCGCCTGCAGGCCTCGGTCGACGCCGAATCTAGAAGCGATAAAATTATTC
28  I I E A R L Q A S V D A E S R S D K I I

1801  ACCTGACTGACGACAGTTTTGACACGGATGTACTCAAAGCGGACGGGGCGATCCTCGTCG
48  H L T D D S F D T D V L K A D G A I L V

1861  ATTTCTGGGCAGAGTGGTGCGGTCCGTGCAAAATGATCGCCCCGATTCTGGATGAAATCG
68  D F W A E W C G P C K M I A P I L D E I

1921  CTGACGAATATCAGGGCAAACTGACCGTTGCAAAACTGAACATCGATCAAAACCCTGGCA
88  A D E Y Q G K L T V A K L N I D Q N P G

1981  CTGCGCCGAAATATGGCATCCGTGGTATCCCGACTCTGCTGCTGTTCAAAAACGGTGAAG
108  T A P K Y G I R G I P T L L L F K N G E

2041  TGGCGGCAACCAAAGTGGGTGCACTGTCTAAAGGTCAGTTGAAAGAGAACCTGGCGGATT
128  V A A T K V G A L S K G Q L K E N L A D

FLAG
2101  ATAAAGATGACGATGACAAATAATAAGCTAGAGG (transcriptional stop)
148  Y K D D D D K

### pMPX-71::malE(1-28)::FXa::Pstl, Sall, XbaI::TrxA(1-109 del 103-107)::FLAG

### Arabinose inducible, clone into Pstl, Sall, Xbal

Made by cutting TOPO Nsil-malE (1-28)::FXa::Pstl, Sall, XbaI::FLAG-NheI insertion with Nsil & Xbal and cloning into pMPX-79 cut with Pstl & Xbal.

### SEQ ID NO.: 280

### pMPX-91 MalE (1-28) MCS TrxA (2-109 del 103-107) fusion vector

SD  Lost PstI +1 malE(1-28)
AGGAGGTTCTGCATATGAAAAT
1                    M K I

AAAAACAGGTGCACGCATCCTCGCATTATCCGCATTAACGACGATGATGTTTTCCGCCTC
4 K T G A R I L A L S A L T T M M F S A S

Factor Xa PstI SalI XbaI
GGCTCTCGCCAAAATCATCGAAGCCCGCCTGCAGGCCTCGGTCGACGCCGAATCTAGAAG
24 A L A K I I E A R L Q A S V D A E S R S

+2 trxA (2-109 del 103-107)

CGATAAAATTATTCACCTGACTGACGACAGTTTTGACACGGATGTACTCAAAGCGGACGG
44 D K I I H L T D D S F D T D V L K A D G

GGCGATCCTCGTCGATTTCTGGGCAGAGTGGTGCGGTCCGTGCAAAATGATCGCCCCGAT
64 A I L V D F W A E W C G P C K M I A P I

TCTGGATGAAATCGCTGACGAATATCAGGGCAAACTGACCGTTGCAAAACTGAACATCGA
84 L D E I A D E Y Q G K L T V A K L N I D

TCAAAACCCTGGCACTGCGCCGAAATATGGCATCCGTGGTATCCCGACTCTGCTGCTGTT
104 Q N P G T A P K Y G I R G I P T L L L F

CAAAAACGGTGAAGTGGCGGCAACCAAAGTGGGTGCACTGTCTAAAGGTCAGTTGAAAGA
124 K N G E V A A T K V G A L S K G Q L K E

FLAG Lost XbaI
GAACCTGGCGGATTATAAAGATGACGATGACAAATAATAAGCTAGAGGTACC (trxn stop)
144 N L A D Y K D D D D K

### pMPX-84::malE(1-28)::FXa::Pstl, Sall, Xbal::TrxA(1-109 del 103-107)::FLAG

### Temperature inducible, clone into Pstl, Sall, Xbal

Made by cutting TOPO Nsil-malE (1-28)::FXa::Pstl, Sall, Xbal::FLAG-Nhel insertion with Nsil & Xbal and cloning into pMPX-90 cut with Pstl & XbaI.

### SEQ ID NO.: 281

### pMPX-96 MalE (1-28) MCS TrxA (2-109 del 103-107) fusion vector

SD  Lost PstI +1 malE(1-28)
AGGAGGTTCTGCATATGAAAAT
1                                    M K I

AAAAACAGGTGCACGCATCCTCGCATTATCCGCATTAACGACGATGATGTTTTCCGCCTC
4   K T G A R I L A L S A L T T M M F S A S

Factor Xa   PstI      SalI      XbaI
GGCTCTCGCCAAAATCATCGAAGCCCGCCTGCAGGCCTCGGTCGACGCCGAATCTAGAAG
24   A L A K I I E A R L Q A S V D A E S R S

+2 trxA (2-109 del 103-107)
CGATAAAATTATTCACCTGACTGACGACAGTTTTGACACGGATGTACTCAAAGCGGACGG
44   D K I I H L T D D S F D T D V L K A D G

GGCGATCCTCGTCGATTTCTGGGCAGAGTGGTGCGGTCCGTGCAAAATGATCGCCCCGAT
64   A I L V D F W A E W C G P C K M I A P I

TCTGGATGAAATCGCTGACGAATATCAGGGCAAACTGACCGTTGCAAAACTGAACATCGA

84 L D E I A D E Y Q G K L T V A K L N I D

TCAAAACCCTGGCACTGCGCCGAAATATGGCATCCGTGGTATCCCGACTCTGCTGCTGTT
104 Q N P G T A P K Y G I R G I P T L L L F

CAAAAACGGTGAAGTGGCGGCAACCAAAGTGGGTGCACTGTCTAAAGGTCAGTTGAAAGA
124 K N G E V A A T K V G A L S K G Q L K E

FLAG Lost XbaI
GAACCTGGCGGATTATAAAGATGACGATGACAAATAATAAGCTAGAGGTACC (trxn stop)
144 N L A D Y K D D D D K

### pMPX-86::malE(1-28)::FXa::Pstl, Sall, Xbal::TrxA(1-109 del 103-107)::FLAG

### Temperature inducible, clone into Pstl, Sall, Xbal

Made by cutting TOPO Nsil-malE (1-28)::FXa::PstI, SalI, XbaI::FLAG-Nhel insertion with Nsil & Xbal and cloning into pMPX-95 cut with Pstl & Xbal.

### SEQ ID NO.: 282

### pMPX-83 MalE (1-370 del 354-364) MCS TrxA (2-109 del 103-107) fusion vector

SD  Lost PstI +1 malE(1-28)
2401   GAATTCAGGCGCTTTTTAGACTGGTCGTAATGAAATTCAGGAGGTTCTGCATATGAAAAT
1                                                    M K I

2461   AAAAACAGGTGCACGCATCCTCGCATTATCCGCATTAACGACGATGATGTTTTCCGCCTC
4      K T G A R I L A L S A L T T M M F S A S

2521   GGCTCTCGCCAAAATCGAAGAAGGTAAACTGGTAATCTGGATTAACGGCGATAAAGGCTA
24     A L A K I E E G K L V I W I N G D K G Y

2581   TAACGGTCTCGCTGAAGTCGGTAAGAAATTCGAGAAAGATACCGGAATTAAAGTCACCGT
44     N G L A E V G K K F E K D T G I K V T V

2641   TGAGCATCCGGATAAACTGGAAGAGAAATTCCCACAGGTTGCGGCAACTGGCGATGGCCC
64     E H P D K L E E K F P Q V A A T G D G P

2701   TGACATTATCTTCTGGGCACACGACCGCTTTGGTGGCTACGCTCAATCTGGCCTGTTGGC
84     D I I F W A H D R F G G Y A Q S G L L A

2761   TGAAATCACCCCGGACAAAGCGTTCCAGGACAAGCTGTATCCGTTTACCTGGGATGCCGT
104    E I T P D K A F Q D K L Y P F T W D A V

2821   ACGTTACAACGGCAAGCTGATTGCTTACCCGATCGCTGTTGAAGCGTTATCGCTGATTTA
124    R Y N G K L I A Y P I A V E A L S L I Y

2881   TAACAAAGATCTGCTGCCGAACCCGCCAAAAACCTGGGAAGAGATCCCGGCGCTGGATAA
144    N K D L L P N P P K T W E E I P A L D K

2941   AGAACTGAAAGCGAAAGGTAAGAGCGCGCTGATGTTCAACCTGCAAGAACCGTACTTCAC
164    E L K A K G K S A L M F N L Q E P Y F T

3001   CTGGCCGCTGATTGCTGCTGACGGGGGTTATGCGTTCAAGTATGAAAACGGCAAGTACGA
184    W P L I A A D G G Y A F K Y E N G K Y D

3061   CATTAAAGACGTGGGCGTGGATAACGCTGGCGCGAAAGCGGGTCTGACCTTCCTGGTTGA
204    I K D V G V D N A G A K A G L T F L V D

3121 CCTGATTAAAAACAAACACATGAATGCAGACACCGATTACTCCATCGCAGAAGCTGCCTT
224 L I K N K H M N A D T D Y S I A E A A F

3181 TAATAAAGGCGAAACAGCGATGACCATCAACGGCCCGTGGGCATGGTCCAACATCGACAC
244 N K G E T A M T I N G P W A W S N I D T

3241 CAGCAAAGTGAATTATGGTGTAACGGTACTGCCGACCTTCAAGGGTCAACCATCCAAACC
264 S K V N Y G V T V L P T F K G Q P S K P

3301 GTTCGTTGGCGTGCTGAGCGCAGGTATTAACGCCGCCAGTCCGAACAAAGAGCTGGCGAA
284 F V G V L S A G I N A A S P N K E L A K

3361 AGAGTTCCTCGAAAACTATCTGCTGACTGATGAAGGTCTGGAAGCGGTTAATAAAGACAA
304 E F L E N Y L L T D E G L E A V N K D K

3421 ACCGCTGGGTGCCGTAGCGCTGAAGTCTTACGAGGAAGAGTTGGCGAAAGATCCACGTAT
324 P L G A V A L K S Y E E E L A K D P R I

Factor Xa
3481 TGCCGCCACCATGGAAAACGCCCAGTCCGCTTTCTGGTATGCCGTGCGTATCGAAGCCCG
344 A A T M E N A Q S A F W Y A V R I E A R

PstI SalI XbaI +2 trxA (2-109 del 103-107)
3541 CCTGCAGGCCTCGGTCGACGCCGAATCTAGAAGCGATAAAATTATTCACCTGACTGACGA
364 L Q A S V D A E S R S D K I I H L T D D

3601 CAGTTTTGACACGGATGTACTCAAAGCGGACGGGGCGATCCTCGTCGATTTCTGGGCAGA
384 S F D T D V L K A D G A I L V D F W A E

3661 GTGGTGCGGTCCGTGCAAAATGATCGCCCCGATTCTGGATGAAATCGCTGACGAATATCA
404 W C G P C K M I A P I L D E I A D E Y Q

3721 GGGCAAACTGACCGTTGCAAAACTGAACATCGATCAAAACCCTGGCACTGCGCCGAAATA
424 G K L T V A K L N I D Q N P G T A P K Y

3781 TGGCATCCGTGGTATCCCGACTCTGCTGCTGTTCAAAAACGGTGAAGTGGCGGCAACCAA
444 G I R G I P T L L L F K N G E V A A T K

FLAG
3841 AGTGGGTGCACTGTCTAAAGGTCAGTTGAAAGAGAACCTGGCGGATTATAAAGATGACGA
464 V G A L S K G Q L K E N L A D Y K D D D

3901 TGACAAATAATAAGCTAGAGG (transcriptional stop)
484 D K

### pMPX-72::malE(1-320 del 354-364)::FXa::Pstl, SalI, XbaI::TrxA(1-109 del 103-107)::FLAG

### Rhamnose inducible, clone into Pstl, SalI, Xbal

Made by cutting TOPO Nsil-malE (1-370 del 354-364)::FXa::Pstl, SalI, XbaI::FLAG-Nhel insertion with Nsil & Xbal and cloning into pMPX-78 cut with Pstl & Xbal.

### SEQ ID NO.: 283

### pMPX-82 MalE (1-370 del 354-364) MCS TrxA (2-109 del 103-107) fusion vector

SD  Lost PstI +1 malE (1-370 del 352-362)
1621    CCATACCCGTTTTTTTGGGCTAGCAGGAGGCCCTGCATATGAAAATAAAAACAGGTGCAC

1 M K I K T G A

1681 GCATCCTCGCATTATCCGCATTAACGACGATGATGTTTTCCGCCTCGGCTCTCGCCAAAA
8 R I L A L S A L T T M M F S A S A L A K

1741 TCGAAGAAGGTAAACTGGTAATCTGGATTAACGGCGATAAAGGCTATAACGGTCTCGCTG
28 I E E G K L V I W I N G D K G Y N G L A

1801 AAGTCGGTAAGAAATTCGAGAAAGATACCGGAATTAAAGTCACCGTTGAGCATCCGGATA
48 E V G K K F E K D T G I K V T V E H P D

1861 AACTGGAAGAGAAATTCCCACAGGTTGCGGCAACTGGCGATGGCCCTGACATTATCTTCT
68 K L E E K F P Q V A A T G D G P D I I F

1921 GGGCACACGACCGCTTTGGTGGCTACGCTCAATCTGGCCTGTTGGCTGAAATCACCCCGG
88 W A H D R F G G Y A Q S G L L A E I T P

1981 ACAAAGCGTTCCAGGACAAGCTGTATCCGTTTACCTGGGATGCCGTACGTTACAACGGCA
108 D K A F Q D K L Y P F T W D A V R Y N G

2041 AGCTGATTGCTTACCCGATCGCTGTTGAAGCGTTATCGCTGATTTATAACAAAGATCTGC
128 K L I A Y P I A V E A L S L I Y N K D L

2101 TGCCGAACCCGCCAAAAACCTGGGAAGAGATCCCGGCGCTGGATAAAGAACTGAAAGCGA
148 L P N P P K T W E E I P A L D K E L K A

2161 AAGGTAAGAGCGCGCTGATGTTCAACCTGCAAGAACCGTACTTCACCTGGCCGCTGATTG
168 K G K S A L M F N L Q E P Y F T W P L I

2221 CTGCTGACGGGGGTTATGCGTTCAAGTATGAAAACGGCAAGTACGACATTAAAGACGTGG
188 A A D G G Y A F K Y E N G K Y D I K D V

2281 GCGTGGATAACGCTGGCGCGAAAGCGGGTCTGACCTTCCTGGTTGACCTGATTAAAAACA
208 G V D N A G A K A G L T F L V D L I K N

2341 AACACATGAATGCAGACACCGATTACTCCATCGCAGAAGCTGCCTTTAATAAAGGCGAAA
228 K H M N A D T D Y S I A E A A F N K G E

2401 CAGCGATGACCATCAACGGCCCGTGGGCATGGTCCAACATCGACACCAGCAAAGTGAATT
248 T A M T I N G P W A W S N I D T S K V N

2461 ATGGTGTAACGGTACTGCCGACCTTCAAGGGTCAACCATCCAAACCGTTCGTTGGCGTGC
268 Y G V T V L P T F K G Q P S K P F V G V

2521 TGAGCGCAGGTATTAACGCCGCCAGTCCGAACAAAGAGCTGGCGAAAGAGTTCCTCGAAA
288 L S A G I N A A S P N K E L A K E F L E

2581 ACTATCTGCTGACTGATGAAGGTCTGGAAGCGGTTAATAAAGACAAACCGCTGGGTGCCG
308 N Y L L T D E G L E A V N K D K P L G A

2641 TAGCGCTGAAGTCTTACGAGGAAGAGTTGGCGAAAGATCCACGTATTGCCGCCACCATGG
328 V A L K S Y E E E L A K D P R I A A T M

Factor Xa PstI
2701 AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI +2 trxA (2-109 del 103-107)
2761 TCGACGCCGAATCTAGAAGCGATAAAATTATTCACCTGACTGACGACAGTTTTGACACGG

368 V D A E S R S D K I I H L T D D S F D T

2821 ATGTACTCAAAGCGGACGGGGCGATCCTCGTCGATTTCTGGGCAGAGTGGTGCGGTCCGT
388 D V L K A D G A I L V D F W A E W C G P

2881 GCAAAATGATCGCCCCGATTCTGGATGAAATCGCTGACGAATATCAGGGCAAACTGACCG
408 C K M I A P I L D E I A D E Y Q G K L T

2941 TTGCAAAACTGAACATCGATCAAAACCCTGGCACTGCGCCGAAATATGGCATCCGTGGTA
428 V A K L N I D Q N P G T A P K Y G I R G

3001 TCCCGACTCTGCTGCTGTTCAAAAACGGTGAAGTGGCGGCAACCAAAGTGGGTGCACTGT
448 I P T L L L F K N G E V A A T K V G A L

FLAG
3061 CTAAAGGTCAGTTGAAAGAGAACCTGGCGGATTATAAAGATGACGATGACAAATAATAAG
468 S K G Q L K E N L A D Y K D D D D K

Lost XbaI
CTAGAGG (transcriptional stop)

### pMPX-71::malE(1-370 del 354-364)::FXa::Pstl, SalI, XbaI::TrxA(1-109 del 103-107)::FLAG

### Arabinose inducible, clone into Pstl, SalI, Xbal

Made by cutting TOPO Nsil-malE (1-370 del 354-364)::FXa::Pstl, SalI, XbaI::FLAG-NheI insertion with Nsil & Xbal and cloning into pMPX-79 cut with Pstl & Xbal.

### SEQ ID NO.: 284

### pMPX-92 MalE (1-370 del 354-364) MCS TrxA (2-109 del 103-107) fusion vector

SD  Lost PstI +1 malE (1-370 del 354-364)
AGGAGGTTCTGCATATGAAAATAAAAACAGGTGCAC
1                    M K I K T G A

GCATCCTCGCATTATCCGCATTAACGACGATGATGTTTTCCGCCTCGGCTCTCGCCAAAA
8    R I L A L S A L T T M M F S A S A L A K

TCGAAGAAGGTAAACTGGTAATCTGGATTAACGGCGATAAAGGCTATAACGGTCTCGCTG
28   I E E G K L V I W I N G D K G Y N G L A

AAGTCGGTAAGAAATTCGAGAAAGATACCGGAATTAAAGTCACCGTTGAGCATCCGGATA
48   E V G K K F E K D T G I K V T V E H P D

AACTGGAAGAGAAATTCCCACAGGTTGCGGCAACTGGCGATGGCCCTGACATTATCTTCT
68   K L E E K F P Q V A A T G D G P D I I F

GGGCACACGACCGCTTTGGTGGCTACGCTCAATCTGGCCTGTTGGCTGAAATCACCCCGG
88   W A H D R F G G Y A Q S G L L A E I T P

ACAAAGCGTTCCAGGACAAGCTGTATCCGTTTACCTGGGATGCCGTACGTTACAACGGCA
108  D K A F Q D K L Y P F T W D A V R Y N G

AGCTGATTGCTTACCCGATCGCTGTTGAAGCGTTATCGCTGATTTATAACAAAGATCTGC
128  K L I A Y P I A V E A L S L I Y N K D L

TGCCGAACCCGCCAAAAACCTGGGAAGAGATCCCGGCGCTGGATAAAGAACTGAAAGCGA

148 L P N P P K T W E E I P A L D K E L K A

AAGGTAAGAGCGCGCTGATGTTCAACCTGCAAGAACCGTACTTCACCTGGCCGCTGATTG
168 K G K S A L M F N L Q E P Y F T W P L I

CTGCTGACGGGGGTTATGCGTTCAAGTATGAAAACGGCAAGTACGACATTAAAGACGTGG
188 A A D G G Y A F K Y E N G K Y D I K D V

GCGTGGATAACGCTGGCGCGAAAGCGGGTCTGACCTTCCTGGTTGACCTGATTAAAAACA
208 G V D N A G A K A G L T F L V D L I K N

2341 AACACATGAATGCAGACACCGATTACTCCATCGCAGAAGCTGCCTTTAATAAAGGCGAAA
228 K H M N A D T D Y S I A E A A F N K G E

2401 CAGCGATGACCATCAACGGCCCGTGGGCATGGTCCAACATCGACACCAGCAAAGTGAATT
248 T A M T I N G P W A W S N I D T S K V N

2461 ATGGTGTAACGGTACTGCCGACCTTCAAGGGTCAACCATCCAAACCGTTCGTTGGCGTGC
268 Y G V T V L P T F K G Q P S K P F V G V

2521 TGAGCGCAGGTATTAACGCCGCCAGTCCGAACAAAGAGCTGGCGAAAGAGTTCCTCGAAA
288 L S A G I N A A S P N K E L A K E F L E

2581 ACTATCTGCTGACTGATGAAGGTCTGGAAGCGGTTAATAAAGACAAACCGCTGGGTGCCG
308 N Y L L T D E G L E A V N K D K P L G A

2641 TAGCGCTGAAGTCTTACGAGGAAGAGTTGGCGAAAGATCCACGTATTGCCGCCACCATGG
328 V A L K S Y E E E L A K D P R I A A T M

Factor Xa PstI
2701 AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI +2 trxA (2-109 del 103-107)
2761 TCGACGCCGAATCTAGAAGCGATAAAATTATTCACCTGACTGACGACAGTTTTGACACGG
368 V D A E S R S D K I I H L T D D S F D T

2821 ATGTACTCAAAGCGGACGGGGCGATCCTCGTCGATTTCTGGGCAGAGTGGTGCGGTCCGT
388 D V L K A D G A I L V D F W A E W C G P

2881 GCAAAATGATCGCCCCGATTCTGGATGAAATCGCTGACGAATATCAGGGCAAACTGACCG
408 C K M I A P I L D E I A D E Y Q G K L T

2941 TTGCAAAACTGAACATCGATCAAAACCCTGGCACTGCGCCGAAATATGGCATCCGTGGTA
428 V A K L N I D Q N P G T A P K Y G I R G

3001 TCCCGACTCTGCTGCTGTTCAAAAACGGTGAAGTGGCGGCAACCAAAGTGGGTGCACTGT
448 I P T L L L F K N G E V A A T K V G A L

FLAG
3061 CTAAAGGTCAGTTGAAAGAGAACCTGGCGGATTATAAAGATGACGATGACAAATAATAAG
468 S K G Q L K E N L A D Y K D D D D K

Lost XbaI
CTAGAGGTACC (transcriptional stop)

### pMPX-84::malE(1-370 del 354-364)::FXa::Pstl, SalI, XbaI::TrxA(1-109 del 103-107)::FLAG

### Temperature inducible, clone into Pstl, SalI, XbaI

Made by cutting TOPO Nsil-malE (1-370 del 354-364)::FXa::Pstl, SalI, XbaI::FLAG-NheI insertion with Nsil & Xbal and cloning into pMPX-90 cut with Pstl & Xbal.

### SEQ ID NO.: 285

### pMPX-97 MalE (1-370 del 354-364) MCS TrxA (2-109 del 103-107) fusion vector

SD  Lost PstI +1 malE (1-370 del 354-364)
AGGAGGTTCTGCATATGAAAATAAAAACAGGTGCAC
1                    M K I K T G A

GCATCCTCGCATTATCCGCATTAACGACGATGATGTTTTCCGCCTCGGCTCTCGCCAAAA
8    R I L A L S A L T T M M F S A S A L A K

TCGAAGAAGGTAAACTGGTAATCTGGATTAACGGCGATAAAGGCTATAACGGTCTCGCTG
28   I E E G K L V I W I N G D K G Y N G L A

AAGTCGGTAAGAAATTCGAGAAAGATACCGGAATTAAAGTCACCGTTGAGCATCCGGATA
48   E V G K K F E K D T G I K V T V E H P D

AACTGGAAGAGAAATTCCCACAGGTTGCGGCAACTGGCGATGGCCCTGACATTATCTTCT
68   K L E E K F P Q V A A T G D G P D I I F

GGGCACACGACCGCTTTGGTGGCTACGCTCAATCTGGCCTGTTGGCTGAAATCACCCCGG
88   W A H D R F G G Y A Q S G L L A E I T P

ACAAAGCGTTCCAGGACAAGCTGTATCCGTTTACCTGGGATGCCGTACGTTACAACGGCA
108  D K A F Q D K L Y P F T W D A V R Y N G

AGCTGATTGCTTACCCGATCGCTGTTGAAGCGTTATCGCTGATTTATAACAAAGATCTGC
128  K L I A Y P I A V E A L S L I Y N K D L

TGCCGAACCCGCCAAAAACCTGGGAAGAGATCCCGGCGCTGGATAAAGAACTGAAAGCGA
148  L P N P P K T W E E I P A L D K E L K A

AAGGTAAGAGCGCGCTGATGTTCAACCTGCAAGAACCGTACTTCACCTGGCCGCTGATTG
168  K G K S A L M F N L Q E P Y F T W P L I

CTGCTGACGGGGGTTATGCGTTCAAGTATGAAAACGGCAAGTACGACATTAAAGACGTGG
188  A A D G G Y A F K Y E N G K Y D I K D V

GCGTGGATAACGCTGGCGCGAAAGCGGGTCTGACCTTCCTGGTTGACCTGATTAAAAACA
208  G V D N A G A K A G L T F L V D L I K N

2341  AACACATGAATGCAGACACCGATTACTCCATCGCAGAAGCTGCCTTTAATAAAGGCGAAA
228   K H M N A D T D Y S I A E A A F N K G E

2401  CAGCGATGACCATCAACGGCCCGTGGGCATGGTCCAACATCGACACCAGCAAAGTGAATT
248   T A M T I N G P W A W S N I D T S K V N

2461  ATGGTGTAACGGTACTGCCGACCTTCAAGGGTCAACCATCCAAACCGTTCGTTGGCGTGC
268   Y G V T V L P T F K G Q P S K P F V G V

2521  TGAGCGCAGGTATTAACGCCGCCAGTCCGAACAAAGAGCTGGCGAAAGAGTTCCTCGAAA
288   L S A G I N A A S P N K E L A K E F L E

2581  ACTATCTGCTGACTGATGAAGGTCTGGAAGCGGTTAATAAAGACAAACCGCTGGGTGCCG
308   N Y L L T D E G L E A V N K D K P L G A

2641  TAGCGCTGAAGTCTTACGAGGAAGAGTTGGCGAAAGATCCACGTATTGCCGCCACCATGG

328 V A L K S Y E E E L A K D P R I A A T M

Factor Xa PstI
2701 AAAACGCCCAGTCCGCTTTCTGGTATGCCGTGCGTATCGAAGCCCGCCTGCAGGCCTCGG
348 E N A Q S A F W Y A V R I E A R L Q A S

SalI XbaI +2 trxA (2-109 del 103-107)
2761 TCGACGCCGAATCTAGAAGCGATAAAATTATTCACCTGACTGACGACAGTTTTGACACGG
368 V D A E S R S D K I I H L T D D S F D T

2821 ATGTACTCAAAGCGGACGGGGCGATCCTCGTCGATTTCTGGGCAGAGTGGTGCGGTCCGT
388 D V L K A D G A I L V D F W A E W C G P

2881 GCAAAATGATCGCCCCGATTCTGGATGAAATCGCTGACGAATATCAGGGCAAACTGACCG
408 C K M I A P I L D E I A D E Y Q G K L T

2941 TTGCAAAACTGAACATCGATCAAAACCCTGGCACTGCGCCGAAATATGGCATCCGTGGTA
428 V A K L N I D Q N P G T A P K Y G I R G

3001 TCCCGACTCTGCTGCTGTTCAAAAACGGTGAAGTGGCGGCAACCAAAGTGGGTGCACTGT
448 I P T L L L F K N G E V A A T K V G A L

FLAG
3061 CTAAAGGTCAGTTGAAAGAGAACCTGGCGGATTATAAAGATGACGATGACAAATAATAAG
468 S K G Q L K E N L A D Y K D D D D K

Lost XbaI
CTAGAGGTACC (transcriptional stop)

### pMPX-86::malE(1-370 del 354-364)::FXa::Pstl, SalI, XbaI::TrxA(1-109 del 103-107)::FLAG

### Temperature inducible, clone into Pstl, SalI, Xbal

Made by cutting TOPO Nsil-malE (1-370 del 354-364)::FXa::Pstl, SalI, XbaI::FLAG-NheI insertion with Nsil & Xbal and cloning into pMPX-95 cut with Pstl & Xbal.

## Claims

1. A composition comprising a fully intact eubacterial minicell, wherein said minicell comprises a therapeutic agent and displays a ligand or binding moiety wherein said ligand or binding moiety is specific for a eukaryotic cell receptor.

2. The composition of claim 1, wherein the therapeutic agent is
a drug,
a small molecule,
a nucleic acid or template thereof,
a combination of a small molecule and a nucleic acid or template thereof,
a combination of a drug and a nucleic acid or template thereof,
a polypeptide, or
a detectable compound or moiety.

3. The composition of claim 2, wherein the therapeutic agent is a drug.

4. The composition of claim 3, wherein the drug is a small molecule drug or a chemotherapy drug.

5. The composition of claim 3, wherein the drug is selected from an anti-depressant; and an agent for the treatment of cancer, obesity, insomnia, schizophrenia, or compulsive disorder.

6. The composition of claim 2, wherein the therapeutic agent is a nucleic acid or template thereof.

7. The composition of claim 6, wherein the nucleic acid or template thereof is selected from RNA and DNA, preferably selected from an antisense oligonucleotide, an aptamer, an antisense transcript, a ribosomal RNA (rRNA), a transter RNA (tRNA), and a molecular decoy.

8. The composition of claim 2, wherein the therapeutic agent is a polypeptide, preferably selected from a soluble polypeptide and a membrane polypeptide, preferably a soluble polypeptide comprising a cellular secretion sequence.

9. The composition of claim 8, wherein the polypeptide is selected from: an antibody or antibody derivative, and an enzyme

10. A composition comprising a fully intact eubacterial minicell, wherein said minicell comprises a detectable compound or moiety and displays a ligand or binding moiety wherein said ligand or binding moiety is specific for a eukaryotic cell receptor.

11. The composition of claim 10, wherein the detectable compound or moiety is a polypeptide or a nucleic acid.

12. The composition of any one of claims 10 to 11, wherein the detectable compound or moiety is detectable by spectroscopic, photochemical, biochemical, immunochemical, electromagnetic, radiochemical, or chemical means; wherein the chemical means comprises fluorescence, chemifluoresence, chemiluminescence, and electrochemilumenscence.

13. The composition of any one of claims 1 to 12, wherein said ligand or binding moiety comprises or is part of; (a) an antibody or antibody derivative; (b) a bacterial membrane protein; (c) a carbohydrate; (d) a polypeptide; (e) a nucleic acid; or (f) a fusion protein, said fusion protein comprising a first polypeptide, said first polypeptide comprising at least one transmembrane or at least one membrane anchoring domain, and a second polypeptide comprising said binding moiety.

14. The composition of any one of claims 1 to 13, wherein said ligand or binding moiety stimulates receptor mediated endocytosis of said minicell upon binding of said minicell to said eukaryotic cell receptor, wherein the therapeutic small molecule drug is released into said eukaryotic cell.

15. A method of treating a disease using the composition of any one of claims 1 to 14.
